# EUROPEAN PATENT APPLICATION

(11) **EP 1 677 113 A1**
(43) Date of publication of application: **05.07.2006**
(21) Application number: 04030994.0
(22) Date of filing: 29.12.2004
(51) Int. Cl.: G01N 33/68, C12N 15/10, C12N 15/00, C12N 5/00, C07K 16/00, C07K 14/00, A61K 38/00, A61K 39/395, A61K 48/00

(54) **Method for the identification of protein-protein interactions in disease related protein networks**

(71) Applicant: Max-Delbrück-Centrum für Molekulare Medizin (MDC), 13125 Berlin (DE)
(72) Inventor: Wanker, Erich, 13187 Berlin (DE); Lalowski, Maciej, 10119 Berlin (DE); Stelzl, Ulrich, 10405 Berlin (DE); Strödicke, Martin, 13347 Berlin (DE); Hänig, Christian, 10179 Berlin (DE); Worm, Uwe, 14513 Teltow (DE); Göhler, Heike, 10713 Berlin (DE)
(74) Representative: Vossius & Partner

(57) **Abstract**

The present invention relates to a method for identification of a protein-protein interaction of protein interaction partners in a disease-related network of proteins comprising the steps of (a) identifying nucleic acid molecules by at least partial 5'sequencing and, optionally, additionally adding recombinantly cloned and sequenced nucleic acid molecules, wherein said nucleic acid molecules encode a selection of proteins suspected to contain one or several of said interaction partners and wherein said nucleic acid molecules are annotated with a positional information; (b) in frame cloning of the nucleic acid molecules of step (a) into prey and bait vectors, wherein one copy of each nucleic acid molecule is cloned into said prey vector and a second copy of each nucleic acid molecule is cloned into said bait vector; (c) transforming a first suitable host cell with the prey vector obtained in step (b) and a second suitable host cell with the bait vector obtained in step (b), wherein said first and said second host cell have a different genetic constitution and can be mated; (d) mating the first suitable host cell of step (c) with the second suitable host cell of step (c), and expressing the proteins encoded by the nucleic acid molecules obtained in step (b); (e) selecting the mated host cell obtained in step (d) on the basis of a selection advantage which is caused by the protein-protein interaction between the protein interaction partner encoded by the nucleic acid molecule of the prey vector contained in said cell and the protein interaction partner encoded by the nucleic acid molecule of the bait vector contained in said cell; and (f) identifying with the positional information obtained in step (a) the protein interaction partners of step (e) and thereby identifying the protein-protein interaction.

### Annex to the application documents - subsequently filed sequences listing

SEQUENCE LISTING

<110>  Max-Delbrück-Centrum für Molekulare Medizin (MDC)

<120>  Method for the identification of Protein-Protein interactions in disease
related protein networks

<130>  K 2997 EP

<140>  EP 04 03 0994.0
<141>  2004-12-29

<160>  624

<170>  PatentIn version 3.1

<210>  1
<211>  592
<212>  DNA
<213>  Homo sapiens

<400>  1
gcccacgcgt ccgcccacgc gtccgcccac gcgtccgtcc acttcctcct gggaaatgag      60
gaggggcccg agtcctatga gttgcagata tgcgtgaagg attactgctt tgcccgggaa     120
gatcgcgtgc tagggctggc tgtgatgcct ctgagggatg tcacagccaa gggcagctgt     180
gcctgctggt gccccttggg ccggaagatc catatggatg agacaggcct gaccattctc     240
cggattttat ctcagaggag caatgacgag gtggcccgag aatttgtgaa actcaaatca     300
gagtctcgtt ccacggagga ggggagctga acaccttcga ctcctgtgcc aatcaggcag     360
cagcaatttc acaaatcagg gccagtggga gttagctgtg taaccggctt agggtctttg     420
cagtcaagag gctgacccct tcagttaaag atatttaagg aaaaatttgg ggtggtgata     480
atatggcttt tcacagaaag ggtcatgaag ccctggccca acaggactgt ggtactaggg     540
gctgggatgt ggggttacca catggagaga ttttccatta agagagaagg ac             592

<210>  2
<211>  569
<212>  DNA

<213>  Homo sapiens

<400>  2
gcggacgcgt gggcacccgc acccgcaccc ccatcaccac caccaccacc accaccacca     60

ccaccacgcc gtcggcctga atctctccca cgtgcgcaag cgctgcctct ccgagaccca    120

cggcgagcgc gagccctgca agaagcggct gactgcgcgc agcatcagca cccccacctg    180

cctggggggc agcccagccg ctgagcgccc ggccgacctg ccaccagccg ccgccctccc    240

gcagcccgag gtcatcctgc tagactcaga cctggatgaa cccatagact tgcgctgcgt    300

caagacgcgc agcgaggccg gggagccgcc cagctccctc caggtgaagc ccgagacacc    360

ggcgtcggcg gcggtggcgg tggcggcggc agcggcaccc accacgacgg cggagaagcc    420

tccagccgag gcccaggacg aacctgcaga gtcgctgagc gagttcaagc ccttctttgg    480

gaatataatt atcaccgacg tcaccgcgaa ctgcctcacc gttactttca aggagtacgt    540

gacggtgtag ccggagggcg tcggaaggg                                      569

<210>  3

<211>  584

<212>  DNA

<213>  Homo sapiens

<400>  3
gctgctggcc agtccacccc tagtccccac ctggtgcctt cacctgcccc atctccaggg     60

cctggtccgg taccccctcg ccccccagca gcagaaccac ccccttgcct gcgccgaggc    120

gccgcagctg cagacctgct ctcctccagc ccggagagcc agcatggcgg cactcagtct    180

cctgggggtg ggcagcccct gctgcagccc accaaggtgg atgcagctga gggtcgtcgg    240

ccgcaggccc tgcggctgat tgagcgggac ccctatgaac atcctgagag gctgcggcag    300

ttgcagcagg agctggaggc ctttcggggt cagctggggg atgtgggagc tctggacact    360

gtctggcgag agctgcaaga tgcgcaggaa catgatgccc gaggccgttc catcgccatt    420

gcccgctgct actcactgaa gaaccggcac caggatgtca tgccctatga cagtaaccgt    480

gtggtgctgc gctcaggcaa ggatgactac atcaatgcca gctgcgtgga ggggctctcc    540

ccatactgcc ccccgctagt ggcaacccag gccccactgc ctgg                     584

<210>  4

<211>  591

<212>  DNA

<213>  Homo sapiens

<400>  4

gcccacgcgt ccgctcatgc tggcaacaag ctggccatgc aggagttcat gatcctccca     60
gtgggagctg agagctttcg ggatgccatg cgactaggtg cagaggtcta ccatacactc    120
aagggagtca tcaaggacaa atacggcaag gatgccacca atgtggggga tgaaggtggc    180
tttgccccca atatcctgga gaacagtgaa gccttggagc tggtgaagga agccatcgac    240
aaggctggct acacggaaaa gatcgttatt ggcatggatg ttgctgcctc agagttttat    300
cgtgatggca aatatgactt ggacttcaag tctcccactg atccttcccg atacatcact    360
ggggaccagc tgggggcact ctaccaggac tttgtcaggg actatcctgt ggtctccatt    420
gaggacccat ttgaccagga tgattgggct gcctggtcca agttcacagc caatgtaggg    480
atccagattg tgggtgatga cctgacagtg accaacccaa aacgtattga gcgggcagtg    540
gaagaaaagg cctgcaactg tctgctgctc aaggtcaacc agatcggctc g             591

<210>  5
<211>  554
<212>  DNA
<213>  Homo sapiens

<220>
<221>  misc_feature
<222>  (212)..(212)
<223>  n = a, c, g or t

<220>
<221>  misc_feature
<222>  (497)..(497)
<223>  n = a, c, g or t

<400>  5
gaagcagcca aagtaacagg caaggcggac cctgccttcg gtctggaaag cagtggcatt     60
gcaggaacca cctctgatga gcctgagcgg attgaggaga gcgggaatga cgaggctcgg    120
gtggaaggcc aggccacaga tgagaagaag gagcccaagg aaccccgaga aggagggggt    180
gctatagagg aggaagcaaa agagaaaacc ancgaggctc ccaagaagga tgaggagaaa    240
gggaaagaag gcgacagtga gaaggagtcc gagaagagtg atggagaccc aatagtcgat    300
cctgagaagg agaaggagcc aaaggaaggg caggaggaag tgctgaagga agtggtggag    360
tctgaggggg aaaggaagac aaaggtggag cgggacattg gcgagggcaa cctctccacc    420
gctgctgccg ccgccctggc cgccgccgca gtgaaagcta agcacttggc tgctgttgag    480
gaaaggaaga tcaaatnttt ggtggccctg ctggtggaga cccagatgaa aaagttggag    540

atcaaacttc ggca 554

<210> 6

<211> 559

<212> DNA

<213> Homo sapiens

<400> 6
gctcaagaac aagaatctga agcacaaaga taccatgtgg atgacggcag gaggcacaga 60

gctcaccacg agcctgaacg gctttccaga aggagtgagg atgagcagag atgggggaaa 120

ggacctggcc aagacagagg gaagaagggg agccaggaca gcggggctcc gggggaggcc 180

atggagagac tgggaagagc gcagaggtgt gacgacagtc cagcacccag aaaagagcga 240

ctggcaaaca aggaccggcc agccttgcag ctgtatgatc caggagctcg cttccgagcg 300

cgagagtgtg gcggaaacag gaggatctgc aaggcagaag gttcggggac tggtcctgag 360

aagagggaag aggcagagtg agtcactgca cgcacctggc ctccatggac gagcaagggc 420

atcccagaaa cgtgtaaatg accccgagtg tgactgggaa ggagaactta ttccttacca 480

ggaaactgga agctaaaaat acagagggtg acgtagaaac acgcagaaac cattctaaag 540

aaagtagtga tcttgtatt 559

<210> 7

<211> 573

<212> DNA

<213> Homo sapiens

<400> 7
gcggacgcgt gggcggattt atttcaagtg aacctggaag tggaggtgga gcccagggac 60

aggccgagcc gggaagcccc accatgggag aactcgagca tgagggggct gaaccccaaa 120

atcctgtttt ccagccggga ggagcagcaa gacattctgt ctaagtttgg gaagccggag 180

cttccccggc agcctggctc cacggctcag tatgatgctg gggcagggtc cccggaagcc 240

gaacccacag actctgactc accgccaagc agcagcgcgg acgccagtcg cttcctgcac 300

acgctggact ggcaggaaga gaaggaggca gagactggtg cagaaaatgc ctcttccaag 360

gagagcgagt ctgccctgat ggaggacaga gacgagagtg aggtgtcaga tgaaggggga 420

tccccgatct ccagcgaggg ccaggaaccc agggccgacc cagagccccc cggcctggca 480

gcagggctgg tgcagcagga cttggttttt gaggtggaga caccggctgt gctgccagag 540

cctgtgccac aggaagacgg ggtcgacctc ctg 573

<210> 8

<211>  533

<212>  DNA

<213>  Homo sapiens

<400>  8
gaccctgatg aagctgttgc ttatggtgca gctgtccagg cagccatctt gtctggagac      60

aagtctgaga atgttcaaga tttgctgctc ttggatgtca ctcctctttc ccttggtatt     120

gaaactgctg gtggagtcat gactgtcctc atcaagcgta ataccaccat tcctaccaag     180

cagacacaga ccttcactac ctattctgac aaccagcctg gtgtgcttat tcaggtttat     240

gaaggcgagc gtgccatgac aaaggataac aacctgcttg gcaagtttga actcacaggc     300

atacctcctg caccccgagg tgttcctcag attgaagtca cttttgacat tgatgccaat     360

ggtatactca atgtctctgc tgtggacaag agtacgggaa aagagaacaa gattactatc     420

actaatgaca agggccgttt gagcaaggaa gacattgaac gtatggtcca ggaagctgag     480

aagtacaaag ctgaagatga gaagcagagg gacaaggtgt catccaagaa ttc            533

<210>  9

<211>  565

<212>  DNA

<213>  Homo sapiens

<400>  9
gacgctgagg acatgacatc caaagattac tactttgact cctacgcaca ctttggcatc      60

cacgaggaga tgctgaagga cgaggtgcgc accctcactt accgcaactc catgtttcat     120

aaccggcacc tcttcaagga caaggtggtg ctggacgtcg gctcgggcac cggcatcctc     180

tgcatgtttg ctgccaaggc cggggcccgc aaggtcatcg ggatcgagtg ttccagtatc     240

tctgattatg cggtgaagat cgtcaaagcc aacaagttag accacgtggt gaccatcatc     300

aaggggaagg tggaggaggt ggagctccca gtggagaagg tggacatcat catcagcgag     360

tggatgggct actgcctctt ctacgagtcc atgctcaaca ccgtgctcta tgcccgggac     420

aagtggctgg cgcccgatgg cctcatcttc ccagaccggg ccacgctgta tgtgacggcc     480

atcgaggacc ggcagtacaa agactacaag atccactggt gggagaacgt gtatggcttc     540

gacatgtctt gcatcaaaga tgtgg                                           565

<210>  10

<211>  581

<212>  DNA

<213>  Homo sapiens

<400> 10
gcttctacag ctgaagccat aggtttaaaa ggaagttctc ctactccccc ttgttctcca 60

gtacaacctt caaaacaact ggaatattta gcaaggattc aaggctttca ggcagcctta 120

agtgccttga aacaattttc tgaacaagga ctggatccaa tcgatggagc aatgaatatc 180

gaaaaaggtt ctcttgaaaa acaagccaag catctgagag agaaagcgga caataaccag 240

gcacccccgg gctccatcgc tcaggactgc aagaaatcaa actcggccgt ctagcagctc 300

ccagaacccg cggctgccac cgcatcctta taaacctgtc agcacgcatg agggtgtctg 360

tgttcaggga aatgaatgac taataccatt atttgagtct tatgtgaaga caacactatt 420

ctaacacgag agataatata catggtactg tttattcaac tggggaaaaa taaaactttg 480

agcatttccc ttggaactcg agatcagatc ataactcatt tgcctagagg cagcagaaat 540

ctgatcctgc tactggagct taaaataaca agtaaagaaa a 581

<210> 11

<211> 585

<212> DNA

<213> Homo sapiens

<400> 11
gcgggatttg ggtcgcggtt cttgtttgtg gatcgctgtg atcgtcactt gacaatgcag 60

atcttcgtga agactctgac tggtaagacc atcaccctcg aggttgagcc cagtgacacc 120

atcgagaatg tcaaggcaaa gatccaagat aaggaaggca tccctcctga ccagcagagg 180

ctgatctttg ctggaaaaca gctggaagat gggcgcaccc tgtctgacta caacatccag 240

aaagagtcca ccctgcacct ggtgctccgt ctcagaggtg ggatgcaaat cttcgtgaag 300

acactcactg gcaagaccat cacccttgag gtggagccca gtgacaccat cgagaacgtc 360

aaagcaaaga tccaggacaa ggaaggcatt cctcctgacc agcagaggtt gatctttgcc 420

ggaaagcagc tggaagatgg gcgcaccctg tctgactaca acatccagaa agagtctacc 480

ctgcacctgg tgctccgtct cagaggtggg atgcagatct tcgtgaagac cctgactggt 540

aagaccatca ccctcgaggt ggagcccagt gacaccatcg agaat 585

<210> 12

<211> 502

<212> DNA

<213> Homo sapiens

<400> 12
gcccacgcgt ccgcagagga tgattcaggc gaggcgtcat ctcagaactc tatgtccagc 60

catggaaagc agggggcccc caagaagcgg aaggcgcctc cacctcccgc cagcattccc 120

atgccggatg atcgagatgc ctatgaggag atcgtgaggc tgcggcagga gaggggccgc 180

ctcctgcaga agatccgggg cctggaacag cacaaggaac agaggcagca ggagtccccg 240

gaggccagct ccctgcacat cctggagaga caggtgcaag agctacagca gttgctggtg 300

gagagacaag aggagaagga gagcctgtga cgggaggtgg agagtttgca gagccggctg 360

tccctgctgg agaacgagcg gcagaatact agctatgacg taaccaccct gcaggatgag 420

gagggtgagc tgcctgacct tccaggggcc gaggtgctgc tgtccagaca actcagtccg 480

tcggcccagg aacacctggc ct 502

<210> 13

<211> 564

<212> DNA

<213> Homo sapiens

<400> 13
gggagagaac catccgggac aactttggag gtggaaacac tgcctgggag gaagagaatt 60

tgtccaaata caaagacagc ctgtcctggg ggaacagaga ggccctgcgg tggctacggg 120

cagtgtgaag gagaagggac acgagggggc agcgggcact gtgactgcca agccggctac 180

gggggtgagg cctgtggcca gtgtggcctt ggctactttg aggcagaacg caacgccagc 240

catctggtat gttcggcttg ttttggcccc tgtgcccgat gctcaggacc tgaggaatca 300

aactgtttgc aatgcaagaa gggctgggcc ctgcatcacc tcaagtgtgt agacattgat 360

gagtgtggca cagagggagc caactgtgga gctgaccaat tctgcgtgaa cactgagggc 420

tcctatgagt gccgagactg tgccaaggcc tgcctaggct gcatgggggc agggccaggt 480

cgctgtaaga agtgtagccc tggctatcag caggtgggct ccaagtgtct cggtgagtct 540

cctgctgatg ggacacaggc acct 564

<210> 14

<211> 462

<212> DNA

<213> Homo sapiens

<400> 14
gggctatctg acaaccagtc ccagcccctg cttacattcc agacagacac agactacacc 60

acaggagaga agattttaac ctgtgtctta cttcctcagc tgccttctga acctacttgt 120

caagagaatc tgcctcgctg tggtgcactg gagaacttgg taaatgatgt ctctgatgaa 180

gcctggaacg agcgtgctgt caacagagtc agtgcgatcc gatttgtgga ggatgagaaa 240

gatgaagaag acaatgagac gagaacactt ctaaggcgag ccactccaca cccaggggag 300

ttaaagcaca tgaaaaagac agtggagaat ttacggaatg acatgaatgc tgctaaagga 360

ctggactcaa acaaaaacga ggtcaatcat gccattgacc gagtgaccac ttttgtgtag 420

agtttcacct tcaagtttta cctcctgtgt cttcctctgc tg 462

<210> 15

<211> 581

<212> DNA

<213> Homo sapiens

<400> 15
gattctgaac ctgctggttt agaaacacca gaagcgaaag tactttttga caaagtagct 60

tctcaagggg aagtagttcg gaaacttaaa actgaaaaag cccctaagga tcaagtagat 120

atagctgttc aagaactcct tcagctaaag gcacagtaca agtctttgat aggagtagag 180

tataagcctg tgtcggccac tggagctgag gacaaagata agaagaagaa agaaaaagaa 240

aataaatctg aaaagcagaa taagcctcag aaacaaaatg atggccaaag gaaagaccct 300

tctaaaaatc aaggaggtgg gctctcatca agtggagcag gagaagggca ggggcctaag 360

aaacagacca ggttgggtct tgaggcaaaa aaagaagaaa atcttgctga ttggtattct 420

caggtcatca caaagtcaga aatgattgaa taccatgaca taagtggctg ttatattctt 480

cgtccctggg cctatgccat ttgggaagcc atcaaggact tttttgatgc tgagatcaag 540

aaacttggtg ttgaaaactg ctacttcccc atgtttgtgt c 581

<210> 16

<211> 576

<212> DNA

<213> Homo sapiens

<400> 16
gcccacgcgt ccgataaact acataccttg gccaattcca gctccctgtg caccagctct 60

aatggaagcc tctcgtctgg catgacctct ggctataata gcagcccagc cttgtcaggc 120

cagctccagt tcccagagaa tatggggcat acaaacttgc ctgattccag ggatgtatgg 180

ataggggatg agcgaggagg ccattctgca gtgaggaaga actctgccta cagtgagttg 240

cggggagtgt tgggcatagc cagctgcctg gtttggggac ttgggggctg atggctgcta 300

gatcttagtg agtcactggt ctgtgggaga gctgttgctg tcctgaggcc ctgatgttca 360

gtgctatcac agggcctcca cctatttctt gtaggccaca gagcctccct gggcagttgc 420

tgctgttcac catccagtct gtccagcttg gggacctgct tttcctcctc ctaccaggat 480

ttggccaagc atgtcgtgga cacttctatg gctgatgtaa tggctgcttg ttcggataat 540

ttgcacaacc tcttcagctg ccaggcaact gctggc 576

<210> 17

<211> 545

<212> DNA

<213> Homo sapiens

<400> 17
gcggacgcgt gggagcagat ggtgcccatc aaggagatga cagacgtgct caaagtggtg 60

aaggaggtgg ccaacctgaa accaaagtcc tgggtccgcc tcaagcgggg catctacaag 120

gatgacattg ctcaggtgga ctacgtggag cccagccaga acaccatctc cctgaagatg 180

atcccacgca tcgactacga tcgcatcaag gcccgcatga gcttgaaaga ctggtttgcc 240

aaaaggaaga agtttaagcg gcctccacag aggctgtttg atgctgagaa gatcaggtcc 300

ctggggggtg atgttgcctc tgatggtgac ttcctcatct ttgaggggaa ccgttacagc 360

cggaagggct ttctgttcaa gagcttcgcc atgtctgctg tgatcacgga gggtgtgaag 420

ccaacactct ctgagctgga aaagtttgag gaccagccag agggcattga cctggaggtg 480

gtgactgaga gcacagggaa ggagcgggag cacaacttcc aacctgggga ccaacgtgga 540

ggtct 545

<210> 18

<211> 406

<212> DNA

<213> Homo sapiens

<400> 18
gcccacgcgt ccgcccacgc gtccgaaagg gaacaagagc ccttcgcctc cagatggctc 60

ccctgccgcc acccccgaga tcagagtcaa ccacgagcca gagccggccg gcggggccac 120

gcccggggcc accctcccca agtccccatc tcagctccgg aaaggcccac cagtccctcc 180

gcctcccaaa cacaccccgt ccaaggaagt caagcaggag cagatcctca gcctgtttga 240

ggacacgttt gtccctgaga tcagcgtgac caccccctcc cagtttgagg ccccggggcc 300

tttctcggag caggccagtc tgctggacct ggactttgac cccctcccgt ccgtgacgag 360

ccctgtgaag gcacccacgc cctctggtca gtcaattcca tgggac 406

<210> 19

<211> 583

<212> DNA

<213> Homo sapiens

<400> 19
gcggacgcgt gggacaaacg tctggatgaa tgggtgacgc atgagcggct ggacctaaag 60
aagatccagt tccccaagaa agaggccaag acccccacta agaacggact tcctgggtcc 120
cgtcctggct ctccagagag agaggtgaaa cggaaggtgg aggtggtttc accagcaact 180
ccagtgccca gcgagacagc cccggcctcg gtttttcccc agaatggagc cgcccgtagg 240
gcagtggcag cccagccagg acggaagcga aaatcgaatt gtttgggcac tgatgaggac 300
tcccaggaca gctctgatgg aataccgtca gcaccacgca tgactggcag cctggtgtct 360
gatcgaagcc acgacgacat cgtcacccgg atgaagaaca ttgagtgcat tgagctgggc 420
cggcaccgcc tcaagccgtg gtacttctcc ccgtacccac aggaactcac cacattgcct 480
gtcctctacc tgtgcgagtt ctgcctcaag tacggccgta gtctcaagtg tcttcagcgt 540
catttgacca agtgtgacct acgacatcct ccaggcaatg aga 583

<210> 20
<211> 557
<212> DNA
<213> Homo sapiens

<400> 20
gcagaaaagc tcagagaagg agctgcacag aggctttcag cagctccttc aggaactcaa 60
ccagcccaga gatggcttcc agctgagcct cggcaatgcc cttttcaccg acctggtggt 120
agacctgcag gacaccttcg taagtgccat gaagacgctg tacctggcag acactttccc 180
taccaacttt agggactctg caggggccat gaagcagatc aatgattatg tggcaaagca 240
aacgaagggc aagattgtgg acttgcttaa gaacctcgat agcaatgcgg tcgtgatcat 300
ggtgaattac atcttcttta aagctaagtg ggagacaagc ttcaaccaca aaggcaccca 360
agagcaagac ttctacgtga cctcggagac tgtggtgcgg gtacccatga tgagccgcga 420
ggatcagtat cactacctcc tggaccggaa cctctcctgc agggtgggtg ggggtcccct 480
accaaggcaa tgccacggct ttgttcattc tccccagtga gggaaagatg cagcaggtgg 540
agaatggact gagtgag 557

<210> 21
<211> 583
<212> DNA
<213> Homo sapiens

<400> 21
gccgcttctt ctacaagtat ggagaaggca aaaggcaagg agtggacctc cacagagaag 60

tcgagggaag aggatcagca ggcttctaat caaccaaatt caattgcttt gccaggaaca 120
tcagcaaaga gaaccaaaga aaaaatgtct atcaaaggca gtaaagtgct ctgccctaag 180
aaaaaggcag agcacactga caaccccaga cctcagaaga agataccaat ccctccatta 240
ccttctaaac tgccacctgt taatctgatt caccgggaca ttctgcgggc ctggtgccaa 300
caattgaagc tgagctccaa aggccagaaa ttggatgcat ataagcgcct gtgtgccttt 360
gcctacccaa atcaaaagga ttttcctagc acagcaaaag aggccaaaat ccggaaatca 420
ttgcaaaaaa aattaaaggt ggaaaagggg gaaacgtccc tgcaaagttc tgagacacat 480
cctcctgaag tggctcttcc tcctgtgggg gagccgcctg ccctggaaaa ttccactgct 540
ctccttgagg gagttaatac agttgtggtg acaacttctg ccc 583

<210> 22
<211> 562
<212> DNA
<213> Homo sapiens

<400> 22
gcccacgcgt ccggaggccg cggcgcggtc actgcgagcc gagccgagcc gcgccgagcc 60
gccccgatcg ccatccggcc tcggcactcg cgcgcgatcc cggccggcgg cgcggcccgg 120
cgggccaggc ggcgccacag cccatggagc tcgagaacat cgtagcgaac acggtgctac 180
tcaaggcccg ggaaggtggc ggtggaaatc gcaaaggcaa aagcaagaaa tggcggcaga 240
tgctccagtt ccctcacatc agccagtgcg aagagctgcg gctcagcctc gagcgtgact 300
atcacagcct gtgcgagcgg cagcccattg ggcgcctgct gttccgagag ttctgtgcca 360
cgaggccgga gctgagccgc tgcgtcgcct tcctggatgg ggcggccgag tatgaagtga 420
ccccggatga caagcggaag gcatgtgggc ggcagctaac gcagaatttt ctgagccaca 480
cgggtcctga cctcatccct gaggtccccc ggcagctggt gacgaactgc acccagcggc 540
tggagcaggg tccctgcaaa ga 562

<210> 23
<211> 463
<212> DNA
<213> Homo sapiens

<400> 23
gcgggaatga atggatgggc ggcctcagcg cccgcccgac cgctgggagg accgacccgg 60
cggggacctg ctgggggcag gacccgggga gcaagatggc cactgtcatc cctggccccc 120
tgagcctagg cgaggacttc taccgcgagg ccatcgagca ctgccgcagt tacaacgcgc 180
gcctgtgcgc cgagcgcagc ctgcgactgc ccttcctcga ctcgcagacc ggcgtggccc 240

agaacaactg ctacatctgg atggagaaga cccaccgcgg gccgggtttg gccccgggac 300

agatttacac gtaccccgcc cgctgttgga ggaagaaacg gagactcaac atcctggagg 360

accccagact caggccctgc gagtacaaga tcgactgtga agcacccctg aagaaggagg 420

gtggcctccc ggaagggccg gtcctcgagg ctctactgtg tgc 463

<210> 24

<211> 570

<212> DNA

<213> Homo sapiens

<400> 24
gcggacgcgt gggcagagag ccatgaaact ccagaaactc catttgtggg tcactggaat 60

ttgagtactg gtgttctgca tcagcgagag agtcctgaat ctgacactgg cagtgctacc 120

acctcctccg atgacataaa gcccagatct gaagactatg atgctggagg gtctcaggat 180

gatgatgggt caaatgacag aggtatctct aaatgtggca ctatgctgtg ccatgatttt 240

cttggaagaa gtagcagtga taccagtact cctgaagaat taaaaattta tgatagtaat 300

ttaagaattg aagtaaaaat gaaaaagcaa agtagtaatg atcttttcca agttaattca 360

acgagtgatg atgaaatccc taggaaaagg ccagaaattt ggtctcgatc tgcaatagtt 420

cactctaggg aaagagaaaa tattccacga ggcagtgtcc agtttgctca ggaaatagat 480

caggtatctt cttcagcaga tgaaacagaa gatgaaagat ctgaagctga aaacgttgca 540

gaaaatttct ctatatctaa cccagctcct 570

<210> 25

<211> 572

<212> DNA

<213> Homo sapiens

<400> 25
gaacatgcct cagtatatcc tgcgagagtt caaggtcaca gatgcccggg atggccagtc 60

ccggactgtc cggagttccg ttacagactg gccggaacag ggtgtgccaa agtcggggga 120

gggcttcatc gacttcattg gccaagtgca taagactaag gagcagtttg gccaggacgg 180

ccccatctct gtccactgca gtgccggcgt gggcaggacg ggcgtcttca tcacgcttag 240

catcgtgctg gagcggatgc ggtatgaagg cgtggtggac atctttcaga cggtgaagat 300

gctacgaacc cagcggccgg ccatggtgca gacagaggat gagtaccagt tctgttacca 360

ggcggcactg gagtacctcg gaagctttga ccactatgca acctaaagcc atggtccccc 420

ccaggcccga caccactggc cccggatgcc tctgcccctc ccgggcggac ctcctgaggc 480

ctggaccccc agtgggcagg gcaggaggtg gcagcggcag cagctgtgtt tctgcaccat 540
ttccgaggac gacgcagccc ctcgagcccc cc 572

<210> 26
<211> 519
<212> DNA
<213> Homo sapiens

<400> 26
gcccacgcgt ccggcggccg tggcggggat gctgcgaggg ggtctcctgc cccaggcggg 60
ccggctgcct accctccaga ctgtccgcta tggctccaag gctgttaccc gccaccgtcg 120
tgtgatgcac tttcagcggc agaagctgat ggctgtgact gaatatatcc ccccgaaacc 180
agccatccac ccatcatgcc tgccatctcc tcccagcccc ccacaggagg agataggcct 240
catcagagag caacgcgaga aggattctgt catgtcggcc aatgggaagc cagatcctga 300
cactgttccg gactcgtagc cagcctgttt agccagccct gcgcataaat acactctgcg 360
ttattggctg tgctctcctc aatgggacat gtggaagaac ttggggtcga ggagtgtgtt 420
tgtcacttgg ttttcactag taatgatatt gtcaggtata gggccacttg gagatgcaga 480
ggattccatt tcagatgtca gtcaccggct tcgtcctta 519

<210> 27
<211> 578
<212> DNA
<213> Homo sapiens

<400> 27
ggggcggagc tggaacgggc caagagcgcc ttgctggagg agcagaagtc tgtcatgctc 60
aagtgcgggg aggagcggcg gcgcctggct gccgagtggg cggagttctc cgcgcagcaa 120
aagctgagta aggagcgggc cgagcgcgag gccgagcggg cattgcaggt ggacacccag 180
cgggagggca ccctcatcag cctggccaag gagcaggctg agctgaagat cagggccagc 240
gagctccggg ccgaggagaa gcagctggca gcggagagag cagccctgga gcaggagcgg 300
caggagctgc ggctggagaa ggagaggatc aacgccaccg ccctgcgtgt caagctccgc 360
gccgaggagg tggagagcat gagcaaggtg gcctcgagaa ggtacgagga gggggagcgg 420
gcattgcgcg aggcccagca ggtgcaggca gagcagcagg cccggttgca ggcggtgcag 480
caacagcagg agcggctgcg gaagcaggag cagcacatgc accaggagca tctgagtctg 540
gcccagcaga ggctgcaact ggaccgcgca cgacagga 578

<210> 28

<211>  576

<212>  DNA

<213>  Homo sapiens


<400>  28
gagcagcagc tccagttctc cctggatcca gaacccattg ctatcaactg tacagccttc      60

aatcacaacg ggaacctgct ggtacagggg cagctgatgg cgtcatccgg ctgtttgttc     120

atccagtgga acatccacaa gagtggcctc aaggtatccg agtacagcct cccctcagat     180

gccacgggcc cctttgtgct gtctggatac agcggctaca agcaggttca agtccccagg     240

ggccgactct tcgcttttga ctcagaggga aattacatgc tgacatgttc tgccacaggc     300

ggcgtcatct acaagctggg tggcgatgag aaggttctgg agagctgctt gagcctaggt     360

ggccaccgag cccctgtggt caccgtggac tggagcactg ccatggactg tgggacctgc     420

ctcaccgcct ccatggatgg caagatcaag ctgaccaccc tcctggccca taaagcctga     480

tgacttgccc caagggccac ccgaggaagc agtattatct gcggtggggg gaggagatat     540

aggacaggaa aaccacgtgc tccactccag ctccct                               576

<210>  29

<211>  561

<212>  DNA

<213>  Homo sapiens


<400>  29
gcccacgcgt ccgcccacgc gtccggggaa gaagaggaag aagagaagga acctgaagca      60

gaacctcctg tacctcccca ggtgtcagta gaggtggcct tgcccccacc tgcagagcat     120

gaagtaaaga aagtgacttt aggagatacc ttaactcgac gttccattag ccagcagaag     180

tccggagttt ccattaccat tgatgaccca gtccgaactg cccaggtgcc ctccccaccc     240

cggggcaaga ttagcaacat tgtccatatc tccaatttgg tccgtccttt cactttaggc     300

cagctaaagg agttgttggg gcgcacagga accttggtgg aagaggcctt ctggattgac     360

aagatcaaat ctcattgctt tgtaacgtac tcaacagtag aggaagctgt tgccacccgc     420

acagctctgc acggggtcaa atggccccag tccaatccca aattcctttg tgctgactat     480

gccgagcaag atgagctgga ttatcaccga ggcctcttgg tggaccgtcc ctctgaaact     540

aagacagagg agcagggaat a                                               561

<210>  30

<211>  504

<212>  DNA

<213>  Homo sapiens

<220>

<221>  misc_feature

<222>  (452)..(452)

<223>  n = a, c, g or t


<400>  30
gaggatcgca gatggtctac agaattggtg cctgaaggcc ttcagtctcc acggggtaca      60

cctgatagtg agagctctca aggaagcttg gaacttctga gctatgagga aagccaaaag     120

agcaaactag agtctgtcat ttcagatgaa ggagacttgc agtttccatc acctaagata     180

tccagcagtc caaaatttga ttcacgggac aatgccctca gtgcctcaaa tgagactagc     240

agtgcagagc atttgaagga tggaactatg aaggaaatgg tggtctgcag ttctgagtct     300

attacctgta aaccacagct gaaagactcc ttcatttcaa atagtctacc tacttttttt     360

tatatccccc aacaagaccc actgaaaaca aattcccaac tagacacaag tatccaaaga     420

aacaaactat tggaaaatga agacacagcg gnggaagctc ttactttgga tatcaacagg     480

gaaactagaa ggtatcactg ctca                                            504

<210>  31

<211>  593

<212>  DNA

<213>  Homo sapiens


<400>  31
gatcatgcaa gattccaaaa ggtacgagct gagattgcca gcaaagagaa agaggaagag      60

gaactgatgg aaaagcccca gaaagaaacc aagaaagatg aggatcctga aaataaaatt     120

gaatttaaaa cacgtctggg ccgcaatgtt taccgaatgc tttttaagag caaagcatat     180

gagcggaatg agttgttcct gccgggccgc atggcctatg tggtagacct ggatgatgag     240

tatgctgaca cagatatccc caccactctt atccgcagca aggctgattg ccccaccatg     300

gaggcccaga ccacactgac cacaaatgac attgtcatta gcaagctgac ccagatcctt     360

tcatacctga ggcagggaac ccgtaacaag aagcttaaga agaaggataa agggaagctg     420

gaagagaaga aacctcctga ggctgacatg aatatttttg aagacattgg ggattacgta     480

ccctccacaa ccaagacacc tcgggacaag gagcgggaga gatatcggga acgggagcgt     540

gatcgggaaa gagacagaga ccgtgaccga gagcgagagc gagaacgaga tcg            593

<210>  32

<211>  598

<212>  DNA

<213>  Homo sapiens

<400>  32
gggacatccc ccaggcttcc ccgcagcagg ttcagcgctg tgtggagctc ctgagccggg     60

ccaagaggcc tctgatggtg ctggggagtc aggccctgct caccccaacg tctgccgaca    120

agcttcgggc tgccgtggag accttgggtg ttccctgctt ccttggaggg atggcacggg    180

ggctgttagg ccgcaaccac cccctccaca tccgggagaa ccgcagtgcg gccctgaaga    240

aggcggatgt cattgtccta gcaggaactg tgtgtgactt ccgcctatcc tatggccgtg    300

tcctcagcca cagcagcaag atcatcatcg tcaatcgtaa tcgggaagag atgttgctca    360

actcagacct cttctggaag ccccaggagg ctgtgcaggg agatgtgggt tccttcgtgc    420

tgaagttagt ggagggcctt cagggccaga cctgcgcccc agactgggtg gaggagctgc    480

gggaagccga ccggcagaag gagcagacct ttcgggagaa ggcagcgatg cctgtggccc    540

agcacctgaa cccagtgcag gtgctgcagc tggtggagga aacgctacct gacaactc      598

<210>  33

<211>  537

<212>  DNA

<213>  Homo sapiens

<400>  33
gcggtgtacc tcagcacccg cacccacggc gccgtcttca acctcgagta ctcgcccgac     60

gggtcagtgc tgacagttgc ttgtgaacaa actgaagttc tgctttttga ccctatatct    120

tcaaagcaca taaaaacact ttctgaagct catgaagact gtgtaaataa tatcagattt    180

cttgataacc ggctgtttgc tacctgctct gatgacacta caatagcact atgggatctg    240

agaaaattga acaccaaagt atgcacttta catggtcaca ctagctgggt gaagaacatc    300

gaatatgata ctaatacaag actcttagta acatcaggat ttgatggaaa tgtcattatt    360

tgggacacta acaggtatac agaagatggg tgtccacata agaaattctt tcacacacgt    420

tttctcatgc gaatgaggtt aacaccagat tgttccaaaa tgttgatttc aacgtcctct    480

ggatatctct taattttgca tgaccttgac ttaactaagt ctttagaagt aggcagc      537

<210>  34

<211>  578

<212>  DNA

<213>  Homo sapiens

<400>  34

gcccacgcgt ccgcccacgc gtccgcccac gcgtccgggt ttagaaggta taaggttcct 60

gatggaaaac cagaaaatga gtttgcgttt aatgcagaat ttaaagataa ggactttgcc 120

attgatatta ttaaaagcac tcatgaccat tggaaagcat tagtgactaa gaaaacgaat 180

ggaaaaggaa tcagttgcat gaatacaact ttgtctgaga gccccttcaa gtgtgatcct 240

gatgctgcca gagccattgt ggatgcttta ccaccaccct gtgaatctgc ctgcacagta 300

ccaacagacg tggataagtg gttccatcac cagaaaaact aatgagattt ctctggaata 360

caagctgata ttgctacatc gtgttcatct ggatgtatta gaagtaaaag tagtagcttt 420

tcaaagcttt aaatttgtag aactcatcta actaaagtaa attctgctgt gactaatcca 480

atatactcag aatgttatcc atctaaagca tttttcatat ctcaactaag ataactttta 540

gcacatgctt aaatatcaaa gcagttgtca tttggaag 578

<210> 35

<211> 585

<212> DNA

<213> Homo sapiens

<400> 35
gcgttcccaa ccagtgagct ggagaaggtg ctcccccaga ccatcctgta taagtactat 60

gagcgaaaag ccgaggagga ggttgcggca gcctacgccg acgagcttgt caggtgcccg 120

tcctgtagct ttccggctct gttggacagt gatgtgaaga ggttcagctg tcctaatcct 180

cactgccgaa aggaaacctg taggaagtgt cagggactct ggaaagaaca taatggcctc 240

acctgtgaag agctggctga aaaagacgac atcaagtacc gtacctctat tgaagaaaaa 300

atgactgctg cccgcattag aaaatgccac aagtgtggga ctggcctcat caaatctgaa 360

ggctgcaacc gcatgtcttg ccgctgtggt gcccagatgt gctacctctg tcgagtttct 420

attaatggat atgaccattt ctgccaacat ccccgctcac caggagcccc ttgccaggag 480

tgttcaagat gctctctctg gaccgatccc actgaagatg atgagaagct tattgaggaa 540

atccagaagg aggctgaaga ggaacagaaa agaaagaatg gagag 585

<210> 36

<211> 572

<212> DNA

<213> Homo sapiens

<400> 36
gcctccctgc atggtatttc ccaggtggtg aaggaccacg tgaccaagcc taccgccatg 60

gcccagggcc gagtggctca cctcattgag tggaagggct ggagcaagcc gagtgactca 120

cctgctgccc tggaatcagc cttttcctcc tattcagacc tcagcgaggg cgaacaagag 180

gctcgctttg cagcaggagt ggctgagcag tttgccatcg cggaagccaa gctccgagca 240

tggtcttcgg tggatggcga ggactccact gatgactcct atgatgagga ctttgctggg 300

ggaatggaca cagacatggc tgggcagctg cccctggggc cgcacctcca ggacctgttc 360

accggccacc ggttctcccg gcctgtgcgc cagggctccg tggagcctga gagcgactgc 420

tcacagaccg tgtccccaga caccctgtgc tctagtctgt gcagcctgga ggatgggttg 480

ttgggctccc cggcccggct ggcctcccag ctgctgggcg atgagctgct tctcgccaaa 540

ctgcccccca gccgggaaag tgccttccgc ag 572

<210> 37

<211> 578

<212> DNA

<213> Homo sapiens

<400> 37
gcggccctca aaccctctcc ctctctgcct gaatttgcca aaatgccaac catccgggca 60

ggtgtggatg ccacagtcca tccccattgc atttcacccc cacatggaca gaggcacaca 120

ctcgcacaca ttcccagcac ctttgactgc tgggggatgc aggccagggt ctgcctggag 180

cctcagggtt gggacttatc tccaaaggag cccacacccc ctcttccagg atgggcactg 240

gctggcagca cttttttccc tcatcacatc cctgacccct gcccagccca catgtacatg 300

ctgtttcctg tctccagggc cctagcctgt ggatctcttc ctcccatgca ctcctaggtg 360

aatgggctca gcaccaaacc tggctgtgcc cattccccac aaccccagct tggcctcctg 420

tgggaaggag aaggcagacc tgcccagagg agaggggcag tgttcccacc cacctgctcc 480

agcacaggaa ccagggagga aagaagggag agggaggttc cattcaccgt cttctgggtt 540

agcgcttgat tggatgtttc aagcaaggga caaggtga 578

<210> 38

<211> 580

<212> DNA

<213> Homo sapiens

<400> 38
gatgatgttg atcttccccc ttgggcaaaa aaacctgaag actttgtgcg gatcaacagg 60

atggccctag aaagtgaatt tgtttcttgc caacttcatc agtggatcga ccttatattt 120

ggctataagc agcgaggacc agaagcagtt cgtgctctga atgtttttca ctacttgact 180

tatgaaggct ctgtgaacct ggatagtatc actgatcctg tgctcaggga ggccatggag 240

gcacagatac agaactttgg acagacgcca tctcagttgc ttattgagcc acatccgcct 300

cggagctctg ccatgcacct gtgtttcctt ccacagagtc cgctcatgtt taaagatcag 360

atgcaacagg atgtgataat ggtgctgaag tttccttcaa attctccagt aacccatgtg 420

gcagccaaca ctctgcccca cttgaccatc cccgcagtgg tgacagtgac ttgcagccga 480

ctctttgcag tgaatagatg gcacaacaca gtaggcctca gaggagctcc aggatactcc 540

ttggatcaag cccaccatct tcccattgaa atggatccat 580

<210> 39

<211> 577

<212> DNA

<213> Homo sapiens

<400> 39
ggctgcccct cctggagcag aaagtgcctt tatctcagcc atccgcagac tgcttggcca 60

gatgcgggga caggctggaa tgagggaggc gtcttcatct ccctgccatc cccctctcac 120

gccacccccg cccccaccgg gctgcaggtg ctgctgatgc gctgggatct gattgaggat 180

aaaaaggaag gagagatgac ccctaccccc tcatccccca gttttgaaaa ggtctaagca 240

agtgagtctg gtggaggagc tgagggaggg agccatggaa ggtgccagaa ggaaggttgg 300

cgggggcacg tgtgggccgt ggcttgggct ggtcagagtg gcgtgagctg cccggcgcct 360

gccctgccca agtgaccagg gaagtgtgtg tgtgtccatg tgtatgcgtg tccgtctgtc 420

tgtctagtgt ctgggtttgg cccaagactg ggctgtagtt acattaatgc ccagccagcc 480

acccctgcca ctcacccctc ctggcccagg ccttgctgac tctctgagct ggggaggtgg 540

gaggccaggc gagcctgact ctgttgatct acccgtg 577

<210> 40

<211> 467

<212> DNA

<213> Homo sapiens

<400> 40
gcccacgcgt ccggaaggag agacaaatca agaaagtgtg cattcacctt ccatgcagat 60

atatgcacac tttctgagaa ggagagacaa atcaagaaac aaactgcatt tgttgagctc 120

gtgaaacaca agcccaaggc aacaaaagag caactgaaag ctgttatgga tgatttcgca 180

gcttttgtag agaagtgctg caaggctgac gataaggaga cctgctttgc cgaggagggt 240

aaaaaacttg ttgctgcaag tcaagctgcc ttaggcttat aacatcacat ttaaaagcat 300

ctcagcctac catgagaata agagaaagaa aatgaagatc aaaagcttat tcatctgttt 360

ttctttttcg ttggtgtaaa gccaacaccc tgtctaaaaa acataaattt ctttaatcat 420

tttgcctctt ttctctgtgc ttcaattaat aaaaaatgga aagaatc 467

<210> 41

<211> 550

<212> DNA

<213> Homo sapiens

<400> 41
   gcccacgcgt ccgcagggcc caaggctggg ctggctccag aaaccacaga gaccccggct 60
   ggggcctcag aaacagccca ggccacagac ctcagcttaa gcccaggagg ggaatcaaag 120
   gccaactgca gccccgaaga cccatgccaa gaaacagtgt ccaaaccaga agtgagcaaa 180
   gaggccactg cagaccaggg gtccaggctg gagtctgcag ccccacctga accagcccca 240
   gagcctgctc cccaaccaga cccccggcca gattcccagc ctacccccaa gccagccctt 300
   caaccagagc tccctaccca ggaggacccc acccctgaga ttctgtctga gagtgtaggg 360
   gaaaagcaag agaatggggc agtggtgccc ctgcaggctg gtgatgggga agagggccca 420
   gcccctgagc ctcactcacc accctcaaaa aatccccccc agccaatggg gccccccccc 480
   gagtgctgca gcagctggtt gaggaggatc gaatgagaag ggcacacagt gggcatccag 540
   gatctccccg 550

<210> 42

<211> 554

<212> DNA

<213> Homo sapiens

<400> 42
ggccatcctg tggctggaga attacctgca gacgtggccc tccaccatcc tagtcgtctc 60

ccacgaccgc aacttcttga atgccatcgc cacagacatc atccacctgc acagccagcg 120

gctagatggt taccggggag actttgagac cttcatcaag agtaagcagg agcggctgct 180

caaccagcag cgtgaatatg aggcgcagca gcagtatcgc cagcacatcc aggttttcat 240

tgaccggttt cgctacaatg ccaacagggc ctctcaagtg cagagtaaac tcaagatgct 300

ggagaagctg cctgagctga agcctgtgga caaggaatca gaggtcgtaa tgaagttccc 360

tgatgggttt gagaagttct cgccgccaat tctgcagcta gatgaggtgg atttctacta 420

cgatccgaag cacgtcatct tcagtcgcct ctctgtgtct gctgatctcg agtctcgcat 480

ctgtgtggtt ggagagaatg gggctgggaa gtctaccatg ctgaagctgc ttttggggga 540

cctggcacct gttc 554

<210> 43

<211> 479

<212>  DNA

<213>  Homo sapiens


<400>  43
ggggcggcgt tggtccggtg cgtcctgttc tacagctatg gccgggccag ctgcaggttt      60

ccgccgcttg ggcgccttgt ccggagctgc ggccttaggc ttcgcttcct acggggcgca     120

cggcgcccaa ttcccagatg cctacgggaa ggagctgttt gacaaggcca acaaacacca     180

cttcttacac agcctggccc tgttaggggt gccccattgc agaaagccac tctgggctgg     240

gttattgcta gcttccggaa cgaccttatt ctgcaccagc ttttactacc aggctctgag     300

tggagacccc agcatccaga ctttggcccc tgcgggaggg accctgctac tcttgggctg     360

gcttgccttg gctctttgag ctcccttttg cttaattact gggttttctg ggcagttttt     420

tttttaaaga gttggagtaa gaagaggatt aaaaaggaaa ggcaaataaa taaaaaaaa      479

<210>  44

<211>  539

<212>  DNA

<213>  Homo sapiens


<220>

<221>  misc_feature

<222>  (109)..(109)

<223>  n = a, c, g or t


<220>

<221>  misc_feature

<222>  (454)..(454)

<223>  n = a, c, g or t


<400>  44
gcagtcattg cccttgaatt actaaattct ggatatgaat ttgatgaagg ctccatcatc      60

tttaataaat tcaggtctgt catctcctat aagacagaag aaaagcccnt cttttccctt     120

aataccgttg caagtgctga cagcatgagt atctatgacg atattgatgc tgacgtgctg     180

caaaattacc aagaatacaa tctggccaac atcatctact actctctgaa ggagtccacc     240

actagtgagc agagtgccag gatgacagcc atggacaatg ccagcaagaa tgcttctgag     300

atgattgaca aattgacatt gacattcaac cgtacccgcc aagctgtcat cacaaaagag     360

ttgattgaaa ttatctctgg tgctgcagct ctggattaat gaaaatcaag ttccatcctc     420

agacaagagg taaagaagga aaattcagcc agtngatttt gtttttagct tactgctgcc 480

tttgtccgaa gaaacttgtc ctccattatt tgaattactg aagacagcaa gatatttgt 539

<210> 45

<211> 553

<212> DNA

<213> Homo sapiens

<220>

<221> misc_feature

<222> (431)..(431)

<223> n = a, c, g or t

<400> 45
   gcccgcattg gagtcatgtc tctcgtcaaa aagaaggtgc aggagtttga gcacatcaat 60
   gggcgttggt caatgccgga actgatgcct gaccccagcg ccgattctaa gcgctcctcc 120
   agagcctcct ctcctaccaa aacgtctccc accactcctg aggcttctgc taccaacagt 180
   ccctgcacct ctaaacctgc tactccagct ccaagtgaga aaggagaagg cataaggaca 240
   ccttttgaga aggaggaagc tgaaaaccag gaggaaaagc cagagaagaa cagcagaatt 300
   ggggagaaga tggagacaga ggctgatgcc cccagcccag ccccatcact tggggagcgg 360
   ctggagccaa ggaagattcc tctagaggat gaggtgccag gggtgcctgg agagatggag 420
   cctgaacctg ngtaccgtgg ggacagagag aagtcagcca cagagtcgac gccaggagaa 480
   aggggggagg agaagccgtt ggatggacag gaacacaggg agaggccgga gggggaaaca 540
   ggggatttgg gca 553

<210> 46

<211> 582

<212> DNA

<213> Homo sapiens

<400> 46
gcccacgcgt ccgcccggtc ccatggggag atcacagaag agagggacat cctgagccgg 60

caacagggag accatgtggc acgcatcctg gagctagagg atgacatcca gaccatcagt 120

gagaaagtgc tgacgaagga agtggagctg gacaggctta gagacacagt gaaggccctg 180

actcgggaac aagagaagct ccttgggcaa ctgaaagaag tacaagcaga caaggagcaa 240

agtgaggctg agctccaagt ggcacaacag gagaaccatc acttaaattt ggacctgaag 300

gaggcgaaga gctggcaaga ggagcagagt gctcaggctc agcgactgaa agacaaggtg 360

gcccagatga aggacaccct aggccaggcc cagcagcggg tggccgagct ggagcccttg 420

aaggagcagc ttcgaggggc ccaggagctt gcagcctcaa gccagcagaa agccaccctt 480

cttggggagg agttggccag tgcagcagca gccagggacc gcaccatagc cgaactacac 540

cgcagccgcc tggaagtggc tgaagttaac ggcaggctgg ct 582

<210> 47

<211> 580

<212> DNA

<213> Homo sapiens

<400> 47
ggcagtagag gtggcggcaa ggggactggt ggtcagcccc cgaggctgga ggagcctggc 60

ctggggggcc tgagcaccct gagtcggaga gcccgcttca ggttgcactt cccccagctg 120

gccctgaggc ggaggctggg gcagctgagc tgcatgtcca gacccgctct gaaactgcgc 180

tcctggccct tgaccgtcct ctactacctc ctgcccttcg gcgccctcag accgctcagc 240

cgggtgggat ggaggcccgt aagcagggtg gctttgtaca agtcagtgcc aacgcgcttg 300

ctgtcacggg cctggggtcg cctcaatcag gtggagctgc cacactggct gcgcaggccc 360

gtctacagcc tgtacatctg gacgtttggg gtgaacatga aagaggccgc tgtggaggac 420

ctgcatcact accgcaacct cagcgagttc ttccggcgca agctgaagcc gcaggcccgg 480

cctgtctgtg gcctgcacag cgtgattagc ccatcggatg gaaggatcct caactttggg 540

caggtgaaga actgtgaggt ggagcaggta aagggggtca 580

<210> 48

<211> 591

<212> DNA

<213> Homo sapiens

<400> 48
gccccgctac cgggttgcgg ccggaagccg ggcgccgcgg ctctgcttcc ctcggggatc 60

tggcgacatg gccagaaagg ctctcaagct tgcttcgtgg accagcatgg ctcttgctgc 120

ctctggcatc tacttctaca gtaacaagta cttggaccct aatgactttg gcgctgtcag 180

ggtgggcaga gcagttgcta cgacggctgt catcagttac gactacctca cttccctgaa 240

gagtgtccct tatggctcag aggagtactt gcagctgaga tctaaggtgc accttcgctc 300

tgccaggcgt ctctgtgagc tctgctgtgc caaccggggc accttcatca aggtgggcca 360

gcacctgggg gctctggact acctgttgcc agaggagtac accagcacgc tgaaggtact 420

gcacagccag gctccacaga gcagcatgca agagatccgc caggtcatcc gagaagatct 480

gggcaaggag atccatgatt tgttccagag cttcgatgac acccctctgg ggacggcctc 540
cctggcccag gtccacaagg cagtgctgca tgatgggcgg acggtggccg t 591

<210> 49
<211> 282
<212> DNA
<213> Homo sapiens

<400> 49
gcccacgcgt ccgcccacgc gtccgccctt gatgtcctgc aaatgaagga ggaggatgtc 60
cttaagttcc ttgcagcagg aacccactta ggtggcacca atcttgactt ccagatggaa 120
cagtgcatct ataaaaggaa aagtgatggc atctatatca taaatctcaa gaggacctgg 180
gagaagcttc tgctggcagc tcgtgcaata gttgccatag aaaaccctgc tgatgtcagt 240
gttatatcct ccaggaatac tggccagagg gctgtgctga ag 282

<210> 50
<211> 508
<212> DNA
<213> Homo sapiens

<400> 50
ggaactcctt ttggtgagcc aggagagtta acattggccg ggaaagatgg ggccaaaaag 60
tggagttcca aacagggtaa ggctgggagg gtggagaggg aagacttccc gtcacctcct 120
ttttcctcat tctcgcagct ggctgagagt gactttgcct ctaccttccg gctgctcaca 180
gtgtttgctt atgggacata cgctgactac ttagctgaag cccggaatct tcctccacta 240
acagaggctc agaagaataa gcttcgacac ctctcagttg tcaccctggc tgctaaagta 300
aagtgtatcc catatgcagt gttgctggag gctcttgccc tgcgtaatgt gcggcagctg 360
gaagaccttg tgattgaggc tgtgtatgct gacgtgcttc gtggctccct ggaccagcgc 420
aaccagcggc tcgaggttga ctacagcatc gggcgggaca tccagcgcca ggacctcagt 480
gccattgccc gaaccctgca ggaatggt 508

<210> 51
<211> 589
<212> DNA
<213> Homo sapiens

<400> 51

gcccacgcgt ccgagcagag tattggtgct gctggaagcc agaaggaagg tccagaggga 60
gccaacctgt tcatctacca cctgccccag gagtttggtg atcaggacct gctgcagatg 120
tttatgccct ttgggaatgt cgtgtctgcc aaggttttca tagacaagca gacaaacctg 180
agcaagtgtt ttggttttgt aagttacgac aatcctgttt cggcccaagc tgccatccag 240
tccatgaacg gctttcagat tggcatgaag cggcttaaag tgcagctcaa acgttcgaag 300
aatgacagca agccctactg agcgtgctcc cctctgagac tggagtgaga gggtcttctg 360
gtaagtgggg gaggagcacc cttaatgatt cgaagccctg aggctgtgtg ttgacagccc 420
tggaccctga tccctgccac tctcgcaggc acagcttgcc ctgaagactc ggctactgcc 480
ttctgtggga gtttcgcttc gtacagagga caagtttgtg ctttggtttc cagtgtttta 540
ctttggagtt taggtgccat atcctgaggt tttttttgtt tttgttttt 589

<210> 52

<211> 575

<212> DNA

<213> Homo sapiens

<400> 52
gagccaattg acaagcggat ctacaagggc acccagccca cctgccacga tttcaaccag 60
ttcactgctg ccaccgagac catctcgctg ctggtgggct tctcagcggg tcaagtgcag 120
tacctggatc tcatcaaaaa ggacaccagc aagctgttca atgaggaggt gattgcagcc 180
cccagagccc ccaacttctc tgccttctca cctacccact atatgccatt aggatgcagc 240
tgcctgcgga acagtgtagg cctgagaggg gcaggaccag ggagagatgg agaatggggt 300
gacagcagct aatgacagct gcctttactg tacccaggcc ctgtgctgtg ttacccttgt 360
gctgaatact ttactccttg ggttttcttt ctttctttct ttcttttatt tatttatttt 420
gagatggagt ctcaccctgt cacccaggct ggagtgcagt ggcgcgatct ctgctcactg 480
caagatccac ctcccgggtt catgccattc tcctgcctca gcctcctgag tagctgggac 540
ttcaggcacc tgctgccacg cccgcctaat ttatt 575

<210> 53

<211> 585

<212> DNA

<213> Homo sapiens

<400> 53
gcaaaaaact tcggagcaga tacgacaaag gtcttttata ttggcctgag aggagagtgg 60
actgagcttc gccgacacga ggtgaccatc tgcaattacg aagcatctgc caacccagca 120
gaccataggg tccatcaggt taccccacag acacacttta tttcctaagg gctggccaag 180

gctcccatag aggcgctgtg tcagtgaaga tgtacgacta cctgttggga aggacaaagg 240

gatgaggctc cagagagagt tggctgccac agcctctgcc aagctttgtc tttggggctt 300

gctgcagaaa cctggcctac ggaagatacg acaccactgg gagggttgtg taggtgccag 360

gggaccatcg tggttctcta gggcgctgtg gaaattgggt cttgggctgg gtggcatctg 420

gcagtcatgg ataacacttg cttttccagt taatgtggcc atgtgattcc aagtgtcatg 480

ttgctttgtg gcaagattgt tgtgtgactt gtttttttgt ttttgttttt gtttttttaa 540

aggaaactat ttgtgggcta taggaaactt tctgatgcct ccgga 585

<210> 54

<211> 534

<212> DNA

<213> Homo sapiens

<400> 54
gatcgagatc gcctatggca ccagctcagg gggcgtgcgg gtcatcgtgc agcacccgga 60

gactgtgggc tcggggcctc agctcttcca gaccttcact gtgcaccgca gccctgtcac 120

caagatcatg ctgtcggaga agcacctcat ctcagtctgt gccgacaaca accacgtgcg 180

gacatggtct gtgactcgct tccgcggcat gatttccacc cagcccggct ccaccccact 240

cgcttccttt aagatcctgg ctctggagtc ggcagatggg catggcggct gcagtgctgg 300

caatgacatt ggcccctacg gtgagcggga cgaccagcaa gtgttcatcc agaaggtggt 360

gcccagtgcc agccagctct tcgtgcgtct ctcatatact gggcagcggg tgtgctccgt 420

gcgctccgtg gacggctcac ccacgacggc cttcacagtg ctggagtgcg agggctcccg 480

gcggctcggc tctcggcccc ggcgctacct gctcactggc caggccaacg gcag 534

<210> 55

<211> 578

<212> DNA

<213> Homo sapiens

<400> 55
gaagactctg acagtgacaa agagtggatt gctgctctgc gtcggaaata tcgaagccga 60

gagcaaaccc tgtcctccag tggtgaaagc tgggagactc tgccggggaa agaagagcgg 120

gaacctccac aggctaaggt gagtgccagt gctggcagca atggcagcaa ttaccttgaa 180

gaagttcgag aaccatctct tcaggaagag caggcatccc tggaagaagg agaaattcct 240

tggctccagt accatgagaa tgacagtagc agtgaggggg ataatgattc tggtcacgag 300

ttgatgcaac ctggggtatt catgctggat ggaaacaaca accttgaaga tgactccagt 360

gtgagcgaag acctagaagt ggattggagc ctctttgatg gatttgcaga tgggttagga 420

gtggctgaag ccatttccta tgtggaccct cagttcctca cctacatggc acttgaagaa 480

cgcctggccc aggcaatgga aactgccctt gcgcacttgg agtctctcgc agtggatgta 540

gaggtggcca atccaccagc aagcaaggag agcattga 578

<210> 56

<211> 562

<212> DNA

<213> Homo sapiens

<400> 56
gcgctgcgct ccgagcgtct gatccgtacc tcgctggacc tggagttaga cctgcaggcg 60

acaagaacct ggcacagcca actgacccag gagatctcgg tgctgaagga gctcaaggag 120

cagctggaac aagccaagag ccacggggag aaggagctgc cacagtggtt gcgtgaggac 180

gagcgtttcc gcctgctgct gaggatgctg gagaagcggc agatggaccg agcggagcac 240

aagggtgagc ttcagacaga caagatgatg agggcagctg ccaaggatgt gcacaggctc 300

cgaggccaga gctgtaagga acccccagaa gttcagtctt tcagggagaa gatggcattt 360

ttcacccggc ctcggatgaa tatcccagct ctctctgcag atgacgtcta atcgccagaa 420

aagtatttcc tttgttccac tgaccaggct gtgaacattg actgtggcta aagttattta 480

tgtggtgtta tatgaaggta ctgagtcaca agtcctctag tgctcttgtt ggtttgaaga 540

tgaaccgact ttttagtttg gg 562

<210> 57

<211> 556

<212> DNA

<213> Homo sapiens

<400> 57
gacaagtctg aggaaaatga ggagccaatg gaaacagatc agaatgcaaa ggaggaagag 60

aagatgcaag tggaccagga ggaaccacat gttgaagagc aacagcagca gacaccagca 120

gaaaataagg cagagtctga agaaatggag acctctcaag ctggatccaa ggataaaaag 180

atggaccaac caccccaagc caagaaggca aaagtgaaga ccagtactgt ggacctgcca 240

atcgagaatc agctattatg gcagatagac agagagatgc tcaacttgta cattgaaaat 300

gagggtaaga tgatcatgca ggataaactg gagaaggagc ggaatgatgc taagaacgca 360

gtggaggaat atgtgtatga aatgagagac aagcttagtg gtgaatatga gaagtttgtg 420

agtgaagatg atcgtaacag ttttactttg aaactggaag atactgaaaa ttggttgtat 480

gaggatggag aagaccagcc aaagcaagtt tatgttgata agttggctga attaaaaaat 540

ctaggtcaac ctatta 556

<210> 58

<211> 577

<212> DNA

<213> Homo sapiens

<400> 58
gggctcagct attcatcaaa cacgagcgcc ggctgcatat gtatgtggtg tactgtcaga 60

ataagcccaa gtcagagcat gtggtgtcag agtttgggga cagctacttt gaggagctcc 120

ggcagcagct ggggcaccgc ctgcagctga acgacctcct catcaaacct gtgcagcgga 180

tcatgaaata ccagctgctg ctcaaggatt ttctcaagta ttacaataga gctgggatgg 240

atactgcaga cctagagctg tggaggtcat gtgctttgtg cccaagcgct gcaacgatat 300

gatgacgctg gggagattgc ggggatttga gggcaaactg actgctcagg ggaagctctt 360

gggccaggac actttctggg tcaccgagcc tgaggctgga gggctgctat cttcccgagg 420

tcgagagagg cgcgtcttcc tctttgagca aatcatcatc ttcagtgaag ccctgggagg 480

aggagtgaga ggtggaacac agcctggata tgtatacaag aacagcatta aggtgagctg 540

cctgggactg gaggggaacc tccaaggtga cccttgc 577

<210> 59

<211> 473

<212> DNA

<213> Homo sapiens

<400> 59
gagggctact actcggagcg agacacaagc aacgtggtac ggcaagtcct ggaggccgtg 60

gcctatttgc actcactcaa gatcgtgcac aggaatctca agctggagaa cctggtttac 120

tacaaccggc tgaagaactc gaagattgtc atcagtgact tccatctggc taagctagaa 180

aatggcctca tcaaggagcc ctgtgggacc cccgagtatc tggccccaga ggtggtaggc 240

cggcagcggt atggacgccc tgtggactgc tgggccattg gagtcatcat gtacatcctg 300

ctttcaggca acccaccttt ctatgaggag gtggaagaag atgattatga gaaccatgat 360

aagaatctct tccgcaagat cctggctggt gactatgagt ttgactctcc atattgggat 420

gatatttcgc aggcagccaa agacctggtc acaaggctga tggaggtgga gca 473

<210> 60

<211> 579

<212> DNA

<213>  Homo sapiens

<400>  60
gggcgtctga gtcagcctga atcagctgaa aagcatgtga cccagaggtt acagcctgag      60
cgggggagcc caaagaagtg tgaagctgaa gaggcagagc caccagctgc cacacagccc     120
caaacctcag agactcagac ctctcatctg ccagaatcag aaagaattca tcacactgtt     180
gaggagaagg aggaagtgac catggacaca agtgaaaaca gacctgaaaa tgatgttcca     240
gaacctccca tgcctattgc agaccaagtc agcaatgatg accgcccgga gggcagtgtt     300
gaagatgagg agaagaaaga gggggccaag aagaacactg ttctggagag acttgtattg     360
agcaagcaaa aaacatttgg aaaggggtgg acttgcatca tctccctact tgggacaccc     420
caagaagagc ttgtgccagt gctggggcgc ctgcccacat gcccttgccc ttttgaggac     480
tgtgggttgc cttgtttgct acagactgtt gggatcccca tctagggcac caagacctcc     540
taaagtttgt gatcgcagtg ccatgctgta aaacactga                            579

<210>  61
<211>  576
<212>  DNA
<213>  Homo sapiens

<220>
<221>  misc_feature
<222>  (533)..(533)
<223>  n = a, c, g or t

<400>  61
gggaaagcag gccgccgtcc gaggcttgcg tctcggcccc tacgggacga ccatctggac      60
ccaccgcgtc cgcttcctgg aagagcaggc cctgccccgc tgggcggcct tcaccatctg     120
gaacgctggc aagcaggggc gccggctgta ccgcagcttg actgccggca gccagcgcaa     180
ggtggtggca ttctgtgacg tggacgagaa caagatcagg aaaggcttct attgccacga     240
ggactctcag gaaagaccca agccccgaat ccccatcctg cacttccgag ccgcccggcc     300
acccttcgtc atctgcgtga agctgaaact tgcaaagcac gaggccctgc ggtgccatgt     360
ctgaggggcc aaggccagga cctccagctc ccgtggactg aggtggctgc atcggggatg     420
tcctcagccc ctgagtgtga acgacccttg cctggggtgg gcaggtgatg ggtgcctcgg     480
gctccaaatg cagccttttt ccccaagcgt ggggagtcca ggctggctgc ggngggctga     540
gcatccgttc tgcagaccgt gggtccagga gtcatt                               576

<210>  62

<211>  471

<212>  DNA

<213>  Homo sapiens

<400>  62
gcccacgcgt ccggcagcac caaatccaag atggcggcca gcaggaggct gatgaaggag      60

cttgaagaaa tccgcaaatg tgggatgaaa aacttccgta acatccaggt tgatgaagct     120

aatttattga cttggcaagg gcttattgtt cctgacaacc ctccatatga taagggagcc     180

ttcagaatcg aaatcaactt tccagcagag tacccattca aaccaccgaa gatcactttt     240

aaaataaaga tctatcaccc aaacatcgac gaaaaggggc aggtctgtct gccagtaatt     300

agtgccgaaa actggaagcc agcaaccaaa accgaccaag taatccagtc cctcatagca     360

ctggtgaatg acccccagcc tgagcacccg cttcgggctg acctagctga agaatactct     420

aaggaccgta aaaaattctg taagaatgct gaagagttta caaagaaata t              471

<210>  63

<211>  569

<212>  DNA

<213>  Homo sapiens

<400>  63
gcccacgcgt ccgcccacgc gtccgacccc agcaagccgg gtgggaatgt tgagggcgcc      60

acgcagtctc tggcggagca gatgaggaag atcgccttgg agtccgaggg gcgccctgag     120

gaacagatgg agtcggataa ctgttcagga ggagatgatg actggaccca tctgtcttca     180

aaagaagtgg acccgtctac aggtgaactc cagtccctac agatgccaga atccgaaggg     240

ccaagctctc tggacccctc ccaggaggga cccacagggc tgaaggaagc tgccttgtac     300

ccacatctcc cgccagaggc tgacccgcgg ctgattgagt ccctctccca gatgctgtcc     360

atgggcttct ctgatgaagg cggctggctc accaggctcc tgcagaccaa gaactatgac     420

atcggagcgg ctctggacac catccagtat tcaaagcatc ccccgccgtt gtgaccactt     480

ttgcccacct cttctgcgtg cccctcttct gtctcatagt tgtgttaagc ttgcgtagaa     540

ttgcaggtct ctgtacgggc cagtttctc                                       569

<210>  64

<211>  487

<212>  DNA

<213>  Homo sapiens

<400>  64
gggggccacc aagggctgcc ttcgggccct ggccatgaag ttcaagacca cacatgcacc      60

gccaggggac acactggtgc atgctgggga cctgctcacc gccctgtact tcatctcccg     120

gggctccatc gagatcctgc ggggcgacgt cgtcgtggcc atcctgggga agaatgacat     180

ctttggggag cctctgaacc tgtatgcaag gcctggcaag tcgaacgggg atgtgcgggc     240

cctcacctac tgtgacctac acaagatcca tcgggacgac ctgctggagg tgctggacat     300

gtaccctgag ttctccgacc acttctggtc cagcctggag atcaccttca acctgcgaga     360

taccaacatg atcccgggct cccccggcag tacggagtta gagggtggct tcagtcggca     420

acgcaagcgc aagttgtcct tccgcaggcg cacggacacg gacacggagc agccagggga     480

ggtgtcg                                                               487

<210>  65

<211>  576

<212>  DNA

<213>  Homo sapiens


<400>  65
gatgtccatc tcttgggata cagccttgga gcccatgctg ctggcattgc aggaagtctg      60

accaataaga aagtcaacag aattactggc ctcgatccag ctggacctaa ctttgagtat     120

gcagaagccc cgagtcgtct ttctcctgat gatgcagatt ttgtagacgt cttacacaca     180

ttcaccagag ggtcccctgg tcgaagcatt ggaatccaga aaccagttgg gcatgttgac     240

atttacccga atggaggtac ttttcagcca ggatgtaaca ttggagaagc tatccgcgtg     300

attgcagaga gaggacttgg agatgtggac cagctagtga agtgctccca cgagcgctcc     360

attcatctct tcatcgactc tctgttgaat gaagaaaatc caagtaaggc ctacaggtgc     420

agttccaagg aagcctttga gaaagggctc tgcttgagtt gtagaaagaa ccgctgcaac     480

aatctgggct atgagatcaa taaagtcaga gccaaaagaa gcagcaaaat gtacctgaag     540

actcgttctc agatgcccta caaagtcttc cattac                               576

<210>  66

<211>  574

<212>  DNA

<213>  Homo sapiens


<400>  66
gcccacgcgt ccggcggtgc cttccggaga agcctgcatg atcccgatgg gctggtggcc      60

acctacatca gcgaggtgca cgaacacgat gggcacctgt acctgggctc tttcaggtcc     120

cccttcctct gcagactcag cctccaggct gtttagccct cccagatagc tgcccctgcc     180

acgcaggcca ggagtcttca cactcaggca ccaggcctgg tccaggagga gctgtggaca 240
cagtcgtggt tcaagtgtcc acatgcacct gttagtccct gagaggtggt gggaatggct 300
gcttcattcc tcgaggatgc ccgggcccca cctgggcttg tctttctgtt tagagggaag 360
tgtaacatat ctgccatgag gaacataaat tcatgtaaag ccattttctc ttaaacaaaa 420
caaaactttc taagtacagt cattctctag gatttgggaa gctccttgca cttggaacag 480
ggctcaggtg ggtggagcag taaggcacta cccagagagc ttgctgctgc ggccctgtcc 540
tgcggcctca aagttcttct ttactatata taac 574

<210> 67
<211> 288
<212> DNA
<213> Homo sapiens

<400> 67
gctctggaga agccgcagcg cgagttgccg ccgctgctgc ccggggccgg cttgccttgc 60
gccatggact ggcagccaga cgagcagggc ctgcagcagg tcctgcagct gctcaaagac 120
tcacagtcgc ccaacacagc cactcagcgc atcgtgcagg ataaactcaa acaactcaat 180
cagtttcctg acttcaacaa ctacctgatt ttcgtcctga cccctctgcc cacgaaaaaa 240
aaaaaaaaaa aaaaaaaaaa aaaaaaaaaa aaaaaaaaaa aaaaaagg 288

<210> 68
<211> 418
<212> DNA
<213> Homo sapiens

<400> 68
ggggacgccg cgcggggcag tgtgggactg gggcggaacg ccgcccggcc gcgggtcggg 60
ctctgtgtca gcagccgggc gggcgctcgg gcgggacatg gcagcctgta cagcccggcg 120
ggccctggcc gtgggcagcc gctggtggtc ccggtcgctg actggggccc ggtggccaag 180
gccgctctgt gcggcggccg gagctggagc cttctcgcca gcgtcgacca cgacgacgcg 240
gaggcacctc tcgtcccgaa accgaccaga gggcaaagtg ttggagacag ttggtgtgtt 300
tgaggtgcca aaacagaatg gaaaatatga gaccgggcag cttttccttc atagcatttt 360
tggctaccga ggtgtcgtcc tgtttccctg gcaggccaga ctgtatgacc gggatgtg 418

<210> 69
<211> 451
<212> DNA

<213>  Homo sapiens

<400>  69
gcggaggcgc ctctctttcc ccgcccctcg cctcggccct ttctcttccc agcacctcgg      60

ctgttccccg gcggcggcag cggcagcggc ggcccacaca gcagcgagag gcgagaggag     120

gctgcctcga ggatgaagtg caaacccaac cagacgcgga cctacgaccc cgaggggttc     180

aagaagcggg cggcgtgcct gtgcttccgg agcgaacgcg aggacgaggt cctgttagtg     240

agtagcagcc ggtacccgga ccgctggatc gtgccgggcg ggggcatgga gcccgaggag     300

gagccgggcg gtgcggcggt ccgagaggtg tacgaagaag cgggagtcaa ggggaagtta     360

ggccggctcc tgggcgtctt cgaacagaac caggatcgca agcacagaac gtacgtgtat     420

gtactgactg tcacggagct gctggaggat t                                    451

<210>  70

<211>  555

<212>  DNA

<213>  Homo sapiens

<400>  70
gaggatttga gtgagttttg tagtgataaa cctaagtctg gagcaaagta tggactgccg      60

gactctttgg ccatcctgtc agagatggga gaagtcacag acggaatgat ggatacaaag     120

atggttcact ttcttacaca ctatgctgac aagattgaat ctgttcattt ttcagaccag     180

ttctctggtc caaaaattat gcaagaggaa ggtcagcctt taaagctacc tgacactaag     240

aggacactgt tgtttacatt taatgtgcct ggctcaggta acacttaccc aaaggatatg     300

gaggcactgc tacccctgat gaacatggtg atttattcta ttgataaagc caaaaagttc     360

cgactcaaca gagaaggcaa acaaaaagca gataagaacc gtgcccgagt agaagagaac     420

ttcttgaaac tgacacatgt gcaaagacag gaagcagcac agtctcggcg ggaggagaaa     480

aaaagagcag agaaggagcg aatcatgaat gaggaagatc ctgagaaaca gcgcaggctg     540

gaggaggctg cattg                                                      555

<210>  71

<211>  556

<212>  DNA

<213>  Homo sapiens

<400>  71
gcattgacag ccattcgcca ccatgtcctt ggaactatca ccaccgacaa aatgatggat      60

gtcactgtga ctatcaagtc ttccatcgac agtgaacccg ccttggtctt aggccctctg     120

aagtctgtgc aggagctgcg gagggagcag cagctggctg agatcgaggc ccgcaggcag 180

gagagggaga aaaacggcaa tgaggaaggt gaagaaagaa tgaccaagcc tcccgtgcag 240

gagatggtag atgagttaca aggccccttc tcgtatgatt tctcttactg ggcgcggtct 300

ggagagaaaa tcactgttac accgtcatct aaagagctgc tcttttatcc cccttcaatg 360

gaagccgttg tcagtggaga aagctgccca gggaagctga tcgagatcca cgggaaggca 420

ggcctgtttt tagaaggcca gatccacccc gagttggaag gagtcgagat tgtcatcagt 480

gaaaaggggg taagttcacc gctgatcaca gtctttactg atgacaaagg tgcctacagt 540

gttggccccc tgcaca 556

<210> 72

<211> 535

<212> DNA

<213> Homo sapiens

<400> 72
gctaaaaatg aaccaagaaa aacaaacgtc acacatgaag aacacacagc ggtggaaaag 60

atctcctccc tggttcgaag ggctgccctc acacacaacg acaaccactt caattatgag 120

aagacacaca actttaaggt ccacacgttc cgaggcccac actggtgtga atattgtgcc 180

aatttcatgt gggggctcat cgcccaaggg gtccggtgct cagactgtgg attgaacgta 240

cacaaacagt gttccaagca cgttcccaat gactgccaac ctgatctcaa gaggatcaag 300

aaagtgtact gttgtgacct cacaacactt gtgaaggctc acaacactca gagacccatg 360

gtggtagaca tatgcattcg ggaaattgaa gcaagaggat taaaatcgga aggcctttac 420

agagtctctg ggttcactga acacattgaa gatgtcaaaa tggcatttga cagagatggt 480

gaaaaggccg atatatctgc caatgtctat ccagacataa acatcatcac tggag 535

<210> 73

<211> 488

<212> DNA

<213> Homo sapiens

<400> 73
gcgggtcccg acgcgacagt gggcgggcca gcgccgaccc cgctgctgcc cccctcggcc 60

cagcctcggt caagatggag ccagagaaca agtacctgcc cgaactcatg gccgagaagg 120

actcgctcga cccgtccttc actcacgcca tgcagctgct gacggcagaa attgagaaga 180

ttcagaaagg agactcaaaa aaggatgatg aggagaatta cttggattta ttttctcata 240

agaacatgaa actgaaagag cgagtgctga tacctgtcaa gcagtatccc aagttcaatt 300

ttgtggggaa gattcttgga ccacaaggga atacaatcaa aagactgcag gaagagactg 360

gtgcaaagat ctctgtattg ggaaagggct caatgagaga caaagccaag gaggaagagc 420

tgcgcaaagg tggagacccc aaatatgccc acttgaatat ggatctgcat gtcttcattg 480

aagtcttt 488

<210> 74

<211> 525

<212> DNA

<213> Homo sapiens

<400> 74
gcaacggtga agacggaaac catcagtttt ggcagtgttt caccgggagg agtaaagcta 60

gaaatttcca cgaaggaagt gccagtagtt cacaccgaaa ccaaaaccat cacatatgaa 120

tcatcacagg tcgatccagg cacagatctg gagccaggcg tgctgatgag tgcacagacg 180

atcacatctg aaaccaccag taccaccacc actacgcaca tcaccaaaac tgtgaaaggg 240

ggcatttcag agacaagaat tgagaagcga atagtcatca cgggggatgc agacattgac 300

catgaccagg cgctggctca ggcaattaaa gaggccaaag agcagcaccc tgacatgtca 360

gtgaccaaag tagtggtcca taaagagaca gagatcacac cagaagatgg agaggattga 420

ccagaggaat aacttagctt gcacatgaat gcagtcatgc aaaccgttag gaaaaccaga 480

gcctatatgg agttccctct tctaacccaa ctgacttgta tctgt 525

<210> 75

<211> 557

<212> DNA

<213> Homo sapiens

<400> 75
   gggaagaagg aaggggcgaa cggcgtgagc tggcgccgaa atgggagaaa gcagcgagtg 60
   agaggggaag gggcgccagg cgagcacccg ggagccagcg ggacctgggc aggggcgccc 120
   ggagcaggcg cgcatggcgg gccccgcgcg gggatccggc tggaagagag cgtagcacgg 180
   ctcgcacgag tccggggccg atgtaccagg tgagcggcca gcgcccctct ggctgcgacg 240
   cgccctatgg agcccccagc gcagccccgg gcccagccca gaccctatcc ctccttcctg 300
   ggctggaggt agtaacagga tccactcacc ctgcggaggc agcaccagag gagggctccc 360
   tggaggaggc ggcaaccccc atgccccaag gcaatggccc tggcatcccc cagggcctgg 420
   acagcactga cctcgacgtc cccacagaag ctgtgacatg ccagcctcag gggaacccct 480
   tgggctgcac cccacttctg ccgaatgact ctggccaccc ctcagagctg ggcggcacca 540
   gacgggcggg gaatggt 557

<210> 76

<211> 575

<212> DNA

<213> Homo sapiens

<400> 76
gagggccccc acctgcagca gtggaaatgg caatgcagat tcagagagca gcggagacgc 60

agcgccagat ggcccacgtg caaatttttc aaaggccaat ccaacaccag atgcccccga 120

tgactcccat ggcccccatg ggtatgaacc cacctcccat gaccagaggt cccagtgggc 180

atttggagcc agggatggga ccgacaggga tgcagcaaca gccaccctgg agccaaggag 240

gattgcctca gccccagcaa ctacagtctg ggatgccaag gccagccatg atgtcagtgg 300

cccagcatgg tcaacctttg aacatggctc cacaaccagg attgggccag gtaggtatca 360

gcccactcaa accaggcact gtgtctcaac aagccttaca aaaccttttg cggactctca 420

ggtctcccag ctctcccctg cagcagcaac aggtgcttag tatccttcac gccaaccccc 480

agccgttggc tgcattcatc aagcagcggg ctgccaagta tgccaactct aatccacaac 540

ccatccctgg gcagcctggc atgccccagg ggcag 575

<210> 77

<211> 575

<212> DNA

<213> Homo sapiens

<400> 77
gcccacgcgt ccgatgcaga gctgcagtca aaactgaagg atttatttaa tgtggatgct 60

tttaagctgg aatcattaga agcaaaaaac agagcattga atgaacagat tgcaagattg 120

gaacaagaaa gagaaaaaga accgaatcgt ctagagtcgt tgagaaaact gaaggcttcc 180

ttacaaggag atgttcaaaa gtatcaggca tacatgagca atttggagtc tcattcagcc 240

attcttgacc agaaattaaa tggtctcaat gaggaaattg ctagagtaga actagaatgt 300

gaaacaataa aacaggagaa cactcgacta cagaatatca ttgacaacca gaagtactca 360

gttgcagaca ttgagcgaat aaatcatgaa agaaatgaat tgcagcagac tattaataaa 420

ttaaccaagg acctggaagc tgaacaacag aagttgtgga atgaggagtt aaaatatgcc 480

agaggcaaag aagcggtatg tcataccatt tccagagtgg caactcatca tagctgacct 540

ttctgaaggt ctgtcccaat taagaagaag tcagt 575

<210> 78

<211> 566

<212>  DNA
<213>  Homo sapiens

<400>  78
ggccgccggg ttgagctgcg gcacacgtgc gacggccgtg atgaagttcg cttaccggtt     60
ttaaatttgc tgggtacggt gtaccggcgt gggaacctaa attttacctg caatggaaat    120
tcagttatca gtcccgtggg caatagagtc actgtatttg accttaaaaa caacaaatct    180
gacacgctgc ccctggccac tcggtacaac gtcaagtgcg tggggctgtc cccggatggc    240
cgcctcgcta tcatcgtcga tgaagggggc gatgcgctgc tggtcagcct ggtctgcagg    300
tctgtgctgc accacttcca cttcaagggc tctgtgcaca gtgtgtcctt ctcccctgat    360
ggcaggaagt ttgttgtcac aaagggtaac attgcccaga tgtatcatgc ccctgggaag    420
aagcgggagt tcaacgcctt cgttctggac aagacctatt ttgggcccta cgatgagacc    480
acctgcatcg actggacgga tgactccagc tgtactcact cagccaggac ggagtgctgt    540
gcatgtggca gtgtgacacg cccccc                                         566

<210>  79
<211>  409
<212>  DNA
<213>  Homo sapiens

<400>  79
gcggacgcgt gggggaagcc ggctcaccca gtgaaaaggg agatcaatga ggagatgaag     60
aactttgcag aaaacaccat gaatgaactc cttggctggt atggctatga taaggttgaa    120
ttaaaagatg gtgaggatat tgaattcagg agctacccta cagatgggga gagccggcag    180
cacatttctg ttctcaaaga aaattctttg ccaaaaccaa aattacccga ggacagtgtt    240
atttcaccat acaatataag cacaggctat tcagggcttg ccactggaaa tggactcagt    300
gactcacctg cagggtcaaa ggatcatggc agtgtgccca ttattgtacc tttaattccc    360
accacctttc ataaagccac cagcagaaga tgatgtgtca aatgtacaa                409

<210>  80
<211>  582
<212>  DNA
<213>  Homo sapiens

<220>
<221>  misc_feature

<222>  (491)..(491)

<223>  n = a, c, g or t


<400>  80
gcccacgcgt ccgcagctgt gttcccggat gcagggtctc cagagggccc aacccttcct      60

gcccagaggc tggagagggg tgggggccct ttggggggtc agcttgcagt ttcctttggg     120

ggcctcctgc cctgtggctt ggtccctggg ccagaactgc tcctgtgggt gtccaggctg     180

ggcccccagg ccctccccca ggcctcaagt tggcctcagc tttcctcccc tgcaggaaat     240

cgtgactatc gtggtgtttg gcgtggagta cttcgtgcgg atctgggccg caggctgctg     300

ctgccggtac cgtggctgga gggggcggct caagtttgcc cggaaaccgt tctgtgtgat     360

tgacatcatg gtgctcatcg cctccattgc ggtgctggcc gccggctccc agggcaacgt     420

ctttgccaca tctgcgctcc ggagcctgcg cttcctgcag attctgcgga tgatccgcat     480

ggaccggcgg ngaggcacct ggaagctgct gggctctgtg gtctatgccc acagcaagga     540

gctggtcact gcctggtaca tcggcttcct ttgtctcatc ct                        582

<210>  81

<211>  448

<212>  DNA

<213>  Homo sapiens


<400>  81
gggcttttcg gacccacgga gcgacagagc gagcggcccc tacggccgtc ggcggcccgg      60

cggcccgaga tgttatctgg gaagaaggcg gcagccgcgg cggcggcggc tgcagcggca     120

gcaaccggga cggaggctgg ccctgggaca gcaggcggct ccgagaacgg gtctgaggtg     180

gccgcgcagc ccgcgggcct gtcgggccca gccgaggtcg ggccgggggc ggtgggggag     240

cgcacacccc gcaagaaaga gcctccgcgg gcctcgcccc ccgggggcct ggcggaaccg     300

ccggggtccg cagggcctca ggccggccct actgtcgtgc ctgggtctgc gacccccatg     360

gaaactggaa tagcagagac tccggagggg cgtcggacca gccggcgcaa gcgggcgaag     420

gtagagtaca gagagatgga tgaaagct                                        448

<210>  82

<211>  396

<212>  DNA

<213>  Homo sapiens


<400>  82
gcggagcccg aggcgtacga gcggcgcgtg tgcttcctgc ttctgcaact ctgcaacggg      60

ctggagcacc tgaaggagca cgggatcatc caccgggacc tgtgcctgga gaacctgctg 120
ctggtgcact gcaccctcca ggccggcccc gggcccgccc ccgcccccgc cccggctccc 180
gcccccgccg ccgacgcgcc tccctgctcc tctgccgccc cgcctgctgg tggcactctc 240
agccccgcag ccggccccgc ctccccggaa gggccccggg agaagcagct gccccggctc 300
atcatcagca actttttgaa ggccaagcag aagccgggca gcaccccaaa ccttgagcag 360
aagaagagcc aggcccggct ggcccccgag atcgtg 396

<210> 83
<211> 458
<212> DNA
<213> Homo sapiens

<400> 83
ggctcgcctt acaactccgc gcggcctcgg tcccctgcgc cgcccgcccc acaacaaaac 60
tcagcgcagc gctcccgggc gcccggttca gagcgacctg cggctcagag cggaggggag 120
actgaccgga gcgcggatcg ggacagcggc cgggacagcg gcgagacgcg cgtgtgtgag 180
cgcgccggac caagcgggcc cagaagcggg tctgcagccc agagggcacc ttctgcaaac 240
atgtctgtgg atcccctatc cagcaaagct ctaaagatca agcgagagct gagcgagaac 300
acgccgcacc tgtcggacga ggcgctgatg gggctgtcgg tgcgcgagct gaaccggcat 360
ctgcgcgggc tctccgccga ggaggtgaca cggctcaagc agcggcgccg cacactcaaa 420
aaccgtggct acgccgccag ctgccgcgtg aagcgcgt 458

<210> 84
<211> 570
<212> DNA
<213> Homo sapiens

<400> 84
gggtctacac cgacttcgac ggcacccgag tgtacagccc cccggagtgg atccgctacc 60
accgctacca cgggcgctcg gccaccgtgt ggtcgctggg cgtgcttctc tacgatatgg 120
tgtgtgggga catccccttc gagcaggacg aggagatcct ccgaggccgc ctgctcttcc 180
ggaggagggt ctctccagag tgccagcagc tgatccggtg gtgcctgtcc ctgcggccct 240
cagagcggcc gtcgctggat cagattgcgg cccatccctg gatgctgggg gctgacgggg 300
gcgccccgga gagctgtgac ctgcggctgt gcaccctcga ccctgatgac gtggccagca 360
ccacgtccag cagcgagagc ttgtgaggag ctgcacctga ctgggagcta ggggaccacc 420
tgccttggcc agacctggga cgcccccaga ccctgacttt ttcctgcgtg ggccgtctcc 480
tcctgcggaa gcagtgacct ctgacccctg gtgaccttcg ctttgagtgc cttttgaacg 540

ctggtcccgc gggacttggt tttctcaagc 570

<210> 85
<211> 511
<212> DNA
<213> Homo sapiens

<400> 85
gtgatcacta cctgctatgg agggcggaac cggaaagtgg ctgaggcctt tgcgcgtaca 60
ggaaagtcca ttgagcagct ggagaaagag ttgctgaatg ggcagaaact gcaggggccc 120
gagacagccc gggagctata cagcatcctc cagcacaagg gcctggtaga caagtttccc 180
ttgttcatgg ctgtgtacaa ggtgtgctac gagggccagc cagtgggtga attcatccac 240
tgcctgcaga atcatccaga acatatgtga gtggggccag ggcccaggcc aggccgcttt 300
tttaccccag tggagaccag cagaagcctg gggtacctag tcaccaggat ctccaggact 360
cccagggagc agagtcttct catcttttca ctggaggaca ggaggctatg gggcccagct 420
acgcacctgg agatcctgaa ctgtcaagcc actggcagcc tcatgccacc acatttgcca 480
gaaatgcagt tgccctgtcc ctctccagat g 511

<210> 86
<211> 480
<212> DNA
<213> Homo sapiens

<400> 86
gagggcctgg agaagccccg ccttgactac tccgagttct tcacggagga cgtgggccag 60
ctgcccggac tgaccatctg gcagatagag aacttcgtgc ctgtgctggt ggaggaagcc 120
ttccacggca agttctacga ggctgactgc tacattgtgc tcaagacctt tctggatgac 180
agcggctccc tcaactggga gatctactac tggattggcg gggaggccac actcgacaag 240
aaagcttgct ctgccatcca cgctgtcaac ttgcgcaact acctgggtgc tgagtgccgc 300
actgtccggg aggagatggg cgatgagagc gaggagttcc tgcaggtgtt tgacaacgac 360
atctcctaca ttgagggtgg aacagccagt ggcttctaca ctgtggaaga cacacactat 420
gtcaccagga tgtatcgtgt gtatgggaaa aagaacatca agttggagcc tgtgcccctc 480

<210> 87
<211> 555
<212> DNA
<213> Homo sapiens

<400> 87
gctgctgtcc ggcctgccac ctcccagcag ccgcagaagg aggatcagaa gatactgacc 60

ttgactgagt accggctcca tggaacagga agcttgccgc ctctgggctc ctggcgatct 120

ggcctcagcc gagcagagag cctggcccgg ggaggtggtg agggcagcat ggccaccagg 180

cccagtaatg ccaaccacct atcccctcaa gccctcaagt ggcgggaata caggaggaag 240

aacccactag ggccacctgg tttgtcaggg agcctagacc gaagatcaca agaagctcgg 300

ctggcccgaa gaaaccctat ctttgagttc cctggctccc tcagtgctgc cagccatctg 360

aactgccggc tgaatggcca agcagtgaag ccgttaccac tgacctgccc tgacttccag 420

gacccctttt ccttgacgga gaagcctcca gctgagtttt gtctgtcccc agatggcagc 480

tcagaggcca tttccattga cctgcttcag aaaaaagggc tggtaaaagc tgttaacatc 540

gctgtggacc tcatt 555

<210> 88

<211> 558

<212> DNA

<213> Homo sapiens

<400> 88
gcccacgcgt ccgcgttaag gcgcagccga cggctgggag gcctaaggcc cgaatccccc 60

gagagcctca cctcagtttc gcggacgaga cgggcccttg tggagttcga gtcgaaccca 120

gaagaaacga gggagcccgg gtctcctccg agtgtgcagc gggctggcct ggggtccccc 180

gaaaggccgc cgaagacaag cccaggatca ccccgtctgc agcagggtgc aggcttggag 240

tcaccccaag ggcagccaga gccaggcgca gcgtcccccc agcgtcagca agatctacac 300

ctggagtcgc ctcaaagaca gccagagtac agtcctgaat ccccacgatg tcagccgaag 360

ccaagtgagg aggcaccaaa gtgttctcag gaccagggag tactggcctc ggagttggcc 420

cagaataaga ggagctgacc ccgggggccc cccagcatca gctaccgccg gtcccaggat 480

caccagagcc ttaccccggt cagcaagctc ccggtccgga gccctctcag ccactactgg 540

agctgacacc cagggcac 558

<210> 89

<211> 547

<212> DNA

<213> Homo sapiens

<400> 89
gccgactacg accccagcca gccgaggaag aaaaagcgcg aggccccctt gacgggcaag 60

aagaagcgca agtcgccctt cgccgcggcc gtggggcagg agaagcccgt gttcgaaccc 120

ggggacaaga cgttcgagga gtacctggat gagtattacc gggtggacta cgaggacatc 180

atcgacgacc tgccctgtcg cttcaagtac cgcacagtgg tgccctgtga ctttggcctc 240

agcactgagg agatcctcgc tgctgacgat aaggagctga accggtggtg ctccctaaag 300

aagacctgca tgtacaggtc agagcaggag gagctgcggg acaagcgggc gtacagccag 360

aaggcccaga actcatggaa aaagcggcag gtcttcaagt cactctgccg agaagaggca 420

gagacacctg cggaagccac agggaagcca cagagagatg aagccggccc acagaggcag 480

ctgccagccc ttgatggcag cttgatgggg ccggagagtc ccccagcaca ggaagaggaa 540

gcccctg 547

<210> 90

<211> 526

<212> DNA

<213> Homo sapiens

<400> 90
gccaacagtt atgcagagtg ctacccagcc acgatggatg acatggctgt ggatagtgat 60

gaggaggtgg attatagcaa aatggaccag ggtaacaaga aggggccctt aggccgttgg 120

gactttgata cccaggaaga atacagcgag tatatgaaca acaaagaagc tttgcccaag 180

gctgcattcc agtatggtat caaaatgtct gaagggcgga aaaccaggcg cttcaaggaa 240

accaatgaca aagcagagct tgatcgccag tggaagaaga ttagtgcaat cattgagaag 300

aggaagaaga tggaagctga tggggttgaa gtcaaaagac caaaatacta atcactagtt 360

acaaccagag atgctccaca aggatatgct ccccacggtt ttctttctac aatttccaaa 420

ggttgcaaga tgtttttttg tggatgaata taaaatttta ttgtgtaatt acttggttcc 480

attaaaattg gttaacttgc aaaaaaaaaa aaaaaaaaaa aaaagg 526

<210> 91

<211> 477

<212> DNA

<213> Homo sapiens

<400> 91
gatcttggtt ctcctgaaga tttcctctcc aaagccttag atcctcctca gctccaagtg 60

atcagcaatg caatgaattt gctccgaaaa attggagctt gtgaattaaa tgagcctaaa 120

ctgactccgt tgggccaaca ccttgcagct ttacctgtga atgtcaagat tggcaagatg 180

cttatttttg gtgccatatt tggctgcctt gacccagtgg caacactagc tgcagttatg 240

acagagaagt ctccttttac cacaccaatt ggtcgaaaag atgaagcaga tcttgcaaaa 300
tcagctttgg ccatggcgga ttcagaccac ctgacgatct acaatgcata tctaggatgg 360
aagaaagcac gacaagaagg aggttatcgt tctgaaatca catactgccg gaggaacttt 420
cttaatagaa catcactgtt aaccctagag gatgtaaagc aggagttaat aaagttg 477

<210> 92
<211> 591
<212> DNA
<213> Homo sapiens

<400> 92
   gaagcgcctc gcgctgattc tcacgggccc ggctgccggc ccccgctctg ccctggattg 60
   gtagcttatg tcgatcttga tgaaagagca attgatgctc tcagggaatt taatgaagaa 120
   ggagctctgt ctgtactaca gcagttcaag gaaagtgact tatcacatgt tcagaacaaa 180
   agtgcatttt tatgtggagt tatgaagacc tacaggcaga gagagaaaca ggggagcaag 240
   gtgcaagagt ccacaaaggg acctgatgaa gcgaagatca aggccttgct tgagagaact 300
   ggttatactc tggatgtaac cacaggacag aggaagtatg gtggtcctcc accagacagt 360
   gtgtactctg gcgtgcaacc tggaattgga acggaggtat ttgtaggcaa aataccaagg 420
   gatttatatg aggatgagtt ggtgcccctt tttgagaagg ccggacccat ttgggatcta 480
   cgtcttatga tggatccact gtccggtcag aatagagggt atgcatttat caccttctgt 540
   ggaaaggaag ctgcacagga agccgtgaaa ctgtgtgaca gctatgaaat t 591

<210> 93
<211> 594
<212> DNA
<213> Homo sapiens

<400> 93
gacgatattt tgaaaccacc gggccgggag agcagacctg agagaattgt tgttataatg 60
agaggattac ctggcagtgg aaagacacat gttgcaaaac ttattcgaga taaggaggta 120
gaatttggag gacctgcacc cagagttcta agcctggatg attacttcat cactgaagtg 180
gaaaaagaag aaaaagatcc agattctgga aagaaagtga aaaagaaggt aatggaatat 240
gaatatgaag ctgagatgga ggagacttac cgcaccagca tgttcaaaac tttcaaaaag 300
actctggatg atggcttttt tcccttcatc atcctggatg ccatcaatga cagagttagg 360
cattttgacc agttttggag tgcagcaaaa accaagggat ttgaggtata tttggctgaa 420
atgagtgcag ataaccagac ttgtggcaag agaaatattc atggaagaaa gcttaaagaa 480
ataaataagg tagagatgga agattttgat gcaaatatcg aagaacagaa agaagaaaag 540

aaagatgcag aggaagagga aagcgaactg ggttacattc cgaaaagcaa atgg          594

<210>  94

<211>  479

<212>  DNA

<213>  Homo sapiens


<220>

<221>  misc_feature

<222>  (322)..(324)

<223>  n = a, c, g or t


<220>

<221>  misc_feature

<222>  (341)..(341)

<223>  n = a, c, g or t


<400>  94
gcccacgcgt ccgcttcaga ggaactactg aaacaaccag actattcgga caagatcaag      60

cagatgctgg tgccacatgg actcctaggc cctggcccaa tagccaatgg tttcccacca     120

gggggtcctg ggggccccaa gggcatgcag cactttcccc ctggacctgg gggacctatg     180

ccaggtcccc atggaggccc tggtgggcca gtgggtccac gtcttctggg tcctccaccc     240

cctccccggg gaggtgatcc cttctgggat ggcccgggcg accctatgcg ggtggcccca     300

atgcgggggg gtccaggacc annncctgga ccataccata naggccgagg tggccgagga     360

ggaaacgaac ctcctcctcc tcctcctcca ttccgaggcg ccagaggagg tcgctctgga     420

ggaggacccc caaatggacg agggggccct ggtgggggca tggttggagg tggtgggca      479

<210>  95

<211>  598

<212>  DNA

<213>  Homo sapiens


<220>

<221>  misc_feature

<222>  (497)..(497)

<223>  n = a, c, g or t

<400> 95
gcccacgcgt ccggtgacgc tctgggggca ggactcctcc aaaattacgt ggaccgtacg 60
gagtcgagaa gcacagagcc tgagttgata caagtgaaga gtgagctgcc cctggatccg 120
ctgccagtcc ccactgagga aggaaacccc ctcctcaaac actatcgggg gcccgcaggg 180
gatgccacgg tcgcctctga gaaggaatca gtcatgtaaa ccccgggagg gaccttccct 240
gccctgctgg gggtgctctt tggacactgg attatgagga atggataaat ggatgagcta 300
gggcttctgg gggtctgcct gcacactctg gggagccagg ggccccagca ccctccagga 360
caggagatct gggatgcctg gctgctggag tacatgtgtt cacaagggtt actcctcaaa 420
acccccagtt ctcactcatg tccccaactc aaggctagaa aacagcaaga tggagaaata 480
atgttctgct gcgtccncac cgtgacctgc ctggcctccc ctgtctcagg gagcaggtca 540
caggtcacca tggggaattc tagcccccac tggggggatg ttacaacacc atgctggt 598

<210> 96
<211> 602
<212> DNA
<213> Homo sapiens

<400> 96
gtggatggaa gacaggttgt tgacatgcga acaaatccag gtgatcccag agaagcacag 60
gaacagcaaa atgacgggaa aggacgtgga aacaaggcag caaaacgaga ccttccacca 120
gcaaagactc atctcatcca agaggatatt ctaccttatt gtagacctac ttttccaaca 180
tcaaataatc ccagagatcc cagttcctca agctcaatgt catcaagagg atcaggaagc 240
agacaaagag aacaagcaaa tgtaggtcga agaaatattg cagaaatgca ggtacttgga 300
ggatatgaaa gaggagaaga taataatgaa gaattagagg aaactgaaag ctgaagacaa 360
ccaagaggct tatgagatct aatgtgaaaa tcatcactca agatgcctcc tgtcagatga 420
cacatgacgc cagataaaat gttcagtgca atcagagtgt acaaattgtc gtttttattc 480
ctcttattgg gatatcattt taaaaacttt attgggtttt tattgttgtt gtttgatccc 540
taaccctaca aagagccttc ctattcccct cgctgttgga gcaaaccatt ataccttact 600
tc 602

<210> 97
<211> 582
<212> DNA
<213> Homo sapiens

<400> 97
gcggacgcgt gggcggacgc gtgggcggac gcgtgggtgg acacagccac agcaacagag 60

caggcaaagc agctggtgaa gtcaggagcc atcagtgcca tcaaggctga gaccaagaac 120

tcaggcttca agcgttctcg aacccttgag gggaagttaa aggaccccga gaagggacca 180

gtccccactt tccagccgtt ccagaggagc atatctgctg atgatgacct gcaagagtca 240

tccagacgtc cccagaggaa atctctgtat gagagctttg tgtcttctag tgatcgactt 300

cgagaactag gaccagatgg agaagaggca gagggcccag gggctggtga tggtccccct 360

cgaagctttg actggggcta tgaagaacgc agtggtgccc actcctcagc ctcccctccc 420

cgaagccgca gccgggaccg cagccatgag aggaaccggg acagagaccg agatcgggag 480

cgggatcgag accgggatcg agacagagac agagagcggg acagggatcg ggatcgggat 540

cgagatcgag accgggaacg ggacagggat cgggagcggg at 582

<210> 98

<211> 599

<212> DNA

<213> Homo sapiens

<400> 98
gcccacgcgt ccgcccacgc gtccgatatt actgatgcca cacttgccat tgtgaaagga 60

gagacagttc cacttgatgt cttgcagatc aagggtgaaa aggaacagcc tgtatttgca 120

atgaccggcc ttcgatgggg atctttcaga gatgctggcg tcaaagttag caagtactgg 180

tatcttgggc ctctaaaaat caaagcagcc cactttttca gcactcttaa ggagtggcct 240

cagactcatc aagcctctat ctcatacacg ggacctacag agagacctcc caatgaacca 300

gaggagaccc ctgtacaaag gccttctttg tacaggagaa tattacgaag gcttgcgtcc 360

tactgggcac aaccacagga tgccctttcc caagaggtga gcccggaggt ctggaaagat 420

gtgcagctgt ccaccattga actgtccatc acaacacgga ataatcagct tgacccgaca 480

agcaaagaag attttctgaa tatctgcatt gaacctgaca ccatcagcaa aggagacttt 540

ataactatag gaagtcgaaa ggtgagaaac cccaagctgc acgtggaggg cacggagtg 599

<210> 99

<211> 569

<212> DNA

<213> Homo sapiens

<400> 99
gtccaggggt tctaccagtg cctacaccaa gagtggctac tgtgtcaaca ggttttcttc 60

acttctgcca ggaggcaaca ggcgaaactc aacagcaaaa gactacacca ttctagattg 120

catttacaat gaggtaaacc agactgattt ccgattctac tggatgcatt caaagttacc 180
agaagaagaa ggactgggag agaaaaccaa gcttaatcct tttaaatttg tggggctaaa 240
gaacttccct tgcactcccg aaagcctgtg tgatgtgcta tctatggatt tcccttttga 300
ggtagatgga cttctcttct accacaaaca gacccactac agccccggaa gcactccctt 360
ggtgggctgg ctgcgcccct acatggtgtc agatgtcctt ggtgtagctg tgccggctgg 420
cccgctgacc accaagccag actatgctgg gcaccagctc cagcagatta tggagcacaa 480
gaagagccag aaggaaggca tgaaggagaa actcacacac aaggcctctg agaatgggca 540
ctatgaattg gagcacctgt ctactccca 569

<210> 100
<211> 597
<212> DNA
<213> Homo sapiens

<400> 100
gaggtgagga cctttgcggt gagtgttaca gctcttaagg tggcgcgtct ggagttgttc 60
gttcctcccg gtgggttcgt ggtcttattg gcttcgggag tgatgctgca gacctttgtg 120
gtgagtgtta cagctcataa aggcagtgtg gacccaaaga gtgagcagca gcaagatcta 180
ttgcaaagac cgaaacaaca aagcttccgc agtgtggaag gggacgggag tgggttgcca 240
gtgctggctt gggcagcctg cttttattct cttatctggc cccacccaca tcctgctgat 300
tggtccattt tacagagagc cgagtggtct gttttgacag ggtgctgatt ggtgcattta 360
caatccctga gctagacaca aaggttctcc acatccccac tagattagct agatacagag 420
tgtcaacaca aaggttctcc aagtccccac cagagtagct agatacagag tgtcgattgg 480
tgcattcaca aaccgtgagc tagacacagg gtgctgattg gtgtgtttac aaaccgtgag 540
ctagatacag agtgccgatt ggtgtattta caatccccta gctagacata aaggttt 597

<210> 101
<211> 599
<212> DNA
<213> Homo sapiens

<400> 101
gctcccctcc tcaattcagg agctggtttt ccatccatcc cagtcggcaa gagccccatg 60
gtggagcagg ctgtgcagac tggttctgct gacaacctga atgctaaaaa gctgttacct 120
ggcaagggca ccacagggac gcagctcaac ggtcgtcagg cccagccgag cagcaagacg 180
gccagcgatg tagtccagcc ggcagctgtg caagctcaag ggcaggtgaa tgacgagaac 240
agaagacctc agaggaggcg atcaggaaac aggcgaacaa ggaatcgctc cagagggcaa 300

aaccgtccaa ctaacgttaa ggaaaacaca atcaaatttg agggtgactt tgatttcgag 360

agtgcaaatg cccagttcaa ccgagaggag cttgacaaag aatttaagaa gaaactgaat 420

tttaaagatg acaaggctga gaagggggaa gagaaggacc tggctgtggt gacccagagt 480

gccgaagcgc ccgctgagga agaccttctg gggcccaact gctactatga caaatccaag 540

tcgttcttcg acaacatctc ttctgaactc aagaccagct ccaggcggac gacgtgggc 599

<210> 102

<211> 584

<212> DNA

<213> Homo sapiens

<400> 102
ggaccagatc aatcactgca cattgaagct gcgtcagtcc accggactga tggagtactg 60

cctggaggtg atcaaggaga acgacccctc cgggttctta cagatctcag atgctctgat 120

caagcgcgtc caggtgtctc aggagcagtg ggtcaaaggc gccctggagc cgaaagtgtc 180

tgcggagttt gatctgactt tggacagcga gccgctgctg caggccatcc accagctgga 240

cttcattcag atgaaatgta gggtgccacc cgtcccccta ctgcagctgg agaaatgctg 300

cacccgtaac aacagcgtca cgctggcctg gaggatgcca cccttcaccc acagccccgt 360

ggacggctac atcctggagc tggacgacgg tgccggggga cagttccggg aagtgtacgt 420

cggtaaggag actttgtgta ccatcgacgg tcttcacttc aacagcacct acaacgcccg 480

agtcaaagct ttcaactctt ctggtgtcgg gccttacagt aaaactgtcg tcctgcagac 540

atccgatgtg gcctggttca catttgaccc caactctggg catc 584

<210> 103

<211> 546

<212> DNA

<213> Homo sapiens

<400> 103
gcccacgcgt ccgagcctcg gcctctgcct cagccgcggc gtcgtcgccc gcgggggcg 60

gcgccgaggc gctggagctg ctggagcact gcggcgtgtg cagagagcgc ctgcgacccg 120

agagggagcc ccgcctgctg ccctgtttgc actcggcctg tagtgcctgc ttagggcccg 180

cggcccccgc cgccgccaac agctcggggg acggcggggc ggcgggcgac ggcaccgtgg 240

tggactgtcc cgtgtgcaag caacagtgct tctccaaaga catcgtggag aattatttca 300

tgcgtgatag tggcagcaag gctgccaccg acgcccagga tgcgaaccag tgctgcacta 360

gctgtgagga taatgcccca gccaccagct actgtgtgga gtgctcggag cctctgtgtg 420

agacctgtgt agaggcgcac cagcgggtga agtacaccaa ggaccatact gtgcgctcta 480

ctgggccagc caagtctcgg gatggtgaac gtactgtcta ttgcaacgta cacaagcatg 540

aacccc 546

<210> 104

<211> 574

<212> DNA

<213> Homo sapiens

<400> 104
gctgctctcc acagggctcc ccgccccacc cggcctgata aagcgcgccg actgggctac 60

aaggccaagc aaggttacgt tatatatagg attcgtgttc gccgtggtgg ccgaaaacgc 120

ccagttccta agggtgcaac ttacggcaag cctgtccatc atggtgttaa ccagctaaag 180

tttgctcgaa gccttcagtc cgttgcagag gagcgagctg gacgccactg tggggctctg 240

agagtcctga attcttactg ggttggtgaa gattccacat acaaattttt tgaggttatc 300

ctcattgatc cattccataa agctatcaga agaaatcctg acacccagtg gatcaccaaa 360

ccagtccaca agcacaggga gatgcgtggg ctgacatctg caggccgaaa gagccgtggc 420

cttggaaagg gccacaagtt ccaccacact attggtggct ctcgccgggc agcttggaga 480

aggcgcaata ctctccagct ccaccgttac cgctaatata agtaaagttt gtaaaattca 540

tacttaataa acaatttagg acagtcatgt ctgc 574

<210> 105

<211> 375

<212> DNA

<213> Homo sapiens

<400> 105
ggcggaagcg gctctgttcg ccgcctctcc caccggcccg atgagctgca gcggctccgg 60

cgcggacccc gaggcggcgc cggcctccgc cgcctcggcc ccgggccccg cgcccccggt 120

ctcggctccc gccgcgctgc cctccagcac cgccgcggag aacaaggcca gccccgcggg 180

gacagcgggg ggacctgggg ctggagcagc tgctgggggc acgggaccct tggcggcgcg 240

ggccggggag ccagctgagc ggtgtggggc gggtgagggc cgggacgggt gagggcgagg 300

gaagcgtctt gggctgggcc cagcggcggg tgagggcaga gggtatagac agacctagct 360

ggggccctgc gcctt 375

<210> 106

<211> 514

<212>  DNA

<213>  Homo sapiens

<400>  106
ggagaaaaca gagagcaata ccaagtggtc atccaggcca aagacatggt cggccagatg      60

ggaggcttat cggggacaac cactgtgaac atcacgctga cagatgtcaa tgacaaccca     120

ccacgtttcc cccagaacac tattcatctt cgagttcttg aatcctcccc agttggcaca     180

gccattggaa gtgtcaaagc aactgatgct gacactggga aaaatgctga agtagaatac     240

cgaattattg atggtgacgg tactgatatg tttgacatcg tgactgagaa ggacacacag     300

gaaggcatca tcactgtgaa aaagccactc gactatgaga gccgaagact ttatactctg     360

aaagtcgaag cagaaaacac ccatgtagat ccccgttttt attacctagg accatttaaa     420

gatactacca tagtgaaaat ctctatagaa gatgtggatg aaccttctgt ttttagtagg     480

tcctcctatc tgtttgaagt tcatgaagat attg                                 514

<210>  107

<211>  577

<212>  DNA

<213>  Homo sapiens

<400>  107
gcttgctgga gctgccgggg gcccagggat cggcagggct gctggcagag gaatcccagc      60

tggggttccc atgccccagg ctcctgcagg acttgctggg ccagtccgtg gggttggcgg     120

gccatcccaa caggtgatga ccccataagg aagaggtact gttgcagccg ctgcagctgc     180

tgccacagcc agtattgccg gggctccaac ccagtaccca cctggccgtg ggggtcctcc     240

cccacctatg ggccgaggag caccccctcc aggcatgatg ggcccacctc ctggtatgag     300

acctcctatg ggtcccccaa tggggatccc ccctggaaga gggactccaa tgggcatgcc     360

ccctccggga atgcggcctc ctccccctgg gatgcgaggc cttctttgac ccttggccac     420

agagtatgga agtagctccg cagaggcgtg ggctcgattc ctcagggcca cgttaccaca     480

gacctgtttg tttcttatgc tgttgttcgt ggagtctcat gggattgtct ggtttccctt     540

acagggcccc ctcccccggg aatgcgctca ccaaggc                              577

<210>  108

<211>  610

<212>  DNA

<213>  Homo sapiens

<400>  108

gcggcgaggc tgaggcggcc ggcgcggggc gcaggcggcg cggccatggg ctcgcagggc 60

agtccggtga agagctacga ctacctgctc aagttcctgc tggtgggcga cagcgacgtg 120

ggcaagggcg agatcctgga gagcctgcag gacggcgcgg cagagtcccc gtacgcctac 180

agtaacggga tcgactacaa gaccaccacc atcctgctgg acggccggcg cgtgaagctg 240

gagctctggg acacgtcggg ccagggccgg ttctgcacca tcttcaggtc ctactccagg 300

ggcgctcagg ggatcctctt ggtgtatgac atcaccaacc gctggtcctt tgacggcatc 360

gaccgctgga tcaaggagat cgatgagcgg caggtcccga cggagcaggc ccgcgcgtac 420

gcagagaaga actgcatgac cttctttgag gtcagccccc tgtgcaactt caacgtcatc 480

gagtccttca cggagctatc ccgcatcgtg ctcatgcggc acggcatgga gaagatctgg 540

aggcccaacc gagtgttcag cctgcaggac ctctgctgcc gggccatcgt ctcctgcacc 600

cccgtgcacc 610

<210> 109

<211> 580

<212> DNA

<213> Homo sapiens

<400> 109
gagctgctga aggagggcca ccgcatggac aagcccgcca actgcacaca cgacctgtac 60

atgatcatgc gggagtgctg gcatgccgcg ccctcccaga ggcccacctt caagcagctg 120

gtggaggacc tggaccgtgt ccttaccgtg acgtccaccg acgagtacct ggacctgtcg 180

gcgcctttcg agcagtactc cccgggtggc caggacaccc ccagctccag ctcctcaggg 240

gacgactccg tgtttgccca cgacctgctg cccccggccc cacccagcag tgggggctcg 300

cggacgtgaa gggccactgg tccccaacaa tgtgaggggt ccctagcagc ccaccctgct 360

gctggtgcac agccactccc cggcatgaga ctcagtgcag atggagagac agctacacag 420

agctttggtc tgtgtgtgtg tgtgtgcgtg tgtgtgtgtg tgtgcacatc cgcgtgtgcc 480

tgtgtgcgtg cgcatcttgc ctccaggtgc agaggtaccc tgagtgtccc cgctgctgtg 540

caacggtctc ctgactggtg ctgcagcacc gaggggcctt 580

<210> 110

<211> 343

<212> DNA

<213> Homo sapiens

<400> 110
ggtgaatcag gcaccggagt gcaggttcgg gggtggaatc cttgggccgc tgggcaagcg 60

gcgagacctg gccagggcca gcgagccgag gacagagggc gcgcggaggg ccgggccgca 120

gccccggccg cttgcagacc ccgccatgga cccgttcctg gtgctgctgc actcggtgtc 180
gtccagcctg tcgagcagtg agctgaccga gctcaagttc ctatgcctcg ggcgcgtggg 240
caagcgcaag ctggagcgcg tgcacagcgg cctaaacctc ttctccatgc tgcttggagc 300
agaacgacct ggagcctggg ctacaccgat ctcctgcgcg agc 343

<210> 111
<211> 496
<212> DNA
<213> Homo sapiens

<400> 111
gcggacgcgt gggcagcggg gagaggtgga gaggcttcag ccctgcctcc caccccttcc 60
ccagtgcatc cacagggacc tggctgcccg caatgtgctg gtgaccgagg acaacgtgat 120
gaagatcgca gacttcgggc tggcccggga cgtgcacaac ctcgactact acaagaagac 180
aaccaacggc cggctgcccg tgaagtggat ggcgcctgag gccttgtttg accgagtcta 240
cactcaccag agtgacgtgt acgtgtcctg cagagctcag gcttcagggg tggaggcggg 300
aactgggcag agccaggacc ccagctgcag tccccaggcc tgtgccctgg agctcctggg 360
tgtggtttct acccctccct gggggcagca gcgcagccct ggcctattcc cctggtgccc 420
gcccaggtgt ctgtcctggg agtctcagga cagcctgacc tcaccttccc ctgcagctgg 480
tcctttgggg tcctgc 496

<210> 112
<211> 526
<212> DNA
<213> Homo sapiens

<400> 112
gggccaaggt gcttctgcat ctggaggaga agacctgtgt ggtgggattt gcagggctgc 60
gccagagagc cctggtggcc gtcacggtca cagacccggc cccggtggcc gactatctga 120
cctcacagtt ctatgccctc aactacagcc tccggcagcg catggacatc ctggatgtgc 180
tgactctggc tgcccaggag ctgtctaggc ctgggtgcct cgggaggact ccccaacctg 240
gctccccaag tcccaacacc ccgtgcctgc cagaggcagc cgtctctcag cctggcagtg 300
ccgtggcgtc tgactggcgg gtggtggtgg aggagcggat cagaagcaag acccggcggc 360
tctccaaggg tggcccgagg cagggcccgg caggcagccc cagcagattc aactccgtgg 420
ccggccactt cttcttcccc ctccttcagc gctttgacag gcctctggtg acccttcgac 480
ctcttgggag aagaccagct ggttctcgga aggctggcgc acacct 526

<210>  113

<211>  612

<212>  DNA

<213>  Homo sapiens


<400>  113
gggaggaggt gttgcccatg gcccccgggg ccccacaagt tactactaca tgctgcccat      60

gaaggtgcgg gcgctgggtc tcaaagtggc actgaccgtc aagctggccc aggacgacct     120

gcacatcatg gactccctag agctgcccac cggagaccca cagtacctga cagagctggc     180

gcactaccgc cgctgggggg actccgtact cctcgtggac ttaacacacg aggagatgcc     240

acagagcatc gtggaggcca cctctaggct taagaccttc aacttgatcc cggctgttgg     300

cctaaatgtg cacagcatgc tcaagcacca gacgctggtc ctgacgctgc ccaccgtcgc     360

cttcctggag gacaagctgc tctggcagga ctcacgttac agacccctct accccgtacc     420

actgttgatg tgaagcacct cttctgagcc aggccgagcc cctggccgac ttgggagcct     480

caggcccacg cccacccttc gaggaaggtg tcacctggac cccttcattc cacggaggaa     540

gctgaggcca cagggagcgg ccatcgccat tgggaagggg cgactccacg gaaagcccag     600

acgggcttct gc                                                         612

<210>  114

<211>  716

<212>  DNA

<213>  Homo sapiens


<400>  114
ggccgccgcc ggggcataga ggagcatcga ctgtacgaaa tggccaacct tcggcgccag      60

ttcaaggagc tgttggagga ccacgggctg ctggctgggg cccaggccgc gcaggtaggg     120

gacagctaca gtcggttgca gcagcgccgg gagcgccggg ccctgcacca gctgaaacgc     180

cagcacgagg agggcgcggg gcgcaggcgc aaggtgctgc ggctgcagga ggagcaggac     240

ggcggctcca gtgacgagga cagggctggc ccagcccccc caggggccag tgatggcgtg     300

gacatccagg atgtgaagtt caagcttcgg catgacctgg cgcagctgca ggccgctgcc     360

agctcagccc aggacctgag ccgcgagcag ctggctctgc tgaagctggt gctgggccgg     420

ggcctgtacc cacagctggc cgtccccgac gccttcaaca gcagccgaaa ggactcagac     480

cagattttcc acacgcaggc caagcatggg cgccgtgctg caccccacct gcgttttcgc     540

tggcaccccc gaggtgctgc acgcacagga tgctggaggc cagcaactgc gacggaagcc     600

gaggcatggc tgcgtgctgg gagtcacatt cagatttggc tgaccttctt gtctgagcca     660

ggccaccaaa gccatgccac gacaaggaca agatgagcag caaacaccag ctcctc         716

<210> 115

<211> 626

<212> DNA

<213> Homo sapiens

<400> 115
gcccacgcgt ccgcccacgc gtccggcggg agcagctacc caggcttccc tggagtcggc 60

cccacggatc atgcggctgg tggccgaatg cagccgctcc agggcccggg caggcgagct 120

gtggctgccg catgggacag tggccactcc tgtgttcatg ccagtgggca cgcaggccac 180

catgaagggc atcacgaccg aacagctgga cgctctgggt tgccgcatct gcctgggcaa 240

tacctaccat ctgggtctaa ggccggtggg tgggctctgc ccgcgcgggg aggcggcgag 300

gcggcgaggc gtggggagcc atggagcgtc ctccaggccc ggacactcct cccaaagtca 360

atcgaccagc tgtattgagc gcccccaatg accaggcctt gtactgggcg tgaaagagca 420

gcagggacta aagctggcca gatctgtctc ccaagaaggg cccccaggga cgcggtcacc 480

cctaacctga cactttcttc catcaaccca gggacccgag ctgatccaga aagccaacgg 540

tctccacggc ttcatgaatt ggcctcataa tctgctaacg gacagcggcg gtttccagat 600

ggtgtcgctg gtgtctctgt ccgagg 626

<210> 116

<211> 605

<212> DNA

<213> Homo sapiens

<400> 116
gggcctgctc cctggagtcc cgggatgtgg tgctggagct catgtcctac ttcctctact 60

ctcccgtgca ctgccggcca gagctcaaga agctgctgca cgggctgggc cttcaggacc 120

cagagggctt cctattcaag gagatgatga cctgggtcca gggcccagac ctggactcca 180

aggccggcct gcgcacttgc tgccaccaga aactggagga catgatccag gagcttcagg 240

ttgggccggc aggggccggg ggggccttgg gaaccctgtt gccttctctg gctttctgca 300

agtcctgggg tcaaagccag ctgaggccac aggctccttc cttgccttgc ccagtcctgg 360

gcctccagcc tccctgccct gagcatctct agggatttcc ctgtgcagct ggaagtcagc 420

agggggtgtc tggcccgaac cagggacagc cctggacctc cttgtcaggg gagtggcagc 480

ccctgccttg ggctgagcat cgggcagaag ggagtttcag tggccatgtg gatgctcgag 540

gagccagggt gcttggccat gggtcacctc ctgacagatg gaaagctcgc agccactgtc 600

tgcag 605

<210> 117

<211> 503

<212> DNA

<213> Homo sapiens

<400> 117
gggagaaggt ctctgaagga gaaaagcaaa gaagctcttt tggcctcaca aaagccattt 60

aaatttatag caagggagga acagaagcga gcagcccggg aaaagcagct gagagacttt 120

cttaagtata aaaagaaaac aaatcgattt aaagccagac ccattcctcg atctacttat 180

ggttcaacta ccaatgacaa gttaaaagaa gaagagctct atcgaaacct taggacacag 240

ctgagagccc aggagcattt acagaactca tctcctctgc cttgtaggtc agcttgcgga 300

tgcaggaacc ccaggtgtcc tgaacaggct gtaaagttga agtgtaaaca caaggttagg 360

tgcccaactc ctgattttga ggaccttcct gagagatacc agaaacacct ctcagaacac 420

aagtctccaa aactcttaac agtgtgtaaa ccatttgatc ttcatgcatc tccacatgca 480

tctattaaaa aaaaaaaaaa agg 503

<210> 118

<211> 435

<212> DNA

<213> Homo sapiens

<400> 118
gcgcccgcat ccgggaccgc aaggcccagc aggtgtttgt ggacgagggc cacacggtgc 60

agtttgtgtg ccgggccgat ggcgacccgc cgcccgccat cctctggctc tcaccccgaa 120

agcacctggt ctcagccaag agcaatgggc ggctcacagt cttccctgat ggcacgctgg 180

aggtgcgcta cgcccaggta caggacaacg gcacgtacct gtgcatcgcg gccaacgcgg 240

gcggcaacga ctccatgccc gcccacctgc atgtgcgcag ctactcgccc gactggcccc 300

atcagcccaa caagaccttc gctttcatct ccaaccagcc gggcgaggga gaggccaaca 360

gcacccgcgc cactgtgcct ttccccttcg acatcaagac cctcatcatc gccaccacca 420

tgggcttcat ctctt 435

<210> 119

<211> 677

<212> DNA

<213> Homo sapiens

<220>

<221>  misc_feature

<222>  (610)..(610)

<223>  n = a, c, g or t


<400>  119
gtggagctct cggagtctgt gcagaaaggc ttccagatgc tggcggatcc ccgctccttc      60

gactccaacg ccttcacgct tctcctccgg gcggcattcc agagtctgct ggacgcccag     120

gcggacgagg ccgtgttaga tcatccagac ttgaaacata tcgacccagt ggttttaaaa     180

cattgtcatg cagcagctgc aacttacata ctagaggcag gaaagcaccg agctgacaag     240

tcaactctaa gcacttatct agaagactgt aaatttgaca gagagcgaat agaactgttt     300

tgcacggaat atcagaataa taagaattcc ctagaaatcc tactgggaag tataggcaga     360

tctctccctc atataacgga tgtttcttgg cgcttggaat atcagataaa gaccaatcaa     420

cttcatagga tgtacagacc tgcatatttg gtgaccttaa gtgtacagaa cactgattcc     480

ccatcctatc cagagattag ttttagttgc agcatggaac aattacagga cttggtgggg     540

aaactgaaag atgcttcgaa aagcctggaa agagcaactc agttgtaact tggggaagtt     600

aacgatccgn ccgagtgcag aggaaaacca gaaacgcctt gccttcagct gaaccaccgt     660

ttgtgcgagc tggatgt                                                    677

<210>  120

<211>  567

<212>  DNA

<213>  Homo sapiens


<400>  120
gcccacgcgt ccgggcgaag tgcgcgcgcg attgctccgg gctctgcgcc tggtgcggcg      60

gctgcgcggc ctgagccagg ccctgcgcga ggccgaagcc gacggcgcgg cctgggtcct     120

gctgtactcc cagaccgcgc cgctgcgcgc ggaactggcc gagcggctac agccgttgac     180

ccaggctgcc tatgtgggcg aggcgcggag gaggctggag agggtccggc gccgccggct     240

gcggcttcgc gagagggccc gggaacgcga ggccgagcgg gaggcagagg ccgcgcgggc     300

agtggaacgc gagcaggaga ttgaccgctg gagggtgaag tgtgtgcagg aggtggagga     360

gaagaagcgg gagcaggaac tcaaagcagc cgctgatggc gtactatctg aagtgaggaa     420

aaaacaagca gataccaaaa gaatggtgga cattctacgg gctttggaga aattgaggaa     480

actgaggaaa gaggctgcag cgaggaaagg ggtctgtcct ccagcctcag cagatgagac     540

ttttacgcat catcttcagc gactgag                                         567

<210>  121

<211>  390

<212>  DNA

<213>  Homo sapiens


<400>  121
gcccacgcgt ccgtgggaaa gaagctgggc gagctgcagg agaagctgag cgagctgaag      60

gaaacggtgg agctgaagag ccaagaggct caaagtctgc agcagcagcg agaccagtac     120

ctgggacacc tgcagcagta tgtggccgcc tatcagcagc tgacctctga gaaggaggtg     180

ctgcataatc agctactgct gcagacccag ctcgtggacc agctgcagca gcaggaagct     240

cagggcaaag cggtggccga gatggcccgc caagagttgc aggaaaccca ggagcgcctg     300

gaagctgcca cccagcagaa tcagcagcta cgggcccagt tgagcctcat ggctcaccct     360

ggggaaggag atggactgga ccgggaggag                                      390

<210>  122

<211>  455

<212>  DNA

<213>  Homo sapiens


<400>  122
ggccagaaac tgaaagaagc caacaagcag cagaacttca acacagggat caaggacttt      60

gacttctggc tgtctgaggt ggaggccctg ctggcatccg aagattatgg caaagacctg     120

gcttctgtga acaacctgct gaaaaagcat caactgctgg aagcagatat atctgcccat     180

gaggatcgcc tgaaggacct gaacagccag gcagacagcc tgatgaccag cagtgccttc     240

gacacctccc aagtaaagga caagagggac accatcaacg ggcgcttcca gaagatcaag     300

agcatggcgg cctcccggcg agccaagctg aatgaatccc atcgcctgca ccagttcttc     360

cgggacatgg atgacgagga gtcctggatc aaggagaaga agctgctggt gggctcagag     420

gactacggcc gggacctaac tggcgtgcag aacct                                455

<210>  123

<211>  462

<212>  DNA

<213>  Homo sapiens


<400>  123
gtgcttatga gccctttctt catctgtccc caggtgtatg gggcatctaa cgtggagctc      60

atcacccgca cacggacaga acatctttca gaacagcaca agggcaaggt caaaggctgt     120

aagacacctt tgcagtcctt cctgggaatc gctgagcagc acgggggccc ccaaaatggg     180

gtgagtgtgt gcaggggtac ccgtaagtgg agggatgtgg atgtggctca agaggaggct 240

tgggaagcgt cctgcttagc atggcctatg tggacctcct ttccacaatg cccaaggcca 300

tgtcagcgcc acactggcag aggggacagc ttgggggcct tgtgacaaca aatgagttgg 360

cctggcatta ggaactgagg ggagctcctg ggcccctccc catgtggtac agaccctgat 420

cactcagacc tctgagccaa gccaaccccc actgccatca ct 462

<210> 124

<211> 290

<212> DNA

<213> Homo sapiens

<400> 124
gcggacgcgt gggctcgcaa atctggaagc ggatcgcgtg gagtgacggt cacaccgcgg 60

cgaattaatt cccaaagact catgttacat gagaaagcca ccaagaagac caaagaaaag 120

gagacaagga tggctcttcc tcagggatgc ttgactttca aggatgtggc tatagaattc 180

tctttggagg agtggaaatg cctgaaccct gcacagaggg ctttatacag ggccgtgatg 240

ttggagaact acaggaacct ggagtctgtg ggattgactt ctaaggactc 290

<210> 125

<211> 510

<212> DNA

<213> Homo sapiens

<400> 125
gcctctgctg agatcaacca agatttgaac atccagctgc tgaaggacgg ctaccggtta 60

gatgagatcc ccgacgacga ggacctagac ctcatccccc ccaagtccgt gaaccccacg 120

tgcatgtgct gccaggccac gtcctccacc gcctgccaca ttcagtagcg ggcggggcca 180

ctgcggtcgc cggggcgggg ccgccaaggg ccgggtagga cgggcagagg ccgacccctt 240

cccagctcgt ctgcctgccc ccggccccgc gcccctacag ccgccaacct cgtataacag 300

tcattgcttc ctcctatggt aggacgtgta cctctgtgtg ttcatggagc tggagaaacc 360

aaaactgggt ctctctccac ccgggagctg aggaggggtg gaggccgttt gttcagttac 420

ctgagggaac ctcacccagc accctgcccc gcttcccagg gctggccaag agagaagact 480

ggacggcagg gaggtcaaca ttaacgagtg 510

<210> 126

<211> 622

<212> DNA

<213>  Homo sapiens

<400>  126
gctccgtggg aaggaggctt gtttaaacta cggatgcttt tcaaagatga ttatccatct      60
tcgccaccaa aatgtaaatt cgaaccacca ttatttcacc cgaatgtgta cccttcgggg     120
acagtgtgcc tgtccatctt ataggaggac aaggactgga ggccagccat cacaatcaaa     180
cagatcctat taggaataca ggaacttcta aatgaaccaa atatccaaga cccagctcaa     240
gcagaggcct acacgattta ctgccaaaac agagtggagt acgagaaaag ggtccgagca     300
caagccaaga agtttgcgcc ctcataagca gcgaccttgt ggcatcgtca gaaggaaggg     360
attggtttgg caagaacttg tttacaacat ttttgcaaat ctaaagttgc tccatacaat     420
gactagtcac ctgggggggt tgggcgggcg ccatcttcca ttgccgccgc gggtgtgcgg     480
tctcgattcg ctgaattgcc cgtttccata cagggtctct tccttcggtc ttttgtattt     540
ttgattgtta tgtaaaactc gcttttattt taatattgat gtcagtattt caactgctgt     600
aaaattataa acttttatac tt                                              622

<210>  127

<211>  530

<212>  DNA

<213>  Homo sapiens

<400>  127
ggatccccag acgctgcgtc acaactcccc cgctggcgcg ggcggggaag caactgcaaa      60
actttccgca aacttcacgg cgtggtggga cctgggctac gaggtccggc cagcccgggg     120
cgggggcgtg cagctgaccc aggtggccag acccgggccg ccagcatccg ccccgccatc     180
ccgcctcttt ctgcgggcgt ctccagcagc ctagcatccc ctacctccat gccgaggagg     240
acccctcccc cggtctggtg gggaaggggc ggagcccagg gcctggccac cagggcgaag     300
attgggggag ggggaggcag agcccttgtt acgtgtgcgg agcgcaacaa ttcttgcacg     360
tcttgtttta gcattttcgt gacccctgtg tcagtgttcc cggtgcagga gtgtagcact     420
tttgcttgtt ggggctgcat ccagaaacct ggggaaaatc tgtccactgg acgtcccaga     480
agaccggcat caccactccc accaactgga ctgctggccg gtagcaaaag                530

<210>  128

<211>  677

<212>  DNA

<213>  Homo sapiens

<220>

<221>  misc_feature

<222>  (594)..(594)

<223>  n = a, c, g or t


<400>  128
ggactaagca tccatatgga gacgccacca gtcaatacaa ttggagaaaa ggacacctct      60

cagccgcaac aagagtggga aaagaacctt cgggagaacc ttgattcagt tattcagatt     120

aggcagcagc cccgagaccc tcctaccgaa acgcttgagc tggaagtaag cccagatcca     180

gccagccaaa ttctagagca tactcaagga gctgaaaaac tggttgctga acttgaagga     240

gactctcata agtctcatgg atcaaccagt cagatgccag aggcccttca agcttctgat     300

ctctggtact gccccaaact aggctcctgc tgccgggtac tggctttggg gtttcctttc     360

ggatcagggc cgccagaggg ctggacagag ctaactatgg gcgtagggcc atggagggag     420

gaaacttggg gggagctcat agagaaatgc ttggagtcca tgtgtcaagg tgaggaagca     480

gagcttcagc tgcctgggca ctctggacct cctgtcaggc tcacactggc atccttcact     540

caaggccgag actcctggga gctggagact agcgagaagg aagccctggc cagngaagaa     600

cgtgcaaggg gcacagaact atttcgagct gggaaccctg aaggagctgc ccgatgctat     660

ggacgggctc ttcggct                                                    677

<210>  129

<211>  524

<212>  DNA

<213>  Homo sapiens


<400>  129
gcggacgcgt gggcagagga gcagatgcaa ggactgtgca ttgaactgaa tgatttcatt      60

gttgctctat cctcagtcca accctctgcc aaaagggaag gctttgtcac tgtccctaat     120

gtgacatggg cagatattgg tgccctggaa gacattagag aggagctcac catggcaata     180

ttggcaccag tacgcaaccc agaccagttc aaagctcttg gattggtgac tccagctggg     240

gtcctccttg ctggtcctcc tggctgtggg aagactctgc tggcgaaggc tgttgcaaat     300

gagtccggac taaattttat atctgtcaag ggccccgaat tactaaacat gtatgttggt     360

gagagtgaac gtgctgtgcg acaagttttt caacgagcca agaactcagc accctgtgtg     420

atattctttg atgaagtgga tgctttatgt cctcgaagat cagaccgaga gacaggggca     480

agtgtccgag tggtgaatca gctacttaca gagatggatg gtct                      524

<210>  130

<211>  351

<212>  DNA

<213>  Homo sapiens

<400>  130
gccggcctct actacaatgg gctcaaggtg ctggcgctgg ccgccgagag cgaccccaat      60

gtgcggactg agggtcacct gcgcctggtg ggggaggggc cgtccgtgct gctcagtgcg     120

gagaccacgg ccaccaccct gctggctgac atggccacca ccatcatgga gactaccacc     180

accatggcca ctaccaccac gcgccggggc cgctccccca cactgaggga cagcaccacc     240

cagaacacag atgacctgct ggtggcctct gctgagtgtc caagcgatga tgaggacctg     300

gaggagtgtg agcccagtac tggaggagag ttaatattgc ccattatcac g              351

<210>  131

<211>  411

<212>  DNA

<213>  Homo sapiens

<400>  131
gagcgaagga agtaccagga agcggagctc ctgaaacacc tagcagagaa acgggaacat      60

gagagagagg tgatccaaaa ggccattgag gaaaacaaca acttcatcaa gatggctaag     120

gaaaaactgg cccagaagat ggaatccaac aaggagaaca gggaggccca cctcgccgcc     180

atgttggaac ggctgcaaga gaaggaaccg cctgctgcgc ggtgactccc gggacctgat     240

cggtggcctc gtcccatgga caagcacgcc gaggaggtgc ggaaaaacaa ggagctgaag     300

gaagaggcct ccaggtaaag cctagaggcc aaagaacttt ccaggtcagc cggacagctc     360

cagcagctcc acgttccagg cagcctcgcc cgccggctgc gctcccagca c              411

<210>  132

<211>  418

<212>  DNA

<213>  Homo sapiens

<400>  132
gctgagaagt ttccagatga aaaaccaggt gataggtttg atcgagacag accgttgaga      60

ggacgtggag gcccgagagg gggtatgcgc ggcagaggca gaggtggccc tgggaacaga     120

gtttttgacg cttttgacca gagaggaaag cgagaatttg aaagatatgg tgggaatgac     180

aaaatagcag tcagaactga agacaacatg ggtggatgtg gagttcgaac ctggggatcg     240

ggtaaagata ccagtgatgt ggagccaact gcaccgatgg aggaacccac agtggtggag     300

gagtcccagg gcaccccgga agaggagtct ccagccaaag ttcctgagtt ggaggtagaa     360

gaagaaaccc aagttcaaga gatgacttta gatgagtgga aaaatcttca agaacaga      418

<210>  133

<211>  447

<212>  DNA

<213>  Homo sapiens


<400>  133
gaagaggatg acctgagtga gctgccaccg ctggaggaca tgggacaacc cccggcggag      60

gaggctgagc agcctggggc cctggcccga gagttccttg ctgccatgga gcccgagccc     120

gccccagccc cggccccaga agagtggctg gacattctgg ggaacgggct gttgaggaag     180

aagacgctgg tcccagggcc gccaggttcg agccgcccgg tcaagggcca ggtggtcacc     240

gtacatctgc agacgtcgct ggagaatggc acacgggtgc aggaggagcc ggagctggtg     300

ttcactctgg gtgactgtga cgtcatccag gccctggatc tcagtgtccc actcatggac     360

gtgggggaga cggccatggt cactgctgac tccaagtact gctacggccc ccaaggcagc     420

aggagcccat acatcccccc gcacgcg                                         447

<210>  134

<211>  364

<212>  DNA

<213>  Homo sapiens


<400>  134
gctccttcac tctaccctaa gaacacagat ctatttgaga tgattgagaa gatgcaggga      60

agcaggatgg atgaacaacg ctgctccttc ccgccgcccc tcaaaacaga ggaggactac     120

attccatacc cgagcgtgca cgaggtcttg gggcgagaag gacccttccc cctcatcctg     180

ctgccccagt ttgggggcta ctggattgag ggcaccaacc acgaaatcac cagcatcccc     240

gagacagagc cactgcagtc gcccacaacc aaggggaagc tcgagtgcaa ccccacagcc     300

cgcatctacc ggaagcactt tctcggcaag gagcatttca attactactc actggacact     360

ggcc                                                                  364

<210>  135

<211>  608

<212>  DNA

<213>  Homo sapiens


<400>  135

gaggcgaagc ggcacaacct ggccattgat ctgatgtatg gaggcatcta ctgttttctg 60

tgccaggact acatctatga caaagacatg gaaataatcg ccaaggagga gcagcgaaaa 120

gcttggaaaa tgcaaggcgt tggagagaag ttttcaactt gggaaccaac caaacgggag 180

cttgaactgc tgaagcacaa cccgaaaagg agaaagatca cctcgaactg caccataggt 240

ctgcgtgggc tgatcaacct tgggaacaca tgcttcatga actgcatcgt gcaggccctg 300

acccacacgc cacttctgcg ggacttcttc ctgtctgaca ggcaccgctg tgagatgcag 360

agccccagct cctgtctggt ctgtgagatg tcctcactgt ttcaggagtt ttactctgga 420

caccggtccc ctcacatccc gtataagttg ctgcacctgg tgtggaccca cgcgaggcac 480

ctagcaggct acgagcagca ggacgcccac gagttcctca tcgcggccct ggacgtgctc 540

caccgacact gcaaaggtga tgacaatggg aggaaggcca acaaccccaa ccactgcaac 600

tgcatcat 608

<210> 136

<211> 676

<212> DNA

<213> Homo sapiens

<400> 136
gctaatgaag cccgagaaat gggtgtgatt gctgccatga gagatggttt tggtttcatc 60

aagtgtgtgg atcgtgatgt tcgtatgttc ttccacttca gtgaaattct ggatgggaac 120

cagctccata ttgcagatga agtagagttt actgtggttc ctgatatgct ctctgctcaa 180

agaaatcatg ctattaggat taaaaaactt cccaagggca cggtttcatt tcattcccat 240

tcagatcacc gttttctggg cacggtagaa aaagaagcca ctttttccaa tcctaaaacc 300

actagcccaa ataaaggcaa agagaaggag gctgaggatg gcattattgc ttatgatgac 360

tgtggggtga aactgactat tgcttttcaa gccaaggatg tggaaggatc tacttctcct 420

caaataggag ataaggttga atttagtatt agtgacaaac agaggcctgg acagcaggtt 480

gcaacttgtg tgcgactttt aggtcgtaat tctaactcca agaggctctt gggttatgtg 540

gcaactctga aggataattt tggatttatt gaaacagcca atcatgataa ggaaatcttt 600

ttccattaca gtgagttctc tggtgatgtt gatagcctgg aaactgggga catggtcgag 660

tatagcttgt ccaaag 676

<210> 137

<211> 581

<212> DNA

<213> Homo sapiens

<400>  137
ggttaccgcc cactcgctgc ggcgcttctg gctccagacc gccctccgga tcggaccctg      60

cgaatggttt tggctatatc ttcatgctgg gcgtcatcac caggcctcct cacagattcc     120

tgtcccttct gtgtcctgga ctccggatac ctcaactctc agtactttgt gctcagccca     180

ggcccagagc catggctatc tcctcttcct cctgcgaact gcccctggtg gctgtgtgcc     240

aggtaacatc gacgccagac aagcaacaga actttaaaac atgtgctgag ctggttcgag     300

aggctgccag actgggtgcc tgcctggctt tcctgcctga ggcatttgac ttcattgcac     360

gggaccctgc agagacgcta cacctgtctg aaccactggg tgggaaactt ttggaagaat     420

acacccagct tgccagggaa tgtggactct ggctgtcctt gggtggtttc catgagcgtg     480

gccaagactg ggagcagact cagaaaatct acaattgtca cgtgctgctg aacagcaaag     540

gggcagtagt ggccacttac aggaagacac atctgtgtga c                         581

<210>  138

<211>  685

<212>  DNA

<213>  Homo sapiens

<400>  138
gggctgggtc cgatgctggg tacgctgcgc gccatggagg gcgaggacgt ggaagacgac      60

cagctgctgc agaagctcag ggccagtcgc cgccgcttcc agaggcgcat gcagcggctg     120

atagagaagt acaaccagcc cttcgaggac accccggtgg tgcaaatggc cacgctgacc     180

tacgagacgc cacagggatt gagaatttgg ggtggaagac taataaagga aagaaacgaa     240

ggagagatcc aggactcctc catgaagccc gcggacagga cagatggctc cgtgcaagct     300

gcagcctggg gtcctgagct tccctcgcac cgcacagtcc tgggagccgc ctgcagtgcc     360

tcaaagccct ttgaaaaatg aattaagaag gaaatacttg acccaagtgg atatactgct     420

acaaggtgca gagtattttg agtgtgcagg taacagagct ggaagggatg tacgtgtgac     480

tccgctgcct tcactggcct cacctgccgt gcctgccccc ggatactgca gtcgtatctc     540

cagaaagagt cctggtgacc cagcgaaacc agcttcatct cccagagaat gggatccttt     600

gcatccttcc tccacagaca tggccttagt acctagaaat gacagcctct ccctacaaga     660

gaccagtagc agcagcttct taagc                                           685

<210>  139

<211>  524

<212>  DNA

<213>  Homo sapiens

<400>  139

ggcggctact tctggtgctg ctgttgggtg gctgctccgg gcgcatccac cggctggcgc 60

tgacggggga gaagcgagcg gacatccagc tgaacagctt cggtttctac accaatggct 120

ctctggaggt ggagttgagc gtcctgcggc tgggcctccg ggaggcagaa gagaagtccc 180

tgctggtggg gttcagtctc agccgggttc ggtctggcag agttcactcc tattcaaccc 240

gggatttcca ggactgccct ctccagaaaa acagtagcag tttcctggtc ctgttcctca 300

tcaacaccaa ggatctgcag gtccaggtgc ggaagtatgg agagcagaag acgttgttta 360

tctttcccgg gctcctcccg gaagcaccct ccaaaccagg gctcccgaag ccacaggcca 420

cagtcccccg caaggtggat ggcggaggga cctctgcagc cagcaagccc aagtcaacac 480

ccgcagtgat tcagggtcct agtgggaagg acaaggacct ggtg 524

<210> 140

<211> 550

<212> DNA

<213> Homo sapiens

<400> 140
gtgacagcag aagaatggaa gaagaaatat gaaaaagaga aagagaaaaa caagactttg 60

aagaatgtta tccagcatct ggagatggag ctaaacaggt ggaggaatgg agaagctgtg 120

cctgaggatg aacagatcag tgccaaggac cagaagaacc tggagccttg tgataacacc 180

cccatcatag acaatattgc tcctgttgtt gctggcatct ctacagagga gaaagagaag 240

tacgatgagg agatctccag tctctacaga caactggatg acaaggatga tgaaattaac 300

cagcagagcc agctggctga aaagctgaag caacagatgt tggatcagga tgagctttta 360

gcttccacaa gaagagacta tgagaagata caggaggagc tgacacgtct ccagattgaa 420

aatgaggcag ccaaggatga ggtgaaagaa gttctccagg ccctggagga gctggctgtc 480

aattatgacc agaaatcaca ggaagtggag gataagaccc gggccaatga gcagctgaca 540

gacgagctgg 550

<210> 141

<211> 518

<212> DNA

<213> Homo sapiens

<400> 141
ggcgggccgc ggcagccgaa caaaggagca ggggcgccgc cgcagggacc cgccacccac 60

ctcccggggc cgcgcagcgg cctctcgtct actgccacca tgaccgccaa cggcacagcc 120

gaggcggtgc agatccagtt cggcctcatc aactgcggca acaagtacct gacggccgag 180

gcgttcgggt tcaaggtgaa cgcgtccgcc agcagcctga agaagaagca gatctggacg 240

ctggagcagc cccctgacga ggcgggcagc gcggccgtgt gcctgcgcag ccacctgggc 300

cgctacctgg cggcggacaa ggacggcaac gtgacctgcg agcgcgaggt gcccggtccc 360

gactgccgtt tcctcatcgt ggcgcacgac gacggtcgct ggtcgctgca gtccgaggcg 420

caccggcgct acttcggcgg caccgaggac cgcctgtcct gcttcgcgca gacggtgtcc 480

cccgccgaga agtggagcgt gcacatcgcc atgcaccc 518

<210> 142

<211> 655

<212> DNA

<213> Homo sapiens

<400> 142
gggaagtccc gtaacggaga cgctggtcag gacgttccca cctcctctga cactgccgag 60

tccgatcgga gaggggtcac cgcctccttc agcgaggagg aggggggcgg agcccgactc 120

aggatcatgg attttcctgg tcactttgaa caaatcttcc agcagctgaa ctaccagaga 180

cttcatggcc agctctgtga ttgtgtcatt gtagtgggga atagacactt taaagcccac 240

cgctccgtgc tggcagcatg cagcacgcat ttccgagccc tgttctcagt ggcagaagga 300

gatcagacca tgaacatgat ccagctggat agcgaggtgg tgacagcaga ggcctttgct 360

gcactgattg acatgatgta tacctccacc ctcatgctgg gggagagcaa tgtaatggat 420

gtcttattgg cagcctctca cctgcatttg aactctgttg ttaaggcatg taaacattac 480

ttaacgacaa ggacgctgcc catgtctccc cccagtgagc gcgttcagga gcagagcgcc 540

cgcatgcagc gctcctttat gctacagcag ctgggactaa gcatcgtgag ctcagccctc 600

aattccagcc agaatggcga ggagcagcca gcccccatga gctctttcat gcgca 655

<210> 143

<211> 660

<212> DNA

<213> Homo sapiens

<400> 143
ggccaccgtg tgccagcccg gaccagctca cagacagccc tgcctgtctt acttgggctg 60

ctcggcttca ctgtctcagg gcccctgaac ctcccagagc cctccgtgat tgttggaatg 120

gtgagcttgt cggccacaga ctgctacatt gtgcatgaga tctacaatgg ggagaatgcc 180

caagaccagt ttgagtacga gctggagcag gccctggaag cccagtacaa gtacattgtg 240

attgagccca ctcgcattgg cgacgagaca gcccgctgga tcaccgtggg caactgcctg 300

cacaagacgg ccgtgctggc gggcaccgcc tgcctcttca ccccgttggc gctgccctta 360

gattattccc actacatttc cctgcccgct ggtgtgctga gcctggcctg ctgcaccctc 420
tatgggatct cctggcagtt tgacccttgc tgcaagtacc aagtggagta cgacgcctat 480
aaactgtcgc gcctgcctct gcacacactc acctcctcca ccccggtggt gctggtccgg 540
aaggacgacc tgcacagaaa gacactgcac aacacgatag cactggccgc cctggtgtac 600
tgtgtaaaga agatttacga actctatgcc gtatgatttc agtagaacag ggagcgaagc 660

<210> 144
<211> 299
<212> DNA
<213> Homo sapiens

<400> 144
ggcggagtca gacggcgcta tgtacgccct cttcctcctg gccagcctcc tgggcgcggc 60
tctagccggc ccggtccttg gactgaaaga atgcaccagg ggctcggcag tgtggtgcca 120
gaatgtgaag acggcgtccg actgcggggc agtgaagcac tgcctgcaga ccgtttggaa 180
caagccaaca gtgaaatccc ttccctgcga catatgcaaa gacgttgcca ccgcagctgg 240
tgatatgctg aaggacaatg ccattgagga ggagatcctt gattacttgg agaagacct 299

<210> 145
<211> 518
<212> DNA
<213> Homo sapiens

<400> 145
gcggatacag ccgacaccat gggtttcgga gacctgaaaa gccctgccgg cctccaggtg 60
ctcactgatt acctggcgga caagagctac atcgaggggc atgtgccatc acaagcagat 120
gtggcagtat ttgaagccgt gtccagccca ccgcctgccg acttgtgtca tgccctacgt 180
tggtataatc acatcaagtc ttacgaaaag gaaaaggcca gcctgccagg agtgaagaaa 240
gctttgggca aatatggtcc tgccgatgtg gaagacacta caggaagtgg agctacagat 300
agtaaagatg atgatgacat tgacctcttt ggatctgatg atgaggagga aagtgaagaa 360
gcaaagaggc taagggaaga acgtcttgcc caatatgaat caaagaaagc caaaaaaccc 420
tgcacttgtt ggcaagtctt ccatcttact agatgtgaaa ccttgggatg atgagacaga 480
tatggcgaaa ttagaggagt gcgtcagaag catttaag 518

<210> 146
<211> 635
<212> DNA

<213>  Homo sapiens

<400>  146
gcccacgcgt ccgcttctgg ggcacaggac gacctgagga aggtcacccg catcgcctac      60
tccatggtga agcagtttgg gatggcacct ggcatcgggc ccatctcctt ccctgaggcg     120
caggagggcc tcatgggcat cgggcagcgc cccttcagcc aaggcctgca gcagatgatg     180
gaccatgaag caagactgct ggtggccaag gcctacagac acaccgagaa ggtgctgcag     240
gacaacctgg acaagttgca ggcgctggca aacgcccttc tggaaaagga agtgataaac     300
tatgaggaca ttgaggctct cattggcccg ccgccccatg ggccgaagaa aatgatcgca     360
ccgcagaggt ggatcgacgc ccagagggag aaacaggact tgggcgagga ggagaccgaa     420
gagacccagc agcctccact tggaggcgaa gagccgactt ggcccaagta gttgggaggt     480
gttggctgca cgtgcgggtg gtccgggaag tgagggctca ctcagccacc ctgagttgct     540
tttcagctga ggtttgcact tcctctcgcg gccctcagta gtccctgcac agtgacttct     600
gagatctgtt gattgatgac ccttttcatg atttt                                635

<210>  147
<211>  669
<212>  DNA
<213>  Homo sapiens

<400>  147
gtgccccttg tagggcgctc gggcatcctg ggcgagctgc acaacaacat cttctgtggt      60
gtggcctgcg gtcggggccg gatggcgggc agtaccttct gtgtgtccta ctcgggcctc     120
ctctgccagt tcaatgagaa gagggtgctg gagaagtgga tcaacctgaa ggtctccctg     180
tcttcctgcc tctgtgtcag ccaggagctc atcttctgtg gctgcacaga tgggatagtc     240
cgcatcttcc aggcccatag cctgcactac ctcgccaacc tgcccaagcc acactacctt     300
ggggtagacg tggcacaggg cctggagccc agcttcctct tccacaggaa ggcggaagca     360
gtctacccag atacagtggc actgaccttc gaccccatcc accagtggct gtcctgcgtg     420
tataaggacc acagcatcta catctgggat gtcaaggaca tcaacagagt gggcaaggtg     480
tggtcagagc tcttccacag ctcctacgtt tggaacgtgg aggtgtatcc tgagtttgaa     540
gaccagagag cttgtttgcc atcaggatcc tttctgactt gttcttcaga caacaccatt     600
cgcttctgga acttggacag cagccctgat tctcactggc agaaaaacat cttcagcatt     660
accctgctg                                                             669

<210>  148
<211>  650
<212>  DNA

<213>  Homo sapiens

<400>  148
gaagagaagg acctggagcc ctgggatgca ggtgaactca cagccaatga ggaacttgag      60
gctttggaaa atgacgtatc taatggatgg gatcccaatg atatgtttcg atataatgaa     120
gaaaattatg gtgtagtgtc tacgtatgat agcagtttat cttcgtatac agtgccctta     180
gaaagagata actcagaaga atttttaaaa cgggaagcaa gggcaaacca gttagcagaa     240
gaaattgagt caagtgccca gtacaaagct cgagtggccc tggaaaatga tgataggagt     300
gaggaagaaa aatacacagc agttcagaga aattccagtg aacgtgaggg gcacagcata     360
aacactaggg aaaataaata tattcctcct ggacaaagaa atagagaagt catatcctgg     420
ggaagtggga gacagaattc accgcgtatg ggccagcctg gatcgggctc catgccatca     480
agatccactt ctcacacttc agatttcaac ccgaattctg gttcagacca aagagtagtt     540
aatgggaggt gttccctggc catcgccttg cccatctcct tcctctagcc caccttctcg     600
ctaccagtca ggtcccaact ctcttccacc tcgggcagcc acccctacac                650

<210>  149
<211>  484
<212>  DNA
<213>  Homo sapiens

<400>  149
gagagcgcac accaggtgga gcaggtgctg ctggcatatt ccaggtgcat ccaggcctgt      60
ggccaggaga gaggccaggt gacaggggcc ctgctccttt ctgtggttgg aggaaagatg     120
agtgaaggga tcaacttctc tgacaaccta tgccggtgtg tggtgatggt gggcatgccc     180
ttccccaaca tcaggtctgc agagctgcag gagaagatgg cctacttgga tcaaaccctc     240
cccagagccc ccggccaggc acccccaggg aaggctctgg tggagaacct gtgcatgaag     300
gccgtcaacc agtccatagg cagggccatc aggcaccaga aggattttgc cagcatagtg     360
ctcctggacc agcgatatgc ccggccccct gtcctggcca agctgccggc ctggatccga     420
gcccgtgtgg aggtcaaagc tacctttggc cccgccattg ctgctgtgca gaagtttcac     480
cggg                                                                  484

<210>  150
<211>  652
<212>  DNA
<213>  Homo sapiens

<400>  150
gatgactacc tgaacggccg ggccgtgcag caccgtgagg tccagggctt cgagtcggcc      60

accttcctag gctacttcaa gtctggcctg aagtacaaga aaggaggtgt ggcatcagga     120

ttcaagcacg tggtacccaa cgaggtggtg gtgcagagac tcttccaggt caaagggcgg     180

cgtgtggtcc gtgccaccga ggtacctgtg tcctgggaga gcttcaacaa tggcgactgc     240

ttcatcctgg acctgggcaa caacatccac cagtggtgtg gttccaacag caatcggtat     300

gaaagactga aggccacaca ggtgtccaag ggcatccggg acaacgagcg gagtggccgg     360

gcccgagtgc acgtgtctga ggagggcact gagcccgagg cgatgctcca ggtgctgggc     420

cccaagccgg ctctgcctgc aggtaccgag gacaccgcca aggaggatgc ggccaaccgc     480

aagctggcca agctctacaa ggtctccaat ggtgcaggga ccatgtccgt ctccctcgtg     540

gctgatgaga accccttcgc ccagggggcc ctgaagtcag aggactgctt catcctggac     600

cacggcaaag atgggaaaat ctttgtctgg aaggcaagca ggcaaacacg ga             652

<210>  151

<211>  652

<212>  DNA

<213>  Homo sapiens


<400>  151
gaaggaatag gagtggcaag gatattggaa aatgtggaag tgaaaggtga aaaacctgct      60

aaattgtgcg ttattgcaga atgtggagaa ttgaaggaag gagatgacgg gggaatattc     120

ccaaaagatg gctctggcga cagtcatcca gatttccctg aggatgcgga tatagattta     180

aaagatgtag ataaaatttt attaataaca gaagacttaa aaaacattgg aaatactttt     240

ttcaaatccc agaactggga gatggctatt aaaaaatatg cagaagtttt aagatacgtg     300

gacagttcaa aggctgttat tgagacagca gatagagcca agctgcaacc tatagcttta     360

agctgtgtac tgaatattgg tgcttgtaaa ctgaagatgt caaattggca gggagcaatt     420

gacagttgtt tagaggctct tgaaatagac ccatcaaata ccaaagcatt gtaccgcaga     480

gctcaaggat ggcaaggatt aaaagaatat gatcaagcat tggctgatct taagaaagct     540

caggggatag caccagaaga taaagctatc caggcagaat tgctgaaagt caaacaaaag     600

ataaaggcac agaaagataa agagaaggca gtatatgcaa aaatgtttgc tt             652

<210>  152

<211>  598

<212>  DNA

<213>  Homo sapiens


<400>  152

ggcggcgggg ccgagagcgt gactcgcccg ctccgcgctg cttcccccgc gccgcctccc 60
cgcgccgctc gcgcagccat gtccgaggag aagcccaagg agggtgtgaa gacagagaat 120
gaccacatca acctgaaggt ggccgggcag gacggctccg tggtgcagtt caagatcaag 180
aggcacacgc cgctgagcaa gctgatgaag gcctactgcg agaggcaggg cttgtcaatg 240
aggcagatca gattcaggtt cgacgggcag ccaatcaatg aaactgacac tccagcacag 300
ctggagatgg aggacgagga caccatcgac gtgttccagc agcagacggg aggtgtgccg 360
gagagcatcc tggcagggca cagtttctag agggcccgtc cccagcccgg gccgtccatc 420
ctcgcattgc tgttgaatgg tgagcacgtg accatgccga ccacaaaggt gtctgcggaa 480
actcgaggac attcaccacg atgattttcc tctctttgat gtacttcaag tgcaactcaa 540
aactatatct gcagggatga atctgtaact taaattgggc caatcagaat tgttatct 598

<210> 153
<211> 589
<212> DNA
<213> Homo sapiens

<400> 153
gcggggacca cgcgtctcat ccatggcttc cgcggactcg cgccgggtgg cagatggcgg 60
cggtgccggg ggcaccttcc agccctacct atacaccttg cggcaggagc tgcagcagac 120
ggacccaacg ctgttgtcag tagtggaggc ggttcttgcg gagctgctga cgctagtctt 180
ctggaagtta atccggagca gaaggagcag tcagagagct gttcttcttg ttggcctttg 240
tgattccggg aaaacgttgc tctttgtcag gttgttaaca ggcctttata gagacactca 300
gacgtccatt actgacagct gtgctgtata cagagtcaac attaacaggg gcaatagtct 360
gaccttgatt gaccttcccg gccatgagag tttgaggctt cagttcttag agcggtttaa 420
gtcttcagcc agggctattg tgtttgttgt ggatagtgca gcattccagc gagaggtgaa 480
agatgtggct gagtttctgt atcaagtcct cattgacagt atgggtctga agaatacacc 540
atcattctta atagcctgca ataagcaaga tattgcaatg gcaaaatca 589

<210> 154
<211> 589
<212> DNA
<213> Homo sapiens

<400> 154
gataagatca gcaacatccc tgatgagtat ttcaagcgtt ttaatgcatt gcagtatctg 60
cgtttatctc acaacgaact ggctgatagt ggaatacctg gaaattcttt caatgtgtca 120
tccctggttg agctggatct gtcctataac aagcttaaaa acataccaac tgtcaatgaa 180

aaccttgaaa actattacct ggaggtcaat caacttgaga agtttgacat aaagagcttc 240

tgcaagatcc tggggccatt atcctactcc aagatcaagc atttgcgttt ggatggcaat 300

cgcatctcat aaaccagtct tccaccggat atgtatgaat gtctacgtgt tgctaacgaa 360

gtcactctta attaatatct gtatcctgga acaatatttt atggttatgt ttttctgaga 420

gtcagttttc atagtatcca tattttatta ctgcttatta cttccatgaa ttttaaaatc 480

tgagggaaat gctttgtaaa catttatttt ttttaaagaa aagatgaaag gcaggcctat 540

ttcatcacaa gaacacacac atatacacga atagacatca aactcaatg 589

<210> 155

<211> 430

<212> DNA

<213> Homo sapiens

<400> 155
ggttttgcat atgaagtact atcaaatcct gagaagcgtg agttatatga cagatacgga 60

gagcaaggtc ttcgggaagg cagcggcgga ggtggtggca tggatgatat tttctctcac 120

atttttggtg ggggattgtt cggcttcatg ggcaatcaga gtagaagtcg aaatggcaga 180

agaagaggag aggacatgat gcatccactc aaagtatctt tagaagatct gtataatggc 240

aagacaacca aactacaact tatcaagaat gtgctctgta gtgcatgcag tggccaaggc 300

ggaaagtctg gagctgtcca aaagtgtagt gcttgtcgag gtcgaggtgt gcgcatcatg 360

atcagacagc tggctccagg gatggtacaa cagatgcagt ctgtgtgctc tgattgtaat 420

ggagaaggag 430

<210> 156

<211> 574

<212> DNA

<213> Homo sapiens

<400> 156
gcccagatct ggcaacagat tacaagagag ttaatcaagt atcaagattc tgtggtaaat 60

attcaaagaa caggctgcca gttggaaaaa cgagatgatt tgcactataa agatgaggga 120

ttcagtaatc agagttccca tcttcaagtt cacagagtcc acactggtga aaaaccctac 180

aaaggagaac attgtgtgaa aagtttcagc tggagctctc atcttcaaat taaccaaagg 240

gctcacgcag gagagaagcc ctacaaatgt gaaaaatgtg ataatgcctt ccgtcggttt 300

tcaagtcttc aagcccatca gagagtccac agtagagcaa aatcatacac aaatgatgca 360

agttacagga gttttagtca gaggtcacat cttccccatc atcagagagt tcccactgga 420

gagaatccat acaaatatga agagtgtggg aagaatgttg ggaaaagctc acattgtcaa 480

gctcctctga tagttcatac gggagagaaa ccctataaat gtgaagagtg tggggtgggc 540

ttcagtcaga gatcatatct tcaagttcat ctga 574

<210> 157

<211> 577

<212> DNA

<213> Homo sapiens

<400> 157
gcccggttct tggaggtgga aacgtcgtgg ccggtggact gcaccctaga cgggcttttt 60

ccagcctcag aggcccaggt ctacctggcg ctgggggacc agatgctgaa tgcgacagtc 120

atgaaccacg gggacacgct aacggccaca gccacagcca cggcgcgcgc ggatcaggag 180

ggtgcccggg agatcgtctg caacgtgacc ctagggggcg agagacggga ggcccgggag 240

aacttgacgg tctttagctt cctaggaccc attgtgaacc tcagcgagcc caccgcccat 300

gaggggtcca cagtgaccgt gagttgcatg gctggggctc gagtccaggt cacgctggac 360

ggagttccgg ccgcggcccc ggggcagcca gctcaacttc agctaaatgc taccgagagt 420

gacgacggac gcagcttctt ctgcagtgcc actctcgagg tggacggcga gttcttgcac 480

aggaacagta gcgtccagct gcgagtcctg tatggtccca aaattgaccg agccacatgc 540

ccccagcact tgaaatggaa agataaaacg agacacg 577

<210> 158

<211> 524

<212> DNA

<213> Homo sapiens

<400> 158
gcacgacccg cactcggacg aggacacgcc gacgtcggac gacctggagc agttcgccaa 60

gcagttcaag cagcggcgca tcaagctggg cttcacgcag gccgacgtgg ggttggcgct 120

gggcacactc tacggcaacg tgttctcgca gaccaccatc tgccgcttcg aggccctgca 180

gctgagcttc aagaacatgt gcaagctcaa gccgctgctg aacaagtggc tggaggaggc 240

ggactcaagc accggcagcc ccacaagcat cgacaagatc gcggcgcagg gccgcaagcg 300

caagaagcgg acctctatcg aggtgagcgt caagggcgcg ctggagagcc acttcctcaa 360

gtgccccaag ccctccgcgc aggagatcac caacctggcc gacagcctgc agctcgagaa 420

ggaggtggtg cgggtctggt tctgcaatcg gcgccaaaag gagaagcgca tgacgccgcc 480

cgggatccaa cagcagacgc cccgacgacg tctactcgca ggtg 524

<210> 159

<211> 520

<212> DNA

<213> Homo sapiens

<400> 159
gcccacgcgt ccggacgccg gcctgcaggc gccatggtct tcctcaccgc gcagctctgg 60

ctgcggaatc gcgtcaccga ccgctacttt cggatccagg aggtgctgaa gcacgccagg 120

cacttccggg gaaggaaaaa tcgctgctac aggttggcgg tcagaaccgt gattcgagcc 180

tttgtgaaat gcaccaaagc ccgatacctg aagaaaaaga acatgaggac cctctggatt 240

aatcgaatta cagctgctag ccaggaacat ggactgaagt atccagcgct cattgggaat 300

ttagttaagt gccaggtgga gctcaacagg aaagtcctag cggatctggc caattacgag 360

ccaaagactt tcaaatcttt ggctgccttg gccagtagga ggcgacacga aggatttgct 420

gctgccttgg gggatgggaa ggaacctgaa ggcatttttt ccagagtgga gcagtaccac 480

tgaggactgt tgctgtattg attaggaaaa gagacagagt 520

<210> 160

<211> 647

<212> DNA

<213> Homo sapiens

<400> 160
gtagatgagc tgggattcct agagattgaa actcccatga tgaacatcat cccaggggga 60

gccgtggcca agcctttcat cacttatcac aacgagctgg acatgaactt atatatgaga 120

attgctccag aactctatca taagatgctt gtggttggtg gcatcgaccg ggtttatgaa 180

attggacgcc agttccggaa tgaggggatt gatttgacgc acaatcctga gttcaccacc 240

tgtgagttct acatggccta tgcagactat cacgatctca tggaaatcac ggagaagatg 300

gtttcaggga tggtgaagca tattacaggc agttacaagg tcacctacca cccagatggc 360

ccagagggcc aagcctacga tgttgacttc accccaccct tccggcgaat caacatggta 420

gaagagcttg agaaagccct gaggatgaag ctgccagaaa cgaacctctt tgaaactgaa 480

gaaactcgca aaattcttga tgatatctgt gtggcaaaag ctgttgaatg ccctccacct 540

cggaccacag ccaggctcct tgacaagctt gttggggagt tcctggaagt gactagcatc 600

aatcctacat tcatctgtga tcacccacag ataatgagcc ctttggc 647

<210> 161

<211> 511

<212> DNA

<213>  Homo sapiens

<400>  161
gaagcccgta aagagatgac tagaaaggct attaagacag tcaaacattt tattgagaag      60
ccaaggaaaa gaaactcaga agacgaagct caagaagcta aggattctaa agttacctat     120
gcagatactt tgaatcatct ggagaaatca cttgcccacc tggaaaccct gagccacagc     180
ttcatccttt ctctgaagaa tagtgagcag gaaacactgc aaaaatacag tcacctctat     240
gatctgtccc gatcagaaaa agagaaactt catgatgaag ctgtggctat ttgtttagat     300
ggtcagcctc tagcaatgat tcagcagctg ctagaggtgg cagttggccc tcttgacatc     360
tcacccaagg atatagtgca gagtgcaatc atgaaaataa tttctgcatt gagtggtggc     420
agtgctgacc ttggtgggcc aagggaccca ctgaaggtcc tggaaggtgt tgttgcagca     480
gtccacgcca gtgtggacaa gggtgaggag c                                    511

<210>  162
<211>  565
<212>  DNA
<213>  Homo sapiens

<400>  162
gaagctgagc aactggagcc tcgagttagt gctctggaag aggacatgga tgatgtggag      60
tccagtgaag aggaagaaga ggaggatgag aagttggaaa gagtgccatc acctgatcac     120
cgccggagaa gctaccgaga cttggacaag ccccgtcgct ctcccacact gcgctacagg     180
aggagtagga gccggtctcc cagaaggcgg agtcgatctc ccaaaaggag aagcccctcc     240
cctcgccgag aaaggcatcg gagcaagagt ccaagacgtc accgcagcag gtcccgagat     300
cggcggcaca gatcccgttc caagtcccca tgtcatcacc gtagtcacag acacaggagc     360
cactcaaagt ctcccgaaag gtctaagaag agccacaaga agagccggag agggaatgag     420
taatggactc agtttggttt tagtccacat ggcctcctgt ggatataagg atatctgtat     480
gtggaaggat taagatctcc cccaggcagc tataagaata ttttagcttt tttcttatca     540
agtttctcaa cctttatttt taatg                                           565

<210>  163
<211>  482
<212>  DNA
<213>  Homo sapiens

<400>  163
gcaatgacct gcacaacttc aatgaattcc tcttccgccg ctggggacac tggatggact      60

ggtccctggc attcctgctg gtcatctctc tactggtcac atatgcatcc ttgctattgg 120

tcctggccct gctcctgcgg ctttgtagac agcccctgca tctgcacagc ctccacaagg 180

tgctgctgct cctcattatg ctgcttgtgg cggctggcct tgtgggactg gacatccaat 240

ggcagcagga gtggcatagc ttgcgtgtgt cactgcaggc cacagcccca ttccttcata 300

ttggagcagc cgctggaatt gccctcctgg cctggcctgt ggctgatacc ttctaccgta 360

tccaccgaag aggtcccaag attctgctac tgctcctatt ttttggagtt gtcctggtca 420

tctacttggc ccccctatgc atctcctcac cctgcatcat ggaacccaga gacttaccac 480

cc 482

<210> 164

<211> 602

<212> DNA

<213> Homo sapiens

<400> 164
gtgctgtccg aggaggaggc cacccagatg gaccagtttg ggcaggcagc aggggtcctc 60

attcacagta tccgagaaat agctcagtct caccgggaca tggagcagga actggcccac 120

gccgtcaatg ccagctccaa gtccatggac cgtgtgtatg gcaagcccag aaccacagag 180

gggctcaact gcagcttcgt cctggaaatg gtgaataact tcagagctct gcgcagtgag 240

acggagctgc tgctgtctgg gaagatggcc ctgcagctgg atccgcctca taaggagcag 300

ctggggagtg ctcttgccga gatggaccga cagctcatga ggctggcaga caccccgtgg 360

ctctgccagt ccgcatagcc cggcgacgaa gagagtgtga tgctggatct tgccaagcgc 420

agtcgcagtg gtaaattccg cctcgtgacc aagtttaaaa aggagaaaaa catcaagaac 480

aaagaagctc acagtagcct gggagccccg gttcacctct gggggacaga ggaggttgct 540

gcctggctgg agcacctcag tctctgtgag tataaggaca tcttcacacg gcacgacatc 600

cg 602

<210> 165

<211> 365

<212> DNA

<213> Homo sapiens

<400> 165
gccctgttaa aagcctcgga agtggaagag attctggatg gcaacgatga gaagtacaag 60

gctgtgtcca tcagcacaga gcccccaccc tacctcaggg aacagaaggc caagaggaac 120

agccagtggg tacccaccct gcccaacagc tcccaccact tagatgccgt gccatgctcc 180

acaaccatca acaggaaccg catgggccga gacaagaaga gaaccttccc cctttgcttt 240

gatgaccatg acccagctgt gatccatgag aacgcatctc agcccgaggt gctggtcccc 300

atccggctgg acatggagat cgatgggcag aagctgcgag acgccttcac ctggaacatg 360

aatga 365

<210> 166

<211> 633

<212> DNA

<213> Homo sapiens

<400> 166
ggtcgccgcc ggggcgactc ggagtcagag gaagatgagc aggactcaga ggaggttcga 60

ttaaaactgg aagagaccag agaagtacag aacttgagga agaggcccaa cggggtgagt 120

gctgtggcct tgctggtggg agagaaggta caagaggaga ccactctagt ggatgatccc 180

tttcagatga agacaggtgg tatggtggat atgaagaaac tgaaggaaag gggcaaagat 240

aagatcagtg aggaggagga cctgcacctg gggacatcgt tttctgcaga aaccaaccga 300

agggatgagg atgcagacat gatgaagtac attgagacag agctaaagaa gaggaaaggg 360

atcgtggaac atgaggaaca gaaagttaag ccaaagaatg cagaggactg tctttatgaa 420

cttccagaaa acatccgtgt ttcctcagca aagaagaccg aggagatgct ttccaaccag 480

atgctgagtg gcattcctga ggtggacctg ggcatcgagt atgtttgaga cccctaggca 540

gaagacacag tgctttccag ttaaaagtta tgggggacag gccaggcacg gtggctcatg 600

cctgtaatcc tatcactttg ggaggctgag gcg 633

<210> 167

<211> 505

<212> DNA

<213> Homo sapiens

<400> 167
gtgatgaaag gcggctccaa ggagtactgg tttgtgctga ctgccgagtc actgtcctgg 60

tacaaggatg aggaggagaa agagaagaag tacatgctgc ctctggacaa cctcaagatc 120

cgtgatgtgg agaagggctt catgtccaac aagcacgtct tcgccatctt caacacggag 180

cagagaaacg tctacaagga cctgcggcag atcgagctgg cctgtgactc ccaggaagac 240

gtggacagct ggaaggcctc gttcctccga gctggcgtct accccgagaa ggaccaggca 300

gaaaacgagg atggggccca ggagaacacc ttctccatgg acccccaact ggagcggcag 360

gtggagacca ttcgcaacct ggtggactca tacgtggcca tcatcaacaa gtccatccgc 420

gacctcatgc caaagaccat catgcacctc atgatcaaca atacgaaggc cttcatccac 480

cacgagctgc tggcctacct atact 505

<210> 168

<211> 589

<212> DNA

<213> Homo sapiens

<400> 168
gagcgccccc aggagtgggc catggagggc ccccgcgacg ggctgaagaa ggagcggcta 60

ctggacgacc gccacgacag cggcctggac tccatgaaag acgaggagta cgagcagatg 120

gtcaaggagc tgcaggagat ccgcctcgag ccgcaggagg tgccgcgcgg ctcggagccc 180

tggaagcagc agctcaccga ggacggggac tcgttcctgc acttggccat catccatgaa 240

gaaaaggcac tgaccatgga agtgatccgc caggtgaagg gagacctggc tttcctcaac 300

ttccagaaca acctgcagca gactccactc cacttggctg tgatcaccaa ccagccagaa 360

attgctgagg cacttctggg agctggctgt gatcctgagc tccgagactt tcgaggaaat 420

acccccctac accttgcctg tgagcagggc tgcctggcca gcgtgggagt cctgactcag 480

tcctgcacca ccccgcacct ccactccatc ctgaaggcta ccaactacaa tggccacacg 540

tgtctacact tagcctctat ccatggctac ctgggcatcg tggagcttt 589

<210> 169

<211> 385

<212> DNA

<213> Homo sapiens

<400> 169
ggcgggtccc cggccacaac aggcttctcc acctccttgc gggggcccat gtggtcccgg 60

cggcggtccc ggcgacgcgc tgggcgcatc ggcggcgggt gtgggtgccg cgggcgtggt 120

ggtgggtgtg ggtggtgccg taggcgcagg ctacaacagt gaggacgagt atgaggcggt 180

tgcagcacgc atcgaggcta tggaccctgc cactgtcgag cagcaggagc attggtttga 240

aaaggcccta cgagacaaga agggcttcat catcaagcag atgaaggagg atggcgcctg 300

tctcttccgg gctgtagctg accaggtgta tggagaccag gacatgcatg aggttgtgcg 360

aaagcattgc atggactatc tgatg 385

<210> 170

<211> 675

<212> DNA

<213> Homo sapiens

<220>

<221>  misc_feature

<222>  (612)..(612)

<223>  n = a, c, g or t


<400>  170
   gggcggccct ggccggggag acgagttgca tgtgttggtt cagctggcga tagcggcggg      60
   agcggagccg gcggggcctg tgcgaccgcc tgggtttgtg aaatggctgc tgacatttct     120
   gaatccagcg gggctgactg caaaggagac ccaaggaaca gtgccaagtt agatgccgat     180
   tacccacttc gagtccttta ttgtggagtc tgttcattac caacagagta ctgtgaatat     240
   atgcctgatg ttgctaaatg tagacaatgg ttagagaaga attttccaaa tgaatttgca     300
   aaacttactg tagaaaattc acccaaacaa gaagctggaa ttagtgaggg tcaaggaaca     360
   gcaggggaag aagaggagaa gaaaaaacag aagagaggtg gaaggggtca aataaaacaa     420
   aaaaaagaag accgtaccac aaaaggttac tatagccaaa attcccagag caaagaagaa     480
   atatgtgaca agagtatgtg gccttgcaac ttttgaaatt gatcttaaag aagcacaaag     540
   attttttgct caaaaattct cctgtggtgc ctcagtaaca ggggaggatg aaattatcat     600
   tcagggagat tntacagatg acataattga tgtcattcag gaaaaatggc cagaggtaga     660
   tgatgacagc atcga                                                      675

<210>  171

<211>  559

<212>  DNA

<213>  Homo sapiens


<400>  171
gcccgccgtc cccacgcacg cgtcccggct cacgcgtccg cccgcccgcc cgcccccgct      60

tgtgccgccc ctaccagaga cccccaggag caggatgtcc ttccagggca agaaaagcat     120

cccccggatc acgagtgacc gccttctgat cagaggtggg aggatcgtga atgacgacca     180

gtccttttac gctgatgtgc acgtggaaga tggcttgata aaacaaatcg gagaaaacct     240

catcgtccct gggggcatca agaccattga cgcccacggc ctgatggtcc ttcctggtgg     300

cgttgacgtc cacacaaggc tgcagatgcc tgtcctgggc atgacaccgg ctgacgactt     360

ctgtcagggc accaaggcag cgctagcagg aggaaccacc atgatcttgg accacgtctt     420

ccccgacacg ggtgtgagcc tgctggcggc ctacgatcag tggcgggagc gggcggacag     480

cgcggcctgc tgcgactact ccctgcacgt ggacatcacc cgatggcatg agagcatcaa     540

ggaggagctg gaggccctg                                                  559

<210> 172

<211> 576

<212> DNA

<213> Homo sapiens

<400> 172
gcaggagaac catcacttaa atttggacct gaaggaggcg aagagctggc aagaggagca 60

gagtgctcag gctcatcgac tgaaagacaa ggtggcccat atgaaggaca ccctatgcca 120

ggcccatcag cgggtggccg agctggagcc cttgaaggag cagcttcgag gggcccagga 180

gcttgcagcc tcaagccagc agaaagccac ccttcttggg gaggagttgg ccagtgcagc 240

agcagccagg gaccgcacca tatccgaact acaccgcagc cgcctggaag tggctgaagt 300

taacggcagg ctggctgagc tcggtttgca cttgaaggaa gaaaaatgcc aatggagcaa 360

ggagcgggca gggctgctgc agagtgtgga ggcagagaag gacaagatcc tgaagctgag 420

tgcagagata cttcgattgg agaaggcagt tcaggaggag aggacccaaa accaagtgtt 480

caagactgag ctggcccggg agaaggattc tagcctggta cagttgtcag aaagtaagcg 540

ggagctgaca gagctgcggt cagccctgcg tgtgct 576

<210> 173

<211> 516

<212> DNA

<213> Homo sapiens

<400> 173
ggcggcggag gatgctgcga gagagaagcc cgtcgccccc aacaattcat agcaagcggc 60

agacgccttc accgagactg gcgctgtcta cccgctacag ccctgatgag atgaacaaca 120

gtcccaactt cgaagaaaag aagaagttcc tgaccatctt caacctgacc cacatcagcg 180

ctgagaagag gaaagacaaa gagagacttg ttgaaatgct ccgtgccatg aagcagaagg 240

cactgtcagc agcagtggcc gactccttga caaactctcc gagggacagt cctgccgtct 300

ccctgagtga accagccacg cagcaagcct ctctggatgt ggagaagccg gttggagttg 360

ctgcttcctt gtctgacatc ccaaaggccg cggagcctga gaagctggaa caggtccggc 420

cccaggagct gtcgagagtc caggagctag ctcctgccag cggggagaag gccaggctga 480

gcgaggcccc tggaggcaaa aagagtctga gcatgc 516

<210> 174

<211> 508

<212> DNA

<213>  Homo sapiens

<400>  174
gcagagccgc ctgcagtgag cgaaaccaca gagaggacag tgttaggaga gtacaatctc      60
ttttctagga agatagaaga gattttgaag caaaagaatg tttcatatgt cagtagagtt     120
tccacaccta tcttttcaac acaagagaag atgaaacggc tttccgagtt catatattct     180
aagacttcca aagctggtgt gcaggagttt gtagatggtt tgcatgagaa gctaaatact     240
attattatta aagcatcagc caagggtggg aatttgccac cagtcagtcc taacgattct     300
ggtgctaaga tagcatcgaa tcctctggaa aggcatgtca taccagtttc ctcaagtgac     360
ttcaacaata aacatctcct tgagccactg tgtagagatc ctttgaaaga taccaactct     420
gatgagcagc attccacttc ggctttaact gaagtagaaa tgaaccagcc tcaacatgcc     480
acagagttaa tggagacttc tgatcata                                        508

<210>  175
<211>  525
<212>  DNA
<213>  Homo sapiens

<400>  175
   gcagaaacgt ttgagcatct tttatcaagc catacagcat cagtgacatg tggtcctctt      60
   cttttggagc ctgaaacaat ttcagaagat atcagtgttg atacatcatg gaaaaataaa     120
   gatgagatga tgccaacaac tgtggtctct ctactttcaa caacagatct tgaaaagggt     180
   tctgtttgta ttagtgacca gttcaacaga gttaacttct ctgaggctga gggtactgag     240
   gtaacctatg aggacgaaag ccagagacaa ccctttgtta aatacgccac gctgatcagc     300
   aactctaaac caagtgaaac tggtgaagaa caagggctta taaatagttc agtcaccaag     360
   tgcttctcta gcaaaaattc tccgttgaaa gattctttct ctaatagctc atgggagata     420
   gaggcccagg cattttttat attatcagat cagcatccca acataatttc accacacctc     480
   acattctcag aaagattgga tgaacttttg aaattggagg gaaat                     525

<210>  176
<211>  487
<212>  DNA
<213>  Homo sapiens

<400>  176
gggcccggcc gcccgagcag gccgcgcgcg ggccgccggg cccgaggcca gagccatggg      60
cgagaccaag atcatctacc acttggatgg gcaggagacg ccgtaccttg tgaagctgcc     120

cctgcccgcc gagcgcgtca ccttggcgga ctttaagggc gttttgcagc gacccagcta 180
taagttcttc ttcaagtcta tggacgacga tttcggagtg gtgaaggagg agatctcgga 240
tgacaatgcc aagctaccat gcttcaatgg ccgggtggag tcctggctgg tgtcagctga 300
gggctcacac ccagacccag cccccttctg tgctgataac ccatcggagc tgccaccacc 360
tatggagcgc acgggaggca tcggggactc ccgaccccca tccttccacc ctcatgctgg 420
tgggggcagc caggagaacc tggacaatga cacagagacg gactctttgg tgtctgccca 480
gcgagag 487

<210> 177
<211> 519
<212> DNA
<213> Homo sapiens

<400> 177
gggatctggc tgaggaggaa gtggaggtgt cactggcccc ggcctttgcc ccaatcttgt 60
gtgggcactg aagggggact acaggttcga gatacttcct cgcgtattgc taaaggagga 120
gttgaccaca ccaaaatgag tctacatggt gctagtgggg gacatgagag atcaagatat 180
agacgaaggt caagtgacag atcacgagat tcatctcatg aaagaacgga gtctcagctc 240
actccttgta ttataaatgt gacttctcca acacgacagc accatgttga acgagaaaaa 300
gatcacagtt cctctcgtcc aagcagtccg cgtcctcaaa aagcatcccc aaatggttcc 360
attagcagtg ctgggaacag cagcagaaac agtagtcagt caagttcaga tggtagctgt 420
aagacagctg ggagatggtt gtttgtatat gaaaatgcaa aagaaggagc tcggaatata 480
agaacgtcag aacgagtgac actaatagtg gataacact 519

<210> 178
<211> 352
<212> DNA
<213> Homo sapiens

<400> 178
ggggtgcccg ggacgaagcc atggccctgg aggcggcggg agggccgccg gaggaaacgc 60
tgtcactgtg gaaacgggag caagctcggc tgaaggccca cgtcgtatac cgggacaccg 120
aggcgtggca gcgagacccc gccttctcgg gtctgcagag ggtcgggggc gttgacgtgt 180
ccttcgtgaa aggggacagt gtccgcgctt gtgcttccct ggtggtgctc agcttccctg 240
agctcgaggt ggtgtatgag gagagccgca tggtcagcct cacagccccc tacgtgtcgg 300
gcttcctggc cttccgagag gtgcccttct tgctggagct ggtgcagcag ct 352

<210> 179

<211> 527

<212> DNA

<213> Homo sapiens

<400> 179
gggctgcccc tgctgctggc tgctgaccgg ctgctgaccg gctgccacct gagtgtggat 60

gtggtcctgg gcgacgtggc tgtgacccat ggtcgcagct actgggcctg cgccgtagac 120

ccagcctcct acttggtcaa ggtgggcgtc gggctggaga gcaagcttca agaaagtttc 180

cagggtgccc ccgatgtgat cagccccagg tacgacccgg acagcgggca cgacagcggt 240

gccgaggatg ccacagtgga ggcgtcgcca cccttcgctt tcctaaccat tggcatgggc 300

aagatcctgc tggggtcggg ggcaagctca aacgcagggc tgacagggag ggatggcccc 360

acagccggct gcacagtgcc cctgccaccc cgcctgggca tctgcctgga ctatgagcgg 420

agccgggttt ccttcctgga tgctgtttcc ttccgtgggc tcttggagtg ccccctggac 480

tgctcagggc ctgtgtgccc tgccttttgc ttcatcgggg gtggcgc 527

<210> 180

<211> 451

<212> DNA

<213> Homo sapiens

<400> 180
ggggcagtgg agccgagtga aggggccccc agggctccac caggccctgc agggctcagt 60

ggtggggagt cgctgctggt gaaacagatc gaggagcaga taaagaggaa cgcggcaggc 120

aaagatggca aagagcgctt gggcggctca gtgctggggc agatcccctt cctgcatttc 180

tgcgaggacg aagacagcga cgaggacgat gagctggaca gcgtgcagca caagaagcag 240

cgtgtgaagc tcccatccaa ggctctggtg cccgaaatgg aagacgagga tgaggaagac 300

gactctgagg atgctatcaa tgagtttgat ttcctgggct caggagagga tggggaaggg 360

gctccagacc ctcggcggtg cactgtggat gggagccccc acgagctgga aagccgtcgg 420

gtcaaactcc aaggcattct ggctgacctg c 451

<210> 181

<211> 510

<212> DNA

<213> Homo sapiens

<400> 181
gcccacgcgt ccgtcaaaga cctaaatcag aagctccagt tagagctaga gaagcttcag 60

gctgactacg agaagctgaa gagcgaagaa cacgagaaga gcaccaagct gcaggagctg 120

acatttctgt acgagcgaca tgagcagtcc aagcaggacc tcaagggtct ggaggagaca 180

gttgcccggg aactccagac cctccacaac cttcgcaagc tgttcgttca agacgtcacg 240

actcgagtca agaaaagtgc agaaatggag cccgaagaca gtggggggat tcactcccaa 300

aagcagaaga tttcctttct tgagaacaac ctggaacagc ttacaaaggt tcacaaacag 360

ctggtacgtg acaatgcaga tctgcgttgt gagcttccta aattggaaaa acgacttatg 420

gctacggctg agagagttaa ggccctggag ggtgcactga aggaggccaa ggagggcgcc 480

atgaaggaca agcgccggta ccagcaggag 510

<210> 182

<211> 538

<212> DNA

<213> Homo sapiens

<400> 182
gctgagccgg cccatcggga gcagagcacc ggccaagcca cgaagtactc ggagctgctg 60

gagctcactg acggagtggt gagcgacatg gctgagactc gcactgctat cgtgcgtgcc 120

tcccgcctgc ccatgtccat catcatcgta agcgtgggca atgctgactt ctctgacatg 180

cggctgctgg atggcgacga cggccccttg cgctgccccc gaggggtgcc tgcagcccga 240

gacattgtcc agttcgagcc cttccgagac ttcaaggatg ctgccccctc tgcactcgcc 300

aagtgtgtcc tggctgaagt gccacggcag gtggtggagt actacgccag ccagggcatc 360

agccctgggg ctcccaggcc ctgcacactg gctacgactc ccagccctat cccgtgactg 420

cctccctccg gaccgacact ccctcagcct ctcagagcct gtcctgaccc tcgagactcc 480

agagaccaat gcctccacct cttggaccag gtgtgccccc tgggttctgg acgagaga 538

<210> 183

<211> 544

<212> DNA

<213> Homo sapiens

<400> 183
ggagacgggg gagcgggggg ctcgtctgtt ccaggagccc tgaaccaaag agcagcggag 60

tttgagaagc cagcagctcg gggttcggca gcagcggtcc catcggctga agttcggggg 120

gggtggggcg ccgagcgcgc ggggtggggg gggtcctggt ctttggcttc tcgactcggt 180

cctgtttcga cagcgaacat gtcgcggcct gtcagaaata ggaaggttgt tgattactca 240

cagtttcagg aatctgatga tgcagatgaa gattatggaa gagattcggg ccctcccact    300

aagaaaattc gatcatctcc ccgagaagct aaaaataaga ggcgatctgg aaagaattca    360

caggaagata gtgaggactc agaagacaaa gatgtgaaga ccaagaagga tgattctcac    420

tcagcagagg atagtgaaga tgaaaaagaa gatcataaaa atgtgcgcca acaacggcag    480

gcggcatcta aagcagcttc taaacagaga gagatgctca tggaagatgt gggcagtgag    540

gaag                                                                 544

<210>  184

<211>  529

<212>  DNA

<213>  Homo sapiens

<400>  184
gcccacgcgt ccgcccacgc gtccgcgaga ttctgctgcc agttcatcac ctccgttacc     60

gcgctctatg acctgtcatc agatgacctc attccaccta tggacttgct tgaaatgatt    120

gtcacctgga tttttgagga cccaaggttg attctcatca cttttttaaa tactccgata    180

gcggccaatc tgccaatagg attcttagag ctcaccccgc tcgttggatt gatccgctgg    240

tgcgtgaagg cacccctggc ttataaaagg aaaaagaagc cccccttatc caatggccat    300

gtcagcaaca aggtcacaaa ggacccgggc gtggggatgg acagagactc ccacctcttg    360

tactcaaaac tccacctcag cgttctgcaa gtgctcatga cgctgcagct gcacctgacc    420

gagaagaatc tgtatgggcg cctggggctg atcctcttcg accacatggt cccgctggta    480

gaggagatca acaggttggc ggatgaactg aaccccctca acgcctccc                529

<210>  185

<211>  576

<212>  DNA

<213>  Homo sapiens

<400>  185
ggccagcatg gcctgccgga agctggcggt ggcgcacccg ctgctgctgc tcaggcacct     60

gcccatgatc gcggcgctcc tgcacggccg cacccacctc aacttccagg agttccggca    120

gcagaaccac ctgagctgct tcctgcacgt gctgggcctg ctggagctgc tgcagccgca    180

cgtgttccgc agcgagcacc agggggcgct gtgggactgc cttctgtcct tcatccgcct    240

gctgctgaat tacaggaagt cctcccgcca tctggctgcc ttcatcaaca agtttgtgca    300

gttcatccat aagtacatta cctacaatgc cccagcagcc atctccttcc tgcagaagca    360

cgccgacccg ctccacgacc tgtccttcga caacagtgac ctggtgatgc tgaaatccct    420

ccttgcaggg ctcatcctgc ccagcaggga cgacaggacc gaccgaggcc tggacgaata    480

gggcgaggag gagagctcag ccggctcctt gcccctggtc agcgtctccc tgttcacccc 540

tctgaccgcg gccgagatgg ccccctacat gaaacg 576

<210> 186

<211> 464

<212> DNA

<213> Homo sapiens

<400> 186
ggtggacctg gtcaacgcca gcttcggcaa gaagctcagg gacgactacc tggcctcgct 60

gcgcccgcgg ctgcactcca tctacgtctc cgaggggtac aacgccgccg ccattctgac 120

catggagccc gtcctggggg gcaccccgta cctggacaaa tttgtggtga gctccagccg 180

ccagggccaa ggctccggcc agatgctgtg ggagtgcctg cggcgggacc ttcagacact 240

tttctggcgc tctcgggtca ccaaccccat caatccctgg tacttcaaac acagtgatgg 300

cagcttctcc aacaagcagt ggatcttctt ctggtttggc ctggctgata tccgggactc 360

ctatgagttg gtcaaccacg ccaagggact gccagactcc tttcacaagc cagcttctga 420

cccatgcagc tgaccctcac catggacact acaggccctg gaat 464

<210> 187

<211> 561

<212> DNA

<213> Homo sapiens

<400> 187
gcggacgcgt gggcggacgc gtgggcggac gcgtggggtg gccgctcacg gtcccgccgc 60

cgctgctgct gctgctgtgc tcaggcctgg ccggacagac tctcttccag aacccagaag 120

agggctggca gctgtacacc tcatcccagg cccctgacgg gaaatgcatc tgcacggccg 180

tgatcccagc gcagagtacc tgctctcgag atggcaggag tcgggagctg cggcaactga 240

tggagaaggt ccagaacgtc tcccagtcca tggaggtcct tgagttgcgg acgtatcgcg 300

acctccagta tgtacgcggc atggagaccc tcatgcggag cctggatgcg cagctccggg 360

cagctgatgg gtccctctcg gccaagagct tccaggagct gaaggacagg atgatggaac 420

tgttgcccct gagctcggtc ctggagcagt acaaggcaga cacgcggacc attgtacgct 480

tgcgggagga ggtgaggaat ctctccggca gtctggcggc cattcatgag gagatgggtg 540

cctacgggta tgaggacctg c 561

<210> 188

<211> 577

<212>  DNA

<213>  Homo sapiens


<400>  188
ggccaaatcc aagccggcag tgagccgcga caaggcctca tacgccagcg acaagcagcg      60

ccaggaggcc cgcaagagac tcctggcggc caagcgggca gcttctgtgc ggcagaactc     120

agccaccgag agcgcagaca gcatcgagat ttatgtcccg gaggcccaga ccaggctctg     180

agaccatgca ggaggaaaga aacgatttta aatcattaaa aacacaaaaa ctaagtgcga     240

acggaacaga gttttctcaa cctttgctat ggttattctg tctagagacc ctgagccaac     300

tttcaaattg acgcatacaa gggctcacaa tttggctttt ttgggtccct cccagcttta     360

ggttatgaag attttactca caaaaaaaat caacaaaaat cacgaaacta gaaaactttt     420

tttttcctct tgctggccgt ggtggactag atagatggac gtcggcaact cccggcccag     480

cctccatact gcggtctttt tactcgttct atctgatgag aactcacact agcttgttta     540

caagatgacg acagtccaag ggcagccttg ggcacct                              577

<210>  189

<211>  615

<212>  DNA

<213>  Homo sapiens


<400>  189
ggtcgcgcgt ttgcgagcct cgggtggtcc tcagggaggg tctctcggcc agaacacgtg      60

gatgcccacc caccactgag cctcatggag gtggtaacat ttggcgatgt ggctgtgcac     120

ttctctcggg aggagtggca gtgtctggac cctggccaga gggccctcta cagggaagtg     180

atgctggaga accacagcag tgtggctgga ctagcaggat tcctggtttt caagcctgag     240

ctgatctctc ggctggagca gggagaagag ccatgggtcc tcgacctgca gggagcagag     300

gggacagagg caccaaggac ctccaagaca gattctacga ttaggactga aaatgagcag     360

gcctgtgagg acatggacat cctaaaatca gaatcctatg ggacagtggt cagaatctcc     420

ccacaggact ttcctcataa tcctggcttt ggagacgttt ctgattctga ggtctggtta     480

gacagtcatc tgggcagtcc cgggctgaaa gtgacaggct ttaccttcca aaataactgt     540

ttgaatgagg agactgtggt tcccaagacc ttcaccaagg acgcacccca gggatgtaag     600

gagctgggaa gcagc                                                      615

<210>  190

<211>  605

<212>  DNA

<213>  Homo sapiens

<400>  190
gagaaaagca acacagtcca gatagtcgta tgtgaaatgc tctcccagcc caggatttct      60
tatgatgccc agtttgaggt cataaaagga cagaccatcg aagtccgttg cgaatcgatc     120
agtggaactt tgcctatttc ttaccaactt ttaaaaacaa gtaaagtttt ggagaatagt     180
accaagaact ctcaatgatc ctgcggtatt caaagacaac cccactgaag acgtcgaata     240
ccagtgtgtt gcagataatt gccattccca cgccaaaatg ttaagtgagg ttctgagggt     300
gaaggtgata gccccggtgg atgaggtcca gatttctatc ctgtcaagta aggtggtgga     360
gtctggagag gacattgtgc tgcaatgtgc tgtgaatgaa ggatctggtc ccatcaccta     420
taagttttac agagaaaaag agggcaaacc cttctatcaa atgacctcaa atgccaccca     480
ggcattttgg accaagcaga aggctaacaa ggaacaggag ggagagtatt actgcacagc     540
cttcaacaga gccaaccacg cctccagtgt ccccagatgc aaaatactga cagtcagagt     600
cattc                                                                 605

<210>  191
<211>  640
<212>  DNA
<213>  Homo sapiens

<400>  191
ggcaacatgg cgtccaggtc taagcggcgt gccgtggaaa gtggggttcc gcagccgccg      60
gatcccccag tccagcgcga cgaggaagag gaaaaagaag tcgaaaatga ggatgaagac     120
gatgatgaca gtgacaagga aaaggatgaa gaggacgagg tcattgacga ggaagtgaat     180
attgaatttg aagcttattc cctatcagat aatgattatg acggaattaa gaaattactg     240
cagcagcttt ttctaaaggc tcctgtgaac actgcagaac taacagatct cttaattcaa     300
cagaaccata ttgggagtgt gattaagcaa acggatgttt cagaagacag caatgatgat     360
atggatgaag atgaggtttt tggtttcata agccttttaa atttaactga aagaaagggt     420
acccagtgtg ttgaacaaat tcaagagttg gttctacgct tctgtgagaa gaactgtgaa     480
aagagcatgg ttgaacagct ggacaagttt ttaaatgaca ccaccaagcc tgtgggcctt     540
ctcctaagtg aaagattcat taatgtccct ccacagatcg ctctgcccat gtaccagcag     600
cttcagaaag atctggcggg ggcacacaga accaataagc                           640

<210>  192
<211>  539
<212>  DNA
<213>  Homo sapiens

<400>  192
gcagcggctg acgggccgga tcgccttcgc cgccgcccgc ccgcaaacct tcgtgcccgg      60

cccgtcctcg cccccgcctc cgccaccgcc tcggcccgca gagcttgccc cctccccacc     120

cgcagacatg tccgagtcca agagcggccc cgagtatgct tcgtttttcg ccgtcatggg     180

cgcctcggcc gccatggtct tcagcgccct gggcgctgcc tatggcacag ccaagagcgg     240

taccggcatt gcggccatgt ctgtcatgcg gccggagcag atcatgaagt ccatcatccc     300

agaggtcatg gctggcatca tcgccatcta cggcctggtg gaggcagtcc tcatcgccaa     360

ctccctgaat gacgacatca gcctctacaa gagcttcctc cagctgggcg ccggcctgag     420

cgtgggcctg agcggcctgg cagccggctt tgccatcggc atcgtggggg acgctggcgt     480

gcggggcacc gcccagcagc cccgactatt cgagggcatg atcctgattc tcatcttcg      539

<210>  193

<211>  504

<212>  DNA

<213>  Homo sapiens


<400>  193
gcccacgcgt ccgcccacgc gtccgcccac gcgtccgcgg acgcgtgggc cgagcttctg      60

gcggtctcaa gcactaccta cgtcagcacc tgggaccccg ccaccgtgcg ccgggccttg     120

cagtgggcgc gctacctgcg ccacatccat cggcgctttg gtcggcatgg ccccattcgc     180

acggctctgg agcggcggct gcacaaccag tggaggcaag agggcggctt tgggcggggt     240

ccagttccgg gattagcgaa cttccaggcc ctcggtcact gtgacgtcct gctctctctg     300

cgcctgctgg agaaccgggc cctcggggat gcagctcgtt accacctggt gcagcaactc     360

tttcccggcc cgggcgtccg ggacgccgat gaggagacac tccaagagag cctggcccgc     420

cttgcccgcc ggcggtctgc ggtgcacatg ctgcgcttca atggctatag agagaaccca     480

aatctccagg aggactctct gatg                                            504

<210>  194

<211>  529

<212>  DNA

<213>  Homo sapiens


<400>  194
ggaaggaggc gaactgtggg ccccggcccc attcattgcc gtggccggcg ggcactgggg      60

ccccgtgttt tcagagtcat ggaggcgcta attcctgtca taaacaagct ccaggacgtc     120

ttcaacacgg tgggcgccga catcatccag ctgcctcaaa tcgtcgtagt gggaacgcat     180

agcagcggaa agagctcagt gctagaaagc ctggtgggga gggacctgct tcccagaggt 240

actggaattg tcacccggag acctctcatt ctgcaactgg tccatgtttc acaagaagat 300

aaacggaaaa catcaggaga agaaaatggg gtggaagcag aagaatgggg taaatttctt 360

cacaccaaaa ataagcttta cacggatttt gatgaaattc gacaagaaat tgaaaatgaa 420

acagaaagaa tttcaggaaa taataaggga gtaagccctg aaccaattca tcttaagatt 480

ttttcaccca acgttgtcaa tttgacactt gtggatttgc caggaatga 529

<210> 195

<211> 583

<212> DNA

<213> Homo sapiens

<400> 195
ggtggtctcc atcacctgcc tcaagcacaa gtcggggggt cacgctgtcc aactcctgag 60

ggccgtgtcc ctaggccagg tggtcatcac caagaaccgc aacgggctgt actaccgctg 120

tcgcgtcatc ggtgccgcct cgcagacctg ctacgaagtg aacttcgacg atggctccta 180

cagcgacaac ctgtaccctg agagcatcac gagtagggac tgtgtccagc tgggaccccc 240

ttccgagggg gagctggtgg agctccggtg gactgacggc aacctctaca aggccaagtt 300

catctcctcc gtcaccagcc acatctacca ggtggagttt gaggacgggt cccagctgac 360

ggtgaagcgt ggggacatct tcaccctgga ggaggagctg cccaagaggg tccgctctcg 420

gctgtcactg agcacggagg caccgcagga gcccgccttc tcgggggagg aggccaaggc 480

cgccaagcgc ccgcgtgtgg gcaccccgct tgccacggag gactccgggc ggagccagga 540

ctacgtggcc ttcgtggaga gcctcctgca ggtgcagggc cgg 583

<210> 196

<211> 442

<212> DNA

<213> Homo sapiens

<400> 196
gatcagagcg tggccaccga tggcccaagc agcacccatc ctgccctgcc aaaggccaga 60

gccgtggctc ccaaacccag cagccggggc gagtatgtgg tggccaagct ggatgacctc 120

gtcaactggg cccgccggag ttctctgtgg cccatgacct tcggcctggc ctgctgcgcc 180

gtggagatga tgcacatggc agcaccccgc tacgacatgg accgctttgg cgtggtcttc 240

cgcgccagcc cgcgccagtc cgacgtcatg atcgtggccg gcacactcac caacaagatg 300

gccccagcgc ttcgcaaggt ctacgaccag atgccggagc cgcgctacgt ggtctccatg 360

gggagctgcg ccaacggagg aggctactac cactattcct actcggtggt gaggggctgc 420

gaccgcatcg tgcccgtgga ca 442

<210> 197

<211> 559

<212> DNA

<213> Homo sapiens

<400> 197
gggctgccga agatggcgga ggtgcaggtc ctggtgcttg atggtcgagg ccatctcctg 60

ggccgcctgg cggccatcgt ggctaaacag gtactgctgg gccggaaggt ggtggtcgta 120

cgctgtgaag gcatcaacat ttctggcaat ttctacagaa acaagttgaa gtacctggct 180

ttcctccgca agcggatgaa caccaaccct tcccgaggcc cctaccactt ccgggccccc 240

agccgcatct tctggcggac cgtgcgaggt atgctgcccc acaaaaccaa gcgaggccag 300

gccgctctgg accgtctcaa ggtgtttgac ggcatcccac cgccctacga caagaaaaag 360

cggatggtgg ttcctgctgc cctcaaggtc gtgcgtctga agcctacaag aaagtttgcc 420

tatctggggc gcctggctca cgaggttggc tggaagtacc aggcagtgac agccacccct 480

ggaggagaag aggaaagaga aagccaagat ccactaccgg aagaagaaac agctcatgat 540

gctacggaaa caggccgag 559

<210> 198

<211> 709

<212> DNA

<213> Homo sapiens

<400> 198
gggagcagct ggagaaagcc aaggcctatg cagccaacag ccaccagggg cagatgctgg 60

cccagtatat agagagcttc acccagggct ccatcgaggc ccacaagagg ggctcccgct 120

tctggatcca ggacaaaggc cccatcgtgg agagttacat cgggttcatc gagagctacc 180

gcgacccctt tggttcccga ggagaatttg aaggtttcgt agctgtggtg aacaaggcca 240

tgagtgccaa gtttgagcgg ctggtggcga gcgcagagca gctgctgaag gagctgccct 300

ggcccccaac ctttgagaag gacaagttcc tcacccctga cttcacctcc ctggatgttc 360

tcaccttcgc tggctccggc atccctgccg gcatcaacat ccccaactac gatgatctga 420

ggcagacgga aggctttaag aacgtgtcgc tggggaatgt gctggctgtg gcctacgcca 480

cgcagcggga gaagcttacc tttctggagg aggatgacaa ggacctgtac atcctctgga 540

aggggccctc cttcgatgtg caggtgggcc tgcacgagct gctgggccat ggcagtggca 600

agctcttcgt acaggacgaa aaaggagcat tcaactttga ccaggaaaca gtgatcaacc 660

cagagacggg cgagcaggtg agggaggcct cagcagagcc ccagggtac 709

<210> 199

<211> 521

<212> DNA

<213> Homo sapiens

<400> 199
gcccacgcgt ccgctgagct gaagaaccgg atcttctcgg ggggtctgag ccccagcctg 60

cggcgcgagg cctggaagtt cctcctaggg tacctcagct gggaaggcac agctgaggag 120

cacaaggccc acatacgcaa gaaaacggat gagtatttcc gcatgaagct gcagtggaaa 180

tctgtgagcc ctgagcagga gcggagaaac tcacttctgc atggataccg cagcctcatc 240

gaaagggatg tgagccgcac tgacaggacc aacaagttct acgagggtcc cgagaacccg 300

gggctgggcc tgctgaacga tatcctcctc acctactgca tgtatcactt cgacctcggc 360

tacgtccagg gcatgagtga tcttctctcc ccgatcctct acgtcattca gaacgaggtg 420

gatgctttct ggagtttctg tggcttcatg gagctcgtgc aagggaactt tgaagagagc 480

caggagacca tgaagcggca actcgggcga ctgctgctgc t 521

<210> 200

<211> 475

<212> DNA

<213> Homo sapiens

<400> 200
gacggcggcg accccaccgc gcatcctgcc aggcctccgg cgcccagcgc cccacgcgcc 60

cccgagcccc cgcgcccccg cgcccctttc ttcgcgcccc cgcccctcgg cccgccaggc 120

ccccttgccg gccacccgcc aggccccgcg ccggcccgcc cgccgcccag gaccggcccg 180

cgccccgcag gccgcccgcc gcccgcgccg ccatgggagt ggagggctgc accaagtgca 240

tcaagtacct gctcttcgtc ttcaatttcg tcttctggct ggctggaggc gtgatcctgg 300

gtgtggccct gtggctccgc catgacccgc agaccaccaa cctcctgtat ctggagctgg 360

gagacaagcc cgcgcccaac accttctatg taggcatcta catcctcatc gctgagggcg 420

ctgtcatgat gttcgtaggc ttcctgggct gctacggggc catccaggaa tccca 475

<210> 201

<211> 443

<212> DNA

<213> Homo sapiens

<400>  201
gggatgtttc ctcagcggcc ccgagcccgc ggggctgtgc ggatggtagg gatgccgacc      60

ctactgagga gcagatggca gaaacagaga gaaacgacga ggagcagttc gaatgccagg     120

aactgctcga gtgccaggtg caggtggggg cccccgagga ggaggaggag gaggaggagg     180

acgcgggcct ggtggccgag gccgaggccg tggctgccgg ctggatgctc gatttcctct     240

gcctctctct ttgccgagct ttccgcgacg gccgctccga ggacttccgc aggacccgca     300

acagcgcaga ggctattatt catggactat ccagtctaac agcttgccag ttgagaacga     360

tatacatatg tcagtttttg acaagaattg cagcaggaaa aacccttgat gcacaggttg     420

aaaatgatga acgaattaca ccc                                             443

<210>  202

<211>  622

<212>  DNA

<213>  Homo sapiens


<400>  202
ggcgtgcagc tccccacagc ggcccgctgg tgtcctggcg caggtggtcc ggcgcctcaa      60

cagacaccgt gtatgacgtg gtggtgtcgg gtggaggcct ggtgggcgct gccatggcct     120

gtgccttggg atatgatatt cactttcatg acaagaaaat cctgttgctc gaagcaggtc     180

caaagaaagt actggagaaa ttgtcagaaa cttacagcaa cagggtcagc tccatttccc     240

ctggctctgc aacgcttctc agtagttttg gtgcctggga ccatatctgc aacatgagat     300

acagagcctt tcggcgaatg caggtgtggg acgcctgctc agaggccctg ataatgtttg     360

ataaggataa tttatatgac atgggctata tcgtggagaa tgatgtcatc atgcatgctc     420

tcactaagca gttggaggct gtgtctgacc gagtgacggt tctctacagg agcaaagcca     480

ttcgctatac ctggccttgt ccatttccta tggccgactc cagcccttgg gttcatatta     540

ccctatgtga tggcagcacc ttccagacca aattgttgat aggtgcagat ggtcacaact     600

ccggagtacg gcaggctgtt gg                                              622

<210>  203

<211>  589

<212>  DNA

<213>  Homo sapiens


<400>  203
gggcgagggg cgccgggaac tggcgtgtgg gactccagac aggagaggct gcgccttccc      60

cgcaccggga ccttcgcgac acaccagatc ctcgcccctg gctcgcgcga acgcacagga     120

tgaccaccac cctcgtgtct gccaccatct tcgacttgag cgaagtttta tgcaagggta 180

acaagatgct caactatagt gctcccagtg cagggggttg cctgctggac agaaaggcag 240

tgggcacccc tgctggtggg ggcttccctc ggaggcactc agtcaccctg cccagctcca 300

agttccacca gaaccagctc ctcagcagcc tcaagggtga gccagccccc gctctgagct 360

cgcgagacag ccgcttccga gaccgctcct tctcggaagg gggcgagcgg ctgctgccca 420

cccagaagca gcccggaggc ggccaggtca actccagccg ctacaagacg gagctgtgcc 480

gcccctttga ggaaaacggt gcctgtaagt acggagacaa gtgccagttc gcacacggca 540

tccacgagct ccgcagcctg acccgccacc ccaagtacaa gacggagct 589

<210> 204

<211> 552

<212> DNA

<213> Homo sapiens

<400> 204
ggtgctgcca aactgctgag gtctgagtgg acaggtgcct cattagctta ctattgccat 60

ccccctacat cttggactcc tcctccatat tttaatgaga gtattgtggt agacatgaaa 120

agcggcttca atctggaaga actggaaaag aacaatgcac agagcataag agccatcaag 180

ggccctcatt tggccaatag catccttttc cagtcttccg gtctgacaaa tggaataata 240

gaagaaaagg acatacatga agaattgcca aaacggaaag aaaggaagca ggaggagagg 300

gaggatgaca aagacagtga ccaggaaact gttgatgaag aagttgatga aaacagctca 360

tgcatgtttg ctgcagaaga gacaggggag gcactgtctg aggagactac agcaggtgaa 420

cagtctacaa ggtcttttat cttggataaa atcattgaag aggatgatgc ttatgacttc 480

agtacagatt atgtgtaaca gaacaatggc tttttatgat tcttttttta acattttaag 540

cagactgcta aa 552

<210> 205

<211> 574

<212> DNA

<213> Homo sapiens

<400> 205
ggccgctgct gccgccgccg ccgcccagag actgctgagc ccgtccgtcc gccgccacca 60

cccactccgg acacagaaca tccagtcatg gataaaaatg agctggttca gaaggccaaa 120

ctggccgagc aggctgagcg atatgatgac atggcagcct gcatgaagtc tgtaactgag 180

caaggagctg aattatccaa tgaggagagg aatcttctct cagttgctta taaaaatgtt 240

gtaggagccc gtaggtcatc ttggagggtc gtctcaagta ttgaacaaaa gacggaaggt 300
gctgagaaaa aacagcagat ggctcgagaa tacagagaga aaattgagac ggagctaaga 360
gagcaaagtc ttctatttga aaatgaaagg agattactac cgttacttgg ctgaggttgc 420
cgctggtgat gacaagaaag ggattgtcga tcagtcacaa caagcatacc aagaagcttt 480
tgaaatcagc aaaaggaaat gcaaccaaca catcctatca gactgggtct ggcccttaac 540
ttctctgtgt tctattatga gattctgaac tccc 574

<210> 206
<211> 632
<212> DNA
<213> Homo sapiens

<400> 206
gccatcacat gggcccgatg tcagagaggc ggcatgagca gagcggcggg cctgagcatg 60
ggcccgagag ggggcctttc cggggtggcc aggactgcag gggtccccct gataggcgtg 120
gccctcaccc agacttcccc gatgacttca gcagaccaga tgacttccac cctgacaagc 180
gctttggcca ccggttacgt gaatttgagg ggcgaggagg acctttaccg caagaagaga 240
agtggaggcg agggggccct gggcctccgt ttccccctga tcacagggaa ttcagcgagg 300
gggatggccg gggtgcagcc cgaggccctc cgggggcatg ggaaggccgc aggcccggag 360
atgaacgttt cccccgggat cccgaggacc cacgttttcg agggcgcaga gaagaaagtt 420
tcagaagagg agccccgccg aggcatgagg gccgtgctcc ccccagagga agggatggtt 480
ttcctggtcc tgaagacttt ggtccagagg agaattttga tgcttctgag gaagcggccc 540
gaggacgaga tctcagaggt cgaggtcggg gtaccccacg aggaggaagg gaagggttta 600
ctttccactc ctgacgagtt ccctcgcttt ga 632

<210> 207
<211> 669
<212> DNA
<213> Homo sapiens

<400> 207
ggcgccgccg acgcccgccc tcccgcagag ccctccgccg acctcttggt gaacctgagc 60
cgctgccgac gcctcatcgt ggtgctttcg gacgccttcc tgagccgggc ctggtgcagc 120
cacagcttcc gggagggcct gtgccggctg ctggagctca cccgcagacc catcttcatc 180
accttcgagg gccagaggcg cgaccccgcg cacccggcgc tccgcctgct gcgccagcac 240
cgccacctgg tgaccttgct gctctggagg cccggctccg tgactccttc ctccgatttt 300
tggaaagaag tgcagctggc gctgccgcgg aaggtgcggt acaggccggt ggaaggagac 360

ccccagacgc agctgcagga cgacaaggac cccatgctga ttcttcgagg ccgagtccct 420
gagggccggg ccctggactc agaggtggac ccggaccctg agggcgacct gagtgtccgg 480
gggcctgttt ttggagagcc atcagctcca ccgcacacca gtggggtctc gctgggagag 540
agccggagca gcgaagtgga cgtctcggat ctcggctcgc gaaactacag tgcccgcaca 600
gacttctact gcctggtgtc caaggatgat atgtagctcc caccccagag tgcaggatca 660
tagggacag 669

<210> 208
<211> 569
<212> DNA
<213> Homo sapiens

<400> 208
gctcggtctc agctctgggc gagcagcggc ctgtctgaag agcatgcagc cccctactc 60
cccgcctggc ctcgccatcc ctgccccccc agcctgaccc ccggccccag catggcccag 120
ggtgccatgc gcttctgctc ggaaggcgac tgtgccatct ccccaccacg atgcccacgc 180
cgctggctcc ccgaaggccc ggtgccccag agccccccag ccagcatgta tggcagcaca 240
ggctccttgc tacggcgagt ggcaggtcca ggtcctcgag gccgggaact gggacgtgtg 300
acagcaccct gtacacctct gcgtggcccc ccctcacccc gtgttgctcc ctcaccctgg 360
gcaccctctt cacccactgg gcagccccca cccggggccc agagctctgt ggtcatcttc 420
cgctttgtgg agaaggccag tgtgaggcca ctgaatgggc tacctgctcc agggggcttg 480
agtcggagct gggatctggg tggggtttct cctcccagcc caccccagcc cttgagcctg 540
gctccaaccg gaagttacgg ctggaagca 569

<210> 209
<211> 658
<212> DNA
<213> Homo sapiens

<400> 209
ggtcccaggg gtgtacacat tcccaactgt gacaagaagg gattttataa gaaaaagcag 60
agtcgccctt ccaaaggcag gaagcggggc ttctgctggt gtgtggataa gtatgggcag 120
cctctcccag gctacaccac caaggggaag gaggacgtgc actgctacag catgcagagc 180
aagtagacgc ctgccgcaag gttaatgtgg agctcaaata tgccttattt tgcacaaaag 240
actgccaagg acatgaccag cagctggcta cagcctcgat ttatatttct gtttgaggag 300
aactgatttt ttttaaacca aagtttataa agaggttttt gaaatgccta tggtttcttt 360

gaatggtaaa cttgagcatc ttttcacttt ccagtagtca gcaaagagca gtttgaattt 420
tcttgtcgct tcctatcaaa atattcagag actcgagcac agcacccaga cttcatgcgc 480
ccgtggaatg ctcaccacat gttggtcgaa gcggccgacc actgactttg tgacttatgc 540
ggctgtgttg cctatgtaga gaacacgctt cacccccact ccccgtacag tgcgcacagg 600
ctttatcgag aataggaaaa cctttaaacc ccggtcatcc ggacatccca acgcatgc 658

<210> 210
<211> 605
<212> DNA
<213> Homo sapiens

<400> 210
gtagagatct accaggagaa ggtattagac ctcctggacc ctgcttcggg agacctggta 60
atccgagaag actgccgggg gaatatcctg attccgggtc tctcccagaa gcccatcagt 120
agctttgctg attttgagcg gcacttcctg ccagccagtc gaaatcggac tgtaggagcc 180
acccggctca accagcgctc ctcccgcagt catgctgtgc tcctggtcaa ggtggaccag 240
cgggaacgtt tggccccatt tcgccagcga gagggaaaac tctacctgat tgacttggct 300
gggtcagagg acaaccggcg cacaggcaac aagggccttc ggctaaaaga gagtggagcc 360
atcaacacct ccctgtttgt cctgggcaaa gtggtagatg cgctgaatca gggcctccct 420
cgtgtacctt atcgggacag caagctcact cgcctattgc aggactctct gggtggctca 480
tcccacagta tccttattgc caacattgcc cctgagagac gcttctacct agacacagtc 540
tccgcactca actttgctgc caggtccaag gaggtgatca atcggccttt taccaatgag 600
agcct 605

<210> 211
<211> 438
<212> DNA
<213> Homo sapiens

<400> 211
ggcccaacct ggatgtggag caggccgagg tcaacttcgg ctggaaccac ctggagccct 60
atgcccattt cctgctctct gtcttcttcg tcatcttctc cttccccatc gccagcaagg 120
actgcatccc ctgctcggag ctggctgtca tcaccggctt ctttaccgtg accagctacc 180
tgagcctgag cacccatgca gagccctaca cgcgcagggc cctggccacc gaggtcaccg 240
ccggcctgct atcgctgctg ccctccatgc ccttgaattg gccctacctg aaggtccttg 300
gccagacctt catcaccgtg cctgtcggcc acctggtcgt cctcaacgtc agcgtcccgt 360
gcctgctcta tgtctacctg ctctatctct tcttccgcat ggcacagctg aggaatttca 420

agggcaccta ctgctacc                                                    438

<210>  212

<211>  520

<212>  DNA

<213>  Homo sapiens


<400>  212
gtgagccctg gcagaccgac tgcaggtagc aggattgcag gaccctctgc ctggcctggc      60

gtttcaggag agaggggaag tggggcctgt gctctgggag gcgtggtcat ccgagacagg     120

agtccagggg agagaggagg ggacaaaggc gccgtctggg ggaggtcgat gagcctgtgc     180

tggcatccgc gggccccacg ctttgccaac tcctccagcc acaggcaagg ccacggctcc     240

gggctgttgc gctctaaggg ttctgtgatt ggatggaaca gagctgctgg ggaggagact     300

ggaagtttct gcattccttc aacagaacat ttaatgaagt actctatata tatatataaa     360

tatatatata aatatatata tatacttcta tttgtgggta ctttaggaaa atgccctttg     420

gtcactgtaa atatgaattg tgaccccatc ccttcccgca tgagcccagt gagtcccagc     480

agctatcagc ctccctgaac gattaaacag ctcctcccag                           520

<210>  213

<211>  262

<212>  DNA

<213>  Homo sapiens


<400>  213
gcccacgcgt ccgagcggag gttctgcttc gaggagctgt cacccaccaa gagctgcatg      60

ctgcaggctg actccgagaa gctgcggcaa gcctgggtcc aggctgagca ggccagcatc     120

gcctccgcct accgcgagag ccctgacagt tgctatagcg agaggctgga ccgcacagca     180

tccccgtcca cgagcagcat cgactccgcc accgacactc gggagcgtgg cgtgaagggc     240

gagagagtgc tgcagcgtgt gc                                              262

<210>  214

<211>  420

<212>  DNA

<213>  Homo sapiens


<400>  214
gggttctgtg aggtagcggc ggcaacgacc atggaggcca cgtcccggga ggcggcgcca      60

gcgaagagct cggcctcggg ccccaacgct ccccccgccc tgttcgagct gtgcgggcgg 120

gcggtgagcg cccatatggg ggttctggag agcggggtgt gggccctccc aggcccaata 180

cttcaaagca tcctacctct gctcaatata tattacttgg agaggattga ggaaactgcc 240

ctcaagaaag gcctctcaac tcaggccatc tggcgccgac tctgggatga actgatgaag 300

acaaggcctt ccagtttgga aagtgtgaca tgttggcgag ccaagtttat ggaggccttt 360

ttttcccatg ttctacgtgg gaccattgat gtgtcttctg acaggcgtct ttgtgatcag 420

<210> 215

<211> 475

<212> DNA

<213> Homo sapiens

<400> 215
gcttcatcga gcgccatggc cgcagcctgc gggccgggag cggccgggta ctgcttgctc 60

ctcggcttgc atttgtttct gctgaccgcg ggccctgccc tgggctggaa cgaccctgac 120

agaatgttgc tgcgggatgt aaaagctctt accctccact atgaccgcta taccacctcc 180

cgcaggctgg atcccatccc acagttgaaa tgtgttggag gcacagctgg ttgtgattct 240

tataccccaa aagtcataca gtgtcagaac aaaggctggg atgggtatga tgtacagtgg 300

gaatgtaaga cggacttaga tattgcatac aaatttggaa aaactgtggt gagctgtgaa 360

ggctatgagt cctctgaaga ccagtatgta ctaagaggtt cttgaggctt ggagtataat 420

ttagattata cagaacttgg cctgcagaaa ctgaaggagt ctggaaagca gcacg 475

<210> 216

<211> 598

<212> DNA

<213> Homo sapiens

<400> 216
gtggcggaga agctgctaca gctgcagaac ttcaacacgc tgatggcagt ggtcgggggc 60

ctgagccaca gctccatctc ccgcctcaag gagacccaca gccacgttag ccctgagacc 120

atcaagctct gggagggtct cacggaacta gtgacggcga caggcaacta tggcaactac 180

cggcgtcggc tggcagcctg tgtgggcttc cgcttcccga tcctgggtgt gcacctcaag 240

gacctggtgg ccctgcagct ggcactgcct gactggctgg acccagcccg gacccggctc 300

aacggggcca agatgaagca gctctttagc atcctggagg agctggccat ggtgaccagc 360

ctgcggccac cagtacaggc caaccccgac ctgctgagcc tgctcacggt gtctctggat 420

cagtatcaga cggaggatga gctgtaccag ctgtccctgc agcgggagcc gcgctccaag 480

tcctcgccaa ccagccccac gagttgcacc ccaccacccc ggcccccggt actggaggag 540

tggacctcgg ctgccaaacc caagctggat caggccctcg tggtggagca catcgaga 598

<210> 217

<211> 522

<212> DNA

<213> Homo sapiens

<400> 217
ggtgtctctg gggcggaggc agcggcagtg gagttcgctg cgcgctgttg ggggccacct 60

gtcttttcgc ttgtgtccct ctttctagtg tcgcgctcga gtcccgacgg gccgctccaa 120

gcctcgacat gtcgtacaac tacgtggtaa cggcccataa gcccaccgcc gtgaacggct 180

gcgtgaccgg acactttact tcggccgaag acttaaacct gttgattgcc aaaaacacga 240

gattagagat ctatgtggtc accgccgagg ggcttcggcc cgtcaaagag gtgggcatgt 300

atgggaagat tgcggtcatg gagcttttca ggcccaaggg ggagagcaag gacctgctgt 360

ttatcttgac agcgaagtac aatgcctgca tcctggagta taaacagagt ggcgagagca 420

ttgacatcat tacgcgagcc catggcaatg tccaggaccg cattggccgc ccctcataga 480

ccggcattat tggcatcatt gaccctgagt gccggatgat tg 522

<210> 218

<211> 542

<212> DNA

<213> Homo sapiens

<400> 218
gcggcaggag gcagcggcgt tggtgggaag cgcagctcga aaagcgatgc cgattctggt 60

ttcctggggc tgcggcccac ttcggtggac ccagcgctga ggcggcggcg gcgaggccca 120

agaaataaga agcggggctg gcggcggctt gctcaggagc cgctggggct ggaggttgac 180

cagttcctgg aagacgtgcg gctacaggag cgcacgagcg gtggcttgtt gtcagaggcc 240

ccaaatgaaa aactcttctt cgtggacact ggctccaagg aaaaagggct gacaaagaag 300

agaaccaaag tccagaagaa gtcactgctt ctcaagaaac cccttcgggt tgacctcatc 360

ctcgagaaca catccaaagt ccctgccccc aaagacgtcc tcgcccacca ggtccccaac 420

gccaagaagc tcaggcggaa ggagcagcta tgggagaagc tggccaagca gggcgagctg 480

ccccgcgagg tgcgcagggc ccaggcccgg ctcctcaacc cttctgcaac aagggccaag 540

cc 542

<210> 219

<211> 671

<212>  DNA

<213>  Homo sapiens


<400>  219
ggggcattgt tattgatgga tttccaagag atgttgccca ggctctatct tttgaggacc      60

aaatctgtac ccccgatttg gtggtattcc tggcttgtgc taatcagaga ctcaaagaaa     120

gattactgaa gcgtgcagaa cagcagggcc gaccagacga caatgtaaaa gctacccaaa     180

ggagactaat gaacttcaag cagaatgctg ctccattggt taaatacttc caggaaaagg     240

ggctcatcat gacatttgat gccgaccgcg atgaggatga ggtgttctat gacatcagca     300

tggcagttga caacaagtta tttccaaaca aagaggctgc agcaggttca agtgaccttg     360

atccttcgat gatattggac actggagaga tcattgatac aggatctgat tatgaagatc     420

agggtgatga ccagttaaat gtatttggag aggacactat gggaggtttc atggaagatt     480

tgagaaagtg taaaattatt ttcataattg gaggtcctgg ctctggcaaa ggcacacagt     540

gtgaaaagct ggtggaaaaa tatggattta cacatctctc aactggcgag ctcctgcgtg     600

aggaactggc atcagaatct gaaagaagca tattgatcag agacattatg gaacgtggag     660

acctggtgcc c                                                          671

<210>  220

<211>  598

<212>  DNA

<213>  Homo sapiens


<400>  220
ggttctcctg catcatcaac ggggaggagc aggagcagac ccaccgggcc atattcaggt      60

ttgtgcctcg acacgaagac gaacttgagc tggaagtgga tgaccctctg ctagtggagc     120

tccaggctga agactactgg tacgaggcct acaacatgcg cactggtgcc cggggtgtct     180

ttcctgccta ttacgccatc gaggtcacca aggagcccga gcacatggca gccctggcca     240

aaaacagtga ctgggtggac cagttccggg tgaagttcct gggctcagtc caggttccct     300

atcacaaggg caatgacgtc ctctgtgctg ctatgcaaaa gattgccacc acccgccggc     360

tcaccgtgca ctttaacccg ccctccagct gtgtcctgga gatcagcgtg cggggtgtga     420

agataggcgt caaggccgat gactcccagg aggccaaggg gaataaatgt agccactttt     480

tccagctaaa aaacatctct ttctgcggat atcatccaaa gaacaacaag tactttgggt     540

tcatcaccaa gcaccccgcc gaccaccggt ttgcctgcca cgtctttgtg tctgaaga       598

<210>  221

<211>  537

<212>  DNA

<213>  Homo sapiens


<400>  221
gcgctgccca cgctgggcgt ggccgtgaac cgcacgctgg ggcaccggct ggagcgtgtg      60

gtgttcctga cgggcgcacg gggccgccgg gccccacctg gcatggcagt ggtgacgctg     120

ggcgaggagc gacccattgg acacctgcac ctggcgctgc gccacctgct ggagcagcac     180

ggcgacgact ttgactggtt cttcctggtg cctgacacca cctacaccga ggcgcacggc     240

ctggcacgcc taactggcca cctcagcctg gcctccgccg cccacctgta cctgggccgg     300

ccccaggact tcatcggcgg agagcccacc cccggccgct actgccacgg aggctttggg     360

gtgctgctgt cgcgcatgct gctgcaacaa ctgcgccccc acctggaagg ctgccgcaac     420

gacatcgtca gtgcgcgccc tgacgagtgg ctgggtcgct gcattctcga tgccaccggg     480

gtgggctgca ctggtgacca cgagggggtg cactatagcc atctggagct gagccct        537

<210>  222

<211>  469

<212>  DNA

<213>  Homo sapiens


<400>  222
gcagagattg aggacgagga ggagaaggtc tccaggtacc gagagctgct ggtgcggctg      60

ccccctgtca accgggccac agtgaaggcc cttatcagcc acctgtactg tgttcagtgc     120

ttctcagact cgccctccga tgaacgtgca caacctggca attgtgtttg ggcccacgct     180

cttccagaca gatgggcagg actacaaggc tggccgtgtg gtggaagacc tcattaacca     240

ctatgtggtg gtgtttagtg tggatgagga agagctcagg aagcagcggg aggagatcac     300

tgccattgtg aagatgcgcg tggctggcac tgccagtggg acccagcatg ccggtgactt     360

catctgcaca gtgtatctgg aagagaagaa ggcagagact gagcagcata tcacggtccc     420

agcatccatg actgctgagg agctcaccct ggagatcctg gatcgccgg                 469

<210>  223

<211>  688

<212>  DNA

<213>  Homo sapiens


<400>  223
gcggacgcgt gggcgcggtg ccgccgctca tctggaccct ggccagcagc gtcgtcatgg      60

gcttggtggg cacctacagc tgcttctgga ccaagtacat gaaccacctg accgtgcaca     120

acagggaggt gctgtacgag ctcatcgaga agcgaggccc ggccacgccc ctcatcaccg 180

tgtccaatca ccagtcctgc atggacgacc ctcatctctg ggggatcctg aaactccgcc 240

acatctggaa cctgaagttg atgcgttggt gaggaggaat gggcccctcg aagtgggccg 300

ggccggcccc acctgcctct gcccagattt gccctcctcc tgctctgccc aggaggtggc 360

gtccagcagt ccaggacccc tgcagctgca gacatctgct tcaccaagga gctacactcc 420

cacttcttca gcttgggcaa gtgtgtgcct gtgtgccgag gagcagaatt tttccaagca 480

gagaatgagg ggaaaggtgt tctagacaca ggcaggcaca tgccaggtgc tggaaaaaga 540

agagagaaag gagatggcgt ctaccagaag gggatggact tcattttgga gaagctcaac 600

catggggact gggtgcatat cttcccagaa ggtcagcagg gctgactggg tcgagccccc 660

ccagtatgag cggggatggg ctcccaag 688

<210> 224

<211> 430

<212> DNA

<213> Homo sapiens

<400> 224
gtgaaaaaag tggagaagtc tgttaatgaa gtgatggaat ggatgaataa tgtcatgaat 60

gctcaggcta aaaagagtct tgatcaggat ccagttgtac gtgctcagga aattaaaaca 120

aaaatcaagg aattgaacaa cacatgtgaa cccgttgtaa cacaaccgaa accaaaaatt 180

gaatcaccca aactggaaag aactccaaat ggcccaaata ttgataaaaa ggaagaagat 240

ttagaagaca aaaacaattt tggtgctgaa cctccacatc agaatggtga atgttaccct 300

aatgagaaaa attctgttaa tatggacttg gactagataa ccttaaattg gcctattcct 360

tcaattaata aaatattttt gccatagtat gtgactctac ataacatact gaaactattt 420

atattttctt 430

<210> 225

<211> 588

<212> DNA

<213> Homo sapiens

<400> 225
gggcacgcgc agccggtgcc tagccggttg gtccctgcgc ccctgccccg ccggccaccg 60

cccccccaca ccgtcccggc cccggcagcc tggccccacc atgaccgagc ggtgcagcct 120

gtggagtgcc ctgtcggccg ccgcctgctg cttctaccgc ggctccttcg tgcagttctc 180

caaggagaag tacatcctgg actcatcgcc agagaaactc cacaaggaat tggaggagga 240

gctcaaactc agcagcacgg atctccgcag ccatgcctgg taccatggcc gcatcccccg 300

agaggtctcg gagaccttgg tacaacgcaa cggcgacttc ctcatccggg actcactcac 360

cagcctgggc gactatgtgc tcacgtgccg ctggcgcaac caggccttgc acttcaagat 420

caacaaggtg gtggtgaagg caggcgagag ctacacacac atccagtacc tgtttgagca 480

ggagagcttt gaccacgtgc ccgccctcgt gcgctatcat gtgggcagcc gcaaggctgt 540

gtcagagcag agtggtgcca tcatctactg cccggtgaac cgcacctt 588

<210> 226

<211> 193

<212> DNA

<213> Homo sapiens

<400> 226
ggactgcccc aaggcccccg ccgccgctcc agcgccgcgc agccaccgcc gccgccgccg 60

cctctcctta gtcgccgcca tgacgaccgc gtccacctgg caggtgcgcc agaactacca 120

ccaggactca gaggccgcca tcaaccgcca gatcaacctg gagctctacg cctcctacgt 180

ttacctgtgc atg 193

<210> 227

<211> 497

<212> DNA

<213> Homo sapiens

<400> 227
gaggcgcttc gggagccgcg gcttatggtg cagacatggc caagtccaag aaccacacca 60

cacacaacca gtcccgaaaa tggcacagaa atggtatcaa gaaaccccga tcacaaagat 120

acgaatctct taagggggtg gaccccaagt tcctgaggaa catgcgcttt gccaagaagc 180

acaacaaaaa gggcctaaag aagatgcagg ccaacaatgc caaggccatg agtgcacgtg 240

ccgaggctat caaggccctc gtaaagccca aggaggttaa gcccaagatc ccaaagggtg 300

tcagccgcaa gctcgatcga cttgcctaca ttgcccaccc caagcttggg aagcgtgctc 360

gtgcccgtat tgccaagggg ctcaggctgt gccggccaaa ggccaaggcc aaggccaagg 420

ccaaggatca aaccaaggcc caggctgcag ccccagcttc agttccagct caggctccca 480

aacgtaccca ggcccct 497

<210> 228

<211> 590

<212> DNA

<213> Homo sapiens

<400>  228
gctgcagcgg tggtcggctg ttgggtgtgg agtttcccag cgcccctcgg gtccgaccct      60

ttgagcgttc tgctccggcg ccagcctacc tcgctcctcg gcgccatgac cacaaccacc     120

accttcaagg gagtcgaccc caacagcagg aatagctccc gagttttgcg gcctccaggt     180

ggtggatcca atttttcatt aggttttgat gaaccaacag aacaacctgt gaggaagaac     240

aaaatggcct ctaatatctt tgggacacct gaagaaaatc aagcttcttg ggccaagtca     300

gcaggtgcca agtctagtgg tggcagggaa gacttggagt catctggact gcagagaagg     360

aactcctctg aagcaagctc cggagacttc ttagatctga agggagaagg tgatattcat     420

gaaaatgtgg acacagactt gccaggcagc ctgcggcaga gtgaagagaa gcccgtgcct     480

gctgcgcctg tgcccagccc ggtggccccg gccccagtgc catccagaag aaatccccct     540

ggcggcaagt ccagcctcgt cttgggttag ctctgactgt cctgaacgct                590

<210>  229

<211>  666

<212>  DNA

<213>  Homo sapiens


<400>  229
gcgcgtccgc cgattcctcc tccttggtcg ccgcgtcctt ggctggcgtc agaaaaatgg      60

ctacaaactt cctagcacat gagaagatct ggttcgacaa gttcaaatat gacgacgcag     120

aaaggagatt ctacgagcag atgaacgggc ctgtggcagg tgcctcccgc cagagctcag     180

gccccggggc ctccagcggc accagcggag accacggtga gctcgtcgtc cggattgcca     240

gtctggaagt ggagaaccag agtctgcgtg gcgtggtaca ggagctgcag caggccatct     300

ccaagctgga ggcccggctg aacgtgctgg agaagagctc gcctggccac cgggccacgg     360

ccccacagac ccagcacgta tctcccatgc gccaagtgga gcccccagcc aagaagccag     420

ccacaccagc agaggatgac gaggatgatg acattgacct gtttggcagt gacaatgagg     480

aggaggacaa ggaggcggca cagctgcggg aggagcggct acggcagtac gcggagaaga     540

aggccaagaa gcctgcactg gtggccaagt cctccatcct gctggatgtc aagccttggg     600

atgatgagac ggacatggcc cagctggagg cctgtgtgcg ctctatccag ctggacgggc     660

tggtct                                                                666

<210>  230

<211>  406

<212>  DNA

<213>  Homo sapiens

<400> 230
gcccacgcgt ccggcggcag gagaggttgt ggtgctagtt tctctaagcc atccagtgcc 60

atcctcgtcg ctgcagcgac acacgctctc gccgccgcca tcactgagca gatgaccctt 120

cgtggcaccc tcaagggcca caacggctgg gtaacccaga tcgctactac cccgcagttc 180

ccggacatga tcctctccgc ctctcgaggt acggactaag ataacaccat catcatgtgg 240

aaactgacca gggatgagac caactatgga attccacagc gtgctctgcg gggtcactcc 300

cactttgtta gtgatgtggt tatctcctca gatggccagt ttgccctctc aggctcctgg 360

gatggaaccc tgcgcctctg ggatctcaca acgggcacca cccacg 406

<210> 231

<211> 468

<212> DNA

<213> Homo sapiens

<400> 231
gcccacgcgt ccgcccacgc gtccgagaac ccactgaaaa acttgagaat catgttgaag 60

ctaaacccat atgcaaagac catgcgccgg aacaccattc ttcgccaggc caggaatcac 120

aagctccggg tggataaggc agctgctgca gcagcggcac tacaagccaa atcagatgag 180

aaggcggcgg ttgcaggcaa gaagcctgtg gtaggtaaga aaggaaagaa ggctgctgtt 240

ggtgttaaga agcagaagaa gcctctggtg ggaaaaaagg cagcagctac caagaaacca 300

gcccctgaaa agaagcctgc agagaagaaa cctactacag aggagaagaa gcctgctgca 360

taaactctta aatttgatta ttccataaag gtcaaatcat tttggacagc ttcttttgaa 420

taaagacctg attatacagg cagtgagaaa aaaaaaaaaa aaaaaaaa 468

<210> 232

<211> 684

<212> DNA

<213> Homo sapiens

<400> 232
gatcagacag agcaggaaga aagaagagaa atcaagcaaa gattgacaag aaagcttaat 60

cagagaccca ctgttgatga attaagagac agaaaaattc tgatacgatt cagtgattac 120

gtggaagtag caaaagcgca ggactatgac aggagggcag acaaaccctg gacgagactg 180

tcagcagcag ataagcaatt cgtaaagaat taaatgagta caaaagtaat gaaatggagg 240

tacatgcatc aagcaagcac ttgacaagat tccacaggcc atagagattt tcttctgaga 300

agaatttgtg tttaattttt tgataccaac actgaacatt catcagggaa ctttcctgaa 360

gttcagctca agactaccct acctgctgtg tttgtgagaa gagtaggatc acacacacag 420
gtgcaatctt gaccacactt acctgcaaga ggagtaacca gaggacacac ttccttcctt 480
ctttggtgtc tgaggagtgt gaactgttgg ggtcagttaa gacccaacat aactctatca 540
gaagaaaact gttgtttgcc tttcaacctt gttttacagt tctgcagtgt aatggaggac 600
gggcaacgtg catgtgcagg ctcaccactc ccaggcctct gacatgaggg acatgtgaca 660
gtgtcattca gtattatgtt caaa 684

<210> 233
<211> 663
<212> DNA
<213> Homo sapiens

<400> 233
ggcgccgcgg ccgggtccct ctccacctgc cgggcggagc gcacgggcca tgggctgagc 60
cccgctgagc ccgccgggcc ggccatgggc gatcgcgagc gcaacaagaa gcggctgctg 120
gagctgctgc gggcgccgga cacaggcaac gcgcactgcg ccgactgcgg ggcggcagat 180
cccgactggg cctcttacaa gctggggatc ttcatctgtc tcaactgctg cggcgtccac 240
cgtaacttcc ctgacatcag cagagttaaa tctgtgcgac ttgacttctg ggacgacagt 300
attgtggagg ttgctgttgt cttgcagttt atgatccaca atggaaacct ccgtgtgaag 360
gccaagttcg aagccagagt cccagctttc tactacatcc cccaggccaa cgactgcctg 420
gtcttaaagg aacaatggat tcgagctaag tatgagagac gggaatttat ggctgatggg 480
gaaaccatct cgctcccagg taaccgagaa ggattcctgt ggaagcgagg aagggacaac 540
tcacagtttc tgagaaggaa gtttgtactt ctggcaagag aaggcctcct gaagtacttc 600
acaaaggaac agggtaaaag ccccaaagct gtcatcagca ttaaggactt gaatgccacc 660
ttc 663

<210> 234
<211> 433
<212> DNA
<213> Homo sapiens

<400> 234
gcccacgcgt ccgaaatgat ccccaagtcc tcgttcagca tcaacagcct ggtgcccgag 60
gcggtccaga acgacaacca ccacgcgagc cacggccacc acaacagcca ccacccccag 120
caccaccacc accaccacca ccatcaccac cacccgccgc cgcccgcccc gcaaccgccg 180
ccgccgccgc agcagcagca gccgccgccg ccgccgcccc cggcaccgca gcccccccag 240
acgcggggcg ccccggccgc cgacgacgac aagggccccc agcagctgct gctcccgccg 300

ccgccaccgc caccaccggc cgccgccctg gacggggcta aagcggacgg gctgggcggt 360

aagggcgagc cgggcggtgg tccgggggag ctggcgcccg tcgggccgga cgagaaggag 420

aagggcgccg gcg 433

<210> 235

<211> 513

<212> DNA

<213> Homo sapiens

<400> 235
gcccacgcgt ccgggctcac ccgccgtgcc atccggcctg aaaaccgtgc tcctggggct 60

gggctgggcc aggatcgcca ggtcccgctc tggggccagc tgctgctttt cctggtcttt 120

gtgatcgtcc tcttcttcat ttaccacttc atgcaggctg aagaaggcaa ccccttctag 180

agggagccat gagggtctgg gcttcagagc taggtctttg gggaagtcct ggctgactct 240

cttagcagtg gggggtgggg tgggggcagg ggcaggggct ttatgtgttt ttgcttgggg 300

ggcgctgggc ctagcccaga gtagtgcttg ctccccctgc cttgtcccac cagggaggca 360

gcagactcag gccctccatg gtcctctttg tcattttgtt gacatgcatt cctccttttg 420

catcttgttg gggggagggg attaaccaaa ggccaccctg actttgtttt tgtggacaca 480

caataaaagc cccgtttatt tgtaaaaaaa aaa 513

<210> 236

<211> 634

<212> DNA

<213> Homo sapiens

<400> 236
gcgctgatgg agcgggaaca cacagacacc ttacgccatc tgaatgtgat gctgatgttc 60

actgagtgtg tgctggacct gacagccatg aggggaggaa accctgagct gtgcacatct 120

gctgtgtcct tgtaccagat ccaggagagt gtggtggtgg accagatcag tcagctgagc 180

aaagactggg ggcgggtgga gcagctggtg ttgtacatga aagcagcaca gctgcttgcg 240

gcttctctgc atcttgccaa agcccagatc aagtccggga aactgagccc atccacagct 300

gtgaaacaag ttgtcaagaa tctgaacgaa cgatataaat tctgcatcac catgtgcaag 360

aaacttacag aaaagctgaa tcgattcttc tctgacaaac agaggtttat tgatgaaatc 420

aacagtgtga ctgcagagaa actcatctat aattgtgctg tagaaatggt tcagtctgca 480

gccctggatg agatgtttca gcagaccgaa gatattgttt atcgctatca taaggcagcc 540

cttcttttgg aaggcctaag taggattcta caggaccctg cagatattga aaatgtgcat 600

aaatataaat gtagtattga gttctgtcac ccca                                634

<210>  237

<211>  663

<212>  DNA

<213>  Homo sapiens


<400>  237
ggcagcagcg acccctactg catcgtgaag gtggacaatg agcccatcat caggacagcc      60

acagtgtgga agaccctgtg ccccttctgg ggtgaggagt accaagtgca cctgccgccc     120

accttccacg ctgtggcttt ctatgtcatg gatgaggatg ccctcagccg ggacgacgtt     180

atcggaaagg tctgccttac aagggacacc atagcctctc accctaaggg taagttctcc     240

cttccctccc acactggtct gcccagtccc tggcctccct cccactcgga gacctcccct     300

ctaggctccg tctggtctcc tgctcaggga aagccatttc tactctcccc agaagccggg     360

gccacgttct gtactcctgg cctctgttct gcagcatgtt cccaagcctg gttgttactg     420

cctcttccct agggtgaaga ggggctgcta tgggtggaat ctgaggcctc tgctggcaga     480

agaaggggcc tccttacact ctattgctga agcataggga ccccttctcc aaatcaggcc     540

agctccttct gtaacccatg gggtcgcctc catctgggcc ctagtactac tgtgtcctaa     600

gtctgaaggg ttggcctaga gccagtccag gctggagatc cctttcaatt atttctggga     660

tgc                                                                   663

<210>  238

<211>  546

<212>  DNA

<213>  Homo sapiens


<400>  238
gcggacgcgt gggcggggcg cgccccaggt ggtccgaagc ccggtccgcc ctccacgcag      60

gtgccccgcg ctccccgctc agccatgtcg tcctacagcc gcgtggcgct ggtgaccggg     120

gccaacaggg gcatcggctt ggccatcgcg cgcgaactgt gccgacagtt ctctggggat     180

gtggtgctca ccgcgcggga cgtggcgcgg ggccaggcgg ccgtgcagca gctgcaggcg     240

gagggcctga gcccgcgctt ccaccaactg gacatcgacg acttgcagag catccgcgcc     300

ctgcgcgact tcctgcgcaa ggagtacggg gggctcaatg tactggtcaa caacgcggcc     360

gttgccttca agagtgatga tccaatgccc tttgacatta aagctgagat gacactgaag     420

acaaattttt ttgccactag aaacatgtgc aacgagttac tgccgataat gaaacctcat     480

gggagagtgg tgaatatcag tagtttgcag tgtttaaggg cttttgaaaa ctgcagtgaa     540

gatctg                                                                546

<210> 239

<211> 542

<212> DNA

<213> Homo sapiens

<400> 239
gtgttgaatg aagaaaatcc aagtaaggcc tacaggtgca gttccaagga agcctttgag 60

aaagggctct gcttgagttg tagaaagaac cgctgcaaca atctgggcta tgagatcaat 120

aaagtcagag ccaaaagaag cagcaaaatg tacctgaaga ctcgttctca gatgccctac 180

aaagtcttcc attaccaagt aaagattcat ttttctggga ctgagagtga aacccatacc 240

aatcaggcct ttgagatttc tctgtatggc accgtggccg agagtgagaa catcccattc 300

actctgcctg aagtttccac aaataagacc tactccttcc taatttacac agaggtagat 360

attggagaac tactcatgtt gaagctcaaa tggaagagtg attcatactt tagctggtca 420

gactggtgga gcagtcccgg cttcgccatt cagaagatca gagtaaaagc aggagagact 480

cagaaaaagg tgatcttctg ttctagggag aaagtgtctc atttgcagaa aggaaagcac 540

ct 542

<210> 240

<211> 679

<212> DNA

<213> Homo sapiens

<400> 240
gtgtctgtgg tggtttgtgt ggttggactc tcccctggtc agtattttta tttccaggag 60

gtgttccctg tcttggctgc aaagcactgt atcatgcagg ccaatgctga gtaccatcag 120

tctatcctgg caaaacagca gaagaaattt ggagaagaaa ttgcaaggtt acagcatgca 180

gcagaactga ttaaaacagt ggcatctcgc tatgatgaat atgttaatgt gaaggatttt 240

tctgacaaaa tcaatcgtgc ccttgctgca gcaaagaagg ataatgactt catttatcat 300

gatcgagttc cagaccttaa agatctagat cctattggca aagccacact tgtgaaatct 360

accccggtca atgtacccat cagtcagaaa tttactgatc tgtttgagaa gatggttccc 420

gtgtcagtac agcagtcttt ggctgcctat aatcagagga aagccgattt gattaacaga 480

tcaattgctc agatgagaga agccaccact ttggcaaatg gggtgctagc ttcccttaat 540

cttccagcag caattgaaga tgtgtctgga gacactgtac ctcagtctat attgactaaa 600

tccagatctg tgattgaaca gggaggcatc cagactgttg atcagttgat taaagaactg 660

cctgaattac tgcaacgaa 679

<210> 241

<211> 504

<212> DNA

<213> Homo sapiens

<400> 241
gggaggcgca ggcgctggct ggcaggtgtc gctaaccgga cggtggtcgc cagggcgaga 60

ggcgggagcc ggagaggtga ggcaggaccc gggctccact gccgcctctc cgagctcttg 120

tgacgcggac ctcagtgcca ggatggctcg gggcgagcgg cggcgccgcg cagtgccggc 180

agagggagtg cggacagccg agagggcggc tcggggaggc cccggccgac gggacggccg 240

gggcggcggg ccgcgtagca cggctggagg agtggctctg gccgtcgtgg tcctgtcttt 300

ggccctgggt atgtcggggc gctgggtgct ggcgtggtac cgtgcgcggc gggcggtcac 360

gctgcactcc gcgcctcctg tgttgcctgc cgactcctcc agccccgccg tggccccgga 420

cctcttctgg ggaacctacc gccctcacgt ctacttcggc atgaagaccc gcagcccgaa 480

gcccctcctc accggtaacc gggc 504

<210> 242

<211> 631

<212> DNA

<213> Homo sapiens

<400> 242
gggatttcgc gccgctcacg cctaacatcg tgcgcgccct caatgacaag ctgtacgaaa 60

agcggaaggt ggcagcgctg gagatcgaga agctggtccg ggagttcgtg gcccagaaca 120

ataccgtgca aatcaagcat gtgatccaga ccctgtccca ggagtttgcc ctgtctcagc 180

acccccacag ccggaaaggg ggcctcatcg gcctggccgc ctgctccatc gcactgggca 240

aggactcagg gctctacctg aaggagctga tcgagccagt gctgacctgc ttcaatgatg 300

cagacagcag gctgcgctac tatgcctgcg aggccctcta caacatcgtc aaggtggccc 360

ggggcgctgt gctgccccac ttcaacgtgc tctttgacgg gctgagcaag ctggcagccg 420

acccagaccc caatgtgaaa agcggatctg agctcctaga ccgcctttta aaggacattg 480

tgactgagag caacaagttt gacctggtga gcttcatccc cttgttgcga gagaggattt 540

actccaacaa ccagtatgcc cggcagttca tcatctcctg gatcctggtt ctggagtcgg 600

tgccagacat taacctgctg gattacctgc c 631

<210> 243

<211> 699

<212>  DNA

<213>  Homo sapiens

<400>  243
gagaagttgg agcactcttg gaaagccctg gtacatgacg gagacactgt ctcagtgcat      60

cgcccaggct tctacgctga acggttccag cgcttcatgt gcaacacagt atttaagaag     120

attcccttac aaccttggaa aagcttgaag ttgcagagtc agagttcacc cattaagcgc     180

aaagcctcag aagacctgga acaagattct gccatctctg tgatcccaag atgtcagccc     240

ttgccccagc aatgctgaat tttcttctac ttggtcatca aaaaaggagt gtaatagaag     300

tgaggggagc tgctcctcca tcttcttcct gaagaagaac cttctctcct tcctcttcct     360

catgaatggg ccttagtgcc tcagagagtt gaggaccgca gcatcccctc cactccagag     420

ttgggtggta cggattttca actggccaac cctttgcctc cactattgaa tttttttcag     480

acccccattc ttcatgctgg aaatgggatt gctggacttg gcagctttct ttcccctcgt     540

ctttgactag gaaccggact cttaatttcc tcaggacaga ctagctggca cattatccct     600

accttagttc tttctctctg actcctggaa gaatactcct gtaatctctg taaaggtttt     660

tgggggataa gggtgtttaa ccacctccca gctttcttc                            699

<210>  244

<211>  527

<212>  DNA

<213>  Homo sapiens

<400>  244
gccaattttg gacccattgg gactccagat aacagggata gaaggactgc agatcggtgg      60

aaaactgata agccagccat gggtgggttt ggcattgatt atctctcagc aacgtcatcc     120

tctgagagca gttggcatca ggccagcact ccgagtggca cctggacagg ccatggccct     180

tccatggagg attcctctgc tgtcctcatg gaaagcctaa agtctatctg gtccagttcc     240

atgatgcatc ctggaccttc tgctctggag cagctgttaa tgcagcagaa gcagaaacag     300

caacggggac aaggcaccat gaaccctcca cactgaggcc aaagtggcaa cctgggaatg     360

aaggctccat aaaccatggc atgttgggtt tgcaggactg gcccacacag tcccctgcag     420

gtggcagccc tcttttctgt ttctcgctgt caagagggtg taagtattcc accagcccgc     480

tgagtgtgca cgaaatgttc gcagtgcaac aaaaagaaaa atccatc                   527

<210>  245

<211>  622

<212>  DNA

<213>  Homo sapiens

<220>

<221>  misc_feature

<222>  (549)..(549)

<223>  n = a, c, g or t


<400>  245
gctgtgcccg aggcgggcga gccttgcccc agggtacctg ctaccccccg aaggatgagc      60

gatccccctc tgagcaccat gcccaccgca cccggcctcc ggaaaccccc ttgcttccgg     120

gagagtgcca gccccagccc ggagccctgg acccctggcc acggggcctg ctccacttcc     180

agtgctgcca tcatggccac tgccacctcc agaaactgtg acaaactcaa gtccttccac     240

ctgtcccccc gaggaccacc cacatctgag cccccacctg tgccagccaa caagcccaag     300

ttcctgaaga tagctgaaga ggacccccca agggaggcag ccatgcccgg actctttgtg     360

ccccccgtgg ctccccggcc tcctgcgctg aagctgccag tgcctgaggc catggcgcgg     420

cccgcagtcc tgcccaggcc agagaagccg cagctcccgc acctccagcg atcacccccc     480

gatgggcaga gtttcaggag cttctccttt gaaaagcccc ggcaaccctc acaggctgac     540

actggcggng acgactcgga cgaggactat gagaaggtgc cactgcccaa ctcggtcttc     600

gtcaacacca cggagtcctg cg                                              622

<210>  246

<211>  563

<212>  DNA

<213>  Homo sapiens


<400>  246
gcccacgcgt ccgggctgga gtgcagtggc gtgatcacgg ctcactgcag cctctacctc      60

ccagtggctg ggactacagg catgtgccac cacacccaga taattttttt tttgtagaga     120

cagggttttg ccatgttacc caggctggtc tcgaattcct gggctcaagt gagccacctt     180

tttttttttt tttttttgga gatggagttt tgcactccag cctgaatgac ggcaagacgc     240

tgtctccaaa aaagaaacaa aaatggttgg aggaagcaaa cacccatgat tttaaagccc     300

tctcccaagc aaatcatgtg catatgcaat tcttgtttca acataatgta aaacacttca     360

tagtgagaga caaaatgaat gcacgattct ctagcaaggc cagtattctt caatcagctt     420

tgagaaccta catttcttat tttggtaaac tactgtcatg tttcctcaat tgtgttaatt     480

gatctaccag gaagaagaca aaaatagagt ttctagtagc tttattaaga aaccctgtta     540

agattggaag gctgatggaa cct                                             563

<210>  247

<211>  542

<212>  DNA

<213>  Homo sapiens

<400>  247
gtctcccgtt gccacggaga ctgggcatct gctccctttg tgctgcccga gtgccttccc      60

cctggggtca aggggtatag gggggcagaa agagaggcag acacacctct agagatgaac     120

aggccgatcc aggtcaagcc agccgacagc gagagccgag gagaccggaa gctctttgtg     180

gggatgctag ggaagcagca gacagatgag gacgtccgga agatgtttga gcccttcggg     240

accatcgacg agtgcactgt gctccggggg ccagatggca ccagcaaagg ctgcgccttc     300

gtgaagttcc agacccacgc tgaggcccag gcggccatca acacccttca cagcagccgg     360

accctgccag gtgcctcgtc cagcctggtg gtgaagtttg ctgacactga gaaggagcga     420

ggtctccgcc gcatgcagca ggtggccacc cagttgggca tgttcagccc catcgccctc     480

cagtttggag cctacagcgc ctacacccag gccctgatgc agcagcaggc ggccctggta     540

gc                                                                    542

<210>  248

<211>  674

<212>  DNA

<213>  Homo sapiens

<400>  248
gtgcatatgc gggacctggt gggcatggga caccacttcc tgccaagtga gcccaccaag      60

tggaatgtag aagacgtcta cgaattcatc cgctctctgc caggctgcca ggagatagca     120

gaggaattcc gtgcccagga aatcgacggg caagccctgc tgctgctcaa ggaggaccac     180

ctgatgagcg ccatgaacat caagctgggg cccgccctga agatctatgc ccgcatcagc     240

atgctcaagg actcctaggg ctggtggcag ccaggattct ggcccagggc gcctcctccc     300

gactgagcag agccagacag acattcctga ggggcccaga aatggggccg gttggagggc     360

aggggctctc cctaggggca tagctggtga ggaggtctgg gcacctcctc catggctctc     420

aggggccttt catttctgtg ggaggggcag agaggtaggt ggcacagaag atggggcttt     480

atgcttgtaa atattgatag cactggcttc ctccaaagtc ccaatactct agccccgctc     540

tcttcccctc tttctgtccc ccattttcca gggggtatat ggtcagggct ccccaacctg     600

agttgggtta cttcaagggc agccagcagg cctggatgga ggcctagaaa gcccttgcct     660

tccttcctcc cact                                                       674

<210>  249

<211>  558

<212>  DNA

<213>  Homo sapiens


<400>  249
gggcaggcta cgccgtggct ggcccctcag cgggctggga aaagagtggc cacggtgacc      60

gtcacccgcc tgccggcacc atgaagtgct ccctgcgggt gtggttcctc tccgtggcct     120

tcctgctggt gttcatcatg tccctgctct tcacctactc gcaccacagc atggccacgc     180

tcccctacct ggactcaggg gccctggatg ggacgcaccg ggtgaagctg gtgcccggct     240

atgccggcct gcagcgcctc agcaaggaga ggctctcggg caagagctgt gcctgtcgcc     300

gctgcatggg cgatgccggt gcctccgact ggtttgacag ccactttgac ggtaacattt     360

cccccgtctg gacccgagag aacatggatc ttccaccgga cgtccagagg tggtggatga     420

tgctgcagcc ccagttcaag tcacacaaca ccaatgaggt gctggagaag ctgttccaga     480

tagtgcctgg cgagaacccc taccgcttcc gggaccccca ccagtgccgg cgctgtgccg     540

tggtggggaa ctcgggca                                                   558

<210>  250

<211>  664

<212>  DNA

<213>  Homo sapiens


<220>

<221>  misc_feature

<222>  (576)..(576)

<223>  n = a, c, g or t


<400>  250
gcggacgcgt gggttgtacg cttgcgggag gaggtgagga atctctccgg cagtctggcg      60

gccattcagg aggagatggg tgcctacggg tatgaggacc tgcagcaacg ggtgatggcc     120

ctggaggccc ggctccacgc ctgcgcccag aagctgggct gtgggaagct gaccggggtc     180

agtaacccca tcaccgttcg ggccatgggg tcccgcttcg gctcctggat gactgacacg     240

atggccccca gtgcggatag ccgggtctgg tacatggatg gctattacaa aggccgccgg     300

gtcctggagt tccgtaccct gggagacttc atcaaaggcc agaactttat ccagcacctg     360

ctgccccagc cgtgggcggg cacgggccac gtggtgtaca acggctccct gttctataac     420

aagtaccaga gcaacgtggt ggtcaaatac cacttccgct cgcgctctgt gctggtgcag     480

aggagcctcc cgggcgccgg ttacaacaac accttcccct actcctgagg cggcttctcc     540

gacatggact tcatggtgga cgagagcggg ctctgngctg tgtacaccac caaccagaac 600
gcgggcaaca tcgtggtcag ccggctggac ccgcacaccc tcgaggtcat gcggtcctgg 660
gaca 664

<210> 251
<211> 656
<212> DNA
<213> Homo sapiens

<400> 251
gcccaggtga tccaggcccc tggcccgcgg gctggtccct gcatcctgcc catcgtccgg 60
gatggcaaga aggtcagcag cacccactat tacttgctgc ccgagcgacc atcctacctg 120
gagcgctacc agcgcttcct gcgtgaggcc cagagccccg aggagcctac ccccctgcct 180
gtgcctctgc tgctgccccc acccagcacc ccagcccccg ccgcccccac ggccaccgtg 240
cggccgatgc cccaggctgc cttggacccc aaggccaact tctccaccaa caacagcaac 300
ccaggggccc ggccaccacc cccgagggcc actgctcggc tgccacagag gggctgccct 360
ggcgatgggc cagaggcggg ccggccagca gacaagatcc agatggtgga gcagctcttc 420
gggctgggtc tgcggcccag aggggagtgc cacaaagtgc tggagatgtt cgactggaac 480
ctggagcagg ccggctgcca ccttctgggc tcctgaggcc ctgcccacca caagcgctga 540
gatgcgtctg gagagccaga gggcctgcct gaaggaatca cctgagcctg tccgtccacc 600
aggagtgggg agatgccccc atccagtcct ggaggacccg ctgctcctgc tgctcc 656

<210> 252
<211> 523
<212> DNA
<213> Homo sapiens

<400> 252
gatgagctgc gcggggcggg cgggccctgc ccggctcgcc gcgctcgccc tgctgacctg 60
cagcctgtgg ccggcacggg cagacaacgc gagccaggag tactacacag cgctcatcaa 120
cgtgacggtg caggagcccg gccgcggcgc cccgctcacg tttcgcatcg accgcgggcg 180
ctacgggctt gactccccca aggccgaggt ccgcggccag gtgctggcgc cgctgcccct 240
ccacggagtt gctgatcatc tgggctgtga tccacaaacc cggttctttg tccctcctaa 300
tatcaaacag aggattgcct tgctgcagag gggaaactgc acgtttaaag agaaaatatc 360
acgggccgct ttccacaatg cagttgctgt agtcatctac aataataaat ccaaagagga 420
gccagttacc atgactcatc caggcactgg agatattatt gctgtcatga taacagattt 480

gaggggtaag gatattttga gctatctgga gaaaaacatc tct 523

<210> 253

<211> 598

<212> DNA

<213> Homo sapiens

<400> 253
gctcaagacg gagtctttgc tccctaaact agatgcagcc ctgagtgggg tggggcttcc 60

tgggtgtcct aagggccccc cctcaccagg acggagtcgc cgtggcaaga cccgtcaccg 120

caaggccagc gccaagggga gctgtgggga cctgcctggg cttcgtacag ctgtgccacc 180

ccatgaacct ggaggaccag gaagcccatg gggcctagga gggggaccct catcctggga 240

ggcctgccct cccgccctcc gtgggcttca tcatgacctc ctgctccgca aaatgtcttc 300

atcgtcccca gacctgctgt cagcagcact agggtcccgg ggccgggggg ccacaggcgg 360

agctggggat cctggctcac cacctccggc ccggggtgac accccaccaa gtgagggctc 420

agcccctggc tccaccagcc catattcacc tgggggagcc aaaggggaac cacctcctcc 480

agtagggcct ggtgaaggtg tggggcttct gggaactgga agggaaggga cctcaggccg 540

gggaggaagc cgggctgggt cccagcactt gaccccagct gcactgctgt acagggct 598

<210> 254

<211> 641

<212> DNA

<213> Homo sapiens

<220>

<221> misc_feature

<222> (592)..(592)

<223> n = a, c, g or t

<400> 254
gcccacgcgt ccgcaatcgg cactgggtat gacctgtcag cctctacatt ctctcctgac 60

ggaagagttt ttcaagttga atatgctatg aaggctgtgg aaaatagtag tacagctatt 120

ggaatcagat gcaaagatgg tgttgtcttt ggggtagaaa aattagtcct ttctaaactt 180

tatgaagaag gttccaacaa aagacttttt aatgttgatc ggcatgttgg aatggcagta 240

gcaggtttgt tggcagatgc tcgttcttta tcagacatag caagagaaga agcttccaac 300

ttcagatcta actttggcta caacattcca ctaaaacatc ttgcagacag agtggccatg 360

tatgtgcatg catatacact ctacagtgct gttagacctt ttggctgcag tttcatgtta 420

gggtcttaca gtgtgaatga cggtgcgcaa ctctacatga ttgacccatc aggtgtttca 480

tacggttatt ggggctgtgc catcggcaaa gccaggcaag ctgcaaagac ggaaatagag 540

aagcttcaga tgaaagaaat gacctgccgt gatatcgtta aagaagttgc anaaataatt 600

tacatagtac atgacgaagt taaggataaa gcttttgaac t 641

<210> 255

<211> 554

<212> DNA

<213> Homo sapiens

<400> 255
gcccacgcgt ccgctttccc cggcagctca ccatcatctt caagaacttc caggagtgtg 60

tggaccagaa ggtgtaccag gctgagatgg acgagctccc ggccgccttc gtggatggct 120

ctaagaacgg tggggacaag cacggggcca acagcctgaa gatcactgag aaggtgtcag 180

gccagcacgt ggagatccag gccaagtaca tcggcaccac catcgtggtg cgccaggtgg 240

gccgctacct gacctttgcc gtccgcatgc cagaggaagt ggtcaatgct gtggaggact 300

gggacagcca gggtctacct ctgcctgcgg ggctgccccc tcaaccagca gatcgacttc 360

caggccttcc acaccaatgc tgagggcacc ggtgcccgca tgctggcagc cgccagccct 420

gcacccacag cccccgagac cttcccatac gagacagccg tggccaagtg caaggagaag 480

ctgccggtgg aggacctgta ctaccaggcc tgcgtcttcg acctcctcac cacgggcgac 540

gtgaacttca cact 554

<210> 256

<211> 472

<212> DNA

<213> Homo sapiens

<220>

<221> misc_feature

<222> (439)..(439)

<223> n = a, c, g or t

<400> 256
gtaattgctg aagcattttg cagtgcccta gaatctgttg ttggctcttt agaacatgat 60

ggaggtgaaa ttctcactga catgaagtat ggacaccttt ggtcagttca ggcagattct 120

ccctgtgttg ctaactggcc agatttgctt tcacagtgtg gctgtggatt atacaatagc 180

caggaagaac tcaactggtc tttcttaaga agcacacgtc gtccatttgt gccacaaagc 240
tgccttccac atgaagctgt gggctcagcc agcaacctga ccttggactg tttgactgca 300
aagcttattg gcctacaggt ggctgtagag acagccaatt tgattttgga tctttcatat 360
gttattgaag ataaaaacta agagaatagc atgttcgtat tacaagagaa acaaataaac 420
tagtctgttg gcaattggna aaaaaaaaaa aaaaaaaaaa aaaaaaaaaa gg 472

<210> 257
<211> 641
<212> DNA
<213> Homo sapiens

<400> 257
gattatgggg aagaagattt acagcttata cacatcaaga gacctgaggg gcggaagccg 60
agcgaagtgg cgcacaagag catcgaggca gtggtggctc ggctagagaa gcagaacggc 120
ctgagcctgg gccatagcac gtgtccggaa gaggtcttcg tggaggcctc gccaggcaca 180
gaggacatgg acagtctata agatgctgtg gtgccccggg ctctgtatga ggagctgctg 240
cgcaactacc agcagcaaca ggaagagatg cgccacctcc agcaggagct ggagcggact 300
cggaggcagc tggtacaaca ggccaagaag ctcaaggagt acggggcact tgtgtctgaa 360
atgaaggagc tccgtgacct taaccggagg ctccaggacg tgctgctcct gcggcttggc 420
agcggtcccg ccattgatct ggaaaaagta aagtcagaat gtctcgagcc cgagccggag 480
ttacggagca ctttcagtga ggaagcaaat acgtcgtcct attaccccgc tcctgcgcct 540
gtcatggaca agtatatcct atacaatggc aaggtccatc tgggaagcgg gatttgggtt 600
gatgaggaga tatggcacca gctacaagta acccaaggag a 641

<210> 258
<211> 672
<212> DNA
<213> Homo sapiens

<400> 258
ggtctccgta gaaacgctgg gactatggcg gagccgactg ggctgttgga gatgtccgag 60
ctccccggag atagcagtgt cccacaggtg ggcaccgcga gtggcgtcag cgacgtactg 120
cggggggcag tcggcggcgg ggttcgggtg caggaggctc gggaaggccc agtggccgaa 180
gctgcgcggt ccatggcgcg gatgccgggc cctgtgcccg ggcccatccc cagcagcgtc 240
ccgggcctgg cctccgcgcc agacccccat cagcagctcg ctttcttata aattaaccgg 300
cagctgttgt tccgcgagta tctggatggt agctccatga ttccggtcag attactacgg 360
gatttcgagg aacgccgcag gctgtttgtg gaaggctgca aggcgaggga agcagccttt 420

gacgcggatc ccccgcagat ggacttcgct gctgtcgcgt ttacggtagc gctgactgcc 480
tccgaggccc tcagtcctct ggccgactga gtcggctgtg atgatgacat tggtggcaag 540
gaatagccaa gtgacccttt tgggatcaaa agaacaggca ggctgtgcca gtttagttaa 600
ctcagtcatt agggggaatg caaactggaa gggaatacgg caatgtgcaa ttgaaggagg 660
aagcacactc cg 672

<210> 259
<211> 545
<212> DNA
<213> Homo sapiens

<400> 259
ggggattact gtgagcgagg ctgcttttct gatttggatc tcattgacaa cctggggcca 60
gccatgatgc tcagtgaccg actgacattc ctgggaaagt atcgcgagtt ccaccgtatg 120
tacggggaga agcgttttgc cgacgcagct tctctccttc tgtccttgat gacgtctcgg 180
attgcccctc ggtctttctg gatgactctg ctgacagatg ccttgcccct tttggaacag 240
aaacaggtga ttttctcagc agaacagact tatgagttga tgcggagtct ggaggacttg 300
acgtcaagaa gacctgtgca tggagaatct gataccgagc agctccagga tgatgacata 360
gagaccacca aggtggaaat gctgagactt tctctggcac gaaatcttgc tcgggcaatt 420
ataagagaag gctcactgga aggttcctga gaactgcttc aatgtggtat ctttgtatgg 480
caatgtatat agatttttta aaagaataaa tgttgttttg caaatgtaaa aaaaaaaaaa 540
aaagg 545

<210> 260
<211> 605
<212> DNA
<213> Homo sapiens

<400> 260
ggggcgcggg cctggcttcg gcctaccggc agccagggcc tgagttccct ggcggaagag 60
gcagcgcgtg cgaccgagaa cccggagcag gtggcgagcg agggtctccc ggagcccgtg 120
ctgcgcaaag tcgagctccc ggtacccact catcgacgcc cagtgcaggc ctgggtcgag 180
tccttgcggg gcttcgagca ggagcgcgtg ggcctggccg acctgcaccc cgatgttttc 240
gccaccgcgc ccaggctgga catactgcac caggttgcta tgtggcagaa gaacttcaag 300
agaattagct atgccaagac caagacgaga gccgaggtgc ggggcggtgg ccggaagcct 360
tggccgcaga aaggcactgg gcgggcccgg catggcagca tccgctctcc gctctggcga 420

ggaggaggtg ttgcccatgg cccccggggc cccacaagtt actactacat gctgcccatg 480
aaggtgcggg cgctgggtct caaagtggca ctgaccgtca agctggccca ggacgacctg 540
cacatcatgg actccctata gctgcccacc ggagacccac agtacctgac agagctggcg 600
cacta 605

<210> 261
<211> 641
<212> DNA
<213> Homo sapiens

<400> 261
ggacggcgac catagtctct ccaggtcaat ggccagcaag gaggggggtc cgagccggcg 60
gcggcggcgg cagtggtggc agcgggagac aaatggaaac ctccacaggg cacagactcc 120
atcaagatgg agaacgggca gagcacagcc gccaagctgg ggctgcctcc cctgacgccc 180
gagcagcagg aggcccttca gaaggccaag aagtacgcca tggagcagag catcaagagt 240
gtgctggtga agcagaccat cgcgcaccag cagcagcagc tcaccaacct gcagatggca 300
gcagtgacaa tgggctttgg agatcctctc tcacctttgc aatcgatggc ggctcatcgg 360
cagcgggcgc tggccatcat gtgccgcgtc tacgtgggct ctatctacta tgagctgggg 420
gaggacacca tccgccaggc ctttgccccc tttggcccca tcaagagcat cgacatgtcc 480
tgggactccg tcaccatgaa gcacaagggc tttgccttcg tggagtatga ggtccccgaa 540
gctgcacagc tggccttgga gcagatgaac tcggtgatgc tggggggcag gaacatcaag 600
gtgggcagac ccagcaacat agggcaggcc cagcccatca t 641

<210> 262
<211> 551
<212> DNA
<213> Homo sapiens

<400> 262
gaggaggacg gcgagagcac ggacagactc atggggaagg gccaggtcca gctggacccg 60
caggccctgc aggacagaga ctggcagcgc accgtcatcg ccatgaatgg gatcgaagta 120
aagctctcgg tcaagttcaa cagcagggag ttcagcttga agaggatgcc gtcccgaaaa 180
cagacagggg tcttcggagt caagattgct gtggtcacca agagagagag gtccaaggtg 240
ccctacatcg tgcgccagtg cgtggaggag atcgagcgcc gaggcatgga ggaggtgggc 300
atctaccgcg tgtccggtgt ggccacggac atccaggcac tgaaggcagc cttcgacgtc 360
aataacaagg acgtgtcggt gatgatgagc gagatggacg tgaacgccat cgcaggcacg 420
ctgaagctgt acttccgtga gctgcccgag cccctcttca ctgacgagtt ctaccccaac 480

ttcgcagagg gcatcgctct ttcagacccg gttgcaaagg agagctgcat gctcaacctg 540

ctgctgtccc t 551

<210> 263

<211> 508

<212> DNA

<213> Homo sapiens

<400> 263
gcgaggggga aatgaaagag cttccggagc agcttcagga acatgctata gaggacaaag 60

aaactataaa caatttagat acttcatcat ctgacttcac tatattacag gaaattgaag 120

agccatccct ggagccagaa aatgagaaaa tactcgacat tttgggggaa acttgtaaat 180

ctgagccagt aaaagaagaa agttccgagc tggagcagcc atttgcacag gacacaagta 240

gcgtggggcc agacagaaag cttgcggagg aagaggacct atttgacagc gcccatccgg 300

aagagggtga tttagatttg gccagcgagt caacagcaca cgctcagtcg agcaaggcag 360

acagcctgtt agcggtagtg aaaagggagc ccgcggagca tccaggcgat ggcgagagga 420

cggactgtga gcctgtaggg ctagagccgg cagttgagca gagtagtgcg gcctccgagc 480

tcgcggaggc ctctagcgag gagctcgc 508

<210> 264

<211> 625

<212> DNA

<213> Homo sapiens

<400> 264
gcccacgcgt ccgcccacgc gtccgcccac gcgtccgccc acgcgtccgc ccacgcgtcc 60

gtccaaaaat cccgccaaga gagccccaga gcagaggaaa atccaaagtg gagagagggg 120

aagaaagaga ccagtgagtc atccgtccag aaggcgggga gagcagcagc ggcccaagca 180

ggagctgcag cgagccgggt acctggactc agcggtagca acctcgcccc ttgcaacaaa 240

ggcagactga gcgccagaga ggacgtttcc aactcaaaaa tgcaggctca acagtaccag 300

cagcagcgtc gaaaatttgc agctgccttc ttggcattca ttttcatact ggcagctgtg 360

gatactgctg aagcagggaa gaaagagaaa ccagaaaaaa aagtgaagaa gtctgactgt 420

ggagaatggc agtggagtgt gtgtgtgccc accagtggag actgtgggct gggcacacgg 480

gagggcactc ggactggagc tgagtgcaag caaaccatga agacccagag atgtaagatc 540

ccctgcatct ggaagaagca atttggcgcg gagtgcaaat accagttcca ggcctgggga 600

gaatgtgacc tgaacacagc cctga 625

<210> 265

<211> 611

<212> DNA

<213> Homo sapiens

<400> 265
gagatccgag agctctgctt ctctgaaaag cgtgtggctt tctggacttg tttcaggcca 60

cccacgagcc agcgggaaag gattgccagc cagagaaaga cgaactccga agtcccaatg 120

cacagatcag cccccagtca aaccaccaag aggagccgat cgccattttc cactactcgt 180

cgtagttggg acgacagcga gagttcggga accaacctga atattgataa tgaggactat 240

tccaggtatc cgccaagaga gtacagagct tcgggtagca gaagaggaat ggcctatgga 300

catattgact cttatggggc agatgatagt gaggaggagg gggctgggcc tgttgagcga 360

ccgccagtga gagggaaaac tggcaagttt aaagatgata agctgtatga cccagagaaa 420

ggggcaaggt ctttggctgg gccacctcca catttctcta gttttagccg tgatgtgaga 480

gaggagcgag acaagttaga cccagtccct gcagcaagat gctcagctag cagagctgac 540

ttcctgccac aaagtagtgt ggcctcacag tcgtcttctg aaggcaagct ggctacaaaa 600

ggtgacagct c 611

<210> 266

<211> 575

<212> DNA

<213> Homo sapiens

<400> 266
ggcctgcggc gctcagtgga aaaatacaac tcagcccgag ctgactttga gcagaagatg 60

ctggactcag ccctgcgctt ccaagccatg gaggagacac acctgaggca catgaaggca 120

ctgctgggct catatgctca ctcggtggag gacacgcacg tgcagattgg gcaggtgcat 180

gaggaattta agcagaacat cgagaacgtc agcgtggaga tgctactcag gaagtttgca 240

gagagtaagg gcacaggccg ggagaagcct ggacctctgg acttcgaggc atacagtgcg 300

gctgccctgc aggaagcgat gaaacgtttg cggggagcca aggcctttcg ccttccagga 360

ctaagccggc gggagcggga gccggagcca cctgcagctg tagatttcct ggagcccgat 420

tcagggacat gtccagaggt ggatgaagaa ggtttcactg tccggcctga tgtgacccat 480

aacagcacgg tcgagccctc ccgtttctcg tccagcgact ccgacttcga cgatgaagag 540

ccccgcaagt tctatgtgca catcaagcct gcccc 575

<210> 267

<211>  607

<212>  DNA

<213>  Homo sapiens


<400>  267
gcaggcacct ctctgtcagc tcccataacc ctggccgaag ctcacctccc cctggctatg      60

ttcctgagcg gcagcagcac attgcccggc aggggtccta caccagcatc aacagtgagg     120

gggagttcat cccatagacc agcgagcagt gcatgctgga tcccctgagc agtgcagaaa     180

attccttgtc tggaagctgc caatccttgg acagcccatc cttccggaaa tcacgaatgt     240

cccgtgccca gagcttccct gacaacagac aggaatactc agatcgggaa actcagcttt     300

atgacaaagg ggtcaaaggt ggaacctacc cccggcgcta ccacgtgtct gtgcaccaca     360

aggactacag tgatggcaga agaacatttc cccgaatacg gcgtcatcaa ggcaacttgt     420

tcaccctggt gccctccagc cgctccctga gcacaaatgg cgagaacatg ggtctggctg     480

tgcaatacct ggacccccgt gggcgcctgc ggagtgcgga cagcgagaat gccctctctg     540

tgcaggagag gaatgtgcca accaagtctc ccagtgcccc catcaactgg cgccggggaa     600

agctcct                                                               607

<210>  268

<211>  347

<212>  DNA

<213>  Homo sapiens


<400>  268
gcttacattg aggatggctt gagggagtgc tgccagcctg tgctggttta catggctccc      60

caggctgagc tgcggggtgg ctcctgggag gagattgact cctccatcaa cccccggcac     120

atggagatgt atgctgaccg agaaagcagg ggatctgttc tggagccaga agggacagta     180

taaatcaaat tccgcagata ggatctggag aaaaccatgc gtcgggtgga cccagtctac     240

atccacttgg ctgagcgatt ggggacccca gagctaagca cagctgagcg gaaggagttg     300

gagaacaagt tgaaggagcg ggaggaattc ctaattccca tttacca                   347

<210>  269

<211>  514

<212>  DNA

<213>  Homo sapiens


<400>  269
gcaaacccca atctcagtgg acaaggtgac cctggcagca accccaacaa acgccagagg      60

cagccccctc tcctgggaga tcaccccgca gaatatggag ggccccacgg tgggtaccac 120

agccattacc atgatgaggg ctacgggccc cccccacctc actacgaagg gagaaggatg 180

ggtccaccag tggggggtca ccgtcggggc ccaagtcgct acggccccca gtatgggcac 240

cccccacccc ctcccccacc acccgagtat ggccctcacg ccgacagccc tgtgctcatg 300

gtctatggct tggatcaatc taagatgaac tgtgaccgag tcttcaatgt cttctgctta 360

tatggcaatg tggagaaggt gaaattcatg aaaagcaagc cgggggccgc catggtggag 420

atggctgatg gctacgctgt agaccgggcc attacccacc tcaacaacaa cttcatgttt 480

gggcagaagc tgaatgtctg tgtctccaag cagc 514

<210> 270

<211> 477

<212> DNA

<213> Homo sapiens

<400> 270
gattttttct ttgaacttga tgccatggat catctgcagt cattcattgc agattgtgat 60

cgtagaacag aagtggccaa gaaaagatta gcagaaactc aagaagagat tagtgctgaa 120

gtagcagcaa aggcagaacg tgttcatgag ttaaatgaag aaattggtaa attgttagcc 180

aaggtggaac aactaggagc tgaagggaat gtggaggaat cccagaaagt aatggatgaa 240

gtagagaaag cacgggcaaa gaaaagagaa gcagaggaag tttatcggaa ttctatgcca 300

gcttccagtt ttcagcagca gaaacttcga gtctgtgaag tctgctctgc ctatttagga 360

cttcatgata atgacagacg actggctgat cattttgggg gtaaactgca cctgggattt 420

attgaaataa gagagaagct tgaagaatta aagagagtcg tagctgagaa gcaggag 477

<210> 271

<211> 638

<212> DNA

<213> Homo sapiens

<400> 271
gcccacgcgt ccgaaaagag tagctcagag gctttattta aaagtttttc tagcgcacca 60

ccactgatac tcacttgttc tgctgacaaa aaacagataa aagataaatc tcatgtcaag 120

atgggaaagg tcaaaattga aagtgagaca tcagagaaga agaaatcaac gttaccgcca 180

tttgatgata ttgtggatcc caatgattca gatgtggagg agaatatatc ctctaaatct 240

gattctgaac aacccagtcc tgccagctcc agctccagct ccagctccag cttcacacca 300

tcccagacca ggcaacaagg tcctttgagg tctataatga aagatctgca ttctgatgac 360

aatgaggagg aatcagatga agtggaggat aacgacaatg actctgaaat ggagaggcct 420

gtaaatagag gaggcagccg aagtcgcaga gttagcttaa gtgatggcag cgatagtgaa 480

agcagttctg cttcttcacc cctacatcac gaacctccac cacccttact aaaaaccaac 540

aacaaccaga ttcttgaagt gaaaagtcca ataaagcaaa gcaaatcaga taagcaaata 600

aagaatggtg aatgtgacaa ggcataccta gatgaact 638

<210> 272

<211> 488

<212> DNA

<213> Homo sapiens

<400> 272
gagaagttct tatggtgcag gtcaagaaag atcttaagac tggtcattca aaggggtttg 60

gctttgttcg ttttacggaa tatgaaacac aagtgaaagt aatgtcacag cgacatatga 120

tagatggacg atggtgtgac tgcaaacttc ctaattctaa gcaaagccaa gatgagcctt 180

tgagaagcag aaaagtgttt gtggggcgct gtacagagga catgactgag gatgagctgc 240

gggagttctt ctctcagtac ggagatgtga tggatgtctt catccccaag ccattcatgg 300

cctttgcctt tgttacattt gcagatgatc agattgcgca gtctctttgt ggagaggact 360

tgatcattaa aggaatcagc gttcatatat ccaatgccga acctaagcac aatagcaata 420

gacagctaga aagaagtgga agatttggtg gtaatccagg tggctttggg aatcagggtg 480

gatttggt 488

<210> 273

<211> 628

<212> DNA

<213> Homo sapiens

<400> 273
gcccacgcgt ccgcggacct aggtcgcggc gacatggcca aacgtaccaa gaaagtcggg 60

atcgtcggta aatacgggac ccgctatggg gcctccctcc ggaaaatggt gaagaaaatt 120

gaaatcagcc agcacgccaa gtacacttgc tctttctgtg gcaaagtaag taaggcaaag 180

tctctggtga gaggagaggg agggcaggtt tcttacccaa gtgaggcctg acttcaaggt 240

attttataag ccgtgtgctg gtgggcagtt ggaattactc ataccgttga ttatgagttt 300

taagataaaa gtgttgatgg taacttcaga ttttgtgaga cgtttttcat ttaaagaaaa 360

ccgcttaaac gttaatgggt aaaataatca tttgacagag tgcccccagc ctaagccaaa 420

cctgctttgt gggaaatgat tccatcagtt ttgtctactg atgtttctgc accaactccc 480

aagatgtctg tgcatctaat accattctac ttgttctgcg agggattttt gtttgataaa 540

ggtttgaacg tttatgtgca aaatactgca ttacagcaat ccttgttttg tttttgaggg 600
agaaagggag gctttcacat ccctgaca 628

<210> 274
<211> 486
<212> DNA
<213> Homo sapiens

<400> 274
gcagccactc accgcccaaa gccttccatg ctggcaagct cttccccttt ggtggcaccc 60
gggctgagcc tgggtccaac ggggcgggcg ggcaggcccg gccagatggg acccccagca 120
gtggcagcag ccgggttcag aggagtgggc cggtggacga ggccctggct gaggagctgg 180
aagccccccg gcccgaagag gaaggctcag gtcacaagct ttaactcggg ggtggggacg 240
ccgggccggc tttggggata gggtagggtg gccaggcctg gggagagggg ctgaagggga 300
caggggaagc ccggtcttag gtcctggtgg ggacctgggt cagtggtacg tggagcctct 360
cagttaaagc ctgcttagta tctccagggc ctttgttagc ctggagcccc gtgcctgacc 420
ctccccaggg agggatgcca aagccccttg tcccaggact gtacccaggg acccccacca 480
tgctcc 486

<210> 275
<211> 672
<212> DNA
<213> Homo sapiens

<400> 275
gctctgggct cactgaaggc atatggcagc tggtacctcc atcactgttt aaaggctcac 60
atatcagtca gggaaacgag gctgaggaaa gagaggagcc ttgggaccac actgaaaaaa 120
ctgaagagga gccggtctct ggcagctcag gaagctggga ccagtcaagc cagccagtgt 180
ttgagaatgt gaacgttaaa tcttttgaca gatgtactgg ccactcggct gagcacacac 240
agtgtgggaa gccacaggaa agtactggga ggggttctgc ttttctcaaa gctgtccagg 300
gtagcgggga cacatctagg cactgtctac ctaccctagc agatgccaaa ggtctccagg 360
acactggggg cactgtgaac tatttctggg gtattccatt ctgccctgat ggagtagacc 420
ctaaccagta taccaaggtc attctctgcc agttggaggt ttatcaaaag agcctgaaaa 480
tggctcagag gcagctcctt aataaaaaag gttttgggga accagtgtta cctagacctc 540
cttctctgat ccagaatgaa tgtggccaag gagagcaggc tagtgagaaa aatgaatgca 600
tctcagaaga tatgggagat gaagacaaag aggagaggca ggagtctagg gcatctgact 660
ggcactcaaa aa 672

<210> 276

<211> 583

<212> DNA

<213> Homo sapiens

<400> 276
gaggagccgc tcctttccac tcgggaaacc ttcagaggag tctcataaag gacacggctg 60

gctgcttttc tcagcgccga agccgcgcca tgctcgtcct cagaagcgcc ctgactcggg 120

cgctggcctc acggacgctg gcgcctcaga tgtgctcatc ttttgctacg ggacccagac 180

aatacgatgg aatattctat gaatttcgtt cttattacct taagccctca aagatgaatg 240

agttcctgga aaattttgag aaaaacgctc atcttcggac agctcactct gaattggttg 300

gatactggag tgtagaattt ggaggcagaa tgaatacagt gtttcatatt tggaagtatg 360

ataattttgc tcatcgaact gaagttcgga aagccttggc caaagataag gaatggcaag 420

aacaattcct cattccaaat ttggctctca ttgataaaca agagagtgag attacttatc 480

tggtaccatg gtgcaaatta gaaaaacctc caaaagaagg agtctatgaa ctggccactt 540

ttcagatgaa acctggtggg ccagctctgt ggggtgatgc att 583

<210> 277

<211> 378

<212> DNA

<213> Homo sapiens

<400> 277
   gcccacgcgt ccgcacagaa ggagctctgt gtgtcgtctg agcagtcgcg agaagatttc 60
   cctccaggac ttgtccaagg agcgccggcc tggtggggct gggggggccc ccatccagga 120
   cgaggatgag ggggaagaag gtcccaccga accaccccct gcagaaccca gaaccctcaa 180
   tggcgtctcc tccccgccgc accccagccc taagagtccc gtgcagcttg aagaggcccc 240
   cttctccagg cgctttggcc tcctgaagac agggagttct ggtgccctgg gtccccctga 300
   aaggcggaca gcggagggag cccctgaggc tgggctgcag cgctcggctt cctcctcctg 360
   gctggaaggg accttcac 378

<210> 278

<211> 488

<212> DNA

<213> Homo sapiens

<400>  278
gtctacatcg tcgggggcta caactggcgt ctcaacaacg tcacgggcat cgtacaggtg      60

tacaacacgg acaccgacga gtgggagcgg gacctgcact tcccggagtc cttcgcaggc     120

atagcctgcg cccccgtcct gctgccccgg gccgggacca ggaggtagcc cccaagaccc     180

ccgggaccct ggcctgaccg catgttgtct ccaagtgggg cttggcgaat gcacgtctgc     240

ctgagaaccc cagtgccccc cttcgcccgg gctgcccttg aggggcctgc tgcgttgata     300

agcccccctc ccaggggtcc ctccctccct ccttcccaaa gcagatcctg gctgcgagtc     360

catccgaggg agcctgccgg caaagcgtct gacatgtggt ggcagcaaat tcgtccccgg     420

ggtggtttcc tcgcctggcc cccgagtccc cacgggctgg cgggtggaat cccaggtctc     480

caggggggt                                                             488

<210>  279

<211>  614

<212>  DNA

<213>  Homo sapiens


<400>  279
ggtggttgtg gcgaggctgt gcggcagggc gcacgggacc tgtgctgcag cggctctctc      60

aggccgtggg tcgtcgctgc agctgccggg aaagaaggaa acgacgactc cgggggcgaa     120

cttggcacac agggaggaag ggaaaggatg catggtcatg gaggctatga ttctgatttt     180

agtgatgatg aacgctgtgg agaatccagc aaaaggaaaa aaaggacagt tgaagatgac     240

ttactgctcc aaaaaccatt tcagaaagaa aaacatggaa aggtggccca taaacaagtt     300

gcagcagaat tgctggatag ggaagaagca agaaatagaa ggtttcatct catagctatg     360

gatgcttatc aaagacatac aaagttcgta aatgactata ttttatacta tggaggcaaa     420

aaagaagact tcaagcgttt gggggaaaat gacaagacag acttggatgt tatacgagaa     480

aatcatagat tcctatggaa tgaggaggac gaaatggaca tgacttggga gaagagactt     540

gctaagaaat actatgataa attatctaag gaatactgca tagcagatct cagtaaatat     600

aaagaaaata agtt                                                       614

<210>  280

<211>  640

<212>  DNA

<213>  Homo sapiens


<400>  280
gcttggaggt accagatccc tgctctggcc caggcaggtt accgggtcct agctatggac      60

atgaaaggct atggagagtc atctgctcct cccgaaatag aagaatattg catggaagtg     120

ttatgtaagg agatggtaac cttcctggat aaactgggcc tctctcaagc agtgttcatt 180

ggccatgact ggggtggcat gctggtgtgg tacatggctc tcttctaccc cgagagagtg 240

agggcggtgg ccagtttgaa tactcccttc ataccagcaa atcccaacat gtcccctttg 300

gagagtatca aagccaaccc agtatttgat taccagctct acttccaaga accaggagtg 360

gctgaggctg aactggaaca gaacctgagt cggactttca aaagcctctt cagagcaagc 420

gatgagagtg ttttatccat gcataaagtc tgtgaagcgg gaggactttt tgtaaatagc 480

ccagaagagc ccagcctcag caggatggtc actgaggagg aaatccagtt ctatgtgcag 540

cagttcaaga agtctggttt cagaggtcct ctaaactggt accgaaacat ggaaaggaac 600

tggaagtggg cttgcaaaag cttgggacgg aagatcctga 640

<210> 281

<211> 522

<212> DNA

<213> Homo sapiens

<400> 281
gggaaccacg tgcagaggct gcaggtggcg ggcagcgggc ccacggactc actgcagggc 60

atcatcctga ccggctggca gaggtacgac cactactctg tgctgtgcga gctgctgccc 120

gcaggagtcc cgtccctggc cgcctgcctg cagttgcttc tacgcggtat gtctggtctg 180

gccaccccaa gccccaccgg cccctccccg aaagacccgg agggcaggag gccaagcacc 240

cagtgctcca accaccctgc tggactgtgg tggtcctcac agtcggagga tggtctcacc 300

gccccagctc agccacccac ctgccctgaa gtccacccag gctgggcctg tgtctgggtc 360

tccaggccta agcccaaagc aggctggaga gtgacgcggg aggcacccct cagacacagt 420

gaatccgcag aaatgcacgc aggtcggagg tggggaacgg ctccaccttt gctgcacaca 480

cgtcctgccc acctggccga gcaggtgtgg ggctcatgcg cc 522

<210> 282

<211> 357

<212> DNA

<213> Homo sapiens

<400> 282
gccaccctcc gctccagcca gctgctctgg gaggccctgg agagcctcgt gaaccgggcc 60

gtgctcctgg ccagcgatgc ccatgaatgc accctggagg aagtggttga gcggctcctg 120

tctgtcaagg ggcgacccat accgagcccc ctggtcaagg cccataaaag cgtccaggcc 180

aacctatacc agagccagag ggcagctccc ccgcaaaaca ctacaacccc caagcccagc 240

gtggagggcc agcagccagc agcagctgct gcctgcgagc ccgtggacca cgcccagagt 300
gagagcatcc tgaaagtttc gcagcccata gccctggagc agcaggcgtc cacccca 357

<210> 283
<211> 564
<212> DNA
<213> Homo sapiens

<400> 283
gcccacgcgt ccggcctggg caggaggctc cagttctccc taaggatggt gcagtcaatg 60
gaccgtcagt tgtaggagat cagactccta ttgaaccaca gacttctata gaaagactga 120
cagaaacaaa agatggctca ggactagagg agaaggtcag ggcaaagctg gttcctagtc 180
aagaggagac taagctgtct gtagaagagt ctgaggcagc tggagatggg gttgatacca 240
aggtagccca gggagctact gagaaatcac ctgaagacaa agttcagata gctgctaatg 300
aagagacaca agagagagaa gaacagatga aagagggtga agaaactgaa ggctcggaag 360
aggatgataa agaaaatgat aagactgaag aaatgccaaa tgattcagtc cttgaaaaca 420
agtctcttca agaaaatgag gaggaggaga ttgggaacct atagcttgcc tgggatatgc 480
tggatttagc aaagatcatt tttaaaaggc aagaaacaaa agaagcacag ctttatgctg 540
cccaggcaca tcttaaactc ggag 564

<210> 284
<211> 610
<212> DNA
<213> Homo sapiens

<400> 284
gggaggaggg aggccgggac aaggcgccgg tgcagcccca gcagtctcca gcggcggccc 60
ccggcggcac ggacgagaag ccgagcggca aggagcggcg ggatgccggg gacaaggaca 120
aagaacagga gctgtctgaa gaggataaac agcttcaaga tgaactggag atgctcgtgg 180
aacgactagg ggagaaggat acatccctgt atcgaccaac ttccatgact tcagtgccca 240
agcctctcaa atttctgcgt ccacactatg gcaaactgaa ggaaatctat gagaacatgg 300
cccctgggga gaataagcgt tttgctgctg acatcatctc cgttttggcc atgaccatga 360
gtggggagcg tgagtgcctc aagtatcggc tagtgggctc ccaggaggaa ttggcatcat 420
ggggtcatga gtatgtcagg catctggcag gagaagtggc taaggagtgg caggagctgg 480
atgacgcaga gaaggtccag cgggagcctc tgctcactct ggtgaaggaa atcgtcccct 540
ataacatggc ccacaatgca gagcatgagg cttgcgacct gcttatggaa attgagcagg 600
tggacatgct 610

<210>  285

<211>  572

<212>  DNA

<213>  Homo sapiens


<400>  285
gccccaaccc ggacccggca cttctcgggt tccgcgactg ccgatcgccc cggcgcggca      60

ccgctccctc aggagtcgcc taggccgcgc agtctcccga cttctcgtca ggctttcgcg     120

ccggcgctcc agcaatcact ggctggagaa ggtgggcgtt ccggctcgag aggaccctgc     180

cgcggctccg gaagagcctc gtcctgggcg gcggtggtgc ggcggtcgcc gttatggcca     240

ctgggctggg cggctgaccg ccgggctagg aaagggccca gggccccgaa tctcggtggc     300

cgctgctcca gcgcggcctg cgccatggcc tcctccgccg cctcctcgga gcatttcgag     360

aagctgcacg agatcttccg cggcctccat gaagacctgt ctttccgaaa ccccatgatg     420

tctaagcttc gaaactaccg gaaggacctt gctaaactcc atcgggaggt gagaagcaca     480

cctttgacag ccacacctgg aggccgagga gacatgaaat atggcatata tgctgtagag     540

aatgagcata tgaatcggct acagtctcaa ag                                   572

<210>  286

<211>  557

<212>  DNA

<213>  Homo sapiens


<220>

<221>  misc_feature

<222>  (457)..(457)

<223>  n = a, c, g or t


<400>  286
gcggacgcgt gggagtatgt ggacctcaag cggctgaaca cgacaggcgt gagcgccggc      60

tgcaccgccg acctgctggt ggggcgcgcc atgactttcc atggccacgg cttccttcgc     120

ctggcgctct cgaacgtggc accgctcact ggcaacgtct actccggctt cggcttccac     180

agcgcccagg acagtgccct gctctactac cgggcgtccc cggatgggct atgccaggtg     240

tccctgcagc agggccgtgt gagcctacag ctcctgagga ctgaagtgaa aactcaagcg     300

ggcttcgccg atggtgcccc ccattacgtc gccttctaca gcaatgccac gggagtctgg     360

ctgtatgtcg atgaccagct ccagcagatg aagccccacc ggggaccacc ccccgagctc     420

cagccgcagc ctgaggggcc cccgaggctc ctcctgngag gcctgcctga gtctggcacc 480

atttacaact tcagtggctg catcagcaac gtcttcgtgc agcggctcct gggcccacag 540

cgcgtatttg atctgca 557

<210> 287

<211> 588

<212> DNA

<213> Homo sapiens

<400> 287
gcgactggag ctcagccgcg cttgtccggt gctaggtcat cctgccaaac acccgaggcc 60

ccaaagatgg tgcaaagtga acttcagcta cagcccagag caggcggacg agctgaagct 120

gcaagctggg gagatcgtgg aaatgataaa ggagattgag gacggctggt ggctggggaa 180

gaagaacggg cagctgggag ccttcccatc caactttgtg gaattgctgg acagtgggcc 240

cccaagcctt ggtaacccag acatgccttc agtcagccct ggtccccagc ggcctcccaa 300

gctgagcagc ctggcctatg acagccctcc agactacctg cagacagacc acagaggata 360

agggctggtg ggaaggagag tgtcaaggac gaagaggagt ttttccagac aactttgtac 420

tcccaccacc cccaatcaag aagctggtcc cacggaaagt ggtatctcgg gaatcagctc 480

ctattaagga accaaaaaag ttgatgccca aaacatccct ccccacagtc aagaagctag 540

caacagccac cactgggccc agcaaagcca agtaaggagc aggatggg 588

<210> 288

<211> 578

<212> DNA

<213> Homo sapiens

<220>

<221> misc_feature

<222> (335)..(335)

<223> n = a, c, g or t

<220>

<221> misc_feature

<222> (486)..(486)

<223> n = a, c, g or t

<400> 288
ggagaaggca ccgcccccac cccgcctcca aagctaaccc tcgggcttga ggggaagagg 60

ctgactgtac gttccttcta ctctggcacc actctccagg ctgccatggg gcccagcacc 120

cctctcctca tcttgttcct tttgtcatgg tcgggacccc tccaaggaca gcagcaccac 180

cttgtggagt acatggaacg ccgactagct gctttagagg aacggctggc ccagtgccag 240

gaccagagta gtcggcatgc tgctgagctg cgggacttca agaacaagat gctgccactg 300

ctggaggtgg cagagaagga gcgggaggca ctcanaactg aggccgacac catctccggg 360

agagtggatc gtctggagcg ggaggtagac tatctggaga cccagaaccc agctctgccc 420

tgtgtagagt ttgatgagaa ggtgactgga ggccctggga ccaaaggcaa gggaagaagg 480

aatganaagt acgatatggt gacagactgt ggctacacaa tctctcaagt gagatcaatg 540

aagattctga agcgatttgg tggcccagct ggtctatg 578

<210> 289

<211> 596

<212> DNA

<213> Homo sapiens


<400> 289
ggtccccttc tacgcggcat ccagtggcca gaagaagcaa caacagtcga agcctcaggg 60

ggaggcccgt gtccgtctaa agggcggcgc ccaccctgga gagggccggg tagaagtcct 120

gaaggccagc acatggggca cagtctgtga ccgcaagtgg gacctgcatg cagccagcgt 180

ggtgtgtcgg gagctgggct tcgggagtgc tcgagaagct ctgagtggcg ctcgcatggg 240

gcagggcatg ggtgctatcc acctgagtga agttcgctgc tctggacagg agctctccct 300

ctggaagtgc ccccacaaga acatcacagc tgaggattgt tcacatagcc aggatgccgg 360

ggtccggtgc aacctacctt acactggggc agagaccagg atccgactca gtgggggccg 420

cagccaacat gaggggcgag tcgaggtgca aataggggga cctgggcccc ttcgctgggg 480

cctcatctgt ggggatgact gggggaccct ggaggccatg gtggcctgta ggcaactggg 540

tctgggctac gccaaccacg gcctgcagga gacctggtac tgggactctg ggaata 596

<210> 290

<211> 459

<212> DNA

<213> Homo sapiens


<400> 290
ggcgaagcag cgcgggcagc gagcgagatg cagcaccgag gcttcctcct cctcaccctc 60

ctcgccctac tggcgctcac ctccgcggtc gccaaaaaga aagataaggt gaagaagggc 120

ggcccgggga gcgagtgcgc tgagtgggcc tgggggccct gcacccccag cagcaaggat 180
tgcggcgtgg gtttccgcga gggcacctgc ggggcccaga cccagcgcat ccggtgcagg 240
gtgccctgca actggaagaa ggagtttgga gccgactgca agtacaagtt tgagaactgg 300
ggtgcgtgtg atgggggcac aggcaccaaa gtccgccaag gcaccctgaa gaaggcgcgc 360
tacaatgctc agtgccagga gaccatccgc gtcaccaagc cctgcacccc caagaccaaa 420
gcaaaggcca aaggtcagcg aaaggagaag ggggtgggg 459

<210> 291
<211> 572
<212> DNA
<213> Homo sapiens

<400> 291
ggccgcgact acctgctgcc cgacgtgcac gacagcgtcc tctaccgcct gtgcctgcag 60
ccgctgccgc tgcgcgctgg gcccgccgcc gccgcgccag agacccccga gccggccgag 120
tgcgtggagt tcaccgccga gccggccggc atgcaggaca tcgtggtggc catgacggcg 180
gtgggcggct ccatctgcgt catgctggtg gtcatctgcc tgctggtggc ctacatcacc 240
gagaacctca tgcgcccggc cctcgcgcgc cccggcctgc gcagacaccc ctgaagcgcg 300
aggccgcccg cccatctttg cgccacctgc ctgagggcgc ctgagctccc aggtcctccg 360
cccgctctcc tctcctcatt cctgctgcct ccttgagatc cccgcggagg aggacctgcc 420
ggcgacggct ccggcacttt ggcatcatca cacgcccttt ccagccgggc gtcagactcc 480
cagactctgg ccccggattg gcccctgggc ttgcgctgct gtgccagccg catccagggc 540
tctccccgct ctccgcaggc ctttatagag gc 572

<210> 292
<211> 599
<212> DNA
<213> Homo sapiens

<400> 292
gctcatatct gcaaagaaca gaccgttcag tatatactaa ctatggtgga tgatatgctg 60
caggaaaatc atcagcgtgt tagcattttc tttgactatg caagatgtag caagaacact 120
gcgtggccct actttctgcc aatgttgaat cgccaggatc ccttcactgt tcatatggca 180
gcaagaatta ttgccaagtt agcagcttgg ggaaaagaac tgatggaagg cagtgactta 240
aattactatt tcaattggat aaaaactcag ctgagttcac agagttcgca gtatgtgcag 300
tgcgtggccg ggtgtttgca gctgatgctc cgggtcaatg agtaccgctt tgcttgggtg 360
gaagcagatg gggtaaattg cataatggga gtgttgagta acaagtgtgg ctttcagctc 420

cagtatcaaa tgattttttc aatatggctc ctggcattca gtcctcaaat gtgtgaacac 480
ctgcggcgct ataatatcat tccagttctg tctgatatcc ttcaggagtc tgtcaaagag 540
aaagtaacaa gaatcattct tgcagcattt cgtaactttt tagaaaaatc aactgaaag 599

<210> 293
<211> 405
<212> DNA
<213> Homo sapiens

<400> 293
ggagcaaagc tccaaagaga attgtttttt ccagaatgcc aatactgggg ctcagagctc 60
cagctaccca ctttggattt tgaagatgtt ttaagatatg atgaacacgc atacaagtgg 120
ctctccaccc tcaagaaagt aggcatagta agactcaccg gagcatctga caaaccagga 180
gaagtttcaa aacttgggaa aaggatgggt ttcctctatc tcacatttta tggacatact 240
tggcaagtgc aagacaaaat cgatgcaaac aatgtggctt acacaactgg gaagctaagc 300
tttcacactg attatccagc cctccatcat ccacctgggg ttcagcttct tcactgcata 360
aagcaaacag tcacaggggg tgattcagaa attgcagatg ggttt 405

<210> 294
<211> 599
<212> DNA
<213> Homo sapiens

<400> 294
gtgaacttta ttgcaagcct ggaacctcac gtcatcatgt atattctctc ttccatttct 60
gaaggactta ctgcacttga caccatggta tgcacaggct gctgctcctg cctggaccac 120
attgtgacat acctcttcaa gcagctgtca cgtagcacca agaagaggac cacacccctg 180
aaccaggaga gcgaccgctt tctgcacatc atgcagcagc atccagagat gatccagcag 240
atgctgtcca cggtgctgaa catcatcatc tttgaagact gtaggaacca gtggtctatg 300
tcccgaccac tacttggctt gatattgctt aatgaaaagt attttctgga cctaagaaac 360
agtattgtga acagccagcc accggagaag cagcaggcca tgcacctgtg ttttgagaac 420
ctgatggaag gcatcgagcg aaatcttctt acgaaaaaca gagacaggtt cacccagaac 480
ctgtcagcat tccgtcgaga agtcaacgac tcgatgaaga attccactta tggcgtgaat 540
agcaatgaca tgatgagctg acacctcctt ggactctacc tgtacagagc agcgtccct 599

<210> 295
<211> 509

<212>  DNA

<213>  Homo sapiens


<220>

<221>  misc_feature

<222>  (276)..(276)

<223>  n = a, c, g or t


<400>  295
ggtccttaga agacgaccag agtggctcct atgcctctac ccactcatca gacagtgagg      60

aggaagaaga ggaggaggaa gaggaggccg ccttccctgg agagcagggc tgggatagcc     120

tgctggggcc tggagcagag agactgcccc tgcacagtac tcccaaggat gggggcccag     180

ggcctggcaa ggccccctgg ccaggagact ttgggaccac agcaaaagag agtagtggca     240

acggggcccc tgaggagcgg ctgcgggaga atgganatgc cctgtctcga gaggggtccc     300

taggccccct tccaggctct tctgcccagc ctcacaaagg catccttaag aagaagtgtc     360

tgcccaccat cagcgagaag agcagcctcc tgcggctccc cctggagcaa tgcacagggt     420

cttcccgggg ctcctccgct agtgagggca gccggggcgg cccccctccc cgcccaccgc     480

cccggcagag cctccaggag cagctgaac                                       509

<210>  296

<211>  555

<212>  DNA

<213>  Homo sapiens


<400>  296
gtttccctgc tgaagagtgg tcgcttctgt gacgtctttg acaaggccct ggtcctgtgc      60

cagatgcacg acttccagga tggtgtcctt tacctttatg agcaggggaa gctgttccag     120

cagatcatgc actaccacat gcagcacgag cagtaccggc aggtcatcag cgtgtgtgag     180

cgccatgggg agcaggaccc ctccttgtgg gagcaggccc tcagctactt cgctcgcaag     240

gaggaggact gcaaggagta tgtggcagct gtcctcaagc atatcgagaa caagaacctc     300

atgccacctc ttctagtggt gcagaccctg gcccacaact ccacagccac actctccgtc     360

atcagggact acctggtcca aaaactacag aaacagagcc agcagattgc acaggatgag     420

ctgcgggtgc ggcggtaccg agaggagacc acccgtatcc gccaggagat ccaagagctc     480

aaggccagtc ctaagatttt ccaaaagacc aagtgcagca tctgtaacag tgccttggag     540

ttgccctcag tccac                                                      555

<210> 297

<211> 430

<212> DNA

<213> Homo sapiens

<400> 297
gctgactatt ttgatgccct gtacaagagg acaggggtgg agccaggggc ccggcctcca 60

gggtttcctg tgctgagcgg ggatttcttc tcctatgcgg accgggagga tcattactgg 120

acaggctatt acacttcccg gcccttctac aagagcttag accgagtcct ggaagcccac 180

ctgcgggggg cagaggttct gtacagcctg gctgcagctc acgctcgccg ctctggtctg 240

gctggccggt acccactgtc tgatttcacc ctcctgacgg aagctcggcg cacattgggg 300

ctcttccagc atcacgatgc catcactggc acggccaagg aggctgtggt ggtggactat 360

ggggtcaggc ttctgcgctc ccttgtcaac ctgaagcagg tcatcattca tgcagcccac 420

tatctggtgc 430

<210> 298

<211> 167

<212> DNA

<213> Homo sapiens

<400> 298
gcccacgcgt ccggtacgcg cgagagcctt gcccatggcg aattgtggat gactgtggtg 60

gggcctttac gatgggtacc attggtggtg gtatctttca agcaatcaaa ggttttcgca 120

attctccagt gggagtaaac cacagactac gagggagttt gacagct 167

<210> 299

<211> 414

<212> DNA

<213> Homo sapiens

<400> 299
ggtgattctt gtctgatagc aatgtctgga cgtggtaagc aaggaggcaa agctcgcgcc 60

aaagcgaaaa cccgctcttc tcgcgctggt ctccagttcc cggtgggccg agtgcaccgc 120

ctgctccgta aaggcaacta cgcagagcgg gttggggcag gcgcgccggt gtacctggcg 180

gcggtgttag agtacctgac cgccgagatc ctggagctgg ccggcaacgc ggctcgcgac 240

aacaagaaga ctcgcataat cccgcgccac ttgcagctgg ccatccgcaa cgacgaggag 300

ctcaacaaac tgctaggccg ggtgaccatt gctcaaggcg gcgtccttac taacatccag 360

gccgtgcttc tgcctaagaa gaccgagagt caccacaagg ccaagggcaa gtga 414

<210> 300

<211> 634

<212> DNA

<213> Homo sapiens

<220>

<221> misc_feature

<222> (306)..(306)

<223> n = a, c, g or t

<400> 300
gcaagaagat tgctgggaca gcgaccggga ccatatgata gaccaatagg aggaagaggg 60

ggttattatg gagctgggcg tggaagtatg tatgacagaa tgcgacgagg aggtgatgga 120

tatgatggtg gttatggagg ttttgatgac tatggtggct ataataatta cggctatgtt 180

tttgatggct ttgatgacag aatgagagat ggaagaggtg tgggaggaca tggctatggt 240

ggagctggtg atgcaagttc aggttttcat ggtggtcatt tcgtacatat gagagggttg 300

cctttncgtg caactgaaaa tgacattgct aatttcttct caccactaaa tccaatacga 360

gttcatattg atattggagc tgatggcaga gccacaggag aagcagatgt agagtttgtg 420

acacatgaag atgcagtagc tgccatgtct aaagataaaa ataacatgca acatcgatat 480

attgaactct tcttgaattc tactcctgga ggcggctctg gcatgggagg ttctggaatg 540

ggaggctacg gaagagatgg aatggataat cagggaggct atggatcagt tggaagaatg 600

ggaatgggga acaattacag tggaggatat ggta 634

<210> 301

<211> 570

<212> DNA

<213> Homo sapiens

<400> 301
ggtgaaggga gaagctcttg cagcaggccg gatgcccacc gctgcttcag ctgcctcctc 60

gcccagctac cctttggccc cattgggccc tcgtctgcct ctccaggatt gtatgttctc 120

aagccttgtc ctgtgttcct ttgtctgatg ctctgtgtat tgctctttga atcgagtttg 180

gaggaatagt tgagttgtat gagtggcggc atgttggtag tgccggactt cctgtttcaa 240

gttttctggg gcctcgctaa ttgaatgtgg aaagtagcac cacttgacgg ctacaagtgc 300

cgactcctga attttcccat ggtgttctga cttcaagggc tggcagccag ggagaatggg 360

cccaggggaa gcaaagacct cttccctctg ccgtttctgt cccacttaac tgacctcact 420

ggaggctaca tcacccaaag tagatgttag aaaacctaaa ttaatgaacc atatttttaa 480

aatcctattt ttcccaaaca gggccctctg cagcccatcc tttccttccg tccttctgaa 540

accacatacc ccaggcccaa gcgccttgct 570

<210> 302

<211> 457

<212> DNA

<213> Homo sapiens

<400> 302
gggcgcctca cggcccgggt agtcttacga ccctggtgcc ctgggctgcc gccctgctcc 60

tcgctctggg cgtggaaagg gctctggcgc tacccgagat atgcacccaa tgtccaggga 120

gcgtgcaaaa tttgtaaaag tggcctttat tgaaaacgac acgagagcaa tgcttttgcc 180

cgttgctgcc tgaatcagaa gggcaccatg ttggggctgg atctccagaa ctgttctctg 240

gaggaccctg gtccaaactt tcatcaggca cataccactg tcatcataga cctgcaagca 300

aaccccctca aaggtgactt ggccaacacc ttccgtggct ttactcagct ccagactctg 360

atactgccac aacatgtcaa ctgtcctgga ggaattaatg cctggaatac tatcacctct 420

tatatagaca accaaatctg tcaagggcaa aagaacc 457

<210> 303

<211> 660

<212> DNA

<213> Homo sapiens

<400> 303
ggctgccggc aggacctttc tctcgctgcc gctgggaccc cgtgtcatcg cccaggccga 60

gcacggaaat ctactttctt caatgtgtta accaatagtc aggcttcagc agaaaacttc 120

ccgttctgca ctattgatcc taatgagagc agagtacctg tgccagatga aaggtttgac 180

tttctttgtc aataccacaa accagcaagc aaaattcctg cctttctaaa tgtggtggat 240

attgctggcc ttgtgaaagg agctcacaat gggcagggcc tggggaatgc ttttttatct 300

catattagtg cctgtgatgg catctttcat ctaacacgtg cttttgaaga tgatgatatc 360

acgcacgttg aaggaagtgt agatcctatt cgagatatag aaataataca tgaagagctt 420

cagcttaaag atgaggaaat gattgggccc attatagata aactagaaaa ggtggctgtg 480

agaggaggag ataaaaaact aaaacctgaa tatgatataa tgtgcaaagt aaaatcctgg 540

gttatagatc aaaagaaacc tgttcgcttc tatcatgatt ggaatgacaa agagattgaa 600

gtgttgaata aacacttatt tttgacttca aaaccaatgg tctacttggt taatctttct 660

<210> 304

<211> 200

<212> DNA

<213> Homo sapiens

<400> 304
gggagcgggg ggcccgcgcc ccgcgccccc cagacatggt gggccacctg catctgcagg 60

gcatggagga cagcctgaag gagcagggcc gggagggctt gctggacagc cccgactccg 120

ggctgccccc cagccccagc cccagcccgc ccttctactc cctggcgccc ggcatcctcg 180

acgcgcgcgc ggggggcgcc 200

<210> 305

<211> 559

<212> DNA

<213> Homo sapiens

<400> 305
gctagaattc ccctctccct ccctagatct cacccccagg atgttgcgga ggctgctgga 60

gcggccttgc acgctggccc tgcttgtggg ctcccagctg gctgtcatga tgtacctgtc 120

actggggggc ttccgaagtc tcagtgccct actgtccaga acgatctcct ctcttagtgg 180

gtcctgtgtc agtgtccttt agcccagtgc catcactggc agagattgtg gagcggaatc 240

cccgggtaga accagggggc cggtaccgcc ctgcaggttg tgagccccgc tcccgaacag 300

ccatcattgt gcctcatcgt gcccgggagc accacctgcg cctgctgctc taccacctgc 360

accccttctt gcagcgccag cagcttgctt atggcatcta tgtcatccac caggctggaa 420

atggaacatt taacagggca aaactgttga acgttggggt gcgagaggcc ctgcgtgatg 480

aagagtggga ctgcctgttc ttgcacgatg tggacctctt gccagaaaat gaccacaatc 540

tgtatgtgtg tgaccccg 559

<210> 306

<211> 529

<212> DNA

<213> Homo sapiens

<400> 306
gaagcctgtc actcaacagg tacgacaagg ttaggtcttc tgaatcatta atgagtgacc 60

agtgtccatg tttatgcatt aaagaattat gggttctact tattcatctt ctagaccaca 120

gaagtaaatg gtttgtctcg gaatcatttt ggaactggtt gaataaacta cttaaaacac 180
tgcttgaaaa atcaagtgac cgaagaagat cctctatgcc tgtaatccag tccagggatc 240
cattaggttt tagttggtgg attattactc atgtagcatc attttacaag tttgatcgcc 300
atggagtacc agatgaaatg agaaaagtgg aatcaaattg gaactttgta gaagaactgc 360
tgaaaaagtc catcagtgtt cagggtgtca ttctagaaga acaattacga atgtatcttc 420
actgttgttt gacactttgt gatttctggg agccaaacat tgcaattgtt accattttat 480
gggaatatta tagtaagaac ctgaatagtt ccttcagtat ttcttggct 529

<210> 307
<211> 410
<212> DNA
<213> Homo sapiens

<400> 307
gcgcggagca ggtggccgcg gcggggcagc tgggccgcca gcttggtgcc tcggggaccg 60
tctcccgctg ctttggtcac cagcccctgc ccgcccgacc cgctccgttc tccggcctgc 120
gagccctgcc ggccggactt tgcgccgcgt ccggcgctgc tgctgcgctc ggggccccgc 180
tcggcgccgg cggtgaccgg gaagcccgcg ttaaaggggc aaccgggacc ctggcccggt 240
atggctgaag tcagcatcga ccagtccaag ctgcctggag tcaaggaagt atgccgagat 300
tttgctgtcc tggaggacca caccctgcct cacagcctgc aggaacaaga gattgagcat 360
catttggcat cgaacgttca gcggaaccgt ttggcccagc atgatctcca 410

<210> 308
<211> 557
<212> DNA
<213> Homo sapiens

<220>
<221> misc_feature
<222> (458)..(458)
<223> n = a, c, g or t

<400> 308
gcgggagcgc gcagtggcgg cagcggcggc gacggcagta acagcggcag ctacagcggg 60
gacgcgagcg gggcggtgac ggtgtgggag gtggtctcac tcttgggaaa actgctgggc 120
accgtcgtcg cgctgaaggt ggttctgtac ctgctccgag tgtgcttagc gatggcctgg 180

aaatccggcg gcgccagcca ctcggagcta atccacaatc tccgcaaaaa tggaatcatc 240
aagacagata aagtatttga agtgatgctg gctacagacc gctcccacta tgcaaaatgt 300
aacccataca tggattctcc acaatcaata ggtttccaag caacaatcag tgctccacac 360
atgcatgcat atgcgctaga acttctattt gatcagttgc atgaaggagc taaagctctt 420
gatgtaggat ctggaagtgg aatccttact gcatgttntg cacgtatggt tggatgtact 480
ggaaaagtca taggaattga tcacattaaa gagctagtag atgactcagt aaataatgtc 540
aggaaggacg atccaac 557

<210> 309
<211> 622
<212> DNA
<213> Homo sapiens

<400> 309
ggggggttct gcttccttga atatgaggat cacaagtcag cagcacaagc cagacgccgg 60
ctgatgagtg gaaaagtaaa agtgtgggga aatgtagtta cagttgaatg ggctgaccct 120
gtggaagaac cagatccaga agtcatggct aaggtaaaag ttttgtttgt gagaaacttg 180
gctactacgg tgacagaaga aatattggaa aagtcatttt ctgaatttgg aaaactcgaa 240
agagtaaaga agttgaaaga ttatgcattt gttcattttg aagacagagg agcagctgtt 300
aaggctatgg atgaaatgaa tggcaaagaa atagaagggg aagaaattga aatagtctta 360
gccaagccac cagacaagaa aaggaaagag cgccaagctg ctagacaggc ctccagaagc 420
actgcgtatg aagattatta ctaccaccct cctcctcgca tgccacctcc aattagaggt 480
cggggtcgtg gtggggggag aggtggatat ggctaccctc cagattacta cggctatgaa 540
gattactatg atgattacta tggttatgat tatcacgact atcgtggagg ctatgaagat 600
ccctactacg gctatgatga tg 622

<210> 310
<211> 502
<212> DNA
<213> Homo sapiens

<400> 310
gcggacgcgt gggcgggctg cccctcggtt gacaatggtc tccaggatgg tctctaccat 60
gctatctggc ctactgtttt ggctggcatc tggatggact ccagcatttg cttacagccc 120
ccggacccct gaccgggtct cagaagcaga tatccagagg ctgcttcatg gtgttatgga 180
gcaattgggc attgccaggc cccgagtgga atatccagct caccaggcca tgaatcttgt 240
gggcccccag agcattgaag gtggagctca tgaaggactt cagcatttgg gtccttttgg 300

caacatcccc aacatcgtgg cagagttgac tggagacaac attcctaagg actttagtga 360

ggatcagggg tacccagacc ctccaaatcc ctgtcctgtt ggaaaaacag cagatgatgg 420

atgtctagaa aacacccctg acactgcaga gttcagtcga gagttccagt tgcaccagca 480

tctctttgat ccggaacatg ac 502

<210> 311

<211> 350

<212> DNA

<213> Homo sapiens

<400> 311
gcgcgcctcg acccggcccc gcgcagcccg ggccctgcct attcctcagc aaccccacgc 60

gtgacaccat cacgccgctg tgggccgcgc ccggccgcgc cgcccgcccg ggacgagcct 120

gctccgccgc ctgctccgag gaggaggacg aagaggacga cgaggaagag gaggatgccg 180

aggacagtgc ggggtccccc gggggcaggg gcacgggccc ctcggcgccg cgggacgcgt 240

cgctggtgta cgacgcggtc aagtacacgc tggtggcgga tgaacacacg cagctggagc 300

tggtgagcct gcggcgctgt gctgggctgg gccacgacag cgaagaggac 350

<210> 312

<211> 354

<212> DNA

<213> Homo sapiens

<400> 312
gcgggagagt agcagtgcct tggaccccag ctctcctccc cctttctctc taaggatggc 60

ccagaaggag aactcctacc cctggcccta cggccgacag acggctccat ctggcctgag 120

caccctgccc cagcgagtcc tccggaaaga gcctgtcacc ccatctgcac ttgtcctcat 180

gagccgctcc aatgtccagc ccacagctgc ccctggccag aaggtgatgg agaatagcag 240

tgggacaccc gacatcttaa cgcggcactt cacaattgat gactttgaga ttgggcgtcc 300

tctgggcaaa ggcaagtttg gaaacgtgta cttggctcgg gagaagaaaa gcca 354

<210> 313

<211> 555

<212> DNA

<213> Homo sapiens

<400> 313

gcgggaaact cggggacccc ggctcggacc tgcccccgcc cccgccgccg cccctggccc 60

cccagaaggc gagtggcggc ggcagcagca gcccgggcag cccagttcac catccatcac 120

tggaggagca gccctcctac aaagatagtc agaaaactaa ggaaaataac caagtaattg 180

tatctacaaa ggatgacaac agcttttcag ataagaacaa ggagcatagc tttttcatca 240

cagactctga tgcttctgga ggagattttt ggagagaaag atcaggtgaa catacacaag 300

aaaccaactc acctcattca ctgaaaaagg atgtagaaaa tatggggaaa gaagaacttc 360

agaaggtttt atttgaacaa atagatttac ggagacgact ggaacaagaa ttccaggtgt 420

taaaaggaaa cacatctttc ccagtattca ataattttca ggatcagatg aaaagggagc 480

tagcctaccg agaagaaatg gtgcaacagt tacaaattat cccctatgca gcaagtttga 540

tcaggaaaga aaagc 555

<210> 314

<211> 461

<212> DNA

<213> Homo sapiens

<400> 314
gtccggattg gagggagcac agatacaggc aaacatatca aggagaatga ctattatact 60

ccaactgggg agttccgtgt ggaccgtgaa ggttctccag tgctgctcaa ctgcctcatg 120

tacaagatgt gttactatcg ctttggacag gtttacacag aagccaagcg tcctccaggc 180

tttgaccgtg tccgaaatgc tgagattggg aataaagact ttgagcttga tgtcctggag 240

gaagcatata ccacagaaca ttggctggtc aggatataca aggtaaagga cctggataat 300

cgaggcttgt caaggacata aatgtcacgt ccagctctga tatgcttcgc actgagcaca 360

tcacatttag gacgttgaag attttttttt tttttttttt tttaatatgc agtttgtaag 420

aacaaaactg gatggcatca gaattgtctg gaagttttgt c 461

<210> 315

<211> 160

<212> DNA

<213> Homo sapiens

<400> 315
ggtcctctgg gccctgatgc caactttact gtccaggaca accgaggtag gtggcctcct 60

acagccatgt ccatacctgc ctctgttccc ctctcctcct cctccacccc tagcccagcc 120

ttttacatgg agacaggccg cccgctcccg gccctgctgg 160

<210> 316

<211>  430

<212>  DNA

<213>  Homo sapiens


<400>  316
gaaaaaatcc ctccccggcg gcggcggcgg cggcggcggc ggcggcggcg gcgccggcgg      60

tggtggcggc cccggggctg agcgctcggc tgcagcggcg cggaggccgt ctccctggtc     120

tgccgcggtc cccgcccgtc ccgccgccgg ctgccatggc aggagccgga ggcggcggct     180

gccccgcggg cggcaacgac ttccagtggt gcttctcgca ggtcaagggg gccatcgacg     240

aggacgtggc cgaagcggac atcatttcca ccgttgagtt taattactct ggagatcttc     300

ttgcaacagg agacaagggc ggcagagttg ttatttttca gcgtgaacaa gagaataaaa     360

gccgccctca ttctagggga gaatataatg tttacagcac ctttcaaagt catgaacccg     420

gagtttgact                                                             430

<210>  317

<211>  487

<212>  DNA

<213>  Homo sapiens


<400>  317
gatctgcagc aaagcacggg ggcgagagac aagaagcatg ccacctccac aactcctacc      60

tggagatgga tctgctgacc gtgccaagga accaaaatga atctgtatca gaaatcggtg     120

ggaagatatt tgagaaggct gtaaagagac tctctagcat tgatggtctt caccaaatta     180

gctctatcgt cccctttctg acggattcca gctgctgtgg ataccataaa gcatcctact     240

accttgcagt cttttatgag actggattaa atgttcctcg ggatcagctg cagggcatgt     300

tgtatagttt ggttggaggc caggggagtg agaggctgtc ttcaatgaat cttgggtata     360

aacactacca gggtattgac aactaccccc tggactggga actgtcgtat gcctactaca     420

gcaacattgc caccaagaca ccccttgacc agcacacact gcaaggagat caggcatatg     480

ttgaaac                                                                487

<210>  318

<211>  583

<212>  DNA

<213>  Homo sapiens


<400>  318
gcttttcagg gcccagcact agaagatttc agtcatctgc caccagaaca gagacgtaaa      60

aaactacagc agcgcattga tgaacttaac agagaactac agaaagaatc agaccaaaaa 120
gatgcactca acaaaatgaa agatgtatat gagaagaatc cacaaatggg ggatccaggg 180
agtttgcagc ctaaattagc agagaccatg aataacattg accgcctacg aatggaaatc 240
cataagaatg aggcttggct ctctgaagtc gaaggcaaaa caggtgggag aggagacaga 300
agacatagca gtgacataaa tcatcttgta acacagggac gagaaagtcc tgagggaagt 360
tacactgatg atgcaaacca ggaagtccgt gggccacccc agcagcatgg tcaccacaat 420
gagtttgatg atgaatttga ggatgatgat cccttgcctg ctattggaca ctgcaaagct 480
atctaccctt ttgatggaca taatgaaggt actctagcaa tgaaagaagg tgaagttctc 540
tacattatag aggaggacaa aggtgacgga tggacaagag ctc 583

<210> 319
<211> 514
<212> DNA
<213> Homo sapiens

<400> 319
ggggtgctgg agaacctcaa acagcagctg gaggaacagg aatacaagct gcagaaggag 60
cagaatttga acatgcaact tttcagtgag atccataatc tgcagaataa gttcagagat 120
ctctcacctc ccagatacga ttcattagtt cagtcccaag ccagggagct ctcccttcaa 180
cggcagcaga ttaaggatgg ccatggcatc tgtgtcatct cccgtcaaca catgaacacc 240
atgattaagg catttgagga gttgctgcag gccagtgatg tggattactg tgtggccgag 300
ggtttccagg aacagctgaa tcaatgtgct gagctgctgg agaaattgga aaagctattt 360
ctcaacggaa aatcagttgg agtggaaatg aacacccaga atgaactgat ggagaggatt 420
gaggaagaca acttaaccta ccaacatctt ctgcctgaat ctcctgagcc ttcagcctct 480
catgcgctct ctgattatga aacatctgaa aagt 514

<210> 320
<211> 476
<212> DNA
<213> Homo sapiens

<400> 320
gagaacacct tgctgctggg gaagaaggtg gtccttgtac cctacacctc ggagcatgtc 60
cccaggtacc acgagtggat gaaatcagag gagctgcagc gtttgacagc ctcggagccg 120
ctgaccctgg agcaggagta tgccatgcag cgcagctggc aggaagatgc agacaagtgt 180
accttcattg tgctggatgc cgagaagtgg caggcccagc caggcgccac cgaagagagc 240
tgcatggtgg gagatgtgaa cctcttcctc acagatctag aagacctcac cttgggggag 300

atcgaggtca tgattgcaga gcccagctgc aggggtaagg gccttggcac tgaggccgtt 360
ctcgcgatgc tgtcttacga aacttcactt tgagcaggtg gctacgagca gtgtttttca 420
ggaggtgacc ctcagactga cagtgagtga gtccgagcat cagtggcttc tggagc 476

<210> 321
<211> 553
<212> DNA
<213> Homo sapiens

<400> 321
gcccacgcgt ccgatcgtat ctttccggca gcttcgggag gaggtgctag gagaactgtc 60
aaatgtggag caagcagctg gcctccgctg gagccgcttt ccagacctca atcgtatctt 120
gaagggacat cgaaagggcg agctgacggt cttcacaggg ccaacaggca gtggaaagac 180
gacattcatc agtgagtatg ccctggattt gtgttcccag ggggtgaaca cactgtgggg 240
tagcttcgag atcagcaatg tgagactagc ccgggtcatg ctgacacagt ttgccgaggg 300
gcggctggaa gatcaactgg acaaatatga tcactgggct gaccgctttg aggacctgcc 360
cctctatttc atgactttcc atggacagca aagcatcagg actgtaatag atacaatgca 420
acatgcagtc tacgtctatg acatttgtca tgtgatcatc gacaacctgc agttcatgat 480
gggtcacgag cagctgtcca cagacaggat cgcagctcaa gactacatca tcggggtctt 540
tcggaagttt gca 553

<210> 322
<211> 486
<212> DNA
<213> Homo sapiens

<400> 322
gcccacgcgt ccgcccacgc gtccgcccac gcgtccgtga aggtgttcgt ctgctgcgcc 60
tccacatcgc actccgggga gaagccggtc cccactctcc cccagttcac catgggcccc 120
aatatgaaga tcaacgtgct ggaagacttt gagggagaga accctcaggt gcccaagctt 180
gagaagagca tcagcgggac cagccccaaa cgggaacacc tgcccctggc cgtgggcatc 240
gccttcttcc tcatgacgtt cttggcctcc tagctctgcc ccctcccctg gggggggaga 300
gatggggcgg ggcttggaag gagcagggag cctttggcct ctccaaggga agcctagtgg 360
gcctagaccc ctcctcccat ggctagaagt ggggcctgca ccatacatct gtgtccgccc 420
cctctacccc ttccccccac gtagggcact gtagtggacc aagcacgggg acagccatgg 480
gtcccg 486

<210>  323

<211>  558

<212>  DNA

<213>  Homo sapiens

<400>  323
gggggatccg cgccatggag gccgcccggg actatgcagg agccctcatc aggcccctga      60

catttatggg atcacagact aagcgagtcc tgttcacccc gctcatgcat ccagctcgcc     120

ctttccgggt ctccaaccat gacaggagca gccggcgtgg ggtgatggca agcagcctgc     180

aggagctcat cagcaagact ctggatgccc tcgtcatcgc taccggactg gtcactctgg     240

tgctggagga agatggcacc gtggtggaca cagaagagtt ctttcagacc ttgggagaca     300

acacgcattt catgatcttg gaaaaaggac agaagtggat gccgggcagc cagcacttcc     360

ccacttgctc gccgccgaag aggtcgggaa tagcgagagt caccttcgac ttgtacaggc     420

tgaaccccaa ggacttcatc ggctgcctta acgtgaaggc caccatgtat gagatgtact     480

ccgtgtccta cgacatccgg tgcacgggac tcaagggcct gctgaggagt ctgctgcggt     540

tcctgtccta ctccgccc                                                   558

<210>  324

<211>  427

<212>  DNA

<213>  Homo sapiens

<400>  324
ggttccggca ggcgctgcag ctggcggcct gcgggctggc cgggggctcg gccgccgtgc      60

tcttctcggc cgtggcggta gggaagccgc gcgcaggcgg ggacgcggag ccacgcccgg     120

ctgagccgcc ggcctgggcg gggggcgcgc ggccgggccc cggtgtctgg gaccccaact     180

gggacaggcg agaaccactg tctctgatca acgtgcggaa gaggaacgtg gaatctgggg     240

aagaagagct ggcgtccaag ctggaccact acaaagccaa ggccacgcgg cacatcttcc     300

tcatcaggca ttcccagtac cacgtggatg gctccctgga gaaggaccgc actctgaccc     360

cgctgggtcg ggagcaggct gaactcactg ggctccgcct ggcaagcttg gggttgaagt    420

ttaataa                                                               427

<210>  325

<211>  505

<212>  DNA

<213>  Homo sapiens

<400> 325
gctcgcctgc cgtctaggag ggaaggggcc ttggatctgt gccccgccca tggaacagcg 60
cccgggctcg gagaggcccc gcgaggccgc aggcgcagac cctcccggag ggctgggcgg 120
gcaccggggt cttcggctgg cttgagggac ccgacccctg ggggaggaat ttggggtctg 180
tgtgtccgaa tgaacgcggg ggttcagatg agtctgtggt gcccccgctt tccatctcct 240
ccggccccgg ccccagagga ctgtctgaga ggccggtggc cgcggatcct ggacgcgcct 300
ccctcctgcc tgtggtttcc ctttattccg gcagcggcgc gagcccaccc ttcacataca 360
ggagctacat ccagaagctt ctcctggggc cacagcgtgg gcttccgaga ctctgcgtga 420
ccccagctct gcccctttgg ggctgcgggt gaagccctca ggaccctccg cgtgcgttgg 480
gcaggtgact ggatgaggga taatg 505

<210> 326
<211> 557
<212> DNA
<213> Homo sapiens

<400> 326
gtttctgaag gaatgaaagt tgagcaggaa tcttcgtctg atgataacct tgaagacttt 60
gatgtagagg aagaagatga agaagcccta atagaacaga gaagaatcca aaggcaggca 120
attgttcaga aatataaata ccttgctgaa gatagcaaca tgtctgtgcc atctgaacca 180
agcagccccc agagcagtac gagaacacga tcaccatctc cagatgacat tctggagcga 240
gtagctgctg atgttaaaga gtatgaacgg gaaaatgttg atacatttga ggcctcagtg 300
aaagccaagc ataatctaat gacagttgaa cagaataatg gttcatctca gaagaagttg 360
ttggcacctg atatgtttac agaatctgat gatatgtttg ctgcgtattt tgatagtgct 420
cgtcttcggg ccgctggcat tggaaaagat ttcaaagaga atcccaacct cagagataac 480
tggaccgatg cagaaggcta ttatcgtgtg aacataggtg aagtcctaga taaacgttac 540
aatgtgtatg gctacac 557

<210> 327
<211> 568
<212> DNA
<213> Homo sapiens

<400> 327
gggaggagga gctggaggtg ctgctggagg aggaggcagc cggcggcgat gaggaccggg 60
aaaaggagat tctcattgaa cggatccagt ccatcaagga ggagaaggag gacatcacct 120

accggctgcc ggagctggac ccaaggggct cggacgagga gaacctggac tcggagacgt 180
cggccagcac cgagagcctg ctggaggagc gggccgggcg gggggcctcg gaaggtcagt 240
attaagggcc ccctgcgcct gctctccctt gccccggcgc gcccaccccg agccccctcc 300
ccaccgtggc cgcccctcca cgacgaaggc cgtcgtcctt cgtaacggtc agagtgaaga 360
ccccccggcg gacccccatc atgcccacgg ccaacatcaa gctcccacca ggcctgccct 420
cccacctgcc tcgctgggca ccgggtgccc gggaggcggc tgccccagtg cggcgccggg 480
agccacctgc ccgccgcccg gaccagatac attccgtgta catcacgccc ggggcagacc 540
tgccagtgca gggcgccctg gagcccct 568

<210> 328
<211> 515
<212> DNA
<213> Homo sapiens

<400> 328
gcggacgcgt gggcaggagg tgggtgggca cattcctggg gtgcttatga gagcgggcct 60
ggggagggcg tccgggctcc ttggaagata ctgagagatg ctggaatatt actgggattt 120
ttctgggaag ctgatgatgt ttctgacatc gctagaatat taatggaaat tcgggggccg 180
ggagatagga atcccggaga ctccgagtcg ttacttggaa aattcctggg tggccggtcc 240
tcttccacct cagggcacag ctggctaccg gtgtggaaac acctgccgag acgtgggggg 300
tatgggaaca tcagaaagtc tagagttaac aggaatcgga ggacagatcg tgggaaagcg 360
gaggctcgct aacgaccctc gaagggacac ccctgcgagg ctaacggaaa cccagactca 420
cccaggggcc gcagccgggg tccactccgc gccaacaccg ctagcgttcc gcccggctcc 480
gcaggcacgg cccctttaaa ttattcactc ctagt 515

<210> 329
<211> 562
<212> DNA
<213> Homo sapiens

<400> 329
gggcgccctg tgtgtcctac ggcggagcag tttcgtaccg gcttcttctc tggggtgggg 60
gtagcctcgc ccggaagcaa ggcctctgga aaaccgcggc ccctgagttg caaacaaatg 120
tcagatccca gatattaagg ctaagacata ctgcatttgt aataccaaag aaaaacgttc 180
ctacctcaaa acgtgaaact tacacagagg attttattaa aaagcagatt gaagagttca 240
acataggaaa gagacattta gccaacatga tgggagaaga tccagaaact ttcactcaag 300
aagatattga cagagctatt gcttaccttt tcccaagtgg tttgtttgag aaacgagcca 360

ggccagtaat gaagcatcct gaacagattt ttccaagaca aagagcaatc cagtggggag 420

aagatggccg tccatttcac tatctcttct atactggcaa acagtcatac tattcattaa 480

tgcatgatgt atatggaatg ttactcaatt tagaaaaaca tcaaagtcac ttgcaagcca 540

aaagtctgct cccagaaaaa ac 562

<210> 330

<211> 452

<212> DNA

<213> Homo sapiens

<400> 330
ggcaggtggc acccagagct gcgcgccgcg gcggcggaag atggctgcgg ccgaggtggc 60

ggacactcag ctgatgcttg gagtcgggct gatcgtgacc atctttccac cgtagaaaag 120

gacacaaatg gagaagttct gtgggtgtgg tgttatcctt ccacgacagc cacattaagg 180

aacctgctgc tgagaaaatg ctgccttaca gatgaaaaca aacttctcca tccctttgtc 240

tttggtcagt acagaagaac atggttttat atcacaacaa ttgaagttcc agattcttcc 300

attttgaaaa aggtgactca tttttctatt gtcctgaccg ccaaagattt taacccagag 360

aagtatgctg ccttcactag gatattgtgt agaatgtacc tgaaacatgg gagcccagtt 420

aaaatgatgg agagttatat tgcagttctc ac 452

<210> 331

<211> 534

<212> DNA

<213> Homo sapiens

<400> 331
ggcggttgcg gcggcagcgg ctgtggttcg agggacaggc tctcacccct ggacgatggc 60

tccccctcca ggttcgactc ccagccgcac agggggcttc ctggggacca ccagccccgg 120

ccccatggcc gagctcttct gggcggccaa ccaggactcg ggggtcagca gttacatcag 180

cgccgccagc cctgccccca gcaccggctt cggccacagt cttgggggcc ctttgattgc 240

cacagccttc accaatgggt accactgaag caggggacgg tggcaggagc gccccagcct 300

gcagctgact gaggaccacg agtgagccag cgagggggcg ggagacctca gccgcagccg 360

ccgccccctc ccctgcagcg actcggaccc gctactgcct gcccccaact ccccgggccc 420

ggcccctgcc ctgctgcccc caacagcgtc tggctcccct actaacgtcc ccctcttcgc 480

ccttgccccc atccccaccc gcccctctcc cggccctgct tttatttatt ttgg 534

<210> 332

<211>  580

<212>  DNA

<213>  Homo sapiens


<400>  332
gccatttcct ttttcacttt ggccaaggag ctgtaccctg gaaactacaa gcccaacatc      60

actcactact ttctccggct gcttcatgac aaggggctgc ttctgcggct ctacacgcag     120

aacatcgatg ggcttgtttt atgagtcctg ccaagcccat aagttgtgct cttggccttg     180

cgtgctcatc tcggaggcgg cagcaacact tcgctttggc ttgaatttca gtgtcgggca     240

tccctgcctc aaagctggtt gaagctcatg gaacctttgc ctctgccacc tgcacagtct     300

gccaaagacc cttcccaggg gaggacattc gggctgacgt gatggcagac agggttcccc     360

gctgcccggt ctgcaccggc gttgtgaagc ccgacattgt gttctttggg gagccgctgc     420

cccagaggtt cttgctgcat gtggttgatt tccccatggc agatctgctg ctcatccttg     480

ggacctccct ggaggtggag ccttttgcca gcttgaccga ggccgtgcgg agctcagttc     540

cccgactgct catcaaccgg gacttggtgg ggcccttggc                           580

<210>  333

<211>  580

<212>  DNA

<213>  Homo sapiens


<400>  333
gctcttattg agagcaatca aactgccagg tggaaaaatg tgaaagtaaa ctttgataac      60

gtaggacttg gatatctgtc tctacttcaa gtagccacgt ttaagggatg gatggatatt     120

atgtatgcag ctgttgtttc acgaaatgta gaattacaac ccaagtatga agacaacctg     180

tacatgtatc tttattttgt catctttatt atttttggtt cattctttac cttgaatctt     240

ttcattggtg tcatcataga taacttcaac caacagaaaa agaagtttgg aggtcaagac     300

atttttatga cagaagaaca gaagaaatac tacaatgcaa tgaaaaaact gggttcaaag     360

aaaccacaaa aacccatacc tcgacctgct aacaaattcc aaggaatggt ctttgatttt     420

gtaaccaaac aagtctttga tatcagcatc atgatcctca tctgccttaa catggtcacc     480

atgatggtgg aaaccgatga ccagagtcaa gaaatgacaa acattctgta ctggattaat     540

ctggtgttta ttgttctgtt cactggagaa tgtgtgctga                           580

<210>  334

<211>  569

<212>  DNA

<213>  Homo sapiens

<400>  334
gccattccca gctatcctta ccggcagggc tgcccaggag ctgcaggaca agcaccagga      60

gcccctccgg gtagctacta ccctggaccc cccaatagtg gagggcagta tggtagtggg     120

ctaccccctg gtgttgttta tgggggtcct gcccctggag ggccttatgg accaccagct     180

ggtggagggc cctatggaca ccccaatcct gggatgttcc cctctggaac tccaggagga     240

ccatatggcg gtgcagctcc cgggggcccc tatggtcagc cacctccaag ttcctacggt     300

gcccagcagc ctgggcttta tggacagggt ggcgcccctc ccaatgtgga tcctgaggcc     360

tactcctggt tccagtcggt ggactcagat cacagtggct atatctccat gaaggagcta     420

aagcaggccc tggtcaactg caattggtct tcattcaatg atgagacctg cctcatgatg     480

ataaacatgt ttgacaagac caagtcaggc cgcatcgatg tctacggctt ctcagccctg     540

tggaaattca tccagcagtg gaagaacct                                       569

<210>  335

<211>  586

<212>  DNA

<213>  Homo sapiens

<400>  335
gcggttttcc tatggagcat gaggtcctac acgttaattc ggaagctaga gggccatcaa      60

agcagtgttg tctcttgtga cttctccttc tactctgccc tgcttgtcac ggcttcttac     120

gataccaatg tgattatttt ggacccctac accggctaaa ggctgaggtc actccaccac     180

acccaggttg accccgccat ggatgacagt gacgtccaca ttagctcact gagatctgtg     240

tgcttctctc cagaaggctt gtaccttgcc acggtggcag atgacagact cctcaggatc     300

tgggccctgg aactgaaaac tcccattgca tttgctccta tgaccaatgg gctttgctgc     360

acattttttc cacatggtgg agtcattgcc acagggacaa gagatggcca cgtccagttc     420

tggacagctc ctagggtcct gtcctcactg aagcacttat gccggaaagc ccttcgaagt     480

ttcctaacaa cttaccaagt cctagcactg ccaatcccca agaaaatgaa agagttcctc     540

acatacagga ctttttaagc aacaccacat cttgtgcttc tttgta                    586

<210>  336

<211>  531

<212>  DNA

<213>  Homo sapiens

<400>  336
   gcccacttgt ccgcgcagtg gaagaaacat tggtttgtgc tgacagattc aagtctcaaa 60
   tattacagag actccactgc tgaggaggca gatgagctgg atggtgagat cgacctgcgt 120
   tcctgcacgg atgtcactga ttacgcggta cagcgcaact atggcttcca gatccacacc 180
   aaggatgctg tctatacctt gtcggccatg acctcaggca tccggcggaa ctggatcgag 240
   gctctgagaa agaccgtacg tccaacttca gccccagatg tcaccaagct ctcggactct 300
   aacaaggaga acgcgctgca cagctacagc acccagaagg gccccctgaa ggcaggggag 360
   cagcgggcgg gctctgaggt catcagccgg ggtggccctc ggaaggcgga cgggcagcgt 420
   caggccttgg actacgtgga gctctcgccg ctgacccagg cttccccgca gcgggcccgc 480
   accccagccc gcactcctga ccgcctggcc aagcaggagg agctggagcg g 531

<210> 337
<211> 475
<212> DNA
<213> Homo sapiens

<400> 337
gcccacgcgt ccggccaggg gcccaggaca gccccgaggc cttcaggtca gaggatgttc 60
tggagtccta cgaaaacccc ccacccatcg tcctgcccag cgacggcttc caggtggact 120
tggaagccaa ctgcctggac gacagcatct accagcacct gctctacgtc cgccactttc 180
tgtggggtct gcggagcaga gccagccccg gcagccctgg caggcctggc agtggggcct 240
cccagccagt gtgccccgag ggtatgcacc acgtggtcct tgacgggcac ctggaggccc 300
cgagctgccc cctggctccc agggaccctg gcccagcagc ccgggaagtg gcctcggagc 360
gtactcttcc cttgaggggg gctccctggg cacaggcccc ccctggaagg caacccggtc 420
gtgggggctc ccaggctggc cacccgcaca cggactcgtt ctgcttgctc acgcc 475

<210> 338
<211> 578
<212> DNA
<213> Homo sapiens

<220>
<221> misc_feature
<222> (375)..(375)
<223> n = a, c, g or t

<220>

<221>  misc_feature
<222>  (502)..(502)
<223>  n = a, c, g or t

<400>  338
gcggacgcgt gggtgggcat ggatgtgctg aacgaggcca ttggtaccct caccgccagg      60
ggggaccgga atgcctgggt ccccaccatg ctcagtgtgt ctgactctct catgactgca     120
caccccattc aggcagaggc cagtacagag gaggagccat tgtggcagtg ccctgtgcgc     180
cttgtgacat ttattggtgt tggccgcgac ccacacacct ttggcctcat cgctgacctg     240
ggccgtcaga gcttccagtg cgcagccttc tggtgccagc cccatgcagg gggactctct     300
gaagctgtgc aggctgcctg tatggttcag taccagaagt gtcttgtggc ctctgcagct     360
cgaggcaagg cctgnggtgc ccaggcccgt gcccgcctgc ggctcaagcg gaccagctcc     420
atggattccc caggaggtcc cctgcccctc cccctgctca aaggaggggt tggcggtgca     480
ggggcaaccc ctcgaaagcg gngtgtcttc tcttttcttg atgccttccg gctgaaaccc     540
tctctgctcc atatgcccta aacttatctg ggaaggct                             578

<210>  339
<211>  587
<212>  DNA
<213>  Homo sapiens

<400>  339
gacaagaagc tgtttgaaga ggagaaattg ctgagacaag aaggaaaatt agagaagatc      60
cagaccaagg caggtgaggc gacagtgaaa tttctaaaaa gctgtcgcct ggaggtaggg     120
atgaagaaca atgtcaaatg ggagctgaac cctgaaatag ttgcccgcca cttctttaag     180
aatcttggtg ttgtggttgc cccacataca ttaaagttac cagcagagcc tatcacacgg     240
tggggcgagt attggtgtga ggtgacggta aatgggcttg atactgtgag agtgcctatg     300
tctgtcgtga actttgagaa gcccaagacc aaaagatata agtactggtt agcccagcaa     360
gctgccaagg ctatggcccc caccagcccc cagatctaaa tctactctcc ctccaaggca     420
gcaaagcaga atcgggagca gtggagcaga aatgtgcagg caccctgatc tcactcccag     480
ctctgaccaa atacagaatt ttagagaaca tctgaagaca tcagactgca ctgcgtatac     540
atgttgaatt cttcattttt gccatcttta actgtcatca ctggggc                   587

<210>  340
<211>  590
<212>  DNA
<213>  Homo sapiens

<400> 340
gagcggcccg atgccgaggg tgccactgag caccgcctgg agaagatccc agagcccatc 60
aaagaggcca ctgccctatt ctacggacct gcagcaccag ccagcggtgc cctcccagag 120
ggccaggtgg attcactgct ttccatcatc tccagccaga gggagcgctt ccgtgcccgg 180
aaccaggagc ttgaggccga gaaccgcctg gcccagcaca ccctccaggc cctgcagagt 240
gagctggaca gcctgcgcgc cgacaacatc aagctctttg agaagatcaa gttcctgcag 300
agctaccctg gccggggcag cggcagtgat gacacggagc tgcggtactc gtcccagtac 360
gaggagcgcc tggacccctt ctcctccttc agcaagcggg agcggcagag gaagtacctg 420
agcttgagtc cctgggacaa ggccaccctc agcatggggc gtctggttct ctccaacaag 480
atggcgcgca ccatcggctt cttctacaca ctgttcctgc actgcctggt cttcctggtg 540
ctctacaagc tggcatggag cgagagcatg gagagggact gtgccacctt 590

<210> 341
<211> 580
<212> DNA
<213> Homo sapiens

<400> 341
gaaccatttg agcgagtatg ggaagtgaaa gatagtaagg aagatagtga tatgttggct 60
gctggaggta aaatcgagag caacttggaa agtaaagtgg ataaaaaatg ttttgcagat 120
agccttgagc aaactaatca cgaaaaagat agtgagagta gtaatgatga tacttctttc 180
cccagtacgc cagaaggtat aaaggatcgt tcaggagcat atatcacatg tgctcccttt 240
aacccagcag caactgagag cattgcaaca aacatttttc ctttgttagg agatcctact 300
tcagaaaata agaccgatga aaaaaaaata gaagaaaaga aggcccaaat agtaacagag 360
aagaatacta gcaccaaaac atcaaaccct tttcttgtag cagcacagga ttctgagaca 420
gattatgtca caacagataa tttaacaaag gtgactgagg aagtcgtggc aaacatgcct 480
gaaggcctga ctccagattt agtacaggaa gcatgtgaaa gtgaattgaa tgaagttact 540
ggtacaaaga ttgcttatga aacaaaaatg gacttggttc 580

<210> 342
<211> 568
<212> DNA
<213> Homo sapiens

<400> 342
gtgattccag ctagcaaggc aggattagtc attggaaaag ggggagaaac tattaaacag 60

cttcaggaac gggctggagt taaaatggtt atgattcaag acgggccgca gaacactggt 120

gctgacaaac ctcttaggat tacaggagac ccatataaag ttcaacaagc caaggaaatg 180

gtgttagagt taattcgtga tcaaggcggt ttcagagaag ttcggaatga gtatgggtca 240

agaataggag gaaatgaagg gatagatgtc cccattccaa gatttgctgt tggcattgta 300

ataggaagaa atggagagat gatcaaaaaa atacaaaatg atgctggtgt tcgcattcag 360

tttaagccag atgatgggac aacacccgaa aggatagcac aaataacagg acctccagac 420

cgatgtcaac atgctgcaga aattattaca gaccttcttc gaagtgttca ggctggtaat 480

cctggtggac ctggacctgg tggtcgagga agaggtagag gtcaaggcaa ctggaacatg 540

ggaccacctg gtggactaca ggaattta 568

<210> 343

<211> 578

<212> DNA

<213> Homo sapiens

<400> 343
gcccacgcgt ccgcccacgc gtccgaaagg catgaggggt cgtcttcgga atctgtgcca 60

cccggcacta ccatttcgag ggtgaagctc ctcgacacca tggtggacac ttttcttcag 120

aagctggtcg ccgccggcag ctaccagaga ttcactgact gctataagtg cttctaccag 180

ttgcagcctg cgatgacaca gcaaatctat gacaagttta tagctcagtt gcagacatct 240

atccgggagg aaatctctga catcaaagag gaggggaacc tagaagctgt cttgaatgcc 300

ttggataaaa ttgtggaaga aggcaaagtc cgcaaagagc cagcctggcg ccccagcggg 360

atcccagaga aggatctgca cagtgttatg gcaccctact tcctgcagca acgggacacc 420

ctgcggcgcc atgtgcagaa acaggaggcc gagaaccagc agctggcaga tgccgtcctg 480

gcagggcgga ggcaggtgga ggagctgcag ctacaggtcc aggcccagca gcaggcctgg 540

caggctctac acagagaaca gagggagctg gttgctgt 578

<210> 344

<211> 599

<212> DNA

<213> Homo sapiens

<400> 344
gcccacgcgt ccgcccacgc gtccgcttga caactgggac agcgtgacct ccaccttcag 60

caagctgcgc gaacagctcg gccctgtgac ccaggagttc tgggataacc tggaaaagga 120

gacagagggc ctgacgcagg agatgagcaa ggatctggag gaggtgaagg ccaaggtgca 180

accctacctg gacgacttcc agaagaagtg gcaggaggag atggagctct accgccagaa 240
ggtggagccg ctgcgcgcag agctccaaga gggcgcgcgc cagaagctgc acgagctgca 300
agagaagctg agcccactgg gcgaggagat gcgcgaccgc gcgcgcgccc atgtggacgc 360
gctgcgcacg catctggccc cctacagcga cgagctgcgc cagcgcttgg ccgcgcgcct 420
tgaggctctc aaggagaacg gcggcgccag actggccgag taccacgcca aggccaccga 480
gcatctgagc acgctcagcg agaaggccaa gcccgcgctc gaggaccttc gacaaggcct 540
gctgcccgtg ctggagagct tcaaggtcag cttcctgagc gctctcgagg agtacacta 599

<210> 345
<211> 596
<212> DNA
<213> Homo sapiens

<400> 345
gtatttgtac cgaacacagg aaagaagagt gggaaattga aggcctccta taaacgggat 60
tgttttagtg ttggcagtaa tgttgatata gatttttctg gaccaaccat ctatggctgg 120
gctgtgttgg ccttcgaagg gtggcttgct ggctatcaga tgagttttga cacagccaaa 180
tccaaactgt cacagaataa tttcgccctg ggttacaagg ctgcggactt ccagctgcac 240
acacatgtga acgatggcac tgaatttgga ggttctatct accagaaggt gaatgagaag 300
attgaaacat ccataaacct tgcttggaca gctgggagta acaacacccg ttttggcatt 360
gctgctaagt acatgctgga ttgtagaact tctctctctg ctaaagtaaa taatgccagc 420
ctgattggac tgggttatac tcagaccctt cgaccaggag tcaaattgac tttatcagct 480
ttaatcgatg ggaagaactt cagtgcagga ggtcacaagg ttggcttggg atttgaactg 540
gaagcttaat gtggtttgag gaaagcatca gatttgtccc tggaagtgaa gagaaa 596

<210> 346
<211> 583
<212> DNA
<213> Homo sapiens

<400> 346
gcaaatctga atcgacagtt tcttttccgg ccctgggatg tcacgaagtt aaagtctgac 60
acggctgctg cagctgtgcg ccaaatgaat ccacatatcc gggtgacaag ccaccagaac 120
cgtgtgggtc ctgacacgga gcgcatctat gatgacgatt ttttccaaaa cctagatggc 180
gtggccaatg ccctggacaa cgtggatgcc cgcatgtaca tggaccgccg ctgtgtctac 240
taccggaagc cactgctgga gtcaggcaca ctgggcacca aaggcaatgt gcaggtggtg 300
atccccttcc tgacagagtc gtacagttcc agccaggacc cacctgagaa gtccatcccc 360

atctgtaccc tgaagaactt ccctaatgcc atcgagcaca ccctgcagtg ggctcgggat 420

gagtttgaag gcctcttcaa gcagccagca gaaaatgtca accagtacct cacagacccc 480

aagtttgtgg agcgaacact gcggctggca ggcactcagc ccttggaggt gctggaggct 540

gtgcagcgca gcctggtgct gcagcgacca cagacctggg ctg 583

<210> 347

<211> 561

<212> DNA

<213> Homo sapiens

<400> 347
gcccacgcgt ccgtgaaagt gctgagattc attgcagagg ttcagaaaag agaccctcgt 60

gactggactg cacagttcat ggaggctgca gatgaggcct tggatgctct ggatgctgct 120

gcagctgagg ccgaagcccg ggctgaagca agaacccgca tgggaattgg agatgaggct 180

gtgtctgggc cctggagctg ggatgacatt gagtttgagc tgctgacctg ggatgaggaa 240

ggagattttg gagatccctg gtccagaatt ccatttacct tctgggccag ataccaccag 300

aatgcccgct ccagattccc tcagaccttt gccggtccca ttattggtcc tggtggtaca 360

gccagtgcca acttcgctgc caactttggt gccattggtt tcttctgggt tgagtgagat 420

gttggatatt gctatcaatc gcagtagtct ttcccctgtg tgaggctgaa gcctcagatt 480

ccttctaaac acagctatct agagagccac atcctgttga ctgaaagtgg catgcaagat 540

aaatttattt gctgttcctt g 561

<210> 348

<211> 562

<212> DNA

<213> Homo sapiens

<400> 348
gctgcactga actctgggtg gaaaaccatc tatcaggtgg acctccccca cacggcgatc 60

caggaagcct ccaacaagaa gaaattcgag gatgccttga gcctcatcca cagcgcctgg 120

cagcggagcg acagtctctg ccgcggccga gcctcccgag acccctggtg ctgaggcaga 180

gctggttggc gtccctggtg ggcaggaaag gaaggcacgc cagccggcag gcacactgtc 240

acggctgttg tcatgctgtc gggagcctac agtccacccc tgccctgggc ggcagaacca 300

ccgagtgtgg cttaagacag ggtccctcca ctccagggat ccagatcagg tgcccggcac 360

ccctgggcat cctgcccgac aggtagcgaa tggaggtcgc tgggggcaga gggtccgagc 420

cctgtgggct ctgcggatcc acgccctcct cccgggcctc cgcctcagtc tgcagaattt 480

ctgccgagtg gcaccgagaa caccatccat ctaaggacga acaaaagaac caggagggcg 540
ggaccccct cttcctctct gg 562

<210> 349
<211> 568
<212> DNA
<213> Homo sapiens

<400> 349
gtctttgcgc accaccgcct ctcactggac ctgctgagcc aaatgagccc aggggacctg 60
gccaaggtgg gcgtctcaga agctggcctg cagcacgaga tcctccggag agtccaggaa 120
ctgctggatg cagccaggat ccagccagag ctgaaaccac caatgggtga ggtcgtcacc 180
cctacggccc cccaggagcc tcctgagtct gtgaggccat ccgctccccc tgcagagctg 240
gaggtgcagg cctcagagtg tgtcgtgtgc ctggaacggg aggcccagat gatcttcctc 300
aactgtggcc acgtctgctg ctgccagcag tgctgccagc cactgcgcac ctgcccgctg 360
tgccgccagg acatcgccca gcgcctccgc atctaccaca gcagctgagt gctgcccgcc 420
cacctgggcc tggtcctagc cctgcctcgg ccactgtgag ccccgggctc ctgctcagcc 480
ttgtgccagc cagactcgta tgaggctccc ccctgccctg ggcccccttc ccactgccca 540
ggagccccca tcctaagctc caagcatg 568

<210> 350
<211> 381
<212> DNA
<213> Homo sapiens

<400> 350
gggcgcccgt cgggccggac gagaaggaga agggcgccgg cgccgggggg gaggagaaga 60
agggggcggg cgagggcggc aaggacgggg aggggggcaa ggagggcgag aagaagaacg 120
gcaagtacga gaagccgccg ttcagctaca acgcgctcat catgatggcc atccggcaga 180
gccccgagaa gcggctcacg ctcaacggca tctacgagtt catcatgaag aacttccctt 240
actaccgcga gaacaagcag ggctggcaga actccatccg ccacaatttg tccctcaaca 300
agtgcttcgt gaaggtgccg cgccactacg acgacccggg caagggcaac tactggatgc 360
tggacccgtc gagcgacgac g 381

<210> 351
<211> 609
<212> DNA

<213>  Homo sapiens

<400>  351
gtgactacaa aggagtgttt acatcgtttt tgtgcagact gcatcatcac agcccttata     60
agtggcaaca aagaatgtcc tacctgtcgg aaaaaactag tttccaaaag atcactaagg    120
ccagacccaa actttgatgc actcatcagc aaaatttatc caagtcgtga tgagtatgaa    180
gctcatcaag agagagtatt agccaggatc aacaagcaca ataatcagca agcactcagt    240
cacagcattg aggaaggact gaagatacag gccatgaaca gactgcagcg aggcaagaaa    300
caacagattg aaaatggtag tggagcagaa gataatggtg acagttcaca ctgcagtaat    360
gcatccacac atagcattca ggaagcaggc cctagtaaca aacggaccaa aacatctgat    420
gattctgggc tagagcttga taataacaat gcagcaatgg caattgatcc agtaatggat    480
ggtgctagtg aaattgaatt agtattcagg cctcatccca cacttatgga aaaagatgac    540
agtgcacaga cgagatacat aaagacttct ggtaacgcca ctgttgatca cttatccaag    600
tatctggct                                                            609

<210>  352
<211>  574
<212>  DNA
<213>  Homo sapiens

<400>  352
gcttgcccgg tggtgctgaa gtggaagcac tgtccccgat ggggctgcct ggggaggagg     60
attcaggtcc tgatgagccg ccctcacccc cgtcaggcct cctcccagcc acggtgcagc    120
cattccatct gagaggcacg agctccacct tctcccagcg cagccgtgac atctttgact    180
gcctggaggg ggcggccaga cgggctccat cctctgtggc ccacaccagc atgagtgaca    240
acggaggctt caagcggccc ctagcgccct caggccggtc tccagtggaa ggcctgggca    300
gggcccatcg gagccctgcc tcaccaaggg tgcctccggt ccccgactac gtggcacacc    360
ccgagcgctg gaccaagtac agcctggaag atgtgaccga ggtcagcgag cagagcaatc    420
aggccaccgc cctggccttc ctgggctccc agagcctggc tgcccccact gactgcgtgt    480
cctccttcaa ccaggatccc tcaagctgtg gggaggggag ggtcatcttc accaaaccag    540
tccgaggggt cgaagccaga cacgagagga agag                                574

<210>  353
<211>  503
<212>  DNA
<213>  Homo sapiens

<400> 353
gcggacgcgt gggcggacgc gtgggcggac gcgtgggcgc aacccctcgc cgccgccgcc 60

gggtcgcgac aaggaggagg aggaggaggt ggccggtgga gactgcatag ggagcacggt 120

ctacagcaaa cactggctct tcggcgtcct cagcggactc atccagattg ttagccctga 180

aaacaccaaa tctagctcat atgatgagga gcagctgacg gagcttgatg aagaaatgga 240

gaatgaaatt tgcagagtat gggatatgtc aatggatgag gacgtggctt tatttctcca 300

agaatttaat gctcctgata tattcatggg agtactggcc aagtccaagt gtcctcgatt 360

aagagaaatc tgtgtgggaa ttttaggtaa tatggcctgt ttccaggaga tatgtgtgtc 420

catcagcagt gataaaaatc ttgggcaggt gttattgcac tgtttgtatg attcagaccc 480

acctactctg ctggaaacaa gca 503

<210> 354

<211> 661

<212> DNA

<213> Homo sapiens


<400> 354
gggcctcggc agcggtgttc tcgcgcttgc gaagcgggct ccggctcggc tcgcggggac 60

tgtgcacgag gttggcgacg ccgccccgcc gggccccaga tcaggatatt tcatgtctga 120

atcgggaccc agctcgagta gtagttgtgg actgcaagaa ggaagccttc cgcctgcagc 180

cctataacgg cgttgccctg cggccctggg acggcaactc tgatgaccgg gtcttgttgg 240

atctgtctgc cttcctcaag accattgcac tgaatggtgt ggaggacgtg cgaaccgtgc 300

tggagcacta tgccctggag gatgacccgc tggcggcttt caaacagcgg caaagccggc 360

tagagcagga ggagcagcag cgcctggccg agctctccaa gtccaacaag cagaacctct 420

tccttggctc cctcaccagc cgcttgtggc ctcgctccaa acagccctga actctgggcc 480

tcctcaaact cagtgcctgg gtccagggcc ccagtgcttc cagaccaaga cttgggccac 540

cacttgtcca ataaagtaca tcccagacgc cacacctgct gtgtcccgag agtctccaga 600

tgggggcatc agggtgaggt ccgggactct tgggtcatcg tcccacagtg gctgatcggc 660

t 661

<210> 355

<211> 653

<212> DNA

<213> Homo sapiens


<400> 355
gccacaacac gcgctacgga tctcaagaac cttttcagca agtatgggaa ggttgtcggg 60

gccaaagtgg taacgaacgc ccgcagcccg ggggctcgat gctatggatt cgtcaccatg 120
tcgacatctg acgaggcgac caagtgcatc agccatctcc acagaactga gctgcatgga 180
cgaatgatct ccgtagagaa ggccaaaaat gagcctgctg ggaaaaagct ttccgacaga 240
aaagagtgcg aagtgaagaa ggaaaaatta tcgagtgtcg acagacatca ttctgtggag 300
atcaaaattg aaaaaactgt aattaagaag gaagagaaga ttgagaagaa ggaggaaaaa 360
aagcctgaag acattaagaa ggaagaaaaa gaccaggatg agctgaaacc cggacctaca 420
aatcggtcta gagtcaccaa atcaggaagc agaggaatgg agcggacggt cgtgatggat 480
aaatcgaaag gagagcccgt cattagcgtg aaaaccacaa gcaggtccaa agagagaagc 540
tccaagagtc aggatcgcaa gtcagaaagc aaagaaaaga gagacatctt gtcgtttgat 600
aaaatcaaag aacaaaggga gagagagcgc cagaggcagc gggaacggga gat 653

<210> 356
<211> 690
<212> DNA
<213> Homo sapiens

<400> 356
gcggacgcgt gggcggacgc gtgggcgaac tgcagccatg gagcaggcac ctccggaccc 60
cgagcggcag ctccagccgg cgcccttgga gccgctgggc tccccagacg ctgggctggg 120
ggctgcggtc ggcaaggaag cggagggggc cggagaagag agctctgggg tcgacacgat 180
gacacacaat aatttttggt tgaagaagat agaaatcagt gtttcagaag cagaaaaacg 240
aactggaaga aatgccatga acatgcaaga aacatatact gcttacctca ttgaaacaag 300
gtcagttgaa cataccgatg gtcagagtgt cctaacagac tcactatggc ggcgatatag 360
tgaatttgag ttgttgagaa gctacctttt agtttactat ccacatattg ttgtgccacc 420
tctgccagaa aaacgggcag aatttgtttg gcataaactc tctgctgaca acatggatcc 480
agattttgtg gagaggcgac ggattggttt agaaaacttt ctcttgagga ttgcttcaca 540
tcccatcctt tgtagagaca aaatcttcta tctgttttta acacaggaag gtaactggaa 600
ggagactgtg aatgaaactg cgtttcagct gaaggagatt tactgacctt aagcactata 660
gtgatgaact gcagtctgtc atctcacatc 690

<210> 357
<211> 668
<212> DNA
<213> Homo sapiens

<400> 357

gtaccttttc tggaggaatc accttgtgga agccaaatct cttcagaaca ttcggtcatt 60
aagccacctc ttggagattc tccagggagt ctttcaaggt cgaaagggga agaggatgac 120
aaatcaaaaa agcagtttgc tttgtattaa tatcctagaa gacacacaag ctgttagagc 180
agtggctttt catccagctg gaggtttata tgctgttggt tcaaattcaa aaactctgag 240
agtatgtgcc tatccagatg taattgatcc aagtgcacat gagactccta agcagccggt 300
ggtacgtttt aaaaggaata aacatcataa aggatccatt tactgtgtgg cctggagtcc 360
ttgtgggcag ttattagcaa caggatcaaa tgacaaatac gtcaaagtgc tgcccttcaa 420
tgcagagact tgtaacgcaa caggaccaga tctggaattt agtatgcatg atggaacaat 480
tagagacttg gcatttatgg aaggcccaga aagcggagga gctattttaa taagtgctgg 540
agcaggggat tgtaacattt atacaaccga ttgtcaaaga ggacagggcc tccatgcttt 600
gagtggacat actgggcata ttttagcact ctatacctgg agtggctgga tgattgcatc 660
tggttccc 668

<210> 358
<211> 710
<212> DNA
<213> Homo sapiens

<400> 358
ggtggcggcc aagcgggata cgggcggcgg gagctgggga acaggcatgg acgtttccgg 60
gcaagagacc gactggcgga gcaccgcctt ccggcagaag ctggtcagtc aaatcgagga 120
tgccatgagg aaagctggtg tggcacacag taaatccagc aaggatatgg agagccatgt 180
tttcctgaag gccaagaccc gggacgaata cctttctctc gtggccaggc tcattatcca 240
ttttcgagac attcataaca agaaatctca agcttccgtc agtgatccta tgaatgcact 300
ccagagcctg actggcggac ctgctgcggg agccgctgga attggcatgc ctcctcgggg 360
cccgggacag tctctgggcg ggatgggtag ccttggtgcc atgggacagc caatgtctct 420
ctcagggcag ccgcctcctg ggacctcggg gatggcccct cacagcatgg ctgtcgtgtc 480
tacggcaact ccacagaccc agctgcagct ccagcaggtg gcgctgcagc agcagcagca 540
acagcagcag ttccagcagc agcagcaggc ggcgctacag cagcagcagc agcagcagca 600
acagcagcag ttccaggctc atcagagtgc catgcagcag cagttccaag cagtagtgca 660
gcagcagcag cagctccagc agcagcagca gcagcagcag catctaatta 710

<210> 359
<211> 673
<212> DNA
<213> Homo sapiens

<400> 359
gcccggttcc cactgaagga tggtccccgg ctgcaggcct ggctgcagca catgggctgt 60
gagcactggg tgcccagctg ccaccagcac ttgtgcagcg agcacttcac accctcctgc 120
ttccagtggc actggggtgt gcgctacctg cggcctgatg cagtgccctc catcttctcc 180
cggggaccac ctgccaagag tcagcggagg acccgaagca cccagaagcc agtctcgccg 240
ccgcctcccc tacagaggaa tacacccctg ccccagagcc ctgccatccc agtctctggc 300
ccagtgcgcc tagtggtgct gggccccaca tcggggagcc ccaagactgt ggccaccatg 360
ctcctgaccc ccctggcccc tgcgccaact cctgagcggt cacaacctga agtccctgcc 420
caacaggccc agaccgggct gggcccagtg ctgggagcac tgcaacgccg ggtgcggagg 480
ctgcaacggt gccaggagcg gcaccaggcg cagctgcagg ccctggaacg gctggcacag 540
cagctacacg gggagagcct gctggcacgg gcacgccggg gtctgcagcg cctgacaaca 600
gcccagaccc ttggacctga ggaatcccaa accttcacca tcatctgtgg agggcctgac 660
atagccatgg tcc 673

<210> 360
<211> 660
<212> DNA
<213> Homo sapiens

<400> 360
gggcagagag aaggggagac agacagaggg tggggctttc ccccttgtct ccagtgccct 60
ttctggtgac cctcggttct tttcccccac caccccccca gcggagccca tcgtggtgag 120
gcttaaggag gtccgactgc agagggacga cttcgagatt ctgaaggtga tcggacgcgg 180
ggcgttcagc gaggtagcgg tagtgaagat gaagcagacg ggccaggtgt atgccatgaa 240
gatcatgaac aagtgggaca tgctgaagag gggcgaggtg tcgtgcttcc gtgaggagag 300
ggacgtgttg gtgaatgggg accggcggtg gatcacgcag ctgcacttcg ccttccagga 360
tgagaactac ctgtacctgg tcatggagta ttacgtgggc ggggacctgc tgacactgct 420
gagcaagttt ggggagcgga ttccggccga gatggcgcgc ttctacctgg cggagattgt 480
catggccata gactcggtgc accggcttgg ctacgtgcac agggacatca aacccgacaa 540
catcctgctg gaccgctgtg gccacatccg cctggccgac ttcggctctt gcctcaagct 600
gcgggcagat ggaacggtgc ggtcgctggt ggctgtgggc accccatact acctgtcccc 660

<210> 361
<211> 564
<212> DNA
<213> Homo sapiens

<400>  361
gcctctctga gtccctcctg gcccccaccc tcccgcagga ggaggcggag gcattgctag      60
agctccaggg ccggcttcag gaggcccaag acaccacaga agccctccga gcccagctgg     120
gggtgcagga ggtgcagctg cagggccttc aaggggccct ccagcagctc cagcaggaga     180
cggagcagaa ctgcaggcgt gagctacagc agatgcatgg gcagctggca ggacttcggg     240
cacggatggc cagcctgcgt cagggctgcg gggacctccg aggtttggtc agcaccttta     300
cccagagctg tcagggttcg ctgagtgagg cccggggcca ggtgtcctgg gccttggggg     360
cactgtcatc tggagggcct ggcactcagc tccctgaggg gcagcaaggg cccccagccg     420
gatgcccagg gcggctgcca gaactcaagg gaaatatccg agtgctgtgt cggctgaggc     480
cagggacatc ttctagcctt gagagtgtgg agcctggccc agggggcacc gtcaccacct     540
gctaccgggg gcgccatcgt cgat                                            564

<210>  362
<211>  186
<212>  DNA
<213>  Homo sapiens

<400>  362
gcgctgagcg gcacggctac atcaagggca tcgtcaagga catcatccac gacccgggcc      60
gcggcgcgcc cctcgccaag gtggtcttcc gggatccgta tcggtttaag aagcggacgg     120
agctgttcat tgccgccgag ggcattcaca cgggccagtt tgtgtattgc ggcaagaagg     180
cccagc                                                                186

<210>  363
<211>  426
<212>  DNA
<213>  Homo sapiens

<400>  363
gcggacgcgt gggcggacgc gtggggcggc agccgccgcc gccgcctgcc ggggcatgtc      60
gtggacgcca gccgagacga acgcgctcat cgcagtgtgg ggcaacgagc ggctggtgga     120
ggcgcggtac cagcagctgg agggagccgg cacggtgttc ggcagcaagg cccccgggcc     180
agccatgtac gagcgcgtgt cccgggccct ggccgagctg ggctacgagc ggaccccgtc     240
ccagtgccgg gagcgcatca agctggttcg atgcccagaa ctgaatgctg tgctccagct     300
gtggcctcac cggtgctgaa tagagaggaa gagccacctt catagcttac atgagctgga     360
gtcagatggc agcactatgg aggactattc acaggaggac tggggaaacc acagtcagga     420

tctcca                                                              426

<210>  364

<211>  648

<212>  DNA

<213>  Homo sapiens


<400>  364
gcccacgcgt ccgcccacgc gtccgcccac gcgtccgtcc agcttcatcc gcagaggagc      60

ctcggccagg cttgccaggg cgcccccagc ccctccccag gccgcgagcg cccctgccgc     120

ggtgcctggc ctcccctccc agactgcagg gacagcaccc ggtaactgcg agtggagcgg     180

aggacccgag cggctgagga gagaggaggc ggcggcttat ctgctacggg gtccggccgg     240

cgccctcccg agggaagaca cagaagaagg gccacagtgc ctgtgtccat cctcaggact     300

ccgcctggcc ccaaatggaa gagactgtct agatattgat gaatgtgcct ctggtaaagt     360

catctgtccc tacaatcgaa gatgtgtgaa cacatttgga agctactact gcaaatgtca     420

cattggtttc gaactgcaat atatcagtgg acgatatgac tgtatagata taaatgaatg     480

tactatggat agccatacgt gcagccacca tgccaattgc ttcaataccc aagggtcctt     540

caagtgtaaa tgcaagcagg gatataaagg caatggactt cggagttctg ctatccctga     600

aaattctgtg aagctacatg cgaacctgga tgtaagtttg gtgagtgc                  648

<210>  365

<211>  640

<212>  DNA

<213>  Homo sapiens


<400>  365
gcccacgcgt ccgcagcgcg tcgcggtcat ggctctcgtt tcaggcctcc acggtgtgca      60

cctggagtca ggagcgcgct tgtgcgtaca gttcggttca gttactaaaa gatgtaactg     120

aactgcagat ccttggtgaa atatctttca acaaatctct atatgaggga ctgaatgcag     180

agaaccacag aactaagatc actgtcgtct tcctgaaaga tgagaagtac cattctttgc     240

ctatcatcat taaaggcagc gttggtggac ttctggtgtt gatcgtgatt ctggtcatcc     300

tgttcaagtg tggctttttt aaaagaaaat atcaacaact gaacttggag agcatcagga     360

aggcccagct gaaatcagag aatctgctcg aagaagagaa ttaggacctg ctatccactg     420

ggagaggcta tcagccagtc ctgggacttg gagacccagc atcctttgca ttactttttc     480

cttcaggatg atctagagca gcatggagct gttggtagaa tattagtttt taaccataca     540

ttgtcccaaa agtgtctgtg cattgtgcaa aaagtaaact taggaaacat ttggtattaa     600

ataaatttac acttttcttt gcaaaaaaaa aaaaaaaagg 640

<210> 366

<211> 647

<212> DNA

<213> Homo sapiens

<400> 366
ggcagagccg aggtcgggcc gcgagcggag ccggctgagc gggcgccgag ctcccgccat 60

ggcccggaac acgctgtcct cgcgcttccg ccgggtggac atcgacgaat ttgacgagaa 120

caaatttgtg gacgagcagg aggaggcggc ggcggcggcg gcggagccag gcccggaccc 180

gagcgaggtg gacgggctcc tgcggcaagg ggacatgctt cgggcattcc atgcagcctt 240

gcggaactct cccgtcaaca ccaagaatca agctgtgaag gagcgagccc agggcgtggt 300

gctgaaagtg ctcacaaact tcaagagcag tgagattgag caggctgtgc agtcactgga 360

cagaaacggc gttgacttgt taatgaagta catttataaa ggctttgaga agcccacaga 420

aaatagcagc gcagtgttac tccagtggca cgaaaaggcc ttatcagtag gaggactagg 480

ctccattata agagttctta cagcaagaaa gactgtttaa aaaaaataaa aagactcatg 540

ttaccttgag aagaattctg gatgcccagg ctggtgaaga atggattgac attggaccat 600

cttcctagga actcccaagt aaactatttc aggacatgta tctgctg 647

<210> 367

<211> 241

<212> DNA

<213> Homo sapiens

<400> 367
gtcggccccg ggaccccggc tccccgccag ccccggcccc ggccccggca ccatgtcgga 60

gaaaagcgtg gaggcagcgg ccgagttgag cgccaaggac ctgaaggaga agaaggagaa 120

ggtggaggag aaggcaagcc ggaaagagcg aaagaaagaa gtggtggagg aggaggataa 180

cggtgctgag gaggaagaag aagaaactgc cgaggatgga gaggaggaag atgaagggga 240

a 241

<210> 368

<211> 592

<212> DNA

<213> Homo sapiens

<400>  368
gaatgttatg aatgtgggaa aaccttctgc ttgaagtcag acctcacaat acatcagaga      60

acgcacacag gggagaaacc ctttgcatgt cctgaatgtg ggaaattctt tagccataag     120

tcaaccctct ctcaacatta tagaacacac acgggggaga aaccctatga atgtcatgaa     180

tgtggaaaaa tcttttacaa taaatcatac ctaacaaaac ataatagaac acatacaggg     240

gagaaaccct atgaatgtaa tgaatgtgga aaaaccttct gccagaagtc acaactcact     300

cagcatcaga gaattcacat aggggagaaa ccctatgaat gtaatgagtg tggaaaagct     360

ttctgccata agtcagctct aattgtacat cagagaaccc atacacaaga aaagccttat     420

aaatgtaacg aatgtggaaa atctttctgt gtgaagtcag gacttatttt acatgagaga     480

aagcacacgg cggagaaacc ctatgaatgc aatgaatgtg ggaaatcctt cagtcacaaa     540

tcatcactca cagtacatta cagggctcac acaggagaga aatcttgtca gt             592

<210>  369

<211>  524

<212>  DNA

<213>  Homo sapiens


<400>  369
gcccacgcgt ccgagtgtct gaaggatggt gctggggatg tggcctttgt caagcactcg      60

actatatttg agaacttggc aaacaaggct gacagggacc agtatgagct gctttgcctg     120

gacaacaccc ggaagccggt agatgaatac aaggactgcc acttggccca ggtcccttct     180

cataccgtcg tggcccgaag tatgggcggc aaggaggact tgatctggga gcttctcaac     240

caggcccagg aacattttgg caaagacaaa tcaaaagaat tccaactatt cagctctcct     300

catgggaagg acctgctgtt taaggactct gcccacgggt ttttaaaagt cccccccagg     360

atggatgcca agatgtacct gggctatgag tatgtcactg ccatccggaa tctacgggaa     420

ggcacatgcc cagaagcccc aacagatgaa tgcaagcctg tgaagtggtg tgcgctgagc     480

caccacgaga ggctcaagtg tgatgagtgg agtgttaaca gtgt                      524

<210>  370

<211>  566

<212>  DNA

<213>  Homo sapiens


<400>  370
gctaaaatgt tggatatgtt gaagcagaca ggaaggccgg agatggttgg agaatttgtt      60

gatgattata ccgtcagagt gattgatgtg tttgctatgc cacagtcagg aacaggtgtc     120

agtgtggagg cagttgatcc agtgttccaa gctaaaatgt tggatatgtt gaagcagaca     180

ggaaggccgg agatggttgt tggttggtat cacagtcacc ctggctttgg ttgttggctt 240

tctggtgtgg atatcaacac tcagcagagc tttgaagcct tgtcggagag agctgtggca 300

gtggttgtgg atcccattca gagtgtaaaa ggaaaggttg ttattgatgc cttcagattg 360

atcaatgcta atatgatggt cttaggacat gaacctagac aaacaacttc gaatctgggt 420

cacttaaaca agccatctat ccaggcatta attcatggac taaacagaca ttattactcc 480

attactatta actatcggaa aaatgaactg gaacagaaga tgttgctaaa tttgcataag 540

aagagttgga tggaaggttt gacact 566

<210> 371

<211> 580

<212> DNA

<213> Homo sapiens

<400> 371
gcccacgcgt ccgaacgggc cctgtcttct cagcatcaag caagaattga aattgagtcc 60

ttctatgaag gagaagactt ttctgagacc ctgactcggg ccaaatttga agagctcaac 120

atggatctgt tccggtctac tatgaagccc gtccagaaag tgttggaaga ttctgatttg 180

aagaagtctg atattgatga aattgttctt gttggtggct cgactcgaat tccaaagatt 240

cagcaactgg ttaaagagtt cttcaatggc aaggaaccat cccgtggcat aaacccagat 300

gaagctgtag cgtatggtgc tgctgtccag gctggtgtgc tctctggtga tcaagataca 360

ggtgacctgg tactgcttga tgtatgtccc cttacacttg gtattgaaac tgtgggaggt 420

gtcatgacca aactgattcc aaggaacaca gtggtgccta ccaagaagtc tcagatcttt 480

tctacagctt ctgataatca accaactgtt acaatcaagg tctatgaagg tgaaagaccc 540

ctgacaaaag acaatcatct tctgggtaca tttgatctga 580

<210> 372

<211> 595

<212> DNA

<213> Homo sapiens

<400> 372
gaaaagagag aaatgtatga gcatcctgtc ttctgcttgg cctcccaagt gatggattta 60

accattcaaa atgtaggccg gttaatcact cctgccaaaa agcttgaaga tacaatacgt 120

cttgctgaac tagtcattga agttcttcag caaaatgagg agcaccacgc acaggccttt 180

gcgtggtggt cagatttaat ggtggagcat gcggagacgt tcctgtcact ctttgcagta 240

gacatggatg cagccttaga ggtgcaacct ccagacacat gggacagttt tccactattt 300

cagctgctga atgattttct ccgtactgac tataatttgt gcaatggaaa atttcacaaa 360

cacctgcaag acctgtttgc cccacttgtt gttagatatg tggatctgat ggagtcctca 420

attgcacaat ccattcacag gggctttgag cgggagtcat gggaaccagt caataatggg 480

tcaggcacct cacaagatct gttttggaaa cttgacgccc ttcagacctt cattcgggac 540

ctgcactggc ctgaagaaga gtttggaaag cacctggaac aacggctgaa gttga 595

<210> 373

<211> 502

<212> DNA

<213> Homo sapiens

<400> 373
gagttgatct ctaatgcttg tgatgccttg gacaagattc gctatgagag cctgacagac 60

ccttcgaagt tggacagtgg taaagagctg aaaattgaca tcatccccaa ccctcaggaa 120

cgtaccctga ctttggtaga cacaggcatt ggcatgacca aagctgatct cataaataat 180

ttgggaacca ttgccaagtc tggtactaaa gcattcatgg aggctcttca ggctggtgca 240

gacatctcca tgattgggca gtttggtgtt ggcttttatt ctgcctactt ggtggcagag 300

aaagtggttg tgatcacaaa gcacaacgat gatgaacagt atgcttggga gtcttctgct 360

ggaggttcct tcactgtgcg tgctgaccat ggtgagccca ttggcagggg taccaaagtg 420

atcctccatc ttacagaaga tcagacagag tacctagaag agaggcgggt caaagaagta 480

gtgaagaagc attctcagtt ca 502

<210> 374

<211> 562

<212> DNA

<213> Homo sapiens

<400> 374
gcccacgcgt ccgcgcgcga ctcccacttc cgcccttttg gctctctgac cagcaccatg 60

gcggttggca agaacaagcg ccttacgaaa ggcggcaaaa agggagccaa gaagaaagtg 120

gttgatccat tttctaagaa agattggtat gatgtgaaag cacctgctat gttcaatata 180

agaaatattg gaaagacgct cgtcaccagg acccaaggaa ccaaaattgc atctgatggt 240

ctcaagggtc gtgtgtttga agtgagtctt gctgatttgc agaatgatga acttgcattt 300

agaaaattca cgctgattac tgaagatgtt cagggtaaaa actgcctgac taacttccat 360

ggcatggatc ttacccgtga caaaatgtgt tccatggtca aaaaatggca gacaatgatt 420

gaagctcacg ttgatgtcaa gactaccgat ggttacttgc ttcgtctgtt ctgtgttggt 480

tttactaaaa aacgcaacaa tcagatacgg aagacctctt atgctcagca ccaacaggtc 540

cgccaaatcc ggaagaagat ga 562

<210> 375

<211> 571

<212> DNA

<213> Homo sapiens

<400> 375
gcccacgcgt ccgcaaaaca ccaaatggcg gatgacgccg gtgcagcggg ggggcccggg 60

ggccctggtg gccctgggat ggggaaccgc ggtggcttcc gcggaggttt cggcagtggc 120

atccggggcc ggggtcgcgg ccgtggacgg ggccggggcc gaggccgcgg agctcgcgga 180

ggcaaggccg aggataagga gtggatgccc gtcaccaagt tgggccgctt ggtcaaggac 240

atgaagatca agtccctgga ggagatctat ctcttctccc tgcctattaa ggaatcacag 300

atcattgatt tcttcctggg ggcctctctc aaggatgagg ttttgaagat tatgccagtg 360

cagaagcaga cccgtgccgg ccagcgcacc aggttcaagg catttgttgc tatcggggac 420

tacaatggcc acgtcggtct gggtgttaag tgctccaagg aggtggccac cgccatccgt 480

ggggccatca tcctggccaa gctctccatc gtccccgtgc gcagaggcta ctgggggaac 540

aagatcggca agccccacac tgtcccttgc a 571

<210> 376

<211> 589

<212> DNA

<213> Homo sapiens

<400> 376
gcgggatttg ggtcgcagtt cttgtttgtg gatcgctgtg atcgtcactt aacaatgcag 60

atcttcgtga agactctgac tggtaagacc atcaccctcg aggttgagcc cagtgacacc 120

atcgagaatg tcaaggcaaa gatccaagat aaggaaggca tccctcctga ccagcagagg 180

ctgatctttg ctggaaaaca gctggaagat gggcgcaccc tgtctgacta caacatccag 240

aaagagtcca ccctgcacct ggtgctccgt ctcagaggtg ggatgcaaat cttcgtgaag 300

acactcactg gcaagaccat cacccttgag gtcgagccca gtgacaccat cgagaacgtc 360

aaagcaaaga tccaggacaa ggaaggcatt cctcctgacc agcagaggtt gatctttgcc 420

ggaaagcagc tggaagatgg gcgcaccctg tctgactaca acatccagaa agagtctacc 480

ctgcacctgg tgctccgtct cagaggtggg atgcagatct tcgtgaagac cctgactggt 540

aagaccatca ccctcgaggt ggagcccagt gacaccatcg agaatgtca 589

<210> 377

<211>  544

<212>  DNA

<213>  Homo sapiens


<400>  377
ggagccatcc tcgtcgctgc agcgacacac gctctcgccg ccgccatgac tgagcagatg      60

acccttcgtg gcaccctcaa aggccacaac ggctgggtaa cccagatcgc tactaccccg     120

cagttcccgg acatgatcct ctccgcctct cgagataaga ccatcatcat gtggaaactg     180

accagggatg agaccaacta tggaattcca cagcgtgctc tgcggagtca ctcccacttt     240

gttagtgatg tggttatctc ctcagatggc cagtttgccc tctcaagctc ctgggatgga     300

accctgcgcc tctgggatct cacaacgggc accaccacga ggcgatttgt gggccatacc     360

aaggatgtgc tgagtgtggc cttctcctct gacaaccggc agatagtctc tggatctcga     420

gataaaacca tcaagctatg gaataccctg ggtgtgtgca aatacactgt ccaggatgag     480

agccactcag agagggtgtc ttgtgtccgc ttctcgccca acagcagcaa ccctatcatc     540

gtct                                                                  544

<210>  378

<211>  455

<212>  DNA

<213>  Homo sapiens


<400>  378
gagcggcccg atgccgaggg tgccactgag caccgcctgg agaagatccc agagcccatc      60

aaagaggcca ctgccctatt ctacggacct gcagcaccag ccagcggtgc cctcccagag     120

ggccaggtgg attcactgct ttccatcatc tccagccaga gggagcgctt ccgtgcccgg     180

aaccaggagc ttgaggccga gaaccgcctg gcccagcaca ccctccaggc cctgcagagt     240

gagctggaca gcctgcgcgc cgacaacatc aagctctttg agaagatcaa gttcctgcag     300

agctaccctg gccggggcag cggcaagtga tgacacggag ctgcggtact cgtcccaaga     360

cgaggagcgc ctggacccct tctcctcctt cagcaagcgg gagcggcaga ggaagtacct     420

gagcttgagt ccctgggaca aggccaccct cagca                                455

<210>  379

<211>  477

<212>  DNA

<213>  Homo sapiens


<400>  379

gcggactacg ccatgttggg gttggggggt cgggtggccg ctgctccggc ctccggggcc 60
ttgcggagac tcaccccttc agcgtcgctg cccccagctc agctcttact gcgggccgct 120
ccgacggcgg tccatcctgt cagggactat gcggcgcaca catctccttc gccaaaagca 180
ggcgccgcca ccgggcgcat cgtggcggtc attggcgcag tggtggacgt ccagtttgat 240
gagggactac caccaattct aaatgccctg gaagtgcaag gcagggagac cagactggtt 300
ttggaggtgg cccagcattt gggtgagagc acagtaagga ctattgctat ggatggtaca 360
gaaggcttgg ttagaggcca caaagtactg gattctggtg caccaatcaa aattcctgtt 420
ggtcctgaga ctttgggcag aatcatgaat gtcattggag aacctattga tgaaaga 477

<210> 380
<211> 526
<212> DNA
<213> Homo sapiens

<400> 380
ggtggcggcg aacgcggaga gcacgccatg aaggcctcgg gcacgctacg agagtacaag 60
gtagtgggtc gctgcctgcc cacccccaaa tgccacacgc cgcccctcta ccgcatgcga 120
atctttgcgc ttaatcatgt cgtcgccaag tcccgcttct ggtactttgt atctcagtta 180
aagaagatga agaagtcttc aggggagatt gtctactgtg ggcaggtgtt tgagaagtcc 240
cccctgcggg tgaagaactt cgggatctgg ctgcgctatg actcccggag cggcacccac 300
aacatgtacc gggaataccg ggacctgacc accgcaggcg ctgtcaccca gtgctaccga 360
gacatgggtg cccggcaccg cgcccgagcc cactccattc agatcatgaa ggtggaggag 420
atcgcggcca gcaagtgccg ccggccggct gtcaagcagt tccacgactc caagatcaag 480
ttcccgctgc cccaccgggt cctgcgccgt cagcacaagc cacgct 526

<210> 381
<211> 514
<212> DNA
<213> Homo sapiens

<400> 381
gcctccatga gtagtggtgg tggcagtggt ggcggttatg gcaatcaaga ccagagtggt 60
ggaggtggca gcggtggcta tggacagcag gaccgtggag gccgcggcag gggtggcagt 120
ggtggcggcg gcggcggcgg cggtggtggt tacaaccgca gcagtggtgg ctatgaaccc 180
agaggtcgtg gaggtggccg tggaggcaga ggtggcatgg gcggaagtga ccgtggtggc 240
ttcaataaat ttggtggccc tcgggaccaa ggatcacgtc atgactccga acaggataat 300
tcagacaaca acaccatctt tgtgcaaggc ctgggtgaga atgttacaat tgagtctgtg 360

gctgattact tcaagcagat tggtattatt aagacaaaca agaaaacggg acagcccatg 420

attaatttgt acacagacag ggaaactggc aagctgaaag gagaggcaac ggtctctttt 480

gatgacccac cttcagctaa agcagctatt gact 514

<210> 382

<211> 502

<212> DNA

<213> Homo sapiens

<400> 382
ggccagaggg cgatcccgcc gcgcagcatc cgtggggacc agattgcctg ggtggaaggc 60

catgaaccag gctgtcgaag cattggtgcc ctcatggccc atgtggacgc cgtcatccgc 120

cactgcgcag ggcggctggg cagctatgtc atcaacgggc gcaccaaggc catggtggcg 180

tgttacccag gcaacgggct cgggtacgta aggcacgttg acaatcccca cggcgatggg 240

cgctgcatca cctgtatcta ttacctgaat cagaactggg acgttaaggt gcatggcggc 300

ctgctgcaga tcttccctga aggccggccc gtggtagcca acatcgagcc actctttgac 360

cggttgctca ttttctggtc tgaccggcgg aacccccacg aggtgaagcc agcctatgcc 420

accaagtacg ccatcactgt ctggtatttt gatgccaagg agcgggcagc agccaaagac 480

aagtatcagc tagcatcagg ac 502

<210> 383

<211> 515

<212> DNA

<213> Homo sapiens

<400> 383
gcgcgggcat gtgcagccgg gaacggcggc gggtacagcg agccttcatg cagggccagt 60

tgcgggtggt ggtggccacg gtggcctttg ggatggggct ggaccggcca gatgtgcggg 120

ctgtgctgca tctggggctg cccccaagct tcgagagcta cgtgcaggcc gtgggccggg 180

ccgggcagtg acgggcagcc tgcccactgc cacctcttcc tgcagcccca gggcgaagac 240

ctgcgagagc tgcgcagaca tgtgcacgcc gacagcacgg acttcctggc tgtgaagagg 300

ctggtacagc gcgtgttccc agcctgcacc tgcacctgca ccaggccgcc ctcggagcaa 360

gaaagggccg tgggtgggga gaggcctgtg cccaagtacc cccctcaaga ggctgagcag 420

cttagccacc aagcagcccc aagacccaga aaggtctgca tgggccatga gcgggcactc 480

ccaatacagc ttaccgtaca ggctttggac atgcc 515

<210> 384

<211>  486

<212>  DNA

<213>  Homo sapiens


<400>  384
gcggacgcgt gggcggctgg cggagcagaa cggattgcag ggtcagccat gtcatctgag      60

cctcccccac caccacagcc ccccacccat caagcttcag tcgggctgct ggacacccct     120

cggagccgtg agcgctcacc atcccctctg cggggcaacg tggtcccaag cccactgccc     180

actcgccgga cgaggacctt ctcggcgacg gtgcgggctt cacagggccc cgtctacaaa     240

ggagtctgca aatgcttctg ccggtccaag ggccatggct tcattacccc agctgatggc     300

ggccccgaca tcttcctgca catctctgat gtggaagggg agtatgtccc agtggaaggc     360

gacgaggtca cctataaaaa gtgctccatc ccacccaaga atgagaagct gcaggccgtg     420

gaggtcgtca tcactcacct ggcaccaggc accaagcatg agacctggtc tggacatgtc     480

atcagc                                                                486

<210>  385

<211>  557

<212>  DNA

<213>  Homo sapiens


<400>  385
gggtacgcgg cactcagcgg ccagaaaggc gacgttgtgg acgtttacca gcgggagttc      60

ctggcgctgc gcgatcggtt gcacgcagct gagcaggaga gcctcaagcg ctccaaggag     120

ctcaacctgg tgctggacga gatcaagagg gccgtgtcag aaaggcaggc gctgctagac     180

agagacggca atcgcacctg ggccgcctaa acagaggacc cccgattgaa gccgtggaac     240

ggctcacacc ggcacgtgct gcacctgccc accgtcttcc atcacctgcc acacctgctg     300

gccaaggaga gcagtctgca gcccgcggtg cgcgtgggcc agggccgcac cggagtgtct     360

gtggtgatgg gcataccgag cgtgcggcgc gaggtgcact cgtacctgac tgacactctg     420

cactcgctca tctccgagct gagcccgcag gagaaggagg actcggtcat cgtggtgctg     480

atcgccgaga ctgactcaca gtacacttcg gcagtgacag agaacatcaa ggccttgttc     540

cccacggaga tccattc                                                    557

<210>  386

<211>  551

<212>  DNA

<213>  Homo sapiens

<400> 386
gcccacgcgt ccgcccacgc gtccgaaagg catgaggggt cgtcttcgga atctgtgcca 60

cccggcacta ccatttcgag ggtgaagctc ctcgacacca tggtggacac ttttcttcag 120

aagctggtcg ccgccggcag ctaccagaga ttcactgact gctataagtg cttctaccag 180

ttgcagcctg cgatgacaca gcaaatctat gacaagttta tagctcagtt gcagacatct 240

atccgggagg aaatctctga catcaaagag gaggggaacc tagaagctgt cttgaatgcc 300

ttggataaaa ttgtggaaga aggcaaagtc cgcaaagagc cagcctggcg ccccagcggg 360

atcccagaga aggatctgca cagtgttatg gcaccctact tcctgcagca acgggacacc 420

ctgcggcgcc atgtgcagaa acaggaggcc gagaaccagc agctggcaga tgccgtcctg 480

gcagggcgga ggcaggtgga ggagctgcag ctacaggtcc aggcccagca gcaggcctgg 540

caggctctac a 551

<210> 387

<211> 455

<212> DNA

<213> Homo sapiens

<400> 387
gcgaccgcca tgtacttgga gcactatctg gacagtatcg agaaccttcc ctgcgaactt 60

cagaggaact tccagctgat gcgagagctg gaccagagga cggaagataa gaaagcagag 120

attgacatcc tggctgcaga gtacatctcc acggtgaaga cgctgtctcc agaccagcgc 180

gtggagcgcc tgcagaagat ccagaacgcc tacagcaagt gcaaggaata cagtgacgac 240

aaagtgcagc tggccatgca gacctacgag atggtggata aacacattcg aaggcttgat 300

gcagacctgg cgcgctttga agcagatctg aaggacaaga tggagggcag tgattttgaa 360

agctccggag ggcgagggtt aaaaaaaggc tggggtcaga aagaaaaaag agggtcccgg 420

ggccgaggca ggaggacatc agaggaagac acacc 455

<210> 388

<211> 461

<212> DNA

<213> Homo sapiens

<400> 388
gcatctttcc ttcgttctcc agaaacccct cctgtatccc ccagggaatc acttcctggg 60

gctgtggtag gggctggggc cactgcagag cccccttaca cagccctggc tgactggaca 120

ctgagggagc ggctgctgcc aggccttctc cctgctgccc ctcgaggcag cctcaccagc 180

cagagcagtg ggcgaggcag cgcttcgttc ctgcggcccc cctccacagc cccctctgca 240
ggaggcagct acctcagccc tgctccagga gacaccagca gctgggccag tggccctgag 300
agatggcccc gaagggagca tgtggtgaca gtcagcaaga ggaggaacac atctgtggac 360
gagaactatg agtgggactc ggaattccct ggggacatgg aattgctgga gactttgcac 420
ctgggcttgg ccagctcccg gctcagacct gaagctgagc c 461

<210> 389
<211> 540
<212> DNA
<213> Homo sapiens

<400> 389
   gcccacgcgt ccgccgagtt tgaccgggaa cagcataccc tcaacctccc agcagtcaac 60
   agcatcacct acgtcctccg cttcctggag aaactctgcc acaaccttcg tagtgacaat 120
   ctgtttggca accagccctt tacacagact cacttgtcac tcactgccat agagtacagc 180
   cacagccacg acaggtacct accaggtgaa acctttgtcc tggggaatag tctggcccgc 240
   tccttggaac cacactcaga ctcaatggac tctgcctcaa atcccaccaa ccttgtcagc 300
   acctcccaaa ggcaccggcc cttgctttca tcctgtggcc tcccaccaag cactgcctca 360
   gctgtgcgca ggctatgctc cagggggtcg gaccgatacc tggagagccg cgatgcctct 420
   cgactgagtg gccgggaccc ctcctcatgg acagtcgagg atgtgatgca gtttgtccgg 480
   gaagctgatc ctcagcttgg accccacgct gacctgtttc gcaaacacga gatcgatggc 540

<210> 390
<211> 490
<212> DNA
<213> Homo sapiens

<400> 390
gaagaaaatg gctatgacag tgatagctgg tgcccaccat tgccagtaca aacttactta 60
caccaaggtc tggaagatga actggaagaa gatgatgata gggtcccaac acctcctgtt 120
cgaggcgtgg cttcttctcc tgctatctcc tttggacagc agtccactgc aactcttact 180
ccatccccac gggaagagat gcaacccatg ctgcaggctc acctggatga gttgacaaga 240
gcctatcagt ttgatatagc aaaacaaaca tggcacattc aaagcaataa tcaacctcca 300
cagcctccag ttccaccgtt aggttatgtg tctggagcct tgatttctga tttggaaacg 360
gatgttgcag atgatgatgc cgacgacgaa gaggaagctt tagaaatccc caggcccctg 420
agagcactgg accagactcc tggatccagc atggacaatc tagacagctc tgtgacagga 480
aaagccttta 490

<210> 391

<211> 491

<212> DNA

<213> Homo sapiens

<400> 391
gagtacgtga agcaccgaca caaactggag aatggtctgg ctgcactcag tccctcaagc 60

aagggctcca tggaggctgg cccttacctg ccccgagccc tgcagcagcc tctggaacag 120

ctgactcggt atgggcggct cctggaggag ctcctgaggg aagctgggcc tgagctcagt 180

tctgagtgcc gggcccttgg ggctgctgta cagctgctcc gggaacaaga ggcccgtggc 240

agagacctgc tggccgtgga ggcggtgcgt ggctgtgaga tagatctgaa ggagcaggga 300

cagctcttgc atcgagaccc cttcactgtc atctgtggcc gaaagaagtg ccttcgccat 360

gtctttctct tcgagcatct cctcctgttc agcaagctca agggccctga aggggggtca 420

gagatgtttg tttacaagca ggcctttaag actgctgata tggggctgac agaaaacatc 480

ggggacagcg g 491

<210> 392

<211> 572

<212> DNA

<213> Homo sapiens

<400> 392
gcggacgcgt gggcggacgc gtgggaagta attcaggggc tggaagcttt gaagaatgag 60

cacaattcca ttttacaaag tttgctggag acactgaagt gtttgaagaa agatgatgaa 120

agtaatttgg tggaggagaa atcaaacatg atccggaagt cactggagat gttggagctc 180

ggcctgagtg aggcacaggt tatgatggct ttgtcaaatc acctgaatgc tgtggagtcc 240

gagaagcaga aactgcgtgc gcaggttcgt cgtctgtgcc aggagaatca gtggctacgg 300

gatgaactgg ccaacacgca gcagaaactg cagaagagtg agcagtctgt ggctcaactg 360

gaggaggaga agaagcatct ggagtttatg aatcagctaa aaaaatatga tgacgacatt 420

tccccatccg aggacaaaga cactgattct accaaagagc ctctggatga ccttttcccc 480

aatgatgaag acgacccagg gcaaggaatc cagcagcagc acagcagtgc agccgcggct 540

gcccagcagg gcggctacga gatccccgcg cg 572

<210> 393

<211> 384

<212> DNA

<213>  Homo sapiens

<400>  393
gaggacagcg aggaggtccg gaggatctgg aaccgagcaa tacctctctg ggagctgcct      60
gatcaggagg aggttcagct ggctgataca atgtttggcc ttaccaaggt tacagatgac     120
acactcatgc gtttcagtgt gcgatatctg cgacctgctc gcagtcttgt cttcccttgg     180
ttctcccctg ggggctcagg attacgaggc ctgaagctcc tagaggctaa atgccagggg     240
gatggagtga gctacgagga aaccactatt ccccgaccca gcgcctacca caatctgttt     300
ggattaccac tgattagtcg tcgagatgct gaggtggtac tgacgagtcg tgagcttgac     360
agcctggcct tgaaccagtc cacg                                            384

<210>  394
<211>  495
<212>  DNA
<213>  Homo sapiens

<400>  394
gtggcggctg ggaccctgta cacgtatcct gaaaactgga gggccttcaa ggctctcatc      60
gcttctcagt acagcggggc tcaggtccgc gtgctctccg caccacccca cttccatttt     120
ggccaaacca accgcacccc tgaatttctc cgcaaatttc ctgccggcaa ggtcccagca     180
tttgagggtg atgatggatt ctgtgtgttt gagagcaacg ccattgccta ctatgtgagc     240
aatgaggagc tgcggggaag tactccagag gcagcatccc aagtggtgca gtgggtgagc     300
tttgctgatt cctatatagt gcccccagcc agtacctggg tgttccccac cttgggcatc     360
atgcaccaca acaaacaggc cactgagaat gcaaaggagg aagtgaggcg aattctgggg     420
ctgctggatg cttacttgaa gacgaggact tttctggtgg gcgaacgagt gacattggct     480
gacatcacag ttgtc                                                      495

<210>  395
<211>  394
<212>  DNA
<213>  Homo sapiens

<400>  395
gtgaagatca atggaaaagt ggctgaaaga ccacaacata tgttgatgag agtatctgtt      60
gggatccaca aagaagacat tgatgcagca attgaaacat ataatcttct ttctgagagg     120
tggtttactc atgcttcgcc cactctcttc aatgctggta ccaaccgccc acaactttct     180
agctgttttc ttctgagtat gaaagatgac agcattgaag gcatttatga cactcttaag     240

caatgtgcat tgatttctaa gtctgctgga ggaattggtg ttgctgtgag ttgtattcgg 300

gctactggca gctacattgc tgggactaat ggcaattcca atggccttgt accgatgctg 360

agagtatata acaacacagc tcgatatgtg gatc 394

<210> 396

<211> 434

<212> DNA

<213> Homo sapiens

<400> 396
gagaagatct ggcaccacac cttctacaac gagctgcgcg tggccccgga ggagcaccca 60

gtgctgctga ccgaggcccc cctgaacccc aaggccaaca gagagaagat gactcacatt 120

atgtttgaga ccttcaacac cccggccatg tacgtggcca tccaggccgt gctgttcctc 180

tacgcctctg ggcgcaccac tggcattgtc atggactctg gagacggggt cacccacacg 240

gtgcccatct acgagggcta cgccctcccc cacgccatcc tgcgtctgga cctggctggc 300

cgggacctga ccgactacct catgaagata ctcactgagc gaggctacag cttcaccacc 360

acggccgagc gggaaatcgt gcgcgacatc aaggagaagc tgtgctacgt cgccctggac 420

ttcgagcagg agat 434

<210> 397

<211> 469

<212> DNA

<213> Homo sapiens

<400> 397
   gcccacgcgt ccgcccacgc gtccgcggac gcgtgggttc tggaacatct gaatacaaca 60
   gaccccaaca gtactttccg ctgtgtccag atgttggaat ggtttgagca tcatggtcac 120
   atttgcattg tttttgaact attgggactt agtacttacg acttcattaa agaaaatggt 180
   tttctaccat ttcgactgga tcatatcaaa aagatggcat atcaaatatg caagtctgtg 240
   aattttttgc acagtaataa gttgactcac acagacttaa agcctgaaaa catcttattt 300
   gtgcagtctg actacacaga ggcgtataat cccaaaataa aacgtgatga acgcacctta 360
   ataaatccag atattaaagt tgtagacttt ggtagtgcaa catatgatga cgaacatcac 420
   agtacattgg tatctacaag acattataga gcacctgaag ttattttag 469

<210> 398

<211> 481

<212> DNA

<213>  Homo sapiens

<220>
<221>  misc_feature
<222>  (241)..(241)
<223>  n = a, c, g or t

<220>
<221>  misc_feature
<222>  (307)..(307)
<223>  n = a, c, g or t

<400>  398
ggcgacccgc gccacggccg ctacctgacg gtggctgcca tcttccgggg ccgcatgtcc      60
atgaaggagg tggacgagca gatgctcaac gtgcagaaca agaacagcag ctacttcgtg     120
gagtggatcc ccaacaacgt gaagacggcc gtgtgcgaca tcccgccccg cggcctgaag     180
atgtcggcca ccttcatcgg caacagcacg gccatccagg agctgttcaa gcgcatctcc     240
nagcagttca cggccatgtt ccggcgcaag gccttcctgc actggtacac gggcgagggc     300
atggacnaga tggagttcac cgaggccgag agcaacatga acgacctggt gtccgagtac     360
cagcagtacc aggacgccac ggccgacgaa caaggggagt tcgaggagga ggagggcgag     420
gacgaggcgt aaatgccccc gcgagacggg ttagggaaag cggaggagga aagcgagggg     480
g                                                                     481

<210>  399
<211>  452
<212>  DNA
<213>  Homo sapiens

<220>
<221>  misc_feature
<222>  (194)..(194)
<223>  n = a, c, g or t

<400>  399
gcccacgcgt ccgcccacgc gtccggtttc attgtgggag cagacaatgt gggctccaag      60
cagatgcagc agatccgcat gtcccttcgt gggaaggctg tggtgctgat gggcaagaac     120

accatgatgc gcaaggccat ccgagggcac ctggaaaaca acccagctct ggagaaactg 180

ctgcctcata tccnggggaa tgtgggcttt gtgttcacca aggaggacct cactgagatc 240

agggacatgt tgctggccaa taaggtgcca gctgctgccc gtgctggtgc cattgcccca 300

tgtgaagtca ctgtgccagc ccagaacact ggtctcgggc ccgagaagac ctcctttttc 360

caggctttag gtatcaccac taaaatctcc aggggcacca ttgaaatcct gagtgatgtg 420

cagctgatca agactggaga caaagtggga gc 452

<210> 400

<211> 342

<212> DNA

<213> Homo sapiens

<400> 400
gcgcttcccg gcgcggtcgc agccctcagc ccacttagga taatggcgac agctgaggta 60

ctgaacattg gtaaaaaatt atatgagggt aaaacaaaag aagtctacga attgttagac 120

agtccaggaa aagtcctcct gcagtccaag gaccagatta cagcaggaaa tgcagctaga 180

aaaaaccacc tggaaggaaa agctgcaatc tcaaataaaa tcaccagttg tatttttcag 240

ttattacagg aagcaggtat taaaactgcc ttcaccacaa aatgtgggga gacagctttc 300

attgcaccgc agtgtgaaat gattccaatt gaatgggttt gc 342

<210> 401

<211> 559

<212> DNA

<213> Homo sapiens

<400> 401
gaggatcagc acatcggaga gaagagcttt gccagcattg ctccttccat ctatggtcat 60

gaagacatca agagaggcct ggctctggcc ctgttcggag gggagcccaa aaacccaggt 120

ggcaagcaca aggtacgtgg tgatatcaac gtgctcttgt gcggagaccc tggcacagcg 180

aagtcgcagt ttctcaagta tattgagaaa gtgtccagcc gagccatctt caccactggc 240

cagggggcgt cggctgtggg cctcacggcg tatgtccagc ggcaccctgt cagcagggag 300

tggaccttgg aggctggggc cctggttctg gctgaccgag gagtgtgtct cattgatgaa 360

tttgacaaga tgaatgacca ggacagaacc agcatccatg aggccatgga gcaacagagc 420

atctccatct cgaaggctgg catcgtcacc tccctgcagg ctcgctgcac ggtcattgct 480

gccgccaacc ccataggagg gcgctacgac ccctcgctga ctttctttga gaacgtggac 540

ctcacagagc ccatcatct 559

<210> 402

<211> 485

<212> DNA

<213> Homo sapiens

<400> 402
gcggacgcgt gggctggtgg caaccggggt cgtggtcggg gaggaaaaag aggaaaccag 60

tcggggaaga atgtgatggt ggagccgcat cggcatgagg gtgtcttcat ttgtcgagga 120

aaggaagatg cactggtcac caagaacctg gtccctgggg aatcagttta tggagagaag 180

agagtctcga tttcggaagg agatgacaaa attgagtacc gagcctggaa ccccttccgc 240

tccaagctag cagcagcaat cctgggtggt gtggaccaga tccacatcaa accgggggct 300

aaggttctct acctcggggc tgcctcgggc accacggtct cccatgtctc tgacatcgtt 360

ggtccggatg gtctagtcta tgcagtcgag ttctcccacc gctctggccg tgacctcatt 420

aacttggcca agaagaggac caacatcatt cctgtgatcg aggatgctcg acacccacac 480

aaata 485

<210> 403

<211> 578

<212> DNA

<213> Homo sapiens

<400> 403
gcagtaaccc tagaagactt atataatggt gcaacaagaa aactggctct gcaaaagaat 60

gtgatttgtg acaaatgtga aggtagagga ggtaagaaag gagcagtaga gtgctgtccc 120

aattgccgag gtactggaat gcaaataaga attcatcaga taggacctgg aatggttcag 180

caaattcagt ctgtgtgcat ggagtgccag ggccatgggg agcggatcag tcctaaagat 240

agatgtaaaa gctgcaacgg aaggaagata gttcgagaga agaaaatttt agaagttcat 300

attgacaaag gcatgaaaga tggccagaag ataacattcc atggtgaagg agaccaagaa 360

ccaggactgg agccaggcga tattatcatt gtgttagatc agaaggacca tgctgttttt 420

actcgacgag gagaagacct tttcatgtgt atggacatac agctcgttga agcactgtgt 480

ggcttccaga agccaatatc tactcttgac aaccgaacca tcgtcatcac ctctcatcca 540

ggtcagattg tcaagcatgg agatatcaag tgtgtact 578

<210> 404

<211> 537

<212> DNA

<213>  Homo sapiens

<400>  404
ggctactgct gccggtgttt gcgtgtggct gggagccagg cctggcgagc ggggtgtgtc     60
gcgatgccgg agctggcagt gcagaaggtg gtggtccacc ccctggtgct gctcagtgtg    120
gtggatcatt tcaaccgaat cggcaaggtt ggaaaccaga agcgtgttgt tggtgtgctt    180
ttggggtcat ggcaaaagaa agtacttgat gtatcgaaca gttttgcagt tccttttgat    240
gaagatgaca aagacgattc tgtatggttt ttataccatg attatttgga aaacatgtat    300
ggaatgttta agaaagtcaa tgccagggaa agaatagttg gctggtacca cacaggccct    360
aaactacaca agaatgacat tgccatcaac gaactcatga aaagatactg tcctaattcc    420
gtattggtca tcattgatgt gaagccgaag gacctatggc tgcctacaga agcgtacatt    480
tcagtggaag aagtccatga tgatggaact ccaacctcga aaacatttga acacgtg       537

<210>  405
<211>  426
<212>  DNA
<213>  Homo sapiens

<400>  405
gattccttga gggaagaaga aaggagagaa tctgctgcag cagacgctgg ctttgctctc     60
tttgagaaga agggagcctt agctgaaagg cagcagctca tcccagaatt ggtgcgaaat    120
ggagatgagg agaatcccct caaaaggggt ctggttgctg cttacagtgg tgacagtgac    180
aatgaggagg agctggtgga gaagacttga gaagtgagga agaaaagcta gctgactgga    240
agaagatggc ctgtctgctc tgccggcgcc agttcccgaa caaagatgcc ctagtcaggc    300
accagcaact ctcagacctt cacaagcaaa acatggacat ctatcgacga tccaggctga    360
gcgagcagga gctggaagcc ttggagctaa gggagagaga gatgaaatac cgagaccgag    420
ctgcag                                                               426

<210>  406
<211>  442
<212>  DNA
<213>  Homo sapiens

<220>
<221>  misc_feature
<222>  (368)..(368)
<223>  n = a, c, g or t

<400>  406
gccggcggcc tcactgctat gggccgcaac aagaagaaga agcgagatgg tgacgaccgg      60

cggccgaggc tcgttcttag cttcgacgag gagaagaggc gggagtacct gacaggcttc     120

cacaagcgga aggtcgagcg aaagaaggca gccattgagg agattaagca gcggctgaaa     180

gaggagcaga ggaagcttcg ggaggagcgc caccaggaat acttgaagat gctggcagag     240

agagaagagg ctctggagga ggcagatgag ctggaccggt tggtgacagc aaagacggag     300

tcggtgcagt atgaccaccc caaccacaca gtcaccgtga ccaccatcag tgacctggac     360

ctctcggngg cccggctgct cgggctgacc ccacctgagg gaggggctgg agacaggtct     420

gaggaggagg cgtcatccac gg                                              442

<210>  407

<211>  465

<212>  DNA

<213>  Homo sapiens


<220>

<221>  misc_feature

<222>  (318)..(318)

<223>  n = a, c, g or t


<400>  407
gccaagatgg cggtgctgtc agctcctggc ctgcgcggct tccggatcct tggtctgcgc      60

tccagcgtgg gcccggctgt gcaggcacga ggtgtccatc agagcgtggc caccgatggc     120

ccaagcagca cccagcctgc cctgccaaag gccagagccg tggctcccaa acccagcagc     180

cggggcgagt atgtggtggc caagctggat gacctcgtca actgggcccg ccggagttct     240

ctgtggccca tgaccttcgg cctggcctgc tgcgccgtgg agatgatgca catggcagca     300

ccccgctacg acatggancc gctttggcgt ggtcttccgc gccagcccgc gccagtccga     360

cgtcatgatc gtggccggca cactcaccaa caagatggcc ccagcgcttc gcaaggtcta     420

cgaccagatg ccggagccgg ctacgtggtc tccatgggga gctgc                     465

<210>  408

<211>  547

<212>  DNA

<213>  Homo sapiens

<400> 408
gagaaacctc atgaatgtag tgaatgtggg aaagtcttta gccagaggtc ccaccttgtc 60

acacaccaga aaatccacac tggagagaag ccctatcagt gcactgaatg tgaaaaagcc 120

ttcaggcggc gttcactcct tattcaacgt cggagaattc atagtggtga gaaaccctat 180

gaatgtaagg aatgtgggaa actcttcatg tggcacacag ctttcctcaa acatcagaga 240

ctgcatgctg gagagaaact tgaagaatgt gagaaaacct tcagcaagga tgaggagctt 300

aggggagagc agaaaattca ccaagaagag aaagcttatc tggtgtaatc agtggtggta 360

gggctttcca gggcagctca gacctcatcg gacatcaggt aactcataca ggagagaaac 420

catatgaatg taaagaatgt gggaaaactt tcaatcagag ctcagacctt ctgagacatc 480

atagaattca cagtggagaa aaaccttatg tatgcaacaa atgtgggaaa tcttttaggg 540

gcagctc 547

<210> 409

<211> 488

<212> DNA

<213> Homo sapiens

<400> 409
gtgcggcgct atatcaagaa ccctcgcatt gtgctgctgg attcttctct ggaatacaag 60

aaaggagaaa gccagactga cattgagatt acacgagagg aggacttcac ccgaattctc 120

cagatggagg aagagtacat ccagcagctc tgtgaggaca ttatccaact gaagcccgat 180

gtggtcatca ctgaaaaggg catctcagat ttagctcagc actaccttat gcgggccaat 240

atcacagcca tccgcagagt ccggaagaca gacaataatc gcattgctag agcctgtggg 300

gcccggatag tcagccgacc agaggaactg agagaagatg atgttggaac aggagcaggc 360

ctgttggaaa tcaagaaaat tggagatgaa tactttactt tcatcactga ctgcaaagac 420

cccaaggcct gcaccattct cctccggggg gctagcaaag agattctctc ggaagtagaa 480

cgcaacct 488

<210> 410

<211> 194

<212> DNA

<213> Homo sapiens

<400> 410
gagttcaagg agctgctgac caaaaagggc agctacagat actacttcaa gaaagtgagc 60

gacgagtttg actgtggggt ggtgtttgag gaggttcgag aggacgaggc cgtcctgccc 120

gtctttgagg agaagatcat cggcaaagtg tataaggtgg actgataggc tggtgggctg 180

gccgctgtgc cagg                                                    194

<210>  411

<211>  479

<212>  DNA

<213>  Homo sapiens


<400>  411
gcagtcattg cccttgaatt actaaattct ggatatgaat ttgatgaagg ctccatcatc      60

tttaataaat tcaggtctgt catctcctat aagacagaag aaaagcccat cttttccctt     120

aataccgttg caagtgctga cagcatgagt atctatgacg atattgatgc tgacgtgctg     180

caaaattacc aagaatacaa tctggccaac atcatctact actctctgaa ggagtccacc     240

actagtgagc agagtgccag gatgacagcc atggacaatg ccagcaagaa tgcttctgag     300

atgattgaca aattgacatt gacattcaac cgtacccgcc aagctgtcat cacaaaagag     360

ttgattgaaa ttatctctgg tgctgcagct ctggattaat gaaaatcaag ttccatcctc     420

agacaagagg taaagaagga aaattcagcc agttgatttt tgtttttagc ttactgctg      479

<210>  412

<211>  575

<212>  DNA

<213>  Homo sapiens


<220>

<221>  misc_feature

<222>  (508)..(508)

<223>  n = a, c, g or t


<400>  412
gagacgctgt accccttcag ctcagtcacc gaggaggagc tcaacttcga gaagggggag      60

accatggagg tgattgagaa gccggagaac gaccccgagt ggtggaaatg caaaaatgcc     120

cggggccagg tgggcctcgt ccccaaaaac tacgtggtgg tcctcagtga cgggcctgcc     180

ctgcaccctg cgcacgcccc acagataagc tacaccgggc cctcgtccag cgggcgcttc     240

gcgggcagag agtggtacta cgggaacgtg acgcggcacc aggccgagtg cgccctcaac     300

gagcggggcg tggagggcga cttcctcatt agggacagcg agtcctcgcc cagcgacttc     360

tccgtgtccc ttaaagcgtc agggaagaac aaacacttca aggtgcagct cgtggacaat     420

gtctactgca ttgggcagcg gcgcttccaa ccatggacga gctggtggaa cactacaaaa     480

aggcgcccat cttcaccagc gagcacgngg gagaagctct acctcgtcag ggccctgcag     540

tgacggcgcc ccggccccac actcgcctcc cgggc                                575

<210>  413

<211>  412

<212>  DNA

<213>  Homo sapiens

<400>  413
gaccagatct acggtttcga gggtgaggtg aaggccaagt acacagccca gatgtacgag      60

ctctttagcg aggtgttcga gtggctcccg ttggcccagt gcatcaacgg caaagtgctg     120

atcatgcacg gaggcctgtt cagtgaagac ggtgtcaccc tggatgacat ccggaaaatt     180

gagcggaatc gacaaccccc agattcaggg cccatgtgtg acctgctctg gtcagatcca     240

cagccacaga acgggcgctc gatcagcaag cggggcgtga gctgtcagtt tgggcctgac     300

gtcaccaagg ccttcttgga agagaacaac ctggactata tcatccgcag ccacgaagtc     360

aaggccgagg gctacgaggt ggctcacgga ggccgctgtg tcaccgtctt ct             412

<210>  414

<211>  411

<212>  DNA

<213>  Homo sapiens

<220>

<221>  misc_feature

<222>  (308)..(308)

<223>  n = a, c, g or t

<400>  414
gaaaagcttg tggtgaaggg gggcaaaaaa aagaagcaag ttctgaagtt cactcttgat      60

tgcacccacc ctgtagaaga tggaatcatg gatgctgcca attttgagca gtttttgcaa     120

gaaaggatca aagtgaacgg aaaagctggg aaccttggtg gaggggtggt gaccatcgaa     180

aggagcaaga gcaagatcac cgtgacatcc gaggtgcctt tctccaaaag gtatttgaaa     240

tatctcacca aaaaatattt gaagaagaat aatctacgtg actggttgcg cgtagttgct     300

aacagcanag agagttacga attacgttac ttccagatta accaggacga agaagaggag     360

gaagacgagg attaaatttc atttatctgg aaaaatttgt atgagttctt g              411

<210>  415

<211>  373

<212>  DNA

<213>  Homo sapiens

<400>  415
gaggacatga cctccgagca gctggatgac attttgaagt accacactga gatagtgttt      60

gccaggacct cccctcagca gaagctcatc attgtggaag gctgccaaag acagggtgct     120

atcgtggctg tgactggtga cggtgtgaat gactctccag ctttgaagaa agcagacatt     180

ggggttgcta tggggattgc tggctcagat gtgtccaagc aagctgctga catgattctt     240

ctggatgaca actttgcctc aattgtgact ggagtagagg aaggtcgtct gatctttgat     300

aacttgaaga aatccattgc ttatacctta accagtaaca ttcccgagat caccccgttc     360

ctgatattta tta                                                        373

<210>  416

<211>  602

<212>  DNA

<213>  Homo sapiens

<400>  416
ggtctgtccg ttcggcccgg tccggcccga agcatggccg gcgtcagctt cagcggccac      60

cgcctggagc tgctggcggc ttacgaggag gtgatccgag aggagagcgc ggccgactgg     120

gctctgtaca catatgaaga tggctccgat gacctcaagc ttgcagcatc aggagaaggg     180

ggcttgcagg agctttcggg acactttgag aaccagaagg tgatgtacgg cttctgcagt     240

gtcaaggact cccaagctgc tctgccaaaa tacgtgctca tcaactgggt gggcgaagat     300

gtgcctgatg cccgcaagtg cgcttgtgcc agccacgtgg ctaaggtggc agagttcttc     360

cagggtgtcg acgtgatcgt gaacgccagc agcgtggaag acatagacgc gggtgccatc     420

gggcagcggc tctctaacgg gctggcgcga ctctccagcc ctgtgctgca ccgactgcgg     480

ctgcgagagg atgagaacgc agagcccgtg ggcaccacct accagaagac ggatgcagct     540

gtggaaatga agcggattac cgagagcagt tctgggagca ggccaagaag gaagaagagc     600

tg                                                                    602

<210>  417

<211>  418

<212>  DNA

<213>  Homo sapiens

<400>  417
gagaccattc caaaagccca gcagatgcac actgggcctg tgcttgatgt ctgctggagt      60

gacgatggga gcaaagtgtt tacggcatcg tgtgataaaa ctgccaaaat gtgggacctc     120

agcagtaacc aagcgataca gatcgcacag catgatgctc ctgttaaaac catccattgg     180

atcaaagctc caaactacag ctgtgtgatg actgggagct gggataagac tttaaagttt     240

tgggatactc gatcgtcaaa tcctatgatg gttttgcaac tccctgaaag gtgttactgt     300

gctgacgtga tataccccat ggctgtggtg gcaactgcag agaggggcct gattgtctat     360

cagctagaga atcaaccttc tgaattcagg aggatagaat ctccactgaa acatcagc       418

<210>  418

<211>  395

<212>  DNA

<213>  Homo sapiens

<400>  418
gcctgcatta accagcccca gttccgggct gtcttggggg aagtgaaact gtgtgagaag      60

atggcccagt ttgatgctaa aaagtttgca gagacccaac ctaaaaagga cacaccacgg     120

aaagagaagg gttcacggga agagaagcag aagccccagg ctgagcggaa ggaggagaaa     180

aaggcggctg cccctgctcc tgaggaggag atggatgaat gtgagcaggc gctggctgct     240

gagcccaagg ccaaggaccc cttcgctcac ctgcccaaga gtacctttgt gttggatgaa     300

tttaaagcgc agtactccaa tgaggacaca ctctctgtgg cactgccata tttctgggag     360

cactttgata aggacggctg gtccctgtgg tactc                                395

<210>  419

<211>  265

<212>  DNA

<213>  Homo sapiens

<220>

<221>  misc_feature

<222>  (160)..(160)

<223>  n = a, c, g or t

<400>  419
gatgccccag gacacagaga ctttatcaaa aacatgatta cagggacatc tcaggctgac      60

tgtgctgtcc tgattgttgc tgctggtgtt ggtgaatttg aagctggtat ctccaagaat     120

gggcagaccc gagagcatgc ccttctggct tacacactgn gtgtgaaaca actaattgtc     180

ggtgttaaca aaatggattc cactgagcca ccctacagcc agaagagata tgaggaaatt     240

gttaaggaag tcagcactta catta                                          265

<210>  420

<211>  444

<212>  DNA

<213>  Homo sapiens


<220>

<221>  misc_feature

<222>  (36)..(36)

<223>  n = a, c, g or t


<220>

<221>  misc_feature

<222>  (178)..(178)

<223>  n = a, c, g or t


<220>

<221>  misc_feature

<222>  (212)..(212)

<223>  n = a, c, g or t


<220>

<221>  misc_feature

<222>  (251)..(251)

<223>  n = a, c, g or t


<220>

<221>  misc_feature

<222>  (319)..(319)

<223>  n = a, c, g or t


<400>  420
gcgcttccct ctccgctgcg tccccgcgcg aagatnggca ccgaggggct gcacgagaac      60

gagacgctgg cgtcgctgaa gagcgaggcc gagagcctca agggcaagct ggaggaggag     120

cgagccaagc tgcacgatgt ggagctgcac caggtggcgg agcgggtgga ggccctgngg     180

cagtttgtca tgaagaccag aaggaccctc anaggccacg ggaacaaagt cctgtgcatg 240

gactggtgca nagataagag gaggatcgtg agctcgtcac aggatgggaa ggtgatcgtg 300

tgggattcct tcaccacana caaggagcac gcggtcacca tgccctgcac gtgggtgatg 360

gcatgtgctt atgccccatc gggatgtgcc attgcttgtg gtggtttgga taataaagtg 420

tctgtgtacc ccttgacgtt tgac 444

<210> 421

<211> 517

<212> DNA

<213> Homo sapiens

<400> 421
ggtggcttag agaagaaaac gcaggttctg cagcctgagg agaagaagac tgtggcatac 60

cacgaagcag gccatgcggt tgccggctgg tatctggagc acgcagaccc gcttttaaag 120

gtatccatca tcccacgtgg caaaggacta ggttatgctc agtatttacc aaaagaacaa 180

tacctctata ccaaagagca gctcttggat aggatgtgta tgactttagg tggtcgagtc 240

tctgaagaaa tcttctttgg aagaattaca actggtgctc aagatgactt gagaaaagta 300

actcagagtg catatgccca aattgttcag tttggcatga atgaaaaggt tgggcaaatc 360

tcctttgacc tcccacgtca gggggacatg gtattggaga aaccttacag tgaagccact 420

gcaagattga tagatgatga agtacgaata cttattaatg atgcttataa aagaacagta 480

gctcttctca cagaaaagaa agctgacgtg gagaagg 517

<210> 422

<211> 448

<212> DNA

<213> Homo sapiens

<220>

<221> misc_feature

<222> (385)..(385)

<223> n = a, c, g or t

<400> 422
ggccgcgacg gacgcaagat ggcgacggcg accatagctc tccaggtcaa tggccagcaa 60

ggaggggggt ccgagccggc ggcggcggcg gcagtggtgg cagcgggaga caaatggaaa 120

cctccacagg gcacagactc catcaagatg gagaacgggc agagcacagc cgccaagctg 180

gggctgcctc ccctgacgcc cgagcagcag gaggcccttc agaaggccaa gaagtacgcc 240
atggagcaga gcatcaagag tgtgctggtg aagcagacca tcgcgcacca gcagcagcag 300
ctcaccaacc tgcagatggc ggctcagcgg cagcgggcgc tggccatcat gtgccgcgtc 360
tacgtgggct ctatctacta tgagnctggg gaggacacca tccgccaggc ctttgccccc 420
tttggcccca tcaagagcat cgacatgt 448

<210> 423
<211> 432
<212> DNA
<213> Homo sapiens

<400> 423
gagcagttca cagaaaaaat gcagtcagat atggagaaaa tccaagaatt aagagaggct 60
cagttatact cagtggacgt gactctggac ccagacacgg cctaccccag cctgatcctc 120
tctgataatc tgcggcaagt gcggtacagt tacctccaac aggacctgcc tgacaacccc 180
gagaggttca atctgtttcc ctgtgtcttg ggctctccat gcttcatcgc cgggagacat 240
tattgggagg tagaggtggg agataaagcc aagtggacca taggtgtctg tgaagactca 300
gtgtgcagaa aaggtggagt aacctcagcc ccccagaatg gattctgggc agtgtctttg 360
tggtatggga aagaatattg ggctcttacc tccccaatga ctgccctacc cctgcggacc 420
ccgctccagc gg 432

<210> 424
<211> 621
<212> DNA
<213> Homo sapiens

<220>
<221> misc_feature
<222> (251)..(251)
<223> n = a, c, g or t

<220>
<221> misc_feature
<222> (253)..(253)
<223> n = a, c, g or t

<220>

<221>  misc_feature

<222>  (513)..(513)

<223>  n = a, c, g or t


<400>  424
gagatgaagg ccttagctgt tgaaggaaaa cgaattggaa aggtgcttga caatgctatt      60

gaaacagaaa aaatgattga aaagtatgaa tcacttgcct ctgaccttct ggaatggatt     120

gaacaaacca tcatcattct gaacaatcgc aaatttgcca attcactggt cggggttcaa     180

cagcagcttc aggcattcaa cacttaccgc actgtggaga aaccacccaa atttactgag     240

aaggggactt ngnggaaagt gctgctcttc accattcaga gcaagatgag ggccaacaac     300

cagaaggtct acatgccccg ggaggggaag ctcatctctg acatcaacaa ggcctgggaa     360

agactggaaa aagcggaaca cgaaagagaa ctggctttgc ggaatgagct cataagacag     420

gagaaactgg aacagctcgc ccgcagattt gatcgcaagg cagctatgag ggagacttgg     480

ctgagcgaaa accagcgtct ggtgtctcag gancaacttt gggtttgacc ttcctgcagt     540

tgaggccgcc acaaaaaagc acgaggccat tgagacagac attgccgcat acgaggagcg     600

tgtgcaggct gtggtagccg t                                               621

<210>  425

<211>  438

<212>  DNA

<213>  Homo sapiens


<400>  425
ggcagctctt gggttttttg tggcttcctt cgttattgga gccaggccta caccccagca      60

accatgtcca agggacctgc agttggtatt gatcttggca ccacctactc ttgtgtgggt     120

gttttccagc acggaaaagt cgagataatt gccaatgatc agggaaaccg aaccactcca     180

agctatgtcg cctttacgga cactgaacgg ttgatcggtg atgccgcaaa gaatcaagtt     240

gcaatgaacc ccaccaacac agtttttgat gccaaacgtc tgattggacg cagatttgat     300

gatgctgttg tccagtctga tatgaaacat tggcccttta tggtggtgaa tgatgctggc     360

aggcccaagg tccaagtaga atacaaggga gagaccaaaa gcttctattc cagaggaggt     420

gtcttctatg gttctgac                                                   438

<210>  426

<211>  415

<212>  DNA

<213>  Homo sapiens

<400> 426
gtctcagaca cctttgcccc ttcgccagac aaaaccatcg actgcgatgt catcaccctg 60

atgggcacac cgtctggcac agctgagccc tatgatggca ccaaggctcg cctctatagc 120

ctgcagtcgg acccagcaac ctactgtaat gaaccagatg ggcccccgga attgtttgac 180

gcctggcttt cccagttctg cttggaggag aagaaggggg agatctcaga gctccttgta 240

ggcagcccct ccatccgggc cctctacacc aagatggttc cagcagctgt ttcccattca 300

gaattctggc atcggtattt ctataaagtc catcagttag agcaggagca ggcccggagg 360

gacgccctga agcagcgggc ggaacagagc atctctgaag agcccggctg ggagg 415

<210> 427

<211> 537

<212> DNA

<213> Homo sapiens


<220>

<221> misc_feature

<222> (458)..(458)

<223> n = a, c, g or t


<400> 427
gcggtcctgg agctgctgca gcggccgggg aacgcgcgct gcgcggactg cggcgccccg 60

gatcccgact gggcctccta cactctgggc gtcttcatct gcctgagctg ctcgggaatc 120

caccggaata tcccccaggt cagcaaggtg aagtccgtcc gcctggacgc ctgggaggag 180

gcccaagtgg agttcatggc ctcccacggg aacgacgccg cgagagccag gtttgagtcc 240

aaagtaccct ccttctacta ccggcccacg ccctccgact gccagctcct tcgagagcag 300

tggatccggg ccaagtacga gcgacaggag ttcatctacc cggagaagca ggagccctac 360

tcggcagggt accgtgaggg ttttctctgg aagcgtggcc gggacaacgg gcagtttttg 420

agccggaagt ttgtgctgac agaacgagaa gggtgctnct gaagtatttc aacagaaatg 480

atgccaagga gcccaaggcc gtgatgaaga tcaagcacct gaacgccacc ttccagc 537

<210> 428

<211> 515

<212> DNA

<213> Homo sapiens

<400> 428
gagcgccggg acgaccccgc cgcgcgcatg agccggtctt cgggccgtag cggctccatg 60

gacccctccg gtgcccaccc ctcggtgcgt cagacgccgt ctcggcagcc gccgctgcct 120

caccggtccc ggggaggcgg agggggatcc cgcgggggcg cccgggcctc gcccgccacg 180

cagccgccac cgctgctgcc gccctcggcc acgggtcccg acgcgacagt gggcgggcca 240

gcgccgaccc cgctgctgcc cccctcggcc acagcctcgg tcaagatgga gccagagaac 300

aagtacctgc ccgaactcat ggccgagaag gactcgctcg acccgtcctt cactcacgcc 360

atgcagctgc tgacggcaga aattgagaag attcagaaag gagactcaaa aaaggatgat 420

gaggagaatt acttggattt attttctcat aagaacatga aactgaaaga gcgagtgctg 480

atacctgtca agcagtatcc caagttcaat tttgg 515

<210> 429

<211> 308

<212> DNA

<213> Homo sapiens

<400> 429
gcagagagaa gtggacagca gattgttggc cctcccagga agaaaagtcc caacgagctg 60

gtggatgatc tctttaaagg tgccaaagag catggagctg tagctgtgga gcgagtgacc 120

aagagccctg gagagaccag taaaccgaga ccatttgcag gaggtggcta ccgccttggg 180

gcagcaccag aggaagagtc tgcctatgtg gcaggagaaa agaggcagca ttccagccaa 240

gatgttcatg tagtattgaa actctggaag agtggattca gcctggataa tggagaactc 300

agaagcta 308

<210> 430

<211> 592

<212> DNA

<213> Homo sapiens

<400> 430
gcggacgcgt gggcagtcag tcaagtagcc gttgatgcac agggagctcc agaaatgcca 60

gcttcccaaa gcaaagcatg cgagacacaa cccaagcaga cttctgtagc cagcgcaaac 120

caggtcagca ggaccgagtc tcctcagggg ttacctacag tccagcggga aaatgcaaaa 180

aaagttctga atccacctgc ccctgcaggt gaccatgcaa ggcttgatgg cctgagtaaa 240

atggacacag agaaggctgc ctcggcaaat cacgtgaagc ccaagcctga actcacttcc 300

atacagggcc aaccagcgtc cacgtcacaa cctctgctga aggcacattc tgaagttttt 360

acaaagccct caggccagca gactctctct ccagacagac aggttcccag gcccacagca 420

ctaccgcgga ggcagcccac tgtgcacttc agcgacgtga gcagcgatga tgacgaggac 480
aggcttgtga tagcaacctg atggatttta ttttttattt gctttttttt tatataacac 540
ttaaaggttt ctttgaaaac cctcctttcc ttaaagcaca tttttctgac at 592

<210> 431
<211> 382
<212> DNA
<213> Homo sapiens

<400> 431
gcgcagaagc agaggctcaa gcccatccag aagatggaag gcatgaccaa cggcgtggcg 60
gtggggcagc agccgagcac ggggctgtcg gacatcagcg cccaggtgca gcagtaccag 120
caatttttgg atgcctctcg gagcctccct gacttcacag agctcgacct ccagggcaag 180
gtgctgcctg agggcgtcgg gcccggggac atcaaagcct tccaggtcct gtaccgggaa 240
cactgtgagg ccattgtcga cgtcatggtg aacctgcagt tcaccctggt ggagacgctg 300
tggaagacct tctggaggta caacctcagc cagcccagtg aggcgccacc gctggctgta 360
catgacgagg ccgagaagcg ac 382

<210> 432
<211> 503
<212> DNA
<213> Homo sapiens

<400> 432
gcgctggccg acgccatcaa caccgagttc aagaacaccc gcaccaacga gaaggtggag 60
ctgcaggagc tgaatgaccg cttcgccaac tacatcgaca aggtgcgctt cctggagcag 120
cagaataaga tcctgctggc cgagctcgag cagctcaagg gccaaggcaa gtcgcgcctg 180
ggggacctct acgaggagga gatgcgggag ctgcgccggc aggtggacca gctaaccaac 240
gacaaagccc gcgtcgaggt ggagcgcgac aacctggccg aggacatcat gcgcctccgg 300
gagaaattgc aggaggagat gcttcagaga gaggaagccg aaaacaccct gcaatctttc 360
agacaggatg ttgacaatgc gtctctggca cgtcttgacc ttgaacgcaa agtggaatct 420
ttgcaagaag agattgcctt tttgaagaaa ctccacgaag aggaaatcca ggagctgcag 480
gctcagattc aggaacagca tgt 503

<210> 433
<211> 481
<212> DNA

<213>  Homo sapiens

<220>
<221>  misc_feature
<222>  (374)..(374)
<223>  n = a, c, g or t

<400>  433
gaggaagctc tgcagatcat ccacagtgcc gagccccggc tcctcccaga tggggcggcc      60
gacggcagct tctacctcca ctcccctgag gggccctcca agccatccct ggcctccccc     120
tacctgcccg aggggacctc caaaccactg tccgacaggc ccaccaaagc accagtgtac     180
atgccacacc ccgagacccc ctcgaaacca tctccctgtc tggtggggga ggcatcgaaa     240
ccgccagccc catccgaggg gtccccgaag gcggtggctt cgtccccagc agccaccaac     300
tccgaggtga aaatgaccag ctttgcagaa cgcaagaaac agctggtgaa ggcagaggct     360
gaggccggag cggngtcccc cacgtccact ccggccccgc cggaggccct gagctcggag     420
atgagtgagc tcagcgcccg gctggaggag aaacgcagag ccatcgaggc tcagaagcga     480
c                                                                     481

<210>  434
<211>  344
<212>  DNA
<213>  Homo sapiens

<400>  434
gaaggatttg taaaccccgg agcgaggttc tgcttacccg aggccgctgc tgtgcggaga      60
cccccgggtg aagccaccgt catcatgtct gaccaggagg caaaaccttc aactgaggac     120
ttgggggata agaaggaagg tgaatatatt aaactcaaag tcattggaca ggatagcagt     180
gagattcact tcaaagtgaa aatgacaaca catctcaaga aactcaaaga atcatactgt     240
caaagacagg gtgttccaat gaattcactc aggtttctct ttgagggtca gagaattgct     300
gataatcata ctccaaaaga actgggaatg gaggaggaaa gatg                      344

<210>  435
<211>  411
<212>  DNA
<213>  Homo sapiens

<400>  435

gctggtggct ccgcggatta taacagcaga gaacatggcg gcccagaggg aatggacccc 60

gatggtgtca tcgagagcaa ctggaatgag attgttgata actttgatga tatgaattta 120

aaggagtctc tccttcgtgg catctatgct tacggttttg agaagccttc cgctattcag 180

cagagagcta ttattccctg tattaaaggg tatgatgtga ttgctcaagc tcagtcaggt 240

actggcaaga cagccacatt tgctatttcc atcctgcaac agttggagat tgagttcaag 300

gagacccaag cactagtatt ggcccccacc agagaactgg ctcaacagat ccaaaaggta 360

attctggcac ttggagacta tatgggagcc acttgtcatg cctgcattgg t 411

<210> 436

<211> 475

<212> DNA

<213> Homo sapiens

<400> 436
gcgctgcccc ctggcaagat cgtggccctc gtgggccagt ctggcggagg aaagaccacc 60

gtggcttccc tgctggagcg cttctacgac cccacggcag gcgtggtgat gctggatggg 120

cgggacctgc gcacccttga cccctcctgg ctccggggcc aggttgtcgg cttcatcagc 180

caggagccca tcctgtttgg gacgaccatc atggaaaaca tccgctttgg gaagctggaa 240

gcttccgatg aagaggtgta cacagccgcc cgggaagcga atgctcacga gttcatcacc 300

agcttccccg agggctacaa cacggtcgtc ggtgaacggg gcactaccct gtctgggggc 360

cagaagcagc gcctggccat cgcccgagcc cttatcaagc agcccacggt gctgatactg 420

gatgaagcta ccagcgcgct ggatgcagag tccgagcggg ttgtacagga ggccc 475

<210> 437

<211> 346

<212> DNA

<213> Homo sapiens

<400> 437
gcccacgcgt ccgggcgtgc cgtggaaagt ggggttccgc agccgccgga tcccccagtc 60

cagcgcgacg aggaagagga aaaagaagtc gaaaatgagg atgaagacga tgatgacagt 120

gacaaggaaa aggatgaaga ggacgaggtc attgacgagg aagtgaatat tgaatttgaa 180

gcttattccc tatcagataa tgattatgac ggaattaaga aattactgca gcagcttttt 240

ctaaaggctc ctgtgaacac tgcagaacta acagatctct taattcaaca gaaccatatt 300

gggagtgtga ttaagcaaac ggatgtttca gaagacagca atgatg 346

<210> 438

<211>  385

<212>  DNA

<213>  Homo sapiens


<400>  438
ggggaagagt ggcaaagggc caaacaggag gcagagagag agaattccaa gtacttggat      60

gaggaactga tggtcctaag ctcaaacagc atgtctctga ccacacggga gccctctctt     120

gccacctggg aagctacctg gagtgaaggg tccaagtctt cagacagaga aggaacccga     180

gcaggagaag aggagaggag acagccacaa gaggaagttg ttcatgagga ccaaggaaag     240

aagccgcagg atcagcttgt tattgagaga gagaggaaat gggagcaaca gcttcaggaa     300

gagcaagagc aaaagcggct tcaggctgag gctgaggagc agaagcgtcc tgcggaggag     360

cagaagcgcc aggcagagat agagc                                           385

<210>  439

<211>  452

<212>  DNA

<213>  Homo sapiens


<400>  439
gcgcgggaga agaaccagcc caagccgtcc ccgaaacgcg agtccggaga ggagttccgc      60

atggagaagt tgaaccagct gtgggagaag gcccagcgac tgcatcttcc tcccgtgagg     120

ctggccgagc tccacgctga tctgaagata caggagaggg acgaactcgc ctggaagaaa     180

ctaaagcttg acggcttgga cgaagatggg gagaaggaag cgagactcat acgcaacctc     240

aatgtcatct tggccaagta tggtctggac ggaaagaagg acgctcggca ggtgaccagc     300

aactccctca gtggcaccca ggaagacggg ctggatgacc ccaggctgga aaagctgtgg     360

cacaaggcga agacctctgg gaaattctcc ggcgaagaac tggacaagct ctggcgggag     420

ttcctgcatc acaaagagaa agttcacgag ta                                   452

<210>  440

<211>  439

<212>  DNA

<213>  Homo sapiens


<400>  440
ggtccccgtt tcccctcccc cttcccttct ccggttgcct tcccgggccc cttacactcc      60

acagtcccgg tcccgccatg tcccagaaac aagaagcagg acacgcagga gaaagaaggt     120

attctgcctg agagagctga agaggcaaag ctaaaggcca aatacccaag cctaggacaa      180

aagcctggag gctccgactt cctcatgaag agactccaga aagggcaaaa gtactttgac    240

tcaggagact acaacatggc caaagccaag atgaagaata agcagctgcc aagtgcagga    300

ccagacaaga acctggtgac tggtgatcac atccccaccc cacaggatct gccccagaga    360

aagtcctcgc tcgtcaccag caagcttgcg ggtggccaag ttgaatgatg ctgcccgggg    420

ctctgccaga tcctgagac                                                 439

<210>  441

<211>  458

<212>  DNA

<213>  Homo sapiens

<220>

<221>  misc_feature

<222>  (368)..(368)

<223>  n = a, c, g or t

<400>  441
gggccctccg ggaagatggc ggccgtgcag gcggccgagg tgaaagtgga tggcagcgag     60

ccgaaactga gcaagaatga gctgaagaga cgcctgaaag ctgagaagaa agtagcagag    120

aaggaggcca aacagaaaga gctcagtgag aaacagctaa gccaagccac tgctgctgcc    180

accaaccaca ccactgataa tggtgtgggt cctgaggaag agagcgtgga cccaaatcaa    240

tactacaaaa tccgcagtca agcaattcat cagctgaagg tcaatgggga agacccatac    300

ccacacaagt tccatgtaga catctcactc actgacttca tccaaaaata tagtcacctg    360

cagcctgngg atcacctgac tgacatcacc ttaaaggtgg caggtaggat ccatgccaaa    420

agagcttctg ggggaaagct catcttctat gatcttcg                           458

<210>  442

<211>  339

<212>  DNA

<213>  Homo sapiens

<400>  442
ggaggctgcg ggcggcggcg ctggacccga cgcggcgaga gaggccccga gatgccgagc     60

aagaagaaga agtacaacgc gcggttcccg ccggcgcgga tcaagaagat catgcagacg    120

gacgaagaga ttgggaaggt ggcggcggcg gtgcctgtca tcatctcccg ggcgctcgag    180

ctcttcctag agtcgctgtt gaagaaggcc tgccaggtga cccagtcgcg gaacgcgaag    240

accatgacca catcccacct gaagcagtgc atcgagctgg agcagcagtt tgacttcttg    300

aaggacctgg tggcatctgt tcccgacatg caggggggac 339

<210> 443

<211> 445

<212> DNA

<213> Homo sapiens

<400> 443
gctagaggta caactgtgac ccctacaaag gaaactgtat cccttggaaa gcacactgta 60

gctctaggag agaagactga gatcactggg gcaacgacca tgacttctgt gggtcatcag 120

tccatgaccc ctggagagaa ggccctgacc cctgtgggtc atcaatctgt gaccactgga 180

cagaagaccc tgacccctgt gggtcatcag tctgtgagcc ctggaggaac gactatgacc 240

cctgtccatt ttcagactga gacccttaga cagaatacag tggcccctag aaggaaggct 300

gtggcccgtg aaaaggtgac tgtcccctcc agaaacatat cagtcacccc tgaagggcag 360

actatgcctt taagagggga gaatttgact tctgaggtgg gcactcaccc caggatgggt 420

aacttgggtc ttcagatgga agctg 445

<210> 444

<211> 243

<212> DNA

<213> Homo sapiens

<400> 444
gacaatggcc ccgatgggat ggagcccgaa ggcgtcatcg agagtaactg gaatgagatt 60

gttgacagct ttgatgacat gaacctctcg gagtcccttc tccgtggcat ctacgcctat 120

ggttttgaga agccctctgc catccagcag cgagccattc taccttgtat caagggttat 180

gatgtgattg ctcaagccca atctgggact gggaaaacgg ccacatttgc catatcgatt 240

ctg 243

<210> 445

<211> 278

<212> DNA

<213> Homo sapiens

<400> 445
gccaccgact gcctgcagct cctggcctac ggcaaccagg agagcaagct gatcatcctg 60

gccaatggtg ggccccaggc cctcgtgcag atcatgcgta actacagtta tgaaaagctg 120

ctctggacca ccagtcgtgt gctcaaggtg ctatccgtgt gtcccagcaa taagcctgcc 180
attgtggagg ctggtgggat gcaggccctg ggcaagcacc tgaccagcaa cagcccccgc 240
ctggtgcaga actgcctgtg gaccctgcgc aacctctc 278

<210> 446
<211> 375
<212> DNA
<213> Homo sapiens

<400> 446
gcacagagcc ttgatggaag ccctgatcca gcccacagca tgggacctga caccccggag 60
ccacccgagg ctgcactctc acccatgtgc atcgactcag ccaccagtgc tgacaccacg 120
ctggacacga caggggacgt cacagtggaa gatgtgaagg atttcctggg ctcctctgag 180
gagtcagaga agaacctgag gaacctggca gaagacgagg cccacgcctg tgccatcctg 240
atcaaatgag gaaggccaga actatcccga cctccacctg ccctgctcgg agctggagtg 300
ctggatccct ggcacctgcc tggcccctta acatagaacc acgcctgcta acctgtaggc 360
tccactggga cctgc 375

<210> 447
<211> 453
<212> DNA
<213> Homo sapiens

<400> 447
gtgaagattg ctaacaagca gttcacagct gttaaaaatg actacgagat gaccttcaat 60
aacgagactt ccgtcatgcc ctgtgaggac gaccatcatt tacctacggt tcagtttgat 120
ttcacgggga ttgatgacct cgagaacaag tcgaaagact cacttgtaga catcatcggg 180
atctgcaaga gctatgaaga cgccactaaa atcacagtga ggtctaacaa cagagaaggt 240
gccaagagga atatctactt gatggacaca tccgggaagg tggtgactgc tacactgtgg 300
ggggaagatg ctgataaatt tgatggttct agacagcccg tgttggctat caaaggagcc 360
cgagtctctg atttcggtgg acggagcctc tccgtgctgt cttcaagcac tatcattgcg 420
aatcctgaca tcccagaggc ctataagctt cgt 453

<210> 448
<211> 348
<212> DNA
<213> Homo sapiens

<400>  448
gcagaaacac caccagtgat ctctgcggtg gtccacgcca cagatgagga aaagctggcg      60

gtcaccaacc agaagtggac gtgcatgaca gtggacctag aggctgacaa acaggactac     120

ccgcagccct cggacctgtc cacctttgta aacgagacca aattcagttc acccactgag     180

gagttggatt acagaaactc ctatgaaatt gaatatatgg agaaaattgg ctcctcctta     240

cctcaggacg acgatgcccc gaagaagcag gccttgtacc ttatgtttga cacttctcag     300

gagagccctg tcaagtcatc tcccgtccgc atgtcagagt ccccgacg                  348

<210>  449

<211>  878

<212>  DNA

<213>  Homo sapiens


<400>  449
gagttgagac cactacaagg atgcagtgta taactatcta ttcgatgatg aagatacccc      60

accaaaccca aaaaaagaga tctcgtcgac gaaaaaagca ggcattcggg tcaccgtcaa     120

gacccccaag gacaaggagg aaattgtgat ctgcgatcga gcctcggtca aggagttcaa     180

agaggaaatc tcccggaggt ttaaggctca gcaggatcag ctggtcctga tcttcgcagg     240

caagatcctc aaggatgggg acacactgaa ccagcacgga atcaaggacg ggctcactgt     300

ccatctggtc atcaagaccc ctcagaaggc tcaagatcca gctgctgcca acttcatgga     360

gctgcagcag cagatgcagc ggcagctgat gtccaatcct gagatgctgt cacagatcat     420

ggagaacccc ctggtccagg atatgatgtc taaccctgat ctgatgcgtc acatgattat     480

ggccaacccc cagatgcagc agttgatgga gcggaaccct gagatcagcc acatgctcaa     540

taaccctgaa ctcatgaggc agacaatgga gcttgctcgg aatccagcca tgaagcaaga     600

gatgaatgta gaaccagctt gagcggccgc agatctatta attgtagata ctgaaaaacc     660

ccgcaaggtc actttaactg tgcatcgtgc accaatctaa attctttcat ttatacatcg     720

ttttgccttt ttttatgtaa ctatacttcc tctaagttta aattttggcc catgtaactt     780

ctgtactaat aaaatttttt taaatgactt gaaattaatg gccctctttt ttttttggaa     840

cctaaattct ttcattgaaa attatattta cgaagggc                             878

<210>  450

<211>  804

<212>  DNA

<213>  Homo sapiens


<400>  450

tgtcgtagat cttcgtcagc agagcttcac cattgaaggg ctggcggttg gggttattcg 60

caacggcgac tggctggaat tcatgtcgac gaaaaaagca ggcaagaagc gcccgaagcc 120

tggaggatgg aacactgggg gcagccgata cccggggcag ggcagccctg gaggcaaccg 180

ctacccacct cagggcggtg gtggctgggg gcagcctcat ggtggtggct gggggcagcc 240

tcatggtggt ggctgggggc agccccatgg tggtggctgg ggacagcctc atggtggtgg 300

ctggggtcaa ggaggtggca cccacagtca gtggaacaag ccgagtaagc caaaaaccaa 360

catgaagcac atggctggtg ctgcagcagc tggggcagtg gtggggggcc ttggcggcta 420

catgctggga agtgccatga gcaggcccat catacatttc ggcagtgact attaggaccc 480

gtactatcgt gaaaacatgc accggtaccc ccacccaatg tactacaggc ccatggatga 540

atacagcaac cggacaactt tttgcacgaa tgcgccaata taccatcaag cagacacggt 600

caccacacca ccaagggggg gactttaccc ggaacgccgt tataaatatg gacccgtggt 660

tgacaaattt tttttacccc ctcccaaaag ggatttcagg ccttttccca gaagggatgg 720

gcatgggcct tttttctttt ccccgggaac cccccgaacc ttttccccaa ttccgaaagg 780

gggaaacccc atttggccgc ccgt 804

<210> 451

<211> 726

<212> DNA

<213> Homo sapiens

<400> 451
tgtcgtagat cttcgtcagc agagcttcac cattgaaggg ctggcggttg gggttattcg 60

caacggcgac tggctggaat tcatgtcgac gaaaaaagca ggccggaacc aggaccgggc 120

cctgagcaac cttgagagca tccctggagg gtataatgcc ctccgccgca tgtacacgga 180

catccaggag cccatgttca gtgctgcccg ggaacagttt ggcaacaatc ccttctcttc 240

cctggccggg aactccgaca gctcatcctc ccagcctctg cggactgaga atcgagagcc 300

cctccctaac ccctggagcc cctcgccccc cacctcccag gcccccgggt ccggtgggga 360

gggcaccgga ggatcgggga ccagccaggt gcacccgaca gtctcgaacc cctttgggat 420

caatgcggct agcctggggt cagggatgtt caatagccca gaaatgcaag cccttctcca 480

gcagatctct gagaaccccc agctgatgca gaaagtgatc ttagcaccct acatgcgcag 540

catgatgcag aacgctggcc cagacccccg actttgtgtg ttaagatgat gggggaatgt 600

gcccctcttt ctcggggaac cccccaatgt cgggagcagg ttcccgctgt gaggtcccaa 660

gtcttcctgg aggagaatgc cggaacccca cagtcaactt ttccattctt taccaaaccc 720

ccgagt 726

<210> 452

<211>  712

<212>  DNA

<213>  Homo sapiens


<400>  452
tgtcgtagat cttcgtcagc agagcttcac cattgaaggg ctggcggttg gggttattcg      60

caacggcgac tggctggaat tcatgtcgac gaaaaaagca ggcttggaag aggaccggga     120

agcaattcga caggagagag aacagcaagc agctatccag cttgagagag caaagtccaa     180

acctgtagca tttgccgtga agacaaatgt gagctactgc ggcgccctgg acgaggatgt     240

gcctgttcca agcacagcta tctcctttga tgctaaagac tttctacata ttaaagagaa     300

atataacaat gattggtgga taggaaggct ggtgaaagag ggctgtgaaa ttggcttcat     360

tccaagtcca ctcagattgg agaacatacg gatccagcaa gaacaaaaaa gaggacgttt     420

tcacggaggg aaatcaagtg gaaattcttt ttcaagtctt ggagaaatgg tatctgggac     480

atttcgagca actcccacat taacaggcaa acagaagcaa aaagtgacgg agcacatttc     540

tccttacgat ggtgtaccgc caaggcgtcc ggtggggtta gtggggcccg cacctgaagg     600

gttaccaggt acagacatga tgcagaaagc ccttttttgt tttcttgaac acaagtttga     660

tgggaggaat ttaataacca gaagggcagg tgtccatttc ttttttaaga gg              712

<210>  453

<211>  536

<212>  DNA

<213>  Homo sapiens


<400>  453
tgtcgtagat cttcgtcagc agagcttcac cattgaaggg ctggcggttg gggttattcg      60

caacggcgac tggctggaat tcatgtcgac gaaaaaagca ggcggccagt attttctaaa     120

ggttctcata cctagttatg ctgctggatc tataattggg aagggaggac agacaattgt     180

tcagttgcaa aaagaaactg gagccaccat caagctgtct aagtccaaag atttttaccc     240

aggtactact gagcgagtgt gcttgatcca gggaacggtt gaagcactga atgcagttca     300

tggattcatt gcagaaaaaa ttcgagaaat gccccaaaat gtggccaaga cagaaccagt     360

cagcattcta caaccccaga ccaccgttaa tccagatcgc atcaaacaaa cattgccatc     420

ttccccaact accaccaagt cctctccatc tgatcccatg accacctcca gagctaatca     480

ggtaaagatt atagttccca acagcacaac aggtctgaat atagggaaag ggaggg         536

<210>  454

<211>  836

<212>  DNA

<213>  Homo sapiens

<220>
<221>  misc_feature
<222>  (523)..(523)
<223>  n = a, c, g or t

<220>
<221>  misc_feature
<222>  (756)..(756)
<223>  n = a, c, g or t

<400>  454
tgtcgtagat cttcgtcagc agagcttcac cattgaaggg ctggcggttg gggttattcg      60
caacggcgac tggctggaat tcatgtcgac gaaaaaagca ggcccagccc gcccctacca     120
cccacctcca ctctttcctc cttcccctca accgccagac agcacccctc gacagccaga     180
ggctagcttt gaaccccatc cttctgtgac acccactgga tatcatgctc ccatggagcc     240
ccccacatct cgaatgttcc aggctcctcc tggggcccct ccccctcacc cacagctcta     300
tcctgggggc actggtggag ttttgtctgg acccccaatg ggtcccaagg ggggaggggc     360
tgcctcatca gtgggggccc ctaatggggg taagcagcac cccccaccca ctactcccat     420
ttcagtatca agctctgggg ctagtggtgc tcccccaaca aagccgccta ccactccagt     480
gggtggtggg aacctacctt ctgctccacc accagccaac ttnccccatg tgacaccgaa     540
cctgcctccc ccaccttgcc tgagacccct caacaatgca tcagcctctt ccccctggcc     600
tgggggccca accactacct ggtcatctgc cctctcccac gccatgggac aggtatgggt     660
ggacttcttc ttgcccagaa gagggcccca ctctggcttc tttaccccaa ctctttgctt     720
ctgcttcctt tttgctcagc ggcccccatg aggttnctta atattctcta ttatagcttt     780
gagaagcttc ctttccaggt tttcctcctt tcttggcttc ccttccaagt tccagg         836

<210>  455
<211>  820
<212>  DNA
<213>  Homo sapiens

<220>
<221>  misc_feature

<222>  (466)..(466)
<223>  n = a, c, g or t

<220>
<221>  misc_feature
<222>  (527)..(527)
<223>  n = a, c, g or t

<220>
<221>  misc_feature
<222>  (534)..(535)
<223>  n = a, c, g or t

<220>
<221>  misc_feature
<222>  (540)..(541)
<223>  n = a, c, g or t

<220>
<221>  misc_feature
<222>  (546)..(546)
<223>  n = a, c, g or t

<220>
<221>  misc_feature
<222>  (549)..(549)
<223>  n = a, c, g or t

<220>
<221>  misc_feature
<222>  (552)..(552)
<223>  n = a, c, g or t

<220>
<221>  misc_feature

<222>  (554)..(554)
<223>  n = a, c, g or t

<220>
<221>  misc_feature
<222>  (592)..(592)
<223>  n = a, c, g or t

<220>
<221>  misc_feature
<222>  (595)..(595)
<223>  n = a, c, g or t

<220>
<221>  misc_feature
<222>  (619)..(619)
<223>  n = a, c, g or t

<400>  455
tgtcgtagat cttcgtcagc agagcttcac cattgaaggg ctggcggttg gggttattcg      60
caacggcgac tggctggaat tcatgtcgac gaaaaaagca ggcaccgacg tcatcatagg     120
gttccaggcc tgctgcaggg gctacctggc caggaaagca tttgccaagc ggcagcagca     180
gcttaccgcc atgaaggtcc tccagcggaa ctgcgctgcc tacctgaagc tgcggaactg     240
gcagtggtgg cggctcttca ccaaggtcaa gccgctgctg caggtgagcc ggcaggagga     300
ggagatgatg gccaaggagg aggggctggt gaaggtcaga gagaagcagc tggctgcgga     360
gaacaggctc acggagatgg agacgctgca gtctcagctc atggcagaga aattgcagct     420
gcaggagcag ctccaggcag aaaccgagct gtgtgccgag gctgangagc tccgggcccg     480
cctgaccgcc aagaagcagg aattagaaga gatctgccat gacctanagc ccanngtgan     540
ngagangang ancnctccca caacctgcag ggcggagaga aagagaatgc ancanaacat     600
tcaggagctt gaggagcanc ttgaagaaga ggagagcgcc cgcaaaagct tgatctggag     660
agggtgacca cgaggccaac tgaaaaactg gaggaggacc gtaattctcg aggacccaac     720
tggaacttgc cagggaaaaa cctggtgaga aagaaatatt agttcccccc cccttcccaa     780
aagggggaaa ctttagacct tccagcttaa aaacagcttg                           820

<210>  456

<211>  542

<212>  DNA

<213>  Homo sapiens

<220>

<221>  misc_feature

<222>  (496)..(496)

<223>  n = a, c, g or t

<400>  456
tgtcgtagat cttcgtcagc agagcttcac cattgaaggg ctggcggttg gggttattcg      60

caacggcgac tggctggaat tcatgtcgac gaaaaaagca ggcctggaga aggcggaaga     120

gctctgttcc caggtgaacg aaggggtgcg ggagaaggag aactctgacc ggctggagtg     180

gatccaggcc cacgtgcagt gtgaaggcct gtctgagcaa cttgtgttca attcagtgac     240

caattgcttg gggccgcgca aatttctgca cagtgggaag ctctacaagg ccaagagcaa     300

caaggagctg tatggcttcc ttttcaacga cttcctcctg ctgactcaga tcacgaagcc     360

tttggggtct tctggcaccg acaaagtctt cagccccaaa tcaaacctgc agtataaaat     420

gtataaaaca cctattttcc taaatgaggt tctagtaaaa ttacccaccg acccttctgg     480

agacgagccc atcttncaca tctcccacat tgaccgcgtc tatactctcc gagcagaaag     540

ca                                                                    542

<210>  457

<211>  540

<212>  DNA

<213>  Homo sapiens

<400>  457
tgtcgtagat cttcgtcagc agagcttcac cattgaaggg ctggcggttg gggttattcg      60

caacggcgac tggctggaat tcatgtcgac gaaaaaagca ggctggctgg agcatgtgca     120

gcaggaggac gagcatcaga gaccaagaaa actccacgaa gaggaaaaac tgaaaaggga     180

ggagagtgtc aaaaagaagg atggcgagga aaaaggcaaa caggaagcac aagacaagct     240

gggtcggctt ttccatcaac accaagaacc agcttatcca gctgtccagg caccctggtc     300

cactgcagaa aaaggtccac ttaccatttc tgtacaggaa aatgtaaaag tggtgtatta     360

ccgggcactg tacccctttg aattcagaag ccatgatgaa attactattc agccaggaga     420

cataatcatt ggggatgggg ggatgaaaga ccaactggag aaacccggtg gctttgaggg     480

gaaattaaaa agaaagaccg gggggggttc ccttcaaact aatgacagaa aatttcttta     540

<210>  458

<211>  840

<212>  DNA

<213>  Homo sapiens


<220>

<221>  misc_feature

<222>  (454)..(454)

<223>  n = a, c, g or t


<220>

<221>  misc_feature

<222>  (659)..(659)

<223>  n = a, c, g or t


<400>  458
tgtcgtagat cttcgtcagc agagcttcac cattgaaggg ctggcggttg gggttattcg      60

caacggcgac tggctggaat tcatgtcgac gaaaaaagca ggcgacagct tctgggaggt     120

ggggaactac aagcggaccg tgaagcgcat cgatgacggc caccgtctat gcaacgacct     180

gatgaactgc gtgcaggagc gcgccaagat cgagaaggcg tacgggcagc agctcaccga     240

ctgggccaag cgttggcgcc agctcatcga gaaaggccca cagtatggca gcctggagcg     300

ggcctggggt gccataatga cagaggcaga caaggtgagc gagctgcacc aggaggtgaa     360

gaacaatctg ctgaatgagg acctggagaa ggtgaagaac tggcagaagg acgcctatca     420

caagcagatc atgggtggct tcaaggggac gaangaggct gaagatggct tccgcaggcc     480

cagaagcctt gggcccaaga gaatgaagag cctgaggcag ccaagaaggc ctaccatttt     540

ggcttgcaaa gaggaaaagc ttgtcatgac acgggagatg aacaagagga cggagcattc     600

gtcacacttg gcagcaaaag agctgcagga caaagtgaca agtgcaagcc ggatgtccna     660

agacccaggg gaaggttaga aaggtcttgg agatgttggc cagaacccaa cccattcact     720

ggaaacatgg agcagggttt tggacaatgc cacaatatta gggaaacgcg gttggtcttc     780

taaatggaaa cccaaatggc cggcccttgg accaaataat tccgggcaaa tttttatatc     840

<210>  459

<211>  833

<212>  DNA

<213>  Homo sapiens

<220>

<221> misc_feature

<222> (577)..(577)

<223> n = a, c, g or t

<220>

<221> misc_feature

<222> (612)..(612)

<223> n = a, c, g or t

<220>

<221> misc_feature

<222> (639)..(639)

<223> n = a, c, g or t

<400> 459
tgtcgtagat cttcgtcagc agagcttcac cattgaaggg ctggcggttg gggttattcg 60

caacggcgac tggctggaat tcatgtcgac gaaaaaagca ggcatggaag agctgatccc 120

gctggtcaac cggctgcaag acgccttctc tgccatcggc cagaacgcgg acctcgacct 180

gccgcagatc gctgtggtgg gcggccagag cgccggcaag agctcggtgc tcgagaattt 240

cgtaggcagg gacttcttgc ctcgaggatc tggcattgtc acccgacgtc ccctggtctt 300

gcagctggtc aatgcaacca cagaatatgc cgagttcctg cactgcaagg gaaagaaatt 360

caccgacttc gaggaggtgc gccttgagat cgaggccgag accgacaggg tcaccggcac 420

caacaagggc atctcgccgg tgcctatcaa cctccgcgtc tactcgccgc acgtgctgaa 480

cctgaccctg gtggacctgc ccggaatgac caaggtcccg gtgggggacc aaccttccga 540

catcgagttc cagatccgag acatgcttat gcagttngtc acccaggaga actgcctcat 600

tcttggcgtg tnccccgcca actctgacct ggccaattnt gacgccctca agtcggccaa 660

gagtgggacc ccacggccag ccaccaatcg ggtcattacc aactggacct gatgacaagg 720

gacaacaatc cctgatgtgc tggagaaaag ttggttcccc tgcgcgaggc tacattggag 780

ggtgaccgga gcccaaggaa tttagggcat gggaaccacc ttgacggccg cct 833

<210> 460

<211> 837

<212> DNA

<213> Homo sapiens

<400> 460
tgtcgtagat cttcgtcagc cgagctgcac cattgaaggg ctggcggatg gggttattcg 60
caacggcgac tggctggaat tcatgtcgac gaaaaaagca ggcgacagag atgttgaaag 120
aatccacggc ggtggaatta acacccttga cattgaacct gttgaaggga gatacatgtt 180
atcaggtggt tcagatggtg tgattgtact ttatgacctt gagaactcca gcagacaatc 240
ttattacaca tgtaaagcag tgtgttccat tggcagagat catcctgatg ttcacagata 300
cagtgtggag actgtacagt ggtatcctca tgacactggc atgttcacat caagctcatt 360
tgataaaact ctgaaagtat gggatacaaa tacattacaa actgcagatg tatttaattt 420
tgaggaaaca gtttacagtc atcatatgtc tccagtctcc accaagcact gtttggtagc 480
agttggtact agaggaccca aagtacaact ttgtgacttg aagtctggat cctgttctca 540
cattctacag ggtcacagac aagaaatatt agcagttttc tggtcttcac gttatgacta 600
tattctggca acagcaagtg ctgacagtag agtaaaatta tgggatgtga gaagagcatc 660
aggatgtttg attactcttg atcaccatat tgcgaaaaga gtacaagctg tttgaatagc 720
caacactgtt cataatggga aaggtattgc ttattgttta cagatgatgg actaacctcc 780
tcctggtgga ccaataatcg aagagggttg gaatagttca tggagaaacc acttggt 837

<210> 461
<211> 533
<212> DNA
<213> Homo sapiens

<400> 461
tgtcgtagat cttcgtcagc agagcttcac cattgaaggg ctggcggttg gggttattcg 60
caacggcgac tggctggaat tcatgtcgac gaaaaaagca ggcccagctc cttctccagg 120
ccttgattat gagcctgagg gagtcccaga caacagccca aatctccaga ataacccaga 180
agaaccaaat ccagaggctc cacatgtgct tgatgtgtac ctggaggagt accctccata 240
cccaccccca ccatacccat ctggggagcc tgaagggccc ggagaagact cggtgagcat 300
gcgcccgcct gaaatcaccg ggcaggtctc tctgcctcct ggtaaaagga caaacttgcg 360
taaaactggc tcagagcgta tcgctcatgg aatgagggtg aaattcaacc cccttgcttt 420
actgctagat tcgtcttttg agggagaatt tgaccttgta cagagaatta tttatgaggt 480
tgatgaccca agcctcccca tgatgaaagc atcgcggctc tttacaatgc tgt 533

<210> 462
<211> 818
<212> DNA

<213>  Homo sapiens

<220>
<221>  misc_feature
<222>  (477)..(477)
<223>  n = a, c, g or t

<220>
<221>  misc_feature
<222>  (495)..(495)
<223>  n = a, c, g or t

<400>  462
tgtcgtagat cttcgtcagc agagcttcac cattgaaggg ctggcggttg gggttattcg     60
caacggcgac tggctggaat tcatgtcgac gaaaaaagca ggcagcctgt ccgggatcgg    120
ccaggaactg ggtgctggtg cctatagcat gagtgatgtc cttgcattgc ccatttttaa    180
gcaagaagag tcgagtttgc ctcctgataa tgagaataaa atcctgcctt ttcaatatgt    240
gctttgtgct gctacctctc cagcagtgaa actccatgat gaaaccctaa cgtatctcaa    300
tcaaggacag tcttatgaaa ttcgaatgct agacaatagg aaacttggag aacttccaga    360
aattaatggg caaatggtga agagtatatt ccgtgtggtg ttccatgaca gaaggcttca    420
gtacactgag catcagcagc tagagggctg gagggtggac cgaccctgag acagaantct    480
tgacatagat atccngatgt ctgtgggtat aatcgattct agggcttatc caacggcact    540
aaatacagtg ggagttctgt gggacccttg caagaggaca tcttgtgtta ttcaggggcc    600
ctggtttacc ccagagtcat tttaagaaac atggtggaga aaaggggggc cctttcgagg    660
ccaaattgaa accttcagga gaaatgaaac ggggaaatta ctggccatta acttggccag    720
ttccaaataa attttaaagc ccaaggccca acggaaccca aaacggttgg gagaaaatgg    780
gaaaacaaac ccttttgaaa gggaaaatat attcctct                            818

<210>  463
<211>  858
<212>  DNA
<213>  Homo sapiens

<400>  463
gtttaatacc actacaatgg atgatgtata taactatcta ttcgatgatg aagatacccc     60
accaaaccca aaaaaagaga tctcgtcgac gaaaaaagca ggcgtttacc aaatgaaaag    120

caaacctcgg ggatactgtc tgatcatcaa caatcacaat tttgcaaaag cacgggagaa 180
agtgcccaaa cttcacagca ttagggacag gaatggaaca cacttggatg caggtacagt 240
agaacccaaa agagaaaagt aaaatatttc ttatgcctat tttttttaaa tcaaaaggga 300
gagaacaaaa gctataccaa aagggccatg tttcaagaaa atggatttaa acatatttcc 360
ctgtggaggg gctttgacca cgacctttga agagcttcat tttgagatca agccccacga 420
tgactgcaca gtagagcaaa tctatgagat tttgaaaatc taccaactca tggaccacag 480
taacatggac tgcttcatct gctgtatcct ctcccatgga gacaagggca tcatctatgg 540
cactgatgga caggaggccc ccatctatga gctgacatct cagttcactg gtttgaagtg 600
cccttccctt gctggaaaac ccaaagtgtt ttttattcag gcttgtcagg gggataacta 660
ccagaaaggt atacctgttg agactgattc agaggagcaa ccctatttag aaatggattt 720
atcatcacct taaacgagat atatcccgga tgaggctgac tttttgctgg ggatggccac 780
tgtgaataac tgtgttttct accgaaaccc tgcagaggga acctggtaca tccagtcact 840
ttgccagagc ctggaagg 858

<210> 464
<211> 824
<212> DNA
<213> Homo sapiens

<220>
<221> misc_feature
<222> (798)..(798)
<223> n = a, c, g or t

<400> 464
taccactaca atggatgatg tatataacta tctattcgat gatgaagata ccccaccaaa 60
cccaaaaaaa gagatcgaat tcatgtcgac gaaaaaagca ggcatggact ttagcagaaa 120
tctttatgat attggggaac aactggacag tgaagatctg gcctccctca agttcctgag 180
cctggactac attccgcaaa ggaagcaaga acccatcaag gatgccttga tgttattcca 240
gagactccag gaaaagagaa tgttggagga aagcaatctg tccttcctga aggagctgct 300
cttccgaatt aatagactgg atttgctgat tacctaccta aacactagaa aggaggagat 360
ggaaagggaa cttcagacac caggcagggc tcaaatttct gcctacaggg tcatgctcta 420
tcagatttca gaagaagtga gcagatcaga attgaggtct tttaagttcc ttttgcaaga 480
ggaaatctcc aaatgcaaac tggatgatga catgaacctg ctggatattt tcatagagat 540
ggagaagagg gtcatcctgg gagaaggaaa gttggacatc ctgaaaagag tctgtgccca 600
aatcaacaag agcctgctga agataatcaa cgactatgaa gaattcagca aatgagaccc 660

agcttgagcg gccgctgcag agatctatga atcgtagata ctgaaaaacc ccgcaagttc 720
actttaactg tgcatcgtgc accatctcaa tttctttcat ttatacattc gtttgccctc 780
ttttatgtaa ctatactnct ctaagtttca atcttggcca tgta 824

<210> 465
<211> 1840
<212> DNA
<213> Homo sapiens

<400> 465
gatgttccag attacgctag cttgggtggt catatggcca tggaggcccc ggggatccga 60
attcgcggcc gcgtcgaccc acagatggct gacagatctc ggcaaaagtg catgtctcag 120
agccttgact tatccgaatt ggccaaagct gctaagaaga agctgcaggc gctcagcaac 180
cggctttttg aggaactcgc catggacgtg tatgacgagg tggatcgaag agaaaatgat 240
gcagtgtggc tggctaccca aaaccacagc actctggtga cagagcgcag tgccgtgccc 300
ttcctgcctg ttaacccgga atactcagcc acgcggaatc aggggcgaca aaagctggcc 360
cgctttaatg cccgagagtt tgccaccttg atcatcgaca ttctcagtga ggccaagcgg 420
agacagcagg gcaagagcct gagcagcccc acagacaacc tcgagctgtc tctgcggagc 480
cagagtgacc tcgacgacca acacgactac gacagcgtgg cctctgacga ggacacagac 540
caggagcccc tgcgcagcac cggcgccact cggagcaacc gggcccggag catggactcc 600
tcggacttgt ctgacggggc tgtgacgctg caggagtacc tggagctgaa gaaggccctg 660
gctacatcgg aggcaaaggt gcagcagctc atgaaggtca acagtagcct gagcgacgag 720
ctccggaggc tgcagcgaga gatccacaag ctgcaggcgg agaacctgca gctccggcag 780
cctccagggc cggtgcccac acctccactc cccagtgaac gggcggaaca cacacccatg 840
gcgccaggcg ggagcacaca ccgcagggat cgccaggcct tttccatgta tgaacctggc 900
tctgccctga agccctttgg gggcccccct ggggacgagc tcactacgcg gctgcagcct 960
ttccacagca ctgagctaga ggacgacgcc atctattcag tgcacgtccc tgctggcctt 1020
taccggatcc ggaaaggggt gtctgcctca gctgtgccct tcactccctc ctccccgctg 1080
ctgtcctgct cccaggaggg aagccgccac acgagcaagc tttcccgcca cggcagtgga 1140
gccgacagtg actatgagaa cacgcaaagt ggggacccac tgctggggct ggaagggaag 1200
aggtttctag agctgggcaa agaggaagac ttccacccag agctggaaag cctggatgga 1260
gacctagatc ctgggcttcc cagcacagag gatgtcatct tgaagacaga gcaggtcacc 1320
aagaacattc aggaactgtt gcgggcagcc caggagttca agcatgacag cttcgtgccc 1380
tgctcagaga agatccattt ggctgtgacc gagatggcct ccctcttccc aaagaggcca 1440
gccctggagc cagtgcggag ctcactgcgg ctgctcaacg ccagcgccta ccggctgcag 1500

agtgagtgcc ggaagacagt gcccccagag cccggcgccc cagtggactt ccagctgctg   1560

actcagcagg tgatccagtg cgcctatgac atcgccaagg ctgccaagca gctggtcacc   1620

atcaccaccc gagagaagaa gcagtgacct ctctccccac accctcacct gcaccctagg   1680

acctcactgg ccataggagc tgggccactc cagacattaa tccccacccc aacagagcca   1740

ctggcacaag tgcccttagt gctgccacac tccctagcag ccaggtggcc ctggtgccca   1800

cccctgtcga gcccctaagg aggggaggtt ggggggcagg                          1840

<210>  466

<211>  1633

<212>  DNA

<213>  Homo sapiens

<220>

<221>  misc_feature

<222>  (957)..(957)

<223>  n = a, c, g or t

<220>

<221>  misc_feature

<222>  (1616)..(1616)

<223>  n = a, c, g or t

<400>  466
gatgttccag attacgctag cttgggtggt catatggcca tggaggcccc ggggatccga     60

atcgcggccg cgtcgacgat tcacatggct ccaccttatc caaatctaaa catgattgag    120

acattcatat gtcaagtgtg tgaggaaacc cttgcacata gtgtggattc ccttgagcag    180

ctgactggaa taaggatgct tagacacctc actatgacta ttgactatca cacactgatt    240

gccaactata tgtccgggtt tctctcctta ttaaccacag ccaatgcgag aacgaagttt    300

cacgttctga aaatgctatt gaatttgtct gaaaatcctg ctgtggcaaa aaaactattc    360

agtgccaaag ctctttcaat atttgtgggt ctctttaaca tagaagagac aaatgataat    420

attcaaattg ttattaaaat gtttcagaat atcagtaaca ttataaaaag tggaaagatg    480

tccttaattg atgatgattt cagtcttgag ccgcttattt ctgcatttcg tgaatttgag    540

gagttagcta agcaactaca agcccaaata gacaaccaaa atgatccctg aggtgggaca    600

acaaagttaa tatgattaac cacctgccgc tgatcagcct tatgttccca aagagccctg    660

agtagtgctt tggtgttcac agtctgtttt tttgttgtaa cttatatttt ttaatgctga    720

tgttaacttt gtcaaactct tgttttgagc tggatcattt tgtggatgcc aaatgaatat    780

caaaactgaa aacacatttg ttgatatttg tcttgctgtc cagattgcgg tatttttcag 840
tattaagttt tcaatgaact gtgtcaccta agtaagctac cctgctattc gttgtttaaa 900
tatatggttc tctatttgag tctgtgtttt caataaagtt ctatgttaaa attggcncac 960
aaaaaaaaaa aaaaaacaaa aaaacaaaaa aaaaaacaaa aaaaaccccc ggaaaatttt 1020
agaatcggaa aatccgaaaa accccggaag gttatttaaa ttgggcactg gcccccaatt 1080
aaattttttt aatttaaacc cgggtttgcc tttctttaag aactaaaccc ccttaaattt 1140
aaaacttggc cctgaaccct ttgaatttat aaattttttt aaaggaccaa aattaaagcc 1200
ccattttttt ttggacccaa atttttttaa aaaaaatttc caagggcctt tttaaaagct 1260
ttggaacttt tccccaaagg ttgggacaag ttcccaacaa ggttgcgggt ttgttctcct 1320
cccccaaatt ttccaaaaga tggaaaaggg ccaatcgttg gtaaatccct gtgtccctct 1380
tataaaaccc aaattcttaa ggtttaggct tttctataaa aacggttaaa aaaaatattt 1440
cacacatttt taatgtcttt tgttttaaaa cccaattttt ttctttaaac ttttccctcc 1500
cccaggtgtc tctccatact tacggacccc cttctcctcc cccctccccg cccgcgcggc 1560
ccgctacccc cttaacaaca ctatcacttt tcgcagcttc ctaagtacgc aggccnccct 1620
ccccggcgcc ctc 1633

<210> 467

<211> 1214

<212> DNA

<213> Homo sapiens

<400> 467
gatgttccag attacgctag cttgggtggt catatggcca tggaggcccc ggggatccga 60
attcgcggcc gcgtcgacct ggtccagaag gctctcaagc ctcctccaat aaagctctat 120
cgggaaacaa atgaaccagt gaaaaccaag acccggacct ttaatacaag tacaggcggt 180
ttgcttctgc ctagcgatac caagaggtct cagatctatg ggagtcgtca gattatactg 240
gagaaagagg aaacagaaga gctaaaacgg tttgatgatc caggtttgat gctcatgggt 300
ttcaagccgt tggtactgct gaagaaacac cattacctga ggccctccct gttcgtgtac 360
ccagaggagt cgctggtgat tgggagctca accctgttca gtgctctgct catcaagtgt 420
ctggagaagg aggttgcagc attgtgcaga tacacacccc gcaggaacat ccctccttat 480
tttgtggctt tggtgccaca ggaagaagag ttggatgacc agaaaattca ggtgactcct 540
ccaggcttcc agctggtctt tttacccttt gctgatgata aaaggaagat gccctttact 600
gaaaaaatca tggcaactcc agagcaggtg ggcaagatga aggctatcgt tgagaagctt 660
cgcttcacat acagaagtga cagctttgag aaccccgtgc tgcagcagca cttcaggaac 720
ctggaggcct tggccttgga tttgatggag ccggaacaag cagtggacct gacatataat 780

gaaggttctg gaagcaaaag gcccaaggtg gagtattcag aagaggagct gaagacccac 840

atcagcaagg gtacgctggg caagttcact gtgcccatgc tgaaagaggc ctgccgggct 900

tacgggctga agagtgggct gaagaagcag gagctgctgg aagccctcac caagcacttc 960

caggactgac cagaggccgc gcgtccagct gcccttccgc agtgtggcca ggctgcctgg 1020

ccttgtcctc agccagttaa aatgtgtttc tcctgagcta ggaagagtct acccgacata 1080

agtcgaggga ctttatgttt ttgaggcttt ctgttgccat ggtgatggtg tagccctccc 1140

actttgctgt tccttacttt actgcctgaa taaagagccc taagtttgta ctaaaaaaaa 1200

aaaaaaaaaa aaaa 1214

<210> 468

<211> 706

<212> DNA

<213> Homo sapiens

<400> 468
gatgttccag attacgctag cttgggtggt catatggcca tggaggcccc ggggatccga 60

attcgcggcc gcgtcgaccc ctcgtccacc gctgccgcct ccttcttctg ccgctcctgg 120

tgctgcttgt gtgctcgttt ggtgcggacc tggtacctct tttgtgaagc ggcagctgag 180

gagactccgg cgctcgccat ggccgacgaa aagcccaagg aaggagtcaa gactgagaac 240

aacgatcata ttaatttgaa ggtggcgggg caggatggtt ctgtggtgca gtttaagatt 300

aagaggcata caccacttag taaactaatg aaagcctatt gtgaacgaca gggattgtca 360

atgaggcaga tcagattccg atttgacggg caaccaatca atgaaacaga cacacctgca 420

cagttggaaa tggaggatga agatacaatt gatgtgttcc aacagcagac gggaggtgtc 480

tactgaaaag ggaacctgct tctttactcc agaactctgt tctttaaaga ccaagattac 540

attctcaatt agaaaactgc aatttggttc caccacatcc tgactactac cgtatagttt 600

tctctattct ttcatttccc ccttccccat tcctttattg tacataaagt aactggtata 660

tgtgcacaag catattgcat tttttttttt ttttaactaa acagcc 706

<210> 469

<211> 900

<212> DNA

<213> Homo sapiens

<400> 469
gatgttccag attacgctag cttgggtggt catatggcca tggaggcccc ggggatccga 60

attcgcggcc gcgtcgacct cccctcgtcc accgctgccg cctccttctt ctgccgctcc 120

tggtgctgct tgtgtgctcg tttggtgcgg acctggtacc tcttttgtga agcggcagct 180

gaggagactc cggcgctcgc catggccgac gaaaagccca aggaaggagt caagactgag 240
aacaacgatc atattaattt gaaggtggcg gggcaggatg gttctgtggt gcagtttaag 300
attaagaggc atacaccact tagtaaacta atgaaagcct attgtgaacg acagggattg 360
tcaatgaggc agatcagatt ccgatttgac gggcaaccaa tcaatgaaac agacacacct 420
gcacagttgg aaatggagga tgaagataca attgatgtgt tccaacagca gacgggaggt 480
gtctactgaa aagggaacct gcttctttac tccagaactc tgttctttaa agaccaagat 540
tacattctca attagaaaac tgcaatttgg ttccaccaca tcctgactac taccgtatag 600
ttttctctat tctttcattt cccccttccc cattccttta ttgtacataa agtaactggt 660
atatgtgcac aagcatattg catttttttt tttttttaac taaacagcca atggtatgtt 720
ttgattgaca tcaagtggag acgggatggg gaaaaatact gattctgtga aaataccccc 780
tttctccatt agtggcaggc tcattcagct cttatcttta tattccagta agttattttg 840
ctctcactgt tttaccaaaa aaaaaaacac cccctaaaaa cccttggcat actttgttca 900

<210> 470
<211> 1342
<212> DNA
<213> Homo sapiens

<400> 470
   gatgttccag attacgctag cttgggtggt catatggcca tggaggcccc ggggatccga 60
   attcgcggcc gcgtcgacgc ggcggcggcg gcggcggctg cagctacgaa cgggaccgga 120
   ggaagcagcg ggatggaggt ggatgcagca gtagtcccca gcgtgatggc ctgcggagtg 180
   actgggagtg tttccgtcgc tctccatccc cttgtcattc tcaacatctc agaccactgg 240
   atccgcatgc gctcccagga ggggcggcct gtgcaggtga ttggggctct gattggcaag 300
   caggagggcc gaaatatcga ggtgatgaac tcctttgagc tgctgtccca caccgtggaa 360
   gagaagatta tcattgacaa ggaatattat tacaccaagg aggagcagtt taaacaggtg 420
   ttcaaggagc tggagtttct gggttggtat accacagggg ggccacctga cccctcggac 480
   atccacgtcc ataagcaggt gtgtgagatc atcgagagcc ccctctttct gaagttgaac 540
   cctatgacca agcacacaga tgccacaatg ctgtttgctg agctgaccta cactctggcc 600
   acagaggaag cggaacgcat tggtgtagac cacgtagccc gaatgacagc aacaggcagt 660
   ggagagaact ccactgtggc tgaacacctg atagcacagc acagcgccat caagatgctg 720
   cacagccgcg tcaagctcat cttggagtac gtcaaggcct ctgaagcggg agaggtcccc 780
   tttaatcatg agatcctgcg ggaggcctat gctctgtgtc actgtctccc ggtgctcagc 840
   acagacaagt tcaagacaga tttttatgat caatgcaacg acgtggggct catggcctac 900
   ctcggcacca tcaccaaaac gtgcaacacc atgaaccagt ttgtgaacaa gttcaatgtc 960
   ctctacgacc gacaaggcat cggcaggaga atgcgcgggc tctttttctg atgagggtac 1020
   ttgaagggct gatggacagg ggtcaggcaa ctatcccaaa ggggagggca ctacacttcc 1080
   ttgagagaaa ccgctgtcat taataaaagg ggagcagccc ctgaaaaaaa aaaaaaaaaa 1140
   aaaaaaaaaa aaaaaaaaaa aacacaaaac aaaaaacaaa aaaacaaaaa cacaaccccc 1200
   cccccccggg ggctttttgt ttgtgatatt gttgaaaccc cccgggtggt tctttttatg 1260
   gggggggggg gcggccccac aataaatttt tttttttatt taacacggcg gggcgccctc 1320
   tcttttataa acaaaaacac cc 1342

<210> 471
<211> 1840
<212> DNA
<213> Homo sapiens

<400> 471
ccggggatcc gaattcgcgg ccgcgtcgac cttatctctg ttcaccttct tgaatcttca 60
gtgccagagt tcttcactac cacaaccaag gagggatatg ataggcggcc agtggatata 120
actcctttag aacaaaggaa attaactttt gatacccatg cattggttca ggacttggaa 180
actcatggat ttgacaaaac acaagcagaa acaattgtat cagcgttaac tgctttatca 240
aatgtcagcc tggatactat ctataaagag atggtcactc aagctcaaca ggaaataaca 300
gtacaacagc taatggctca tttggatgct atcaggaaag acatggtcat cctagagaaa 360
agtgaatttg caaatctgag agcagagaat gaggtgatac aaattttaat ttattactta 420
taaaataact gatctcataa taagcatcct acttaaggaa taagttaata agaccaagta 480
ataattccat gtggttagta aacaataagt ttttagattt aaaaacatac tatttgttta 540
accataccta atctttttct atataaatca tagtgctttg catactgaag agaataagag 600
ttacatttta tacatataaa accacataaa acaaaatctt tgagctatat atataaaatt 660
tgtatataat ggtttcagag ctgagagtaa tacggcattt tctattttat ttaatcctgt 720
actaaatgtc tttgtgactt aaaagtatat ttctgtaatt aagtcagaat tttaagttat 780
gttatccact atagcttatt tttaaattta gaaaatcagt ttttaaaagt ataatataaa 840
aacaaatgca ttttgctcat aagaattatt ttctgaatac agtggcttac tagtttcttt 900
tgaagatttt tttccttatc aattctgtat tagaaatcca tagattgtcc aaattttggt 960
aatctagggt ttcagatttt tttaatcata atatagactt ggtttaatgc taattgcatg 1020
tgatctttcc tgtacctaga aaatgaaaat tgaattagac caagttaagc aacaactaat 1080
ggtaagtgaa ttttttttct agctgttaat ttcagaacaa atattcgtta caagatcatt 1140
ttttaaatat ctttgctttc tttaaagcat gaaaccagtc gaatcagagc agataataaa 1200
ctggatatca acttagaaag gagcagagta acagatatgg taatgttctt ttaaatgtac 1260
tttcttggtt acttactcct tttttatttt tagacttttt ttttaacttc ctgctgcttc 1320

atatcctata gtttacagat caagaaaagc aacttatgga aacaactaca gaatttacaa 1380
aaaaggtaaa gtaatatgta aaaatgtctc ttttgtacag ttcttgtgcc ttgtataact 1440
gcttttatgt gtaccagttt ataagtttag acttttaaaa actacccttg tagagtctct 1500
ggcttcctca tagctattat agttaaccag cacttttggg gaacctggca ccaggtagat 1560
ggtcgcccat gagccatgcc cttgaagtaa gaatcaagat ttccacatag tgtgcttttt 1620
taaactaaat tacttctata atagaaaata actatttttt gggccaggca cagtagctca 1680
cacctgttaa tcctgcactt tgggaggctg aggcaggagt tcaagaccag tctgggcaac 1740
atagcaagac cctgtctcaa tatcaaaaaa aaaaaaaaac aaaaaaaaaa aactcgagag 1800
atctatgaat cgtagatact gaaaaacccc gcaagttcac 1840

<210> 472
<211> 1648
<212> DNA
<213> Homo sapiens

<220>
<221> misc_feature
<222> (1429)..(1429)
<223> n = a, c, g or t

<220>
<221> misc_feature
<222> (1431)..(1432)
<223> n = a, c, g or t

<220>
<221> misc_feature
<222> (1438)..(1439)
<223> n = a, c, g or t

<220>
<221> misc_feature
<222> (1441)..(1441)
<223> n = a, c, g or t

<220>
<221> misc_feature
<222> (1450)..(1453)
<223> n = a, c, g or t

<220>
<221> misc_feature
<222> (1455)..(1457)
<223> n = a, c, g or t

<220>
<221> misc_feature
<222> (1459)..(1461)
<223> n = a, c, g or t

<220>
<221> misc_feature
<222> (1463)..(1464)
<223> n = a, c, g or t

<220>
<221> misc_feature
<222> (1467)..(1468)
<223> n = a, c, g or t

<220>
<221> misc_feature
<222> (1470)..(1471)
<223> n = a, c, g or t

<220>
<221> misc_feature
<222> (1473)..(1485)
<223> n = a, c, g or t

<220>

<221>  misc_feature

<222>  (1495)..(1496)

<223>  n = a, c, g or t


<220>

<221>  misc_feature

<222>  (1500)..(1514)

<223>  n = a, c, g or t


<220>

<221>  misc_feature

<222>  (1572)..(1572)

<223>  n = a, c, g or t


<220>

<221>  misc_feature

<222>  (1612)..(1612)

<223>  n = a, c, g or t


<400>  472
ccggggatcc gaattcgcgg ccgcttcgac cggcggcgat cggacggctg catttacggg      60

gtctcccgga gggccagagt cgtggcttac agaagagacg aaatgtggtc tgagggacga     120

tatgaatatg aaagaattcc gagagaacga gcacctcctc gaagtcatcc cagtgatgaa     180

tctggttata gatggacaag agacgatcat tctgcaagca ggcaacctga atacagggac     240

atgagagatg gctttagaag aaaaagtttc tactcttccc attatgcgag agagcggtct     300

ccttataaaa gggacaatac ttttttcaga gaatcacctg ttggccgaaa ggattctcca     360

cacagcagat ctggttccag tgtcagtagc agaagctact ctccagaaag gagcaaatca     420

tactctttcc atcagtctca acatagaaat aaagagaggc ctgtccagtc tttgaaaaca     480

tcaagagata cttcaccctc aagtggttca gcagtttctt catcaaaggt gttagacaaa     540

cccagtaggc taactgaaaa ggaacttgct gaggctgcaa gcaagtgggc tgctgaaaag     600

ctagagaaat cagatgaaag taacttgcct gaaatttctg agtatgaggc gggatccaca     660

gcaccattgt ttactgacca gccagaggaa cctgagtcaa acacaacaca tgggatagaa     720

ttatttgaag atagtcagct aaccactcgc tctaaagcaa tagcatcaaa aaccaaagag     780

attgaacagg tttaccgaca agactgtgaa actttcggga tggtggtgaa aatgctgatt 840
gaaaaagatc cttcattaga aaagtctata cagtttgcat tgaggcagaa tttacatgaa 900
ataggtgagc ggtgtgttga agaactcaag catttcattg cagagtatga tacttccact 960
caagattttg gagagccttt ttagattttt ctgctcaggc taaaaaaaaa aaaaaaacag 1020
tttctaaaaa ttttttttcc tggattgtgt aaatccttaa tatatggaat ttttgtgatg 1080
cagagaaata ctttatgata gttgacaaca tttcagtatt aaataaacat ctaaaatagt 1140
ctgtttaaaa tgtttgattc aaatttttgt atttttttta gagatggggt tcaccatgtt 1200
ggccaggcta gtctctaact cctggcctca agtgatctgc ctgcctcagc ctctcaaagt 1260
gttgagatta caggcgtgag ccaccgctcc ctgcccacac atataccatc tgaaatgtta 1320
gaattctgag ttgggatttt attgacttgt tgcttgcttt ttcttagctt tggaacttga 1380
atatgttaaa gggactatcc tataaactga atccttggga tctttgggna nnaaaaanna 1440
naaaaataan nnntnnnann nannaannan nannnnnnnn nnnnnccccc ccccnngccn 1500
nnnnnnnnnn nnnntttttt tttttgtttt ttacaccccc ggcccccccc cgcgccccgc 1560
ccccccaccc cntctttata caggccgcgc ctttcttttg cacccccccc gnacaacccg 1620
cgcccccccc cctttaaata taaaaaaa 1648

<210> 473

<211> 1109

<212> DNA

<213> Homo sapiens


<220>

<221> misc_feature

<222> (1092)..(1092)

<223> n = a, c, g or t


<220>

<221> misc_feature

<222> (1095)..(1095)

<223> n = a, c, g or t


<400> 473
ccggggatcc gaattcgcgg ccgcgtcgac gtcgacgcgg tcgcggtcga cgcggccgcg 60
gtttctgcaa aggcagaaaa agtacatgag ttaaatgaaa aaataggaaa actccttgct 120
aaagccgaac agctaggggc tgaaggtaat gtggatgaat cccagaagat tcttatggaa 180
gtggaaaaag ttcgtgcgaa gaaaaaagaa gctgaggaag aatacagaaa ttccatgcct 240

gcatccagtt ttcagcagca aaagctgcgt gtctgcgagg tctgttcagc ctaccttggt 300
ctccatgaca atgaccgtcg cctggcagac cacttcggtg gcaagttaca cttggggttc 360
attcagatcc gagagaagct tgatcagttg aggaaaactg tcgctgaaaa gcaggagaag 420
agaaatcagg atcgcttgag gaggagagag gagagggaac gggaggagcg tctgagcagg 480
aggtcgggat caagaaccag agatcgcagg aggtcacgct cccgggatcg gcgtcggagg 540
cggtcaagat ctacctcccg agagcgacgg aaattgtccc ggtcccggtc ccgagataga 600
catcggcgcc accgcagccg ttcccggagc cacagccggg gacatcgtcg ggcttcccgg 660
gaccgaagtg cgaaatacaa gttctccaga gagcgggcat ccagagagga gtcctgggag 720
agcgggcgga gcgagcgagg gcccccggac tggaggcttg agagctccaa cgggaagatg 780
gcttcacgga ggtcagaaga gaaggaggcc ggcgagatct gaacccgtct cccgggtgct 840
gtaaatagtc tgataaacgt tcacacagtc taaaattacc ctttatattt gctgaataca 900
actcatcttt tgtagtttaa aatttctatt gttttggagc tagctgtgag tttctagaag 960
tgtacagagt tgctcctgtg ttcccgggtc atgttgagta ggaataaata aatctgatgc 1020
tgccaaaaaa aaaaaaaaaa aactcgagag atctatgaat cgtagatact gaaaaacccc 1080
gcaagttcac tncanacgtg agggtgtaa 1109

<210> 474
<211> 970
<212> DNA
<213> Homo sapiens

<400> 474
aaaaaagcag gcatggtcgg cggtggcggg aagcgcaggc ccggcgggga ggggccgcag 60
tgtgaaaaaa caactgatgt gaagaaaagt aaattctgtg aagctgatgt ctccagtgac 120
cttcgaaaag aagtagaaaa tcattataag ctttctttac ctgaagattt ctatcacttc 180
tggaagttct gtgaagaact tgatcctgaa aagccatctg attcactttc tgcaagcctt 240
ggacttcaat tagttggtcc ttatgatatc cttgctggaa aacataaaac gaagaaaaaa 300
tcaacaggcc tgaattttaa ccttcactgg aggttttact atgatcctcc tgagttccag 360
accattatta ttggagataa taaaactcag taccacatgg ggtatttcag attatttttg 420
acgaaaaaac ttagagaaat aacggataaa aagaaaatca atctcttgaa aaacatagat 480
gaaaaactca cagaagcagc cagagaattg gggtactcgc ttgaacagag aaccgtgaag 540
atgaaacaga gagataagaa agttgtgaca aagacctttc atggtgcagg cttggttgtt 600
ccagtagata aaaatgatgt tgggtaccga gagctccctg aaacagatgc tgacctcaag 660
agaatttgca agacaatagt tgaggctgca agtgatgagg agagactaaa agcttttgct 720
cccattcagg aaatgatgac ttttgtgcag tttgctaatg atgaatgtga ttatggcatg 780

gggcttgaat tgggaatgga tctcttttgc tatggctcac attattttca taaagttgct 840
ggccagcttt tacctcttgc atataatctg ttgaagagga atctgtttgc agaaattatt 900
gaggagcatc tggcaaacag aagtcaagag aacatagacc aacttgctgc ttaattaacc 960
cagctttctt 970

<210> 475
<211> 827
<212> DNA
<213> Homo sapiens

<400> 475
aaaaaagcag gcatgctgga agccatggcg gagcccagtc ccgaagatcc acctccgacc 60
cttaagccag agactcagcc accagagaaa cggcggagaa caattgagga tttcaacaaa 120
ttctgcagtt ttgttttggc atatgctggt tacattcccc ctagcaaaga ggaaagtgac 180
tggccagcct ctggctccag ctctccattg cgaggagaga gtgcggccga cagtgatggc 240
tgggactcgg ccccctcaga tcttcgaacc atccagactt ttgttaagaa agcaaagtca 300
tccaagagaa gggcagctca agcaggtccc acccagccag gacccccaag gtccactttc 360
tctcgtctgc aggcccccga cagtgctacc ttgcttgaga agatgaagct caaggactct 420
ctctttgatc tggatgggcc caaagtggca tctcctttgt cccccacatc cctgacacat 480
acctcccggc cccctgctgc tcttaccccc gtgccccttt cccaggggga cctctcccat 540
cctcctcgaa agaaggaccg aaagaaccga aagttggggc caggagctgg ggctggcttt 600
ggggtgcttc ggaggcctcg gccaactcct ggggatgggg aaaagagatc tcgaatcaag 660
aagagcaaga agcggaagtt aaaaaaggca gaacgggggg atagactccc acctcctggg 720
catccccagg caccccccag tgatacagac tctgaagagg aggaggaaga ggaggaagag 780
gaagaagaag aagagatggc aacagtgtta attaacccag ctttctt 827

<210> 476
<211> 865
<212> DNA
<213> Homo sapiens

<400> 476
aaaaaagcag gcatggataa atcaggaata gattctcttg accatgtgac aactgatgct 60
gtggaacttg caaatcgaag tgataactct tctgatagca gcttatttaa aactcagtgt 120
atcccttact cacctaaagg ggagaaaaga aaccccattc gaaaatttgt tcgtacacct 180
gaaagtgttc acgcaagtga ttcatcaagt gactcatctt ttgaaccaat accattgact 240
ataaaagcta tttttgaaag attcaagaac aggaaaaaga gatataaaaa aaagaaaaag 300

gggaggtacc agccaacagg aagaccacgg ggaagaccag aaggaaggag aaatcctata 360
tactcactaa tagataagaa gaaacaattt agaagcagag gatctggctt cccattttta 420
gaatcagaga atgaaaaaaa cgcaccttgg agaaaaattt taacgtttga gcaagctgtt 480
gcaagaggat tttttaacta tattgaaaaa ctgaagtatg aacaccacct gaaagaatca 540
ttgaagcaaa tgaatgttgg tgaagattta gaaaatgaag attttgacag tcgtagatac 600
aaatttttgg atgatgatgg atccatttct cctattgagg agtcaacagc agaggatgag 660
gatgcaacac atcttgaaga taacgaatgt gatatcaaat tggcagggga tagtttcata 720
gtaagttctg aattccctgt aagactgagt gtatacttag aagaagagga tattactgaa 780
gaagctgctt tgtctaaaaa gagagctaca aaagccaaaa atactggaca gagaggcctg 840
aaaatttaat taacccagct ttctt 865

<210> 477
<211> 700
<212> DNA
<213> Homo sapiens

<400> 477
aaaaaagcag gcatggaaag actcttagga ggacaactag gactagaagg tttcatattt 60
gcccatgtga aaggaatcga aaaagaagtg aatgtgtata aatctgagga ttcacttggt 120
ctcaccatta cagataatgg tgttggctat gcttttataa agagaattaa agatggtggt 180
gttattgact cagttaaaac aatctgtgtt ggggatcata ttgaatccat aaatggagaa 240
aatattgttg ggtggcgtca ctatgatgtt gctaagaagt taaaggaatt aaaaaaggag 300
gaactcttta ctatgaagtt aatagaacct aagaaggcat ttgaaataga gccgaggtca 360
aaggctggaa agtcatcagg agaaaaaatt ggttgtggaa gggcaacact tcgcctgaga 420
tcaaaaggtc ctgccaccgt ggaagaaatg ccttctgaaa ccaaagcaaa ggcaattgaa 480
aagattgatg atgttcttga gttgtacatg ggaattcgag atattgattt agccaccaca 540
atgtttgaag ctggaaagga caaagtaaat ccagatgaat ttgctgtggc acttgacgaa 600
actcttggag actttgcgtt cccagacgaa tttgtctttg atgtttgggg agtcattggt 660
gatgccaaac gaagaggatt ttaattaacc cagctttctt 700

<210> 478
<211> 601
<212> DNA
<213> Homo sapiens

<400> 478

aaaaaagcag gcatggttca gcaggatgtt cagggcctta aagcacagtc agcaggactc 60
ggcatctccc agcacctgct ctccggttgt catggtaaca tcatccccaa cccaaccacc 120
ttgtccagcc gagagacagc aatcataagg agggacctcg gtttcccccg aggatcctgg 180
gcttcctttc tgaaacgctt gcttctgagc tcagcaacca ggaacaccag gccagcccat 240
ccccagcacc tctgtggaga tgagggacaa agtcctacag tccctcttcc tgttctgatg 300
agaaagggag ggaagaaaac ataccccgag cgcctgcaat atggtcatga cactttcaaa 360
aagcctgtgc tatggagtca tgatcagaaa ccagagtgtg gagagggtca gcagcctgcc 420
tcagagcagc cagctaggcg gggagtggta aatttgggac ttgtacccag gcatgactgg 480
ctccgagccc agtgctccac tctatggaat gttccctggg cctcagttgc tttcctttcc 540
tttgcaggcc gcgggctgct gccactctgg cagctggtga gttaattaac ccagctttct 600
t 601

<210> 479
<211> 586
<212> DNA
<213> Homo sapiens

<400> 479
aaaaaagcag gcatggcagc gcagaaggac cagcagaaag atgccgaggc ggaagggctg 60
agcggcacga ccctgctgcc gaagctgatt ccctccggtg caggccggga gtggctggag 120
cggcgccgcg cgaccatccg gccctggagc accttcgtgg accagcagcg cttctcacgg 180
ccccgcaacc tgggagagct gtgccagcgc ctcgtacgca acgtggagta ctaccagagc 240
aactatgtgt tcgtgttcct gggcctcatc ctgtactgtg tggtgacgtc ccctatgttg 300
ctggtggctc tggctgtctt tttcggcgcc tgttacattc tctatctgcg caccttggag 360
tccaagcttg tgctctttgg ccgagaggtg agcccagcgc atcagtatgc tctggctgga 420
ggcatctcct tcccctcttc ctggctggct ggtgcgggct cggccgtctt ctgggtgctg 480
ggagccaccc tggtggtcat cggctcccac gctgccttcc accagattga ggctgtggac 540
ggggaggagc tgcagatgga acccgtttaa ttaacccagc tttctt 586

<210> 480
<211> 503
<212> DNA
<213> Homo sapiens

<400> 480
aaaaaagcag gcatgaaaaa gccaagtgaa cgtggcagag tgaggaggag acaggagcgt 60
gtgcaccttc catctgtgag aggcacactt cagtctgggt tcaagatgca gaatggtgcc 120

tacagcaaaa aaaaaaaaca ccctcctccc ttctttacca tttgaatgga cattttcctt 180

acctgtgatc ccaacagaaa cagatccaga cctatcatgt gaagtccacg ttccaggatc 240

agaagtaacc agtttatgga ctgagcttac acgggaaagt ctacccccga ctccttctgg 300

atagtaacat acacagctgc ataaaaacgt ctccaagggg acatacgatg catttgcttg 360

gtgtcccagc caagctcccc accggcgacc tcactgttcc ttagagctcg agagctcgtc 420

tcctatcaat cagagaaccc catcagctgt gaccaacaga gctggagccc tctgtggagg 480

gagctgaccc acccagcttt ctt 503

<210> 481

<211> 517

<212> DNA

<213> Homo sapiens

<220>

<221> misc_feature

<222> (298)..(298)

<223> n = a, c, g or t

<400> 481
aaaaaagcag gcatgctccc tcacaacatc ccgtccagcc tgagcctgct caccagcatg 60

gtggatgaca tgtggcatta cgctggggac cagtccactg attttaactg gtacacccgc 120

cgagccatgc tggctgccat ctacaacaca acagagctgg tgatgatgca ggactcctct 180

ccagactttg aggacacttg gcgcttcctg gaaagccggg ttaatgatgc aatgaacatg 240

ggccacactg ccaagcaggt aaagtccaca ggagaggcac tggtgcaagg actcatgngt 300

gcagcagtga cgctcaagaa cttgacaggt ctaaaccagc gtcggtgaga ggaaggggta 360

taagctacaa tgcctagaag agaatgagcg gacagattga aagagctttg aaaagtataa 420

ggtgccatcc acataacctg gtgttcacga gaacacacta aaggactcct gagtcactac 480

cacagccacc tggaaaccac aaggacccag ctttctt 517

<210> 482

<211> 972

<212> DNA

<213> Homo sapiens

<400> 482
aaaaaagctg gcacccctgg tccaggtatg gcttccagaa aagagaatgc gaagagtgca 60

aacagagtgc taagaataag ccagttggat gcacttgaac taaacaaggc cctggagcag 120
ctagtttggt cccagtttac tcagtgcttt catggattta aacctgggct gttagctcgc 180
tttgagccag aggtgaaagc gtgcttatgg gttttcttgt ggagattcac catctactcc 240
aaaaatgcca cagtgggaca gtcagttttg aatattaagt acaaaaatga tttttcccct 300
aacctgagat atcagccacc cagtaaaaat caaaaaatct ggtatgctat ttgtacaatt 360
ggtggcaggt ggttagaaga acgatgctat gatttgtttc gaaaccatca tttagcatca 420
tttgggaaag tcaagcagtg tgtgaatttt gtgattggac ttttgaaatt aggtgggctg 480
attaattttt ttgattttcc ttcagagggg aaagtttgca actttgacag aacgtctcct 540
aggtattcat tctgtatttt gcaagcctca aaacatacgt gaagttggct ttgaatacat 600
gaatagggaa cttctctggc atggttttgc tgaatttctg atttttctct taccacttat 660
caatgtccag aagttgaaag ccaagctgtc ttcatggtgt attcctctta ctggtgcacc 720
taatagtgac aatacattag ccaccagtgg caaagaatgc gctctatgtg gagagtggcc 780
caccatgcct cacaccatag gatgtgagca tattttctgt tatttctgtg ctaagagtag 840
tttcttattt gacgtgtact ttacttgtcc taagtgtggc acagaagtac acagtctgca 900
gccactgaaa tcaggaatcg agatgtcaga agtaaatgct cttccaggcc caggcggcga 960
cccagctttc tt 972

<210> 483
<211> 575
<212> DNA
<213> Homo sapiens

<400> 483
aaaaaagcag gcatgaggtt gaatcagaac accttgctgc tggggaagaa ggtggtcctt 60
gtaccctaca cctcggagca tgtgcccagc aggtaccacg agtggatgaa atcagagggg 120
ctgcagcgtt tgacagcctc ggagccgctg accctggagc aggagtatgc catgcagtgc 180
agctggcagg aagatgcaga caagtgtacc ttcattgtgc tggatgccga gaagtggcag 240
gcccagccag gcgccaccga agagagctgc atggtgggag atgtgaacct cttcctcaca 300
gatctagaag acctcacctt gggggagatc gaggtcatga ttgcagagcc cagctgcagg 360
ggtaagggcc ttggcactga ggccgttctc gcgatgctgt cttacgaaac ttcactttga 420
gcaggtggct acgagcagtg tttttcagga ggtgaccctc agactgacag tgagtgagtc 480
cgagcatcag tggcttctgg agcagaccag ccacgtggaa gagaagcctt acagagatgg 540
gtcggcagag ccctgttaat taacccagct ttctt 575

<210> 484
<211> 1069

<212>  DNA

<213>  Homo sapiens


<400>  484
aaaaaagcag gcatggtgct gtgggagtcc ccgcggcagt gcagcagctg gacactttgc      60

gagggctttt gctggctgct gctgctgccc gtcatgctac tcatcgtagc ccgcccggtg     120

aagctcgctg ctttccctac ctccttaagt gactgccaaa cgcccaccgg ctggaattgc     180

tctggttatg atgacagaga aaatgatctc ttcctctgtg acaccaacac ctgtaaattt     240

gatggggaat gtttaagaat tggagacact gtgacttgcg tctgtcagtt caagtgcaac     300

aatgactatg tgcctgtgtg tggctccaat ggggagagct accagaatga gtgttacctg     360

cgacaggctg catgcaaaca gcagagtgag atacttgtgg tatcagaagg atcatgtgcc     420

acagatgcag gatcaggatc tggagatgga gtccatgaag gctctggaga aactagtcaa     480

aaggagacat ccacctgtga tatttgccag tttggtgcag aatgtgacga agatgccgag     540

gatgtctggt gtgtgtgtaa tattgactgt tctcaaacca acttcaatcc cctctgcgct     600

tctgatggga aatcttatga taatgcatgc caaatcaaag aagcatcgtg tcagaaacag     660

gagaaaattg aagtcatgtc tttgggtcga tgtcaagata acacaactac aactactaag     720

tctgaagatg ggcattatgc aagaacagat tatgcagaga atgctaacaa attagaagaa     780

agtgccagag aacaccacat accttgtccg gaacattaca atggcttctg catgcatggg     840

aagtgtgagc attctatcaa tatgcaggag ccatcttgca ggtgtgatgc tggttatact     900

ggacaacact gtgaaaaaaa ggactacagt gttctatacg ttgttcccgg tcctgtacga     960

tttcagtatg tcttaatcgc agctgtgatt ggaacaattc agattgctgt catctgtgtg    1020

gtggtcctct gcatcacaag ggccaaactt taattaaccc agctttctt                1069

<210>  485

<211>  707

<212>  DNA

<213>  Homo sapiens


<400>  485
aaaaaagctg gcacccctgg tccaggtatg acagtgctgg cgccagcctg gagcccaaca      60

acctatctcc tcctgctgct gctgctgagc tcgggactca gtgggaccca ggactgctcc     120

ttccaacaca gccccatctc ctccgacttc gctgtcaaaa tccgtgagct gtctgactac     180

ctgcttcaag attacccagt caccgtggcc tccaacctgc aggacgagga gctctgcggg     240

ggcctctggc ggctggtcct ggcacagcgc tggatggagc ggctcaagac tgtcgctggg     300

tccaagatgc aaggcttgct ggagcgcgtg aacacggaga tacactttgt caccaaatgt     360

gcctttcagg agacctccga gcagctggtg gcgctgaagc cctggatcac tcgccagaac     420

ttctcccggt gcctggagct gcagtgtcag cccgactcct caaccctgcc acccccatgg 480
agtccccggc ccctggaggc cacagccccg acagccccgc agccccctct gctcctccta 540
ctgctgctgc ccgtgggcct cctgctgctg gctgctgcct ggtgcctgca ctggcagagg 600
acgcggcgga ggacaccccg ccctggggag caggtgcccc ccgtccccag tccccaggac 660
ctgctgcttg tggagcaccc aggcccaggc ggcgacccag ctttctt 707

<210> 486
<211> 457
<212> DNA
<213> Homo sapiens

<400> 486
aaaaaagcag gcatggctgt agctgcagta aaatgggtga tgtcaaagag aactatcttg 60
aaacatttat ttccagtcca aaatggagct ttatattgtg tttgtcataa atctacgtat 120
tctcctctac cagatgacta taattgcaac gtagagcttg ctccgacttc tgatggcagg 180
acaatagtat gctaccaccc ttctgtggac attccatatg aacacacaaa acctatccct 240
cggccagatc ctgtgcataa taatgaagaa acacatgatc aagtgctgaa aaccagattg 300
gaagaaaaag ttgaacacct tgaggaagga cctatgatag aacaacttag caaaatgttc 360
tttactacta agcaccgttg gtatcctcat ggacggtatc acagatgtcg taagaatctg 420
aatcctccaa aagacagtta attaacccag ctttctt 457

<210> 487
<211> 850
<212> DNA
<213> Homo sapiens

<400> 487
aaaaaagcag gcatgctgaa gccagccctg gagccccgag ggggcttctc cttcgagaaa 60
tgccaaagaa atgcatcatt ggaacgcgtc ctcccggggc tcaaggtccc tcacgcacgc 120
aagaccggga ccaccatcgc gggcctggtg ttccaagacg gggtcattct gggcgccgat 180
acgcgagcca ctaacgattc ggtcgtggcg gacaagagct gcgagaagat ccacttcatc 240
gcccccaaaa tctactgctg tggggctgga gtagccgcgg acgccgagat gaccacacgg 300
atggtggcgt ccaagatgga gctacacgcg ctatctacgg gccgcgagcc ccgcgtggcc 360
acggtcactc gcatcctgcg ccagacgctc ttcaggtacc agggccacgt gggtgcatcg 420
ctgatcgtgg gcggcgtaga cctgactgga ccgcagctct acggtgtgca tccccatggc 480
tcctacagcc gtctgccctt cacagccctg ggctctggtc aggacgcggc cctggcggtg 540
ctagaagacc ggttccagcc gaacatgacg ctggaggctg ctcaggggct gctggtggaa 600

gccgtcaccg ccgggatctt gggtgacctg ggctccgggg gcaatgtgga cgcatgtgtg 660
atcacaaaga ctggcgccaa gctgctgcgg acactgagct cacccacaga gcccgtgaag 720
aggtctggcc gctaccactt tgtgcctgga accacagctg tcctgaccca gacagtgaag 780
ccactaaccc tggagctagt ggaggaaact gtgcaggcta tggaggtgga ttaattaacc 840
cagctttctt 850

<210> 488
<211> 550
<212> DNA
<213> Homo sapiens

<400> 488
aaaaaagcag gcatggcgct gcgagtggtg cggagcgtgc gggccctgct ctgcaccctg 60
cgcgcggtcc cgttacccgc cgcgccctgc ccgccgaggc cctggcagct gggggtgggc 120
gccgtccgta cgctgcgcac tggacccgct ctgctctcgg tgcgtaaatt cacagagaaa 180
cacgaatggg taacaacaga aaatggcatt ggaacagtgg gaatcagcaa ttttgcacag 240
gaagcgttgg gagatgttgt ttattgtagt ctccctgaag tttggacaaa attgaacaaa 300
caagatgagt ttggtgcttt ggaaagtgtg aaagctgcta gtgaactcta ttctccttta 360
tcaggagaag taactgaaat taatgaagct cttgcagaaa atccaggact tgtaaacaaa 420
tcttgttatg aagatggttg gctgatcaag atgacactga gtaacccttc agaactagat 480
gaacttatga gtgaagaagc atatgagaaa tacataaaat ctattgagga ttaattaacc 540
cagctttctt 550

<210> 489
<211> 775
<212> DNA
<213> Homo sapiens

<400> 489
aaaaaagcag gcatgggcaa taccagcagt gagcgcgccg cgctggagcg gcatggtggc 60
cataagacgc cccggaggga cagctcgggg ggcaccaagg acggggacag gcccaagatc 120
ctgatggaca gccccgaaga cgccgacctc ttccactccg aggaaatcaa ggcaccagag 180
aaggaggaat tcctggcctg gcagcatgat ctggaagtga atgataaagc tcccgcccag 240
gctcggccaa cggtgtttcg atggacgggg ggcggaaagg aagtttactt atctgggtcc 300
ttcaacaact ggagtaaact tcccctcacc agaagccaca ataactttgt agccatcctg 360
gatctgccgg aaggagagca tcagtacaag ttctttgtgg atggtcagtg gacgcacgac 420

ccttccgagc ccatagtaac cagccagctt ggcacagtta acaacatcat tcaagtgaag 480
aaaactgact ttgaagtatt tgatgcttta atggtggatt cccaaaagtg ctccgatgtg 540
tctgagctgt ccagttctcc cccaggaccc taccatcagg agccctacgt ctgcaaaccc 600
gaagagcgct ttcgggcacc ccctattctc cccccacatc tcctccaggt catcctgaac 660
aaggacacgg ggatttcctg tgatccagct ttgcttcctg agcccaatca cgtcatgctg 720
aaccacctat acgcgctgtc tatccaaagg atggattaat taacccagct ttctt 775

<210> 490
<211> 505
<212> DNA
<213> Homo sapiens

<400> 490
aaaaaagcag gcatgatgca gaagctactc aaatgcagtc ggcttgtcct ggctcttgcc 60
ctcatcctgg ttctggaatc ctcagttcaa ggttatccta cgcagagagc caggtaccaa 120
tgggtgcgct gcaatccaga cagtaattct gtaaactgcc ttgaagaaaa aggaccaatg 180
ttcgaactac ttccaggtga atccaacaag atcccccgtc tgaggactga cctttttcca 240
aagacgagaa tccaggactt gaatcgtatc ttcccacttt ctgaggacta ctctggatca 300
ggcttcggct ccggctccgg ctctggatca ggatctggga gtggcttcct aacggaaatg 360
gaacaggatt accaactagt agacgaaagt gatgctttcc atgacaacct taggtctctt 420
gacaggaatc tgccctcaga cagccaggac ttgggtcaac atggattaga agaggatttt 480
atgttttaat taacccagct ttctt 505

<210> 491
<211> 706
<212> DNA
<213> Homo sapiens

<400> 491
aaaaaagcag gcatggtcat ggcggagggg acggcagtgc tgaggcggaa caggccgggc 60
accaaggcgc aggatttcta taattggcct gatgaatcct ttgatgaaat ggacagtaca 120
ctagctgttc aacagtatat tcaacagaac ataagagcag attgctccaa tattgacaaa 180
attcttgaac cacctgaagg ccaagatgaa ggtgtgtgga agtatgaaca tttaaggcag 240
ttctgccttg agctaaatgg acttgctgtc aaacttcaga gtgaatgcca tccagatact 300
tgcactcaaa tgacagcaac tgaacaatgg atttttcttt gtgcagctca taaaactcca 360
aaagagtgtc ctgctataga ctatactaga cacacacttg atggtgctgc atgtcttctg 420
aatagcaata aatattttcc cagcagggtt agcataaagg aatcatctgt agcgaaacta 480

ggatcagtat gccgtaggat ttacagaata ttttcacatg cttattttca tcatcggcag 540

atatttgatg aatatgaaaa tgaaacattt ttgtgtcatc ggtttactaa gtttgtgatg 600

aaatacaatt tgatgtccaa ggataacctg attgtaccaa ttttagaaga ggaagtacag 660

aattcagttt ctggggaaag tgaagcttaa ttaacccagc tttctt 706

<210> 492

<211> 851

<212> DNA

<213> Homo sapiens

<400> 492
aaaaaagctg gcacccctgg tccaggtatg ccgctgcccg ttgcgctgca gacccgcttg 60

gccaagagag gcatcctcaa acatctggag cctgaaccag aggaagagat cattgccgag 120

gactatgacg atgatcctgt ggactacgag gccaccaggt tggagggcct accaccaagc 180

tggtacaagg tgttcgaccc ttcctgcggg ctcccttact actggaatgc agacacagac 240

cttgtatcct ggctctcccc acatgacccc aactccgtgg ttaccaaatc ggccaagaag 300

ctcagaagca gtaatgcaga tgctgaagaa aagttggacc ggagccatga caagtcggac 360

aggggccatg acaagtcgga ccgcagccat gagaaactag acaggggcca cgacaagtca 420

gaccggggcc acgacaagtc tgacagggat cgagagcgtg gctatgacaa ggtagacaga 480

gagagagagc gagacaggga acgggatcgg gaccgcgggt atgacaaggc agaccgggaa 540

gagggcaaag aacggcgcca ccatcgccgg gaggagctgg ctccctatcc caagagcaag 600

aaggcagtaa gccgaaagga tgaaaagtta gaccccatgg accctagctc atactcagac 660

gccccccggg gcacgtggtc aacaggactc cccaagcgga atgaggccaa gactggcgct 720

gacaccacag caggtgggcc cctcttccag cagcggccgt atccatcccc aggggctgtg 780

ctccgggcca atgcagaggc ctcccgaacc aagcagcagg atccaggccc aggcggcgac 840

ccagctttct t 851

<210> 493

<211> 410

<212> DNA

<213> Homo sapiens

<400> 493
aaaaaagcag gcatggtctg ggacttagct gatgaagtgc tctgcaccac ccagcttcga 60

ggagatgggg actggggttt ccccttgctt gacatcttaa ctacttcact gcaggaaagg 120

ggaggaggga caagaggagg accaatcccc aagggacacg gagacccaca ttaggttttg 180

gaacccacgc ctggtactat ctgaatttaa aagccttgct tgactcaggc ggggctgatc 240
aaaggccatt tgctgtttta gagtgttcct atgtgtgctt ggcagacaga gttgtctttt 300
tattgcttcc attcccacta agacctccat cccccactgc ccccccaacc ccttggtgag 360
cttggtgaga tgtgtttttt ttttcttttt ttaattaacc caactttctt 410

<210> 494
<211> 1862
<212> DNA
<213> Homo sapiens

<400> 494
aaaaaagcag gcatgaatgt gggcttatca gaaactcaaa atggaggcat gtctcaagaa 60
gcagtgggaa atataaaagt tacaaagtct ccccagaaat ccactgtatt aagcaatgga 120
gaagcagcaa tgcagtcttc caattcagaa tcaaaaaaga aaaagaagaa aaagagaaaa 180
atggtgaatg atgctgagcc tgatacgaaa aaagcaaaaa ctgaaaacaa agggaaatct 240
gaagaagaaa gtgccgagac tactaaagaa acagaaaata atgtggagaa gccagataat 300
gatgaagatg agagtgaggt gcccagtctg cccctgggga ctgacaggag cttttgagga 360
tacttcgttt gcttctctat gtaatcttgt caatgaaaac actctgaagg caataaaaga 420
aatgggtttt acaaacatga ctgaaattca gcataaaagt atcagaccac ttctggaagg 480
cagggatctt ctagcagctg caaaaacagg cagtggtaaa accctggctt ttctcatccc 540
tgcagttgaa ctcattgtta agttaaggtt catgcccagg aatggaacag gagtccttat 600
tctctcacct actagagaac tagccatgca aacctttggt gttcttaagg agctgatgac 660
tcaccacgtg catacctatg gcttgataat gggtggcagt aacagatctg ctgaagcaca 720
gaaacttggt aatgggatca acatcattgt ggccacacca ggccgtctgc tggaccatat 780
gcagaatacc ccaggattta tgtataaaaa cctgcagtgt ctggttattg atgaagctga 840
tcgtatcttg gatgtggggt ttgaagagga attaaagcaa attattaaac ttttgccaac 900
acgtagacag actatgctct tttctgccac ccaaactcga aaagttgaag acctggcaag 960
gatttctctg aaaaaggggc cattgtatgt tggcgttgat gatgataaag cgaatgcaac 1020
agtggatggt cttgaacagg gatatgttgt ttgtccttct gaaaagagat tccttctgct 1080
ctttacattc cttaagaaga accgaaagaa gaagcttatg gtcttctttt catcttgtat 1140
gtctgtgaaa taccactatg agttgctgaa ctacattgat ttgcccgtct tggccattca 1200
tggaaagcaa aagcaaaata agcgtacaac cacattcttc cagttctgca atgcagattc 1260
gggaacacta ttgtgtacgg atgtggcagc gagaggacta gacattcctg aagtcgactg 1320
gattgttcag tatgaccctc cggatgaccc taaggaatat attcatcgtg tgggtagaac 1380
agccagaggc ctaaatggga gagggcatgc cttgctcatt ttgcgcccag aagaattggg 1440
ttttcttcgc tacttgaaac aatccaaggt tccattaagt gaatttgact tttcctggtc 1500

taaaatttct gacattcagt ctcagcttga gaaattgatt gaaaagaatt actttcttca 1560

taagtcagcc caggaagcat ataagtcata catacgagcc tatgattccc attctctgaa 1620

acagatcttt aatgttaata acctaaattt gcctcaggtt gctctgtcat ttggtttcaa 1680

ggtgcctccc ttcgttgatc tgaacgtcaa cagtaatgaa ggcaagcaga aaaagcgagg 1740

aggtggtggt ggatttggct accagaaaac caagaaagtt gagaaatcca aaatctttaa 1800

acacattagc aagaaatcat ctgacagcag gcagttctct cattaattaa cccagctttc 1860

tt 1862

<210> 495

<211> 388

<212> DNA

<213> Homo sapiens

<400> 495
aaaaaagcag gcatgtctcg ctccgtggcc ttagctgtgc tcgcgctact ctctctttct 60

ggcctggagg ctatccagcg tactccaaag attcaggttt actcacgtca tccagcagag 120

aatggaaagt caaatttcct gaattgctat gtgtctgggt ttcatccatc cgacattgaa 180

gttgacttac tgaagaatgg agagagaatt gaaaaagtgg agcattcaga cttgtctttc 240

agcaaggact ggtctttcta tctcttgtac tacactgaat tcacccccac tgaaaaagat 300

gagtatgcct gccgtgtgaa ccatgtgact ttgtcacagc ccaagatagt taagtgggat 360

cgagacattt aattaaccca gctttctt 388

<210> 496

<211> 398

<212> DNA

<213> Homo sapiens

<400> 496
aaaaaagctg gcacccctgg tccaggtatg gcctctgtct ccgagctcgc ctgcatctac 60

tcggccctcg ttctgcacga cgatgaggtg acagtcacgg aggataagat caatgccctc 120

attaaagcag ccggtgtaaa tgttgagcct ttttggcctg gcttgtttgc aaaggccctg 180

gccaacgtca acattgggag cctcatctgc aatgtagggg ccggtggacc tgctccagca 240

gctggtgctg caccagcagg aggtcctgcc ccctccactg ctgctgctcc agctgaggag 300

aagaaagtgg aagcaaagaa agaagaatcc gaggagtctg atgatgacat gggctttggt 360

ctttttgacc caggcccagg cggcgaccca gctttctt 398

<210> 497

<211>  535

<212>  DNA

<213>  Homo sapiens


<400>  497
aaaaaagcag gcatgaacag caaaggtcaa tatccaacac agccaaccta ccctgtgcag      60

cctcctggga atccagtata ccctcagacc ttgcatcttc ctcaggctcc accctatgcc     120

gatgctccac ctgcctactc agagctctat cgtccgagct ttgtgcaccc aggggctgcc     180

acagtcccca ccatgtcagc cgcatttcct ggagcctctc tgtatcttcc catggcccag     240

tctgtggctg ttgggccttt aggttccaca atccccatgg cttattatcc agtcggtccc     300

atctatccac ctggctccac agtgctggtg gaaggagggt atgatgcagg tgccagattt     360

ggagctgggg ctactgctgg caacattcct cctccacctc ctggatgccc tcccaatgct     420

gctcagcttg cagtcatgca gggagccaac gtcctcgtaa ctcagcggaa ggggaacttc     480

ttcatgggtg gttcagatgg tggctacacc atctgttaat taacccagct ttctt          535

<210>  498

<211>  542

<212>  DNA

<213>  Homo sapiens


<400>  498
aaaaaagctg gcacctctgg tccaggtatg acgaccccca ataaaggaaa caaggccttg      60

aaggtgaagc gggagccggg tgagaatggc accagcctga cggatgagga gctggtgacc     120

atgtcggtgc gggagctgaa ccagcacctg cggggcctgt ccaaggagga gatcgtccag     180

ctgaagcagc gccggcgcac gctcaagaac cgcggctacg ctgccagctg ccgcgtgaag     240

cgggtgacgc aggaggagga gctggagaag cagaaggcgg agctgcagca ggaggtgggg     300

aagctggcct cagagaacgc cagcatgaag ctggagctcg acgcgctgcg ctccaagtac     360

gaggcgctgc agaccttcgc ccggacggtg gcccgcagcc ccgtggcgcc agcccggggc     420

ccccttgccg ccggcctggg gcccctcgtc ccaggcaagg tggccgccac cagcgtcatc     480

acaatagtaa agtccaagac ggatgcccga tcgccaggcc caggcggcga cccagctttc     540

tt                                                                    542

<210>  499

<211>  775

<212>  DNA

<213>  Homo sapiens

<400> 499
aaaaaagcag gcatggaggg tgtagaagag aagaagaagg aggttcctgc tgtgccagaa 60
acccttaaga aaaagcgaag gaatttcgca gagctgaaga tcaagcgcct gagaaagaag 120
tttgcccaaa agatgcttcg aaaggcaagg agggagctta tctatgaaaa agcaaagcac 180
tatcacaagg aatataggca gatgtacaga actgaaattc gaatggcgag gatggcaaga 240
aaagctggca acttctatgt acctgcagaa cccaaattgg cgtttgtcat cagaatcaga 300
ggtatcaatg gagtgagccc aaaggttcga aaggtgttgc agcttcttcg ccttcgtcaa 360
atcttcaatg gaacctttgt gaagctcaac aaggcttcga ttaacatgct gaggattgta 420
gagccatata ttgcatgggg gtaccccaat ctgaagtcag taaatgaact aatctacaag 480
cgtggttatg gcaaaatcaa taagaagcga attgctttga cagataacgc tttgattgct 540
cgatctcttg gtaaatacgg catcatctgc atggaggatt tgattcgtga gatctatact 600
gttggaaaac gcttcaaaga ggcaaataac ttcctgtggc ccttcaaatt gtcttctcca 660
cgaggtggaa tgaagaaaaa gaccacccat tttgtagaag gtggagatgc tggcaacagg 720
gaggaccaga tcaacaggct tattagaaga atgaattaat taacccagct ttctt 775

<210> 500
<211> 2890
<212> DNA
<213> Homo sapiens

<400> 500
aaaaaagcag gcatggctga cctcagtctt gcagatgcat taacagaacc atctccagac 60
attgagggag agataaagcg ggacttcatt gccacactag aggcagaggc ctttgatgat 120
gttgtgggag aaactgttgg aaaaacagac tatattcctc tcctggatgt tgatgagaaa 180
accgggaact cagagtcaaa gaagaaaccg tgctcagaaa ctagccagat tgaagatact 240
ccatcttcta aaccaacact cctagccaat ggtggtcatg gagtagaagg gagcgatact 300
acagggtctc caactgaatt ccttgaagag aaaatggcct accaggaata cccaaatagc 360
cagaactggc cagaagatac caacttttgt ttccaacctg agcaagtggt cgatcctatc 420
cagactgatc cctttaagat gtaccatgat gatgacctgg cagatttggt ctttccctcc 480
agtgcgacag ctgatacttc aatatttgca ggacaaaatg atcccttgaa agacagttac 540
ggtatgtctc cctgcaacac agctgttgta cctcaggggt ggtctgtgga agccttaaac 600
tctccacact cagagtcctt tgtttcccca gaggctgttg cagaacctcc tcagccaacg 660
gcagttccct tagagctagc caaggagata gaaatggcat cagaagagag gccaccagca 720
caagcattgg aaataatgat gggactgaag actactgaca tggcaccatc taaagaaaca 780
gagatggccc tcgccaagga catggcacta gctacaaaaa ccgaggtggc attggctaaa 840

gatatggaat cacccaccaa attagatgtg acactggcca aggacatgca gccatccatg 900
gaatcagata tggccctagt caaggacatg gaactaccca cagaaaaaga agtggccctg 960
gttaaggatg tcagatggcc cacagaaaca gatgtatctt cagccaagaa tgtggtactg 1020
cccacagaaa cagaggtagc cccagccaag gatgtgacac tgttgaaaga aacagagagg 1080
gcatctccta taaaaatgga cttggctcct tccaaggaca tgggaccacc caaagaaaac 1140
aagatagtcc cagccaagga tttggtatta ctctcagaaa tagaggtggc acaggctaat 1200
gacattatat catccacaga aatatcctct gctgagaagg tggctttgtc ctcagaaaca 1260
gaggtagccc tggccaggga catgacactg cccccggaaa ccaacgtgat cttgaccaag 1320
gataaagcac tacctttaga agcagaggtg gccccagtca aggacatggc tcaactccca 1380
gaaacagaaa tagccccggc caaggatgtg gctccgtcca cagtaaaaga agtgggcttg 1440
ttgaaggaca tgtctccact atcagaaaca gaaatggctc tgggcaagga tgtgactcca 1500
cctccagaaa cagaagtagt tctcatcaag aacgtatgtc tgcctccaga aatggaggtg 1560
gccctgactg aggatcaggt cccagccctc aaaacagaag ctcccaccac cattggtggg 1620
ttgaataaaa aacccatgag ccttgcttca ggcttagtgc cagctgcccc acccaaacgc 1680
cctgccgtcg cctctgccag gccttccatc ttaccttcaa aagacgtgaa gccaaagccc 1740
attgcagatg caaaggctcc tgagaagcgg gcctcaccat ccaagccagc ttctgcccca 1800
gcctccagat ctgggtccaa gagcactcag actgttgcaa aaaccacaac agctgctgct 1860
gttgcctcaa ctggcccaag cagtaggagc ccctccacgc tcctgcccaa gaagcccact 1920
gccattaaga ctgagggaaa acctgcagaa gtcaagaaga tgactgcaaa gtctgtacca 1980
gctgacttga gtcgcccaaa gagcacctcc accagttcca tgaagaaaac caccactctc 2040
agtgggacag cccccgctgc aggggtggtt cccagccgag tcaaggccac acccatgccc 2100
tcccggccct ccacaactcc tttcatagac aagaagccca cctcggccaa acccagctcc 2160
accacccccc ggctcagccg cctggccacc aatacttctg ctcctgatct gaagaatgtc 2220
cgctccaagg ttggctccac ggaaaacatc aagcatcagc ctggaggagg ccgggccaaa 2280
gtagagaaaa aaacagaggc agctgctaca acccgaaagc ctgaatctaa tgcagtcact 2340
aaaacagccg gcccaattgc aagtgcacag aaacaacctg cggggaaagt ccagatagtc 2400
tccaaaaaag tgagctacag ccatattcag tccaagtgtg gttccaagga caatattaag 2460
catgtccctg gaggtggtaa tgttcagatt cagaacaaga aagtggacat ctctaaggtc 2520
tcctccaagt gtgggtctaa ggctaacatc aagcacaagc ctggtggagg agatgtcaag 2580
attgaaagtc agaagttgaa cttcaaggag aaggcccagg ccaaggtggg atccctcgat 2640
aatgtgggcc acctacctgc aggaggtgct gtgaagactg agggcggtgg cagcgaggct 2700
cctctgtgtc cgggtccccc tgctggggag gagccggcca tctctgaggc agcgcctgaa 2760
gctggcgccc ccacttcagc cagtggcctc aatggccacc ccaccctgtc agggggtggt 2820
gaccaaaggg aggcccagac cttggacagc cagatccagg agacaagcat ttaattaacc 2880
cagctttctt 2890

<210> 501

<211> 466

<212> DNA

<213> Homo sapiens

<400> 501
aaaaaagcag gcatgaagtt taatcccttt gtgacttccg accgaagcaa gaatcgcaaa 60

aggcatttca atgcaccttc ccacattcga aggaagatta tgtcttcccc tctttccaaa 120

gagctgagac agaagtacaa cgtgcgatcc atgcccatcc gaaaggatga tgaagttcag 180

gttgtacgtg gacactataa aggtcagcaa attggcaaag tagtccaggt ttacaggaag 240

aaatatgtta tctacattga acgggtgcag cgggaaaagg ctaatggcac aactgtccac 300

gtaggcattc accccagcaa ggtggttatc actaggctaa aactggacaa agaccgcaaa 360

aagatcctcg aacggaaagc caaatctcgc caagtaggaa aggaaaaggg caaatacaag 420

gaagaaacca ttgagaagat gcaggattaa ttaacccagc tttctt 466

<210> 502

<211> 536

<212> DNA

<213> Homo sapiens

<400> 502
aaaaaagctg gcacccctgg tccaggtatg gcggccgaga gaaagacaag gttgtccaag 60

aatctactgc gcatgaagtt tatgcaaagg ggactggact cagaaaccaa gaaacaacta 120

gaagaagaag aaaagaaaat cattagtgaa gagcactggt acttggattt gccagagctt 180

aaagagaaag agagtttcat aatagaagag cagagtttct tactatgtga agatcttctc 240

tatggaagaa tgtcattcag aggatttaat cctgaggttg agaaattgat gcttcagatg 300

aatgctaagc acaaagcaga agaagttgaa gatgaaacag tagagcttga tgtgtcagat 360

gaagagatgg ctagaagata tgagaccttg gtggggacaa ttgggaaaaa gtttgccaga 420

aagagagacc atgccaatta tgaagaagat gaaaatggag acataacacc aattaaagca 480

aagaagatgt tcttaaagcc ccaggatcca ggcccaggcg acgacccagc tttctt 536

<210> 503

<211> 1657

<212> DNA

<213> Homo sapiens

<400>  503
aaaaaagcag gcatggcctc caatgaaggc agtcgcatca gtgctgcctc tgtggccagc      60

actgccattg ctgcggccca gtgggccatc tcacaggcct cccaaggtgg ggagggtacc     120

tgggccacct ccgaggagcc gccggtcgac tacagctact accaacagga tgagggctat     180

ggcaacagcc agggcacaga gtcttccctc tatgcccatg gctacctcaa gggcaccaag     240

ggccctggca tcactggaac caaaggggat cccactggag caggtcccga ggcctcccta     300

gagcctgggg ccgactctgt gtcgacgcag gctttctctc gcgcccagcc tggtgctgct     360

cctggcatct accaacaatc agccgaggcg agcagtagcc agggcactgc tgccaacagc     420

cagtcgtata ccatcatgtc acccgctgtg ctcaaatctg agctccagag ccctacccat     480

cctagttctg ctctcccacc ggctaccagc cccactgccc aggaatccta cagccagtac     540

cctgttcccg acgtctctac ctaccagtac gatgagacct ccggctacta ctatgacccc     600

cagaccggcc tctactatga ccccaactcc cagtattact acaatgctca gagccagcag     660

tacctgtact gggatgggga gaggcggacc tatgttcccg ccctggagca gtcggccgac     720

ggacataagg agacaggggc accctcgaag gagggcaaag agaagaagga gaagcacaag     780

accaagacag ctcaacagat tgccaaggac atggaacgct gggcccgcag tctcaacaaa     840

caaaaagaaa acttcaaaaa tagcttccag cctatcagct ccctgcgaga tgacgagagg     900

cgggagtcag ccactgcaga tgctggctat gccatcctcg agaagaaggg agcactagcc     960

gagagacagc acaccagcat ggatctcccg aaattggcca gtgacgaccg cccaagccct    1020

ccgcgaggac tggtggcagc ctacagcggg gagagtgaca gtgaggagga gcaggagcgt    1080

gggggccctg agcgggagga gaagctcacc gactggcaga agctggcctg tctgctctgc    1140

cgacgccagt tccccagcaa agaggcgctc atccggcacc agcagctctc agggctccac    1200

aagcaaaacc ttgagattca ccggcgagcc cacttgtcag aaaacgagct agaagcacta    1260

gagaagaatg acatggagca aatgaagtac cgggaccgtg cagctgaacg cagggaaaag    1320

tatggcatcc ccgagccgcc agagcccaag aggaggaagt acggcggcat atccacagcc    1380

tctgtagact tcgagcagcc tactcgggac gggctgggca gtgacaacat tggcagtcgg    1440

atgctgcagg ccatgggctg gaaagagggc agcggcctgg gccgcaagaa gcagggcatt    1500

gtaacgccta tcgaggccca aacacgggtg cggggctccg gcctgggtgc acggggcagc    1560

tcctacgggg tcacctcaac cgagtcctac aaggagacac tgcacaagac aatggtgacc    1620

cgcttcaacg aggcccagta attaacccag ctttctt                             1657

<210>  504

<211>  397

<212>  DNA

<213>  Homo sapiens

<400> 504
aaaaaagcag gcatggccgc ccccgtggat ctagagctga agaaggcctt cacagagctt 60

caagccaaag ttattgacac tcaacagaag gtgaagctcg cagacataca gattggacag 120

ctaaacagaa cgaaaaagca tgcacatctt acagatacag agatcatgac tttggtagat 180

gagactaaca tgtatgaagg tgtaggaaga atgtttattc ttcagtccaa ggaagcaatt 240

cacagtcagc tgttagagaa gcagaaaata gcagaagaaa aaattaaaga actagaacag 300

aaaaagtcct acctggagcg aagcgttaag gaagctgagg acaacatccg ggagatgctg 360

atggcacgaa gggcccatta attaacccag ctttctt 397

<210> 505

<211> 1300

<212> DNA

<213> Homo sapiens

<400> 505
aaaaaagcag gcatggcatc tgatgacttt gacatagtgg ttgaggccat gctggaagct 60

ccctataaaa aagaagagga tgagcaacaa aggaaagaag ttaaaaagga ttatcctagc 120

aataccacca gcagcaccag caacagtggc aatgagacca gtggaagcag caccatcggg 180

gagacaagca atcgtagtcg agatcgggat cggtatagac ggagaaatag tcggagccga 240

agtccaggtc ggcagtgtcg tcaccgtagc cgtagctggg atcgtcgaca tggtagtgag 300

tcgcgaagtc gggaccatcg tcgtgaggat cgtgtgcatt acaggagtcc tccacttgcc 360

actggttata ggtatggaca cagtaagagt cctcatttca gagagaagag cccagtcagg 420

gagccagttg ataatctgag tcctgaggag cgtgatgccc gcacagtttt ctgtatgcag 480

ttagctgccc gaattcggcc tcgagatctg gaggactttt tctctgctgt aggcaaggtt 540

cgcgatgtac gtatcatctc agatcggaac tcacgtcgtt ctaagggcat tgcctacgtg 600

gaattctgtg aaatccagtc tgtgccactg gccattgggc tgactgggca gcggttgctg 660

ggagtgccta tcattgtaca ggcttcacag gcagagaaaa accgactggc agccatggcc 720

aacaacctgc aaaagggcaa tggtggacca atgcgcctct atgtgggttc cctgcacttc 780

aatatcactg aagacatgct ccggggcatc tttgagccct ttggtaaaat tgataatatt 840

gtcctgatga aggactcaga tacaggccgc tctaaaggtt atggcctcat cacgttctct 900

gattctgagt gtgcccggcg ggccctggaa cagttgaatg ggtttgagct tgctggtcga 960

cctatgaggg ttggccatgt gactgagcga ctggatggtg gcacagacat cacttttcct 1020

gatggggacc aggagctgga tctgggatca gcaggtggac gttttcagct catggcaaaa 1080

ctggcagaag gcgctggaat ccaactgcca agcactgctg ctgctgctgc tgccgccgcc 1140

gcccaggctg ctgccttgca actgaatgga gcagttccct tgggggccct gaatccagca 1200

gctctgactg ctctgagtcc agccctgaac cttgcctccc agtgtttcca gctctccagc 1260

ctctttaccc cccagaccat ttaattaacc cagctttctt 1300

<210> 506
<211> 811
<212> DNA
<213> Homo sapiens

<400> 506
aaaaaagcag gcatgtccca tcactggggg tacggcaaac acaacggacc tgagcactag 60
cataaggact tccccattgc caagggagag cgccagtccc ctgttgacat cgacactcat 120
acagccaagt atgacccttc cctgaagccc ctgtctgttt cctatgatca agcaacttcc 180
ctgaggatcc tcaacaatgg tcatgctttc aacgtggagt ttgatgactc tcaggacaaa 240
gcagtgctca agggaggacc cctggatggc acttacagat tgattcagtt tcactttcac 300
tggggttcac ttgatggaca aggttcagag catactgtgg ataaaaagaa atatgctgca 360
gaacttcact tggttcactg gaacaccaaa tatggggatt ttgggaaagc tgtgcagcaa 420
cctgatggac tggccgttct aggtattttt ttgaaggttg gcagcgctaa accgggcctt 480
cagaaagttg ttgatgtgct ggattccatt aaaacaaagg gcaagagtgc tgacttcact 540
aacttcgatc ctcgtggcct ccttcctgaa tccctggatt actggaccta cccaggctca 600
ctgaccaccc ctcctcttct ggaatgtgtg acctggattg tgctcaagga acccatcagc 660
gtcagcagcg agcaggtgtt gaaattccgt aaacttaact tcaatgggga gggtgaaccc 720
gaagaactga tggtggacaa ctggcgccca gctcagccac tgaagaacag gcaaatcaaa 780
gcttccttca attaattaac ccagctttct t 811

<210> 507
<211> 1219
<212> DNA
<213> Homo sapiens

<400> 507
aaaaaagcag gcatggctgg ctgtggtgaa attgatcatt caataaacat gcttcctaca 60
aacaggaaag cgaacgagtc ctgttctaat actgcacctt ctttaaccgt ccctgaatgt 120
gccatttgtc tgcaaacatg tgttcatcca gtcagtctgc cctgtaagca cgttttctgc 180
tatctatgtg taaaaggagc ttcatggctt ggaaagcggt gtgctctttg tcgacaagaa 240
attcccgagg atttccttga caagccaacc ttgttgtcac cagaagaact caaggcagca 300
agtagagaaa atggtgaata tgcatggtat tatgaaggaa gaaatgggtg gtggcagtac 360
gatgagcgca ctagtagaga gctggaagat gctttttcca aaggtaaaaa gaacactgaa 420
atgttaattg ctggctttct gtatgtcgct gatcttgaaa acatggttca atataggaga 480

aatgaacatg gacgtcgcag gaaggttaag cgagatataa tagatatacc aaagaaggga 540
gtagctggac ttaggctaga ctgtgatgct aataccgtaa acctagcaag agagagctct 600
gctgacggag cggacagtgt atcagcacag agtggagctt ctgttcagcc cctagtgtct 660
tctgtaaggc ccctaacatc agtagatggt cagttaacaa gccctgcaac accatcccct 720
gatgcaagca cttctctgga agactctttt gctcatttac aactcagtgg agacaacaca 780
gctgaaagga gtcatagggg agaaggagaa gaagatcatg aatcaccatc ttcaggcagg 840
gtaccagcac cagacacctc cattgaagaa actgaatcag atgccagtag tgatagtgag 900
gatgtatctg cagttgttgc acagcactcc ttgacccaac agagactttt ggtttctaat 960
gcaaaccaga cagtacccga tcgatcagat cgatcgggaa ctgatcgatc agtagcaggg 1020
ggtggaacag tgagtgtcag tgtcagatct agaaggcctg atggacagtg cacagtaact 1080
gaagtttaaa taaaaatgtc ttcagctcca tgctcaaggt tgaaagggtt acctgtaaat 1140
ttctgcccac ataacattat actcatccct agtagtgcat tttgggagtt ggggtgggaa 1200
ggggtaaccc agctttctt 1219

<210> 508
<211> 168
<212> DNA
<213> Homo sapiens

<400> 508
aaaaaacccc gcagagagag cagatttgaa gcaactcatg gttcatgctt ttatcaagag 60
atctgatgct gaggaagtgg attttgcagg ttggctctgc tccaccatcg gccttaacca 120
gcccagcaca ccaacccatg ctgctggcgt ctaagaccca gctttctt 168

<210> 509
<211> 235
<212> DNA
<213> Homo sapiens

<400> 509
aaaaaagcag gcatgttcta cgtgggcctg ttcttcgtca atgtgctgat cctgtactac 60
gccttcctca tggagtacat cgtcctcaac gtgggcctcg tcttcctgtt cgaggacatg 120
gaccaggcgc tcctggacct cggcgtgctc tccgaccccg gctcgggcct ttacgatgct 180
gactcggagc tcgacgtctt tgatgcgtac ttggattaat taacccagct ttctt 235

<210> 510
<211> 619

<212>  DNA

<213>  Homo sapiens


<400>  510
aaaaaagcag gcatgtctga aggggacagt gtgggagaat ccgtccatgg gaaaccttcg      60

gtggtgtaca gatttttcac aagacttgga cagatttatc agtcctggct agacaagtcc     120

acaccctaca cggctgtgcg atgggtcgtg acactgggcc tgagctttgt ctacatgatt     180

cgagtttacc tgctgcaggg ttggtacatt gtgacctatg ccttggggat ctaccatcta     240

aatcttttca tagcttttct ttctcccaaa gtggatcctt ccttaatgga agactcagat     300

gacggtcctt cgctacccac catacagaac gaggaattcc gccccttcat tcgaaggctc     360

ccagagttta aattttggca tgcggctacc aagggcatcc ttgtggctat ggtctgtact     420

ttcttcgacg ctttcaacgt cccggtgttc tggccgattc tggtgatgta cttcatcatg     480

ctcttctgta tcacgatgaa gaggcaaatc aagcacatga ttaagtaccg gtacatcccg     540

ttcacacatg ggaagagaag gtacagaggc aaggaggatg ccggcaaggc cttcgccagt     600

taattaaccc agctttctt                                                  619

<210>  511

<211>  745

<212>  DNA

<213>  Homo sapiens


<220>

<221>  misc_feature

<222>  (180)..(180)

<223>  n = a, c, g or t


<400>  511
   aaaaaagcag gcatgggctc tcctaggcct cctgggatga gggagccacc aggacccagt      60
   gctgtgatgc ctgctcttcc ctctaccagc acctgcccgc ccagagacca gggcacccct     120
   gaagtccagc ccacccctgc aaaggacaca tggaagggca agcggcctcg atcccagcan     180
   gagaacccag agagccagcc tcagaagagg ccacgcccct cagccaagcc ctccgtcgta     240
   gctgaggtca agggcagcgt ctcggccagc gaacagggca ccttgaatcc cacggctcaa     300
   gaccccttcc agctctccgc tcctggcgtc tccttgaagg aggctgcaaa tgttgtggtc     360
   aagtgcctca cccctttcta caaggagggc aagtttgctt ccaaggagtt gtttaaaggc     420
   tttgcccgcc acctctcaca cttgctgact cagaagacct ctcctggaag gagcgtgaaa     480
   gaagaggccc agaacctcat caggcacttc ttccatggcc gggcccggtg cgagagcgaa     540
   gctgactggc atggcctgtg tggcccccag agatgaccaa ctgctggctg ggcagggccc 600
   gcgtcctccc ccagattcta gcatgggtca tcctgggcct cacctgctga tgccagggcc 660
   atcgtctttt ctcagtccct ctcctttcca accatacttg gctttgggga tgaccccaga 720
   caccccctga atacccagct ttctt 745

<210> 512
<211> 946
<212> DNA
<213> Homo sapiens

<400> 512
aaaaaagcag gcatgaccaa ccagtacggt attctcttca aacaagagca agcccatgat 60
gatgccattt ggtcagttgc ttgggggaca aacaagaagg aaaactctga gacagtggtc 120
acaggctccc tagatgacct ggtgaaggtc tggaaatggc gtgatgagag gctggaccta 180
cagtggagtc tggagggaca tcagctggga gtggtgtctg tggacatcag ccacaccctg 240
cccattgctg catccagctc tcttgatgct catattcgtc tttgggactt ggaaaatggc 300
aaacagataa agtccataga tgcaggacct gtggatgcct ggactttggc cttttctcct 360
gattcccagt atctggccac aggaactcat gtcgggaaag tgaacatttt tggtgtggaa 420
agtgggaaaa aggaatattc tttggacacg agaggaaaat tcattcttag tattgcatat 480
agtcctgatg ggaaatacct agccagtgga gccatagatg gaatcatcaa tatttttgat 540
attgcaactg gaaaacttct gcataccctg gaaggccatg ccatgcccat tcgctccttg 600
accttttccc cggactccca gctccttgtc actgcttcag atgatggcta catcaagatc 660
tatgatgtac aacatgccaa tttggctggc acgctgggcg gccatgcctc ctgggtgctg 720
aacgttgcat tctgtcctga tgacactcac tttgtttcca gttcgtctga caaaaatgta 780
aaagtttggg atgttggaac gaggacttgt gttcacacct tctttgatca ccaggatcag 840
gtctggggag taaaatacaa tggaaatggt tcaaaaattg tgtctgttgg agatgaccag 900
gaaattcaca tatatgattg tccaatttaa ttaacccagc tttctt 946

<210> 513
<211> 903
<212> DNA
<213> Homo sapiens

<400> 513
aaaaaagcag gcatggaggt gaccggggac gccggggtac cagaatctgg cgagatccgg 60
actctaaagc cgtgtctgct gcgccgcaac tacagccgcg aacagcacgg cgtggccgcc 120

tcctgcctcg aagacctgag gagcaaggcc tgtgacattc tggccattga taagtccctg 180

acaccagcca ccctggtcct ggcagaggat ggcaccatag tggatgatga cgattacttt 240

ctgtgtctac cttccaatac taagcttgtg gcattggcta gtaatgagaa atgggcatac 300

aacaattcag atggaggtac agcttggatt tccaagagtc ctttgatgta gatgaaacag 360

acagcggggc agggttgaag tggaagaatg tggccaggca gctgaaagaa gatctgtcca 420

gcatcatcct cctatcagag gaggacctcc agagtccaaa gctgcctttg gtgaggaggt 480

ggatgcagta gacacgggta tcagcagaga gacctcctcg gacgttgcgc tggcgagcca 540

catccttact gcactgaggg agaagcaggc tccagagctg agcttatcta gtcaggattt 600

ggaggtgggc ggaaaccagg gtcactgagc tacagaggag gacatgccct gggatgtagt 660

agtatcatgc agagttggtt accaaggaag accccaaagc actggctgtt gccttgaact 720

gggacatgaa gaagacggag actgttcagg aggcctgtga gcgggagctc gccctgcgcc 780

tgcagcagac gcagagcttg cattctctcc ggagcatctc agcaagcaag gcctcaccac 840

ctggtgacct gcagaatcct aagcgagcca gacaggatcc cacttaatta acccagcttt 900

ctt 903

<210> 514

<211> 571

<212> DNA

<213> Homo sapiens

<400> 514
aaaaaagcag gcatgaggga ctgtgttgct aagagcgttg ggggcaaagc caggctggtt 60

ccttggcctc ggggtttcct gggtcgggga cacggtgaag aggctccagc gggacctgcc 120

catcagtcct gggccaggag gggctccaag cagcacccag cggtccgggg gagtctcgga 180

cccggcatgc gtggctggca gacctgggag agccagggca gggttttgcg ttcagagaag 240

gattgcccca gagacccgtg gtggacttca tgggtgctga gtggcccgtg tgacagtgat 300

gacacgaagg cttcggcgtt tgagtgggtg caggtgcacg ccagggcttg gtgcttccct 360

gcctggccct ggagggagct gggtggcctg gcttcagggg aagacaggag ccaggacaca 420

cgtcagccca gcaggtgtgg ggggtgctgc agccctcggc agtggggtca ggccctgggg 480

gatgtttcca atggtgggca gcctggccag gccggagaag acatgttcac gggcatctat 540

cagatgcccc cttaattaac ccagctttct t 571

<210> 515

<211> 556

<212> DNA

<213> Homo sapiens

<400> 515
aaaaaagcag gcatgagctc ccagattcgt cagaattatt ccaccgacgt ggaggcagcc 60
gccaacagcc tggtcaattt gtacctgcag gcctcctaca cctacctctc tctgggcttc 120
tatttcgacc gcgatgatgt ggctctggaa ggcgtgagcc acttcttccg cgaattggcc 180
gaggagaagc gcgagggcta cgagcgtctc ctgaagatgc aaaaccagcg tggcggccgc 240
gctctcttcc aggacatcaa gaagccagct gaagatgagt ggggtaaaac cccagacgcc 300
atgaaagctg ccatggccct ggagaaaaag ctgaaccagg cccttttgga tctccatgcc 360
ctgggttctg cccgcacgga cccccatctc tgtgacttcc tggagactca cttcctagat 420
gaggaagtga agcttatcaa gaagatgggt gaccacctga ccaacctcca caggctgggt 480
ggcccggagg ctgggctggg cgagtatctc ttcgaaaggc tcactctcaa gcacgattaa 540
ttaacccagc tttctt 556

<210> 516

<211> 1186

<212> DNA

<213> Homo sapiens

<400> 516
aaaaaagcag gcatggcggc tgctgctgca cgctggaacc atgtgtgggt cggcaccgag 60
actgggatct tgaaaggggt aaatcttcag cgaaaacagg cggcgaactt cacggccgga 120
ggacagccgc ggcgcgagga ggcagtgagc gccctgtgtt ggggcaccgg cggcgagacc 180
cagatgctgg tgggctgcgc ggacaggacg gtgaagcact tcagcaccga ggatggcata 240
ttccagggtc agagacactg cccgggcggg gagggcatgt tccgtggcct cgcccaggcc 300
gacggcaccc tcatcacatg tgtggattct gggattctca gagtctggca tgacaaggac 360
aaggacacat cctctgaccc actcctggaa ctgagagtgg gccctggggt gtgtaggatg 420
cgccaagacc cagcacaccc ccatgtggtt gccacaggtg ggaaagagaa tgctttgaag 480
atatgggacc tgcagggctc tgaggaacct gtgttcaggg ccaagaacgt gcggaatgac 540
tggctggact tgcgggttcc catctgggac caggacatac agtttctccc aggatcacag 600
aagcttgtca cctgcacagg gtaccaccag gtccgtgttt atgatccagc atccccccag 660
cgccggccag tcctagagac cacctatgga gagtacccac taacagccat gaccctcact 720
ccgggaggca actcagtgat tgtgggaaac actcatgggc agctggcaga aattgacctt 780
cggcaagggc gtctactggg ctgtctgaag gggctggcag gcagtgtgcg tgggttgcag 840
tgccaccctt caaagcctct actagcctcc tgtggcttgg acagagtctt gaggatacac 900
aggatccaga atccacgggg tctggagcat aaggtttatc tcaagtctca attgaactgc 960
ctcctcttgt caggcaggga caactgggag gatgagcccc aagagcctca agaacccaac 1020

aaggtgcccc tagaagacac agagacagat gaactttggg catccttgga ggcagctgcc 1080

aagcggaagc tctcgggttt ggagcagccc caaggagctc tccaaacgag acggagaaag 1140

aagaagcggc ctgggtccac cagcccttaa ttaacccagc tttctt 1186

<210> 517

<211> 406

<212> DNA

<213> Homo sapiens

<400> 517
aaaaaagcag gcatggcgga cgaggagctt gaggcgctga ggagacagag gctggccgag 60

ctgcaggcca aacacgggga tcctggtgat gcggcccaac aggaagcaaa gcacagggaa 120

gcagaaatga gaaacagtat cttagcccaa gttctggatc agtcggcccg ggccaggtta 180

agtaacttag cacttgtaaa gcctgaaaaa actaaagcag tagagaatta ccttatacag 240

atggcaagat atggacaact aagtgagaag gtatcagaac aaggtttaat agaaatcctt 300

aaaaaagtaa gccaacaaac aaaaaagaca acaacagtga aattcaacag aagaaaagta 360

atggactctg atgaagatga cgattattaa ttaacccagc tttctt 406

<210> 518

<211> 628

<212> DNA

<213> Homo sapiens

<400> 518
aaaaaagcag gcatgcttgt gtgtgccctg aaaactgcag agaatccaca aaataccaga 60

gcctggttaa accccactga ggttgaggaa acgtcccatg cagtcaactg ttgctactta 120

aatgcagctg tttctgcatt ttttgatcgc tgcagaacat ctgaggtagt agaaatccga 180

gcactgagaa cagatggaga gcacatgaaa aaggaagaat taaatgtgtg caaccacatg 240

gaggtgggcc aaacaggtag ccagaacttg accacaggaa cagacaaacc ggaggcagca 300

agtccctttg gccctgatct ggtccctgta gcacagaggt ccgaagaacc gccttcagaa 360

agaggaggca tatttggaag tgatgggaaa gactttgtgg acaaggatgg agaacacttt 420

ggaaaattgc attgtgccaa gagacaacaa ataatgagca acagtgaaaa tgcaattgag 480

gaacagattg cctcaaatac tactacgact cctcaaacat tcatttctac ggatgacttg 540

ctggactgct tggtgaatcc acaagtaacc aggatagtgg cacaactttt gatacaagga 600

agaagttttt aattaaccca gctttctt 628

<210> 519

<211>  1480

<212>  DNA

<213>  Homo sapiens


<400>  519
aaaaaagcag gcatgggccg gaccgtggtc gtgctgggcg gaggcatcag cggcttggcc      60

gccagttacc acctgagccg ggccccctgc ccccctaagg tggtcctagt ggagagcagt     120

gagcgtctgg gaggctggat tcgctccgtt cgaggcccta atggtgctat ctttgagctt     180

ggacctcggg gaattaggcc agcgggagcc ctaggggccc ggaccttgct cctgagggca     240

tgtggagagc aggtttctga gcttggcttg gattcagaag tgctgcctgt ccggggagac     300

cacccagctg cccagaacag gttcctctac gtgggcggtg ccctgcatgc cctacccact     360

ggcctcaggg ggctactccg cccttcaccc cccttctcca aacctctgtt ttgggctggg     420

ctgagggagc tgaccaagcc ccggggcaaa gagcctgatg agactgtgca cagttttgcc     480

cagcgccgcc ttggacctga ggtggcgtct ctagccatgg acagtctctg ccgtggagtg     540

tttgcaggca acagccgtga gctcagcatc aggtcctgct ttcccagtct cttccaagct     600

gagcaaaccc atcgttccat attactgggc ctgctgctgg gggcagggcg gaccccacag     660

ccagactcag cactcattcg ccaggccttg gctgagcgct ggagccagtg gtcacttcgt     720

ggaggtctag agatgttgcc tcaggccctt gaaacccacc tgactagtag gggggtcagt     780

gttctcagag gccagccggt ctgtgggctc agcctccagg cagaagggcg ctggaaggta     840

tctctaaggg acagcagtct ggaggctgac cacgttatta gtgccattcc agcttcagtg     900

ctcagtgagc tgctccctgc tgaggctgcc cctctggctc gtgccctgag tgccatcact     960

gcagtgtctg tagctgtggt gaatctgcag taccaaggag cccatctgcc tgtccaggga    1020

tttggacatt tggtgccatc ttcagaagat ccaggagtcc tgggaatcgt gtatgactca    1080

gttgctttcc ctgagcagga cgggagcccc cctggcctca gagtgactgt gatgctggga    1140

ggttcctggt tacagacact ggaggctagt ggctgtgtct tatctcagga gctgtttcaa    1200

cagcgggccc aggaagcagc tgctacacaa ttaggactga aggagatgcc gagccactgc    1260

ttggtccatc tacacaagaa ctgcattccc cagtatacac taggtcactg gcaaaaacta    1320

gagtcagcta ggcaattcct gactgctcac aggttgcccc tgactctggc tggagcctcc    1380

tatgagggag ttgctgttaa tgactgtata gagagtgggc gccaggcagc agtcagtgtc    1440

ctgggcacag aacctaacag ttaattaacc cagctttctt                          1480

<210>  520

<211>  955

<212>  DNA

<213>  Homo sapiens

<400>  520
aaaaaagcag gcatgatcat ctctcgagag atgtttaacc ctatgtatgc cttgttccgt      60

acctcacctg gtgatcgagt cacctacacc atcaatccat cttcccactg caaccccaac     120

cacctcagct acttcaagtt tgtcggacgc attgtggcca aagctgtata tgacaaccgt     180

cttctggagt gctactttac tcgatccttt tacaaacaca tcttgggcaa gtcagtcaga     240

tatacagata tggagagtga agattaccac ttctaccaag gtctggttta tctgctggaa     300

aatgatgtct ccacactagg ctatgacctc accttcagca ctgaggtcca agagtttgga     360

gtttgtgaag ttcgtgacct caaacccaat ggggccaaca tcttggtaac agaggagaat     420

aagaaggagt atgtacacct ggtatgccag atgagaatga caggagccat ccgcaagcag     480

ttggcggctt tcttagaagg cttctatgag atcattccaa agcgcctcat ttccatcttc     540

actgagcagg agttagagct gcttatatca ggactgccca ccattgacat cgatgatctg     600

aaatccaaca ctgaatacca caagtaccag tccaactcta ttcagatcca gtggttctgg     660

agagcattgc gttctttcga tcaagctgac cgtgccaagt tcctccagtt tgtcacgggt     720

acttccaagg tacccctgca aggctttgct gccctcgaag gcatgaatgg cattcagaag     780

tttcagatcc atcgagatga caggtccaca gatcgcctgc cttcagctca cacatgtttt     840

aatcagctgg atctgcctgc ctatgagagc tttgagaagc tccgccacat gctactgttg     900

gctatccagg agtgctctga aggctttggg ctggcttaat taacccagct ttctt          955

<210>  521

<211>  547

<212>  DNA

<213>  Homo sapiens


<400>  521
aaaaaagcag gcatgtgtga gaactgcatg acaaccaacc agaagaaggc gctcaaggtg      60

gagcacacca gccggctgaa ggccgccttt gtgaaggcgc tgcagcagga acaggagatt     120

gagcagcggc tcctgcagca gggcacggcc cctgcacagg ccaaggccga gcccaccgct     180

gccccacacc ccgtgctgaa gcaggcctcc agccagctgt cccggggttc ggccacgacg     240

ccccgaggtg tcctgcacac gttcagtccg tcacccaaac tgcagaactc agcctcggcc     300

acagccctgg tcagcaggac cggcagacat tctgagagaa ccgtgagcgc cggcaagggc     360

agcgccacct ccaactggaa gaagacgccc ctcagcacag gcgggaccct tgcgtttgtc     420

agcccaagcc tggcggtgca caagagctcc tcggccgtgg accgccagcg agagtacctc     480

ctggacatga tcccaccccg ctccatcccc cagtcagcca cgtggaatta attaacccag     540

ctttctt                                                               547

<210>  522

<211>  737

<212>  DNA

<213>  Homo sapiens


<400>  522
aaaaaagctg gcacccctgg tccaggtctg ttcgagcctg tgagctacac cttcacgtac      60

ctgctgggtg acagagagtc ccgggaggcc gttctgatcg acccagtcct ggaaacagcg     120

cctcgggatg cccagctgat caaggagctg gggctgcggc tgctctatgc tgtgaatacc     180

cactgccacg cggaccacat tacaggctcg gggctgctcc gttccctcct ccctggttgc     240

cagtctgtca tctcccgcct tagtggggcc caggctgact tacacattga ggatggagac     300

tccatccgct tcgggcgctt cgcgttggag accagggcca gccctggcca caccccaggc     360

tgtgtcacct tcgtcctgaa tgaccacagc atggccttca ctggagatgc cctgttgatc     420

cgtgggtgtg ggcggacaga cttccagcaa ggctgtgcca agaccttgta ccactcggtc     480

catgaaaaga tcttcacact tccaggagac tgtctgatct accctgctca cgattaccat     540

gggttcacag tgtccaccgt ggaggaggag aggactctga accctcggct caccctcagc     600

tgtgaggagt ttgtcaaaat catgggcaac ctgaacttgc ctaaacctca gcagatagac     660

tttgctgttc cagccaacat gcgctgtggg gtgcagacac ccactgcccc aggcccaggc     720

ggcgacccag ctttctt                                                    737

<210>  523

<211>  299

<212>  DNA

<213>  Homo sapiens


<400>  523
aaaaaagctg gcacccctgg tccaggtatg ctaattaaag tgaagacgct gaccggaaag      60

gagattgagg ttgacattga acctacagac aaggtggagc gaatcaagga gcgtgtggag     120

gagaaagagg gaatcccccc acaacagcag aggctcatct acagtggcaa gcagatgaat     180

gatgagaaga cagcagctga ttacaagatt ttaggtggtt cagtccttca cctggtgttg     240

gctctgagag gaggaggtgg tcttaggcag ccaggcccag gcggcgaccc agctttctt      299

<210>  524

<211>  1205

<212>  DNA

<213>  Homo sapiens


<400>  524

aaaaaagctg gcacccctgg tccaggtatg gaacggctgc agaagcaacc acttacctcc 60
cccgggagcg tgagcccctc ccgagattcc agtgtgcctg gctctccctc cagcatcgtg 120
gccaagatgg acaatcaggt gctgggctac aaggacctgg ctgccatccc caaggacaag 180
gccatcctgg acatcgagcg gcccgacctc atgatctacg agcctcactt cacttattcc 240
ctcctggaac acgtggagct gcctcgcagc cgcgagcgct cgctgtcacc caaatccaca 300
tcccccccac catccccaga ggtgtgggcg gacagccggt cgcctggaat catctctcag 360
gcctcggccc ccagaaccac tggaaccccc cggaccagcc tgccccattt ccaccaccct 420
gagacctccc gcccagattc caacatctac aagaagcctc ccatctataa gcagagagag 480
tccgtgggag gcagccctca gaccaagcac ctcatcgagg atctcatcat cgagtcatcc 540
aagtttcctg cagcccagcc cccagacccc aaccagccag ccaaaatcga aaccgactac 600
tggccatgcc ccccgtctct ggctgttgtg gagacagaat ggaggaagcg gaaggcgtct 660
cggaggggag cagaggaaga ggaggaggag gaagatgacg actctggaga ggagatgaag 720
gctctcaggg agcgtcagag agaggaactc agtaaggtta cttccaactt gggaaagatg 780
atcttgaaag aagagatgga aaagtcattg ccgatccgaa ggaaaacccg ctctctgcct 840
gaccggacac ccttccatac ctccttgcac cagggaacgt ctaaatcttc ctctctcccc 900
gcctatggca ggaccaccct gagccggcta cagtccacag agttcagccc atcagggagt 960
gagactggaa gcccaggcct gcagatctat ccctatgaaa tgctagtggt gaccaacaag 1020
gggcgaacca agctgccacc gggggtggat cggatgcggc ttgagaggca tctgtctgcc 1080
gaggacttct caagggtatt tgccatgtcc cctgaagagt ttggcaagct ggctctgtgg 1140
aagcggaatg agctcaagaa gaaggcctct ctcttcccag gcccaggcgg cgacccagct 1200
ttctt 1205

<210> 525
<211> 579
<212> DNA
<213> Homo sapiens

<400> 525
aaaaaagcag gcatgatgac tgcatccaac tcgcaggtgc gccagaacta ccaccaggac 60
tcagaggccg ccatcaaccg ccagatcaac ctggagctct acgcctccta cgtttacctg 120
tccatgtctt actactttga ccgcgatgat gtggctttga agaactttgc caaatacttt 180
cttcaccaat ctcatgagga gagggaacat gctgagaaac tgatgaagct gcagaaccaa 240
cgaggtggcc gaatcctcct tcaggatatc aagaaaccag actgtgatga ctgggagagc 300
gggctgaatg caatggagtg tgcattacat ttggaaaaaa tgtgaatcag tcactactgg 360
aactgcgcaa actggccact gacaaaaatg acccccattt gtgtgacttc attgagacac 420
attacctgaa tgagcaggtg aaagccatca aagaattggg tgaccacgtg accaacttgc 480

gcaagatggg agcgcccgaa tctggcttgg cggaatatct ctttgacaag cacaccctgg 540

gagaaagtga taatgaaagt taattaaccc agcttttttt 579

<210> 526

<211> 183

<212> DNA

<213> Homo sapiens

<400> 526
aaaaaagcag gcatgtcttc tacaagactt tcaggattaa gcgattcctg gccaagaaac 60

aaaagcaaaa tcgtcccatt ccccagtgga ttcggatgaa aactggaaat aaaatcaggt 120

acaactccaa aaggagacat tggagaagaa ccaagctggg tctttaatta acccagcttt 180

ctt 183

<210> 527

<211> 398

<212> DNA

<213> Homo sapiens

<400> 527
aaaaaagcag gctccaccat gtcccagaaa cgagaagaag agaaccctgc ggaggagacc 60

ggcgaggaga agcaggacac gcaggagaaa gaaggtattc tgcctgagag agctgaagag 120

gcaaagctaa aggccaaata cccaagccta ggacaaaagc ctggaggctc cgacttcctc 180

atgaagagac tccagaaagg gcaaaagtac tttgactcag gagactacaa catggccaaa 240

gccaagatga agaataagca gctgccaagt gcaggaccag acaagaacct ggtgactggt 300

gatcacatcc ccaccccaca ggatctgccc cagagaaagt cctcgctcgt caccagcaag 360

cttgcgggtg gccaagttga atgagaccca gctttctt 398

<210> 528

<211> 1276

<212> DNA

<213> Homo sapiens

<400> 528
aaaaaagcag gcatgactgg ggagcgaccc agcacggcgc tcccggacag acgctggggg 60

ccgcggattc tgggcttctg gggaggctgt agggtttggg tgtttgccgc gatcttcctg 120

ctgctgtctc tggcagcctc ctggtccaag gctgagaacg acttcggtct ggtgcagccg 180

ctggtgacca tggagcaact gctgtgggtg agcgggagac agatcggctc agtggacacc 240
ttccgcatcc cgctcatcac agccactccg cggggcactc ttctcgcctt tgctgaggcg 300
aggaaaatgt cctcatccga tgagggggcc aagttcatcg ccctgcggag gtccatggac 360
cagggcagca catggtctcc tacagcgttc attgtcaatg atggggatgt ccccgatggg 420
ctgaaccttg gggcagtagt gagcgatgtt gagacaggag tagtatttct tttctactcc 480
ctttgtgctc acagggccgg ctgccaggtg gcctctacca tgttggtatg gagcaaggat 540
gatggtgttt cctggagcac accccggaat ctctccctgg atattggcac tgaagtgttt 600
gcccctggac cgggctctgg tattcagaaa cagcgggagc cacggaaggg ccgcctcatc 660
gtgtgtggcc atgggacgct ggagcgggac ggagccttct gtctcctcag cgatgatcat 720
ggtgcctcct ggcgctacgg aagtggggtc agcggcatcc cctacggtca gcccaagcag 780
gaaaatgatt tcaatcctga tgaatgccag ccctatgagc tcccagatgg ctcagtcgtc 840
atcaatgccc gaaaccagaa caactaccac tgccactgcc gaattgtcct ccgcagctat 900
gatgcctgtg atacactaag gccccgtgat gtgaccttcg accctgagct cgtggaccct 960
gtggtacctg caggagctgt agtcaccagc tccggcattg tcttcttctc caacccagca 1020
catccagagt tccgagtgaa cctgaccctg cgatggagct tcagcaatgg tacctcatgg 1080
cggaaagaga cagtccagct atggccaggc cccagtggct attcatccct ggcaaccctg 1140
gagggcagca tggatggaga ggagcaggcc ccccagctct acgtcctgta tgagaaaggc 1200
cggaaccact acacagagag tatctccgtg gccaaaatca gtgtctatgg gacactttaa 1260
ttaacccagc tttctt 1276

<210> 529
<211> 604
<212> DNA
<213> Homo sapiens

<400> 529
aaaaaagcag gcatggcggt ggccagggcc ggggtcttgg gagtccagtg gctgcaaagg 60
gcatcccgga acgtgatgcc gctgggcgca cggacagcct cccacatgac caaggacatg 120
ttcccggggc cctatcctag gatcccagaa gaacgggccg ccgccgccaa gaagtataat 180
atgcgtgtgg aagactacga accttacccg gatgatggca tggggtatgg cgactacccg 240
aagctccctg accgctcaca gcatgagaga gatccatggt atagctggga ccagccgggc 300
ctgaggttga actggggtga accgatgcac tggcacctag acatgtacaa caggaaccgt 360
gtggatacat cccccacacc tgtttcttgg catgtcatgt gtatgcagct cttcggtttc 420
ctggctttca tgatattcat gtgctgggtg ggggacgtgt accctgtcta ccagcctgtg 480
ggaccaaagc agtatcctta caataatctg tacctggaac gaggcggtga tccctccaaa 540
gaaccagagc gggtggttca caatgagatc tgaggaggct tcgtgggctt tacccagctt 600

tctt 604

<210> 530

<211> 643

<212> DNA

<213> Homo sapiens

<400> 530
aaaaaagcag gcatgggagc cgtgactgac gacgaagtta tacggaagcg tctcctcatt 60

gatggagatg gtgctggaga tgatcggaga attaatctgc tagtgaagag tttcattaaa 120

tggtgcaact ctgggtccca ggaagaggga tatagccagt accaacgtat gctgagcacg 180

ctgtctcaat gtgaattttc aatgggcaaa actttactag tatatgatat gaatctcaga 240

gaaatggaaa attatgaaaa aatttacaag gaaatagaat gtagcatagc tggagcacat 300

gaaaaaattg ctgagtgcaa aaagcaaatt cttcaagcaa aacgaatacg aaaaaatcgc 360

caagaatatg atgctttggc aaaagtgatt cagcaccatc cagacaggca tgagacatta 420

aaggaactag aggctctggg aaaagaatta gagcatcttt cacacattaa agaaagtgtt 480

gaagataagc tggaattgag acggaaacag tttcatgttc ttcttagtac catccatgaa 540

cttcagcaaa cattggaaaa tgatgaaaaa ccctcagagg tagaagaagc tcaggaagca 600

agcatggaat cagatcctaa gccttaatta acccagcttt ctt 643

<210> 531

<211> 526

<212> DNA

<213> Homo sapiens

<400> 531
aaaaaagcag gcatggtcaa ccccaccgtg ttcttcgaca ttgccgtcga cggcgagccc 60

ttgggccgcg tctcctttga gctgtttgca gacaaggtcc caaagacagc agaaaatttt 120

cgtgctctga gcactggaga gaaaggattt ggttataagg gttcctgctt tcacagaatt 180

attccagggt ttatgtgtca gggtggtgac ttcacacgcc ataatggcac tggtggcaag 240

tccatctatg gggagaaatt tgaagatgag aacttcatcc taaagcatac gggtcctggc 300

atcttgtcca tggcaaatgc tggacccaac acaaatggtt cccagttttt catctgcact 360

gccaagactg agtggttgga tggcaagcat gtggtgtttg gcaaagtgaa agaaggcatg 420

aatattgtgg aggccatgga gcgctttggg tccaggaatg gcaagaccag caagaagatc 480

accattgctg actgtggaca actcgattaa ttaacccagc tttctt 526

<210> 532

<211>  449

<212>  DNA

<213>  Homo sapiens


<400>  532
aaaaaagcag gctccaccat gcgcgaggag aacaagggca tgcccagtgg gggcggcagc      60

gatgagggtc tggccagcgc cgcggcgcgg ggactagtgg agaaggtgcg acagctcctg     120

gaagccggcg cggatcccaa cggagtcaac cgtttcggga ggcgcgcgat ccaggtcatg     180

atgatgggca gcgcccgcgt ggcggagctg ctgctgctcc acggcgcgga gcccaactgc     240

gcagaccctg ccactctcac ccgaccggtg catgatgctg cccgggaggg cttcctggac     300

acgctggtgg tgctgcaccg ggccggggcg cggctggacg tgcgcgatgc ctggggtcgt     360

ctgcccgtgg acttggccga tgagcggggc caccgcgacg ttgcagggta cctgcgcaca     420

gccacggggg actgagaccc agctttctt                                       449

<210>  533

<211>  454

<212>  DNA

<213>  Homo sapiens


<400>  533
aaaaaagcag gcatgtcaaa catgagatgt gtggactgtg gcacttgcct gggtcacaca      60

cggaggcatc ctaccctttt ctggggaaag acactgcctg ggctgacccc ggtggcggcc     120

ccagcacctc agcctgcaca gtgtccccca ggttccgaag aagatgctcc agcaacacag     180

cctgggcccc agctcgcggg acccgacccc ccgtgggctc ccgtgttttg taggagactt     240

gccagagccg ggcacattga gctgtgcaac gccgtgggct gcgtcctttg gtcctgtccc     300

cgcagccctg gcagggggca tgcggtcggg caggggctgg agggaggcgg gggctgccct     360

tgggccaccc ctcctagttt gggaggagca gatttttgca ataccaagta tagcctatgg     420

cagaaaaaat gtctttaatt aacccagctt tctt                                 454

<210>  534

<211>  878

<212>  DNA

<213>  Homo sapiens


<400>  534
aaaaaagctg gcccccctgg tccaggtatg gagaagctga ctgagaagtt ccagcgcttc      60

acacctttca ccctgggcaa ggagttcaaa gaaggacaca gctactacta catctccaaa     120

cccatccacc agcatgaaga ccgctgcttg aggttgaagg tgactgtcag tggcaaaatc 180

actcacagtc ctcaggccca tgtcaatcca caggagaaga gacttgcagc agatgaccca 240

gaggtgcggg ttctacatag catcggtcac agtgctgccc cacgcctctt cccacttgcc 300

tggactgtgc tgctccttcc acttctgctg ctgcaaaccc cgtgaaggtg tatgccacac 360

ctggccttaa agagggacag gctgaagaga gggacaggca ctccaaacct gtcttggggc 420

cactttcaga gcccccagcc ctgggaacca ctcccaccac aggcataagc tatcacctag 480

cagcctcaaa acgggtcagt attaaggttt tcaaccggaa ggaggccaac cagcccgaca 540

gtgccatccc caccttcacc tcggagggat ggagaaagaa gtggagacag tcctttccca 600

ccattcctgc ctttaagcca aagaaacaag ctgtgcaggc atggtccctt aaggcacagt 660

gggagctgag ctggaagggg ccacgtggat gggcaaagct tgtcaaagat gccccctcca 720

ggagagagcc aggatgccca gacgaactga ctgaaggaaa agcaagaaac agtttcttgc 780

ttggaagcca ggtacaggag aggcagcatg cttgggctga cccagcatct cccaccaaga 840

ccccgttggc caggcccagg cggcgaccca gctttctt 878

<210> 535

<211> 1137

<212> DNA

<213> Homo sapiens

<400> 535
aaaaaagcag gcatgagcag caccttagct aagatcgcgg agatagaagc agagatggct 60

cggactcaaa agaacaaggc cacagcacac cacttagggc tgcttaaggc tcgtcttgct 120

aagcttcgtc gagaactcat tactccaaag ggtggtggtg gtggaggtcc aggagaaggt 180

tttgatgtgg ccaagacagg tgatgctcga attggatttg ttggttttcc atctgtgggg 240

aagtcaacac tgcttagtaa cctggcaggg gtatattctg aggtggcagc ctatgaattc 300

actactctga ccactgtgcc tggtgtcatc agatacaaag gtgccaagat ccagctcctg 360

gatctcccag gtatcattga aggtgccaag gatgggaaag gtagaggtcg tcaagtcatt 420

gcagtggccc gaacctgtaa cttgatcttg attgttctgg atgtcctgaa acctttggga 480

cataagaaga taattgaaaa tgagctggaa ggctttggca ttcgcttgaa cagcaaaacc 540

ccccaacatt ggctttaaga agaaggacaa gggaggcatt aatctcacag ccacttgccc 600

ccagagtgag ctggatgctg aaactgtgaa gagcattctg gctgaataca agattcataa 660

tgccgatgtg actctacgta gtgatgctac agctgatgac ctcattgatg tggtggaagg 720

aaacagagtt tatatcccct gtatctatgt gttaaataag attgaccaaa tctccattga 780

ggaattggat atcatctata aggtgcctca ctgtgtaccc atctctgccc atcaccgctg 840

gaattttgat gacctattgg aaaagatctg ggactatctg aaactagtga gaatttacac 900

caaacccaaa ggccagttac cagattacac atccccagtg gtgcttcctt actccaggac 960
cacagtggag gatttctgca tgaagattca caaaaatctt atcaaagaat ttaaatagca 1020
gtgctctggt ctggggtctc tctgtgaaac acaatcctca gaaagtgggt aaagaccata 1080
cgttggagga tgaggatgtc attcaaattg tgaagaatta attaacccag ctttctt 1137

<210> 536
<211> 1355
<212> DNA
<213> Homo sapiens

<400> 536
aaaaaagcag gcatggacgc tcccccgggc ggggttgagt cggcgctcag ctgcttctct 60
ttcaaccagg actgcacatc cctagcaatt ggaactaaag ccgggtataa gctgttttct 120
ctgagttctg tggagcagct ggatcaagtc cacggaagca atgaaatccc ggacgtctac 180
atcgtggagc gcctcttctc cagcagcctg gtggtggtag tcagtcacac aaaaccacgg 240
cagatgaacg tgtatcactt caagaaaggc acagagatct gtaattacag ctactccagc 300
aacatcttgt ccataaggct gaaccggcaa aaggctgctg gtttgcctag aagagtccat 360
ttatattcac aacattaaag acatgaagct gttgaagacc ctcctggata ttcctgcaaa 420
cccaacaggt ctatgtgctc tctctatcaa ccattccaat tcttacctgg cctatcctgg 480
aagcctgact tcaggggaga ttgtgcttta tgatggaaac tccctgaaaa cagtctgcac 540
tattgctgac catgagggaa cactagctgc catcaccttc aatgcctcag gctccaaact 600
agcaagtgcg tctgaaaaag gcacagtcat ccgggtgttc tctgtccctg atgggcaaaa 660
gctctatgag ttccggagag ggatgaaaag gtatgcgaca atcagctctc tagtgttcag 720
tatggattca caattcctct gcgcctccag taacaccgag acggtacaca tcttcaagct 780
ggaacaggtc accaacagtc gaccagaaga gccttcgacc tggagtggct acatgggaaa 840
gatgtttatg gctgctacca actacctccc tacccaggtg tcagacatga tgcatcagga 900
cagggctttt gccactgcac gcttgaactt ctccggacag aggaacatct gtaccctctc 960
aacgatccag aagttgccac ggctgctagt tgcgtcatcc agtggacacc tttatatgta 1020
caatttggat cctcaggatg gaggagagtg tgtcttaatc aaaacccaca gcttgcttgg 1080
ctcaggaaca acagaagaga ataaagaaaa tgacctcaga ccttccttac ctcagtctta 1140
tgcagcgacc gtagccagac caagtgcatc ttcagcctcc acggtgccag gttattctga 1200
ggacggcggg gcgctgcgag gagaagttat tcctgaacat gagtttgcga cgggaccagt 1260
gtgtcttgat gatgagaatg agtttcctcc tataatcttg tgccgtggaa atcagaaggg 1320
caaaacgaag cagtcttaat taacccagct ttctt 1355

<210> 537

<211>  1476

<212>  DNA

<213>  Homo sapiens

<220>

<221>  misc_feature

<222>  (65)..(65)

<223>  n = a, c, g or t

<220>

<221>  misc_feature

<222>  (78)..(78)

<223>  n = a, c, g or t

<220>

<221>  misc_feature

<222>  (85)..(85)

<223>  n = a, c, g or t

<220>

<221>  misc_feature

<222>  (87)..(89)

<223>  n = a, c, g or t

<220>

<221>  misc_feature

<222>  (90)..(90)

<223>  n = a, c, g or t

<220>

<221>  misc_feature

<222>  (92)..(92)

<223>  n = a, c, g or t

<400>  537

aaaaaagcag gcatggagcg ggacgaaccg ccgcctagcg gagaggggaa ggccggcggg 60
ggctncggcg ggggttcntg aaggntnnnn gngggggggt tcccttccgc cgcgcgcaac 120
ggtttctgtc aggatgaatt ggcagagctt gacccaggca ctatttctgt ttcagatgat 180
cgggctgaac aacgaacctg tctcatttgt ggggaccgcg ctacaggctt gcactatggg 240
atcatctcct gtgagggctg caaagggttt ttcaagcgga gcatttgcaa caaacgggta 300
tatcgatgca gtcgtgacaa gaactgtgtc atgtctcgga agcagaggaa caggtgccag 360
tactgccgcc tgctcaaatg cctccagatg gggatgaacc ggaaggctat cagagaagat 420
ggcatgcctg gaggccggaa taagagcatt gggccagtcc agatatcgga agaagaaatc 480
gaaaggatca tgtctgggca ggagtttgag gaagaggcca atcactggag caaccatggt 540
gatagtgacc acagttcccc tgggaacagg gcttcggaga gcaaccagcc ctcaccaggc 600
tccacactgt cttccaggtc tgtggaactg aatggattca tggccttcag ggaacagtac 660
atgggaatgt ctgtgcctcc acattaccaa tatataccgc acctttttag ctattctggc 720
cactcaccac ttctgcccca acaagctcgc agcctggatc cccagtcata cagtctgatt 780
caccagctgt tatcagccga ggacctggaa ccattgggca cgcccatgtt gattgaagat 840
ggatacgctg tgacacaggc agaactattt gccctgcttt gccgcctggc cgacgagctg 900
ctctttaggc agattgcctg gatcaagaaa ctgcctttct tctgcgagct ctcaatcaag 960
gattacacgt gcctcttgag ctctacgtgg caggagctaa tcctgctgtc ttccctcacc 1020
gtttacagca agcagatctt tggggaactg gctgatgtca ctgccaagta ctcgccctcc 1080
gatgaagaac tacacagatt tagtgatgaa gggatggagg tgatcgagcg gctcatctac 1140
ctctatcaca agttccatca gctaaaggtc agcaacgagg agtatgcttg catgaaagca 1200
attaacttcc taaatcaaga tatcaggggt ctgaccagtg cctcacagct ggaacaattg 1260
aataaacgat actggtacat ttgccaggat tttactgaat ataaatacac acatcagccg 1320
aaccgctttc ctgatctcat gatgtgctta cctgagattc gatatattgc aggaaagatg 1380
gtgaatgtgc ccctggagca gctgcccctc ctctttaagg tggtgctgca ttcctgcaag 1440
accatgtggg caaggattaa ttaacccagc tttctt 1476

<210> 538
<211> 624
<212> DNA
<213> Homo sapiens

<400> 538
aaaaaagctg gcacccctgg tccaggtatg actgagtaca aactggtggt ggttggagca 60
ggtggtgttg ggaaaagcgc actgacaatc cagctaatcc agaaccactt tgtagatgaa 120
tatgatccca ccatagagga ttcttacaga aaacaagtgg ttatagatgg tgaaacctgt 180
ttgttggaca tactggatac agctggacaa gaagagtaca gtgccatgag agaccaatac 240

atgaggacag gcgaaggctt cctctgtgta tttgccatca ataatagcaa gtcatttgcg 300
gatattaacc tctacaggga gcagattaag cgagtaaaag actcggatga tgtacctatg 360
gtgctagtgg gaaacaagtg tgatttgcca acaaggacag ttgatacaaa acaagcccac 420
gaactggcca agagttacgg gattccattc attgaaacct cagccaagac cagacagggt 480
gttgaagatg ctttttacac actggtaaga gaaatacgcc agcaccgaat gaaaaaaact 540
caacagcagt gatgatggga ctcagggttg tatgggattg ccatgtgtgg tgatgccagg 600
cccaggcggc gacccagctt tctt 624

<210> 539
<211> 689
<212> DNA
<213> Homo sapiens

<400> 539
aaaaaagctg gcacccctgg tccaggtatg gattatttgc tcatgatttt ctctctgctg 60
tttgtggctt gccaaggagc tccagaaaca gtcttaggcg ctgagctcag cgcggtgggt 120
gagaacggcg gggagaaacc cactcccagt ccaccctggc ggctccgccg gtccaagcgc 180
tgctcctgct cgtccctgat ggataaagag tgtgtctact tctgccacct ggacatcatt 240
tgggtcaaca ctcccgagca cgttgttccg tatggacttg gaagccctag gtccaagaga 300
gccttggaga atttacttcc cacaaaggca acagaccgtg agaatagatg ccaatgtgct 360
agccaaaaag acaagaagta ctggaatttt tgccaagcag gaaaagaact cagggctgaa 420
gacattatgg agaaagactg gaataatcat aagaaaggaa aagactgttc caagcttggg 480
aaaaagtgta tttatcagca gttagtgagg ggaagaaaaa tcagaagaag ttcagaggaa 540
cacctaagac aaaccaggtc ggagaccatg agaaacagcg tcaaatcatc ttttcatgat 600
cccaagctga aaggcaagcc ctccagagag cgttatgtga cccacaaccg agcacattgg 660
ccaggcccag gcggcgaccc agctttctt 689

<210> 540
<211> 1113
<212> DNA
<213> Homo sapiens

<400> 540
aaaaaagcag gcatggatat acaaatggca aacaatttta ctccgccctc tgcaactcct 60
cagggaaatg actgtgacct ctatgcacat cacagcacgg ccaggatagt aatgcctctg 120
cattacagcc tcgtcttcat cattgggctc gtgggaaact tactagcctt ggtcgtcatt 180

gttcaaaaca ggaaaaaaat caactctacc accctctatt caacaaattt ggtgatttct 240
gatatacttt ttaccaccgc tttgcctaca cgaatagcct actatgcaat gggctttgac 300
tggagaatcg gagatgcctt gtgtaggata actgcgctag tgttttacat caacgcatat 360
gcaggtgtga actttatgac ctgcctgagt attgaccgct tcattgctgt ggtgcaccct 420
ctacgctaca acaagataaa aaggattgaa catgcaaatg cgtgtgcata tttgtctgga 480
ttctagtatt tgctcagaca ctcccactcc tcatcaaccc tatgtcaaag caggaggctg 540
aaaggattac atgcatggag tatccaaact ttgaagaaac taaatctctt ccctggattc 600
tgcttggggc atgtttcata ggatatgtac ttccacttat aatcattctc atctgctatt 660
ctcagatctg ctgcaaactc ttcagaactg ccaaacaaaa cccactcact gagaaatctg 720
gtgtaaacaa aaaggctctc aacacaatta ttcttattat tgttgtgttt gttctctgtt 780
tcacacctta ccatgttgca attattcaac atatgattaa gaagcttcgt ttctctaatt 840
tcctggaatg tagccaaaga cattcgttcc agatttctct gcactttaca gtatgcctga 900
tgaacttcaa ttgctgcatg gaccctttta tctacttctt tgcatgtaaa gggtataaga 960
gaaaggttat gaggatgctg aaacggcaag tcagtgtatc gatttctagt gctgtgaagt 1020
cagcccctga agaaaattca cgtgaaatga cagaaacgca gatgatgata cattccaagt 1080
cttcaaatgg aaattaatta acccagcttt ctt 1113

<210> 541
<211> 1113
<212> DNA
<213> Homo sapiens

<400> 541
aaaaaagcag gcatgacggg gaaaaagtcc tcccgggaga aacggcgcaa acgtagcagt 60
caggaggcgg ccgcagcgct cgcggccccg gacatcgtac ccgcgttggc cagcggcagc 120
agtggaagca ctagcggctg cgggagcgcc gggggctgcg ggagcgtcag ctgctgtggg 180
aacgccaatt ttagtggaag tgtcaccggc ggtgggagtg gcggcagctg ttggggcggg 240
agcagcgtgg agcgcagcga gcgccggaag cggaggagta ccgactcttc cagcgtctcg 300
ggctccttgc agcaggaaac taaatatatt ttgccaactt tggaaaaaga attattcttg 360
gcagagcaca gtgaccttga agaaggtgga ctggacctga ctgtgtcatt gaaaccagtt 420
agtttctata tatcagacaa aaaagaaatg cttcagcagt gcttctgtat tataggagag 480
aaaaagttac agaagatgct tcctgatgtg ttaaagaact gttcaataga agaaattaaa 540
aaactatgcc aggaacagtt agagctcctg tctgaaaaaa aattttgaag attcttgagg 600
gtgacaatgg gatggactct gatatggaag aggaagcaga tgatggttct aagatgggat 660
ctgatttagt cagtcagcaa gacatctgta tagattctgc ttcatccgtg agagagaata 720
agcaacctga aggtttggaa ttaaaacaag gaaaagggga agatagtgat gtactcagta 780

taaatgcaga tgcttatgac agcgacatag aaggcccatg caacgaagaa gcagctgctc 840

ccgaggcacc agaaaataca gtccaaagtg aagctggtca gatagatgac ctggagaaag 900

acattgagaa aagtgtgaat gagattctag gactggcaga gtctagccca aacgaaccca 960

aagcagccac cctggctgtt cctccaccag aagatgttca accttctgca cagcaactgg 1020

agctgctaga acttgagatg agggcaagag cgattaaagc cctaatgaaa gctggtgata 1080

taaaaaagcc agcttaatta acccagcttt ctt 1113

<210> 542

<211> 517

<212> DNA

<213> Homo sapiens

<400> 542
aaaaaagcag gcatgttacc attgcaaggt gcccagatgc tgcagatgct ggagaaatcc 60

ttgaggaaga gcctcccagc atccttaaag gtttatggaa ctgtctttca cataaaccat 120

ggaaatccat tcaatctgaa ggctgtggtg gacaagtggc ctgattttaa tacagtggtt 180

gtctgccctc aggagcagga tatgacagat gaccttgatc actataccaa tacttaccaa 240

atctactcca aagatcccca aaactgtcag gaattccttg gatcaccaga actcatcaac 300

tggaaacagc atttacagat tcaaagttca cagcctagcc tgaatgaggc tatacaaaat 360

cttgcagcca ttaagtcctt caaagtcaaa caaacacaac gcattctcta tatggcagct 420

gaaacagcca aggaactgac tcctttcctg ctgaaatcaa agatttatct tcccagtggt 480

ggcaaaccca aggccattta attaacccag ctttctt 517

<210> 543

<211> 1545

<212> DNA

<213> Homo sapiens

<220>

<221> misc_feature

<222> (1096)..(1096)

<223> n = a, c, g or t

<400> 543
aaaaaagctg gcacccctgg tccaggtatg acaccgctcg tctcccgcct gagtcgtctg 60

tgggccatca tgaggaagcc acgagcagcc gtgggaagtg gtcacaggaa gcaggcagcc 120

agccaggaag ggaggcagaa gcatgctaag aacaacagtc aggccaagcc ttctgcctgt 180
gatggcctgg ccaggcagcc ggaagaggtg gtattgcagg cctctgtctc ctcataccat 240
ctattcagag acgtagctga agtcacagcc ttccgaggga gcctgctaag ccggtacgac 300
caagagaaac gggacctacc atggagaaga cgggcagaag atgagatgga cctggacagg 360
cgggcatatg ctggaggtga atcaactctg ggctggcctg ggctactatt ctcgtggccg 420
gcggctgcag gagggagctc ggaaggtggt agaggagcta gggggccaca tgccacgtac 480
agcagagacc ctgcagcagc tcctgcctgg cgtggggcgc tacacagctg gggccattgc 540
ctctatcgcc tttggccagg caaccggtgt ggtggatggc aacgtagcac gggtgctgtg 600
ccgtgtccga gccattggtg ctgatcccag cagcaccctt gtttcccagc agctctgggg 660
tctagcccag cagctggtgg acccagcccg gccaggagat ttcaaccaag cagccatgga 720
gctaggggcc acagtgtgta ccccacagcg cccactgtgc agccagtgcc ctgtggagag 780
cctgtgccgg gcacgccaga gagtggagca ggaacagctc ttagcctcag ggagcctgtc 840
gggcagtcct gacgtggagg agtgtgctcc caacactgga cagtgccacc tgtgcctgcc 900
tccctcggag ccctgggacc agaccctggg agtggtcaac ttccccagaa aggccagccg 960
caagcccccc agggaggaga gctctgccac ctgtgttctg gaacagcctg gggcccttgg 1020
ggcccaaatt ctgctggtgc agaggcccaa ctcaggtctg ctggcaggac tgtgggagtt 1080
cccgtccgtg acctgngagc cctcagagca gcttcagcgc aaggccctgc tgcaggaact 1140
acagcgttgg gctgggcccc tcccagccac gcacctccgg caccttgggg aggttgtcca 1200
caccttctct cacatcaagc tgacatatca agtatatggg ctggccttgg aaggacaaac 1260
cccagtgacc accgtaccac caggtgcccg ctggctgacg caggaggaat ttcacaccgc 1320
agctgtttcc accgccatga aaaaggtttt ccgtgtgtat cagggccaac agccagggac 1380
ctgtatgggt tccaaaaggt cccaggtgtc ctctccgtgc agtcggaaaa agccccgcat 1440
gggccagcaa gtcctggata atttctttcg gtctcacatc tccgctgatg cacacagcct 1500
caacagtgca gcccagccag gcccaggcgg cgacccagct ttctt 1545

<210> 544
<211> 490
<212> DNA
<213> Homo sapiens

<400> 544
aaaaaagcag gcatgaaggc tccctgtcct cagctgccat gtcccccagc ttctcaccct 60
ctctgtctcc tctggctgct cgctcacccg ttgtctcacc atttggggtg gcccagggtc 120
cctcagcctc agcactcagc gcagagtctg gcctggagcc acctgatgac acggagctgc 180
acatctagct gtggcccagg ctgggccccg acctgggatg cgcacagtgt ccccaacgca 240
ggccccactc tgagcctgcc ctgggcagcc tcggactatg actggctacg gggaggccac 300

caccaggccc cagctctcca ccctgaactc cccagccccc tcagagtact aggaccacag 360

aagccctgtt gctcactgac ctgtgaccag gtccaatgtg gggagaaata tgaaggaggt 420

agcagccctg ggttctcctc agtgagggat ccctgccctg caccagcacc ttaattaacc 480

cagctttctt 490

<210> 545

<211> 709

<212> DNA

<213> Homo sapiens

<400> 545
aaaaaagcag gcatgtcgtg ggttcaagca gcctccttga tccagggccc tggagacaaa 60

gggggcgtgt ttgacgaaga agcagacgag tcgctcctgg cgcagcggga atggcagagt 120

aacatgcaaa gacgagtcaa agaaggttat agagatggaa tagatgctgg caaagcagtt 180

actcttcaac agggcttcaa tcaaggttat aagaaaggtg cagaagtcat tttaaactat 240

ggacgactcc gaggaacatt gagtgctttg ctctcctggt gtcaccttca taataataat 300

tcaactttga tcaataaaat aaacaatctt ctggatgcag ttggccagtg tgaagagtat 360

gtgctcaaac atctgaaatc aatcactcca ccgtcccatg ttgtagattt attggactcc 420

attgaggata tggacctttg tcatgtagtt ccagctgaga aaaagattga tgaagctaaa 480

gatgaaagac tctgtgaaaa taatgctgag tttaacaaaa actgtagcaa gagccatagt 540

gggatagatt gttcatatgt agaatgttgt agaacacagg agcatgcaca ttcagaaaac 600

ccaagcccca catggatttt ggaacagaca gccagtttag ttaaacagct ggacctatca 660

gtagatgtat tacaacacct caaacaactt taattaaccc agctttctt 709

<210> 546

<211> 340

<212> DNA

<213> Homo sapiens

<400> 546
aaaaaagcag gcatggccca ttacaaagcc gccgactcga agcgtgagca gttccggagg 60

tacttggaga agtcgggggt gctggacacg ctgaccaagg tgttggtagc cttatatgaa 120

gaaccagaga aacctaacag tgctttggat tttttaaagc atcacttagg agctgctact 180

ccagaaaatc cagaaataga gctgcttcgc ctagaactgg ccgaaatgaa agagaagtat 240

gaagctattg tagaagaaaa taaaaaactg aaagcaaagc ttgctcagta tgaaccacct 300

caggaggaga agcgtgctga ttaattaacc cagctttctt 340

<210>  547

<211>  1061

<212>  DNA

<213>  Homo sapiens


<400>  547
aaaaaagcag gctccaccat ggcgaccggt ggctaccgga ccagcagcgg cctcggcggc      60

agcaccacag acttcctgga ggagtggaag gcgaaacgcg agaagatgcg cgccaagcag     120

aaccccccgg gcccggcccc cccgggaggg ggcagcagcg acgccgctgg gaagcccccc     180

gcgggggctc tgggcacccc ggcggccgcc gctgccaacg agctcaacaa caacctcccg     240

ggcggcgcgc gggccgcacc tgccgtcccc ggtcccgggg gcgtgaactg cgcggtcggc     300

tccgccatgc tgacgcgggc gcccccggcc cgcggcccgc ggcggtcgga ggacgagccc     360

ccagccgcct ctgcctcggc tgcaccgccg ccccagcgtg acgaggagga gccggacggc     420

gtcccagaga agggcaagag ctcgggcccc agtgccagga aaggcaaggg gcagatcgag     480

aagaggaagc tgcgggagaa gcggcgctcc accggcgtgg tcaacatccc tgccgcagag     540

tgcttagatg agtacgaaga tgatgaagca gggcagaaag agcggaaacg agaagatgca     600

attacacaac agaacactat gcagaatgaa gctgtaaact tactagatcc aggcagttcc     660

tatctgctac aggagccacc tagaacagtt tcaggcagat ataaaagcac aaccagtgtc     720

tctgaagaag atgtctcaag tagatattct cgaacagata gaagtgggtt ccctagatat     780

aacagggatg caaatgtttc aggtactctg gtttcaagta gcacactgga aaagaaaatt     840

gaagatcttg aaaaggaagt agtaagagaa agacaagaaa acctaagact tgtgagactg     900

atgcaagata aagaggaaat gattggaaaa ctcaaagaag aaattgattt attaaataga     960

gacctagatg acatagaaga tgaaaatgaa cagctaaagc aggaaaataa aactctttta    1020

aaagttgtgg gtcagctgac caggtaggac ccagctttct t                        1061

<210>  548

<211>  530

<212>  DNA

<213>  Homo sapiens


<400>  548
aaaaaagcag gcatggcggt ggcggacctc gctctcattc ctgatgtgga cgccgactcc      60

gacggcgtct tcaagtatgt gctgatccga atccactcgg ctccccgctc cggggctccg     120

gctgcagaga gcaaggagat cgtgcgcggc tacaagtggg ctgagtacca tgcggacatc     180

tacgacaaag tgtcgggcga catgcagaag caaggctgcg actgtgagtg tctgggcggc     240

gggcgcacct cccaccagag tcaggacaag aagattcacg tgtacggcta ttccatggcc     300

tatggtcctg cccagcacgc catttcaact gagaaaatca aagccaagta ccccgactac 360

gaggtcacct gggctaacga cggctactga gcactcccag cccggggcct gctgcctcca 420

gcagccactt cagagccccc gcctttgcct gcactcctct tgcagggctg gccctgtctg 480

ctcctgcggc agcctctggt gacgtgcgtc caccacgacc cagctttctt 530

<210> 549

<211> 825

<212> DNA

<213> Homo sapiens

<400> 549
aaaaaagcag gcatgcacag gctgtttact ccacagagtg gctttgagaa tcagatgaga 60

ctgtgctggc gaaggccctg tgggaatgag gaacgctgta gtgtttgctg gtccctgttt 120

ctgcccccag gaaagcagct gtgtgaggag gagcgccggg ccatgcaggc tgccctggac 180

tccgtcgtct gccacacgcc cctcaacaac cttggctttt cccggaaggg cagcgcgctc 240

accttcagtg tggccttcca ggctctgagg acggggctct tcgagctaag ccagcacatg 300

aaactgaagc tgcagttcac cgccagcgtg tcccaccctc cacccgaggc ccggcccctc 360

tcccgcaaga gcagccccag cagccctgct gtccgggact tggtggagag gcatcaggct 420

agcctgggcc gctcccagtc cttctcccac cagcagcctt cccgaagcca cctcatgagg 480

tcgggcagtg tgatggagcg cagagccatc acgccccctg tggcctctcc tgttggccgc 540

cccctctacc tgcccccgga caaggctgtg ttgtctctgg acaagattgc caagcgcgag 600

tgcaaggtcc tggtggtgga acccgtcaag tagcaccgtg ccagctctgt tccctcttac 660

actccagaga cccaacgccc ccagagggga tccctgctcc cgggctgtgc ctcccctggg 720

atgcctccca gacgggggtg aagaggcctg gcagagctgc ctgtcttgtg tctgctgatg 780

agggatgggg gaagaagctg tgaagtgggc ggacccagct ttctt 825

<210> 550

<211> 580

<212> DNA

<213> Homo sapiens

<400> 550
aaaaaagcag gcatgacgac cgcgtccacc tcgcaggtgc gccagaacta ccaccaggac 60

tcagaggccg ccatcaaccg ccagatcaac ctggagctct acgcctccta cgtttacctg 120

tccatgtctt actactttga ccgcgatgat gtggctttga agaactttgc caaatacttt 180

cttcaccaat ctcatgagga gagggaacat gctgagaaac tgatgaagct gcagaaccaa 240

cgaggtggcc gaatcttcct tcaggatatc aagaaaccag actgtgatga ctgggagagc 300
gggctgaatg caatggagtg tgcattacat ttggaaaaaa atgtgaatca gtcactactg 360
gaactgcaca aactggccac tgacaaaaat gacccccatt tgtgtgactt cattgagaca 420
cattacctga atgagcaggt gaaagccatc aaagaattgg gtgaccacgt gaccaacttg 480
cgcaagatgg gagtgcccga atctggcttg gcggaatatc tctttgacaa gcacaccctg 540
ggagacagtg ataatgaaag ttaattaacc cagctttctt 580

<210> 551
<211> 1981
<212> DNA
<213> Homo sapiens

<400> 551
aaaaaagcag gcatggcgga cgtgacggtg gaggttcgcg gctctaacgg ggctttctac 60
aagggattta tcaaagatgt tcatgaagac tcccttacag ttgtttttga aaataattgg 120
caaccagaac gccaggttcc atttaatgaa gttagattac caccaccacc tgatataaaa 180
aaagaaatta gtgaaggaga tgaagtagag gtatattcaa gagcaaatga ccaagagcca 240
tgtgggtggt ggttggctaa agttcggatg atgaaaggag aattttatgt cattgaatat 300
gctgcttgtg acgctactta caatgaaata gtcacatttg aacgacttcg gcctgtcaat 360
caaaataaaa ctgtcaaaaa aaataccttc tttaaatgca cagtggatgt tcctgaggat 420
ttgagagagg cgtgtgctaa tgaaaatgca cataaagatt ttaagaaagc agtaggagca 480
tgcagaattt tttaccatcc agaaacaaca cagctaatga tactgtctgc cagtgaagca 540
actgtgaaga gagtaaacat cttaagtgac atgcatttgc gaagtattcg tacgaagttg 600
atgcttatgt ccagaaatga agaggccact aagcatttag aatgcacaaa acaacttgca 660
gcagcttttc atgaggaatt tgttgtgaga gaagatttaa tgggcctggc aataggaaca 720
catggtagta acatccagca agctaggaag gttcctggag ttaccgccat tgagctagat 780
gaagatactg gaacattcag aatctacgga gagagtgctg atgctgtaaa aaaggctaga 840
ggtttcttgg aatttgtgga ggattttatt caggttccta ggaatctcgt tggaaaagta 900
attggaaaaa atggcaaagt tattcaagaa atagtggaca aatctggtgt ggttcgagtg 960
agaattgaag gggacaatga aaataaatta cccagagaag acggtatggt tccatttgta 1020
tttgttggca ctaaagaaag cattggaaat gtgcaggttc ttctagagta tcatattgcc 1080
tatctaaagg aagtagaaca gctaagaatg gaacgcctac agattgatga acagctgcga 1140
cagattggta tgggtttcag accttcttcc accagagggc ctgaaaaaga gaaaggatat 1200
gccactgatg aaagtaccgt ctcttctgta caaggttcta ggtcttatag cggaagaggc 1260
agaggtcgtc ggggacctaa ttacacctcc ggttatggta caaattctga gctgtctaac 1320
ccctctgaaa cggaatctga gcgtaaagac gagctgagtg attggtcatt ggcaggagaa 1380

gatgatcgag acagccgaca tcagcgtgac agcaggagac gcccaggagg aagaggcaga 1440
agtgtttcag ggggtcgagg tcgtggtgga ccacgtggtg gcaaatcctc catcagttct 1500
gtgctcaaag atccagacag caatccatac agcttacttg ataatacaga atcagatcag 1560
actgcagaca ctgatgccag cgaatctcat cacagtacta accgtcgtag gcggtctcgt 1620
agacgaagga ctgatgaaga tgctgttctg atggatggaa tgactgaatc tgatacagct 1680
tcagttaatg aaaatgggct agtcacagtt gcagattata tttctagagc tgagtctcag 1740
agcagacaaa gaaacctccc aagggaaact ttggctaaaa acaagaaaga aatggcaaaa 1800
gatgtgattg aagagcatgg tccttcagaa aaggcaataa acggcccaac tagtgcttct 1860
ggcgatgaca tttctaagct acagcgtact ccaggagaag aaaagattaa taccttaaaa 1920
gaagaaaaca ctcaagaagc agcagtcctg aatggtgttt cttaattaac ccagctttct 1980
t 1981

<210> 552
<211> 542
<212> DNA
<213> Homo sapiens

<400> 552
aaaaaagctg gcacccctgg tccaggtatg gacatccccc agaccaagca ggacctggag 60
ctcccaaagt tggcagggac ctggcactcc atggccatgg cgaccaacaa catctccctc 120
atggcgacac tgaaggcccc tctgagggtc cacatcacct cactgttgcc cacccccgag 180
gacaacctgg agatcgttct gcacagatgg gagaacaaca gctgtgttga gaagaaggtc 240
cttggagaga agactgagaa tccaaagaag ttcaagatca actatacggt ggcgaacgag 300
gccacgctgc tcgatactga ctacgacaat ttcctgtttc tctgcctaca ggacaccacc 360
acccccatcc agagcatgat gtgccagtac ctggccagag tcctggtgga ggacgatgag 420
atcatgcagg gattcatcag ggctttcagg cccctgccca ggcacctatg gtacttgctg 480
gacttgaaac agatggaaga gccgtgccgt ttcccaggcc caggcggcga cccagctttc 540
tt 542

<210> 553
<211> 958
<212> DNA
<213> Homo sapiens

<400> 553
aaaaaagcag gcatggacga gaaggtgttc accaaggagc tggaccagtg gatcgagcag 60

ctgaacgagt gcaagcagct gtccgagtcc caggtcaaga gcctctgcga gaaggctaaa 120
gaaatcctga caaaagaatc caacgtgcaa gaggttcgat gtccagttac tgtctgtgga 180
gatgtgcatg ggcaatttca tgatctcatg gaactgttta gaattggtgg caaatcacca 240
gatacaaatt acttgtttat gggagattat gttgacagag gatattattc agttgaaaca 300
gttacactgc ttgtagctct taaggttcgt taccgtgaac gcatcaccat tcttcgaggg 360
aatcatgaga gcaaacagat cacacaagtt tatggtttct atgatgaatg tttaagaaaa 420
tatggaaatg caaatgtttg gaaatatttt acagatcttt ttgactatct tcctctcact 480
gccttggtgg atgggcagat cttctgtcta catggtggtc tctcgccatc tatagataca 540
ctggatcata tcagagcact tgatcgccca caagaagttc cccatgaggg tccaatgtgt 600
gacttgctgt ggtcagatcc agatgaccgt ggtggttggg gtatatctcc tcgaggagct 660
ggttacacct ttgggcaaga tatttctgag acatttaatc atgccaatgg cctcacgttg 720
gtgtctagag ctcaccagct agtgatggag ggatataact ggtgccatga ccggaatgta 780
gtaacgattt tcagtgctcc aaactattgt tatcgttgtg gtaaccaagc tgcaatcatg 840
gaacttgacg atactctaaa atactctttc ttgcagtttg acccagcacc tcgtagaggc 900
gagccacatg ttactcgtcg taccccagac tacttccttt aattaaccca gctttctt 958

<210> 554
<211> 720
<212> DNA
<213> Homo sapiens

<400> 554
aaaaaagcag gcatgaagct catcgtgggc atcggaggca tgaccaacgg cggcaagacc 60
acgctgacca acagcctgct cagagccctg cccaactgct gcgtgatcca tcaggatgac 120
ttcttcaagc cccaagacca aatagcagtt ggggaagacg gcttcaaaca gtgggacgtg 180
ctggagtctc tggacatgga ggccatgctg gacaccgtgc aggcctggct gagcagcccg 240
cagaagtttg cccgtgccca cggggtcagc gtccagccag aggcctcgga cacccacatc 300
ctcctcctgg aaggcttcct gctctacagc tacaagcccc tggtggactt gtacagccgc 360
cggtacttcc tgaccgtccc gtatgaagag tgcaagtgga ggagaagtac ccgcaactac 420
acagtccctg atcccccggg cctcttcgat ggccacgtgt ggcccatgca ccagaagtat 480
aggcaggaga tggaggccaa cggtgtggaa gtggtctacc tggacggcat gaagtcccga 540
gaggagctct tccgtgaagt cctggaagac attcagaact cgctgctgaa ccgctcccag 600
gaatcagccc cctccccggc tcgcccagcc aggacacagg gacccggacg cggatgcggc 660
cacagaacgg ccaggcctgc agcgtcccag caggacagca ttaattaacc cagctttctt 720

<210> 555

<211>  571

<212>  DNA

<213>  Homo sapiens


<400>  555
aaaaaagcag gcatggactg gcctcacaac ctgctgtttc ttcttaccat ttccatcttc      60

ctggggctgg gccagcccag gagccccaaa agcaagagga aggggcaagg gcggcctggg     120

cccctggtcc ctggccctca ccaggtgcca ctggacctgg tgtcacggat gaaaccgtat     180

gcccgcatgg aggagtatga gaggaacatc gaggagatgg tggcccagct gaggaacagc     240

tcagagctgg cccagagaaa gtgtgaggtc aacttgcagc tgtggatgtc caacaagagg     300

agcctgtctc cctggggcta cagcatcaac cacgacccca gccgtatccc cgtggacctg     360

ccggaggcac ggtgcctgtg tctgggctgt gtgaacccct tcaccatgca ggaggaccgc     420

agcatggtga gcgtgccggt gttcagccag gttcctgtgc gccgccgcct ctgcccgcca     480

ccgccccgca cagggccttg ccgccagcgc gcagtcatgg agaccatcgc tgtgggctgc     540

acctgcatct tttaattaac ccagctttct t                                    571

<210>  556

<211>  1141

<212>  DNA

<213>  Homo sapiens


<400>  556
aaaaaagcag gcatggagaa catggatgat acctcaggct ctagcttcga ggatatgggt      60

gagctgcatc agcgcctgcg cgaggaagaa gtagacgctg atgcagctga tgcagctgct     120

gctgaagagg aggatggaga gttcctgggc atgaagggct ttaagggaca gctgagccgg     180

caggtggcag atcagatgtg gcaggctggg aaaggacaag cctccagggc cttcagcttg     240

tacgccaaca tcgacatcct cagaccctac tttgatgtgg agcctgctca ggtgcgaagc     300

aggctcctgg agtccatgat ccctatcaag atggtcaact tcccccagaa aattgcaggt     360

gaactctatg gacctctcat gctggtcttc actctggttg ctatcctact ccatgggatg     420

aagacgtctg acactattat ccgggagggc accctgatgg gcacagccat tggcacctgc     480

ttcggctact ggctgggagt ctcatccttc atttacttcc ttgcctacct gtgcaacgcc     540

cagatcacca tgctgcagat gttggcactg ctgggctatg gcctctttgg gcattgcatt     600

gtcctgttca tcacctataa tatccacctc cacgccctct tctacctctt ctggctgttg     660

gtgggtggac tgtccacact gcgcatggta gcagtgttgg tgtctcggac cgtgggcccc     720

acacagcgac tgctcctctg tggcaccctg gctgccctac acatgctctt cctgctctat     780

ctgcattttg cctaccacaa agtggtagag gggatcctgg tcacactgga gggccccaac      840

atcccgccca tccagagggt ccccagagac atccctgcca tgctccctgc tgctcggctt 900

cccaccaccg tcctcaacgc cacagccaaa gctgttgcgg tgaccctgca gtcacactga 960

ccccacctga aattcttggc cagtcctctt tcccgcagct gcagagagga ggaagactat 1020

taaaggacag tcctgatgac atgtttcgta gatggggttt gcagctgcca ctgagctgta 1080

gctgcgtaag tacctccttg atgcctgtcg gcacttctga aaggcacaac ccagctttct 1140

t 1141

<210> 557

<211> 415

<212> DNA

<213> Homo sapiens

<400> 557
aaaaaagcag gcatggcggg gagcggcttc cgccaagcta cttctaccgc cagcaccttc 60

gtgaagccca ttttcagtcg ggacatgaac gaggccaagc ggagggtgcg cgagctctac 120

cgcgcctggt atcgggaggt gccgaacact gtgcaccaat tccagctgga catcactgtg 180

aaaatgggac gggataaagt ccgagaaatg tttatgaaga atgcccatgt cacagacccc 240

agggtggttg atcttctggt cattaaggga aagatcgaac tggaagaaac aattaaagta 300

tggaagcagc ggacacatgt tatgcggttc ttccatgaaa cagaagcgcc aaggccaaag 360

gatttcctat ccaagttcta tgttggccac gatccttaat taacccagct ttctt 415

<210> 558

<211> 1467

<212> DNA

<213> Homo sapiens

<400> 558
aaaaaagcag gcatgtccaa aaaaatcagt ggcggttctg tggtagagat gcaaggagat 60

gaaatgacac gaatcatttg ggaattgatt aaagagaaac tcattattcc ctacgtggaa 120

ttggatctac atagctatga tttaggcata gagaatcgtg atgccaccaa cgaccaagtc 180

accaaggatg ctgcagaagc tataaagaag cataatgttg gcgtcaaatg tgccactatc 240

actcctgatg agaagagggt tgaggagttc aagttgaaac aaatgtggaa atcaccaaat 300

ggcaccatac gaaatattct gggtggcacg gtcttcagag aagccattat ctgcaaaaat 360

atcccccggc ttgtgagtgg atgggtagag cctatcatca taggtcgtca tgcttatggg 420

gatcaataca gagcaactga ttttgttgtt cctgggcctg gaaaagtaga gataacctac 480

acaccaagtg acggaaccca aaaggtgaca tacctggtac ataactttga agaaggtggt 540

ggtgttgcca tggggatgta taatcaagat aagtcaattg aagattttgc acacagttcc 600

ttccaaatgg ctctgtctaa gggttggcct ttgtatctga gcaccaaaaa cactattctg 660
aagaaatatg atgggcgttt taaagacatc tttcaggaga tatatgacaa gcagtacaag 720
tcccagtttg aagctcaaaa gatctggtat gagcataggc tcatcgacga catggtggcc 780
caagctatga aatcagaggg aggcttcatc tgggcctgta aaaactatga tggtgacgtg 840
cagtcggact ctgtggccca agggtatggc tctctcggca tgatgaccag cgtgctggtt 900
tgtccagatg gcaagacagt agaagcagag gctgcccacg ggactgtaac ccgtcactac 960
cgcatgtacc agaaaggaca ggagacgtcc accaatccca ttgcttccat ttttgcctgg 1020
accagagggt tagcccacag agcaaagctt gataacaata aagagcttgc cttctttgca 1080
aatgctttgg aagaagtctc tattgagaca attgaggctg gcttcatgac caagggcttg 1140
gctgcttgca ttaaaggttt acccaatgtg caacgttctg actacttgaa tacatttgag 1200
ttcatggata aacttggaga aaacttgaag atcaaactag ctcaggccaa actttaagtt 1260
catacctgag ctaagaagga taattgtctt ttggtaacta ggtctacagg tttgcatttt 1320
tctgtgttac actcaaggat aaaggcaaaa tcaattttgt aatttgttta gaagccagag 1380
tttatctttt ctataagttt acagcctttt tcttatatat acagttattg ccacctttgt 1440
gaacatggca agggacccag ctttctt 1467

<210> 559
<211> 164
<212> DNA
<213> Homo sapiens

<400> 559
aaaaaagctg gcacccctgg tccaggtatg aaagccttca gtcccgtgag gtgcgttagg 60
aaaaacagcc tgttggacca cagcctgggc atctcccgga gcaaaacccc tgtggacgac 120
ctgatgagcc tgctgccagg cccaggcggc gacccagctt tctt 164

<210> 560
<211> 385
<212> DNA
<213> Homo sapiens

<400> 560
aaaaaagcag gcatgcgcgg acggaagatg gcgtccgcca cccgtctcat ccagcggctg 60
cggaactggg cgtccgggca tgacctgcag gggaagctgc agctacgcta ccaggagatc 120
tccaagcgaa ctcagcctcc tcccaagctc cctgtgggtc ctagccacaa gctctccaac 180
aattactatt gcactcgcga tggccgccgg gaatctgtgc ccccttccat catcatgtcg 240

tcgcagaagg cgctggtgtc aggcaagcca gcagagagct ctgctgtagc tgccactgag 300

aagaaggcgg tgactccagc tcctcctata aagaggtggg agctgtcctg ggaccagcct 360

tacctttaat taacccagct ttgtt 385

<210> 561

<211> 1078

<212> DNA

<213> Homo sapiens

<400> 561
aaaaaagcag gcatgaaccg gaaagcgctg cgctgcctgg gccacctctt tctcagcctg 60

ggcatggtct acctccggat cggtggcttc tcctcagtgg tagctctggg cgcaagcatc 120

atctgtaaca agatcccagg cctggctccc agacagcggg cgatctgcca gagccggccc 180

gacgccatca tcgtcatagg agaaggctca caaatgggcc tggacgagtg tcagtttcag 240

ttccgcaatg gccgctggaa ctgctctgca ctgggagagc gcaccgtctt cgggaaggag 300

ctcaaagtgg ggagccggga ggctgcgttc acctacgcca tcattgccgc cggcgtggcc 360

cacgccatca cagctgcctg tacccagggc aacctgagcg actgtggctg cgacaaagag 420

aagcaaggcc agtaccaccg ggacgagggc tggaagtggg gtggctgctc tgccgacatc 480

cgctacggca tcggcttcgc caaggtcttt gtggatgccc gggagatcaa gcagaatgcc 540

cggactctca tgaacttgca caacaacgag gcaggccgaa agatcctgga ggagaacatg 600

aagctggaat gtaagtgcca cggcgtgtca ggctcgtgca ccaccaagac gtgctggacc 660

acactgccac agtttcggga gctgggctac gtgctcaagg acaagtacaa cgaggccgtt 720

cacgtggagc ctgtgcgtgc cagccgcaac aagcggccca ccttcctgaa gatcaagaag 780

ccactgtcgt accgcaagcc catggacacg gacctggtgt acatcgagaa gtcgcccaac 840

tactgcgagg aggacccggt gaccggcagt gtgggcaccc agggccgcgc ctgcaacaag 900

acggctcccc aggccagcgg ctgtgacctc atgcgctgtg ggcgtggcta caacacccac 960

cagtacgccc gcgtgtggca gtgcaactgt aagttccact ggtgctgcta tgtcaagtgc 1020

aacacgtgca gcgagcgcac ggagatgtac acgtgcaatt aattaaccca gctttctt 1078

<210> 562

<211> 466

<212> DNA

<213> Homo sapiens

<400> 562
aaaaaagcag gcatggtggg tcggaacagc gccatcgccg ccggtgtatg cggggccctt 60

ttcattgggt actgcatcta cttcgaccgc aaaagacgaa gtgaccccaa cttcaagaac 120

aggcttcgag aacgaagaaa gaaacagaag cttgccaagg agagagctgg gctttccaag 180

ttacctgacc ttaaagatgc tgaagctgtt cagaagttct tccttgaaga aatacagctt 240

ggtgaagagt tactagctca aggtgaatat gagaagggcg tagaccatct gacaaatgca 300

attgctgtgt gtggacagcc acagcagtta ctgcaggtct tacagcaaac tcttccacca 360

ccagtgttcc agatgcttct gactaagctc ccaacaatta gtcagagaat tgtaagtgct 420

cagagcttgg ctgaagatga tgtggattaa ttaacccagc tttctt 466

<210> 563

<211> 1582

<212> DNA

<213> Homo sapiens

<220>

<221> misc_feature

<222> (803)..(803)

<223> n = a, c, g or t

<400> 563
aaaaaagcag gcatgttgcg cgctgccgcc cgcttcgggc cccgcctggg ccgccgcctc 60

ttgtcagccg ccgccaccca ggccgtgcct gcccccaacc agcagcccga ggtcttctgc 120

aaccagattt tcataaacaa tgaatggcac gatgccgtca gcaggaaaac attccccacc 180

gtcaatccgt tcactggaga ggtcatctgt caggtagctg aaggggacaa ggaagatgtg 240

gacaaggcag tgaaggccgc ccgggccgcc ttccagctgg gctcaccttg gcgccgcatg 300

gacgcatcac acaggggccg gctgctgaac cgcctggccg atctgatcga gcgggaccgg 360

acctacctgg cggccttgga gaccctggac aatggcaagc cctatgtcat ctcctacctg 420

gtggatttgg acatggtcct caaatgtctc cggtattatg ccggctgggc tgataagtac 480

cacgggaaaa ccatccccat tgacggagac ttcttcagct acacacgcca tgaacctgtg 540

ggggtgtgcg ggcagatcat tccgtggaat ttcccgctcc tgatgcaagc atggaagctg 600

ggcccagcct tggcaactgg aaacgtggtt gtgatgaagg tagctgagca gacacccctc 660

accgccctct atgtggccaa cctgatcaag gaggctggct ttccccctgg tgtggtcaac 720

attgtgcctg gatttggccc cacggctggg gccgccattg cctcccatga ggatgtggac 780

aaagtggcat tcacaggctc cantgagatt ggccgcgtaa tccaggttgc tgctgggagc 840

agcaacctca agagagtgac cttggagctg ggggggaaaa gccccaacat catcatgtca 900

gatgccgata tggattgggc cgtggaacag gcccacttcg ccctgttctt caaccagggc 960

cagtgctgct gtgccggctc ccggaccttc gtgcaggagg acatctatga tgagtttgtg 1020

gagcggagcg ttgcccgggc caagtctcgg gtggtcggga acccctttga tagcaagacc 1080
gagcaggggc cgcagttgga tgaaactcag tttaagaaga tcctcggcta catcaacacg 1140
gggaagcaag agggggcgaa gctgctgtgt ggtgggggca ttgctgctga ccgtggttac 1200
ttcatccagc ccactgtgtt tggagatgtg caggatggca tgaccatcgc caaggaggag 1260
atcttcgggc cagtgatgca gatcctgaag ttcaagacca tagaggaggt tgttgggaga 1320
gccaacaatt ccacgtacgg gctggccgca gctgtcttca caaaggattt ggacaaggcc 1380
aattacctgt cccaggccct ccaggcgggc actgtgtggg tcaactgcta tgatgtgttt 1440
ggagcccagt caccctttgg tggctacaag atgtcgggga gtggccggga gttgggcgag 1500
tacgggctgc aggcatacac tgaagtgaaa actgtcacag tcaaagtgcc tcagaagaac 1560
tcttaattaa cccagctttc tt 1582

<210> 564

<211> 841

<212> DNA

<213> Homo sapiens

<400> 564
aaaaaagcag gcatgttgag gaccgagctt cgggattacc gaggcttaga acagctgaag 60
gcccagctgg tggctgggga tgatgaggag gctggttgga ctgagctggc ggagcaggaa 120
ttgtttcgcc tgctgaggac tgagttgata ggagaccagc tagcccagct caacaccgtt 180
ttccaagcac cccctgatgt gtggaagaaa gtgttagggg gaataaccgc tggaaaggaa 240
ccccccaatg gaatactgcc cccctttgaa ctcctgtgcc agtgtctgtg ccagctggag 300
cctcgatggc tgccaccctt tgtggagctg gcacagcagc agggcgggcc gggctggggg 360
gcagggggcc caggactgcc cctgtatcgc cgagctctgg cagtgctagg tgaggagggg 420
accaggcctg aggctctgga gctagagctg ctcttgagca gtgggcggcc taaagctgtg 480
ctccaagctg tcgggcagct ggtgcaaaag gaacaatggg atcgggctct ggatgctggc 540
ctggccctcg gcccctccag tcccctcctt tgccgcctgt gcccaccaga actggctcca 600
gctgagctcc tgcttctact gaggacatac ctcccagatg aggtggggcc cccaacccca 660
ttccctgagc ctggagcaga gccccctctc actgtgggct tgctcaaagc cctgctggag 720
cagactgggg ctcaaggatg gctgtcgggc ccagttctaa gcccatatga ggacatccta 780
tgggacccca gcactccacc cccgactcca cctcgggacc tttaattaac ccagctttct 840
t 841

<210> 565

<211> 814

<212> DNA

<213>  Homo sapiens

<400>  565
aaaaaagcag gcatgtctga cgaggaagtt gaacaggtgg aggagcagta cgaagaagaa      60
gaggaagccc aggaggaagc tgcagaagtc catgaggaag ttcatgaacc agaggaagtt     120
caagaagagg agaaaccgag acccaaactc actgctccta agatcccaga aggggagaaa     180
gtggacttcg atgacatcca gaagaagcgt cagaacaaag acctaatgga gctccaggcc     240
ctcatcgaca gccactttga agcccggaag aaggaggagg aggagctggt cgctctcaaa     300
gagagaatcg agaagcgccg tgcagagaga gcggagcagc agaggattcg tgcagagaag     360
gagagggagc gccagaacag actggcggag gaaaaggcca gaagggagga ggaggatgcc     420
aagaggaggg cagaggacga cctgaagaag aagaaagctc tgtcttccat gggagccaac     480
tacagcagct acctggccaa ggctgaccag aagagaggca agaagcagac agcccgggaa     540
atgaagaaga agattctggc tgagagacgc aagccgctca acatcgatca ccttggtgaa     600
gacaaactga gggacaaggc caaggagctc tgggagaccc tgcaccagct ggagattgac     660
aagttcgagt ttggggagaa gctgaaacgc cagaaatatg acatcaccac gctcaggagc     720
cgcattgacc aggcccagaa gcacagcaag aaggctggga ccccagccaa gggcaaagtc     780
ggcgggcgct ggaattaatt aacccagctt tctt                                 814

<210>  566
<211>  1133
<212>  DNA
<213>  Homo sapiens

<400>  566
aaaaaagcag gcatgaccac agccctggaa cctgaggacc aaaaaggact tctgataatt      60
aaggcagagg accattactg gggacaggat tccagctcac aaaagtgcag tcctcacagg     120
agggaactct atagacaaca cttcaggaag ctctgctatc aggatgcacc tggaccccgt     180
gaagctctta cccagctgtg ggagctctgc cgtcagtggc tgaggccaga atgccacacc     240
aaggagcaga ttttagacct gctggtgcta gaacagttcc tgagcattct tcctaaagac     300
ctgcaagcat gggtgcgtgc acaccatcca gagactggag aggaggcagt gacggtactg     360
gaggatctgg agagagagtt tgatgaacct ggaaagcagg tcccaggcaa ttcagaaaga     420
cgggacatac tcatggacaa gttggccccc ttgggaaggc catatgaatc actgactgtc     480
cagctccatc ccaaaaagac ccagctggag caggaagctg ggaaaccaca aaggaatggc     540
tgtttgcttc tcaaacatag aataggaccc gtttgtgtac tgtttgtttg ttacaggtga     600
taaaactagg actaagaatg aagagttgtt ccagaaggaa gatgtgccca aagacaagga     660
attccttggg gagataaatg acagactgaa caaagatact cctcagcatc ctaagtccaa     720

agatattatt gaaaatgagg gcagatcaga atggcaacag agggaaagaa gacgatataa 780

atgtgatgaa tgtgggaaaa gtttcagtca tagctcagac cttagtaaac acaggagaac 840

tcacacggga gagaagccct ataaatgtga tgagtgtgga aaagccttca ttcagcgctc 900

acatctcatt ggacatcata gagtacacac gggagtaaaa ccctataaat gtaaagaatg 960

tgggaaagac ttcagtgggc gcacaggtct tattcagcat cagagaatcc acacaggtga 1020

aaaaccctat gaatgtgatg agtgtggaag gcctttccga gtaagttcag ctcttattag 1080

acatcaaaga attcataccg caaataaact ctattaatta acccagcttt ctt 1133

<210> 567

<211> 1111

<212> DNA

<213> Homo sapiens

<400> 567
aaaaaaatgg agtgaccaga acattgaata tgagtaccaa aaccccagaa gaagcttcag 60

gagtctcata gaagagaaag tcaatgaaat taaagaagac agtcattgtg gagaaacttt 120

tacccaggtt ccagatgaca gactgaactt ccaggagaag aaagcttctc ctgaagtaaa 180

atcatgtgac agctttgtgt gtgcagaagt tggcataggt aactcatctt ttaatatgag 240

catcagaggt gacactggac acaaggcata tgagtatcag gaatatggac caaagccata 300

taagtgtcaa caacctaaaa ataagaaagc cttcaggtat cgcccatcca ttagaacaca 360

agaaagggat cacactggag agaaacccta tgcttgtaaa gtctgtggaa aaacctttat 420

tttccattca agcattcgaa gacacatggt aatgcacagt ggggatggaa cttataaatg 480

taaattttat gggaaagcct tccattcttt cagtttatat cttatccatg aaagaactca 540

cactggagag aaaccatatg aatgtaaaca atgtggtaaa tcctttactt attctgctac 600

cctccaaata catgaaagaa ctcacactgg ggagaagccc tatgaatgta gcaaatgtga 660

taaagcattt catagttcta gttcctatca tagacatgaa agaagtcaca tgggagagaa 720

gccttatcaa tgcaaagaat gtggaaaagc atttgcatat accagttctc ttcgtagaca 780

tgaaaggacc cactctggga aaaaaccgta tgaatgtaag caatatgggg aaggcttatc 840

ctatcttata agttttcaaa cacacataag aatgaactct ggagaaagac cttataaatg 900

taagatatgt gggaaaggct tttattctgc caagtcattt caaacacatg aaaaaactca 960

cactggagag aaacgctata aatgcaagca atgtggtaaa gccttcaatc tttccagttc 1020

ctttcgatac catgaaagga ttcacactgg agagaaaccc tatgagtgta agcagtgtgg 1080

gaaagccttt aacgagccgg aacccagctt t 1111

<210> 568

<211> 1045

<212>  DNA

<213>  Homo sapiens


<400>  568
aaaaaagcag gcatggggaa aaaacagaac agaaaaactg gaaactctaa aacgcagagc      60

acctcccctc ctccaaagga acgcagttcc tcaccagcaa cggaacaaag ctggatggag     120

aatgattttg acgagctgag agaagaaggc ttcagacgat caaattactc tgagctacgg     180

gaggacattc aaaccaaagg caaagaagtt gaaaactttg aaaaaaattt agaagaatgt     240

ataactagaa taaccaatac agagaagtgc ttaaaggagc tgatggagct gaaaaccaag     300

gctcgagaac tacgtgaaga atgcagaagc ctcaggagcc aatgcgatca actggaagaa     360

agggtatcag caatggaaga tgaaatgaat gaaatgaagc aagaagggaa gtttagagaa     420

aaaagaataa aaagaaacga gcaaagcctc caagaaatat gggactatgt gaaaagacca     480

aatctacgtc tgattggtgt acctgaaagt gatgtggaga atggaaccaa gttggaaaac     540

actctgcagg atattatcca ggagactttc cccaatctag caaggcaggc caacgttcag     600

attcaggaaa tacagagaac gccacaaaga tactcctcga gaagagcaac tccaagacac     660

ataattgtca gattcaccaa agttgaaatg aaggaaaaaa tgttaagggc agccagagag     720

aaaggtcgag ttaccctcaa aggaaagccc atcagactaa cagcggatct ctcggcagaa     780

accctacaag ccagaagaga gtgggggcca atattcaaca ttcttaaaga aaagaatttt     840

caacccagaa tttcatatcc agccaaacta agcttcataa gtgaaggaga aataaaatac     900

tttacagaca agcaaatgct gacagatttt gtcaccacca ggcctgccct aaaagagctc     960

ctgaaggaag cgctaaacat ggaaaggaac aaccggtacc agccactgca aaatcatgcc    1020

aaaatgtaat taacccagct ttctt                                          1045

<210>  569

<211>  899

<212>  DNA

<213>  Homo sapiens


<400>  569
aaaaaagctg gcacccctgg tccaggtatg gctatgatgg aggtccaggg gggacccagc      60

ctgggacaga cctgcgtgct gatcgtgatc ttcacagtgc tcctgcagtc tctctgtgtg     120

gctgtaactt acgtgtactt taccaacgag ctgaagcaga tgcaggacaa gtactccaaa     180

agtggcattg cttgtttctt aaaagaagat gacagttatt gggaccccaa tgacgaagag     240

agtatgaaca gcccctgctg gcaagtcaag tggcaactcc gtcagctcgt tagaaagatg     300

attttgagaa cctctgagga aaccatttct acagttcaag aaaagcaaca aaatatttct     360

cccctagtga gagaaagagg tcctcagaga gtagcagctc acataactgg gaccagagga     420

agaagcaaca cattgtcttc tccaaactcc aagaatgaaa aggctctggg ccgcaaaata 480
aactcctggg aatcatcaag gagtgggcat tcattcctga gcaacttgca cttgaggaat 540
ggtgaactgg tcatccatga aaaagggttt tactacatct attcccaaac atactttcga 600
tttcaggagg aaataaaaga aaacacaaag agcgacaaac gaatggtcca agatatttac 660
aaatacacaa gttatcctga ccctatattg ttgatgaaaa gtgctagaaa tagttgttgg 720
tctaaagatg cagaatatgg actctattcc atctatcaag gggaatattt tgagcttaag 780
gaaaatgaca gaaaaaatgt ttctgtaaca aatgagcact tgatagacat ggaccatgaa 840
gccagttttt ttggggcctt tttagttggc ccaggcccag gcggcgaccc agctttctt 899

<210> 570
<211> 952
<212> DNA
<213> Homo sapiens

<400> 570
aaaaaagcag gcatgctgct gcttctgctg cttctggggc caggctccgg gcttggtgct 60
gtcgtctctc aacatccgag cagggttatc tgtaagagtg gaacctctgt gaagatcgag 120
tgccgttccc tggactttca ggccacaact atgttttggt atcgtcagtt cccgaaacag 180
agtctcatgc tgatggcaac ttccaatgag ggctccaagg ccacatacga gcaaggcgtc 240
gagaaggaca agtttctcat caaccatgca agcctgacct tgtccactct gacagtgacc 300
agtgcccatc ctgaagacag cagcttctac atctgcagtg ctaggacagg ggcagatacg 360
cagtattttg gcccaggcac ccggctgaca gtgctcgagg acctgaaaaa cgtgttccca 420
cccgaggtcg ctgtgtttga gccatcagaa gcagagatct cccacaccca aaaggccaca 480
ctggtgtgcc tggccacagg cttctacccc gaccacgtgg agctgagctg gtgggtgaat 540
gggaaggagg tgcacagtgg ggtcagcaca gacccgcagc ccctcaagga gcagcccgcc 600
ctcaatgact ccagatactg cctgagcagc cgcctgaggg tctcggccac cttctggcag 660
aacccccgca accacttccg ctgtcaagtc cagttctacg ggctctcgga gaatgacgag 720
tggacccagg atagggccaa acctgtcacc cagatcgtca gcgccgaggc ctggggtaga 780
gcagactgtg gcttcacctc cgagtcttac cagcaagggg tcctgtctgc caccatcctc 840
tatgagatct tgctagggaa ggccaccttg tatgccgtgc tggtcagtgc cctcgtgctg 900
atggccatgg tcaagagaaa ggattccaga ggttaattaa cccagctttc tt 952

<210> 571
<211> 586
<212> DNA
<213> Homo sapiens

<400>  571
aaaaaagcag gcatggcact gcaacaggcc cagatgttgc tgagcatgaa cagcctggag      60

gcggtgaatg cgggcgtgca gcagaacaac acagagtcct ttgctgtcgc actctgccac     120

ctcgcagagc tacacgcgga gcagggctgt tttgctgcag cttctgaagt gttaaagcac     180

ttgaaggaac gatttccgcc taatagtcag cacgcccagt tatggatgct atgtgatcaa     240

aaaatacagt ttgacagagc aatgaatgat ggcaaatatc atttggctga ttcacttgtt     300

acaggaatca cagctctcaa tagcatagag ggtgtttata ggaaagcggt tgtattacaa     360

gctcagaacc aaatgtcaga ggcacataag cttttacaaa aattgttggt tcattgtcag     420

aaactgaaga acacagaaat ggtgatcagt gtcctactgt ccgtggcaga gctgtactgg     480

cgatcttcct cccctaccat cgcgctgccc atgctcctgc aggctctggc cctctccaag     540

gagtaccggt tacattattt gggcccttaa ttaacccagc tttctt                    586

<210>  572

<211>  735

<212>  DNA

<213>  Homo sapiens


<400>  572
aaaaaagcag gcatgcgcct gaagctgttc tccatcctgt ccaccgtgct gctcagagcc      60

acggacacca tcaactccca ggggcagttt cccagctacc tcgagacggt gacaaaggac     120

atcctggccc ccaatctcag tggcatgcgg ggaggacagc cgcggccatc cgcacggctg     180

ccgtgtcctg cctctgggcg ctcaccagca gcgaggtcct gtcggcagag cagatacggg     240

acgtgcagga aacactgatg ccccaggtcc tgaccaccct ggaggaggat tcgaagatga     300

cgcgactgat ctcatgccgt attatcaaca cgttcttaaa aacctcgggc ggcatgacgg     360

atccagagaa actcatcagg atttatcctg aactcttaaa acgcctagat gacgtgtcca     420

acgatgtgag gatggcagcc gcctccacct tggtcacctg gctgcagtgt gtcaagggtg     480

ccaacgcaaa atcctactat cagagcagtg tccagtacct gtaccgagag ttgctggttc     540

accttgacga tccatagagg gccatccagg atgcaatttt agaggtcctc aaagagggca     600

gcgggctgtt cccagatctc ctggtgaggg agacggaggc cgtcatccac aagcaccgct     660

cggccaccta ctgcgagcag ctcctgcagc atgtgcaggc cgtgccagcc acacattaat     720

taacccagct ttctt                                                      735

<210>  573

<211>  1070

<212>  DNA

<213>  Homo sapiens

<400> 573
aaaaaagctg gcacccctgg tccaggtatg gcaccgtccc tcacccagga gatcctcagc 60

cgcctgggcc tggccagcaa gactgcagcg tgggggaccc tgggcaccct caggaccttc 120

ttgaacttca gcgtggacaa ggatgcgcag aggctactga gggccattac tggccaaggc 180

gtggaccgca gtgccattgt ggacgtgctg accaaccgga gcagagagca aaggcagctc 240

atctcacgaa acttccagga gcgcacccaa caggacctga tgaagtctct acaggcagca 300

ctttccggca acctggagag gattgtgatg gctctgctgc agcccacagc ccagtttgac 360

gcccaggaat tgaggacagc tctgaaggcc tcagattctg ctgtggacgt ggccattgaa 420

attcttgcca ctcgaacccc accccagctg cagtagtgcc tggcagtcta caaacacaat 480

ttccaggtgg aggctgtgga tggcatcaca tctgagacca gtggcatctt gcaggacctg 540

ctgttggccc tggccaaggg gggccgtgac agctactctg gaatcattga ctataatctg 600

gcagaacaag atgtccaggc actgcagcgg gcagaaggac ctagcagaga ggaaacatgg 660

gtcccagtct tcacccagcg aaatcctgaa cacctcatcc gagtgtttga tcagtaccag 720

cggagcactg ggcaagagct ggaggaggct gtccagaacc gtttccatgg agatgctcag 780

gtggctctgc tcggcctagc ttcggtgatc aagaacacac cgctgtactt tgctgacaaa 840

cttcatcaag ccctccagga aactgagccc aattaccaag tcctgattcg catccttatc 900

tctcgatgtg agactgacct tctgagtatc agagctgagt tcaggaagaa atttgggaag 960

tccctctact cttctctcca ggatgcagtg aaaggggatt gccagtcagc cctcctggcc 1020

ttgtgcaggg ctgaagacat gccaggccca ggcggcgacc cagctttctt 1070

<210> 574

<211> 1050

<212> DNA

<213> Homo sapiens

<400> 574
aaaaaagcag gctccaccat ggcaccgtcc ctcacccagg agatcctcag ccacctgggc 60

ctggccagca agactgcagc gtgggggacc ctgggcaccc tcaggacctt cttgaacttc 120

agcgtggaca aggatgcgca gaggctactg agggccatta ctggccaagg cgtggaccgc 180

agtgccattg tggacgtgct gaccaaccgg agcagagagc aaaggcagct catctcacga 240

aacttccagg agcgcaccca acaggacctg atgaagtctc tacaggcagc actttccggc 300

aacctggaga ggattgtgat ggctctgctg cagcccacag cccagtttga cgcccaggaa 360

ttgaggacag ctctgaaggc ctcagattct gctgtggacg tggccattga aattcttgcc 420

actcgaaccc caccccagct gcaggagtgc ctggcagtct acaaacacaa tttccaggtg 480

gaggctgtgg atggcatcac atctgagacc agtggcatct tgcaggacct gctgttggcc 540

ctggccaagg ggggccgtga cagctactct ggaatcattg actataatct ggcagaacaa 600
gatgtccagg cactgcagcg ggcagaagga cctagcagag aggaaacatg ggtcccagtc 660
ttcacccagc gaaatcctga acacctcatc cgagtgtttg atcagtacca gcggagcact 720
gggcaagagc tggaggaggc tgtccagaac cgtttccatg gagatgctca ggtggctctg 780
ctcggcctag cttcggtgat caagaacaca ccgctgtact ttgctgacaa acttcatcaa 840
gccctccagg aaactgagcc caattaccaa gtcctgattc gcatccttat ctctcgatgt 900
gagactgacc ttctgagtat cagagctgag ttcaggaaga aatttgggaa gtccctctac 960
tcttctctcc aggatgcagt gaaaggggat tgccagtcag ccctcctggc cttgtgcagg 1020
gctgaagaca tgtgagaccc cagctttctt 1050

<210> 575
<211> 460
<212> DNA
<213> Homo sapiens

<400> 575
aaaaaagcag gcatgtcggg ccgcggcaag actggcggca aggcccgcgc caaggccaag 60
tcgcgctcgt cgcgcgccgg cctccagttc ccagtgggcc gtgtacaccg gctgctgcgg 120
aagggccact acgccgagcg cgttggcgcc ggcgcgccag tgtacctggc ggcagtgctg 180
gagtacctca ccgctgagat cctggagctg gcgggcaatg cggcccgcga caacaagaag 240
acgctaatca tcccccgcca cctgcagctg gccatccgca acgacgagga gctcaacaag 300
ctgctgggcg gcgtgacgat cgcccaggga ggcgtcctgc ccaacatcca ggccgtgctg 360
ctgcccaaga agaccagcgc caccgtgggg ccgaaggcgc cctcgggcgg caagaaggcc 420
acccaggcct cccaggagta ttaattaacc cagctttctt 460

<210> 576
<211> 620
<212> DNA
<213> Homo sapiens

<400> 576
aaaaaagctg gcacccctgg tccaggtatg gactgcagga agatggcccg cttctcttac 60
agtgtgattt ggatcatggc catttctaaa gtctttgaac tgggattagt tgccgggctg 120
ggccatcagg aatttgctca tccatctcgg ggatacctgg ccttcagaga tgacagcatt 180
tggccccagg aggagcctgc aattcggcct cggtcttccc agcgtgtgcc gcccatgggg 240
atacagcaca gtaaggagct aaacagaacc tgctgcctga atgggggaac ctgcatgctg 300

gggtcctttt gtgcctgccc tccctccttc tacggacgga actgtgagca cgatgtgcgc 360
aaagagaact gtgggtctgt gccccatgac acctggctgc ccaagaagtg ttccctgtgt 420
aaatgctggc acggtcagct ccgctgcttt cctcaggcat ttctacccgg ctgtgatggc 480
cttgtgatgg atgagcacct cgtggcttcc aggactccag aactaccacc gtctgcacgt 540
actaccactt ttatgctagt tggcatctgc ctttctatac aaagctacta tccaggccca 600
ggcggcgacc cagctttctt 620

<210> 577
<211> 1597
<212> DNA
<213> Homo sapiens

<400> 577
aaaaaagcag gcatggaagt ggacggcacg aggagatgcc gctgccggtt caggtgttta 60
acttgcagca gtaggtcaga cctcgtcctg cccggcacgg ccggggactt cagcctgagc 120
gccagcctgt cggcctgtac gctgctctac gagggggccg tggagcccat gcagatcgac 180
gtggaccccc aggaagaccc gcagaatgca cctgacgtca actacgtggt ggagaacccc 240
agcctggatc tggaacagta cgcggccagc tacagcggcc tgatgcgcat cgaacggctg 300
cagttcattg ctgatcactg ccccacgctg cgggtggagg ccctgaagat ggccctctcc 360
ttcgtgcaga gaacctttaa cgtggacatg tacgaggaga tccaccgcaa gctctcagag 420
gccaccaggg agctgcagaa cgcacccgac gccatccctg agagcggcgt ggagccccca 480
gccctggaca cggcctgggt ggaggccacg cggaagaagg cgctgctgaa gctggagaag 540
ctggacacag acctgaagaa ctacaagggc aactccatca aagagagcat ccggcgcggc 600
cacgacgacc tgggcgacca ctacctggac tgtggggacc tcagcaacgc cctcaagtgc 660
tattcccggg cccgggacta ctgcaccagc gccaaacacg tcatcaacat gtgcctcaat 720
gtcatcaagg tcagcgtcta cttgcagaat tggtctcatg tgctcagcta cgtcagcaag 780
gctgagtcca ccccagagat tgccgagcga ggagagcgtg acagccagac ccaggccatc 840
ctcaccaagc tcaagtgtgc cgcaggcttg gcagagctgg ccgccaggaa gtacaagcag 900
gctgccaagt gcctcctgct ggcttccttt gatcactgtg acttccctga gctgctgtcc 960
cccagcaacg tggccatcta cggtggcctg tgcgccttgg ctacctttga ccggcaggag 1020
ctgcagcgca atgtcatctc cagcagctcc ttcaagttgt tcttggagct ggagccacag 1080
gtccgagaca tcatcttcaa attctacgag tccaagtacg cctcatgtct caagatgctg 1140
gacgagatga aggacaacct gctcctggac atgtatctgg ccccccatgt caggaccctg 1200
tacacccaga ttcgcaaccg tgccctcatc cagtatttca gcccctacgt gtcagccgac 1260
atgcatagga tggcggcagc cttcaatacc acggtggccg ccctggagga cgagctgacg 1320
cagctaatcc tggaggggct gatcagtgcc cgtgtggact cacacagcaa gatcctatac 1380

gcccgggacg tggatcagcg cagcaccacc tttgagaagt ctctgttgat gggcaaggag 1440

ttccagcgcc gcgccaaggc catgatgctg cgggcagctg tgctccgcaa ccagatccat 1500

gtcaagtccc cgcccagaga agggagccag ggggagctga ctccagccaa cagccagtcc 1560

cggatgagca ccaacattta attaacccag ctttctt 1597

<210> 578

<211> 817

<212> DNA

<213> Homo sapiens

<400> 578
aaaaaagcag gcatgggctg tcgggatgtc cacgcagcca cagtcctttc cttcctgtgt 60

ggaatcgcct cagtagcagg cctctttgca gggactctgc ttcccaactg gagaaaatta 120

cgattgatca cattcaacag aaacgggaag aacctgactg tttacacagg cctgtgggtg 180

aaatgtgccc ggtatgacgg gagcagtgac tgcctgatgt acgacactac ttggtactca 240

tcagttgacc agctggacct gcgtgtcctc cagtttgccc tacccctcag catgctgatc 300

gccatgggtg ccctgctgct ctgcctgatt ggaatgtgca acactgcctt caggtcctcg 360

gtgcccaaca tcaaactggc caagtgtctg gtcaatagtg caggttgcca cctggtggct 420

gggctgctat ttttcctggc aggtactgtg agcctctccc catctatctg ggtcatcttt 480

tataacatcc atctgaacaa gaagtttgag ccagtctttt catttgacta tgcagtgtat 540

gtcactattg ctagtgctgg gggcctgttt atgacttccc ttatactatt tatttggtat 600

tgtacatgca aatctttgcc ttctcctttc tggcaaccat tgtactccca tccacccagt 660

atgcatactt actcacagcc ctattcagca cgccctcgcc tctctgccat tgaaattgac 720

attccagtag tttcacacac cacttaatgg ggaaatagtt aattgttaaa gaaaacttct 780

tgtagcctca cattcccctt gtgcacccag ctttctt 817

<210> 579

<211> 394

<212> DNA

<213> Homo sapiens

<400> 579
aaaaaagcag gcatgaggat catgctgcta ttcacagcca tcctggcctt cagcctagct 60

cagagctttg gggctgtctg taaggagcca caggaggagg tggttcctgg cgggggccgc 120

agcaagaggg atccagatct ctaccagctg ctccagagac tcttcaaaag ccactcatct 180

ctggagggat tgctcaaagc cctgagccag gctagcacag atcctaagga atcaacatct 240

cccgagaaac gtgacatgca tgacttcttt gtgggactta tgggcaagag gagcgtccag 300
ccagactctc ctacggatgt gaatcaagag aacgtcccca gctttggcat cctcaagtat 360
cccccgagag cagattaatt aacccagctt tctt 394

<210> 580
<211> 829
<212> DNA
<213> Homo sapiens

<400> 580
aaaaaagcag gcatggtgtg tctgaagctc cctggaggct ccagcttggc agcgttgaca 60
gtgacactga tggtgctgag ctcccgactg gctttcgctg gggacacccg accacgcttc 120
ttggagctgc ttaagtctga gtgtcatttc ttcaatggga cggagcgggt gcggttcctg 180
gagagacact tccataacca ggaggagtac gcgcgcttcg acagcgacgt gggggagtac 240
cgggcggtga gggagctggg gcggcctgat gccgagtact ggaacagcca gaaggacctc 300
ctggagcaga agcggggcca ggtggacaat tactgcagac acaactacgg ggttggtgag 360
agcttcacag tgcagcggcg agtccatcct caggtgactg tgtatcctgc aaagacccag 420
cccctgcagc accacaacct cctggtctgc tctgtgagtg gtttctatcc aggcagcatt 480
gaagtcaggt ggttccggaa cggccaggaa gagaaggctg gggtggtgtc cacgggcctg 540
atccagaatg gagactggac cttccagacc ctggtgatgc tagaaacagt tcctcggagt 600
ggagaggttt acacccgcca agtggagcac ccaagcgtaa cgagccctct cacagtggaa 660
tggagtgcac ggtctgaatc tgcacagagc aagatgctga gtggagtcgg gggctttgtg 720
ctgggcctgc tcttccttgg ggccgggctg ttcatctact tcaggaatca gaaaggacac 780
tctggacttc agccaacagg attcctgagc taattaaccc agctttctt 829

<210> 581
<211> 1066
<212> DNA
<213> Homo sapiens

<400> 581
aaaaaagcag gcatgaacaa ggacttgcta agaagagtct tggtcttgat gaatttaaag 60
cacacttttc tggccttgtg tgcccttcgc tttatgaggc ggataattgg acttaaagat 120
gaattttata atcgttacat caccaaggga aatctttttg agccagttat aaatgcactt 180
ctggataatg gaactcggta taatctgttg aattcagctg ttattgagtt gtttgaattt 240
ataagagtgg aagatatcaa gtctcttact gcccatatag ttgaaaactt ttataaagca 300
cttgaatcga ttgaatatgt tcagacattc aaaggattga agactaaata tgagcaagaa 360

aaagacagac aaaatcagaa actgaacagt gtaccatcta tattgcgtag taacagattt 420
cgcagagatg caaaagcctt ggaagaggat gaagaaatgt ggtttaatga agatgaagaa 480
gaggaaggaa aagcagttgt ggcaccagtg gaaaaaccta agccagaaga tgattctcca 540
gataattatg aaaagtttat ggagactaaa aaagcaaaag aaagtgaaga caaggaaaac 600
cttcccaaaa ggacatctcc tggtggcttc aaatttactt tctcccactc tgccagtgct 660
gctaatggaa caaacagtaa atctgtagtg gctcagatac caccagcaac ttctaatgga 720
tcctcttcca aaaccacaaa cttgcctacg tcagtaacag ccaccaaggg aagtttggtt 780
ggcttagtgg attatccaga tgatgaagag gaagatgaag aagaagaatc gtcccccagg 840
aaaagacctc gtcttggctc ataaaatatt tattagggga ccctcaacat gtggtcttac 900
aatgctgcaa ctgttcagtg agctgaaaat ctgaatcaga aagctttctc aattgaactt 960
ataaaatata caaggagtag caaaagacag tatatcagct aagagagttt agttctaata 1020
aaaatcaggc ttcccaggaa cttgattgct tgcacccagc tttctt 1066

<210> 582
<211> 818
<212> DNA
<213> Homo sapiens

<400> 582
aaaaaagctg gcacccctgg tccaggtatg atcctaaaca aagctctgat gctgggggcc 60
ctcgccctga ccaccgtgat gagcccttgt ggaggtgaag acattgtggc tgaccacgtt 120
gcctcttacg gtgtaaactt gtaccagtct tacggtccct ctggccagtt cacccatgaa 180
tttgatggag acgaggagtt ctatgtggac ctggagagga aggagactgt ctggaagttg 240
cctctgttcc acagacttag atttgacccg caatttgcac tgacaaacat cgctgtgcta 300
aaacataact tgaacatcct gattaaacgc tccaactcta ccgctgctac caatgaggtt 360
cctgaggtca cagtgttttc caagtctccc gtgacactgg gtcagcccaa caccctcatc 420
tgtcttgtgg acaacatctt tcctcctgtg gtcaacatca cctggctgag caatgggcac 480
tcagtcacag aaggtgtttc tgagaccagc ttcctctcca agagtgatca ttccttcttc 540
aagatcagtt acctcacctt cctcccttct gctgatgaga tttatgactg caaggtggag 600
cactggggcc tggatgagcc tcttctgaaa cactgggagc ctgagattcc agcacctatg 660
tcagagctca cagagactgt ggtctgtgcc ctggggttgt ctgtgggcct cgtgggcatt 720
gtggtgggga ccgtcttgat catccgaggc ctgcgttcag ttggtgcttc cagacaccaa 780
gggcccttgc caggcccagg cggcgaccca gctttctt 818

<210> 583
<211> 514

<212>  DNA

<213>  Homo sapiens


<400>  583
aaaaaagcag gcatggcccg agcagtgttt ggtgtgaaat gtttctctta ttctacgagt     60

ctgattggcc ttacatttct gccatacatt tttacacaaa ataatgctat ttctgaaagt    120

gtgcctctgc ctattcaaat catattctat ggcatcatgg gaagctttac ggtgatcacc    180

ccagtgctgc ttcactttat tacaaaaggc tatgtcattc gattgtacca tgaggccaca    240

acagacactt ataaagccat tacctacaat gctatgcttg cagaaacgag tacagtgttt    300

caccagaatg atgtgaagat tccagatgct aaacatgtat ttaccacatt ttatgctaaa    360

acaaaatcac tgttagttaa tccagtgctc tttccaaacc gtgaagacta tatccatcta    420

atgggttatg acaaagaaga atttattttg tatatggaag aaaccagtga agagaaacgg    480

cataaagatg agaattaatt aacccagctt tctt                                514

<210>  584

<211>  775

<212>  DNA

<213>  Homo sapiens


<400>  584
aaaaaagcag gcatgttgtc aacagttggt tcattccttc aggacctaca aaatgaagat     60

aagggtatca aaactgcagc catcttcaca gcagatggca acatgatttc agcttctacc    120

ttgatggata ttttgctaat gaatgatttt aaacttgtca ttaataaaat agcatatgat    180

gtgcagtgtc caaagagaga aaaaccaagt aatgagcaca ctgctgagat ggaacacatg    240

aaatccttgg ttcacagact atttacaatc ttgcatttag aagagtctca gaaaaagaga    300

gagcaccatt tactggagaa aattggccac ctgaaggaac agctgcagcc ccttgaacag    360

gtgaaagctg gaatagaagc tcattcggga gccaaaacca gtggactcct gtgggctgga    420

ttggcactgc tgtccattca gggtggggca ctggcctggc tcacgtggtg ggtgtactcc    480

tgggatatca tggagccagt tacatacttc gtcacatttg caaattctat ggtctttttt    540

gcatacttta tagtcactcg acaggattat acttactcag ctgttaagag taggcaattt    600

cttcagttct tccacaagaa atcaaagcaa cagcactttg atgtgcagca atacaacaag    660

ttaaaagaag accttgctaa ggctaaagaa tccctgaaac aggcgcgcca ttctctctgt    720

ttgcaaatgc aagtagaaga actcaatgaa aagaattaat taacccagct ttctt         775

<210>  585

<211>  331

<212>  DNA

<213>  Homo sapiens


<400>  585
aaaaaagcag gcatggagga agccagtgaa ggtggaggaa atgatcgtgt gcggaacctg      60

caaagtgagg tggagggagt taagaatatt atgacccaga atgtggagcg gatcctggcc     120

cggggggaaa acttggaaca tctccgcaac aagacagagg atctggaagc cacatctgag     180

cacttcaaga cgacatcgca gaaggtggct cgaaaattct ggtggaagaa cgtgaagatg     240

attgtcctta tccgcgtgat tgtttttatc atcatcctct tcattgtgct ctttgccact     300

ggtgccttct cttaattaac ccagctttct t                                    331

<210>  586

<211>  586

<212>  DNA

<213>  Homo sapiens


<400>  586
aaaaaagcag gcatggggaa aaaacaaaac aagaagaaag tggaggaggt gctagaagag      60

gaggaagagg aatatgtggt ggaaaaagtt ctcgaccgtc gagtggtaaa gggcaaagtg     120

gagtacctcc taaagtggaa gggattctca gatgaggaca acacatggga gccagaagag     180

aacctggatt gccccgacct cattgctgag tttctgcagt cacagaaaac agcacatgag     240

acaggtaaat cagagggagg caagcgcaaa gctgattctg attctgaaga taagggagag     300

gagagcaaac caaagaagaa gaaagaagag tcagaaaagc cacgaggctt tgctcgaggt     360

ttggagccgg agcggattat tggagctaca gactccagtg gagagctcat gttcctgatg     420

aaatggaaaa actctgatga ggctgacctg gtccctgcca aggaagccaa tgtcaagtgc     480

ccacaggttg tcatatcctt ctatgaggaa aggctgacgt ggcattccta cccctcggag     540

gatgatgaca aaaaagatga caagaattaa ttaacccagc tttctt                    586

<210>  587

<211>  512

<212>  DNA

<213>  Homo sapiens


<400>  587
aaaaaagctg gcacccctgg tccaggtatg tcgaccccgg cccggaggag gctcatgcgg      60

gatttcaagc ggttacaaga ggacccacct gtgggtgtca gtggcgcacc atctgaaaac     120

aacatcatgc agtggaatgc agttatattt ggaccagaag ggacaccttt tgaagatggt     180

acttttaaac tagtaataga attttctgaa gaatatccaa ataaaccacc aactgttagg 240

tttttatcca aaatgtttca tccaaatgtg tatgctgatg gtagcatatg tttagatatc 300

cttcagaatc gatggagtcc aacatatgac gtatcttcta tcttaacatc aattcagtct 360

ctgctggatg aaccgaatcc taacagtcca gccaatagcc aggcagcaca gctttatcag 420

gaaaacaaac gagaatatga gaaaagagtt tcggccattg ttaacaaagc tggaatgatt 480

caccaggccc aggcggcgac cacagctttc tt 512

<210> 588

<211> 1006

<212> DNA

<213> Homo sapiens

<400> 588
aaaaaagcag gcatggacat gtggacggcg ctgctcatcc tgcaagcctt gttgctaccc 60

tccctggctg atggtgccac ccctgccctg cgctttgtag ccgtgggtga ctggggaggg 120

gtccccaatg ccccattcca cacggcccgg gaaatggcca atgccaagga gatcgctcgg 180

actgtgcaga tcctgggtgc agacttcatc ctgtctctag gggacaattt ttactttact 240

ggtgtgcaag acatcaatga caagaggttc caggagacct ttgaggacgt attctctgac 300

cgctcccttc gcaaagtgcc ctggtacgtg ctagccggaa accatgacca ccttggcaat 360

gtctctgccc agattgcata ctctaagatc tccaagcgct ggaacttccc cagccctttc 420

taccgcctgc acttcaagat cccacagacc aatatgtctg tggccatttt tatgctggac 480

acagtgacac tatgtggcaa ctcagatgac ttcctcagcc agcagcctga gaggccccga 540

gacgtgaagc tggcccgcac acagctgtcc tggctcaaga aacagctggc ggcggccagg 600

gaggactaca tgctggtggc tggccactac cccgtgtggt ccatagccga gcacgggcct 660

acccactgcc tggtcaagca gctacggcca ctgctggcca catacggggt cactgcctac 720

ctgtgcggcc acgatcacaa tctgcagtac ctgcaagatg agaatggcgt gggctacgtg 780

ctgagtgggg ctgggaattt catggacccc tcaaagcggc accagcgcaa ggtccccaac 840

ggctatctgc gcttccacta tgggactgaa gactcactgg gtggctttgc ctatgtggag 900

atcagctcca aagagatgac tgtcacttac atcgaggcct cgggcaagtc cctctttaag 960

accaggctgc cgaggcgagc caggccttaa ttaacccagc tttctt 1006

<210> 589

<211> 556

<212> DNA

<213> Homo sapiens

<400>  589
aaaaaagcag gcatggagaa aattccagtg tcagcattct tgctccttgt ggccctctcc      60

tacactctgg ccagagatac cacagtcaaa cctggagcca aaaaggacac aaaggactct     120

cgacccaaac tgccccagac cctctccaga ggttggggtg accaactcat ctggactcag     180

acatatgaag aagctctata taaatccaag acaagcaaca aacccttgat gattattcat     240

cacttggatg agtgcccaca cagtcaagct ttaaagaaag tgtttgctga aaataaagaa     300

atccagaaat tggcagagca gtttgtcctc ctcaatctgg tttatgaaac aactggcaaa     360

cacctttctc ctgatggcca gtatgtcccc aggattatgt ttgttgaccc atctctgaca     420

gttagagccg atatcactgg aagatattca aatcgtctct atgcttacga acctgcagat     480

acagctctgt tgcttgacaa catgaagaaa gctctcaagt tgctgaagac tgaattttaa     540

ttaacccagc tttctt                                                     556

<210>  590

<211>  53

<212>  DNA

<213>  Homo sapiens


<400>  590
aaaaaaaagt tcagcgacat gcatctagtg gctccttcct acccagcttt ctt             53

<210>  591

<211>  557

<212>  DNA

<213>  Homo sapiens


<400>  591
aaaaaagctg gcacccctgg tccaggtatg agtttagctc tgagaagtga gcttgtagtg      60

gacaaaacaa agaggaaaaa aagaagagaa ctgtctgagg aacagaaaca agaaattaaa     120

gatgcttttg aactatttga tacagacaaa gatgaagcaa tagattatca tgaattaaag     180

gtggcaatga gagccttggg gtttgatgta aaaaaagctg atgtactgaa gattcttaaa     240

gattatgaca gagaagccac agggaaaatc acctttgaag attttaatga agttgtgaca     300

gactggatat tggaaagaga tccccatgaa gaaatactca aggcatttaa actatttgat     360

gatgatgatt caggtaaaat aagcttgagg aatttgcgaa gtgttgctag agaattgggt     420

gaaaacatga gtgatgaaga acttcgagct atgatagaag aatttgacaa agatggtgat     480

ggagaaataa accaagagga gttcattgct attatgactg gtgacattcc aggcccaggc     540

ggcgacccag ctttctt                                                    557

<210>  592

<211>  1366

<212>  DNA

<213>  Homo sapiens


<400>  592
aaaaaagcag gcatgcgcga gatcgtgcac atccaggcgg gccagtgcgg caaccagatc      60

ggcgccaagt tttgggaggt catcagtgat gagcatggga ttgaccccac tggcagttac     120

catggagaca gtgatttgca gctggagaga atcaatgttt actacaatga agccactggt     180

aacaaatatg ttcctcgggc catcctcgtg gatctggagc caggcacgat ggattcggtt     240

aggtctggac cattcggcca gatcttcaga ccagacaatt tcgtgtttgg ccagagtgga     300

gccgggaata actgggccaa gggccactac acagagggag ccgagctggt cgactcggtc     360

ctggatgtgg tgaggaagga gtcagagagc tgtgactgtc tccagggctt ccagctgacc     420

cactctctgg ggggcggcac ggggtccggg atgggcaccc tgctcatcag caagatccgg     480

gaagagtacc cagaccgcat catgaacacc ttcagcgtca tgccctcacc caaggtgtca     540

gacacggtgg tggagcccta caacgccacc ctctcggtcc accagctggt ggaaaacaca     600

gatgaaacct actgcattga caacgaggcc ctgtatgaca tctgcttccg caccctgaag     660

ctgaccaccc ccacctacgg ggacctcaac cacctggtgt cggccaccat gagcggggtc     720

accacctgcc tgcgcttccc gggccagctg aacgcagacc tgcgcaagct ggcggtgaac     780

atggtgccct tccctcgcct gcacttcttc atgcccggct tcgcgcccct gaccagccgg     840

ggcagccagc agtaccgggc gctcacggtg cccgagctca cccagcagat gttcgactcc     900

aagaacatga tggccgcctg cgacccgcgc cacggccgct acctgacggt ggctgccatc     960

ttccggggcc gcatgtccat gaaggaggtg gacgagcaga tgctcaacgt gcagaacaag    1020

aacagcagct acttcgtgga gtggatcccc aacaacgtga agacggccgt gtgcgacatc    1080

ccgccccgcg gcctgaagat gtcggccacc ttcatcggca acagcacggc catccaggag    1140

ctgttcaagc gcatctccga gcagttcacg gccatgttcc ggcgcaaggc cttcctgcac    1200

tggtacacgg gcgagggcat ggacgagatg gagttcaccg aggccgagag caacatgaac    1260

gacctggtgt ccgagtacca gcagtaccag gacgccacgg ccgacgaaca aggggagttc    1320

gaggaggagg agggcgagga cgaggcttaa ttaacccagc tttctt                   1366

<210>  593

<211>  472

<212>  DNA

<213>  Homo sapiens


<400>  593
aaaaaagcag gcatggcttc tcatcgtctg ctcctcctct gccttgctgg actggtattt      60

gtgtctgagg ctggccctac gggcaccggt gaatccaagt gtcctctgat ggtcaaagtt 120
ctagatgctg tccgaggcag tcctgccatc aatgtggccg tgcatgtgtt cagaaaggct 180
gctgatgaca cctgggagcc atttgcctct gggaaaacca gtgagtctgg agagctgcat 240
gggctcacaa ctgaggagga atttgtagaa gggatataca aagtggaaat agacaccaaa 300
tcttactgga aggcacttgg catctcccca ttccatgagc atgcagaggt ggtattcaca 360
gccaacgact ccggcccccg ccgctacacc attgccgccc tgctgagccc ctactcctat 420
tccaccacgg ctgtcgtcac caatcccaag gattaattaa cccagctttc tt 472

<210> 594
<211> 1417
<212> DNA
<213> Homo sapiens

<400> 594
aaaaaagcag gcatgagcgg cctggatggg gtcaagagga ccactcccct ccaaacccac 60
agcatcatta tttctgacca agtcccgagc gaccaggacg cacaccagta cctgaggctc 120
cgcgaccaaa gcgaggcgac acaggtgatg gcggagccgg gtgagggagg ctcggagacc 180
gtcgcgctcc cgccttcacc gccttcagag gagggggcgg taccccagga tcccgcgggc 240
cgtggcggta ctccccagat ccgagttgtt gggggtcgcg gtcatgtggc gatcaaagcc 300
gggcaggaag agggccagcc tcccgccgaa ggcctggcag ccgcttctgt ggtgatggca 360
gccgaccgca gcctgaaaaa gggcgttcag ggtggagaga aggccctaga aatctgtggc 420
gcccagagat ccgcgtctga gctgacggcg ggggcggagg ctgaggcgga ggaggtgaag 480
acaggaaagt gcgccaccgt ctcagcagcc gtggctgaga gggagagcgc tgaggtggtg 540
gtgaaggaag gcctggcgga gaaggaggta atggaggagc agatggaggt agaggagcag 600
ccgccagaag gtgaagaaat agaagtggcg gaggaggata gattggagga ggaggcgagg 660
gaggaagaag ggccctggcc tttgcatgag gctctccgca tggaccctct gaaggccatc 720
cagctggaac tggacactgt gaatgctcag gccgacaggg ccttccaaca gctggagcac 780
aagtttgggc ggatgcgtcg acactacctg gagcggagga actacatcat tcagaatatc 840
ccgggcttct ggatgactgc ttttcgaaac cacccccagt tgtccgccat gattaggggc 900
caagatgcag agatgttaag gtacataacc aatttagagg tgaaggaact cagacaccct 960
agaaccggtt gcaagttcaa gttcttcttt agaagaaacc cctacttcag aaacaagctg 1020
attgtcaagg aatatgaggt aagatcctcc ggccgagtgg tgtctctttc tactccaatt 1080
atatggcgca gggggcatga accccagtcc ttcattcgca gaaaccaaga cctcatctgc 1140
agcttcttca cttggttttc agaccacagc cttccagagt ccgacaaaat tgctgagatt 1200
attaaagagg atctgtggcc aaatccactg caatactacc tgttgcgtga aggagtccgt 1260

agagcccgac gtcgcccgct aagggagcct gtagagatcc ccaggccctt tgggttccag 1320
tctggttaac atttgccctt gggaatactc ctgcacaagg tctcctacca ccttctgctg 1380
gacctgtgct tgggcatcag caatacccag ctttctt 1417

<210> 595
<211> 544
<212> DNA
<213> Homo sapiens

<400> 595
aaaaaagcag gcatggtgaa gctgaacagt aaccccagcg agaagggaac caagccgcct 60
tcagttgagg atggcttcca gaccgtccct cccatcactc ccttggaggt taatcactta 120
cagctgcctg ctccagaaaa ggtgattgtg aagacaagaa cggaatatca gccggaacag 180
aagaacaaag ggaagttccg ggtgccaaaa atcgctgaat ttacggtcac catccttgtc 240
agcctggccc tagctttcct tgcgtgcatc gtgttcctgg tggtttacaa agccttcacc 300
tatgatcaca gctgcccaga gggattcgtc tataagcaca aacgctgtat cccagcctcc 360
ctggatgctt actactcctc ccaggacccc aattccagaa gccgcttcta cacagtcatc 420
agccactaca gcgtggccaa gcagagcact gcccgggcca tcgggccgtg gctgtcagca 480
gccgctgtca tccatgagcc caagccgccc aagacccagg gccattaatt aacccagctt 540
tctt 544

<210> 596
<211> 472
<212> DNA
<213> Homo sapiens

<400> 596
aaaaaagcag gcatggcgct gaaacggatt aataaggaac ttagtgattt ggcccgtgac 60
cctccagcac aatgttctgc aggtccagtt ggggatgata tgtttcattg gcaagccaca 120
attatgggac ctaatgacag cccatatcaa ggcggtgtat tctttttgac aattcatttt 180
cctacagact accccttcaa accacctaag gttgcattta caacaagaat ttatcatcca 240
aatattaaca gtaatggcag catttgtctc gatattctaa gatcacagtg gtcgcctgct 300
ttaacaattt ctaaagttct tttatccatt tgttcactgc tatgtgatcc aaacccagat 360
gaccccctag tgccagagat tgcacggatc tataaaacag acagagataa gtacaacaga 420
gtatctcggg aatggactca gaagtatgcc atttaattaa cccagctttc tt 472

<210> 597

<211>  1181

<212>  DNA

<213>  Homo sapiens

<400>  597
aaaaaagcag gcatgtctac gcgggagtcc tttaacccgg aaagttacga attggacaaa      60

agcttccggc taaccagatt cactgaactg aagggcacag gctgcaaagt gccccaagat     120

gtcctgcaaa aattgctgga atctttacag gagaaccact tccaagaaga tgagcagttt     180

ctgggagccg ttatgccaag gcttggcatt ggaatggata cttgtgtcat tcctttgagg     240

cacggtgggc tttccttggt tcaaaccaca gattacattt acccgatcgt agacgaccct     300

tacatgatgg gcaggatagc gtgtgccaat gtcctcagtg acctctatgc aatgggggtc     360

acggaatgtg acaatatgct gatgctcctt ggagtcagta ataaaatgac cgacagggaa     420

agggataaag tgatgcctct gattatccaa ggttttaaag acgcagctga ggaagcagga     480

acatctgtaa caggcggcca aacagtacta aacccctgga ttgtcctggg aggagtggct     540

accactgtct gccaacccaa tgaatttatc atgccagaca atgcagtgcc aggggacgtg     600

ctggtgctga caaaacccct ggggacacag gtggcagtgg ctgtgcacca gtggctggat     660

atccctgaga aatggaataa gattaaacta gtggtcaccc aagaagatgt agagctggcc     720

taccaggagg cgatgatgaa catggcgagg ctcaacagga cagctgcagg actcatgcac     780

acgttcaatg cccacgccgc cactgacatc acgggcttcg ggattttggg ccatgcgcag     840

aacctggcca agcagcagag gaacgaggtg tcgtttgtaa ttcacaacct cccggtgctg     900

gccaagatgg ctgcggtgag caaggcctgc ggaaacatgt tcggcctcat gcacgggacc     960

tgcccggaga cttcaggcgg ccttctgatc tgtttaccac gtgagcaagc agctcggttc    1020

tgtgcagaga taaagtcccc caaatatggt gaaggccacc aagcatggat tattgggatt    1080

gtagagaagg gcaaccgcac agccagaatc atagacaaac cccggatcat cgaggtcgca    1140

ccacaagtgg ccactcaaaa cttaattaac ccagctttct t                        1181

<210>  598

<211>  1639

<212>  DNA

<213>  Homo sapiens

<400>  598
aaaaaagcag gcatggcgct caccagcttt ttacctgcac ccactcagct atctcaggac      60

cagcttgagg ctgaagaaaa ggcaagatcc cagagatcac ggcagacctc actggtctcc     120

tcccgaagag aacctccccc gtacggatac cggaaaggct ggatacctcg gttattagag     180

gattttggag atggaggtgc ttttccagag atccatgtgg cccagtatcc actggatatg     240

ggacgaaaga aaaaaatgtc gaatgcgctg gccattcagg tggattctga aggaaaaatt 300
aaatatgatg caattgctcg acaaggacag tcaaaagaca aggtcattta tagcaaatac 360
actgacctgg ttccaaagga ggttatgaat gcagatgatc cagacctgca aaggcccgat 420
gaagaagcta ttaaagagat aacagaaaag acaagagtag ccttagaaaa atctgtatca 480
cagaaggtcg ccgcagccat gccagttcga gcagctgaca aattggctcc tgctcagtat 540
atccgataca caccatctca gcaaggagtg gcattcaact ctggagctaa acagagggtt 600
attcggatgg tagaaatgca gaaagatcca atggagcctc caaggttcaa gattaataag 660
aaaattcccc ggggaccacc ttctcctcct gcgcctgtcg tgcattctcc tagccgaaag 720
atgactgtaa aggaacaaca agagtggaag attcctcctt gtatttctaa ctggaaaaat 780
gcaaagggtt atacaattcc attagacaaa cgtctggctg ctgatggaag aggactacag 840
acagtacaca taaatgaaaa tttcgccaaa ttggcagaag ccctctacat tgctgatcgg 900
aaggctcgtg aagctgtgga aatgcgtgcc caagtagaga gaaaaatggc tcagaaagaa 960
aaggaaaaac atgaagagaa acttagagaa atggcccaga aagccaggga aagaagagct 1020
gggatcaaaa ctcatgtgga aaaagaggat ggggaggcac gtgagaggga tgaaatccgg 1080
catgacaggc gaaaagagag acagcatgac cggaatcttt ccagggcagc tcctgataag 1140
aggtcgaaac ttcagagaaa tgaaaatcgg gatatcagtg aagttattgc tctcggtgtt 1200
cctaatcctc ggacttccga tgaagttcag tatgaccaaa ggctcttcaa ccaatccaag 1260
ggtatggaca gtggatttgc aggtggagaa gatgaaattt ataatgttta tgatcaagcc 1320
tggagaggtg gtaaagatat ggcccagagt atttataggc ccagtaaaaa tctggacaag 1380
gacatgtatg gtgatgacct agaagccaga ataaagacca acagatttgt tcccgacaag 1440
gagttttctg gttcagaccg tagacagaga ggccgagaag gaccagttca gtttgaggaa 1500
gatccttttg gtttggacaa gtttttggaa gaagccaaac agcatggtgg ctctaaaaga 1560
ccctcagata gcagccgccc caaggaacac gagcatgaag gcaagaagag gaggaaggat 1620
taattaaccc agctttctt 1639

<210> 599
<211> 658
<212> DNA
<213> Homo sapiens

<400> 599
aaaaaagctg gcacccctgg tccaggtatg gggaaccact tgactgagat ggcgcccact 60
gcctcctcct tcttgcccca cttccaagcc ctgcatgtcg tggtcattgg gctggactct 120
gctggaaaga cctccctcct ttaccgcctc aagttcaagg agtttgtcca gagtgtcccc 180
accaaaggct tcaacaccga gaagatccgg gtgcccctcg ggggatcgcg tggcatcacc 240
ttccaagtgt gggacgtcgg ggggcaggag aagctgcgac cactgtggcg ctcttatacc 300

cgccggacag acggtctagt gtttgtggtg gacgctgcgg aggctgagcg gctggaggaa 360

gccaaggtgg agttgcaccg aatcagccgg gcctcggaca accagggcgt gccagtgctg 420

gtgctggcca acaagcagga ccagcccggg gcactgagcg ctgctgaggt ggagaagagg 480

ctggcagtcc gagagccagc agccgccact cccactcatg tgcaaggctg cagcgctgtg 540

gacggtctgg gcctgcagca gggccttgag cgcctctatg agatgatcct caagaggaag 600

aaggcagctc ggggtggcaa gaagaacggc caggcccagg cggcgaccca gctttctt 658

<210> 600

<211> 748

<212> DNA

<213> Homo sapiens

<400> 600
aaaaaagcag gcatgttcct ggttaactcg ttcttgaagg gcggcggcgg cggcggcggc 60

ggcggcggtg gtggaggcgg cggtggcggt ggaacggcca tgcgcatcct aggcggagtc 120

atcagcgcca tcagcgaggc ggctgcgcag tacaacccgg agcccccgcc cccacgcaca 180

cattactcca acattgaggc caacgagagt gaggaggtcc ggcagttccg gagactcttt 240

gcccagctgg ctggagatga catggaggtc agcgccacag aactcatgaa cattctcaat 300

aaggttgtga cacgacaccc tgatctgaag actgatggtt ttggcattga cacatgtcgc 360

agcatggtgg ccgtgatgga tagcgacacc acaggcaagc tgggctttga ggaattcaag 420

tacttgtgga acaacatcaa aaggtggcag gccatataca aacagttcga cactgaccga 480

tcagggacca tttgcagtag tgaactccca ggtgcctttg aggcagcagg gttccacctg 540

aatgagcatc tctataacat gatcatccga cgctactcag atgaaagtgg gaacatggat 600

tttgacaact tcatcagctg cttggtcagg ctggacgcca tgttccgtgc cttcaaatct 660

cttgacaaag atggcactgg acaaatccag gtgaacatcc aggagtggct gcagctgact 720

atgtattctt aattaaccca gctttctt 748

<210> 601

<211> 568

<212> DNA

<213> Homo sapiens

<400> 601
aaaaaagcag gcatgctgtc tctagacttt ttggacgatg tgcggcggat gaacaagcgg 60

cagctctatt atcaagtcct aaattttgga atgattgtct catcggcact aatgatctgg 120

aaggggttaa tggtaataac tggaagtgaa agtccgattg tagtggtgct cagtggcagc 180

atggaacctg catttcatag aggagatctt ctctttctaa caaatcgagt tgaagatccc 240
atacgagtgg gagaaattgt tgtttttagg atagaaggaa gagagattcc tatagttcac 300
cgagtcttga agattcatga aaagcaaaat gggcatatca agtttttgac caaaggagat 360
aataatgcgg ttgatgaccg aggcctctat aaacaaggac aacattggct agagaaaaaa 420
gatgttgtgg ggagagccag gggatttgtt ccttatattg gaattgtgac gatcctcatg 480
aatgactatc ctaaatttaa gtatgcagtt ctctttttgc tgggtttatt cgtgctggtt 540
catcgtgatt aattaaccca gctttctt 568

<210> 602
<211> 442
<212> DNA
<213> Homo sapiens

<400> 602
aaaaaagcag gcatgcccaa cttcgccggc acctggaaga tgcgcagcag cgagaatttc 60
gacgagctgc tgaaggcact gggtgtgaac gccatgctga ggaaagtggc cgtagcggct 120
gcgtccaagc cgcacgtgga gatccgccag gacggggatc agttctacat caagacatcc 180
accacggtgc gcaccactga gatcaacttc aaggtcggag aaggctttga ggaggagacc 240
gtggacggac gcaagtgcag gagtttagcc acttgggaga atgagaacaa gatccactgc 300
acgcaaactc ttcttgaagg ggacggcccc aaaacctact ggacccgtga gctggccaac 360
gatgaactta tcctgacgtt tggcgccgat gacgtggtct gcaccagaat ttatgtccgg 420
gattaattaa cccagctttc tt 442

<210> 603
<211> 1744
<212> DNA
<213> Homo sapiens

<400> 603
aaaaaagcag gcatgaaagc tgtgctgcgc aaggctggag gcacgggccc tggggaagag 60
aagcagttcc tggaggtctg ggagaagaac cggaagctca agagcttcaa cctgtcagcg 120
ctggagaaac atgggcctgt ttatgaggat gactgctttg gctgcctgtc ctggtcgcac 180
tcggagacac acttgttgta tgtggcagag aagaagcgcc ccaaggccga gtccttcttt 240
cagaccaaag ccttggacgt cagtgccagc gatgatgaga tagccaggct gaagaagcca 300
gaccaagcca tcaaggcatt ttgggcccct ggagatgctg gtgtggtgtt tgtgggctgg 360
tggcatgagc ccttccggtt gggcatccgc ttttgcacca atcgcaggtc agccctgtat 420
tatgtggacc tcatcggggg gaagtgtgag ctcctctcgg atgactccct ggctgtctct 480

tctccccggc tgagcccaga ccaatgtcgc attgtctacc tgcagtaccc atctctgatc 540
ccccatcacc aatgcagcca gctgtgcctg tatgactggt ataccaaggt tacctcagtg 600
gtggtagatg ttgtgcctcg gcagctggga gagaacttct ctgggatcta ctgcagcctt 660
ctgcctttgg gatgctggtc agctgacagc cagagagtgg tctttgactc ggctcagcgc 720
agccggcagg acctgtttgc tgtggacacc caagtgggca ctgtgacctc cctcacagct 780
ggagggtcag gtgggagctg gaagttgctc acaattgacc aggacctcat ggtggcacag 840
ttttccacac ccagcctacc tccaaccctg aaagttgggt tcctgccttc tgcagggaag 900
gagcagtcag tgttgtgggt gtccctggag gaggccgagc ccattcccga catccactgg 960
ggcatccggg tgctacagcc acccccagag caagagaatg tgcagtatgc tggccttgac 1020
tttgaagcaa tcctgctgca gcctggcagc cctccagata agacccaagt gcccatggtg 1080
gtcatgcccc acgggggccc ccattcatcc tttgtcactg cctggatgct gttcccagcc 1140
atgctttgca agatgggctt tgcggtacta ctagtgaact atcgtggctc cacgggcttt 1200
ggccaggaca gcatcctctc cctcccaggc aatgtgggcc accaggatgt gaaggatgtc 1260
cagtttgcag tggaacaggt gctccaggag gaacactttg atgcaagcca tgtggccctt 1320
atgggtggtt cccatggtgg cttcatttcc tgccacttga ttggtcagta cccagagacc 1380
tacagggcct gcgtggcccg gaaccccgtg atcaacatcg cctccatgtt gggctccact 1440
gacatccctg actggtgcgt ggtggaggct ggctttcctt tcagcagtga ctgcctgcca 1500
gacctcagcg tgtgggctga gatgctggac aaatcgccca tcagatacat ccctcaggtg 1560
aagacaccac tgttactgat gttgggccag gaggaccggc gtgtgccctt caagcagggc 1620
atggagtatt accgtgccct caagacccgg aatgtgcctg ttcggctcct gctctatccc 1680
aaaagcaccc acgcattatc agaggtggag gtggagtcag cttcttaatt aacccagctt 1740
tctt 1744

<210> 604
<211> 1513
<212> DNA
<213> Homo sapiens

<400> 604
aaaaaagcag gcatgccgcg ggtgtacatc ggccgcctga gctaccaggc ccgggagcgc 60
gatgtggagc gcttctttaa gggctacggg aagatcctgg aggtggatct gaagaacgga 120
tatggttttg tggagtttga tgatctgcgt gatgcagatg atgctgttta tgaactgaat 180
ggcaaagacc tttgtggtga gcgagtaatt gttgagcatg cccgcggccc acggcgagat 240
ggcagttacg gttctggacg cagtggatat ggttatagaa gaagtggccg agataaatat 300
ggccctccta ctcgcacaga gtacagactt attgtggaga atttgtcaag tcggtgcagc 360

tggcaagacc taaaggatta tatgcgtcag gcaggagaag tgacttatgc agatgctcac 420
aagggacgca aaaatgaagg ggtgattgaa tttgtatctt attctgatat gaaaagagct 480
ttggaaaagt tggatggaac tgaagtcaat gggagaaaaa tcagattagt tgaagacaag 540
ccaggttcca gacgacgccg gtcctactcc agaagccgga gtcattcaag gtctcgctct 600
cgaagcagac attcccgtaa gagcagaagc cgaagtggca gcagcaaaag cagtcattct 660
aagagtagat ctcggtccag gtcgggctcc cgctcccgga gcaagagccg gagccggagc 720
cagagtcgga gccggagcaa gaaagagaaa agcaggagcc ccagcaagga caagagccgc 780
agccgcagcc atagcgctgg caagagccgc agcaagagca aagaccaagc tgaagagaag 840
atccaaaaca atgacaatgt cgggaaaccc aagagccgga gtcctagcag gcataaaagt 900
aagagcaaaa gtcggagcag gagtcaggag aggagagtgg aggaggagaa gcgagggagt 960
gtgagcaggg gcaggagcca ggagaagagc ctccgccaga gtcggagccg gagcaggagc 1020
aaagcgggca gcaggagccg gagcaggagc cgcagcaaga gcaaggacaa gaggaagagc 1080
aggaagagaa gcagagagga gagccgcagt cgcagtcgca gccgcagcaa gagtgagagg 1140
agcagaaagc gaggcagcaa gcgagacaac aaggcgggca gcagcaagaa gaagaagaag 1200
gaagacactg accgctccca gtccagatct ccatcccgct ccgtgtcaaa ggagcgggaa 1260
catgccaagt ctgaatccag ccagagggaa ggtcgaggag agagtgagaa tgctggcacc 1320
aatcaggaga cccggtccag gtcgagatcc aattccaaat cgaaaccaaa ccttccatca 1380
gaatcacgct ccagatcaaa gtcagcttca aaaacccgat ctcggtccaa gtctagatcc 1440
aggtctgctt ccagatcgcc ctcccgatct agatctaggt cccactcaag gtcttaatta 1500
acccagcttt ctt 1513

<210> 605
<211> 602
<212> DNA
<213> Homo sapiens

<400> 605
aaaaaagctg gcacccctgg tccaggtatg ggcttgctct caattttgcg caagttgaaa 60
agtgcaccag accaggaggt gagaatactt ctcctgggct tggataatgc tggcaagacc 120
actcttatga agcagcttgc atctgaagac atcagccaca tcacacctac acagggtttc 180
aacatcaaaa gtgtacaatc acaaggtttt aaactgaatg tatgggacat tggtggacag 240
aggaaaatca gaccatactg gaagaattat tttgaaaata ccgatattct tatatatgta 300
atcgacagtg cagacagaaa aagatttgaa gagacgggtc aggaactagc ggaattactg 360
gaggaagaaa aactaagttg tgtgccagtg ctcatctttg ctaataagca ggatttgctc 420
acagcagccc ctgcctctga aattgcagaa ggactgaacc tgcataccat ccgcgaccga 480
gtctggcaga tccagtcttg ctcagctctc acaggagagg gcgttcagga tggcatgaac 540

tgggtctgca aaaatgtcaa tgcaaagaag aaaccaggcc caggcggcga cccagctttc 600
tt 602

<210> 606
<211> 581
<212> DNA
<213> Homo sapiens

<400> 606
aaaaaagctg gcacccctgg tccaggtatg gtgagaatgg ctggagcaac acctgttttt 60
attcccctga gatctaaacc tgtttatgga aaaagatggt ctagttctga ctggacatta 120
gatcctcaag aactggaaag taaatttaat cccaaaacca aagctattat actaaatact 180
ccacataacc cacttggcaa ggtgtataac agagaggaac tgcaagtaat tgctgacctt 240
tgcatcaaat atgacacact ctgcatcagt gatgaggttt atgaacggct tgtatattct 300
ggaaataagc acttaaaaat agctactttt ccaggtatgt gggagagaac aataacaata 360
ggaagtgctg gaaagacttt cagtgtaact ggctggaagc ttggctggtc cattggtcca 420
aatcatttga taaaacattt acagacagtt caacaaaaca cgatttatac ttgtgcaact 480
cctttacagg aagccttggc tcaagctttc tggattgaca tcaagcgcat ggatgaccca 540
ggaatgttac ttccaggccc aggcggcgac ccagctttct t 581

<210> 607
<211> 1320
<212> DNA
<213> Homo sapiens

<400> 607
   aaaaaagcag gcatggccct gctgcactcc ggccgcgtcc tccccgggat cgccgccgcc 60
   ttccacccgg gcctcgccgc cgcggcctct gccagagcca gctcctggtg gacccatgtg 120
   gaaatgggac ctccagatcc cattctggga gtcactgaag cctttaagag ggacaccaat 180
   agcaaaaaga tgaatctggg agttggtgcc taccgggatg ataacggaaa gccttacgtg 240
   ctgcctagcg tccgcaaggc agaggcccag attgccgcaa aaatttggac aaggaatacc 300
   tgcccattgg gggactggct gaattttgca aggcatctgc agaactagcc ctgggtgaga 360
   acagcgaagt cttgaagagt ggccggtttg tcactgtgca gaccatttct ggaactggag 420
   ccttaaggat cggagccagt tttctgcaaa gattttttaa gttcagccga gatgtctttc 480
   tgcccaaacc aacctgggga aaccacacac ccatcttcag ggatgctggc atgcagctac 540
   aaggttatcg gtattatgac cccaagactt gcggttttga cttcacaggc gctgtggagg 600
   atatttcaaa aataccagag cagagtgttc ttcttctgca tgcctgcgcc cacaatccca 660
   cgggagtgga cccgcgtccg gaacagtgga aggaaatagc aacagtggtg aagaaaagga 720
   atctctttgc gttctttgac atggcctacc aaggctttgc cagtggtgat ggtgataagg 780
   atgcctgggc tgtgcgccac ttcatcgaac agggcattaa tgtttgtctc tgccaatcat 840
   atgccaagaa catgggctta tatggtgagc gtgtaggagc cttcactatg gtctgcaaag 900
   atgcggatga agccaaaagg gtagagtcac agttgaagat cttgatccgt cccatgtatt 960
   ccaaccctcc cctcaatggg gcccggattg ctgctgccat tctgaacacc ccagatttgc 1020
   gaaaacaatg gctgcaagaa gtgaaaggca tggctgaccg catcattggc atgcggactc 1080
   aactggtctc caacctcaag aaggagggtt ccacccacaa ttggcaacac atcaccgacc 1140
   aaattggcat gttctgtttc acagggctaa agcctgaaca ggtggagcgg ctgatcaagg 1200
   agttctccat ctacatgaca aaagatggcc gcatctctgt ggcaggggtc acctccagca 1260
   acgtgggcta ccttgcccat gccattcacc aggtcaccaa ttaattaacc cagctttctt 1320

<210> 608
<211> 262
<212> DNA
<213> Homo sapiens

<400> 608
aaaaaagcag gcatgcccaa gtgtcccaag tgcaacaagg aggtgtactt cgccgagagg 60
gtgacctctc tgggcaagga ctggcatcgg ccctgcctga agtgcgagaa atgtgggaag 120
acgctgacct ctgggggcca cgctgagcac gaaggcaaac cctactgcaa ccacccctgc 180
tacgtagcca tgtttgggcc taaaggcttt gggcggggcg gagccgagag ccacactttc 240
aattaattaa cccagctttc tt 262

<210> 609
<211> 379
<212> DNA
<213> Homo sapiens

<400> 609
aaaaaagcag gcatgaaggc gaagcggcgg cggccggact tggatgagat tcaccgcgag 60
ctgcggcctc agggatccgc acgaccccag cccgacccaa acgccgagtt cgaccccgac 120
ctgccagggg gcggtctgca ccgctgtctg gcctgcgcga ggtacttcat cgattccacc 180
aacctgaaga cccacttccg atccaaagac cacaagaaaa ggctgaagca gctgagcgtc 240
gagccctaca gtcaggaaga ggcggagagg gcagcgggta tgggatccta tgtgcccccc 300
aggcggctgg cagcgcccac ggaagtgtcc actgaggtcc ctgagatgga tacctctact 360

taattaaccc agctttctt                                                379

<210>  610

<211>  786

<212>  DNA

<213>  Homo sapiens


<400>  610
aaaaaagcag gcatggaatc cgaaatggaa acgcagagcg ccggggcaga ggagggcttt      60

acccaggtca cccgcaaggg tggccgacgg gcgaagaaac gacaggctga acagctgtcc     120

gcagcaggag agggcgggga tgcgggccgc atggacacag aggaggccag gccggcgaag     180

aggcccgtct tcccacccct ctgtggggac gggctcctga gtgggaaaga agaaacaagg     240

aaagttccag tcccagctaa cagatacaca ccattgaaag aaaactggat gaagatattt     300

actcctattg tggaacattt gggacttcag atacgcttta acttgaaatc aaggaatgta     360

gaaatcagga cttgtaaaga aaccaaggat gttagtgctc tgacaaaagc agctgatttt     420

gtgaaagctt ttattctcgg ctttcaggtg gaggatgcac ttgccctcat caggttggat     480

gacctcttcc tagagtcttt tgaaattaca gatgttaaac ccctaaaggg agaccatcta     540

tccagggcaa taggaagaat cgctggcaaa ggaggaaaaa ccaaattcac catagagaat     600

gtgacacgga caaggatagt tttggctgat gtgaaagttc acatccttgg ctccttccaa     660

aatatcaaga tggcaagaac tgccatttgc aacctaatct tgggaaatcc tccttccaag     720

gtttatggca atattcgagc tgtggctagc agatcagcga tcgattttaa ttaacccagc     780

tttctt                                                                786

<210>  611

<211>  316

<212>  DNA

<213>  Homo sapiens


<400>  611
aaaaaagcag gcatggccga cgaaaagccc aaggaaggag tcaagactga gaacaacgat      60

catattaatt tgaaggtggc ggggcaggat ggttctgtgg tgcagtttaa gattaagagg     120

catacaccac ttagtaaact aatgaaagcc tattgtgaac gacagggatt gtcaatgagg     180

cagatcagat tccgatttga cgggcaacca atcaatgaaa cagacacacc tgcacagttg     240

gaaatggagg atgaagatac aattgatgtg ttccaacagc agacgggagg tgtctattaa     300

ttaacccagc tttctt                                                     316

<210>  612

<211>  568

<212>  DNA

<213>  Homo sapiens


<400>  612
aaaaaagcag gcatggctaa aacagcaatg gcctacaagg aaaaaatgaa ggagctgtcc      60

atgctgtcac tgatctgctc ttgcttttac ccggaacctc gcaacatcaa catctatact     120

tacgatgata tggaagtgaa gcaaatcaac aaacgtgcct ctggccaggc ttttgagctg     180

atcttgaagc caccatctcc tatctcagaa gccccacgaa ctttagcttc tccaaagaag     240

aaagacctgt ccctggagga gatccagaag aaactggagg ctgcagagga aagaagaaag     300

tctcaggagg cccaggtgct gaaacaattg gcagagaaga gggaacacga gcgagaagtc     360

cttcagaagg ctttggagga gaacaacaac ttcagcaaga tggcggagga aaagctgatc     420

ctgaaaatgg aacaaattaa ggaaaaccgt gaggctaatc tagctgctat tattgaacgt     480

ctgcaggaaa aggagaggca tgctgcggag gtgcgcagga acaaggaact ccaggttgaa     540

ctgtctggtt aattaaccca gctttctt                                        568

<210>  613

<211>  289

<212>  DNA

<213>  Homo sapiens


<400>  613
aaaaaagcag gcatgagttt acccctcaat cccaaacctt tcctcaatgg actaacagga      60

aagccagtga tggtgaaact taagtgggga atggagtaca agggctatct ggtatctgta     120

gatggctaca tgaacatgca gcttgcaaat acagaagaat acatagatgg agctttgtct     180

ggacatctgg gtgaagtttt aataaggtgt aataatgtcc tttatatcag aggtgtggaa     240

gaagaggaag aagatgggga aatgagagat taattaaccc agctttctt                 289

<210>  614

<211>  470

<212>  DNA

<213>  Homo sapiens


<400>  614
aaaaaagcag gccttcatat tgttgtacat gcagatgaat tagaagaact tatcaactac      60

tatcaggatc gtggctattt tgaagagctg atcaccatgt tggaagcagc actgggactt     120

gagcgagctc acatgggaat gtttactgaa ttagctattc tatactctaa atttaagcct     180

cagaaaatga gggagcacct ggagctgttc tggtctagag tgaatattcc caaggtgcta 240
agagctgcag aacaagctca tctttgggca gaactggtgt ttttgtatga caagtatgaa 300
gaatatgata atgccataat taccatgatg aatcatccaa ctgatgcctg gaaagaaggg 360
caattcaaag atatcattac caaggttgcc aatgtggaac tatactacag agcaatacag 420
ttctacttag aattcaagcc tctgttgtta aattgagacc cagctttctt 470

<210> 615
<211> 1216
<212> DNA
<213> Homo sapiens

<400> 615
aaaaaagcag gcatgccggg ccggagttgc gtcgccttag ccctcctggc tgccgccgtc 60
agctgtgccg tcgcgcagca cgcgccgccg tggacagagg actgcagaaa atcaacctat 120
cctccttcag gaccaacgta cagaggtgca gttccatggt acaccataaa tcttgactta 180
ccaccctaca aaagatggca tgaattgatg cttgacaagg caccagtgct aaaggttata 240
gtgaattctc tgaagaatat gataaataca ttcgtgccaa gtggaaaaat tatgcaggtg 300
gtggatgaaa aattgcctgg cctacttggc aactttcctg gcccttttga agaggaaatg 360
aagggtattg ccgctgttac tgatatacct ttaggagaga ttatttcatt caatattttt 420
tatgaattat ttaccatttg tacttcaata gtagcagaag acaaaaaagg tcatctaata 480
catgggagaa acatggattt tggagtattt cttgggtgga acataaataa tgatacctgg 540
gtcataactg agcaactaaa acctttaaca gtgaatttgg atttccaaag aaacaacaaa 600
actgtcttca aggcttcaag ctttgctggc tatgtgggca tgttaacagg attcaaacca 660
ggactgttca gtcttacact gaatgaacgt ttcagtataa atggtggtta tctgggtatt 720
ctagaatgga ttctgggaaa gaaagatgcc atgtggatag ggtttctcgc tagaacagtt 780
ctggaaaata gcacaagtta tgaagaagcc aagaatttat tgaccaagac caagatattg 840
gccccagcct actttatcct gggaggcaac cagtctgggg aaggttgtgt gattacacga 900
gacagaaagg aatcattgga tgtatatgaa ctcgatgcta agcagggtag atggtatgtg 960
gtacaaacaa attatgaccg ttggaaacat cccttcttcc ttgatgatcg cagaacgccc 1020
gcaaagatgt gtctgaaccg caccagccaa gagaatatct catttgaaac catgtatgat 1080
gtcctgtcaa caaaacctgt cctcaacaag ctgaccgtat acacaacctt gatagatgtt 1140
accaaaggtc aattcgaaac ttacctgcgg gactgccctg acccttgtat aggttgttaa 1200
ttaacccagc tttctt 1216

<210> 616
<211> 514

<212>  DNA

<213>  Homo sapiens


<400>  616
aaaaaagcag gcatggggtg ctgctacagc agcgagaacg aggactcgga ccaggaccga      60

gaggagcgga agctgctgct ggaccctagc agccccccta ccaaagctct caatggagcc     120

gagcccaact accacagcct gccttccgct cgcactgatg tgcaggccct gctctcttcc     180

atccttgcca agacagccag caacatcatt gatgtgcctg ctgcagactc acagggcatg     240

gagcagcatg agtacatgga ccgtgccagg cagtacagca cccgcttggc tgtgctgagc     300

agcagcctga cccattggaa gaagctgcca ccgctgccgt ctcttaccag ccagccccac     360

caagtgctgg ccagtgagcc catcccgttc tctgatttgc agcaggtctc caggatagct     420

gcttatgcct acagtgcact ttctcagatc cgtgtggacg caaaagagga gctggttgta     480

cagtttggga tcccttaatt aacccagctt tctt                                514

<210>  617

<211>  272

<212>  DNA

<213>  Homo sapiens


<400>  617
aaaaaagctg gcacccctgg tccaggtatg tcttatcaac agcagcaatg taagcagccc      60

tgccagccac ctcctgtgtg ccgcctgcca aagcgtccag agccatgtcc acccctgaag     120

tgccctgagc cctgcccacc accaaagtgt ccacagccct gcccacctca gcagtgccag     180

cagaaatatc ctctggtgac accttcccca ccctgccagc caaagtgtcc acccaagagc     240

aagccaggcc caggcggcga cccagctttc tt                                  272

<210>  618

<211>  83

<212>  DNA

<213>  Homo sapiens


<400>  618
ggccaggggc atggcgacag gcccccctcc ccacttggcc accaggctgc ctgccagggg      60

agacctggga cctggacacc cag                                             83

<210>  619

<211>  322

<212>  DNA

<213>  Homo sapiens

<400>  619
tgtacaaaaa agcaggcatg aggaccctcg ccatccttgc tgccattctc ctggtggccc      60

tgcaggccca ggctgagcca ctccaggcaa gagctgatga ggttgctgca gccccggagc     120

agattgcagc ggacatccca gaagtggttg tttcccttgc atgggacgaa agcttggctc     180

caaagcatcc aggctcaagg aaaaacatgg actgctattg cagaatacca gcgtgcattg     240

caggagaacg tcgctatgga actgcatcta ccagggaaga ctctgggcat tctgctgtta     300

attaacccag ctttcttgta ca                                              322

<210>  620

<211>  548

<212>  DNA

<213>  Homo sapiens

<400>  620
tgtacaaaaa agcaggcatg gcacccaaga gggccaagag aaggacagta gagggcggaa      60

gctccagcgt cttctccatg ttcgaccaga ctcagatcca ggagttcaaa gaggccttca     120

ctgtgatcga ccagaaccgt gatggtatta tagacaagga ggaccttcgg gacaccttcg     180

cagccatggg ccgcctcaat gtgaagaatg aggagttgga tgccatgatg aaggaagcca     240

gcggtcccat caacttcacc gtcttcctga ccatgttcgg ggagaagctc aagggtgccg     300

accctgagga tgtgatcacc ggagccttca aggtcttgga ccctgaggga aagggcacca     360

tcaagaagaa gttcctggag gagctgctga ccacgcagtg tgaccgcttc tcccaggagg     420

agatcaagaa catgtgggcg gccttccccc ccgacgtggg cggcaacgtc gactacaaaa     480

acatctgcta cgtcatcacg cacggcgacg ccaaggacca ggattaatta acccagcttt     540

cttgtaca                                                              548

<210>  621

<211>  287

<212>  DNA

<213>  Homo sapiens

<400>  621
cacccctggt ccaggtatgg cccacaagca gatctactac tcggacaagt acttcgacga      60

acactacgag tacctgcatg ttatgttacc cagagaactt tccaaacaag tacctaaaac     120

tcatctgatg tctgaagagg agtggaggag acttggtgtc caacagagtc taggctgggt     180

tcattacatg attcatgagc cagaaccaca tattcttctc tttagacgac ctcttccaaa 240

agatcaacaa aaaccaggcc caggcggcga cccagctttc ttgtaca 287

<210> 622

<211> 559

<212> DNA

<213> Homo sapiens

<400> 622
ttgtcagcag agcttcacca ttgaagggct ggcggttggg gttattcgca acggcgactg 60

gctggaattc atgtcgacca cctttgactt caaaatcaag tatgctgaga ctaaggccca 120

tgccaaggca attgagatgg aattgaggca gatggaggtg gcccaggcca atcgacacat 180

gtccctgctg acagccttta tgcctgacag ctttctttgg ccaggtgggg accatgactg 240

cgttctggtg ctgttgctca tgcctcgtct tatttgcaag gcagagctta ttcggaagca 300

ggcccaggag aagtttgaac taagtgagaa ctgtttagaa gcgcctgggc tgcgaggagc 360

tgctggggag caacttaact ttgctgctgg acttggggac ttgtttagcc ttctgaggcc 420

acgcttcacc gctttgagca tgccctttta atgcagtgtg gatgtgtata aaaaaggggc 480

agcctgtccc ctgagatgag tggctatgag cgctctttgg attccctaat tgactgttgc 540

acaaggatca cctggatgg 559

<210> 623

<211> 557

<212> DNA

<213> Homo sapiens

<400> 623
tcgtcagcag agcttcacca ttgaagggct ggcggttggg gttattcgca acggcgactg 60

gctggaattc atgtcgacca aaaagaaccg ctggaccagc attgaggagc ggcgcatcca 120

ccaggagagc gagctgcact cctacctttc caggctaatt gccgcgaacc gtgaaaggaa 180

cctgaaaaag tcccagcgaa cccacgaggg tgatgaggac aacagccacg tccggcccca 240

gcaggcctgc tttgaggcca accacgacag gtccatggcg gacatggacg agcttttttc 300

tcaggtggat gagaagagga agaaccgaga catccccgac tacttgtgtg gcaagatcag 360

tttagagctg atgcgggagc cgtgcatcac gcccagtggt atcacctacg accgcaggga 420

catcgaggag cacctgcagc gtgtgggtca tttagacccc gagacccgag ccctctgacc 480

caggaacagc tcatccacaa cttggtatga gaggaggtat tgcgcattca tctctgagag 540

tgctgggtgg ggactcg 557

<210>  624

<211>  564

<212>  DNA

<213>  Homo sapiens

<400>  624
tcgtcagcag agcttcacca ttgaagggct ggcggttggg gttattcgca acggcgactg      60

gctggaattc atgtcgacct ccagggtgct gctggtcatc gacgagccgc acaccgactg     120

ggcaaaatac ttcaaaggga aaaagatcca tggagaaatt gacattaaag taaaacaggc     180

cgaattctct gatctcaacc ttgtggccca tgccaatggt ggattctctg tggatatgga     240

agttcttcgg aatggggtga aggtcgtgcg gtctctgaag ccggattttg tgctgatccg     300

ccagcacgcc ttcagcatgg cacgcaacgg agactaccgc agtttggtca ttgggctgca     360

gtatgctgga atccccagtg ttaactcctt gcattctgtc tacaactttc gtgacaagcc     420

ctgggtgttt gcccagatgg ttcgactgca taagaaactg gggacaggag aatttcctct     480

aattgatcag accttttacc ccaattacaa aaaaatgtta gcagtccacg tactccgggg     540

tttgaagatt ggcaccacac tctg                                            564

## Description

The present invention relates to a method for identification of a protein-protein interaction of protein interaction partners in a disease-related network of proteins comprising the steps of (a) identifying nucleic acid molecules by at least partial 5' sequencing and, optionally, additionally adding recombinantly cloned and sequenced nucleic acid molecules, wherein said nucleic acid molecules encode a selection of proteins suspected to contain one or several of said interaction partners and wherein said nucleic acid molecules are annotated with a positional information; (b) in frame cloning of the nucleic acid molecules of step (a) into prey and bait vectors, wherein one copy of each nucleic acid molecule is cloned into said prey vector and a second copy of each nucleic acid molecule is cloned into said bait vector; (c) transforming a first suitable host cell with the prey vector obtained in step (b) and a second suitable host cell with the bait vector obtained in step (b), wherein said first and said second host cell have a different genetic constitution and can be mated; (d) mating the first suitable host cell of step (c) with the second suitable host cell of step (c), and expressing the proteins encoded by the nucleic acid molecules obtained in step (b); (e) selecting the mated host cell obtained in step (d) on the basis of a selection advantage which is caused by the protein-protein interaction between the protein interaction partner encoded by the nucleic acid molecule of the prey vector contained in said cell and the protein interaction partner encoded by the nucleic acid molecule of the bait vector contained in said cell; and (f) identifying with the positional information obtained in step (a) the protein interaction partners of step (e) and thereby identifying the protein-protein interaction.

Furthermore, the present invention relates to a method for identification of a protein-protein interaction of protein interaction partners in a disease-related network of proteins comprising the steps of (a) identifying nucleic acid molecules by partial 5' sequencing and, optionally, additionally adding recombinantly cloned and sequenced nucleic acid molecules, wherein said nucleic acid molecules encode a selection of proteins suspected to contain one or several of said interaction partners and wherein said nucleic acid molecules are annotated with a positional information; (b) in frame cloning of the nucleic acid molecules of step (a) into prey and bait vectors, wherein one copy of each nucleic acid molecule is cloned into said prey vector and a second copy of each nucleic acid molecule is cloned into said bait vector; (c) transforming a first suitable host cell with the prey vector obtained in step (b) and a second suitable host cell with the bait vector obtained in step (b), wherein said first and said second host cell have a different genetic constitution and can be mated; (d) pooling at least two of the second suitable host cells obtained in step (c); (e) mating the first suitable host cell of step (c) with the at least two second suitable host cells of step (d), and expressing the proteins encoded by the nucleic acid molecules obtained in step (b); (f) selecting the mated host cell obtained in step (e) on the basis of a selection advantage which is caused by the protein-protein interaction between the protein interaction partner encoded by the nucleic acid molecule of the prey vector contained in said cell and the protein interaction partner encoded by the nucleic acid molecule of the bait vector contained in said cell; and (g) identifying with the positional information obtained in step (a) the protein interaction partners of step (f) and thereby identifying the protein-protein interaction.

Several documents are cited throughout the text of this specification. The disclosure content of the documents cited herein (including any manufacture's specifications, instructions, etc.) is herewith incorporated by reference. The present invention is based on scientific experiments which have been performed on biological specimen derived from diseased patients. Patients have given their consent to use the specimen for the study which is disclosed in the present invention. In case of deceased patients, the consent has been given by a relative.

With the identification of >35.000 genes in the human genome the challenge arises to assign biological function to all proteins and to link these proteins to physiological pathways and disease processes. Since protein-protein interactions (PPIs) play a role in most events in a cell, clues to the function of an unknown protein can be obtained by investigating its interaction with other proteins whose function are already known. Thus, if the function of one protein is known, the function of the binding partners can be inferred (deduced). This allows the researcher to assign a biological function to uncharacterized proteins by identifying protein-protein interactions.

Elucidation of protein-protein interactions is particularly desired when it comes to the generation of new drugs. For many diseases, the available drug portfolio is insufficient or inappropriate to provide a cure or to prevent onset of the disease.

Most cellular processes in the human organism are regulated by protein-protein interactions which taken together form a protein-protein interaction networks. The dysfunction of such regulatory networks often fundamentally relates to diverse human disorders. In recent years, much effort has been devoted to developing protein microarrays for the identification of PPls or other specific biochemical activities, because they allow parallel detection of addressable elements in a single experiment (*40-43*). 5,800 purified yeast proteins, e.g., were printed onto glass slides and employed in proof-of-principle experiments for the detection of novel calmodulin-and phospholipid-interacting proteins by Zhu *et al.* (*44*). Moreover, protein arrays were used for identifying novel targets of phosphodiesterases, methyltransferases (*45*) and protein kinases (*46*, *47*). All of this shows that the production of protein arrays is basically feasible and that they could be used for systematic large-scale interaction and activity screens. One major drawback, however, of protein arrays to date is that they only work with purified proteins. Protein purification, however, is often difficult, time consuming and expensive.

Furthermore, protein/protein interactions can be identified by an affinity purification approach followed by MS-based protein identification *(48, 49).* This includes the so-called "TAP-tag" technology. For that approach, a gene-specific cassette containing a tandem affinity purification tag was inserted by a homologous recombination process in yeast at the 3' end of the corresponding gene. After growing cells, protein expression and formation of protein-protein interactions, complexes were purified using affinity purification and analyzed by MALDI-TOF MS. The above-mentioned techniques, however, only allow the identification of stable highly abundant protein complexes. Additionally, protein array technologies used in the prior art often are based on expressed proteins which are not modified by post-translational modifications. Said modifications, however, are important for the identification of protein interactions in networks of proteins. To carry out similar experiments in mammalian systems large amounts of material is required.

The lack of knowledge about protein interaction networks involved in pathological processes results in a drawback with respect to the development of disease therapies related to said networks.

As discussed, the techniques and methods described above show major disadvantages with respect to establishing biologically relevant protein networks. Furthermore, the prior art did not even disclose methods that might be easily handled and automated with regard to establishing protein interaction partners in protein networks related to diseases. Thus, the technical problem underlying the present invention therefore was to provide such methods.

The solution to said technical problem is achieved by providing the embodiments characterized in the claims.

Accordingly, the present invention relates to a method for identification of a protein-protein interaction of protein interaction partners in a disease-related network of proteins comprising the steps of (a) identifying nucleic acid molecules by at least partial 5' sequencing and, optionally, additionally adding recombinantly cloned and sequenced nucleic acid molecules, wherein said nucleic acid molecules encode a selection of proteins suspected to contain one or several of said interaction partners and wherein said nucleic acid molecules are annotated with a positional information; (b) in frame cloning of the nucleic acid molecules of step (a) into prey and bait vectors, wherein one copy of each nucleic acid molecule is cloned into said prey vector and a second copy of each nucleic acid molecule is cloned into said bait vector; (c) transforming a first suitable host cell with the prey vector obtained in step (b) and a second suitable host cell with the bait vector obtained in step (b), wherein said first and said second host cell have a different genetic constitution and can be mated; (d) mating the first suitable host cell of step (c) with the second suitable host cell of step (c), and expressing the proteins encoded by the nucleic acid molecules obtained in step (b); (e) selecting the mated host cell obtained in step (d) on the basis of a selection advantage which is caused by the protein-protein interaction between the protein interaction partner encoded by the nucleic acid molecule of the prey vector contained in said cell and the protein interaction partner encoded by the nucleic acid molecule of the bait vector contained in said cell; and (f) identifying with the positional information obtained in step (a) the protein interaction partners of step (e) and thereby identifying the protein-protein interaction.

Furthermore, the present invention relates to a method for identification of a protein-protein interaction of protein interaction partners in a disease-related network of proteins comprising the steps of (a) identifying nucleic acid molecules by partial 5' sequencing and, optionally, additionally adding recombinantly cloned and sequenced nucleic acid molecules, wherein said nucleic acid molecules encode a selection of proteins suspected to contain one or several of said interaction partners and wherein said nucleic acid molecules are annotated with a positional information; (b) in frame cloning of the nucleic acid molecules of step (a) into prey and bait vectors, wherein one copy of each nucleic acid molecule is cloned into said prey vector and a second copy of each nucleic acid molecule is cloned into said bait vector; (c) transforming a first suitable host cell with the prey vector obtained in step (b) and a second suitable host cell with the bait vector obtained in step (b), wherein said first and said second host cell have a different genetic constitution and can be mated; (d) pooling at least two of the second suitable host cells obtained in step (c); (e) mating the first suitable host cell of step (c) with the at least two second suitable host cells of step (d), and expressing the proteins encoded by the nucleic acid molecules obtained in step (b); (f) selecting the mated host cell obtained in step (e) on the basis of a selection advantage which is caused by the protein-protein interaction between the protein interaction partner encoded by the nucleic acid molecule of the prey vector contained in said cell and the protein interaction partner encoded by the nucleic acid molecule of the bait vector contained in said cell; and (g) identifying with the positional information obtained in step (a) the protein interaction partners of step (f) and thereby identifying the protein-protein interaction.

In accordance with the present invention, the term "protein interaction partner" relates to proteins and disease-related proteins that either directly interact with each other (direct interaction) or that interact via one or several protein bindings/interactions with each other. In the latter case, there is no direct contact between all interacting proteins.

The term "protein" as used in the present invention refers to a (poly)peptide. The term "(poly)peptide" refers alternatively to a peptide or to polypeptides. Peptides conventionally are covalently linked amino acids of up to 30 residues, whereas polypeptides comprise 31 and more amino acid residues.

The term "disease related network of proteins" as used in the present invention relates to a network of proteins of direct and indirect protein interaction partners, wherein at least one protein of said network is known to be the causative agent of a disease or known to be involved in the onset or progression of a disease.

Preferably, said protein is selected from table S6.

The term "recombinantly cloned and sequenced nucleic acids" refers to nucleic acids which are obtained by recombinatorial cloning. Said recombinatorial clones might be obtained, for example, by using the Gateway recombinational cloning system. ORFs flanked by the recombinational sites B1 and B2 are directionally cloned into a P1::ccdB::P2 donor vector using the BP reaction.The BP Reaction is a recombination reaction between a PCR fragment (ORF) and a Donor vector. For the reaction to take place, the gene of interest is amplified with the help of an attB tagged primer pair. The donor vector includes attP sites. The PCR product that includes the attB sites combines with the donor vector that includes the attP sites resulting in the formation of an entry clone. This integration reaction between the attB and the attP sites forms the basis of this reaction. The resulting entry clone contains the gene of interest flanked by attL sites (L1, L2) and can be . selected, for example, by using the kanamycine-resistance marker (Km^{r}). ORFs flanked in the Entry clone by L1 and L2 sites are directionally transferred into R1::ccdB::R2 destination vectors using the LR reaction. The resulting destination clones are selected using the ampicillin resistance marker (Amp^{r}).

The term "in frame cloning" relates to the cloning of a nucleic acid molecule in such a frame into an expression vector that allows the expression of the respective biological relevant protein.

Preferably, said nucleic acid molecule is DNA, more preferably cDNA or genomic DNA.

The term "bait vector" relates to any vector which is used for the expression of a protein fused with a DNA-binding domain.

Preferably, said DNA-binding domain is a Gal4 DNA-binding domain.

The term "prey vector" relates to any vector which is used for the expression of a protein fused with a transcription-activation domain.

Preferably, said transcription-activation domain is a LexA transcription-activation domain.

Preferably, the bait and prey vectors are selected from the group consisting of pGAD424, pGAD424(x), pGAD424rev(x), pGAD-C(1,2,3), pOAD, pC-ACT.1, pC-ACT.2, pGADGH, pGAD10, pACT1, pACT2, pPC86, pNGAD-L(1,2,3), pJG4-5, pMW102, pMW104, pNB42(1,2,3), pNB42 (1R,2R,3R), pVP16, pSD.06 (a,b,c), pWITCH(T), pWITCH(U), pASV(x), pDEST™22, pGBT9, pGBT9(x), pGBT9rev(x), pGBD-C(1,2,3), pGBDU-C(1,2,3), pGBDK-C(1,2,3), pGBREP, pOBD-2, pGB-MEL1, pAS1, pAS2, pODB8, pPC62, pPC97, pCD.1, pCD.2, pNGBD-U(1,2,3), pBTM116, pBTM117, pFBL23, pEG202, pJK202, pMW101, pMW103, pGILDA, pGKS3, pGKS6, pGKS8, pBL1, and pDEST™32.

The term "first suitable host cell" relates to any cell that can be used for mating with the "second suitable host cell"

The term "second suitable host cell" relates to any cell that can be used for mating with the "first suitable host cell".

The term "mating" refers to the process of fusion between the first and the second suitable host cell. Preferably, said fusion is achieved by mating between the two cells. For example, S. cerevisiae haploid cells of different mating types. S. cerevisiae haploid cells can be of one of two mating types, Roman a or Greek α. Roman haploids secrete a pheromone that in Greek haploids stimulates processes that lead to mating and Greek haploids secrete a pheromone that stimulates Roman haploids to mate with Greeks. Genes determining Greek or Roman status are alleles of the same locus, the MAT locus.
Diploid cells resulting from the mating of Romans and Greeks do not make any pheromones or pheromone receptors. Haploid specific genes are repressed by cooperative binding of a1 and α2 proteins, products of the MATa and MATα alleles, to a haploid-specific response element in the UAS regions of haploid-specific genes.

The term "selecting the mated host cell on the basis of a selection advantage" refers to a selection process where, for example, only a diploid suitable host cell resulting from the mating process is growing on, for example, SDII selection agar plates. This results in the creation of a diploid strain from mating the first suitable host cell with the second suitable host cell that allows the selection for growth in the absence of leucine, tryptophan, histidine or uracil, where the production of histidine and uracil are coupled to transcriptional activation under control of respective promotors.

The term "identifying with the positional information the protein interaction partners" relates to the determination of the protein identity throughout the present invention. This term comprises the 5' sequencing at the beginning of the screening procedure and the identification of the biological relevant encoded protein. This information is part of the clone identity in all subsequent steps of the present invention's method.

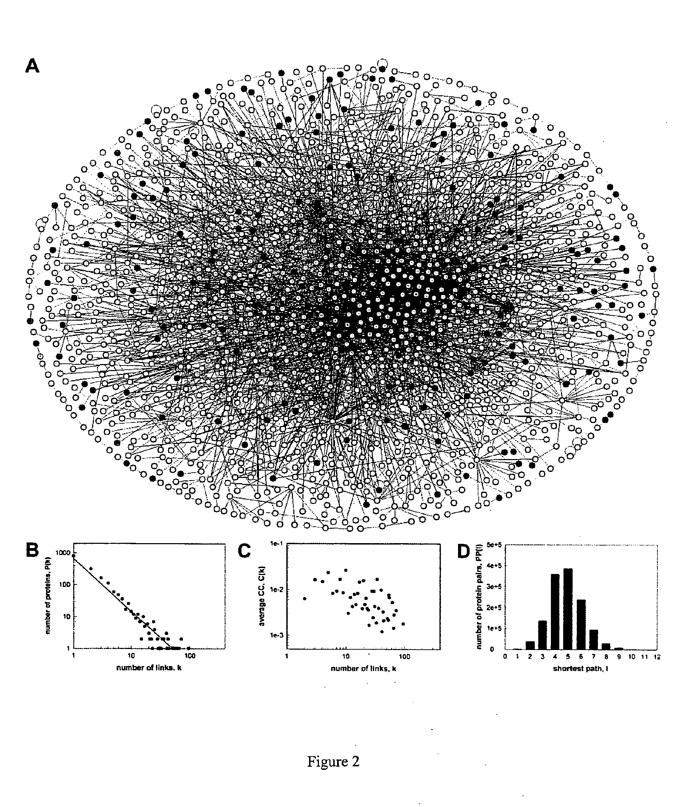

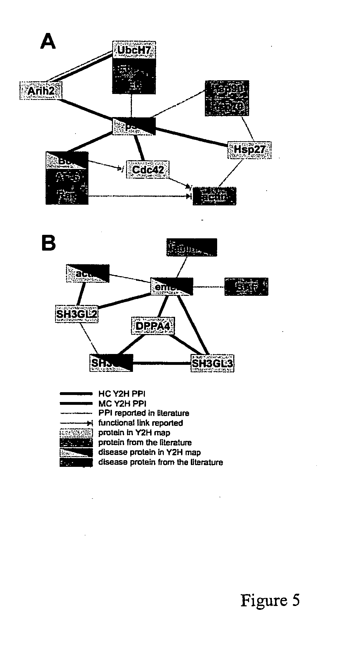

The term "thereby identifying the protein-protein interaction" relates to a method for, e.g. automatic, transmission of protein-protein interaction data from the experimental output into a electronic database. One embodiment of said method is further illustrated in Examples 3 and 4 and Figures 2 to 4. For example, a digitized photo of growing and/or β-galactosidase stained yeast cells is overlayed with an electronical visual grid, which is part of the Visual Grid (GPC Biotech) program. After a manual click on a field which covers stained and/or growing yeast cells, the respective information about a protein-protein information can be transferred to the electronic database. Biologically relevant protein-protein interactions are obtained by applying a bioinformatic scoring system involving loop motif analysis and experimental verification. Biologically relevant protein-protein interactions are interactions which occur in a biologically cellular system under physiological conditions. Such biological relevant protein-protein interactions are further illustrated, for example, in the protein networks established for p53 and emerin shown in Figure 5.

The term "pooling at least two of the second suitable host cells" relates to the method of the present invention, wherein at least two, preferably at least four, more preferably at least 8 and most preferably at least 12 of the second suitable host cells are pooled before mating said cells with the first suitable host cells of step (c). This pooling is achieved, for example, by automated means. By pooling said cells, the method of the present invention can be carried out in a more efficient manner .

As has been outlined above and in other terms, the invention solves the recited technical problem by a highly reliable and reproducible method for the identification of protein-protein interactions of protein interaction partners in disease-related networks of proteins. In particular, as mentioned above, prior art methods rely on protein array and protein affinity purification technologies and on the high abundance of interacting proteins. However, as explained, said techniques only allowed the identification of relatively stable protein interactions formed in a cell or in an in vitro system and did not allow the unambiguous identification of temporarily occurring protein-protein interactions. Furthermore, said in vitro techniques lacked posttranslational protein modifications. In contrast to this, the method of the present invention also allows the identification of temporarily occurring protein-protein interactions, also including proteins of low abundance. Additionally, the in vivo yeast two hybrid system which is part of the present invention's method also has the potential to identify protein-protein interactions based on post-translational modifications, which are often important for protein interactions, in particular in a biological context.

The most encouraging result obtained in accordance with the present invention was the finding that 5' sequencing and in frame cloning of nucleic acid molecules encoding potential protein interaction partners highly improved the quality of the resulting protein-protein interaction data. The characterization of expression clones used in the present invention's method is a precondition for the elimination of the redundancy in the generated bait and prey library and it enables in frame cloning, which guarantees the expression of only biological relevant proteins. So far, the expression of artificial peptides and proteins was the major drawback in most screens for protein interactions. A considerable amount of artificial background information is produced, which handicaps the proper identification of biological relevant protein-protein interactions.

The characterisation of expression clones used in the present invention's method is the basis to create a non redundant matrix of expressing human proteins in yeast, that can be screened in an highly efficient array format. Interactions are identified because of their above mentioned positional information, i.e. identification of interactions does not require any further experiments such as plasmid isolation, PCR, retransformation or sequencing.

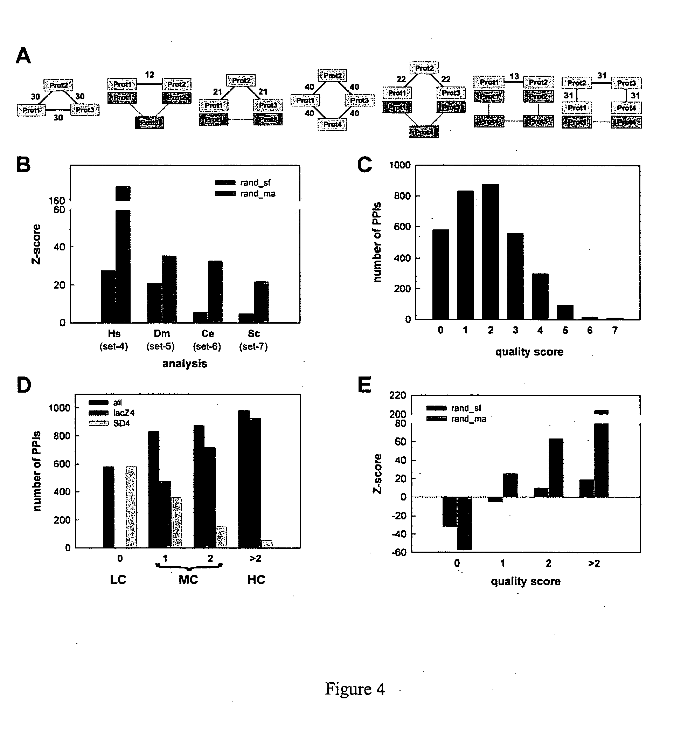

Finally, the method of the present invention can be automated, therefore further augmenting the quality of the resulting protein-protein interaction data.
The interaction network is statistically analysed by a novel quality scoring system allowing to score for biological confidence. One embodiment of carrying out said scoring system is further illustrated in Example 4 and Figure 4.

In order to carry out the invention, it is envisaged that the following step may be performed:
1. The E. coli hEX1 Library was arrayed in 384 well plates using a spotting robot. Clones obtained in step were used for isolation of plasmid DNA in the 384 well format using a liquid handling robot
2. Plasmid DNAs obtained in step (1) were sequenced from the 5' end.
3. A bioinformatic analysis was performed for the following topics using sequences obtained in step (2): a) Identification of the protein which was encoded by a certain cDNA. b) Frame identification of the ORF. c) Identification of the ORF start in the respective cDNA F d) Identification of a non redundant set of cDNAs expressing the longest ORF for every protein
4. Selected plasmid DNAs obtained in step (1) and analysed in step (3) were arrayed in 96 well plates using a pipetting robot
5. Aliquots of plasmid DNAs rearrayed in step (4) were transferred in 96 well plates using a multichannel pipetting system and digested using two different enzymes.
6. Restriction fragments obtained in step (5) were separated in agarose gels.
7. cDNAs obtained in step (6) were isolated from agarose gels and arrayed in the 96 well format
8. cDNAs obtained in step (7) were directionally cloned in the 96 well format in the appropriate bait- and prey vector
9. E. coli cells were transformed with plasmid DNAs obtained in step (8) and the cells were plated onto selective agar plates
10. Picking of 3-4 clones per cloned cDNA, automated isolation of plasmid DNA in 96 well format (step 1) and analysis using steps (5) and (6)
11. Based on the analysis of 3-4 obtained in step (10), one bait plasmid and one prey plasmid were selected for further transformation of yeast cells
12. Transformation of yeast cells using plasmid DNA obtained in step (10) in the 96 well format
13. Isolation of transformed different clones and generation of 96 well bait plates and 384 well prey plates
14. Replication of bait and prey plates obtained in step (13) using a pipetting robot
15. After over night growing, pooling of 8 bait clones and mixing with one set of the prey array in 384 plates obtained in step (14) representing all different prey clones, using pipetting robot.
16. Spotting of bait and prey mixtures onto YPD solid agar plates using a robotic system.
17.After mating and cell growing, transfer of cells obtained in step (16) onto selective agar plates with and without a nylon membrane.
18. Identification of grown cells on selective agar plates according to positional information.
19. Identification of ß-galactosidase staining of grown cells on nylon membranes according to positional information.
20. Identification of protein-protein interactions and transmission of protein-protein interaction data to a electronic database using digitized images of grown cells obtained in step (18), digitized images of stained cells obtained in step (19) and a visual grid computer software.
21. Quality scoring for the identification of biologically relevant interactions.

In a preferred embodiment of the present invention's method at least one of said protein interaction partners in the disease related network of proteins, when mutated or un-physiologically expressed, is involved in the etiology of a disease

Table S6 relates to proteins identified with the method of the present invention and their possible involvement in diseases.

In another preferred embodiment of the present invention's method said annotation of positional information is achieved by arraying the nucleic acid molecules.

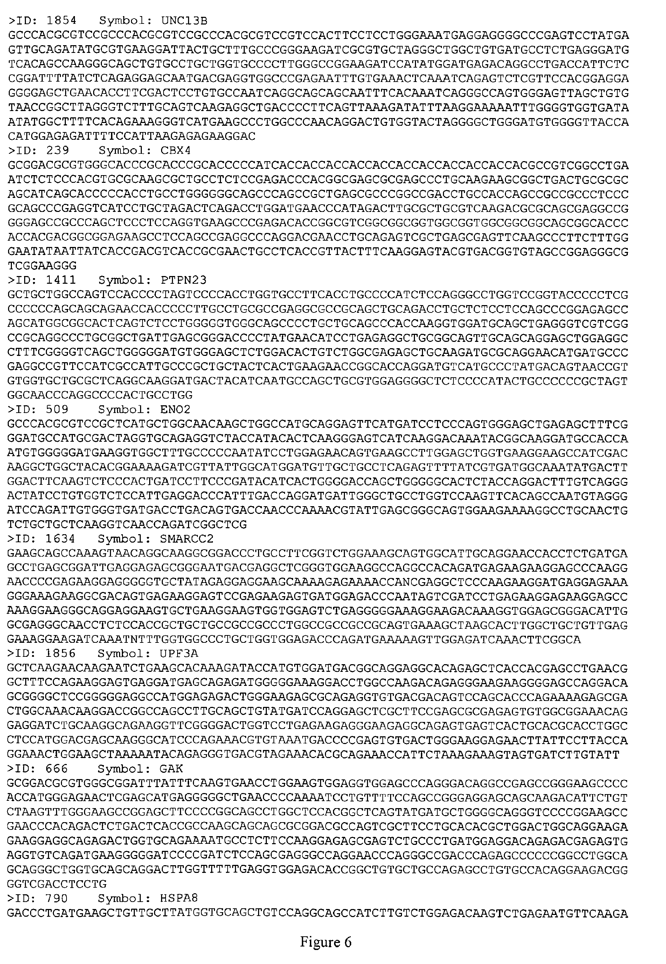

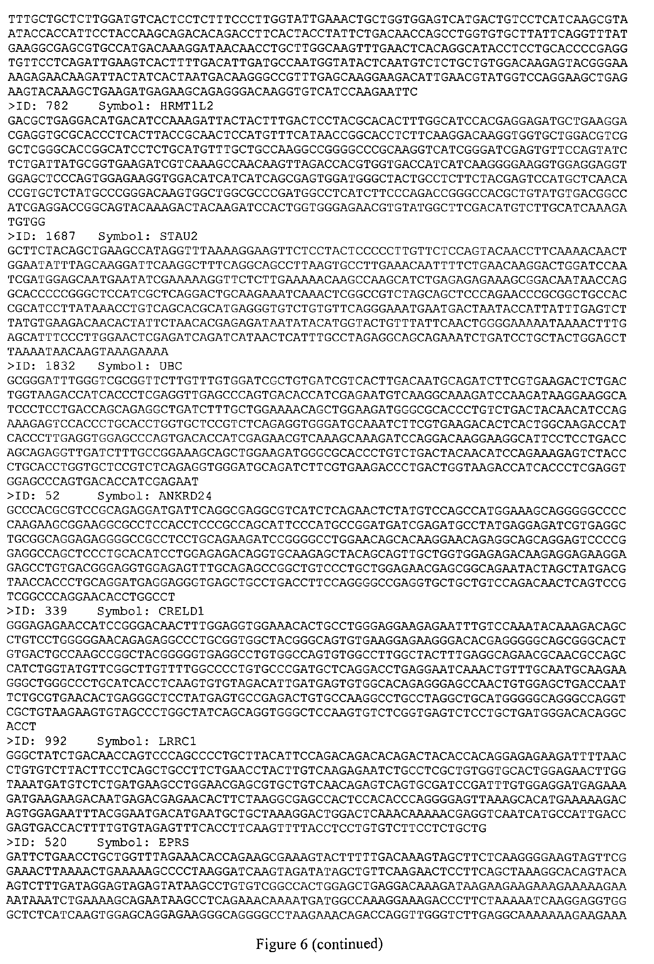

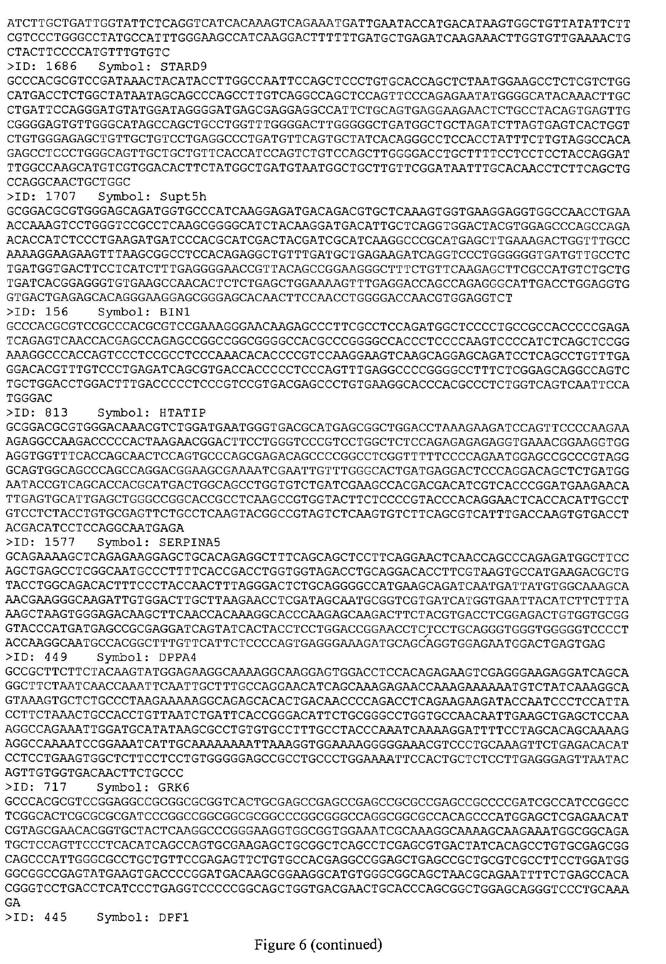

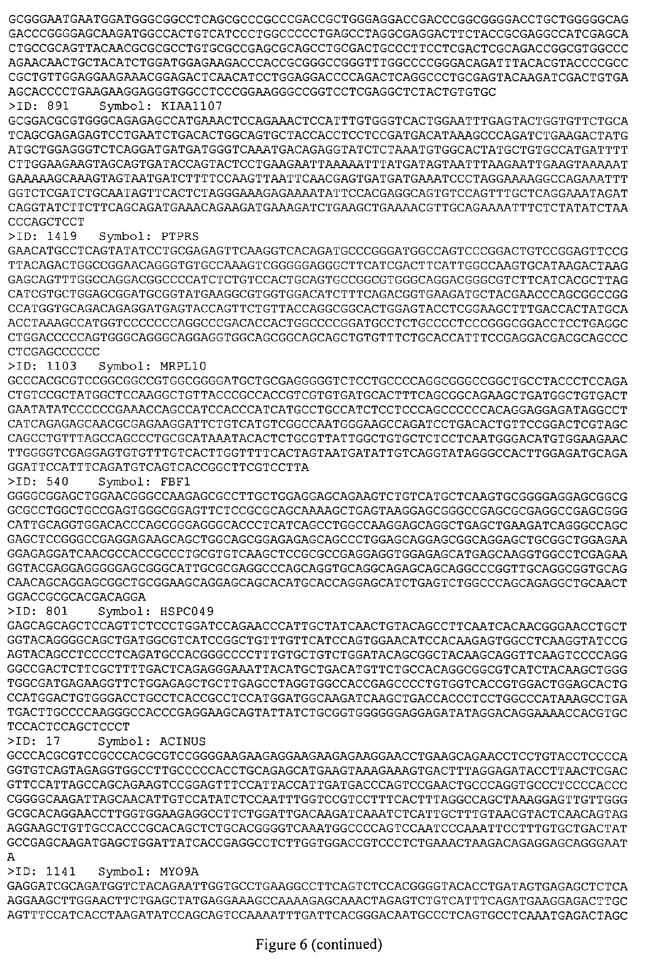

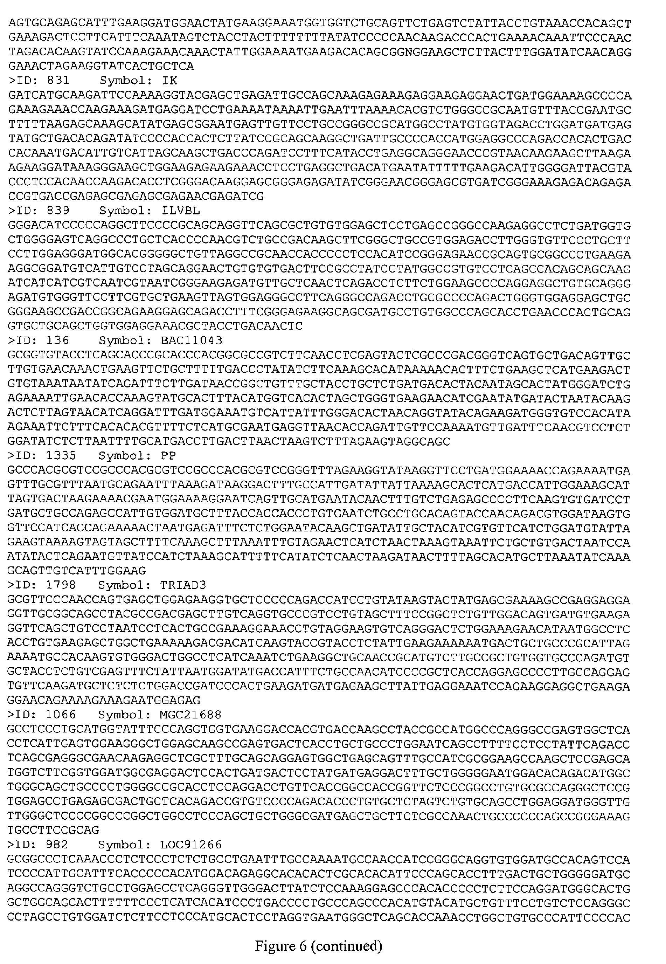

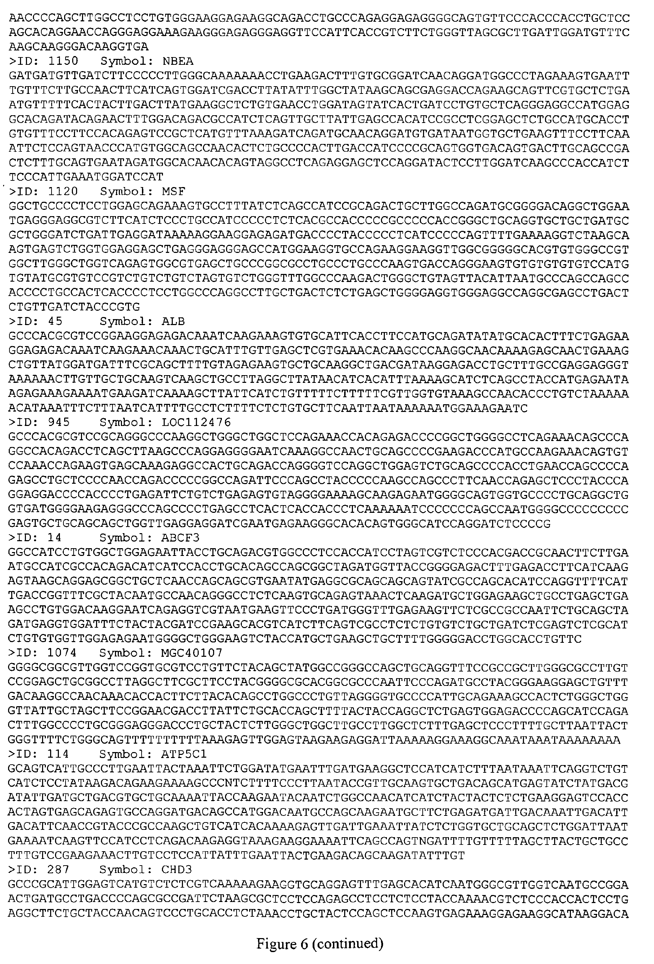

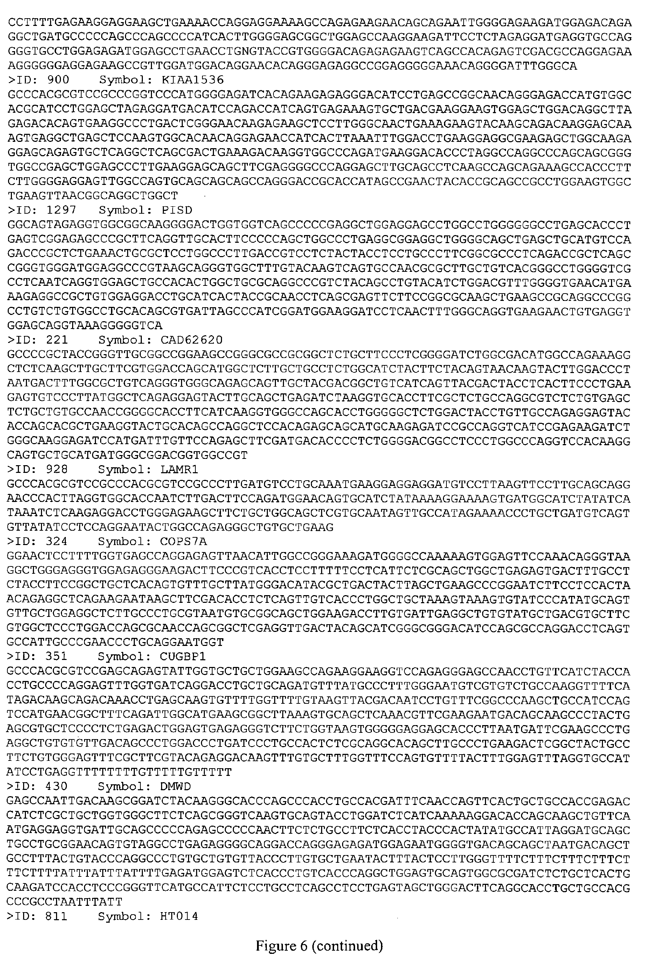

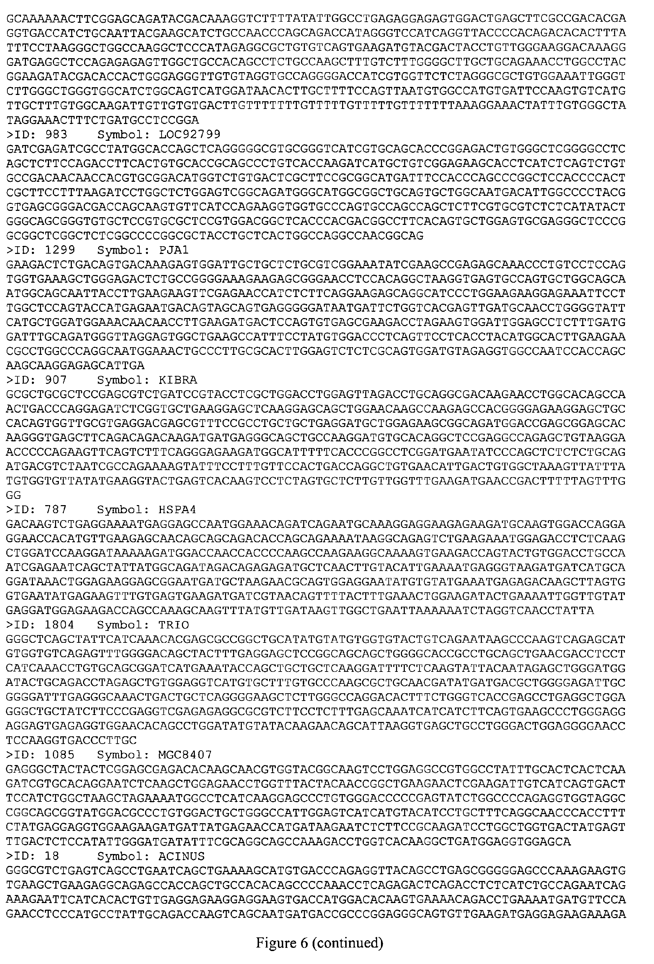

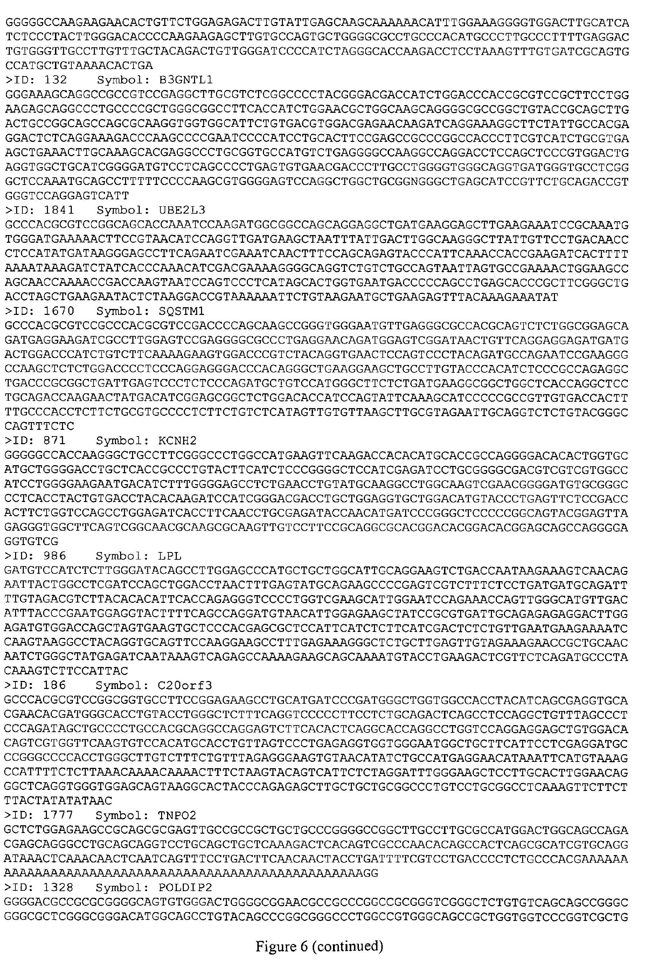

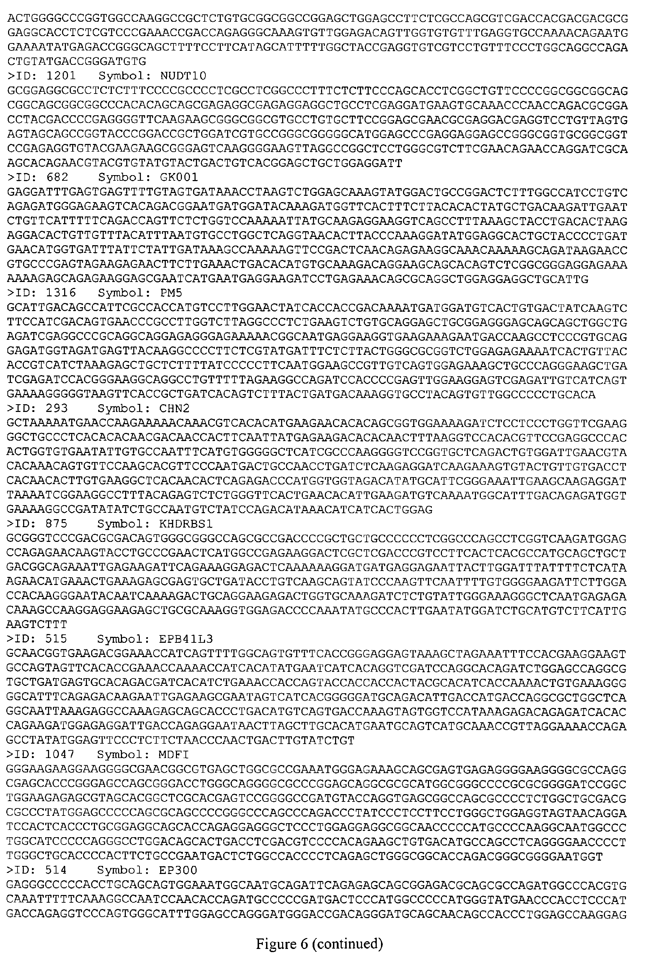

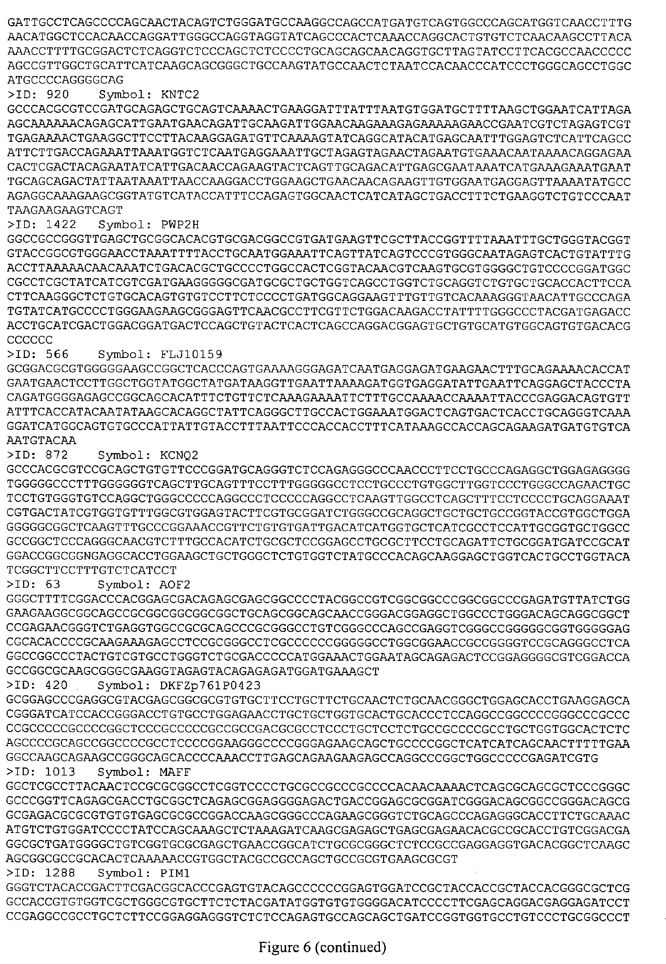

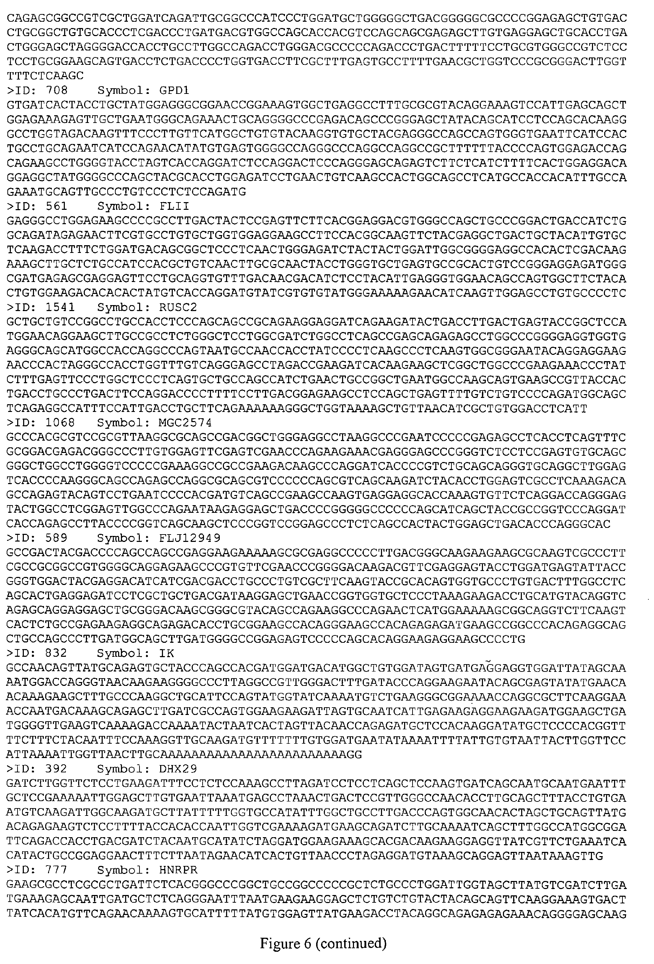

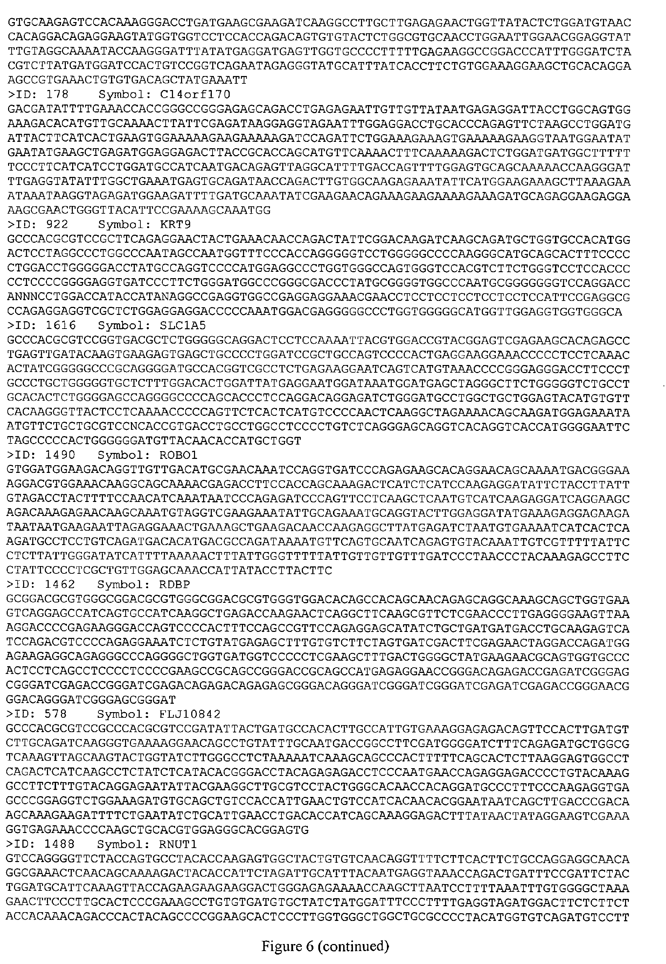

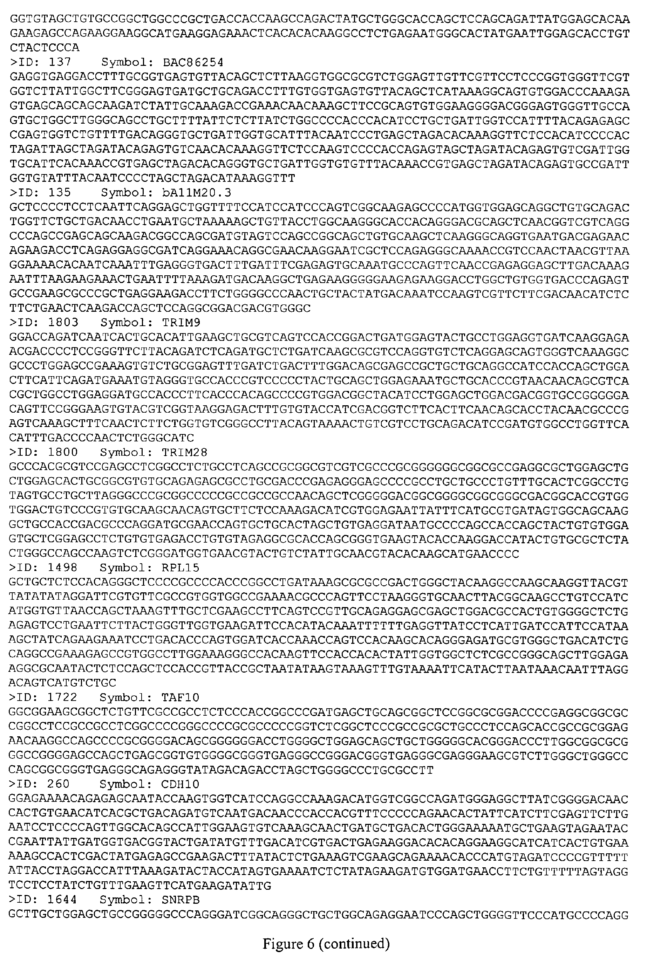

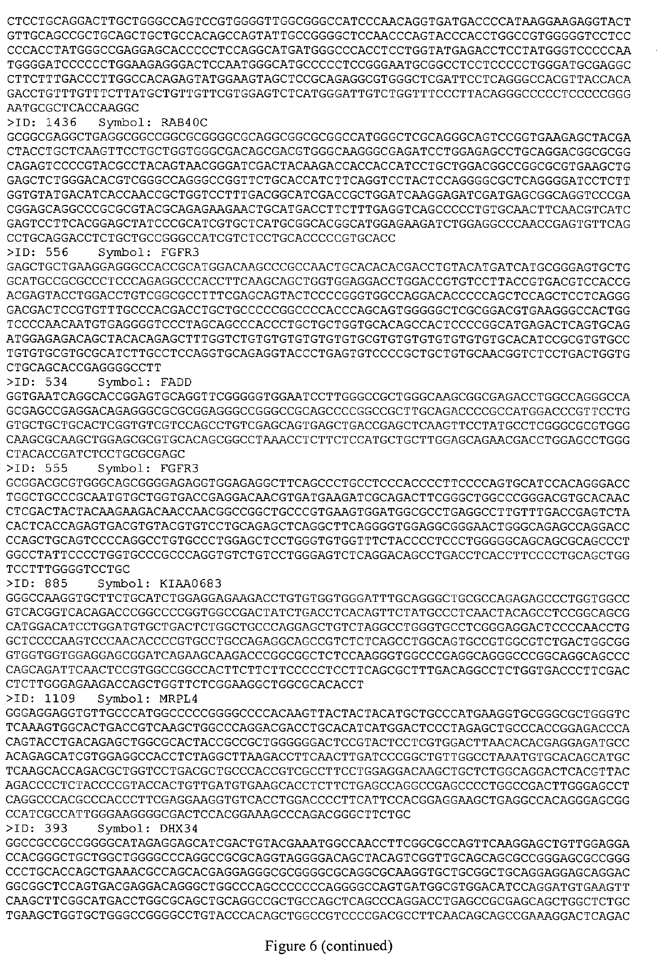

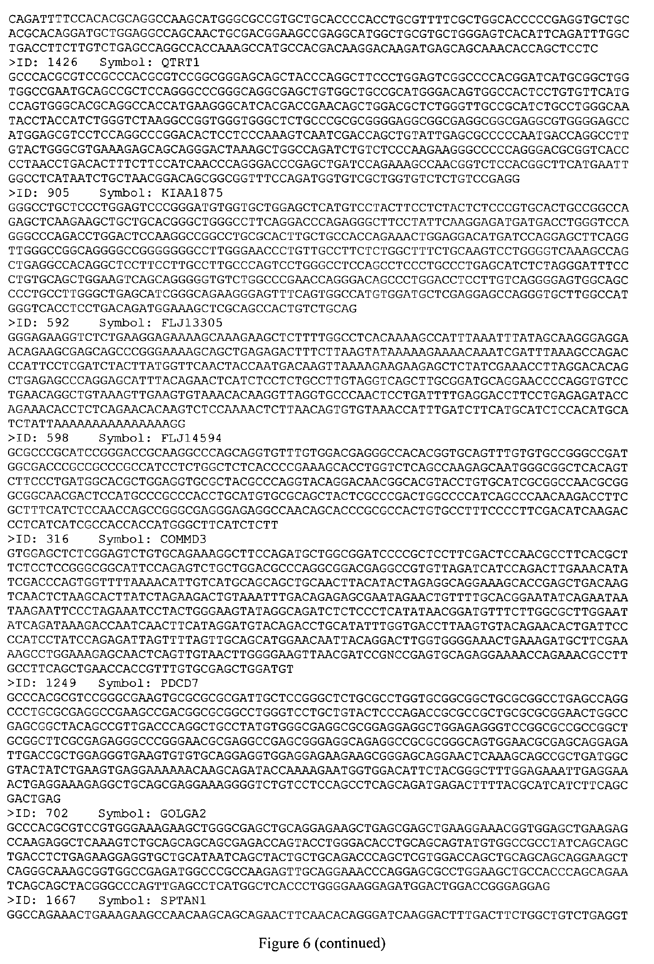

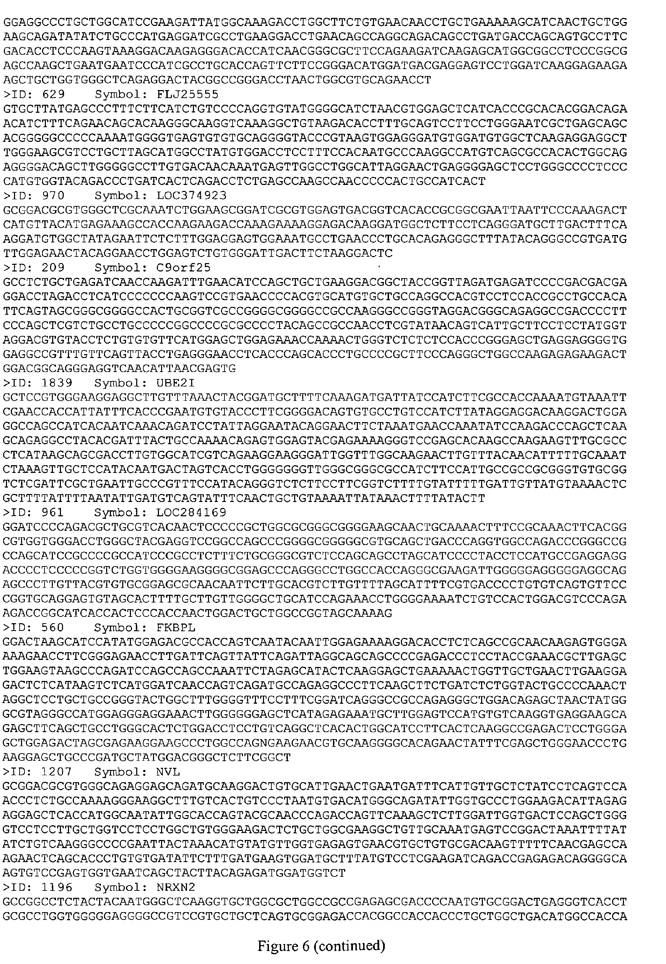

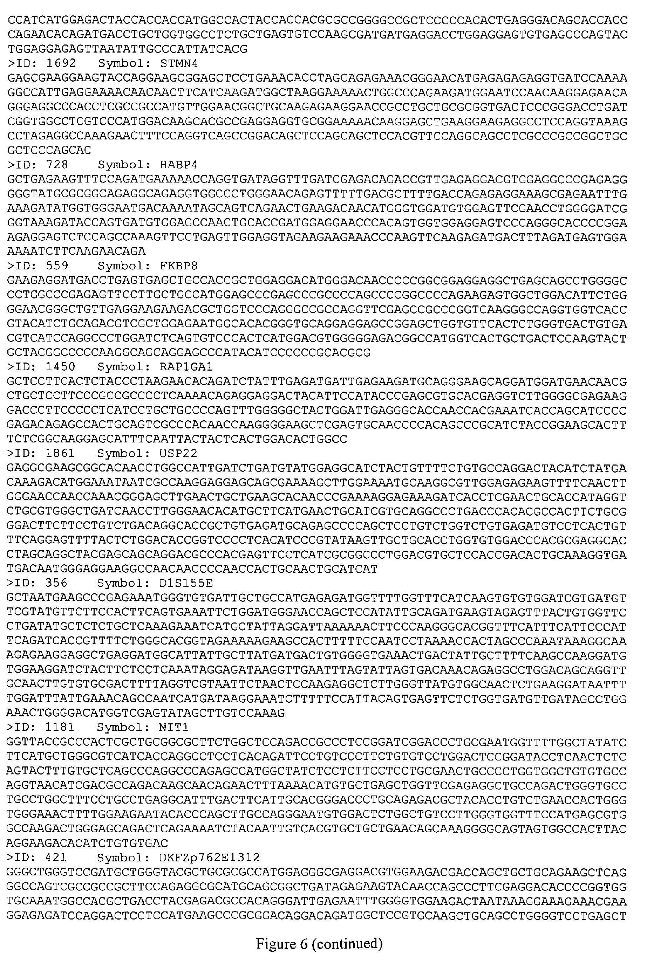

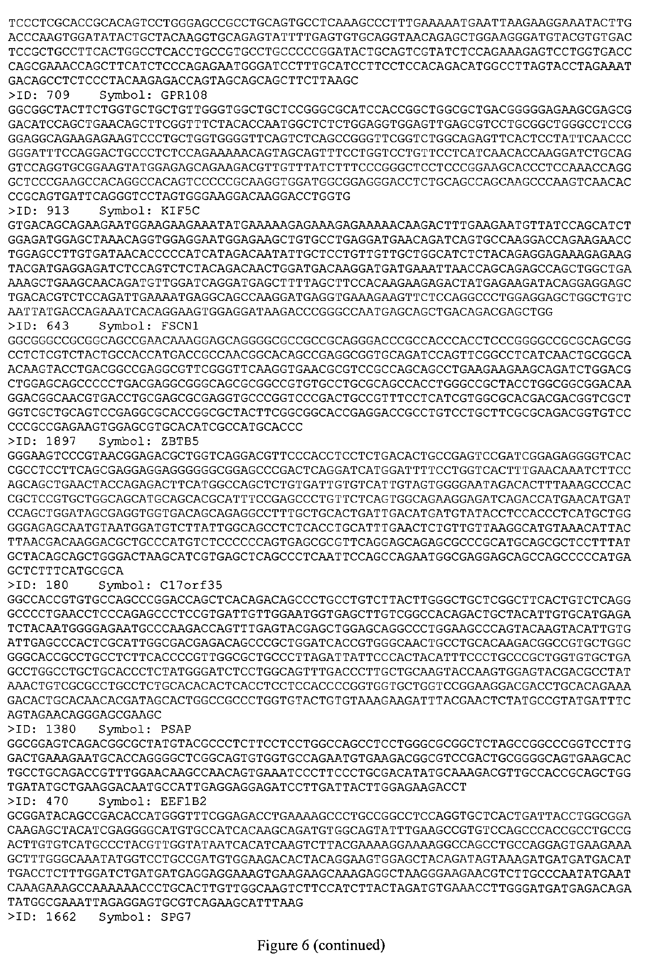

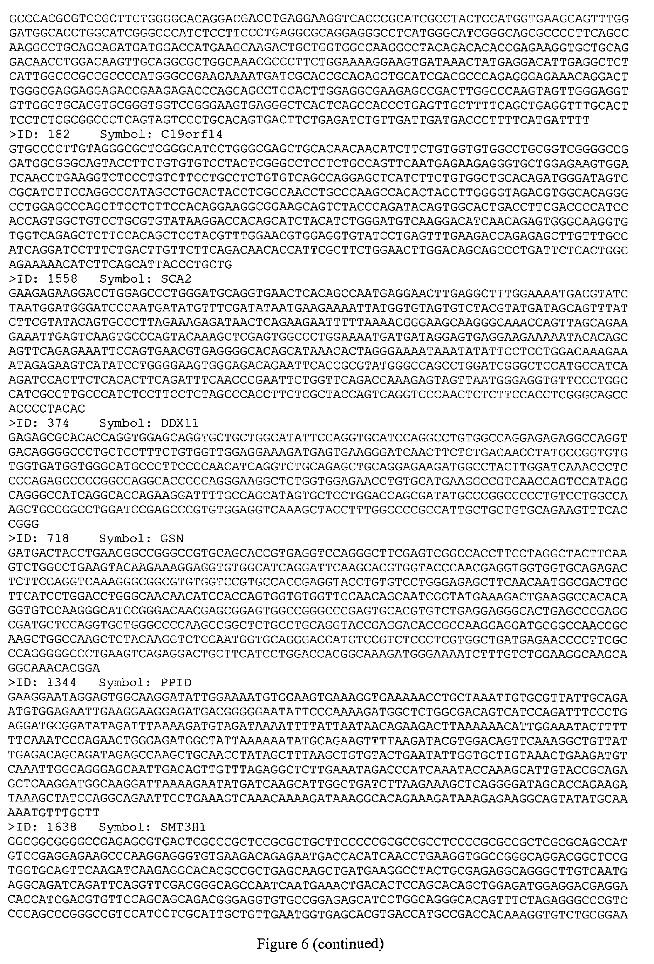

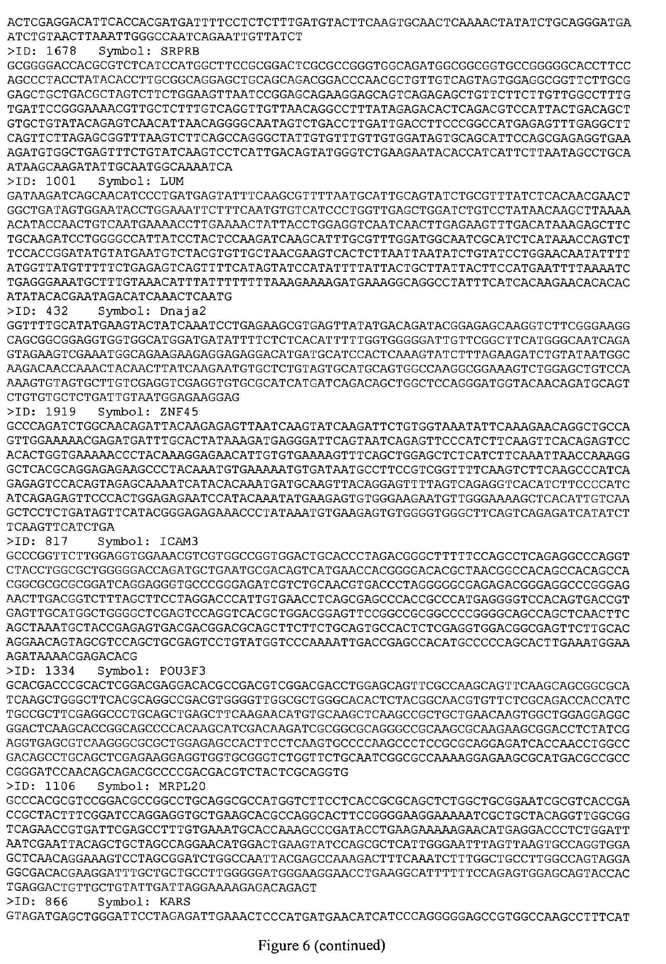

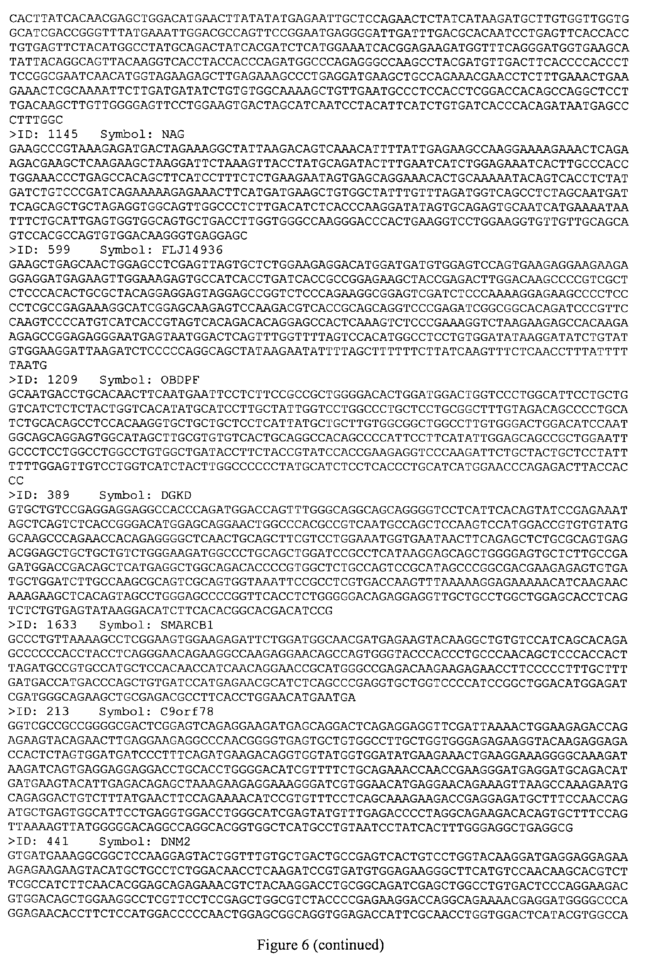

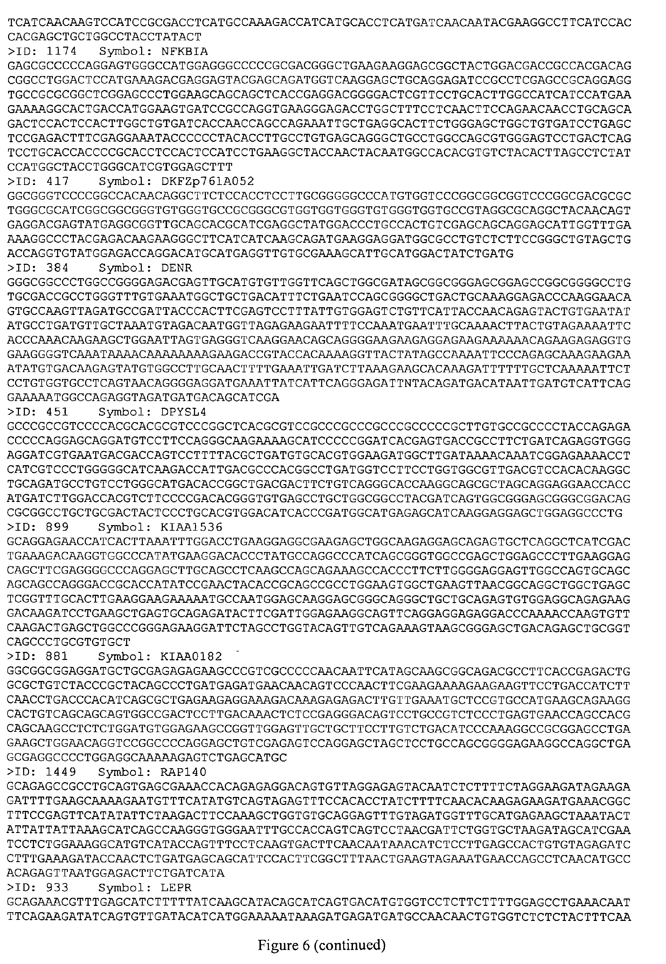

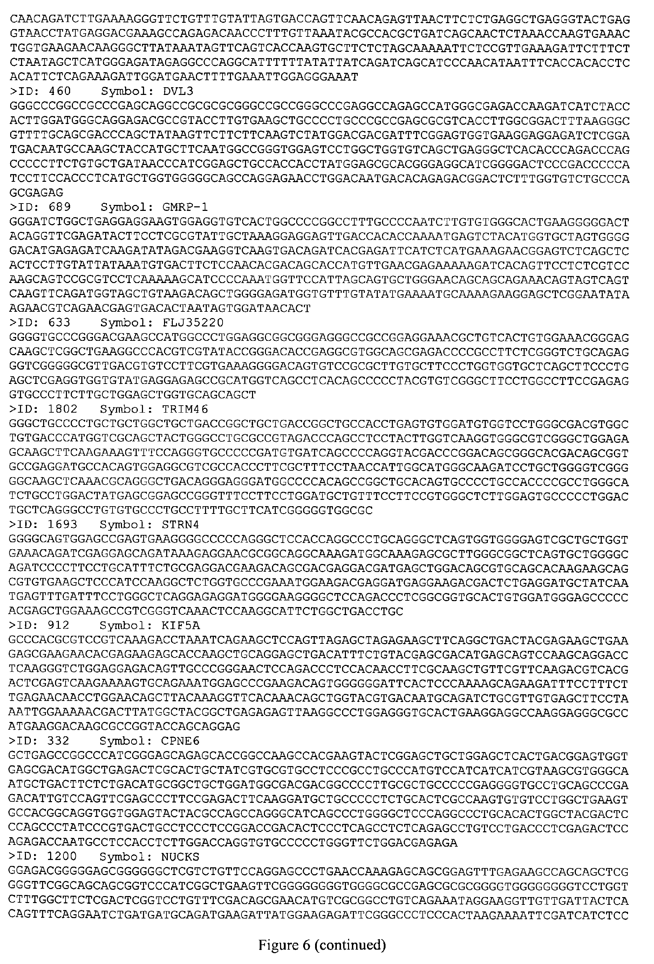

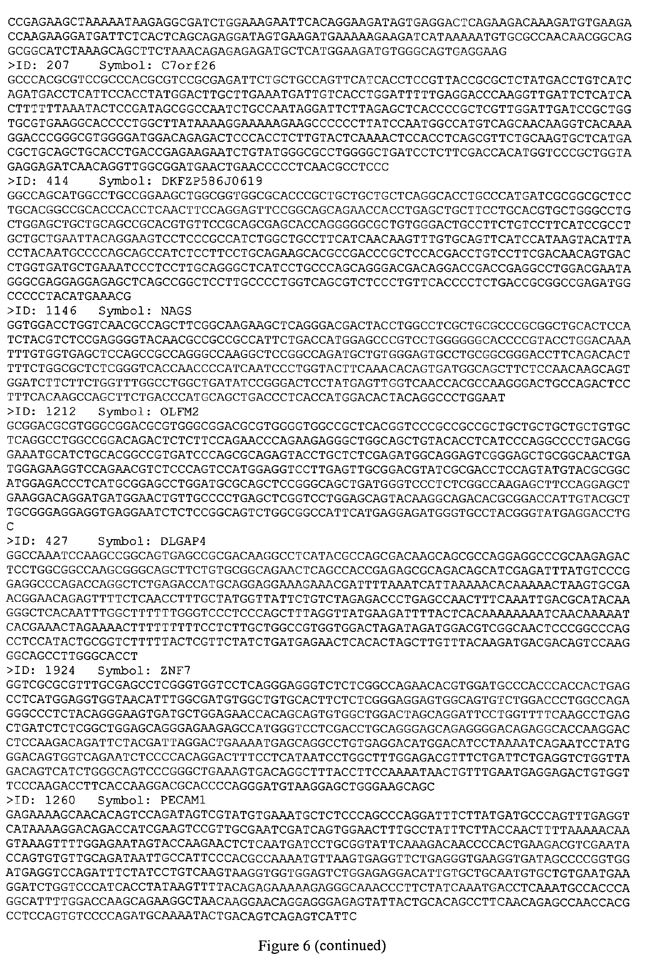

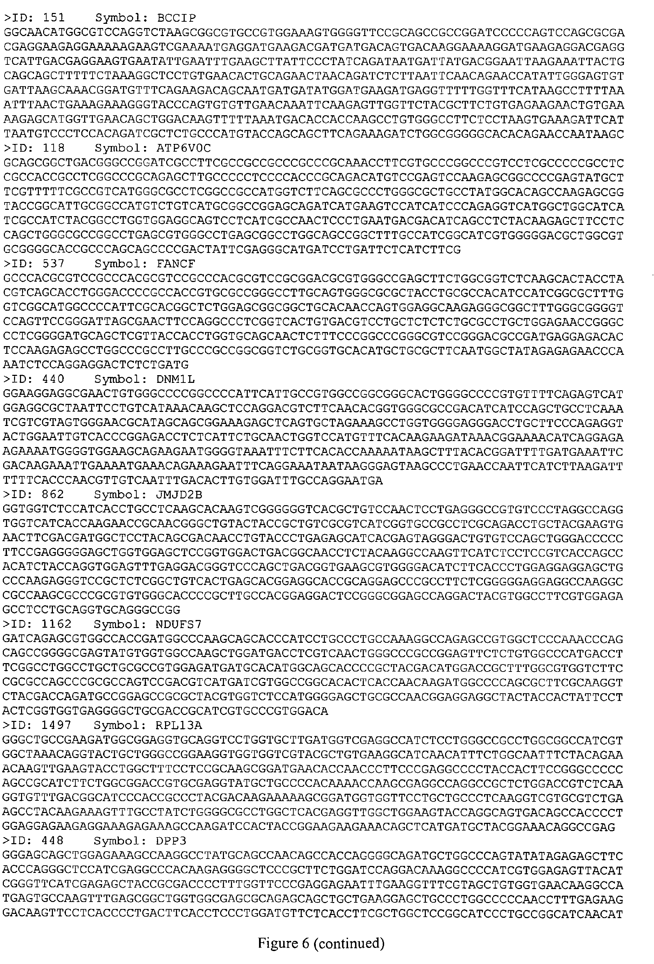

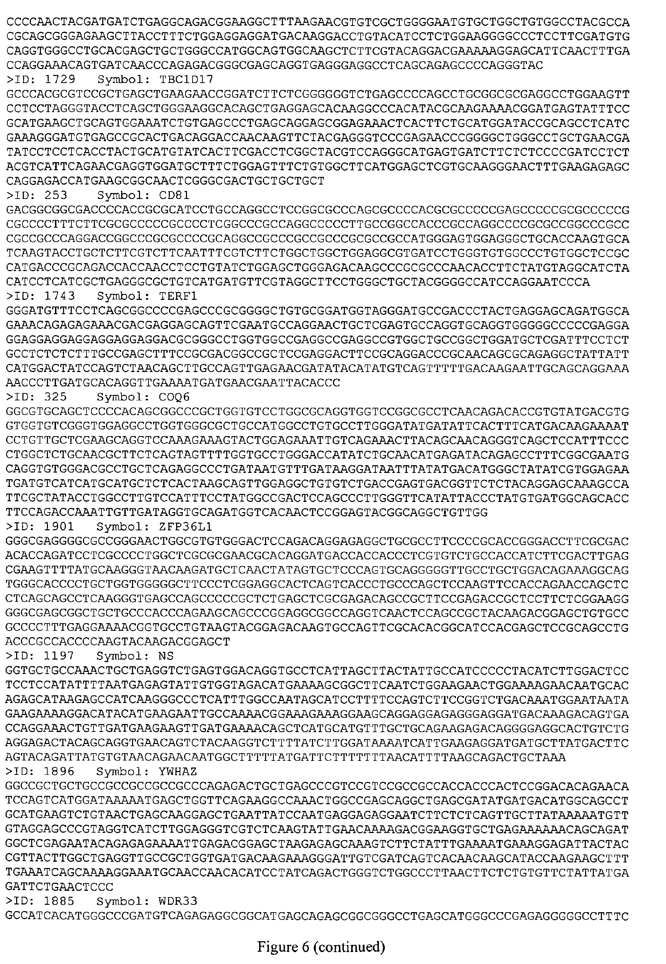

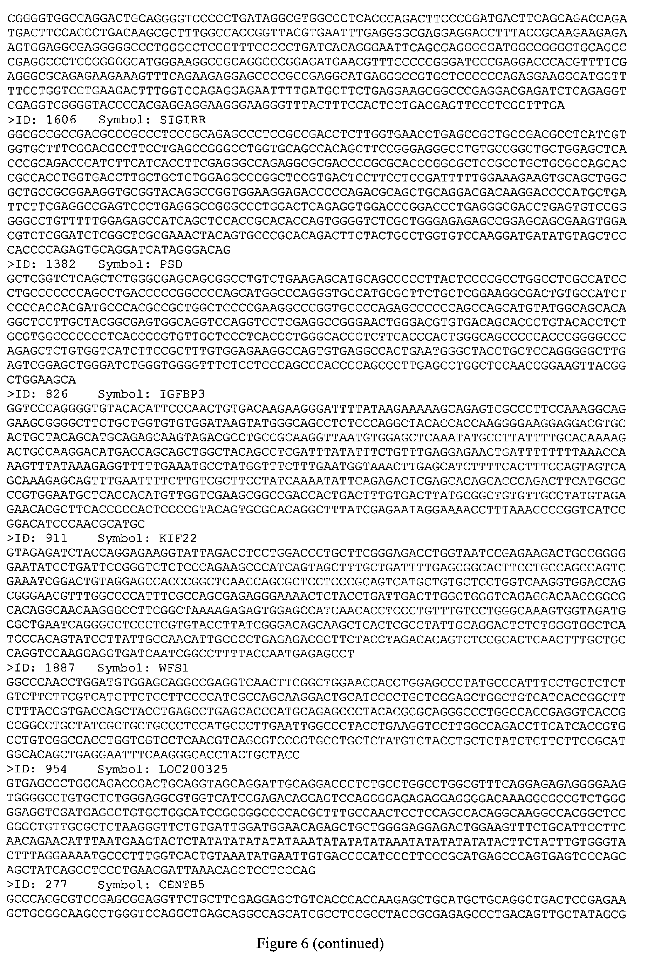

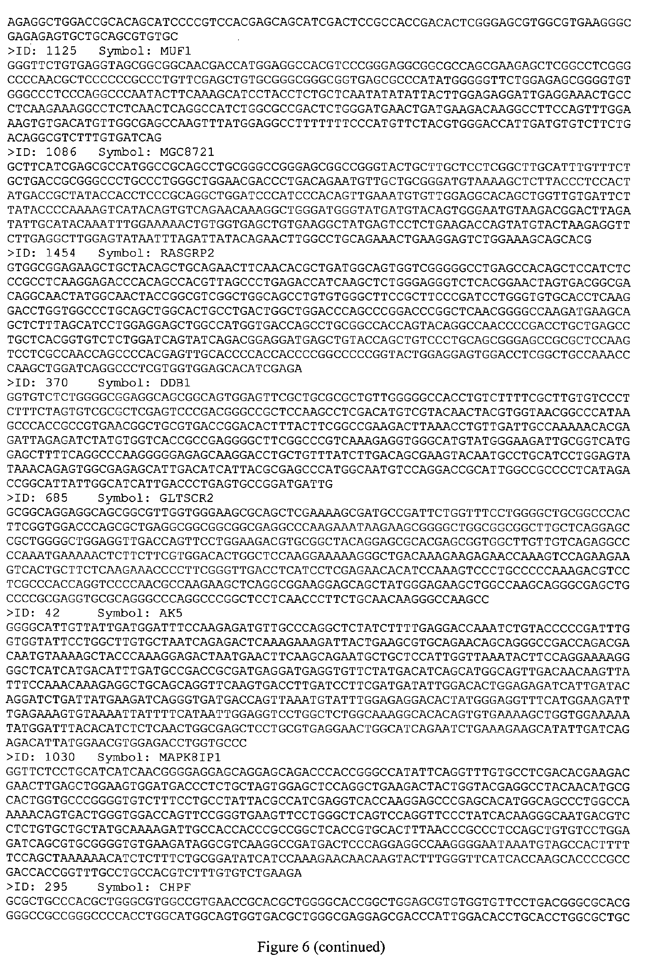

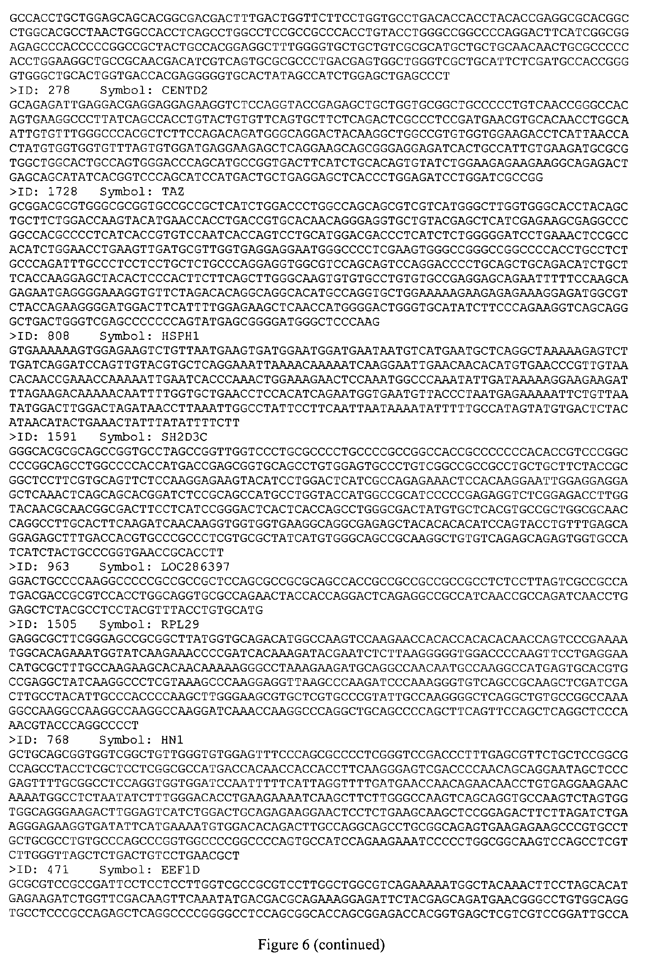

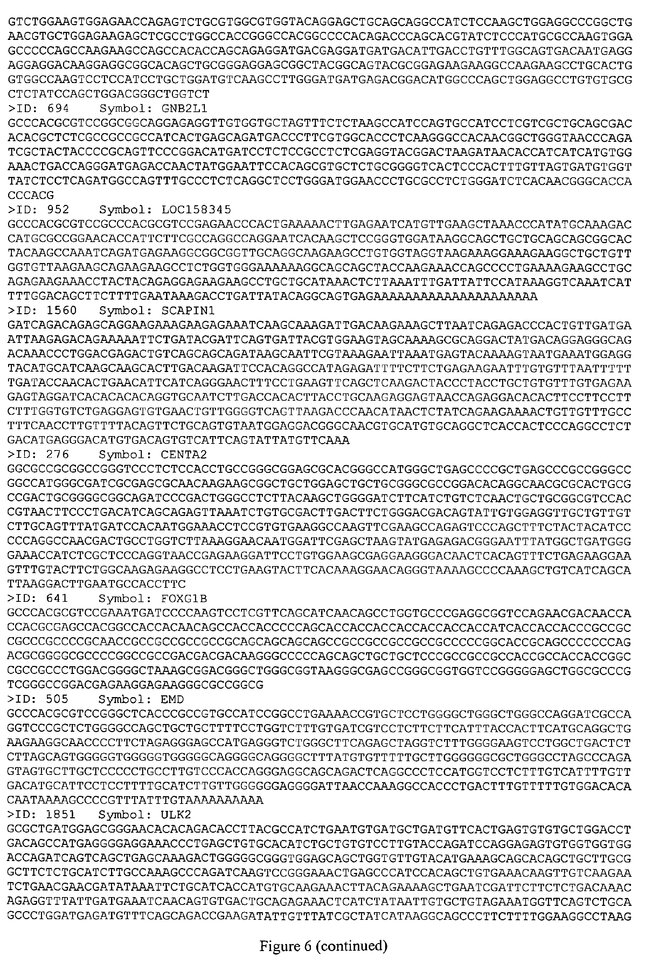

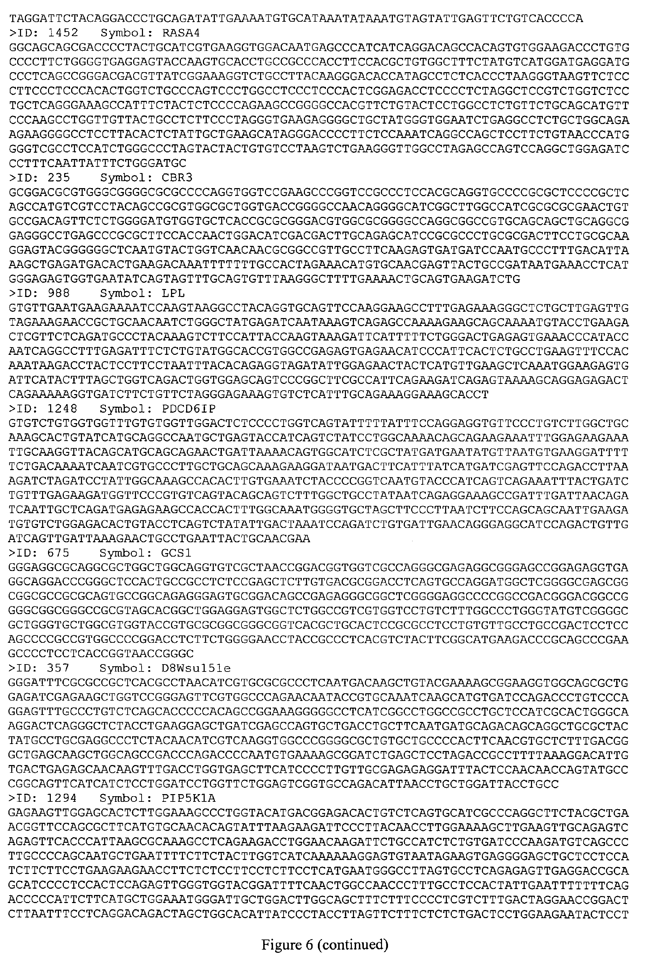

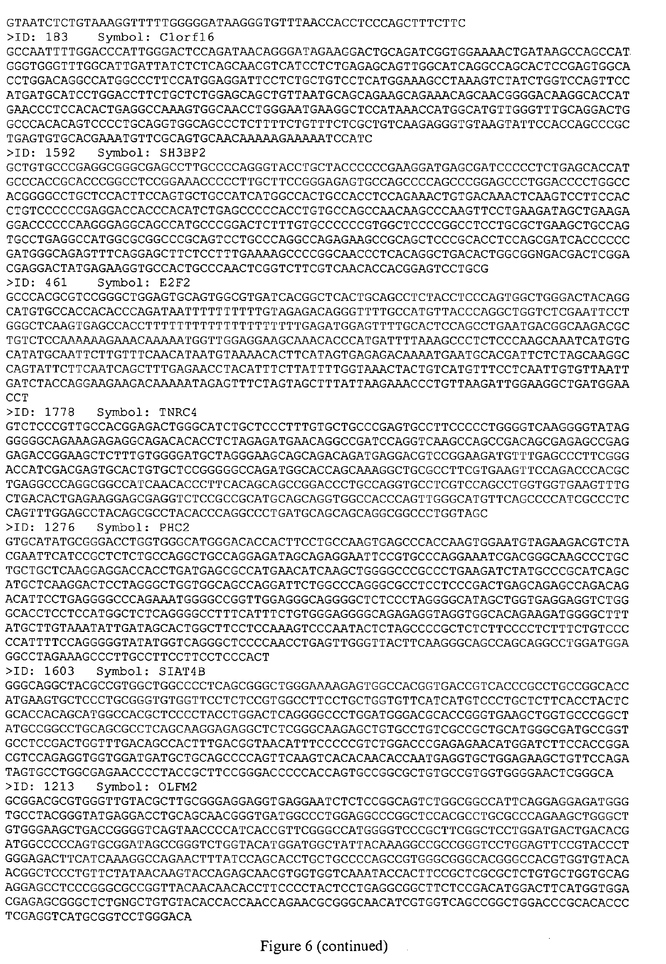

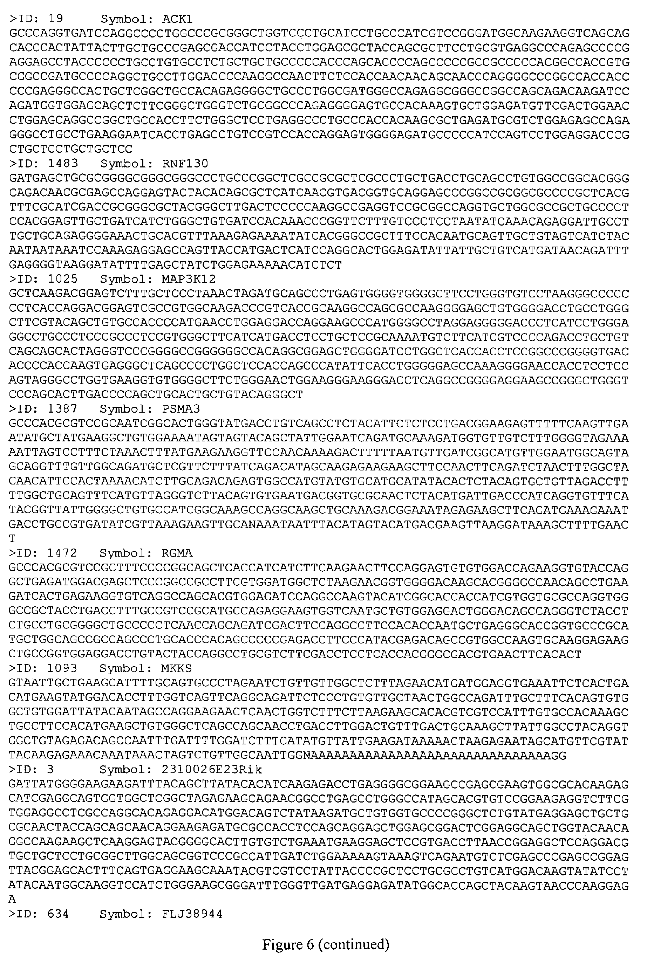

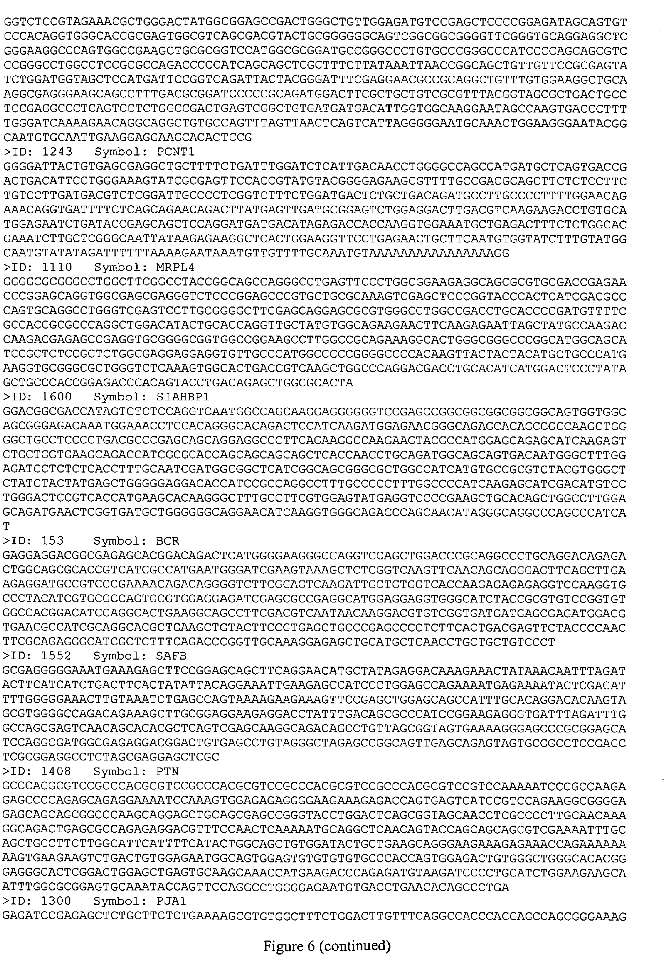

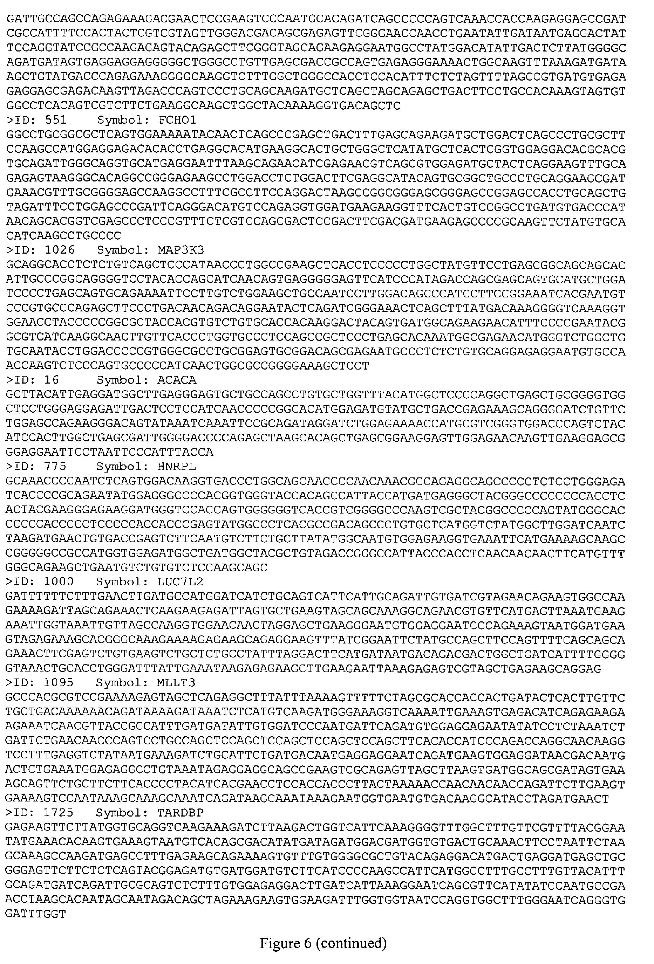

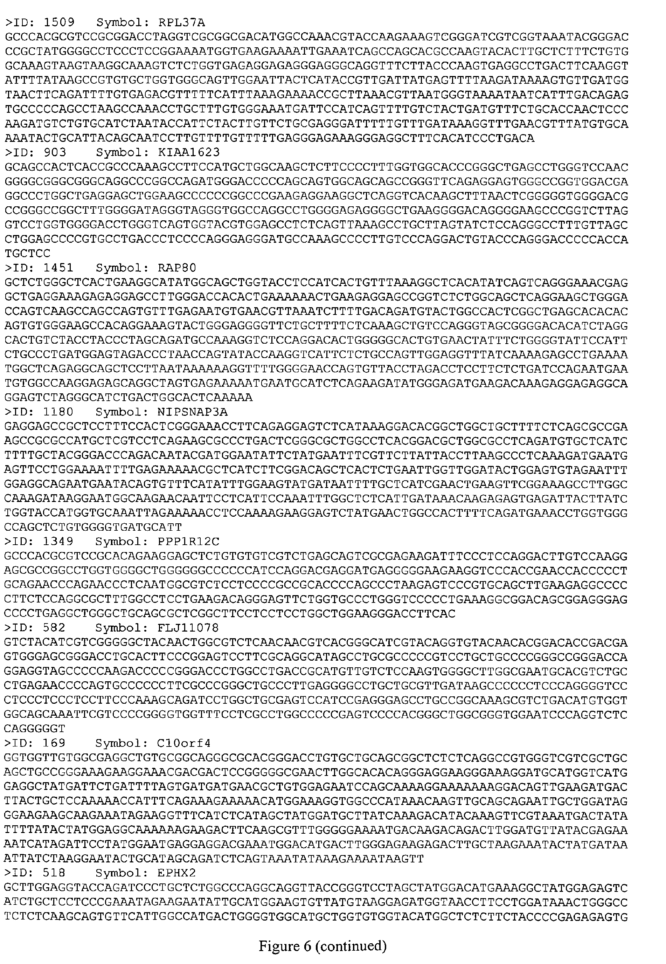

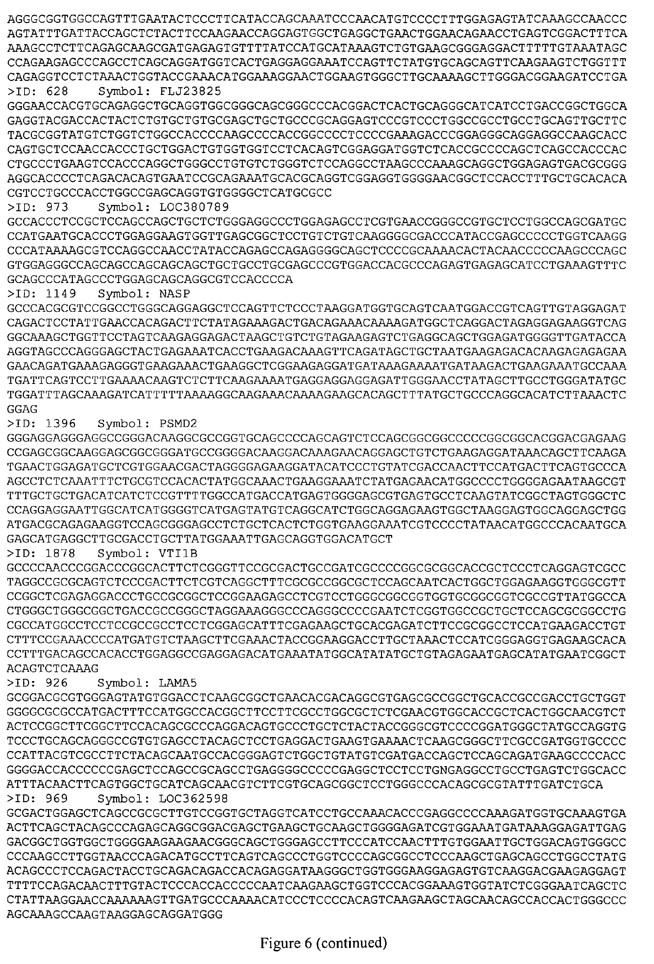

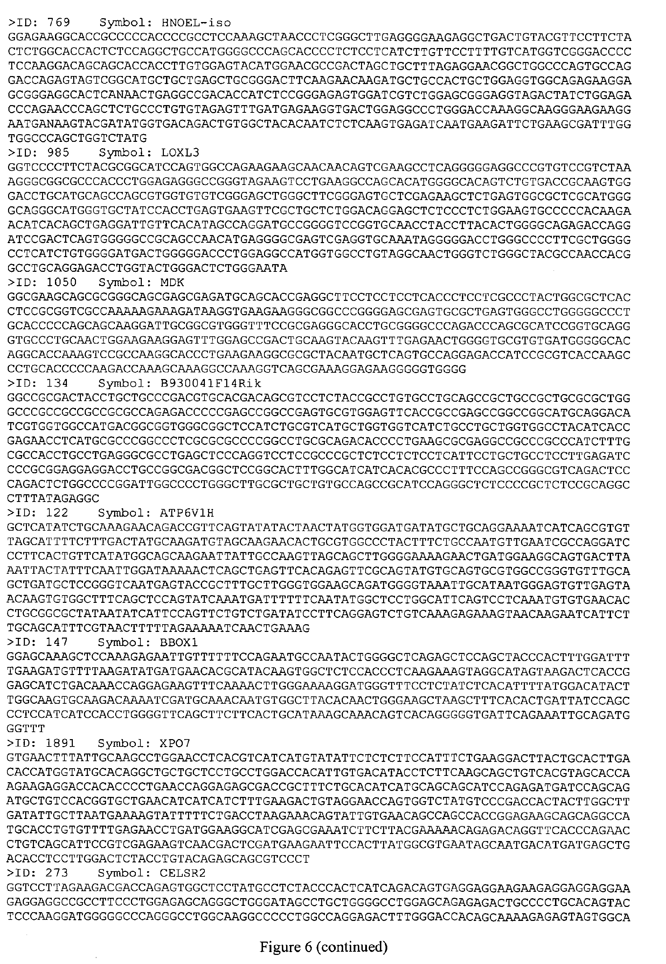

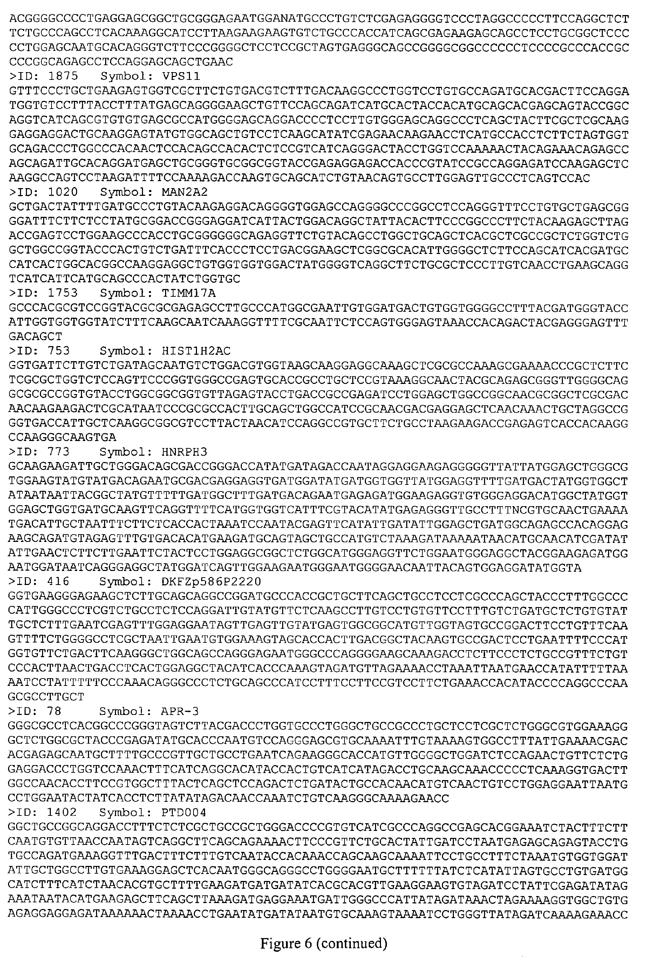

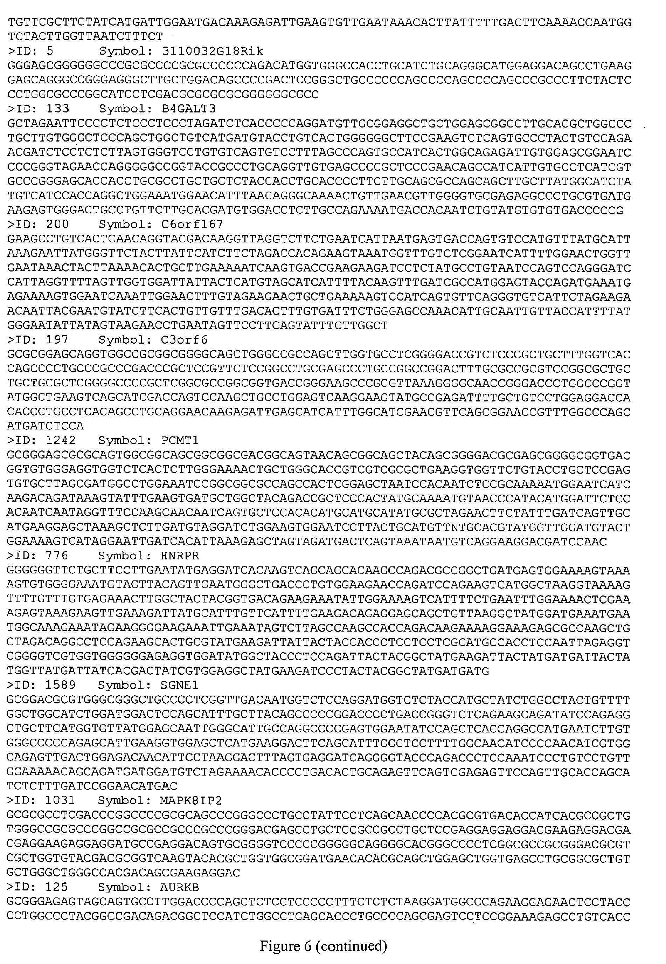

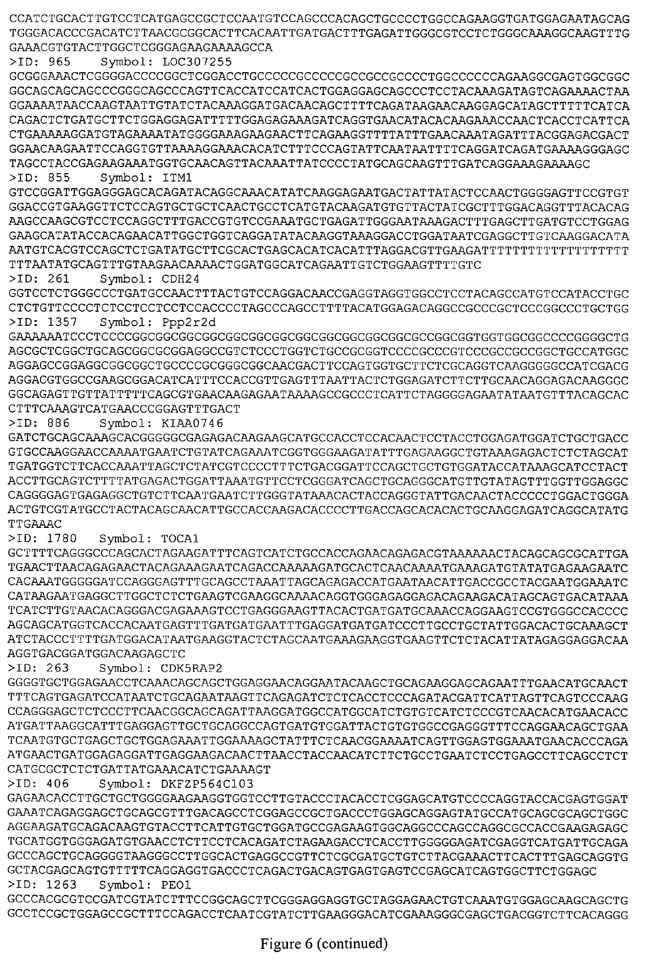

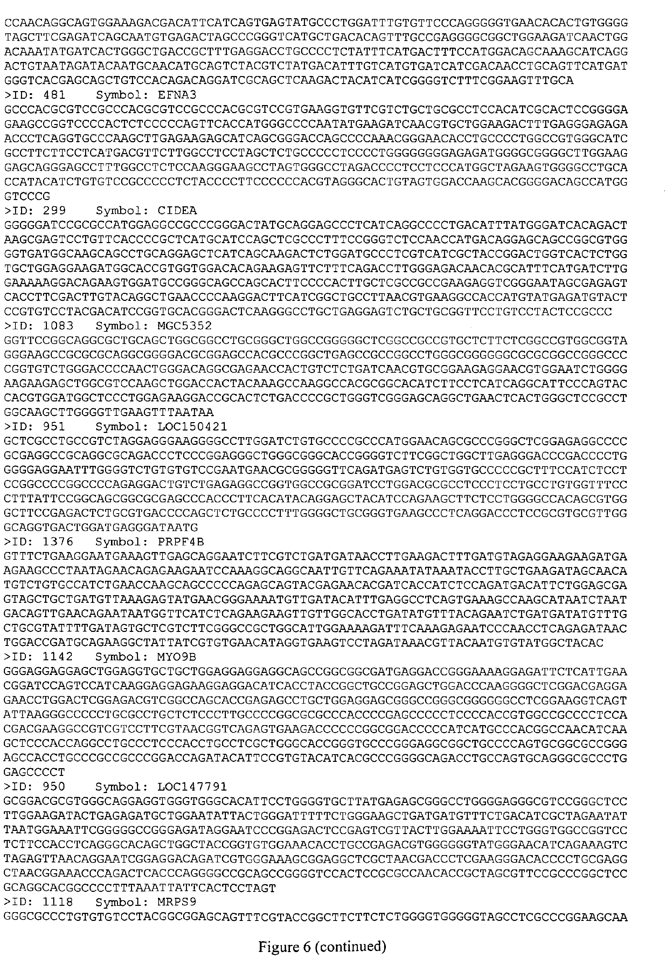

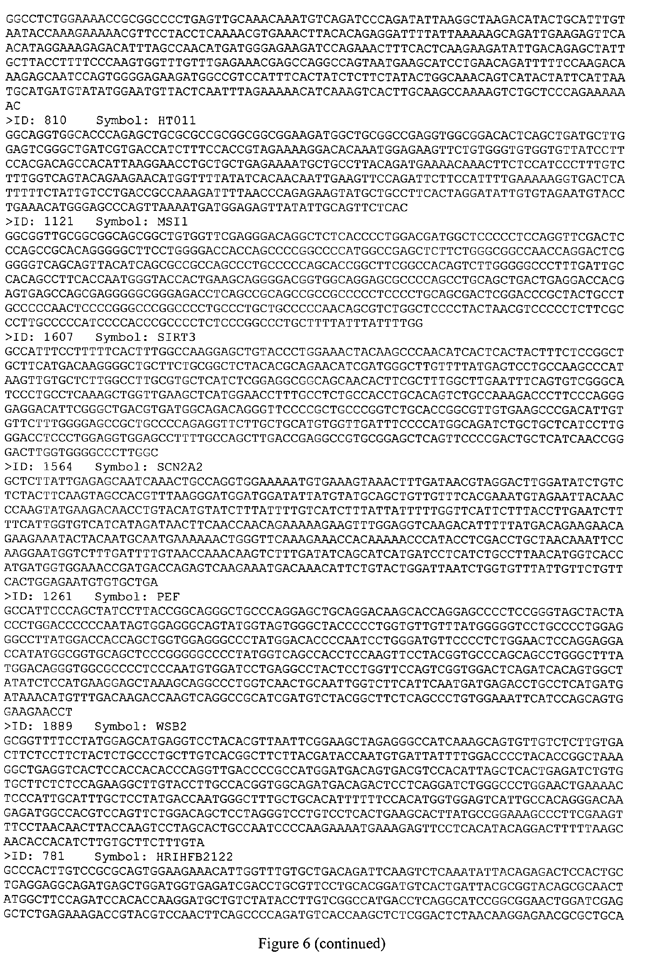

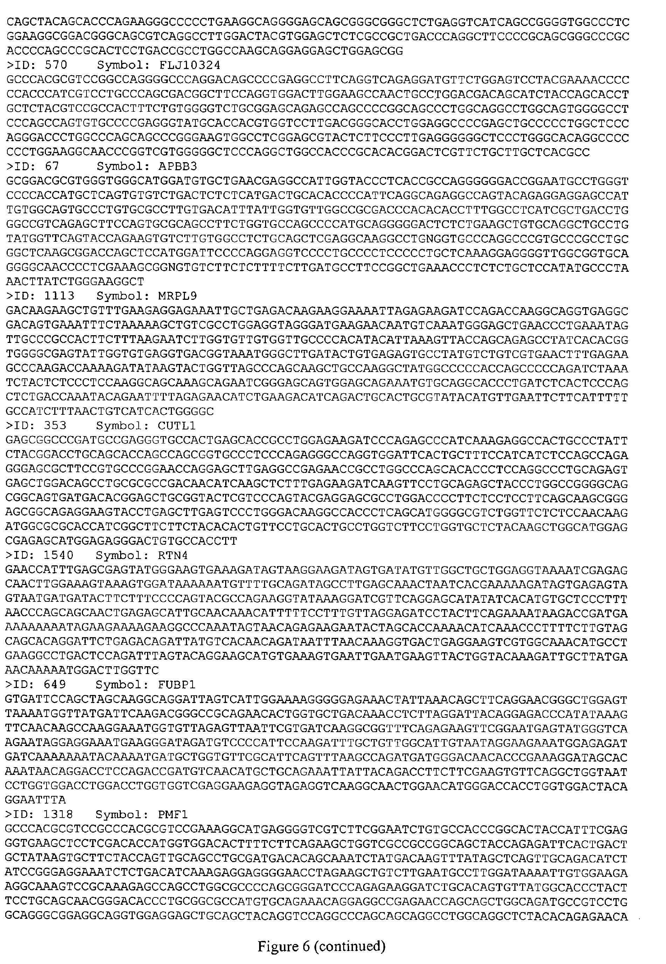

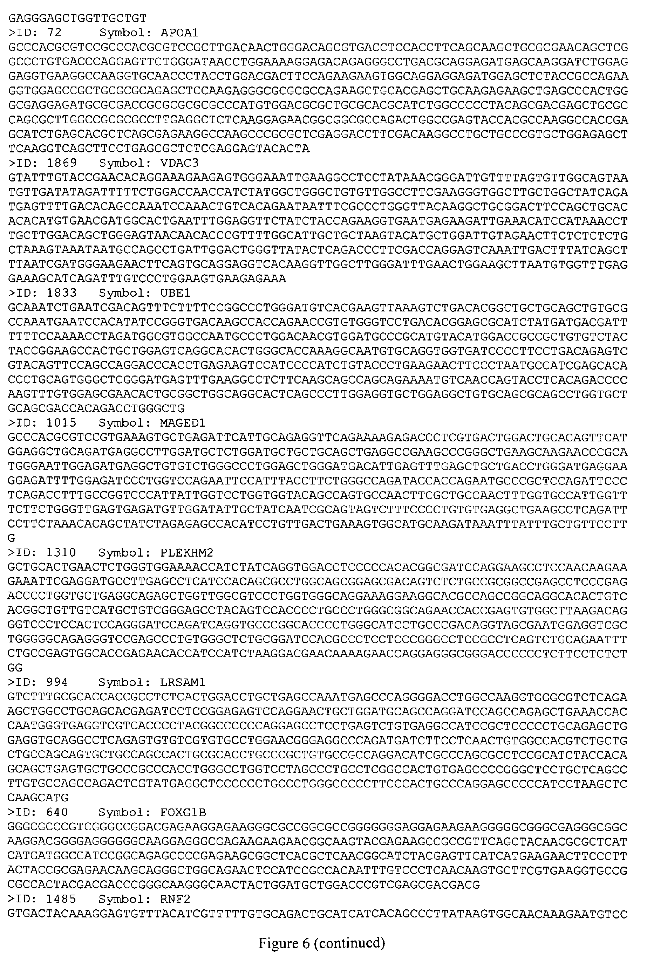

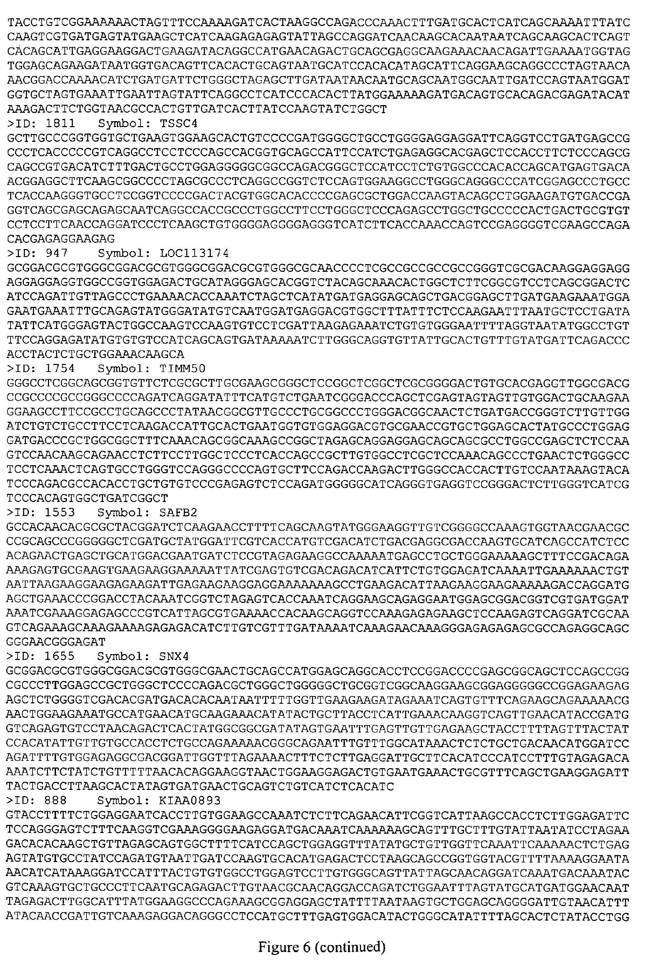

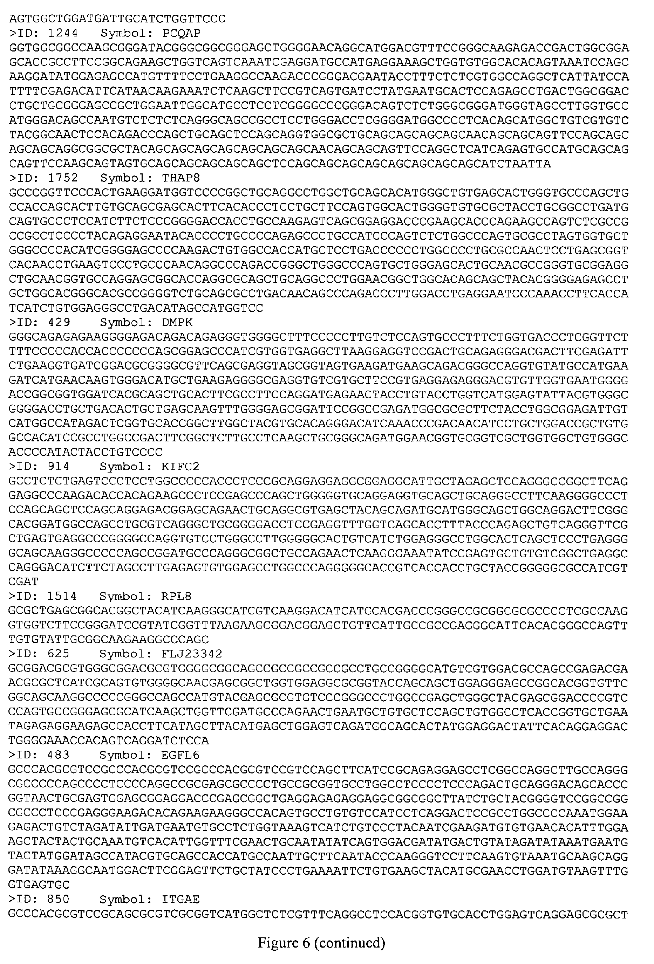

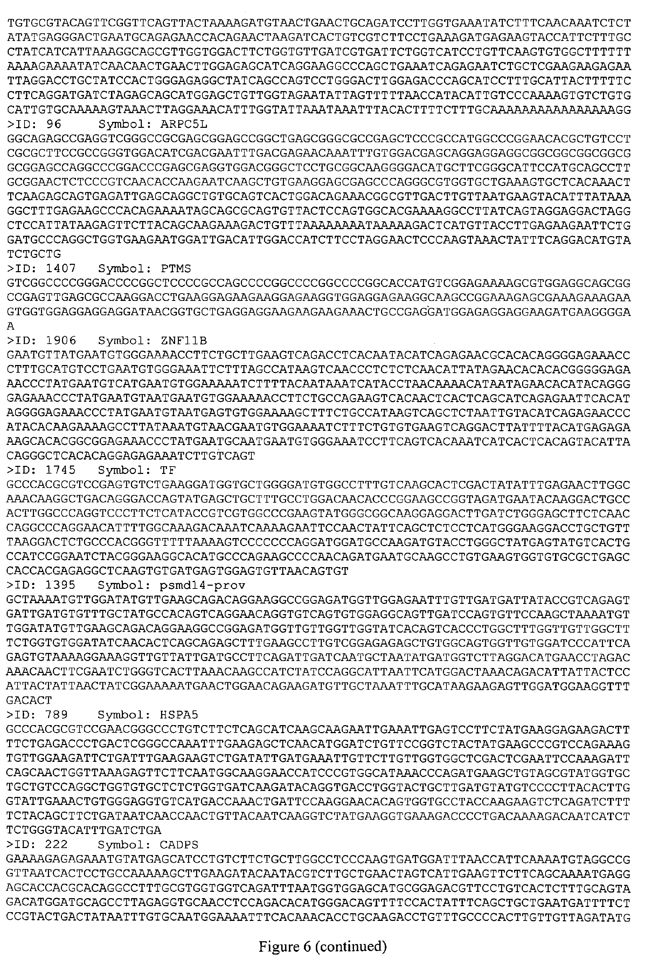

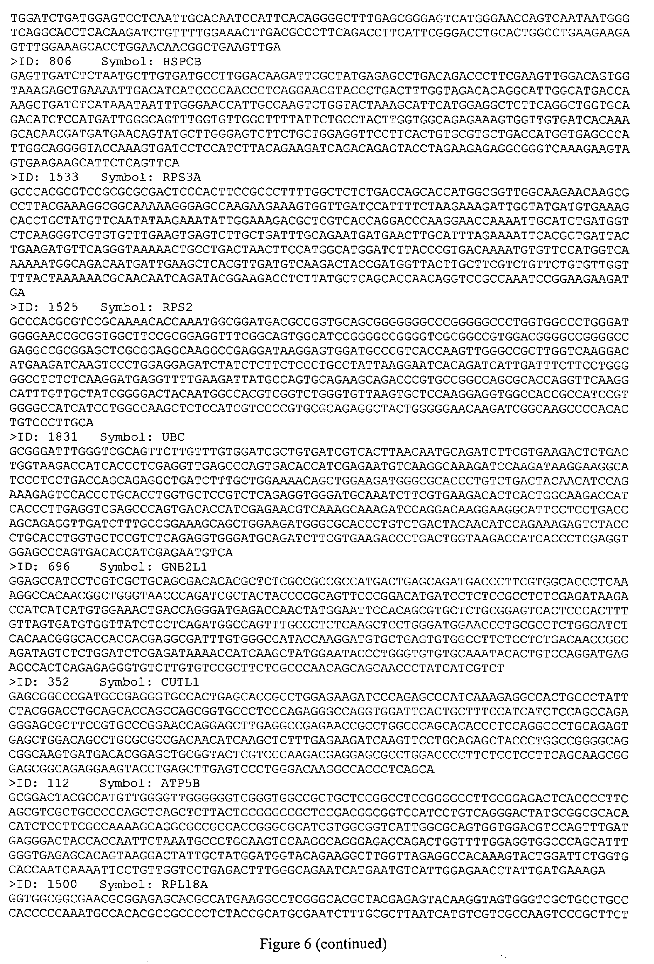

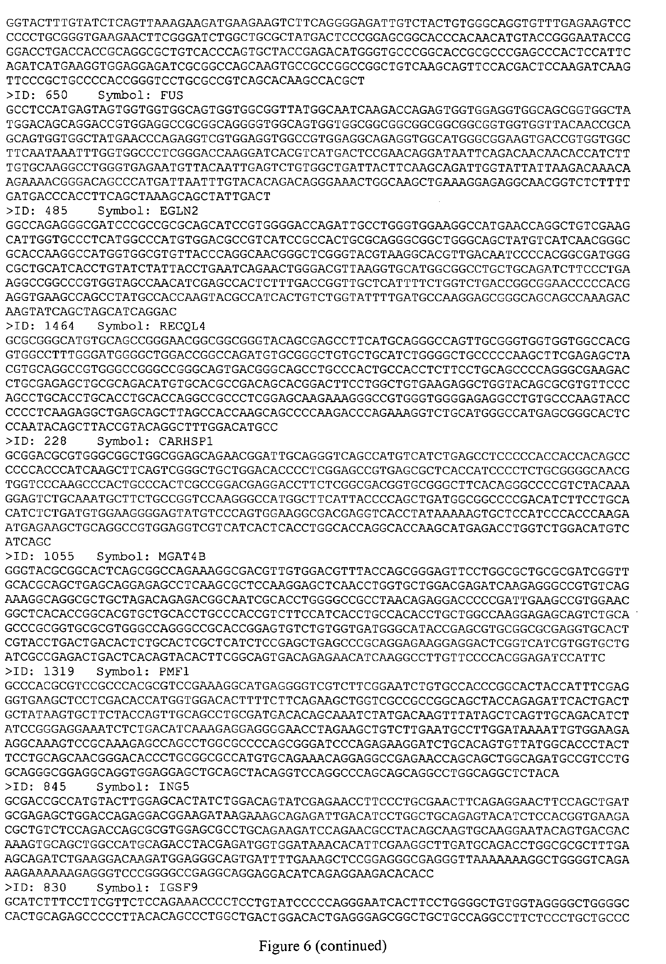

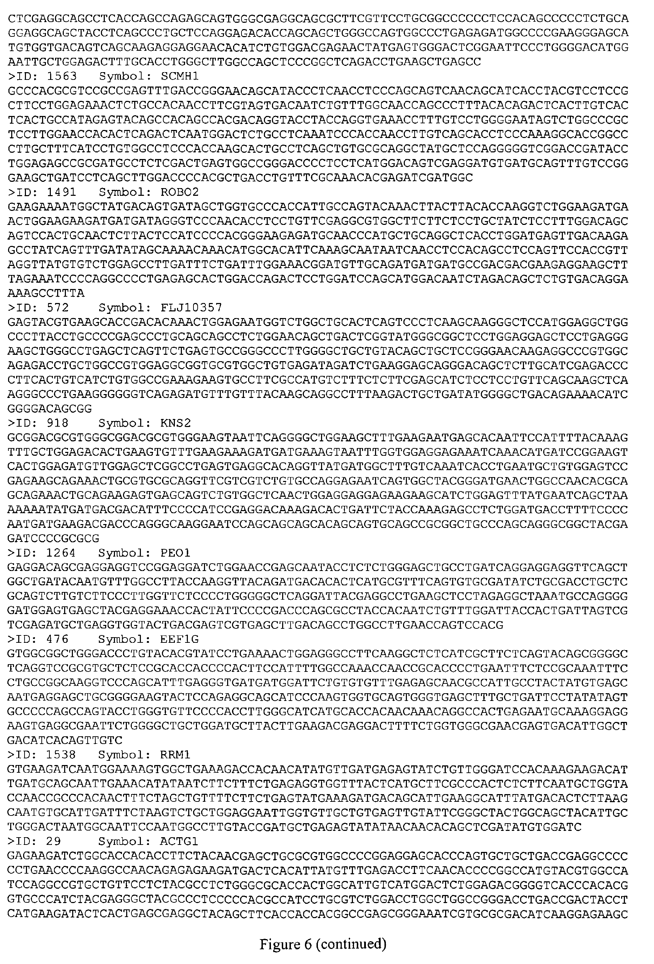

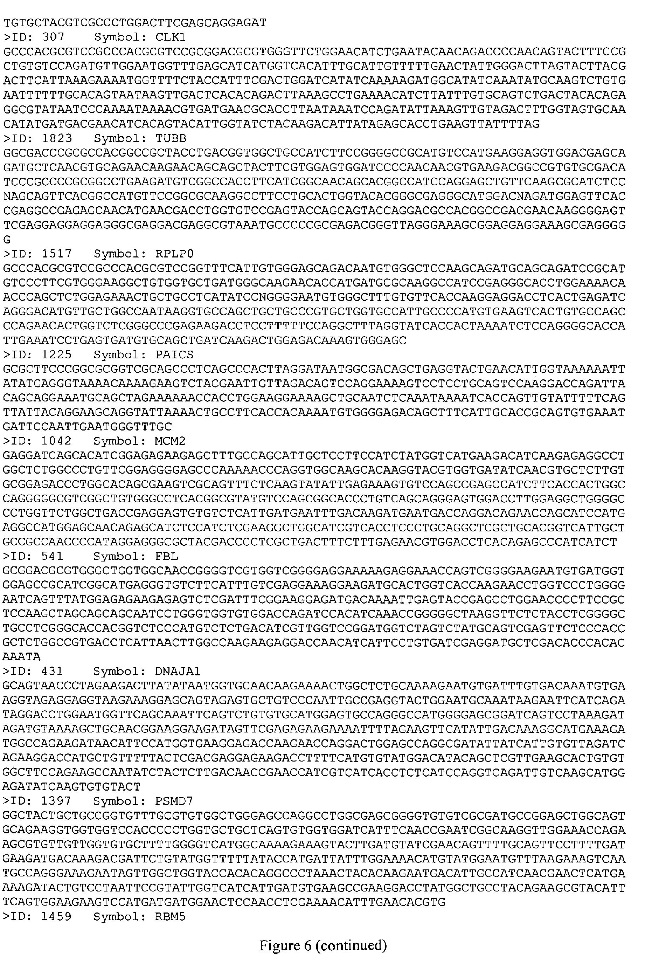

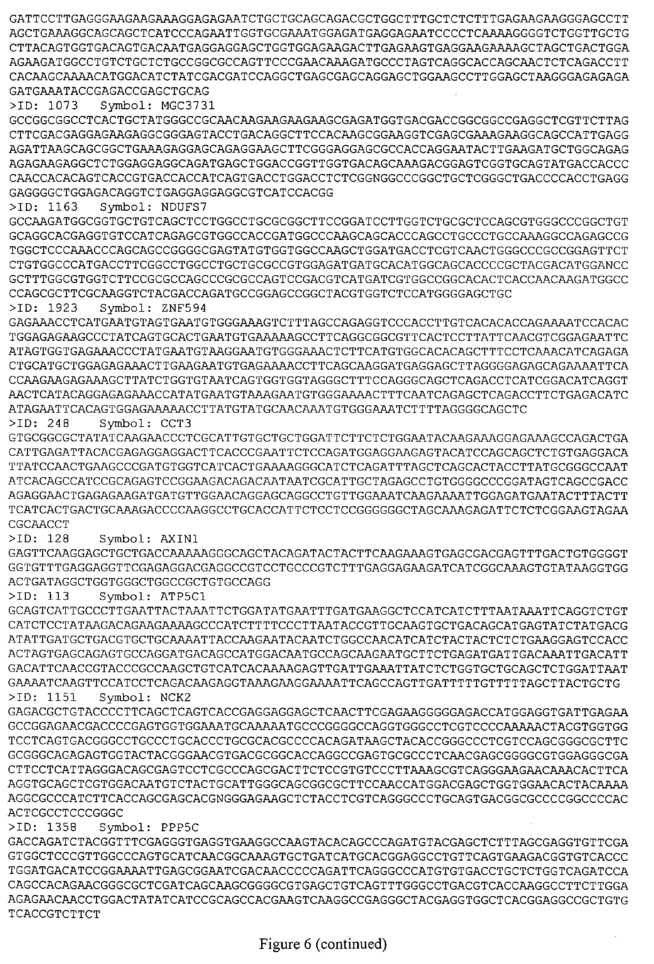

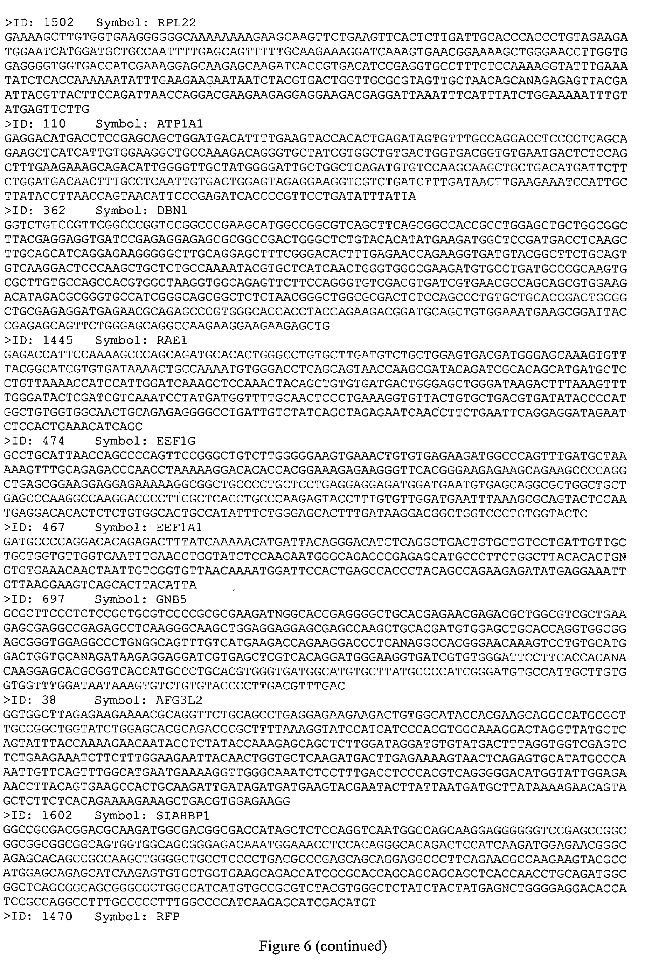

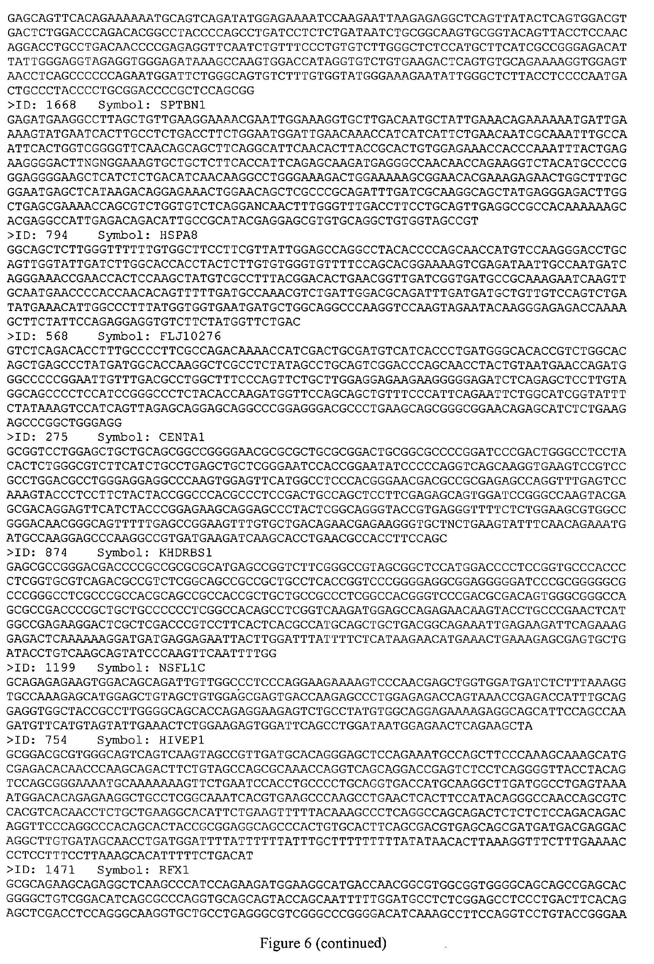

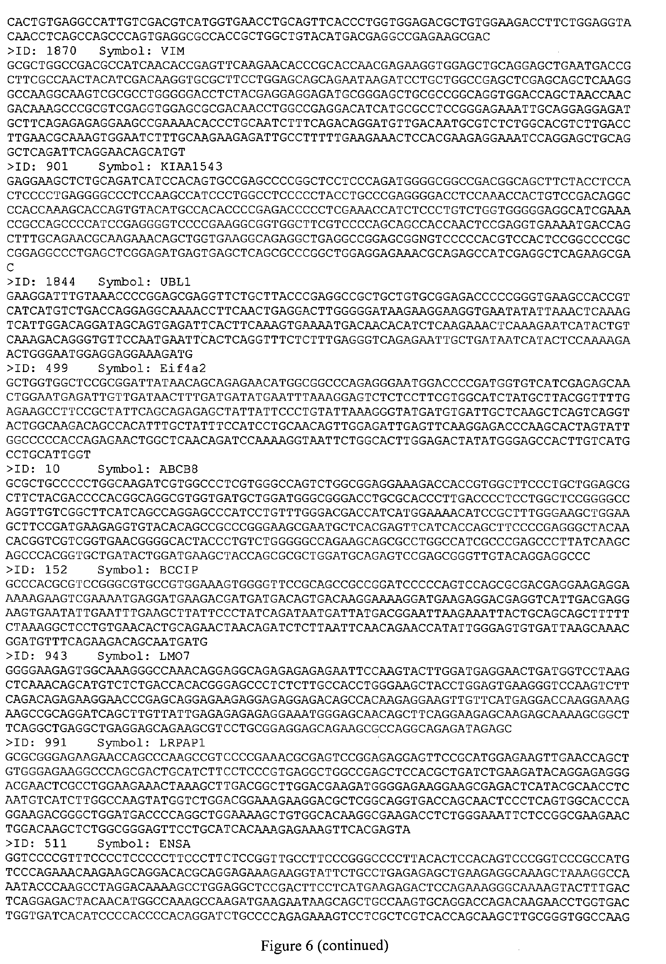

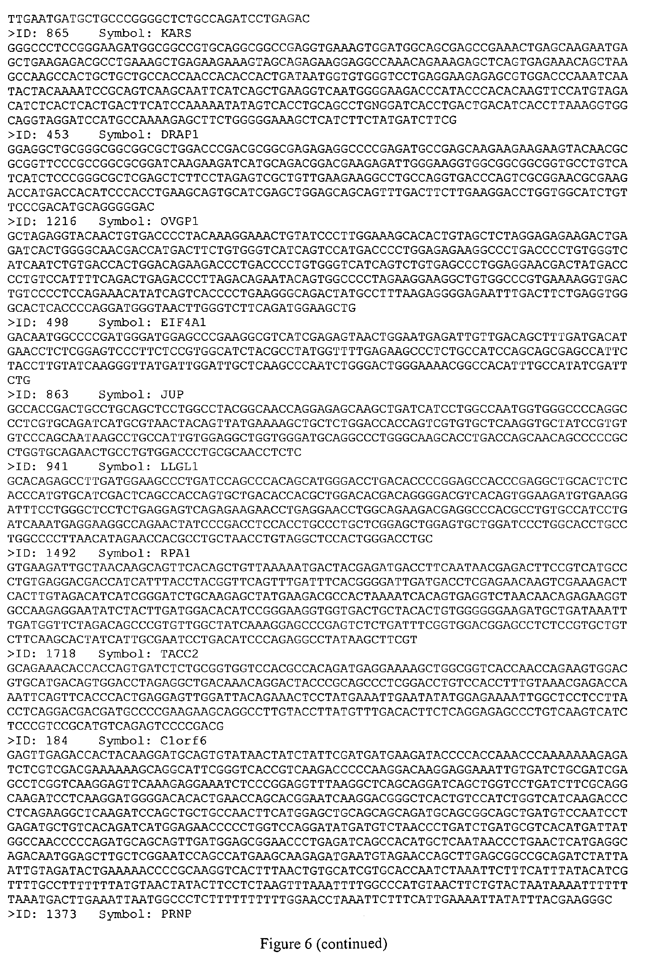

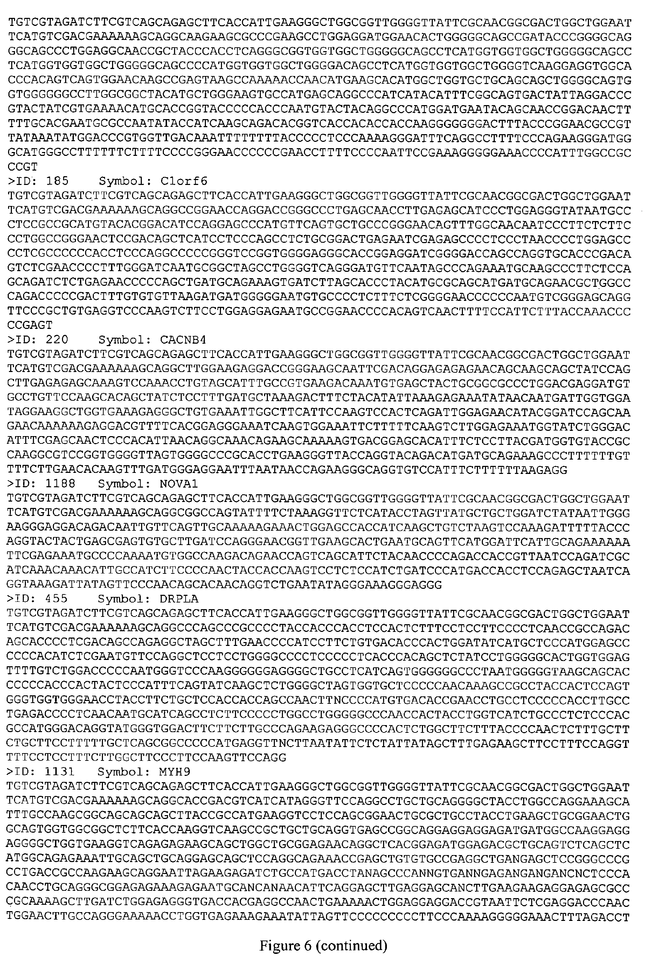

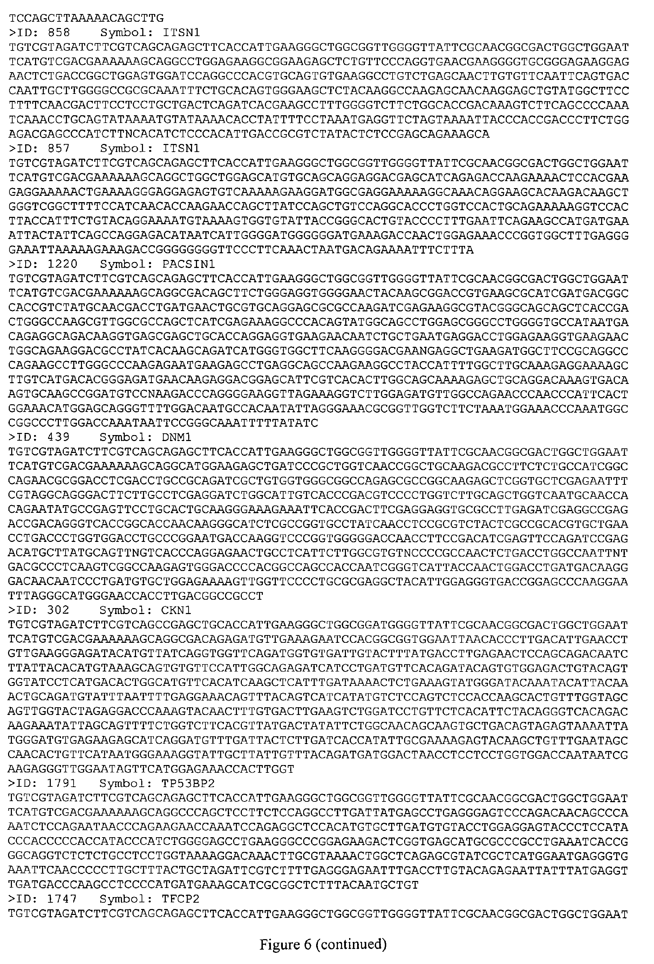

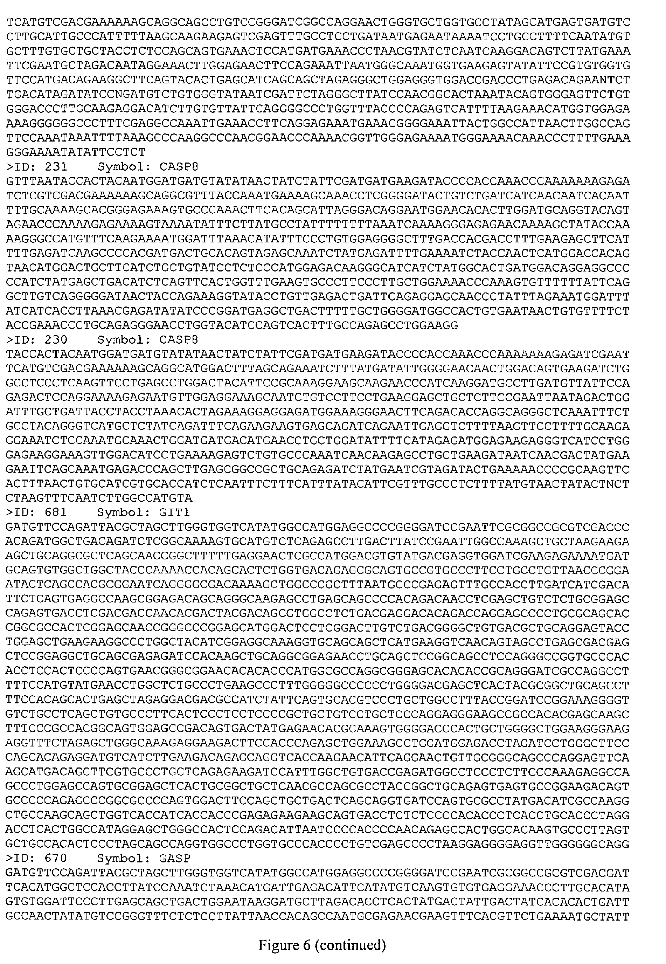

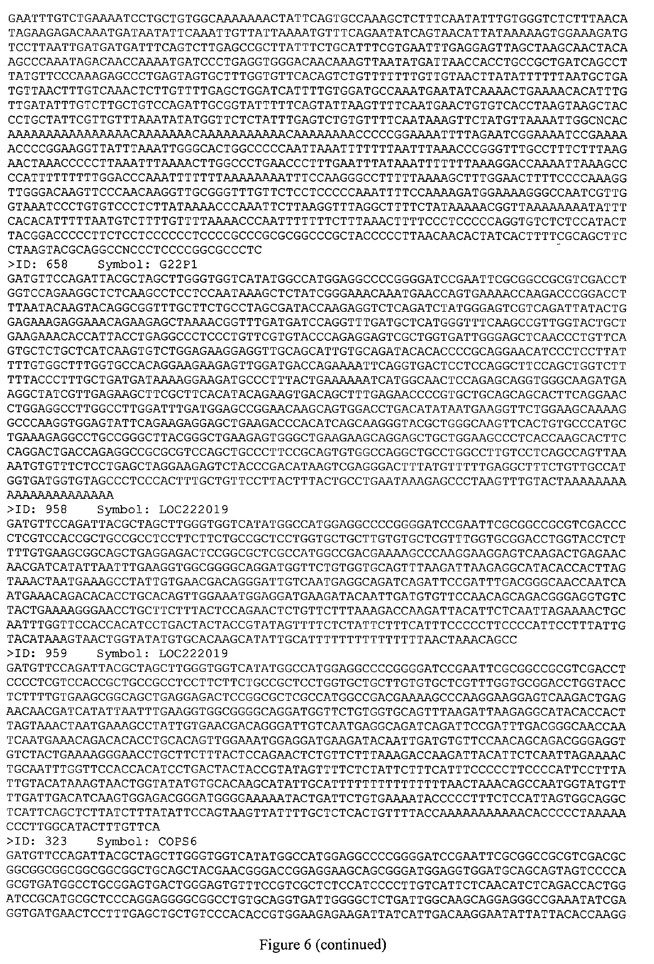

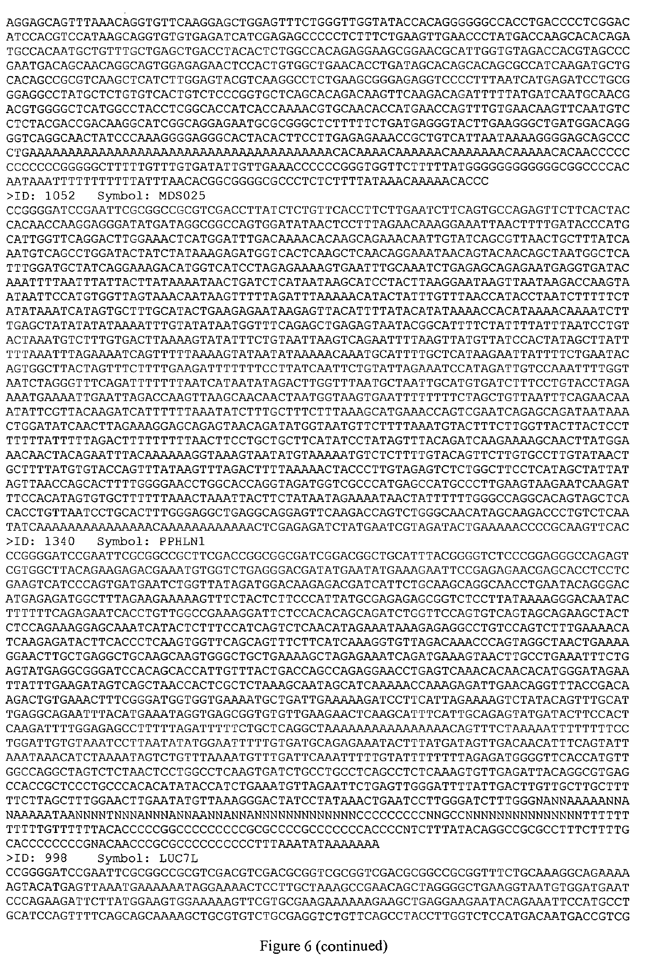

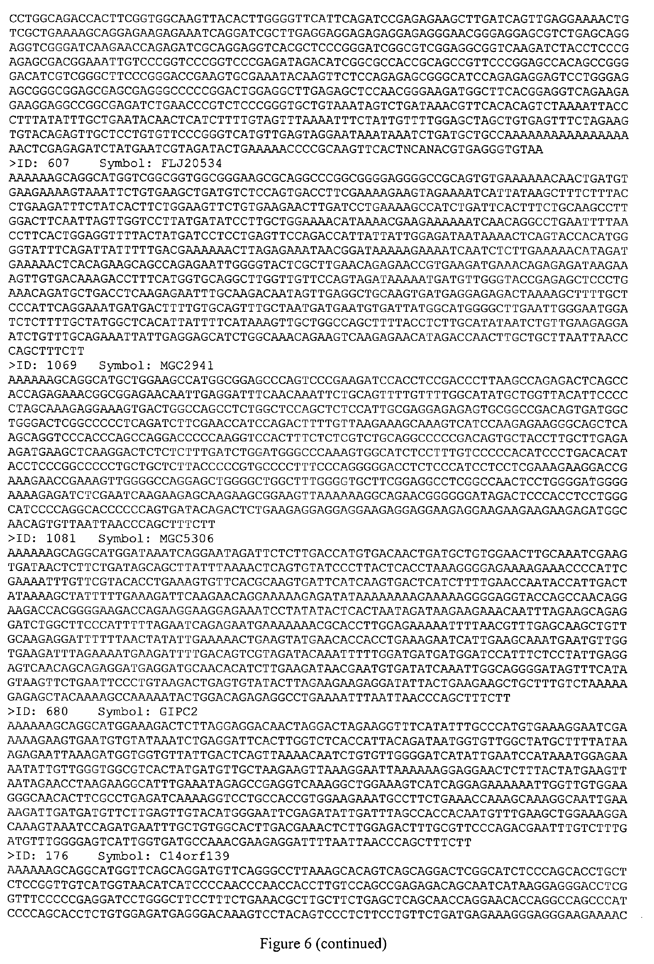

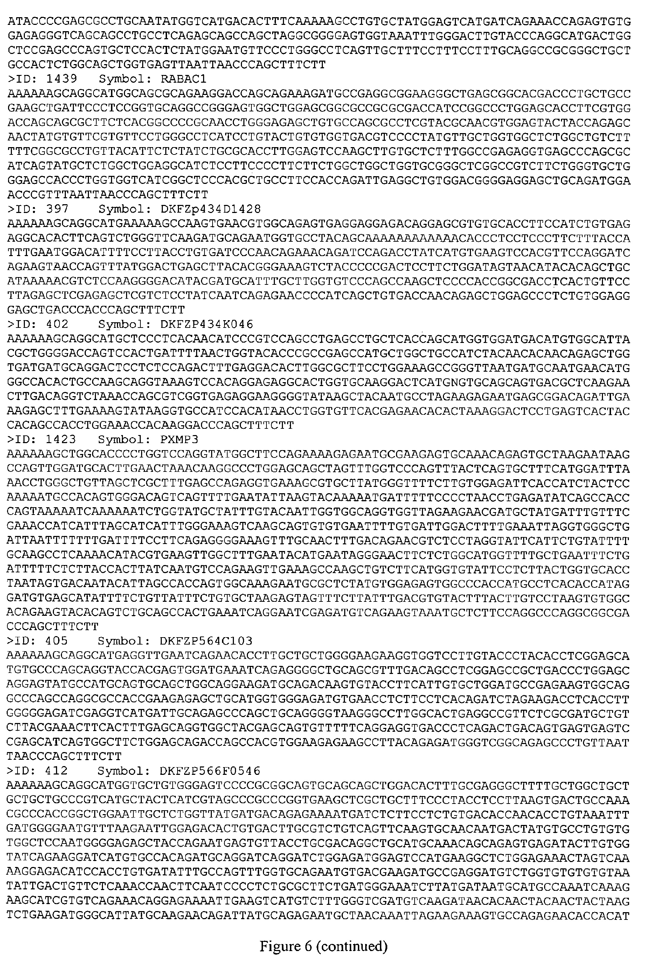

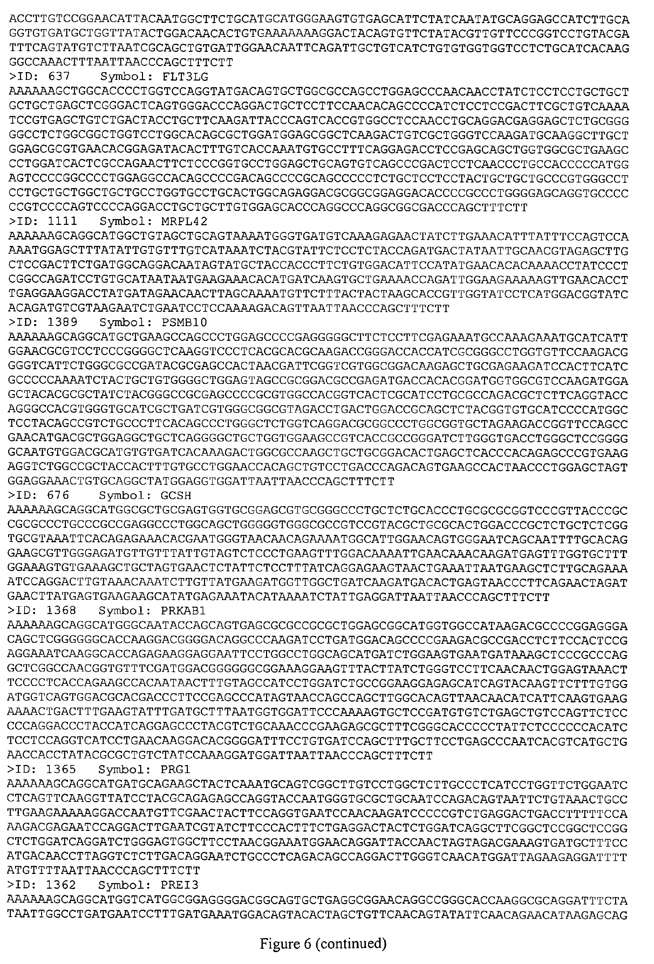

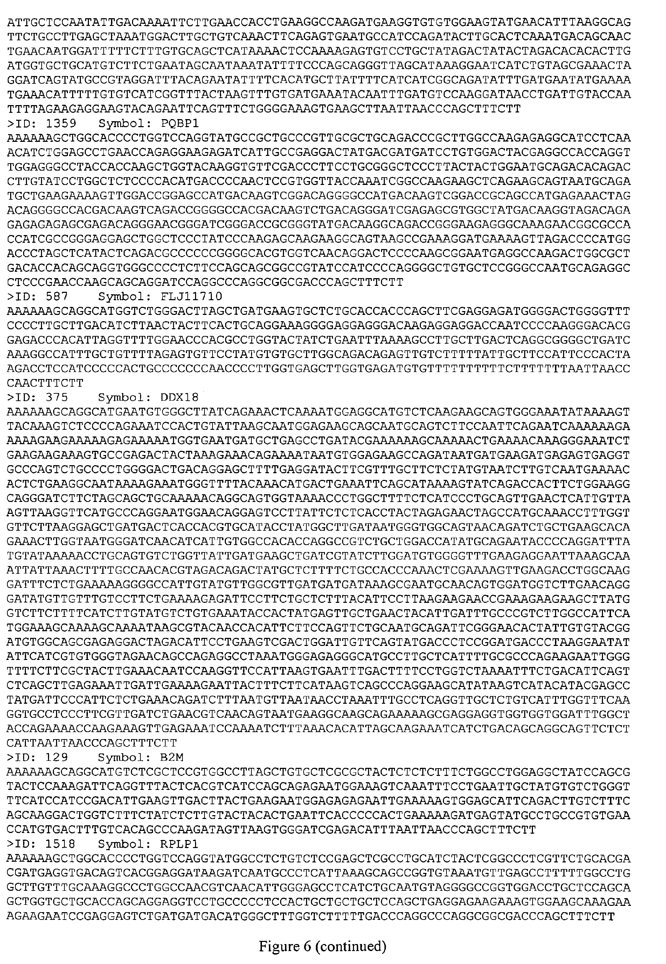

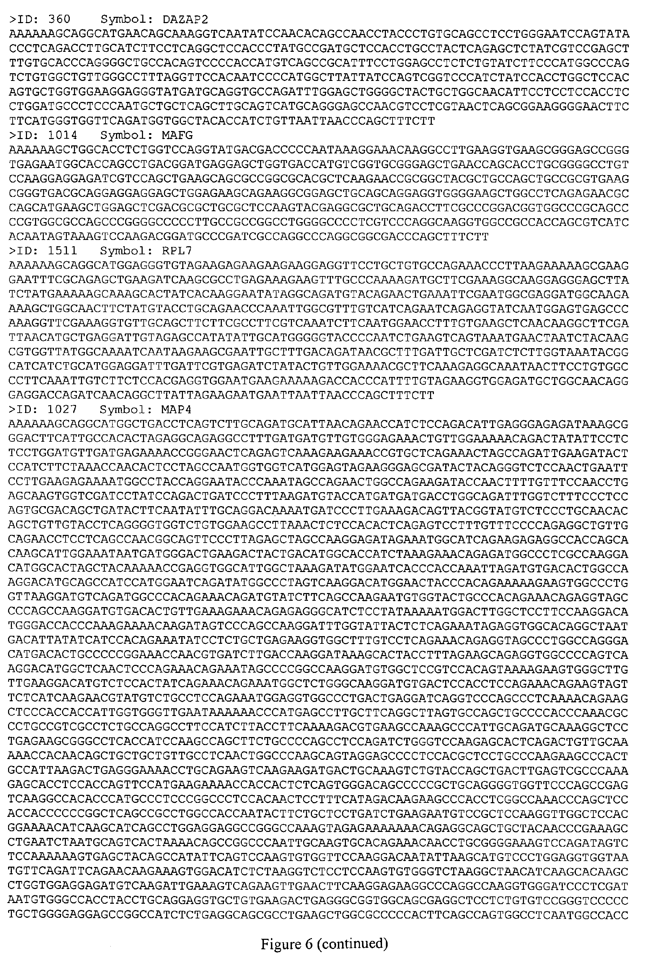

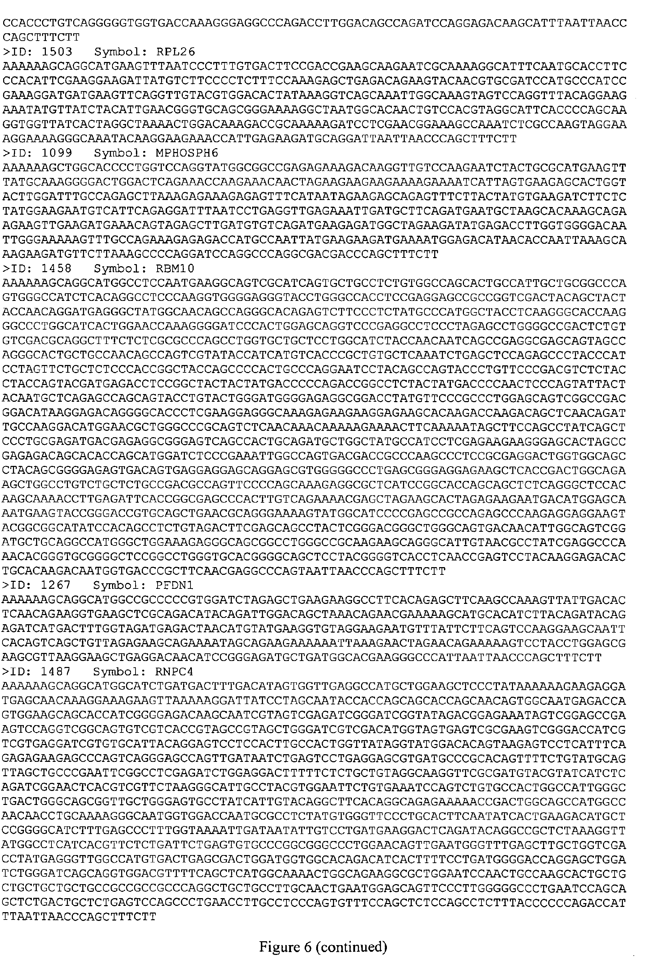

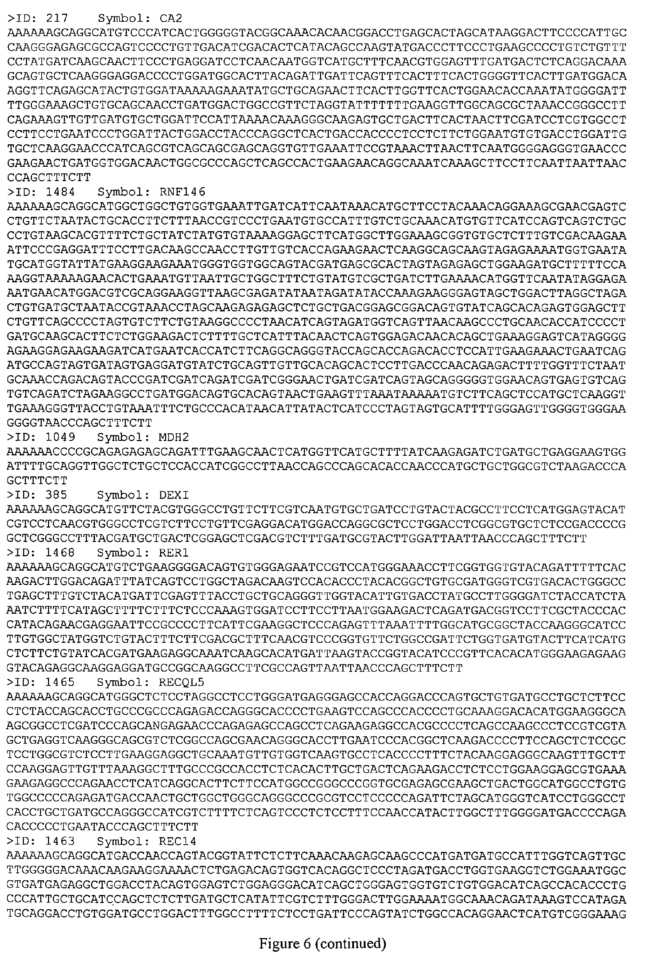

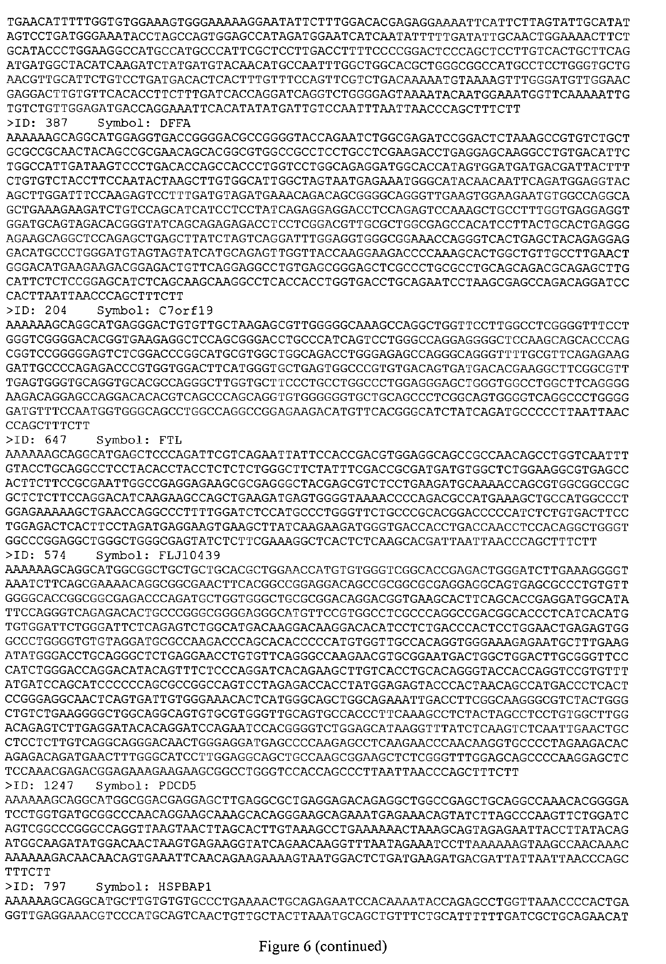

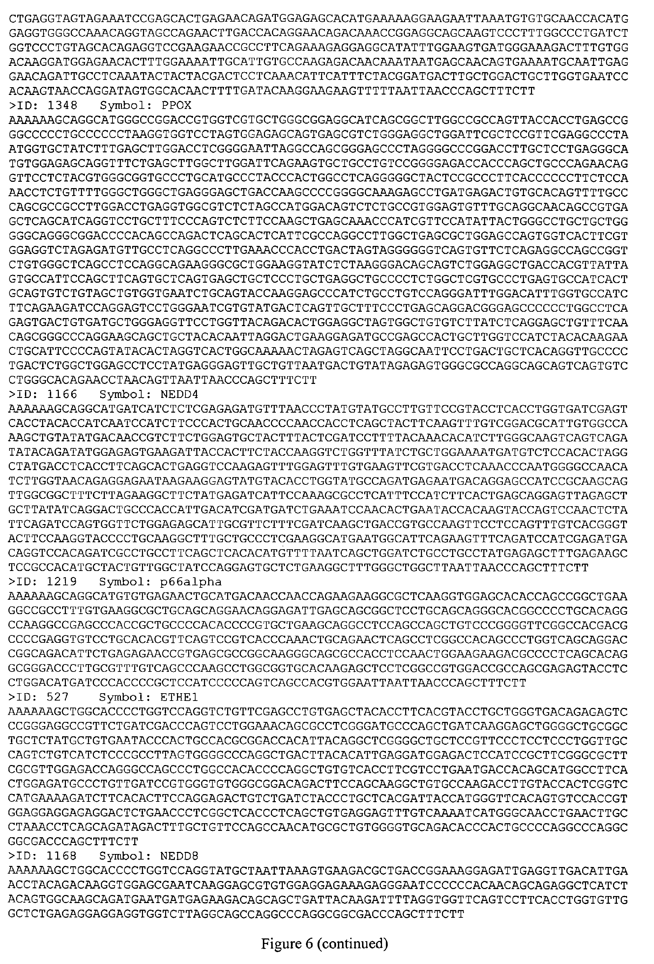

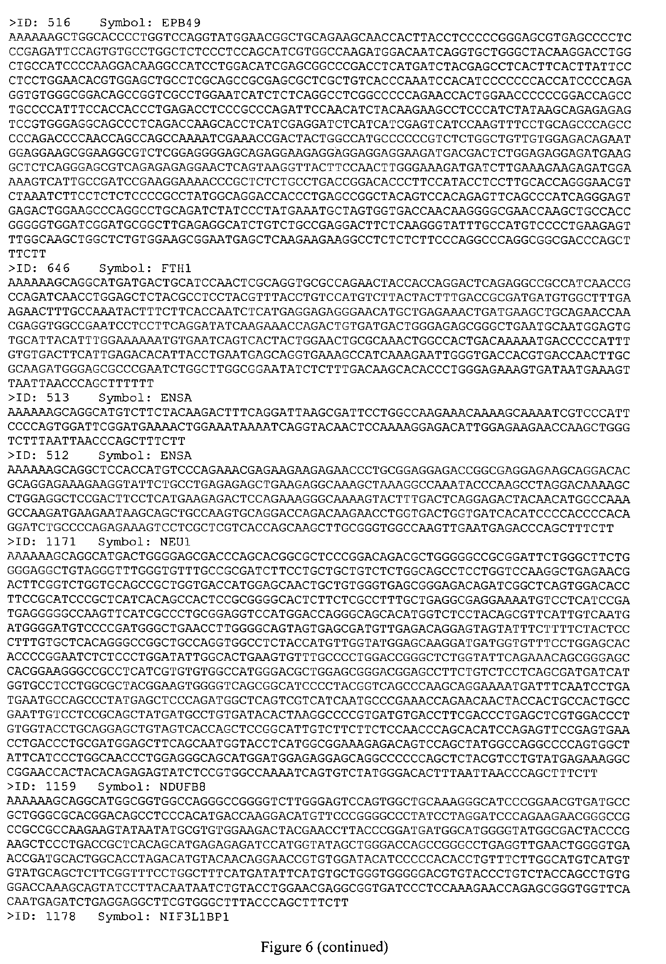

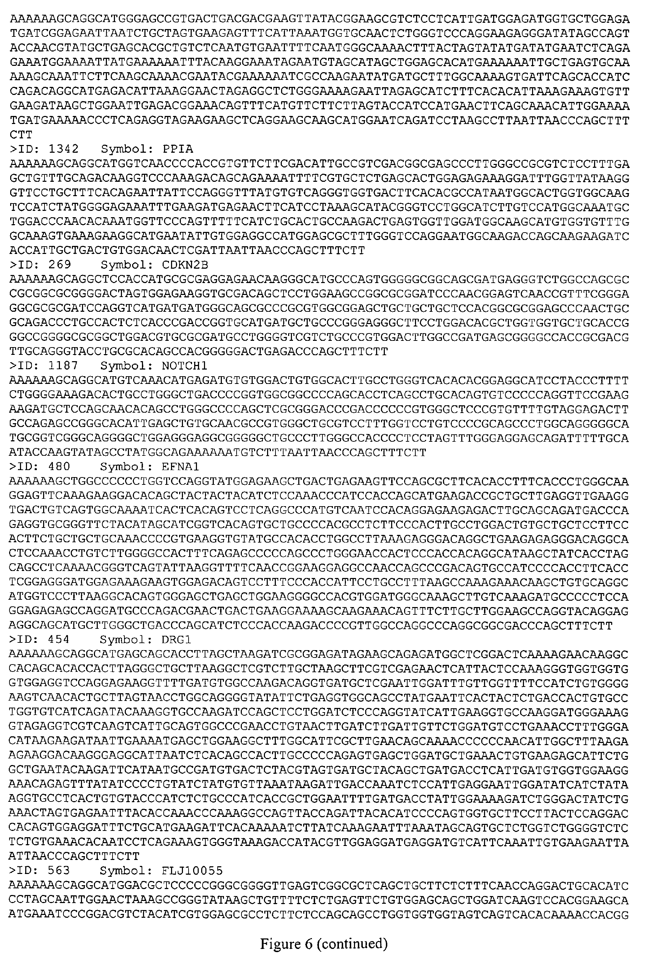

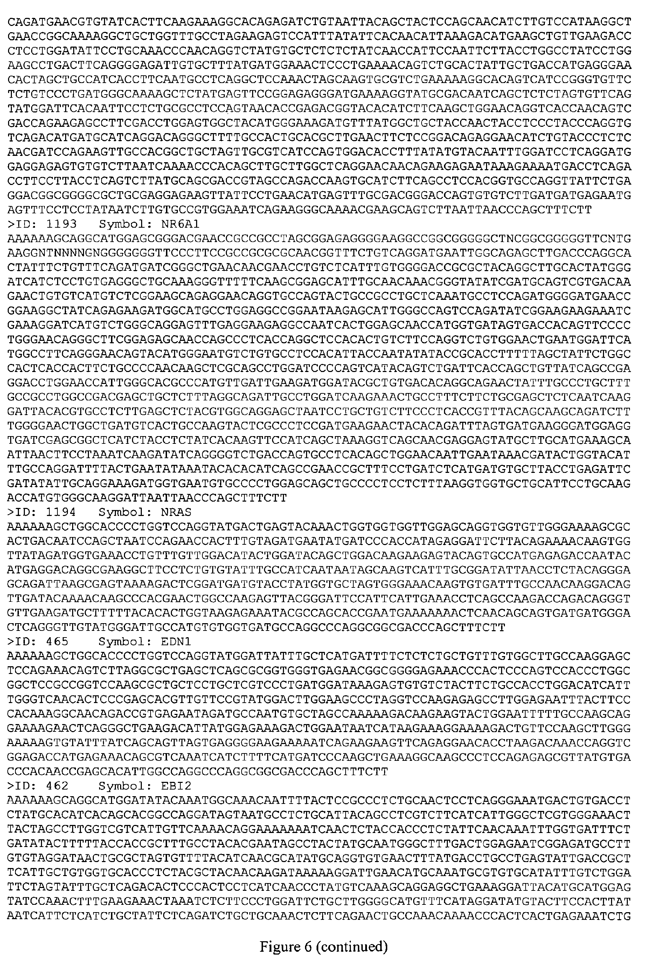

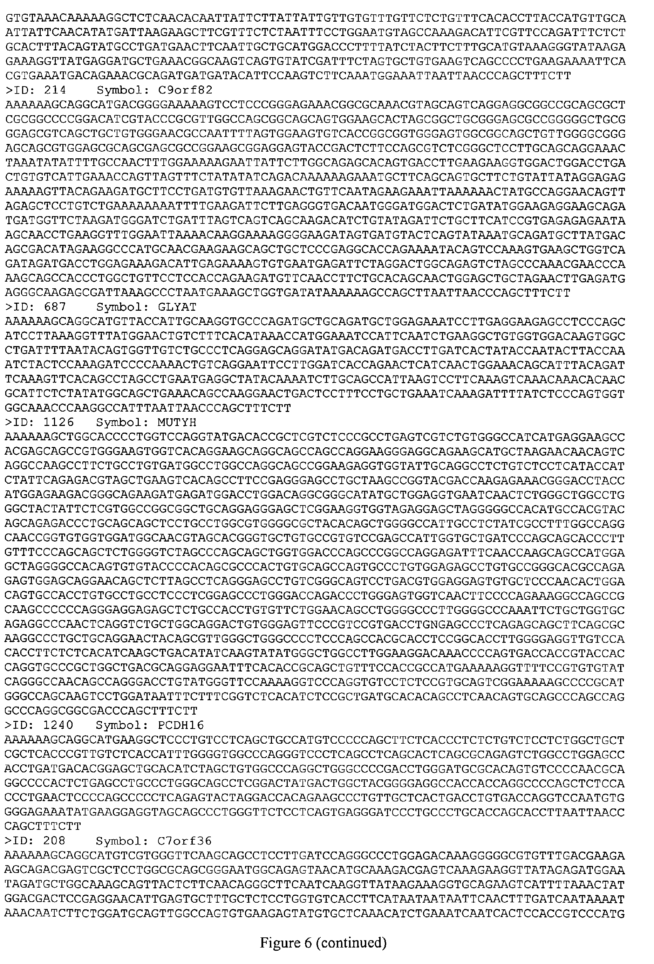

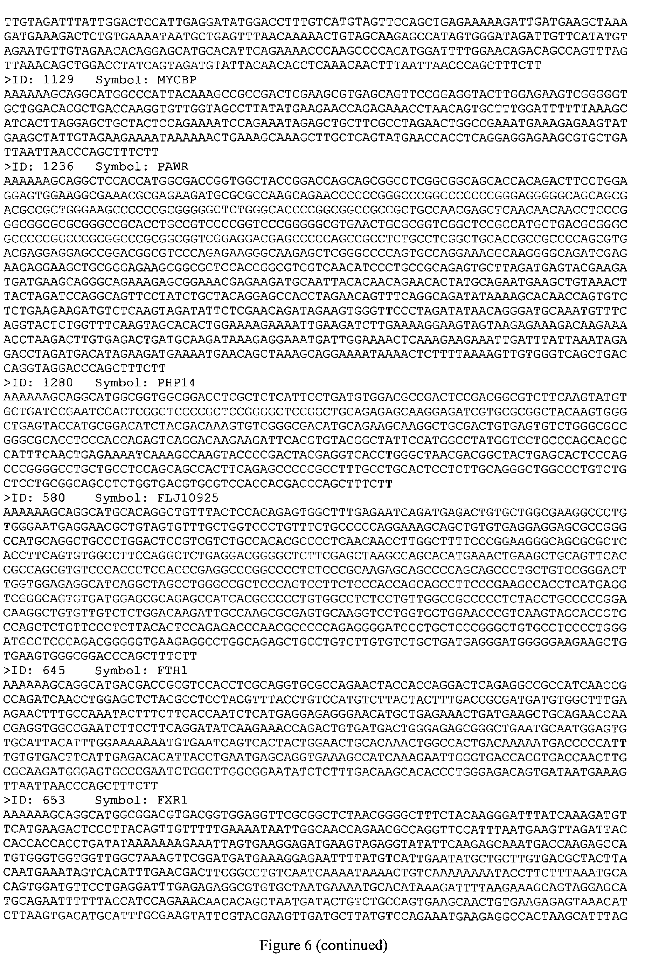

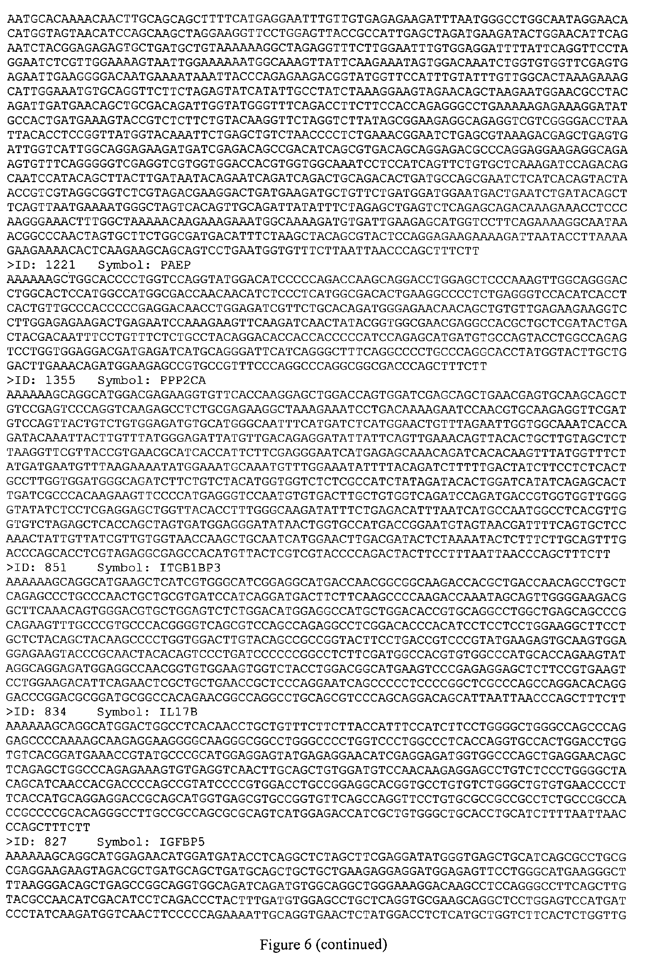

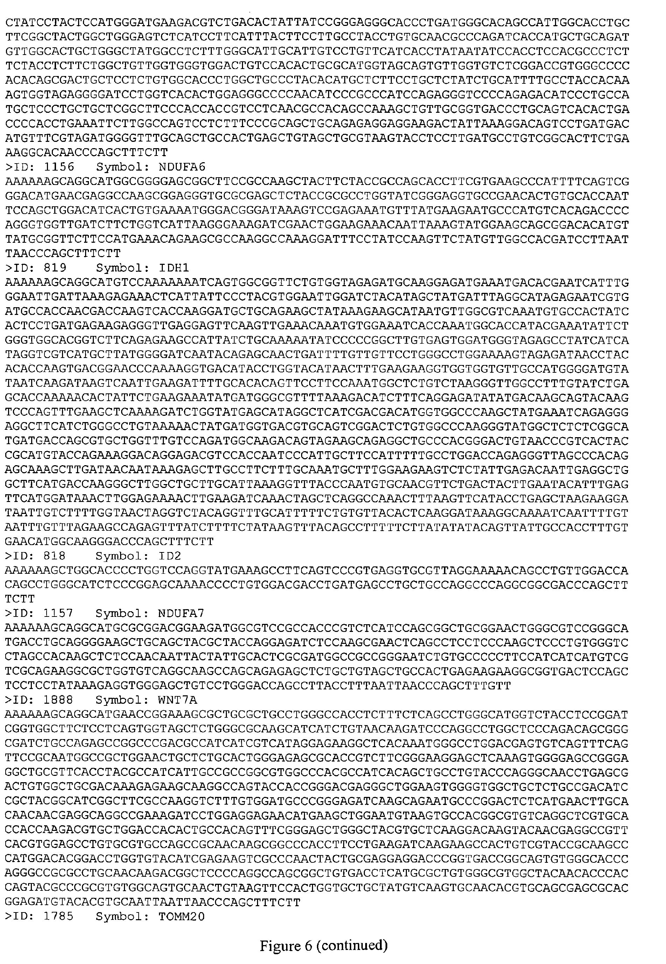

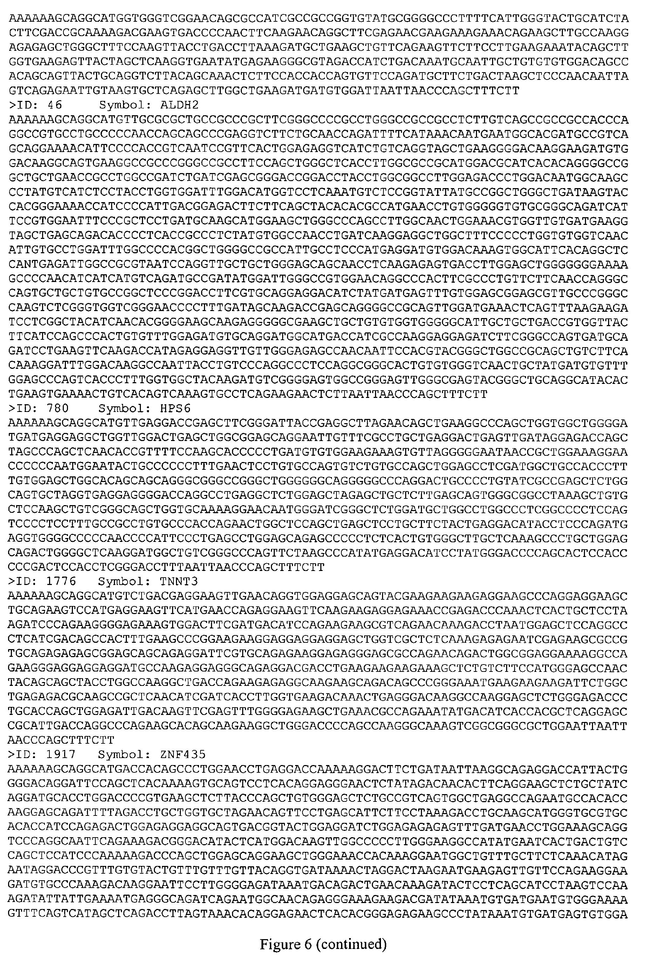

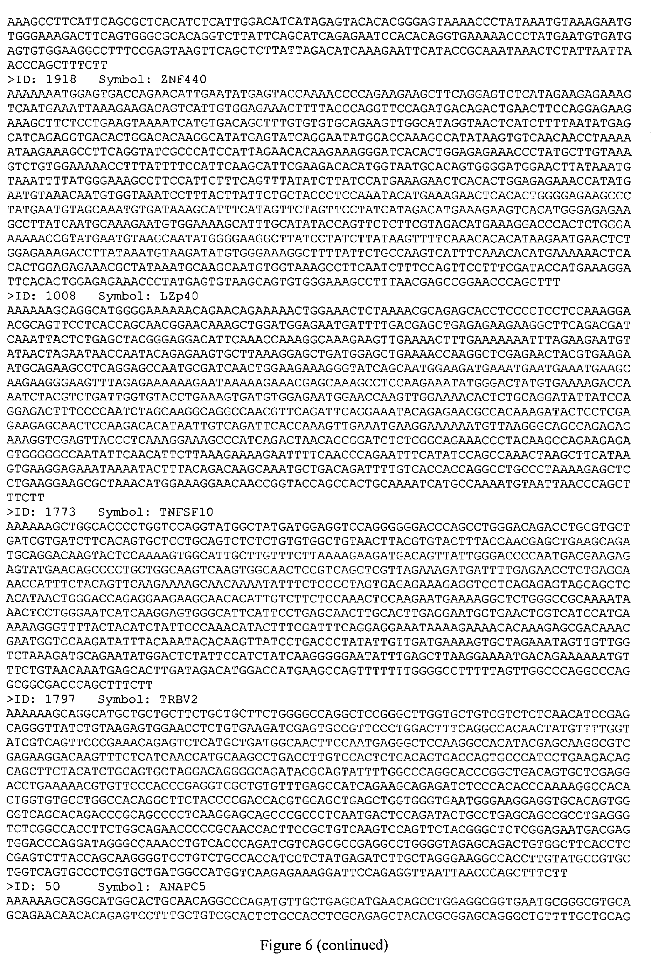

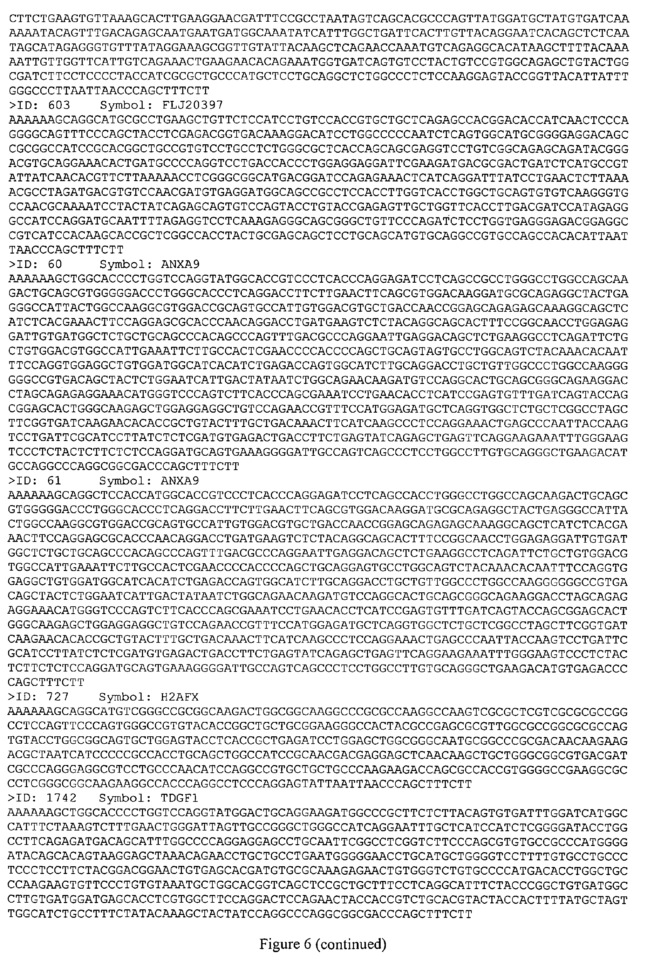

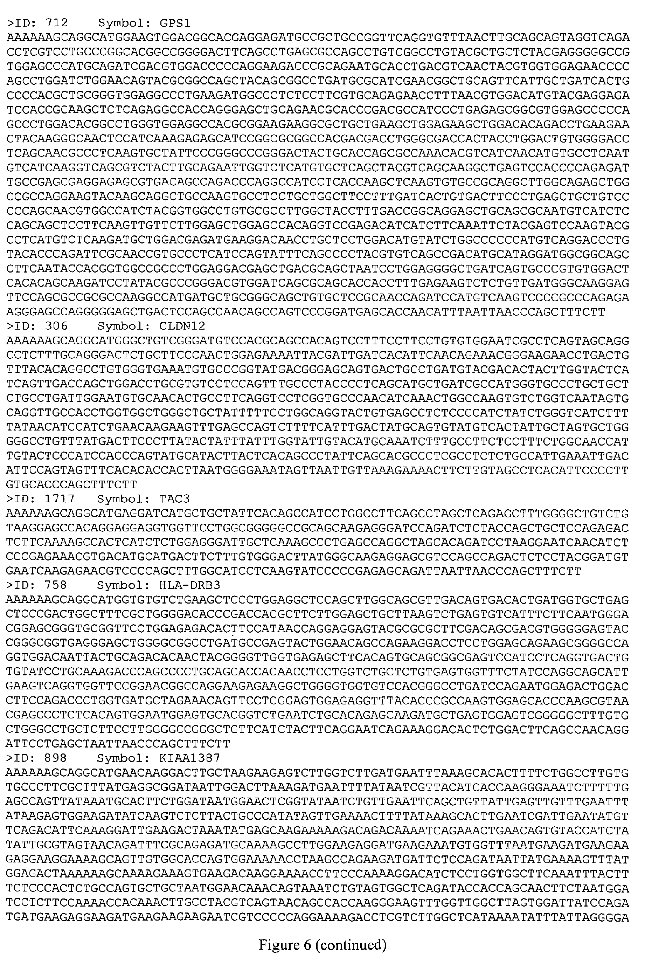

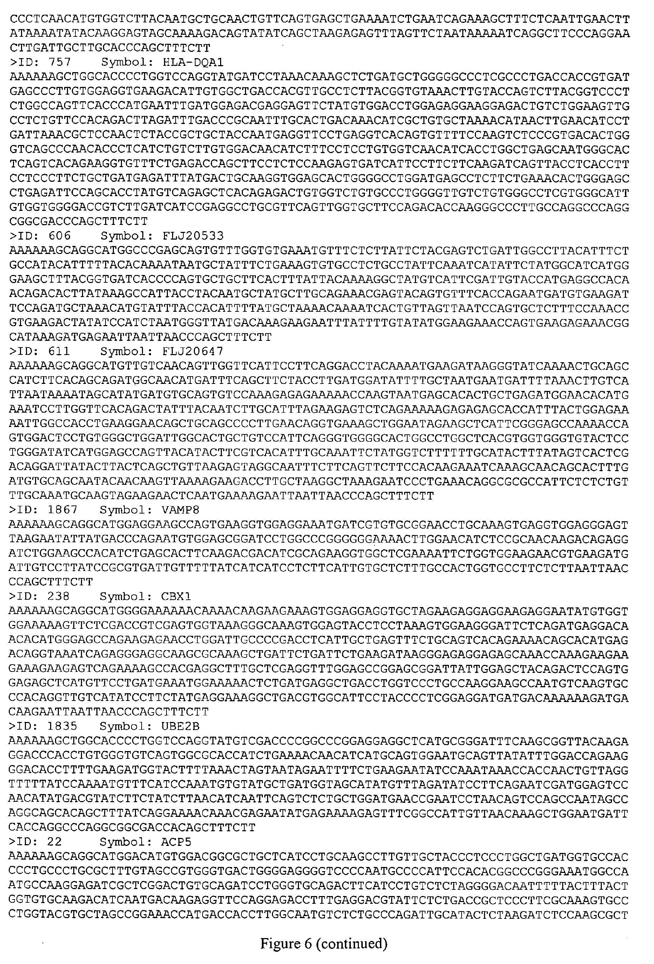

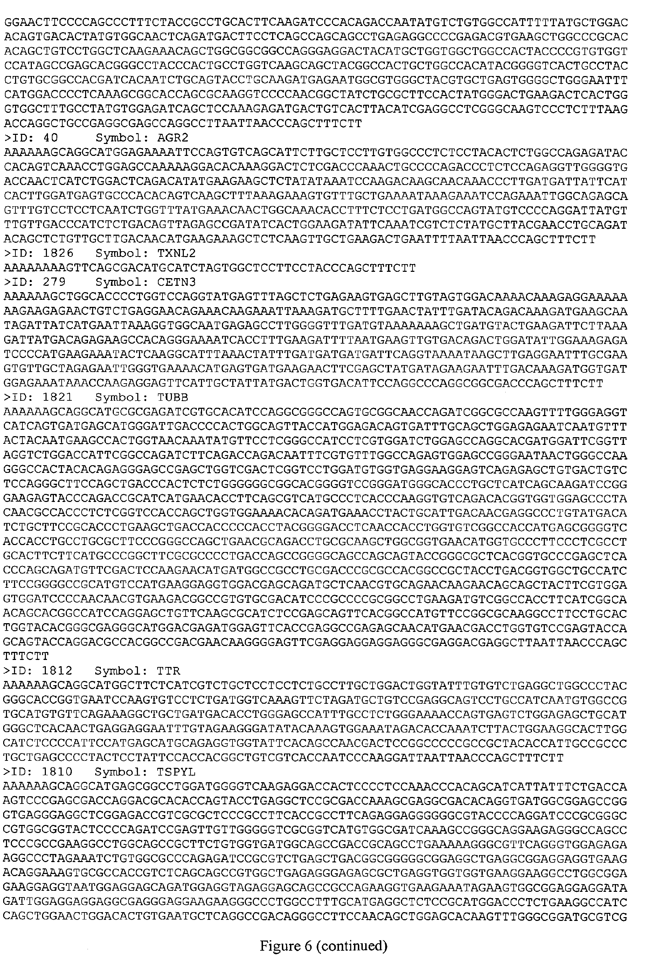

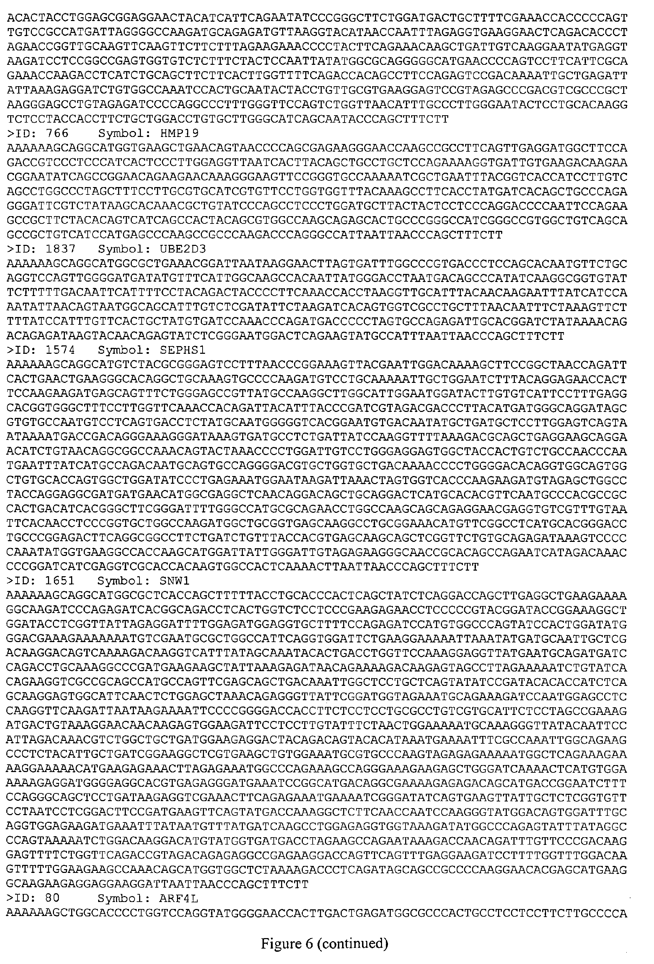

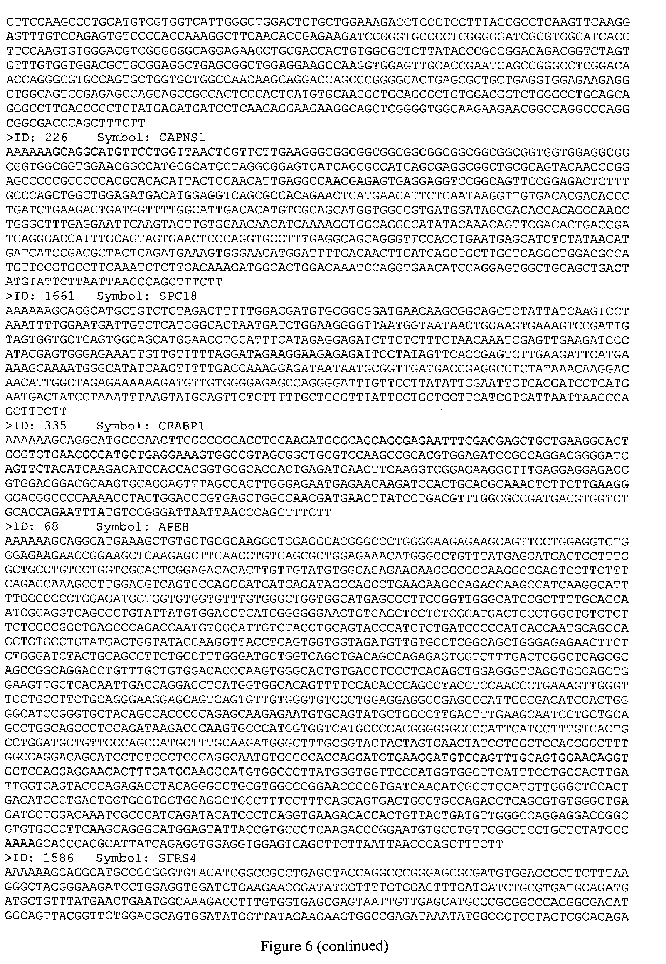

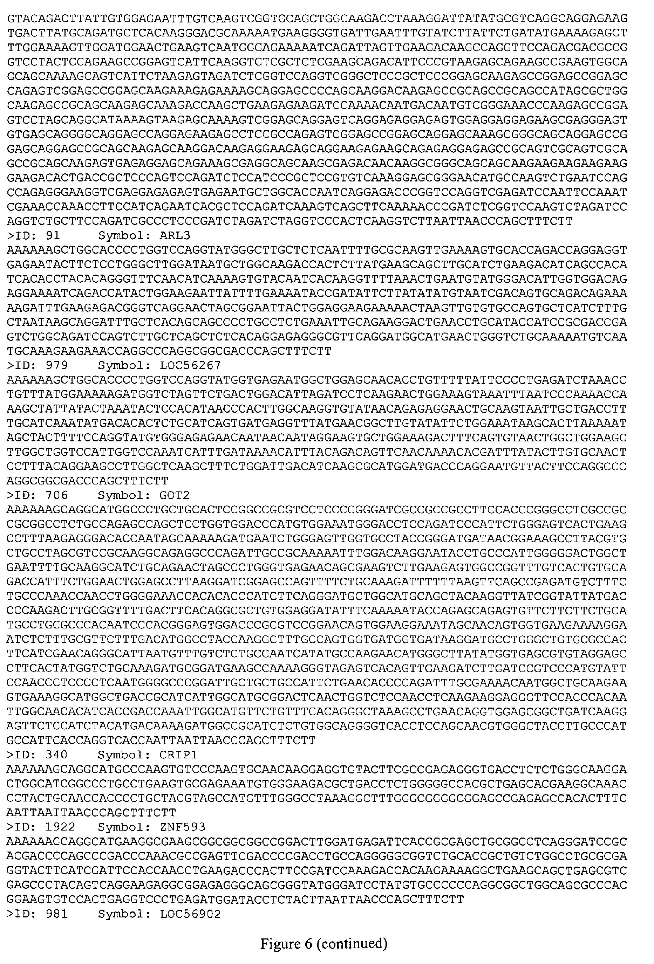

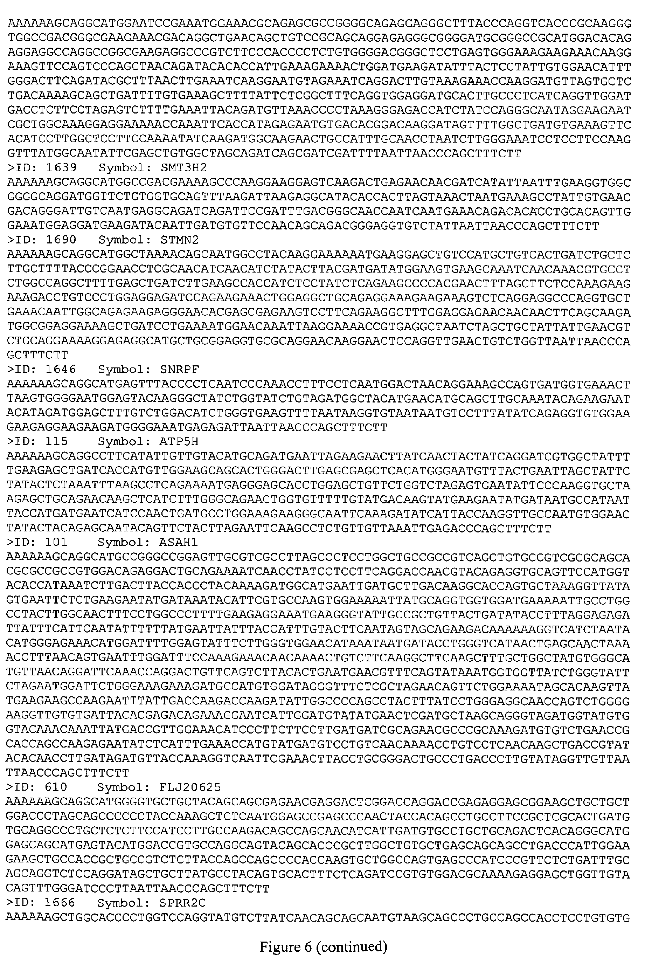

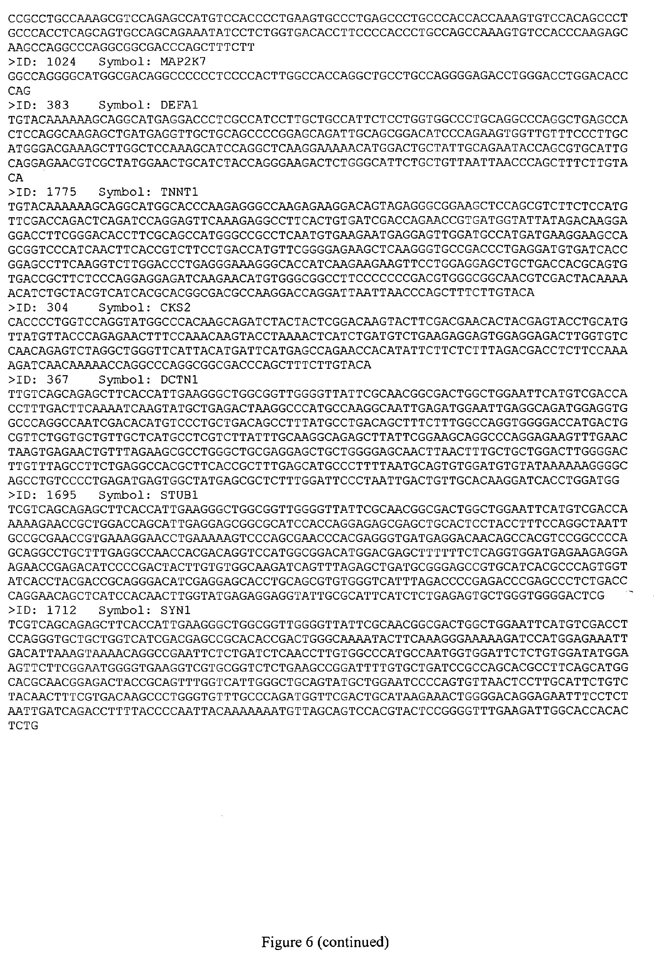

In another preferred embodiment of the present invention's method said nucleic acid molecules identified by said at least partial 5' sequencing are completely sequenced and further characterized by bioinformatic means. Examples of partially 5' sequenced nucleic acid molecules as identified by the present invention's method are further illustrated in Figure 6.

The term "bioinformatic means" in connection with the present invention relates to, but is not limited to, for example, base calling and quality clipping of sequencer trace data with PHRED (trim_cutoff = 0.05), vector sequence clipping with CROSS_MATCH. cDNAs are translated into protein sequences (Transseq, EMBOSS) with an open reading frame of more than 50 amioacids and BLASTP against the nr (NCBI) or TrEMBL (Swiss-Prot) protein databases with e-values less than 10⁻¹⁵. Hits with the highest SCORE and IDENTIY are selected. The sequences are clustered using the GAP3 program, and the longest open reading frame is selected in every Contig to obtain a non-redundant set of cDNAs encoding for human proteins. The cDNAs are then mapped to a NCBI gene locus and an official gene name is assigned.

In another preferred embodiment of the present invention's method, said first and said second suitable host cell is a prokaryotic or a eukaryotic cell. Said eukaryotic cell preferably is a mammalian cell.

In another preferred embodiment of the present invention's method said first suitable host cell is a MAT alpha yeast cell, and said second suitable host cell is a MAT a yeast cell.

Preferably, said MATalpha yeast cell comprises the genetic background [*MATα his3Δ200 trp1-910 leu2-3,112 ade2 LYS2::(lexAop)*_{*4*}*-HIS3 URA3::(lexAop)*_{*8*}*-lacZ GAL4 gal80 can* 1 *cyh2*]*.*

Preferably, said MATa yeast cell comprises the genetic background *[MATa his3Δ200 trp1-901 leu2-3*,*112 LYS2::(lexAop)*_{*4*}*-HIS3 ura3::(lexAop)*_{*8*}*-lacZ ADE2::(lexAop)*_{*8-*}*URA3 GAL4 gal80 can 1 cyh*2].

In another preferred embodiment of the present invention's method said proteins expressed in the mated host cell are chimaeric proteins.

The term "chimaeric protein" relates to bait and prey proteins fused with a DNA or an activation domain respectively.
In another preferred embodiment of the present invention's method said selection of the mated host cell comprises the step of carrying out a beta-Galactosidase assay.

It is envisaged to carry out said beta-Galactosidase assay as described in the following paragraph:

Experimentally, the diploid yeast is spotted in a high density format (3x3) on nylon membranes that are placed on selective SD4 solid agar medium. After growth for 7 d at 30 °C, yeast colonies that have been formed in the selective media in the absence of histidine and uracil are assayed for the activation of the third, the lacZ, reporter. Yeast colonies are subjected to freeze and thaw cycles in liquid nitrogen in order to lyse the cells partially. The membranes are placed on filter paper that is soaked with the X-Gal substrate in Z-buffer. The membrane is incubated on the filter for 4 h at 37 °C. Positive interactions are detected by blue staining of the colonies.

In another preferred embodiment of the present invention's method the identified protein-protein interactions are biologically evaluated.

Said biological evaluation can be carried out, but is not limited to, as described in the following paragraph:

Experimentally, randomly selected interactions are verified by in vitro Ni-Agarose pull down assays and co-immunoprecipitation. Interactions recapitulated independently in these assays are unlikely to be experimental false positives. Interactions can be verified with a rate of approximately 65 %.
Interactions are verified statistically by bioinformatic means. The term "bioinformatic means" in connection with the novel statistical verification method and the novel quality scoring system in the present invention relates to the assignments of D. *melanogaster, C. elegans* and *S. cerevisiae* orthologous proteins, by calculating pairwise orthologue clusters with the InParanoid program (version 1.35), using standard algorithm parameters. About 60% of the proteins identified with the method of the present invention have orthologues in *D. melanogaster,* and *C. elegans* and about 30% have homologues in *S. cerevisiae.* Mathematical random sets are computed and scale free random versions of the network are established. Network parameters for the experimental data, the random networks and the networks from homolgous organisms are subsequently calculated, for example, with a modified version of TopNet. Common three and four loop motifs in an *in silico* combined human, *D. melanogaster, C. elegans* and *S. cerevisiae* protein interaction network are identified with the experimental network. The loop motifs are computed with the random networks. The loop motifs are then compared with the experimental and randomly computed networks by means of a Z-calculation.
Quality scores are then assigned to individual interactions according to experimental and bioinformatic verificiation data. Quality scores are added and grouped into quality sets according to quality scores. The scoring system provides a first judgment indicating possible biological importance of the interaction.

One embodiment of carrying out the above process is further illustrated in Examples 3 and 4 and Figures 2 to 4.

The present invention further relates to a nucleic acid molecule encoding a protein interaction partner identified by the method of the invention, wherein said protein interaction partner is a protein selected from any one of tables S1, S2 or S6.

In a preferred embodiment, the present invention's nucleic acid molecule is DNA, or RNA, and preferably cDNA, or genomic DNA or synthetic DNA or mRNA

In another preferred embodiment of the invention, the nucleic acid molecule is double stranded or single stranded.

In another preferred embodiment of the invention, the nucleic acid molecule is of vertebrate, nematode, insect, bakterium or yeast. Preferably, the nematode is Caenorhabditis elegans. In another more preferred embodiment of the present invention, the insect is Drosophila, preferably Drosophila melanogaster. In another more preferred embodiment of the present invention, the vertebrate is human, mouse rat, Xenopus laevis, zebrafish.

In yet another preferred embodiment of the present invention, the nucleic acid molecule is fused to a heterologous nucleic acid molecule. In a further preferred embodiment of the present invention, the heterologous protein encoded by said heterlogous nucleic acid molecule is an immunoglobulin Fc domain.

In another preferred embodiment of the present invention the nucleic acid molecule is labeled. Labeled nucleic acid molecules may be useful for purification or detection. Suitable labels include fluorochromes, e.g. fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein (JOE), 6-carboxy-X-rhodamine(ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), radioactive labels, e.g. ³²P, ³⁵S, ³H; etc. The label may also be a two stage system, where the DNA is conjugated to biotin, haptens, etc. having a high affinity binding partner, e.g. avidin, specific antibodies, etc., where the binding partner is conjugated to a detectable label. In the case of amplification the label may be conjugated to one or both of the primers. The pool of nucleotides used in the amplification may also be labeled, so as to incorporate the label into the amplification product. Alternatively, the double strand formed after hybridization can be detected by anti-double strand DNA specific antibodies or aptamers etc.

In a more preferred embodiment said heterologous nucleic acid molecule encodes a heterologous polypeptide. Preferably said heterologous protein, fused to the protein encoded by the nucleic acid molecule of the present invention, is a DNA binding protein selected from the group consisting of GAL4 (DBP) and LexA (DBP). Also preferred in accordance with the present invention are activation domains selected from the group consisting of GAL4(AD) and VP16(AD). Also preferred are proteins selected from the group consisting of GST, His Tag, Flag Tag, Tap Tag, HA Tag and Protein A Tag.

Thus, the sequence encoding the protein may be fused to a marker sequence, such as a sequence encoding a peptide which facilitates purification of the fused protein. In certain preferred embodiments of this aspect of the invention, the marker amino acid sequence is a hexa-histidine peptide, such as the tag provided in a pQE vector (QIAGEN, Inc., 9259 Eton Avenue, Chatsworth, CA, 91311), among others, many of which are commercially available. As described in Gentz *et al., Proc. Natl. Acad. Sci. USA* 86:821-824 (1989), for instance, hexa-histidine provides for convenient purification of the fusion protein. The "HA" tag is another peptide useful for purification which corresponds to an epitope derived from the influenza hemagglutinin protein, which has been described by Wilson *et al*., *Cell 37*: *767* (1984).

The protein may be expressed in a modified form, such as a fusion protein, and may include not only secretion signals, but also additional heterologous functional regions. For instance, a region of additional amino acids, particularly charged amino acids, may be added to the N-terminus of the protein to improve stability and persistence in the host cell, during purification, or during subsequent handling and storage. Also, peptide moieties may be added to the protein to facilitate purification. Such regions may be removed prior to final preparation of the protein. The addition of peptide moieties to proteins to engender secretion or excretion, to improve stability and to facilitate purification, among others, are familiar and routine techniques in the art. A preferred fusion protein comprises a heterologous region from immunoglobulin that is useful to stabilize and purify proteins.
The present invention also relates to a method of producing a vector comprising the nucleic acid molecule the present invention. Furthermore, the present invention relates to a vector produced said method.

The present invention also relates to a vector comprising the nucleic acid molecule of the present invention. Preferably said vector is a transfer or expression vector selected from the group consisting of pACT2; pAS2-1; pBTM116; pBTM117; pcDNA3.1; pcDNAI; pECFP; pECFP-C1; pECFP-N1; pECFP-N2; pECFP-N3; pEYFP-C1; pFLAG-CMV-5 a, b, c; pGAD10; pGAD424; pGAD425; pGAD427; pGAD428; pGBT9; pGEX-3X1; pGEX-5X1; pGEX-6P1; pGFP; pQE30; pQE30N; pQE30-NST; pQE31; pQE31N; pQE32; pQE32N; pQE60; pSE111; pSG5; pTET-CMV-AS; pTET-CMV-F°-AS; pTET-CMV-F°-S; pTET-CMV-MCS; pTET-CMV-S; pTK-Hyg; pTL1; pTL10; pTL-HA0; pTL-HA1; pTL-HA2; pTL-HA3; pBTM118c; pGEX-6P3; pACGHLT-C; pACGHLT-A; pACGHLT-B; pUP; pcDNA3.1-V5His; pMalc2x. Preferably, the bait and prey vectors are selected from the group consisting of pGAD424, pGAD424(x), pGAD424rev(x), pGAD-C(1,2,3), pOAD, pC-ACT.1, pC-ACT.2, pGADGH, pGAD10, pACT1, pACT2, pPC86, pNGAD-L(1,2,3), pJG4-5, pMW102, pMW104, pNB42(1,2,3), pNB42 (1R,2R,3R), pVP16, pSD.06 (a,b,c), pWITCH(T), pWITCH(U), pASV(x), pDEST™22, pGBT9, pGBT9(x), pGBT9rev(x), pGBD-C(1,2,3), pGBDU-C(1,2,3), pGBDK-C(1,2,3), pGBREP, pOBD-2, pGB-MEL1, pAS1, pAS2, pODB8, pPC62, pPC97, pCD.1, pCD.2, pNGBD-U(1,2,3), pBTM116, pBTM117, pFBL23, pEG202, pJK202, pMW101, pMW103, pGILDA, pGKS3, pGKS6, pGKS8, pBL1, and pDEST™32.

Said expression vectors may particularly be plasmids, cosmids, viruses or bacteriophages used conventionally in genetic engineering plasmids, cosmids, viruses and bacteriophages used conventionally in genetic engineering that comprise the aforementioned nucleic acid. Preferably, said vector is a gene transfer or targeting vector. Expression vectors derived from viruses such as retroviruses, vaccinia virus, adeno-associated virus, herpes viruses, or bovine papilloma virus, may be used for delivery of the nucleic acid into targeted cell population. Methods which are well known to those skilled in the art can be used to construct recombinant viral vectors; see, for example, the techniques described in Sambrook et al., Molecular Cloning A Laboratory Manual, Cold Spring Harbor Laboratory (1989) N.Y. and Ausubel et al., Current Protocols in Molecular Biology, Green Publishing Associates and Wiley Interscience, N.Y. (1989).

In yet a further preferred embodiment of the invention the vector contains an additional expression cassette for a reporter protein, selected from the group consisting of ß-galactosidase, luciferase, green fluorescent protein and variants thereof.

Preferably, said vector comprises regulatory elements for expression of said nucleic acid molecule. Consequently, the nucleic acid of the invention may be operatively linked to expression control sequences allowing expression in eukaryotic cells. Expression of said nucleic acid molecule comprises transcription of the sequence nucleic acid molecule into a translatable mRNA. Regulatory elements ensuring expression in eukaryotic cells, preferably mammalian cells, are well known to those skilled in the art. They usually comprise regulatory sequences ensuring initiation of transcription and, optionally, a poly-A signal ensuring termination of transcription and stabilization of the transcript, and/or an intron further enhancing expression of said nucleic acid. Additional regulatory elements may include transcriptional as well as translational enhancers, and/or naturally-associated or heterologous promoter regions. Possible regulatory elements permitting expression in eukaryotic host cells are the AOX1 or GAL1 promoter in yeast or the CMV-, SV40-, RSV-promoter (Rous sarcoma virus), CMV-enhancer, SV40-enhancer or a globin intron in mammalian and other animal cells. Beside elements which are responsible for the initiation of transcription such regulatory elements may also comprise transcription termination signals, such as the SV40-poly-A site or the tk-poly-A site, downstream of the nucleic acid molecule. Furthermore, depending on the expression system used leader sequences capable of directing the protein to a cellular compartment or secreting it into the medium may be added to the coding sequence of the aforementioned nucleic acid and are well known in the art. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an C- or N-terminal identification peptide imparting desired characteristics, e.g., stabilization or simplified purification of expressed recombinant product. In this context, suitable expression vectors are known in the art such as Okayama-Berg cDNA expression vector pcDVI (Pharmacia), pCDM8, pRc/CMV, pcDNA1, pcDNA3, the Echo™ Cloning System (Invitrogen), pSPORT1 (GIBCO BRL) or pRevTet-On/pRevTet-Off or pCI (Promega).

The present invention also relates to a method of producing a host cell comprising genetically engineering cells with the nucleic acid molecule or the vector of the present invention. The present invention also relates to a host cell produced said method. Furthermore, the present invention relates to a host cell comprising the vector of the present invention. Preferably, said host cell contains an endogenous nucleic acid molecule which is operably associated with a heterologous regulatory control sequence, including the regulatory elements contained in the vector of the present invention.

The present invention also relates to a method of producing a protein, comprising culturing the host cell of the present invention under conditions such that the protein encoded by said polynucleotide is expressed and recovering said protein.

The present invention also relates to a protein comprising an amino acid sequence encoded by a nucleic acid molecule of the present invention, or which is chemically synthesized, or is obtainable from the host cell of the present invention, or which is obtainable by a method of the present invention or which is obtainable from an in vitro translation system by expressing the nucleic acid molecule of the present invention or the vector of the present invention.

In another preferred embodiment of the invention, the protein is of vertebrate, nematode, insect, bakterium or yeast. Preferably, the nematode is Caenorhabditis elegans. In another more preferred embodiment of the present invention, the insect is Drosophila, preferably Drosophila melanogaster. In another more preferred embodiment of the present invention, the vertebrate is human, mouse rat, Xenopus laevis, zebrafish.

In another preferred embodiment, the protein of the present invention is fused to a heterologous protein. Such a fusion protein may include not only secretion signals, but also additional heterologous functional regions. For instance, a region of additional amino acids, particularly charged amino acids, may be added to the N-terminus of the protein to improve stability and persistence in the host cell, during purification, or during subsequent handling and storage. Also, peptide moieties may be added to the protein to facilitate purification. Such regions may be removed prior to final preparation of the protein. The addition of peptide moieties to proteins to engender secretion or excretion, to improve stability and to facilitate purification, among others, are familiar and routine techniques in the art. A preferred fusion protein comprises a heterologous region from immunoglobulin that is useful to stabilize and purify proteins.

In a preferred embodiment of the present invention, the protein of the present invention is fused to a heterologous protein which is an immunoglobulin Fc domain or Protein A domain. In another preferred embodiment of the present invention, the protein the protein is labelled. Preferably, the label is selected from the group consisting of fluorochromes, e.g. fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein (JOE), 6-carboxy-X-rhodamine(ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), radioactive labels, e.g. ³²P, ³⁵S, ³H; etc. The label may also be a two stage system, where the protein or protein is conjugated to biotin, haptens, etc. having a high affinity binding partner, e.g. avidin, specific antibodies, etc., where the binding partner is conjugated to a detectable label. In another preferred embodiment of the present invention the label is a toxin, radioisotope, or fluorescent label.

In another preferred embodiment of the present invention, the protein contains or lacks an N-terminal methionine. it is well known in the art that the N-terminal methionine encoded by the translation initiation codon generally is removed with high efficiency from any protein after translation in all eukaryotic cells. While the N-terminal methionine on most proteins also is efficiently removed in most prokaryotes, for some proteins this prokaryotic removal process is inefficient, depending on the nature of the amino acid to which the N-terminal methionine is covalently linked.

The present invention also relates to a protein complex comprising at least two proteins, wherein said at least two proteins are selected from the group of interaction partners listed in table S2 and/or S6. The term "protein complex" refers to a compound stably comprising at least two proteins. Preferably, said stability allows to purify said protein complex.

The present invention also relates to an antibody specifically recognizing the protein of the present invention or specifically reacting with the protein complex of the present invention. This antibody is characterized in not recognizing the individual components of the protein complex but rather the complex itself. As such, said antibody recognizes a combined epitope, composed of amino acids of two different proteins within the protein complex. Dissociation of the complex will be detrimental to antibody recognition. Therefore, antibody binding depends on the integrity of the protein complex.

In a preferred embodiment, the antibody of the present invention is polyclonal, monoclonal, chimeric, single chain, single chain Fv, human antibody, humanized antibody, or Fab fragment

In a more preferred embodiment of the present invention the antibody is labeled. Preferably, the label is selected from the group consisting of fluorochromes, e.g. fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein (JOE), 6-carboxy-X-rhodamine(ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA), radioactive labels, e.g. ³²P, ³⁵S, ³H; etc. The label may also be a two stage system, where the antibody is conjugated to biotin, haptens, etc. having a high affinity binding partner, e.g. avidin, specific antibodies, etc., where the binding partner is conjugated to a detectable label. In another preferred embodiment of the present invention the label is a toxin, radioisotope, or fluorescent label.

In a preferred embodiment of the present invention, the antibody is immobilized to a solid support. Preferably, the solid support may be the surface of a cell, a microtiter plate, beads or the surface of a sensor capable of detecting binding of the antibody or to the antibody.
In another preferred embodiment of the present invention, the methods for identifying a compound further comprise producing said compound. In yet another preferred embodiment of the present invention, the method for identifying said compound further comprise modifiying to achieve (i) modified site of action, spectrum of activity, organ specificity, and/or (ii) improved potency, and/or (iii) decreased toxicity (improved therapeutic index), and/or (iv) decreased side effects, and/or (v) modified onset of therapeutic action, duration of effect, and/or (vi) modified pharmakinetic parameters (resorption, distribution, metabolism and excretion), and/or (vii) modified physico-chemical parameters (solubility, hygroscopicity, color, taste, odor, stability, state), and/or (viii) improved general specificity, organ/tissue specificity, and/or (ix) optimized application form and route by (i) esterification of carboxyl groups, or (ii) esterification of hydroxyl groups with carbon acids, or (iii) esterification of hydroxyl groups to, e.g. phosphates, pyrophosphates or sulfates or hemi succinates, or (iv) formation of pharmaceutically acceptable salts, or (v) formation of pharmaceutically acceptable complexes, or (vi) synthesis of pharmacologically active polymers, or (vii) introduction of hydrophilic moieties, or (viii) introduction/exchange of substituents on aromates or side chains, change of substituent pattern, or (ix) modification by introduction of isosteric or bioisosteric moieties, or (x) synthesis of homologous compounds, or (xi) introduction of branched side chains, or (xii) conversion of alkyl substituents to cyclic analogues, or (xiii) derivatisation of hydroxyl group to ketales, acetales, or (xiv) N-acetylation to amides, phenylcarbamates, or (xv) synthesis of Mannich bases, imines, or transformation of ketones or aldehydes to Schiffs bases, oximes, acetales, ketales, enolesters, oxazolidines, thiozolidines or combinations thereof.

The present invention also relates to a method of diagnosing a disease in a biological sample comprising the steps of (a) contacting the sample with an antibody specific for a protein of any one of tables S1, S2 or S6 or an antibody specific for the protein complex of the present invention; and (b) detecting binding of the antibody to a protein complex, wherein the detection of binding is indicative of a disease or of a predisposition to develop a disease. Preferably, binding is detected by measuring the presence of a fluorescent label bound to the protein complex.
The present invention also relates to a diagnostic agent/composition comprising the nucleic acid molecule of the present invention, the protein of the present invention including/or the protein mentioned in any of of table S1, S2 or S6, the antibody of the present invention, an antibody specifically reacting with a protein selected from any one of tables S1, S2 or S6 and/or a protein selected from any one of tables S1, S2 or S6.

Moreover, the present invention also relates to a pharmaceutical composition comprising the nucleic acid molecule of the present invention, the protein of the present invention, the interfering compound identified with a method of the present invention, the antibody of the present invention, an antibody specifically reacting with a protein selected from any one of tables S1, S2 or S6 and/or a protein selected from any one of tables S1, S2 or S6 .

The pharmaceutical composition will be formulated and dosed in a fashion consistent with good medical practice, taking into account the clinical condition of the individual patient, the site of delivery of the pharmaceutical composition, the method of administration, the scheduling of administration, and other factors known to practitioners. The "effective amount" of the pharmaceutical composition for purposes herein is thus determined by such considerations.
As a general proposition, the total pharmaceutically effective amount of pharmaceutical composition administered parenterally per dose will be in the range of about 1 µg protein /kg/day to 10 mg protein /kg/day of patient body weight, although, as noted above, this will be subject to therapeutic discretion. More preferably, this dose is at least 0.01 mg protein /kg/day, and most preferably for humans between about 0.01 and 1 mg protein /kg/day for the peptide. If given continuously, the pharmaceutical composition is typically administered at a dose rate of about 1 µg/kg/hour to about 50 µg/kg/hour, either by 1-4 injections per day or by continuous subcutaneous infusions, for example, using a mini-pump. An intravenous bag solution may also be employed. The length of treatment needed to observe changes and the interval following treatment for responses to occur appears to vary depending on the desired effect.

Pharmaceutical compositions of the invention may be administered orally, rectally, parenterally, intracistemally, intravaginally, intraperitoneally, topically (as by powders, ointments, drops or transdermal patch), bucally, or as an oral or nasal spray. By "pharmaceutically acceptable carrier" is meant a non-toxic solid, semisolid or liquid filler, diluent, encapsulating material or formulation auxiliary of any type. The term "parenteral" as used herein refers to modes of administration which include intravenous, intramuscular, intraperitoneal, intrasternal, subcutaneous and intraarticular injection and infusion.

The pharmaceutical composition is also suitably administered by sustained-release systems. Suitable examples of sustained-release compositions include semipermeable polymer matrices in the form of shaped articles, e.g., films, or mirocapsules. Sustained-release matrices include polylactides (U.S. Pat. No. 3,773,919, EP 58,481), copolymers of L-glutamic acid and gamma-ethyl-L-glutamate (Sidman, U. et al., *Biopolymers* 22:547-556 (1983)), poly (2- hydroxyethyl methacrylate) (R. Langer et al., *J*. *Biomed. Mater. Res.* 15:167-277 (1981), and R. Langer, *Chem. Tech.* 12:98-105 (1982)), ethylene vinyl acetate (R. Langer et al., Id.) or poly-D- (-)-3-hydroxybutyric acid (EP 133,988). Sustained-release pharmaceutical composition also include liposomally entrapped protein, antibody, protein, peptide or nucleic acid. Liposomes containing the pharmaceutical composition are prepared by methods known per se: DE 3,218,121; Epstein et al., Proc. Natl. Acad. Sci. (USA) 82:3688-3692 (1985); Hwang et al., Proc. Natl. Acad. Sci. (USA) 77:4030-4034 (1980); EP 52,322; EP 36,676; EP 88,046; EP 143,949; EP 142,641; Japanese Pat. Appl. 83-118008; U.S. Pat. Nos. 4,485,045 and 4,544,545; and EP 102,324. Ordinarily, the liposomes are of the small (about 200-800 Angstroms) unilamellar type in which the lipid content is greater than about 30 mol. percent cholesterol, the selected proportion being adjusted for the optimal therapy.

For parenteral administration, in one embodiment, the pharmaceutical composition is formulated generally by mixing it at the desired degree of purity, in a unit dosage injectable form (solution, suspension, or emulsion), with a pharmaceutically acceptable carrier, i.e., one that is non-toxic to recipients at the dosages and concentrations employed and is compatible with other ingredients of the formulation. For example, the formulation preferably does not include oxidizing agents and other compounds that are known to be deleterious to proteins.

Generally, the formulations are prepared by contacting the components of the pharmaceutical composition uniformly and intimately with liquid carriers or finely divided solid carriers or both. Then, if necessary, the product is shaped into the desired formulation. Preferably the carrier is a parenteral carrier, more preferably a solution that is isotonic with the blood of the recipient. Examples of such carrier vehicles include water, saline, Ringer's solution, and dextrose solution. Non-aqueous vehicles such as fixed oils and ethyl oleate are also useful herein, as well as liposomes. The carrier suitably contains minor amounts of additives such as substances that enhance isotonicity and chemical stability. Such materials are non-toxic to recipients at the dosages and concentrations employed, and include buffers such as phosphate, citrate, succinate, acetic acid, and other organic acids or their salts; antioxidants such as ascorbic acid; low molecular weight (less than about ten residues) proteins, e.g., polyarginine or tripeptides; proteins, such as serum albumin, gelatin, or immunoglobulins; hydrophilic polymers such as polyvinylpyrrolidone; amino acids, such as glycine, glutamic acid, aspartic acid, or arginine; monosaccharides, disaccharides, and other carbohydrates including cellulose or its derivatives, glucose, manose, or dextrins; chelating agents such as EDTA; sugar alcohols such as mannitol or sorbitol; counterions such as sodium; and/or nonionic surfactants such as polysorbates, poloxamers, or PEG. The proteinacous components of the pharmaceutical composition are typically formulated in such vehicles at a concentration of about 0.1 mg/ml to 100 mg/ml, preferably 1-10 mg/ml, at a pH of about 3 to 8. It will be understood that the use of certain of the foregoing excipients, carriers, or stabilizers will result in the formation protein or protein salts.

The components of the pharmaceutical composition to be used for therapeutic administration must be sterile. Sterility is readily accomplished by filtration through sterile filtration membranes (e.g., 0.2 micron membranes). Therapeutic components of the pharmaceutical composition protein compositions generally are placed into a container having a sterile access port, for example, an intravenous solution bag or vial having a stopper pierceable by a hypodermic injection needle.

The components of the pharmaceutical composition ordinarily will be stored in unit or multi-dose containers, for example, sealed ampoules or vials, as an aqueous solution or as a lyophilized formulation for reconstitution. As an example of a lyophilized formulation, 10-ml vials are filled with 5 ml of sterile-filtered 1% (w/v) aqueous protein solution, and the resulting mixture is lyophilized. The infusion solution is prepared by reconstituting the lyophilized protein using bacteriostatic Water-for-Injection.

The invention also provides a pharmaceutical/diagnostic pack or kit comprising one or more containers filled with one or more of the ingredients of the pharmaceutical/diagnostic compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the proteins of the components of the pharmaceutical composition invention may be employed in conjunction with other therapeutic compounds.

The figures show:

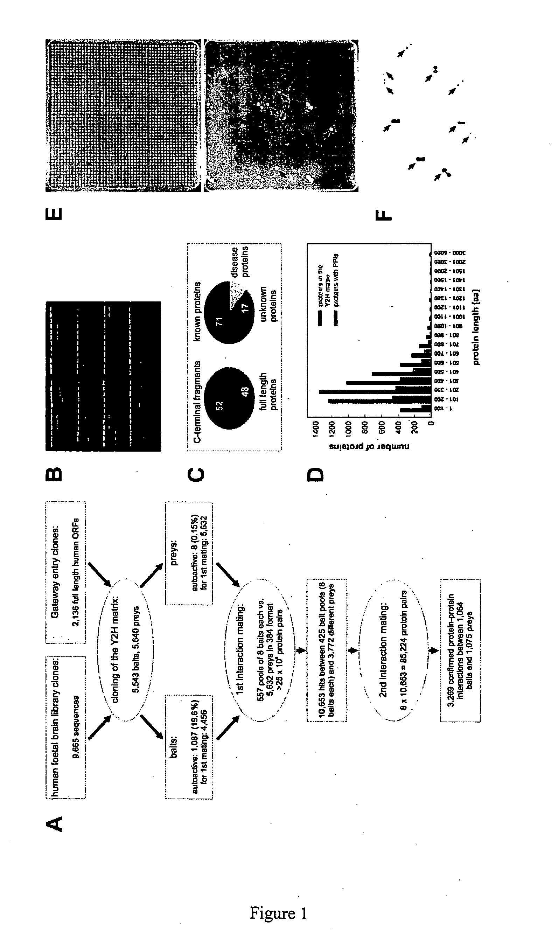

### Figure 1. Automated Y2H matrix interaction mating

**A.** Process of the systematic automated large-scale Y2H matrix interaction mating. Three major steps: (i) creation of a human protein matrix in yeast, (ii) high-throughput screening of the Y2H matrix with pools of 8 baits (1^{st} interaction mating) and (iii) confirmation of identified interactions using individual pairs of baits and preys (2^{nd} interaction mating).
**B.** Insertion of human cDNA fragments into Y2H plasmids by systematic restriction cloning in 96-well microtiter plate format. Agarose gel analysis of insert size of 32 different human cDNAs (3 clones each) after subcloning into pGAD426.
**C.** Characterization of proteins in the Y2H matrix. Left panel, fraction of full-length human proteins (orange) and C-terminal fragments (red) in the matrix. Right panel, fraction of known (red), unknown (orange) and disease proteins (yellow), according to OMIM criteria.
**D.** Length distribution of human proteins used for Y2H screening (red bars) and of proteins yielding interactions (orange bars).
**E**. Identification of Y2H PPIs using the pooled mating approach (1^{st} interaction mating). Top panel, yeast clones spotted by robot onto SDII (-Trp-Leu) agar plates to select for diploid yeasts expressing bait and prey fusions. Bottom panel, PPIs identified (arrows) by assaying the growth of diploid yeasts on SDIV (-Trp-Leu-His-Ura) agar plates.
**F**. Confirmation of Y2H interactions by analyzing yeast clones expressing single pairs of bait and prey proteins (2^{nd} interaction mating). Using spotting robot, diploid yeast clones were gridded in duplicates onto nylon membranes (3x3 pattern) placed on SDIV agar plates. After incubation for 7 days at 30°C membranes were assayed for β-galactosidase activity and positive clones were identified (arrows).

### Figure 2. Properties of the human Y2H PPI network

**A**. A global view of the Y2H network. Colored circles represent proteins (nodes): Light blue, known proteins; Yellow, proteins of unknown function; Orange, disease proteins. Interactions (links) connecting the nodes are represented by color-coded lines: High confidence (red), medium confidence (blue) and low confidence interactions (green). A giant network with 1,613 nodes and 3,131 links as well as 43 small networks with less than 6 nodes are shown.
**B**. Degree distribution of the Y2H network. The plot shows the number of proteins with a given link (k) in the network. The power-law degree distribution [P(k) ~ k^{Y}] with a degree exponent y of 1.78 indicates that the Y2H PPI network has scale-free properties.
**C**. Distribution of the clustering coefficients of the network proteins. The clustering coefficient [CCp = 2nₚ/k(k-1), with nₚ as the number of links connecting the kp neighbors of node p to each other] provides a measure for the interconnectivity in the neighborhood of a protein. The average clustering coefficient of all nodes with k links was plotted against the number of links. The distribution indicates that the Y2H network has a modular hierarchical structure.
**D.** Distribution of the shortest path (I) between pairs of proteins in the Y2H network. On average, any two proteins in the network are connected via 4.85 links, indicating that the network has small world properties.

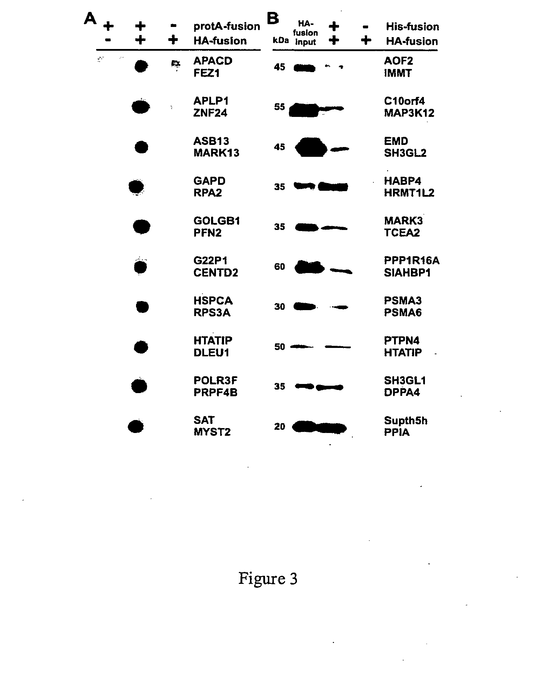

### Figure 3. Verification of Y2H interactions by coimmunoprecipitation and pull-down assays.

**A**. Membrane filter coimmunoprecipitation assay. Pairs of proteins were transiently expressed as hemagglutinin- (HA) and protein A-tagged fusions in COS-1 cells. Cleared cell lysates were filtered through a membrane coated with human IgG to retain the protein A fusion protein. After washing, the HA-tagged protein, bound to the protein A fusion partner, was detected on the membrane using anti-HA antibody. Identities of the protein A- and HA-tagged fusions are as indicated.
**B***. In vitro* pull-down assay. Proteins were expressed as His-tagged fusions in *E*. *coli* and their interacting partners as HA-fusions in COS-1 cells. After immobilization of the His-tagged fusion proteins to Ni-NTA beads, the interaction partners were pulled-down from COS-1 cell extracts. Binding was detected by SDS-PAGE and immunoblotting using anti-HA antibody. Identities of the His- and HA-tagged fusions are as indicated.

### Figure 4. A confidence scoring system for Y2H PPls

**A.** Network motifs. Y2H PPI data were analyzed for three and four protein interaction loops in combination with human reference data and PPI data from model organisms. Light blue: Proteins from the Y2H map. Dark blue: Proteins from the different reference data sets. Blue lines: Y2H PPIs. Grey lines: Reference data interactions. Two digits specify Y2H interaction loop motifs. The first digit refers to the number of links in the Y2H map, while the second digit indicates the number of links in the reference data. The sum of the digits indicates the size of the interaction loop (**Table S2**).
**B.** Statistical significance of loop motif analysis. Z-score values [(#*PP*/*S*ᵣₑₐₗ - #*PP*/*S*_{rand})/SD_{rand}] were calculated from the number of loop motif interactions from the experimental Y2H network (Nᵣₑₐₗ, LacZ4 set) and the random model networks, N_{rand_ma} and N_{rand_sf}. Values for human (Hs, set **4**), *Drosophila* (Dm, set 5), *C. elegans* (Ce, set 6) and *S*. *cerevisiae* (Sc, set 7) are presented. Statistical analysis revealed that interactions from Nᵣₑₐₗ appear with higher frequency in three and four protein interaction loops than interactions from the randomized networks (**Table S4**).
**C.** Distribution of the Y2H interactions according to their quality scores. Based on experimental and bioinformatic criteria, 6,123 quality points were allocated to 2,688 Y2H PPIs.
**D.** Results of the confidence analysis. The number of Y2H interactions in low (LC), medium (MC) and high (HC) confidence data sets are shown. The confidence difference between the LacZ4 and SD4 interactions is reflected by their distribution in the three quality sets.
**E.** Statistical significance of the quality point scoring system. Z-score values [(*#PPls*ᵣₑₐₗ - #*PPls*_{rand})/SD_{rand}] were calculated from the number of interactions that scored quality points in the bioinformatic loop analyses from Nᵣₑₐₗ (LacZ4 set), N_{rand_sf} and N_{rand_ma}. Interactions without quality score (0) appear with very low frequency in Nᵣₑₐₗ. In contrast, interactions with 2 and more quality points are found significantly more often in Nᵣₑₐₗ than in the randomized networks (**Table S5**).

### Figure 5. Local network views. For selected disease proteins such as p53 (A) or emerin (B) novel interacting proteins were identified by the automated Y2H screening. Based on the available PPI information, potential functional links to cellular pathways are discussed in the text.

### Figure 6. 5' nucleotide sequence data of so far unknown cDNAs in the fasta format. These sequences refer to cDNAs of cDNA-clones shown in table S1. In the header the identifier (ID) refers to the internal clone identifier of table S1 and the symbol is the official gene/protein symbol found in the NCBI database.

The examples illustrate the invention:

### Example 1: Particular methods and materials used in the Examples

### Subcloning of human cDNAs into Y2H plasmids

9,665 cDNAs of the hEx1 library (1) were sequenced from the 5' end to determine the identity and the reading frame of each cDNA fragment. BLASTP analysis against the nr (NCBI) or TrEMBL (Swiss-Prot) databases revealed a non-redundant set of 4,275 cDNAs, which were inserted by restriction cloning into the Y2H plasmids pGAD426 and pBTM117c, respectively. For recombinational cloning of human full-length ORFs, 2,136 human cDNA fragments were PCR amplified with specific primer pairs and BP-cloned into pDONR201 (Invitrogen, Carlsbad, US). Recombinant clones were sequenced and annotated using BLASTP searches. cDNA fragments were then shuttled into the Y2H vectors pBTM116-D9 and pGAD426-D3 by recombinational cloning (http://www.rzpd.de/products/orfclones/).

### Automated Y2H screening

Plasmids encoding bait and prey proteins were transformed into L40ccU *MAT***a** and L40ccα *MAT***α** yeast strains (2). For interaction mating 5 µl liquid cultures of the *MAT***α** yeast strains were replicated in 384-well MTPs using a pipetting robot (Biomek FX), grown, and mixed with 40 µl pooled *MAT***a** strains (8 baits). The yeast mixtures were then transferred onto YPD agar plates using a spotting robot (KBiosystems) and incubated for 36 h at 30°C. After mating, the clones were automatically picked from the plates and transferred into 384-well MTPs containing SDII (-Leu-Trp) liquid medium. For selection of PPIs, diploid yeasts were spotted onto SDIV (-Leu-Trp-Ura-His) agar plates as well as nylon membranes placed on SDIV agar plates. After 7 days of incubation at 30 °C, digitized images of the agar plates and nylon membranes were assessed for growth and β-galactosidase activity using the software Visual Grid (GPC Biotech). For confirmation of interactions, the 8 baits from each pool were arrayed in 96-well MTPs (Biomek 2000) and mated with the positive preys identified in the 1^{st} mating screen. After 36 h at 30°C, yeast cultures were spotted onto SDII agar plates for selection of diploid cells expressing both protein fusions. After 4 days at 30 °C, the yeast colonies were assayed on SDIV agar plates as well as nylon membranes (2). Details of the robotics systems used are available at http://www.mdc-berlin.de/neuroprot/labequip.htm.

### Membrane coimmunopurification and pull-down assays

For expression of HA- and protein A-tagged fusions, cDNA fragments were subcloned into pTL-HA or pcDNA3.1-protA, respectively, and cotransfected pair wise into COS-1 cells. Cell extracts were assessed for the expression of both proteins by SDS-PAGE and immunoblotting and filtered (approx. 3 µg protein extract) through a nitrocellulose membrane (Schleicher & Schuell) coated with human IgG (Sigma, 1:1000 in PBS) using a 96-well dot blot apparatus. Membranes were washed six times and probed with the anti-HA monoclonal antibody 12CA5 (Roche Diagnostics) for detection of PPls.
For *in vitro* pull-down assays, cDNA fragments were subcloned into pQE30-NST (*1*) or pTL-HA. Soluble protein extracts of His- and HA-tagged fusions prepared from *E*. *coli* and COS-1 cells, respectively, were assessed by SDS-PAGE and Western blotting. His-tagged fusions were bound to Ni²⁺NTA agarose beads (approx. 300 µg protein). After extensive washing (50 mM HEPES-KOH pH 7.4, 300 mM NaCl, 1 % NP-40, 5 mM imidazol, 1 mM DTT) they were incubated for 2 h at 4°C with 300 µg COS-1 cell extract containing the potential interacting HA-tagged fusion protein.

Following six wash cycles bound proteins were eluted from the beads and analyzed by SDS-PAGE and immunoblotting using the anti-HA antibody.

### Computational analysis of the PPI map

Using LL.out_hs (NCBI, 13-05-2004 release) proteins were mapped to a unique gene locus via their Accession Numbers obtained in the BLASTP search against the nr (NCBI) or TrEMBL (Swiss-Prot) databases. The BLAST analysis of Y2H clones and the orthology assignments computed with the InParanoid program (3) against the predicted proteome of *D. melanogaster* (FlyBase release r3.2.0), *C. elegans* (WormBase release WS121) and *S*. *cerevisiae* (SGD ORF set of 02-04-2004), are presented in **Table S1.** A PPI human reference set of 9,153 PPls between 3,810 proteins was obtained by combining the entries from *HRPD* (8,153 PPIS, *(4)), MINT* (1,230 PPls, (5)) and *BIND* **(414** PPIS, (*6*)) databases (status 13-05-04). For the assignment of interolog clusters we referred to the complete *D. melanogaster* Y2H data set (*7*) (20,439 PPIs; 6,991 proteins), the *C*. *elegans* WI5 data set (8) (5,534 PPls; 3,227 proteins) and the manually curated catalogue of PPIs from *S. cerevisiae* from MIPS (9) (8,946 PPls; 4,525 proteins). In the random model analyses, average and standard deviation values were calculated for nine N_{rand_ma} and N_{rand_sf} networks (**Table S4** and **S5**), respectively, using different randomized sets of proteins originating from the Y2H matrix. Topological analysis, e.g. degree distribution, clustering coefficients and path lengths, was carried out with TopNet (*10*). GO assignments were performed according to Gene Ontology annotation (24-05-2004) (*11*). Sequence and PPI data are available at www.mdc-berlin.de/neuroprot/database.htm.

### Example 2:

### Construction of a human Y2H PPI map

For identification of interactions by a Y2H matrix approach (10), 9,665 cDNA fragments from a human fetal brain expression library *(11)* were sequenced from the 5' end to determine the identity as well as the open reading frames (ORFs) of the encoded proteins **(Fig. 1A**). Sequence analysis revealed a non-redundant set of cDNAs that were introduced into activation domain (AD) and DNA binding domain (DBD) Y2H vectors by restriction cloning (**Fig. 1B).** Using this approach, 3,510 baits (DBD-X) and 3,589 preys (AD-Y) were generated. In addition to restriction cloning, a "GATEWAY recombinational cloning" approach was used to shuttle full-length human ORFs from entry vectors into DBD and AD plasmids, yielding an additional 2,033 baits and 2,051 preys. In total, 11,183 Y2H clones encoding 5,543 bait and 5,640 prey proteins were generated for interaction screening. 48% of the plasmids contained full-length ORFs, whereas 52% coded for C-terminal fragments of large human proteins **(Fig. 1C, left).** 12% of the cDNAs encoded disease proteins, and 17% proteins of unknown function (**Fig 1C, right**). The length distribution of the proteins is shown in **Fig**. **1D.** Three quarters of the proteins were of a predicted length between 100 and 500 amino acids, a preferred size for Y2H analysis (7).

To generate a matrix for interaction mating (*10*), a MATα yeast strain was individually transformed with AD-Y plasmids and the resulting yeast clones were arrayed in 384-well microtiter plates. Simultaneously, the DBD-X plasmids were introduced into a MATa strain and assembled in 96-well plates. Baits (19.6%), which activated the *HIS3, URA3 and lacZ* reporter genes after mating with a MATα strain expressing an AD protein, were excluded from the automated Y2H analysis. For interaction screening, 8 clones expressing non-activating baits were pooled and mixed with the 5,632 prey clones. The bait and prey mixtures were then spotted onto YPD agar plates for interaction mating. For Y2H selection, the diploid yeast clones were transferred from the YPD to selective plates lacking uracil and histidine (**Fig. 1E**). In addition, for β-galactosidase assays the clones were spotted onto membrane filters and placed on selective plates. Positive clones, which grew on selective plates and/or turned blue upon lacZ reporter gene activation, were detected with image analysis tools directly linked to a PPI database. All positive preys identified in the first pooled mating screens were individually retested for interactions with each of the 8 baits in a second mating assay (**Fig. 1F**). Thus, an unambiguously positive interaction was only assigned to a pair of proteins after two independent mating assays. More than 25 million protein pairs (4,456 baits x 5,632 preys) were examined systematically and 3,269 interactions among 1,064 baits and 1,075 preys were identified **(Fig. 1A** and **Table S1 and S2).** The Y2H interactions were grouped into two data sets: those that activated all three reporter genes, *HIS3, URA3* and *lacZ* (LacZ4 set) and those that activated only the two growth reporters *HIS3* and *URA3* (SD4 set). The LacZ4 and SD4 sets comprise 2,123 and 1,146 interactions, respectively.

### Example 3:

### Properties of the Y2H network and experimental verification of interactions

For analysis of the protein interaction data, each cDNA was mapped to an NCBl gene locus (**Table S1**). After collapsing 83 interactions that occurred in bait/prey and prey/bait configurations or that mapped pair wise to the same gene loci, a total of 3,186 unique interactions between 1,705 different human proteins was obtained. Computational analysis of the data revealed one giant network of 3,131 interactions between 1,613 proteins and 43 small isolated networks with less than 6 proteins (**Fig. 2A**). On average, proteins in the network had 1.87 interaction partners. 804 proteins were linked to a single interactor, while several proteins had more than 50 interactions, acting as hubs that are characteristic of protein interaction networks (12). The interaction map resembles a scale-free network (13) in that the degree distribution of the proteins decreases slowly, following a power-law (**Fig. 2B**). Moreover, the clustering coefficient diminishes with the number of interactions per protein (**Fig. 2C**), indicating a hierarchical modularity of the network (*14*). The minimal distances between any two proteins in the network are shown in **Fig. 2D.** A mean shortest path length of 4.85 was calculated, indicating that the network possesses small world properties (*15*). These characteristics appear to be generic features of protein interaction networks and are descriptive for the efficiency and robustness of biological systems *(13, 15).*
To evaluate the quality of the Y2H data sets, a representative sample of Y2H interactions was randomly selected for verification assays, because interactions recapitulated independently are unlikely to be experimental false positives (2, 7, 3). Using a membrane coimmunoprecipitation assay **(Fig. 3A),** 23 protein pairs of the SD4 set and 95 pairs of the LacZ4 set were tested with success rates of 11/23 (48%) and 63/95 (66%), respectively. In addition, 41 interactions of the SD4 and 91 of the LacZ4 sets were analyzed using a pull-down assay **(Fig. 3B).** With this assay, interactions were verified with success rates of 25/41 (61%) and 63/91 (69%). These results demonstrate that the obtained Y2H data contain a large fraction of reliable interactions.

### Example 4:

### Scoring of high confidence Y2H PPls

To enable an accurate and meaningful biological evaluation, the calculation of confidence scores is critical in Y2H interaction mapping (8, 7). A scoring system was developed to obtain high confidence data, by allocating quality points to Y2H interactions. Experimental and topological criteria were applied (see below), which are known indicators of confidence. According to these criteria, the PPIs from the obtained network were assigned to 7 data sets of higher confidence **(Table 1**), with PPIs scoring one quality point for each assignment. Interactions were allocated to different numbers of sets, receiving fewer or more quality points. The resulting scores were used to group the interactions into the three categories of high, medium and low confidence.
Y2H interactions identified by activation of three reporter genes (LacZ4 set) are of higher confidence than interactions detected only with two reporters (SD4 set). Therefore, a quality point each was awarded to the 2,123 LacZ4 PPIs (**Table 1**, set 1), but not to the SD4 interactions. Previous studies revealed that Y2H interactions detected several times are of higher quality than interactions found only once (7). Consequently, 150 quality points were assigned to interactions appearing repeatedly, either with different clones or as bait/prey and prey/bait combinations (set 2).

As Y2H interactions, that can be validated independently, are regarded of higher quality than unconfirmed interactions (2, 7, 3), we also allocated quality points to the 161 interactions verified by *in vitro* binding experiments and coimmunoprecipitations **(Fig. 3**). To identify interactions that were found in other laboratories, the Y2H PPI map was compared to a human PPI reference data set (*11*) extracted from the databases *HPRD, MINT,* and *BIND* containing 9,153 interactions between 3,810 proteins. The Y2H network and the human reference set have 671 proteins in common, that are involved in 481 interactions. 45 interactions were detected in the reference data corresponding to a coverage rate of 9.4%. In addition, *in silico* searches for evolutionarily conserved interactions or "interologs" (*16*), using the PPI data from *D. melanogaster* (Dm (*8*)), *C*. *elegans* (WI5 (7)) and *S. cerevisiae* (MIPS (*11*)), revealed 64 interaction pairs. In total, 222 Y2H interactions obtained a quality point, due to independent experimental verification in one or more cases (set 3).

Because cellular functions are carried out by stably or transiently associated groups of proteins, it was reasoned that interactions in functional modules (13, 15) are of higher confidence than others. Therefore, Y2H interactions that are present in three and four protein interaction loops - potential functional motifs (17, 18) - were identified in the combined Y2H and human PPI data from *HPRD, MINT* and *BIND* (**Fig. 4A**). The analysis revealed that 248 Y2H interactions participate in three protein loops and 1,784 are involved in four protein loops. 229 PPI_{S}, counted once, were present in both three and four protein loops. Together 1,803 interactions were grouped into confidence set 4 (**Table 1).** Clustering analysis was also performed with the Dm, WI5 and MIPS PPI data, yielding 989, 492 and 344 Y2H PPIs, respectively, involved in three and four protein interolog loops (sets 5-7).

To determine the statistical significance of three and four loop motifs in the Y2H network, comparable random model networks were analyzed. Starting from the same number of nodes and links as in the Y2H network (Nᵣₑₐₗ), two topologically distinct randomized networks (N_{rand_ma} and N_{rand_sf}) were computer-generated. N_{rand_ma} (ma: mathematical) is a mathematical random network and has a regular topology (13). N_{rand_sf} (sf: scale free) has randomized links, however, it maintains the degree distribution, *i.e.* the number of links per protein, of Nᵣₑₐₗ (*19*). N_{rand_sf} shows the minimal possible topological variation to Nᵣₑₐₗ and is the most stringent control for statistical examination.

In analogy to the analyses of the interactions from Nᵣₑₐₗ (sets 4-7), the appearance of the computer-generated interactions from N_{rand_ma} and N_{rand_sf} in three or four protein loop motifs were determined. The number of random interactions with the number of Y2H interactions found in the motifs were then compared. To arrive at a measure for the statistical significance of the obtained data, Z-score value calculations were employed (18). The Y2H interactions appeared at significantly higher numbers in loop motifs than the ones obtained from the randomized networks (**Fig. 4B**). This was most pronounced for the human and *Drosophila* data. Lower Z-score values were obtained for the C. *elegans* and yeast data, which might reflect a lower degree of homology to human protein interaction pairs. In conclusion, it can be said that the real Y2H interaction network in comparison to the computer-generated model networks contains larger numbers of interaction clusters, which can be seen as an indicator of functional relevance (*17*, *18).*

As proteins of similar cellular function and localization tend to form PPI clusters, the Y2H PPIs were also classified according to Gene Ontology (GO) categories (11). We found that both proteins of 290, 321 and 337 PPIs annotated to the same component, function and cellular process, respectively **(Table S2** and **S3).** However, statistical analysis revealed that the interactions assigned to these three GO categories did not appear with higher frequency in Nᵣₑₐₗ than in the random networks N_{rand_ma} and N_{rand_sf} (data not shown). Therefore, the results of the GO classification were not included in our quality scoring system, although GO annotated interaction pairs are generally regarded as interactions of higher confidence (8).

In total, 6,123 quality points were allocated to 2,688 (82%) of our 3,269 Y2H PPIs (**Fig. 4C**). 581 (18%) Y2H interactions did not receive quality points and were classified as of low confidence (LC set). 1,706 interactions (52%) obtained 1-2 quality points and were classified as of medium confidence (MC set). Finally, 982 high confidence interactions were identified with three or more quality points (HC set, 30%), involving 405 different human proteins.
The rating of high, medium and low confidence interactions with the quality scoring system was compared to the LacZ4/SD4 classification (**Fig. 4D**). Clearly, the distribution of the SD4 and LacZ4 interactions between the LC, MC and HC sets confirms that the LacZ4 set contains PPIs of higher confidence. Thus, the confidence difference between the LacZ4 and the SD4 set is reflected in the quality scoring system of the method of the present invention.

Finally, the statistical significance of the confidence rating was also verified. As shown in **Fig. 4E,** interactions with few quality points appeared with higher frequency in the random networks than in Nᵣₑₐₗ, while interactions with several quality points (>2) appeared with higher frequency in the Y2H network. This indicates that high confidence PPIs, i.e. PPIs assigned to several confidence sets, have a higher statistical significance than PPIs with fewer assignments, supporting the quality scoring system as an apt indicator of the biological relevance of evaluated protein interactions.

### Example 5:

### Networks containing human disease-related proteins

The Y2H interaction map contains numerous small local interaction networks that can be examined with respect to their functional relevance. A detailed look at a cluster of interactions incorporating previous knowledge about the participating proteins may yield valuable information. Networks containing human disease proteins were selected, because they are highly relevant for understanding pathological processes and the development of therapeutic strategies. In this study, 515 novel interactions for 218 known human disease proteins (Table S1 and **S2**) were identified. 136 of these interactions were part of the high confidence PPI data set.

### PPI network of p53

Several new interactions were identified for the tumor suppressor protein p53 (TP53, NCBI Locuslink 7157) that is inactivated in about half of all human cancers. p53 is activated by different types of cellular stress integrating numerous signals controlling cell fate (20). Previous studies revealed that p53 is a highly connected protein, participating in more than 100 interactions with proteins involved in stress response activation, regulation of gene expression, or the control of cell proliferation. 41 new p53 interactors were identified, most of which function in similar processes as the known interaction partners, supporting the integrative role of p53 for cell survival (20). For example, as outlined in **Fig. 5A**, a new interaction between p53 and the 27 kDa heat shock protein Hsp27 (HSPB1, 3315) was detected that associates with Hsp70 and Hsp90 and most likely plays a role in the regulation of p53 conformation and activity under stress conditions (21). Hsp27 has also been shown to modulate actin polymerization, suggesting that p53, via its association with Hsp27 (22), may influence this process in mammalian cells. Another p53 interactor identified was Cdc42, a Rho family GTPase, which regulates actin assembly, migration and cell polarization in response to extra cellular stimuli (23). In addition, Rho signaling pathways are linked with different oncogenic signaling cascades contributing to various aspects of tumourogenesis (24). Thus, the binding of p53 to Cdc42 may not only affect actin polymerisation and cell morphology, but also influence survival, growth and progression of tumors.
The Y2H screen also demonstrated an interaction between p53 and the breakpoint cluster region protein Bcr (BCR, 613), a multidomain protein modulating Rho-type GTPases (25) **(Fig. 5A**). However, Bcr is a well-known oncogene that is activated by chromosomal rearrangements. As part of the chimeric fusion Bcr-Abl, it is found in 95% of all patients with chronic myeloid leukaemia (26). Recent studies have shown that Bcr forms a trimeric complex with Ras and the Ras-interacting protein AF-6 (25). Similarly to Bcr, AF-6 is a fusion partner of ALL-1 causally linked to a subset of acute lymphoblastic leukaemia (27). The p53-Bcr interaction suggests that p53 may also modulate Ras-mediated cell signaling, a process altered in many proliferating cancer cells (28).
Another interesting new p53 interaction partner is ubiquitin E3 ligase Arih2 (ARIH2, 10425) (29). This protein is upregulated during differentiation of acute leukemia cells (30) and interacts with UbcH7, an ubiquitin-conjugating enzyme (UBE2L3, 7332) (31). UbcH7 together with E6-AP, an ubiquitin protein E3 ligase (32), mediates human papillomavirus E6-induced degradation of p53 in cervical cancer (33). E6-AP has also been found mutated in Angelman syndrome, a neurological disorder. As previous studies have demonstrated that p53 activity is highly regulated by E3 ubiquitin ligases (20), it is suggeed that Arih2 binding may also influence the steady state levels of p53. Moreover, it could act as an oncoprotein that promotes p53 ubiquitination and degradation.

### PPI network of emerin

Furthermore, several new interaction partners for emerin were identified (EMD, 2010) (**Fig. 5B**), a nuclear envelope protein which, when mutated, causes X-linked Emery-Dreifuss muscular dystrophy (EDMD), characterized by progressive skeletal muscle weakness and cardiac conduction system deficiency (34). An autosomal dominant form of EDMD is caused by mutations in the *LMNA* gene coding for alternatively spliced lamins A/C, the main components of the nuclear lamina. Emerin interacts directly with lamins A/C and also associates with BAF, a chromatin binding protein critical for emerin reassembly into the nuclear envelope (35). Although 60% of the EDMD patients do not have mutations in emerin or lamin A/C, there is growing evidence that disruption/formation of tissue specific protein complexes involving emerin underlies EDMD development (36). With the present invention's method, it could be determined that emerin binds to the Src-homology 3 proteins SH3GL2 (6456) and SH3GL3 (6457), which are implicated in membrane trafficking and endocytosis (37). Moreover, an interaction between emerin and the uncharacterized developmental pluripotency associated protein 4 (DPPA4, 5521) was detected, which also binds SH3GL3 and SH3GL1 (6455). This suggests that emerin together with actin (38), DPPA4, and the SH3 domain-containing proteins SH3GL1, SH3GL2 and/or SH3GL3 may form a functional cluster that could be critical for emerin function *in vivo.* Although emerin is expressed in most human tissues, the disease affects only skeletal muscle, tendons and heart (36). Thus, a detailed analysis of the new tissue specific interaction partners (39) may lead to a better understanding of the molecular mechanisms underlying EDMD.

### References

1. H. Ge, A. J. Walhout, M. Vidal, Trends Genet. 19, 551 (2003).
2. H. Goehler et al., Mol. Cell, 15, 853 (2004).
3. M. Tewari et al., Mol. Cell 13, 469 (2004).
4. J. C. Rain et al., Nature 409, 211 (2001).
5. T. Ito et al., Proc. Natl Acad. Sci. USA 98, 4569 (2001).
6. P. Uetz et al., Nature 403, 623 (2000).
7. S. Li et al., Science 303, 540 (2004).
8. L. Giot et al., Science 302, 1727 (2003).
9. S. Hanash, Nature 422, 226 (2003).
10. P. Legrain, L. Selig, FEBS Lett. 480, 32 (2000).
11. See supporting data online.
12. H. Jeong, S. P. Mason, A. L. Barabasi, Z. N. Oltvai, Nature 411, 41 (2001).
13. A. L. Barabasi, Z. N. Oltvai, Nat. Rev. Genet. 5, 101 (2004).
14. E. Ravasz, A. L. Somera, D. A. Mongru, Z. N. Oltvai, A. L. Barabasi, Science 297, 1551 (2002).
15. S. H. Strogatz, Nature 410, 268 (2001).
16. H. Yu et al., Genome Res. 14, 1107 (2004).
17. S. Wuchty, Z. N. Oltvai, A. L. Barabasi, Nat. Genet. 35, 176 (2003).
18. E. Yeger-Lotem et al., Proc. Natl Acad. Sci. USA 101, 5934 (2004).
19. R. Milo et al., Science 298, 824 (2002).
20. E. A. Slee, D. J. O'Connor, X. Lu, Oncogene 23, 2809 (2004).
21. M. Zylicz, F. W. King, A. Wawrzynow, Embo J. 20, 4634 (2001).
22. I. Kindas-Mugge et al., Cell Biol. Int. 26, 109 (2002).
23. M. Fukata, M. Nakagawa, K. Kaibuchi, Curr. Opin. Cell Biol. 15, 590 (2003).
24. J. Rao, N. Li, Curr. Cancer Drug Targets 4, 345 (2004).
25. G. Radziwill, R. A. Erdmann, U. Margelisch, K. Moelling, Mol. Cell Biol. 23, 4663 (2003).
26. O. Hantschel, G. Superti-Furga, Nat. Rev. Mol. Cell Biol. 5, 33 (2004).
27. R. Prasad et al., Cancer Res. 53, 5624 (1993).
28. M. McMahon, D. Woods, Biochim. Biophys. Acta 1471, M63 (2001).
29. M. Aguilera, M. Oliveros, M. Martinez-Padron, J. A. Barbas, A. Ferrus, Genetics 155, 1231 (2000).
30. B. A. van der Reijden, C. A. Erpelinck-Verschueren, B. Lowenberg, J. H. Jansen, Protein Sci 8, 1557 (1999).
31. G. Martinez-Noel, R. Niedenthal, T. Tamura, K. Harbers, FEBS Lett. 454, 257 (1999).
32. L. Huang et al., Science 286, 1321 (1999).
33. M. Scheffner, Pharmacol. Ther. 78, 129 (1998).
34. A. E. Emery, Lancet 359, 687 (2002).
35. L. Mounkes, S. Kozlov, B. Burke, C. L. Stewart, Curr. Opin. Genet. Dev. 13, 223 (2003).
36. L. Bengtsson, K. L. Wilson, Curr. Opin. Cell Biol. 16, 73 (2004).
37. W. B. Huttner, A. A. Schmidt, Trends Cell Biol. 12, 155 (2002).
38. G. Lattanzi et al., Biochem. Biophys. Res. Commun. 303, 764 (2003).
39. C. Giachino et al., Genomics 41, 427 (1997).
40. A.Q. Emili, G. Cagney, Nat. Biotechnol. 18, 393 (2000)
41. E.J. Grayhack & E.M. Phizicky, Curr. Opin. Chem. Biol. 5, 34 (2001)
42. H. Zhu & M. Snyder, Curr. Opin. Chem. Biol. 7, 55 (2003)
43. T. Feilner et al., Curr. Proteomics (in press) (2004)
44. H. Zhu et al., Science 293, 2101 (2001)
45. M. R. Martzen et al., Science 286, 1153 (1999)
46. H. Zhu et al., Nat. Genet. 26, 283 (2000)
47. A. Kramer et al., Phytochemitry 65, 1777 (2004)
48. A.C. Gavin et al., Nature 415, 141 (2002)
49. Y. Ho et al., Nature 415, 180 (2002)

Development of a scoring system using experimental and topological criteria to obtain high confidencial data, by allocating quality points to yeast two-hybrid interactions. According to this criteria PPIs (Protein-Protein interactions) from the network generated by the method of the present invention are assigned to 7 datasets of higher confidence.

**Table 1: PPI confidence data sets**

| **PPI set** | **Assignment** | **Number of quality points** |
|---|---|---|
| Set 1 | *HIS3, URA3* and *LacZ* Y2H reporter activity | 2,123 |
| Set 2 | Y2H multiple hits | 150 |
| Set 3 | Experimental verification | 222 |
| Set 4 | PPIs found in human interaction clusters | 1,803 |
| Set 5 | PPIs found in orthologous *D. melanogaster* clusters | 989 |
| Set 6 | PPIs found in orthologous *C. elegans* clusters | 492 |
| Set 7 | PPIs found in orthologous *S. cerevisiae* clusters | 344 |
| | Total assignments | 6,123 |

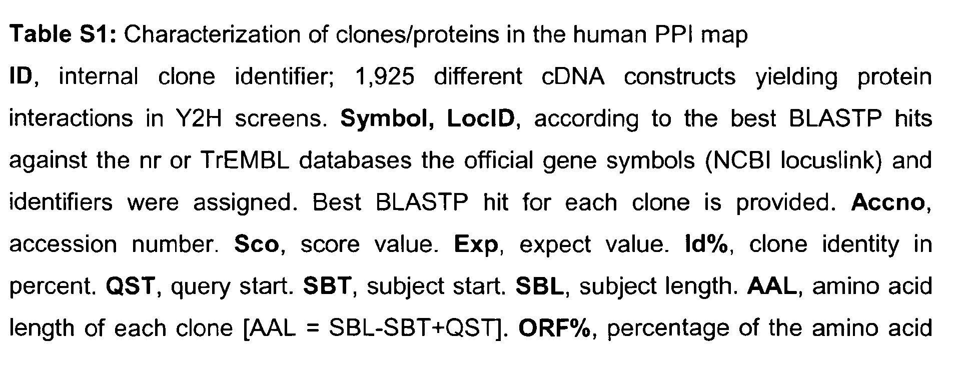

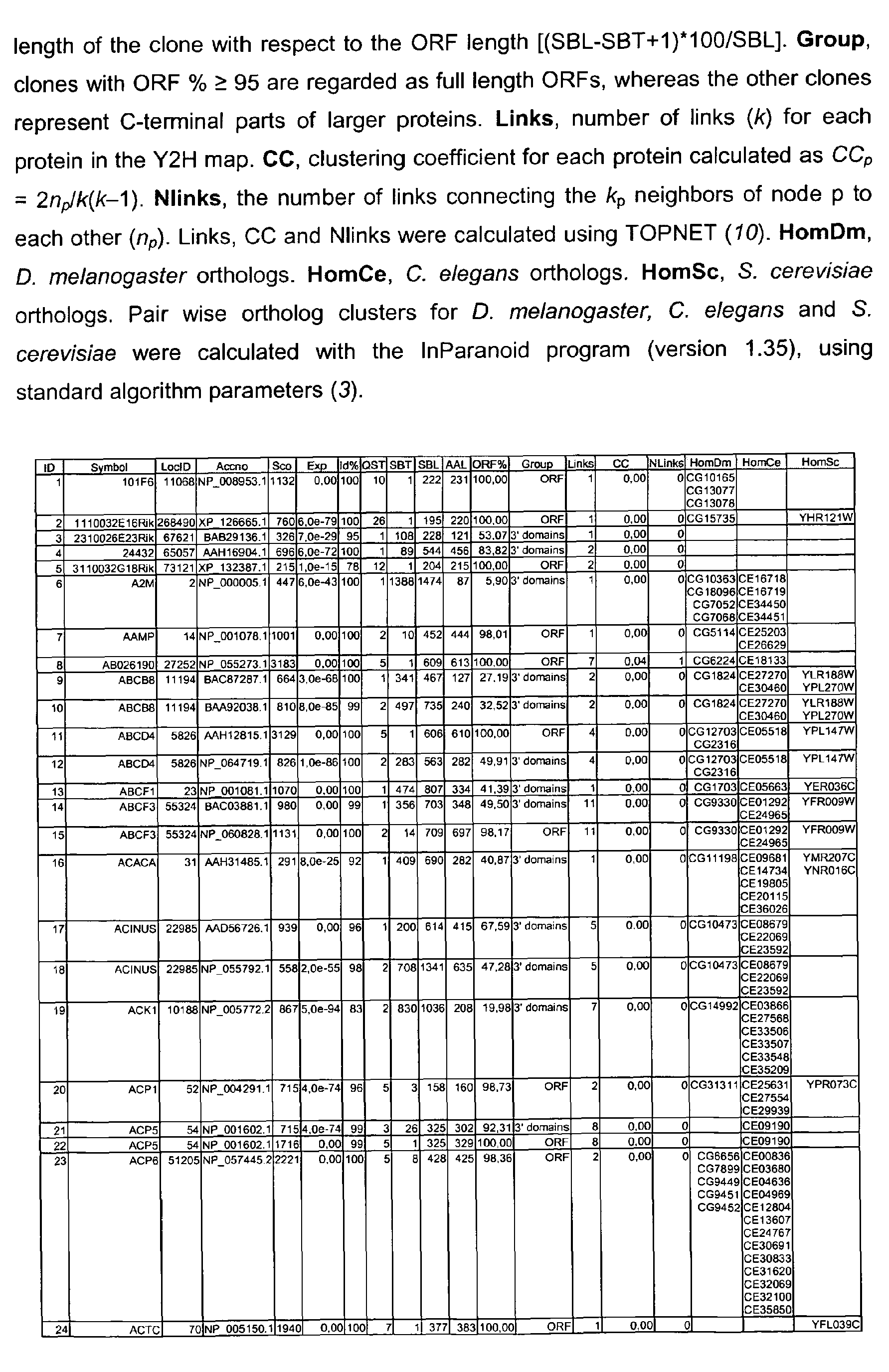

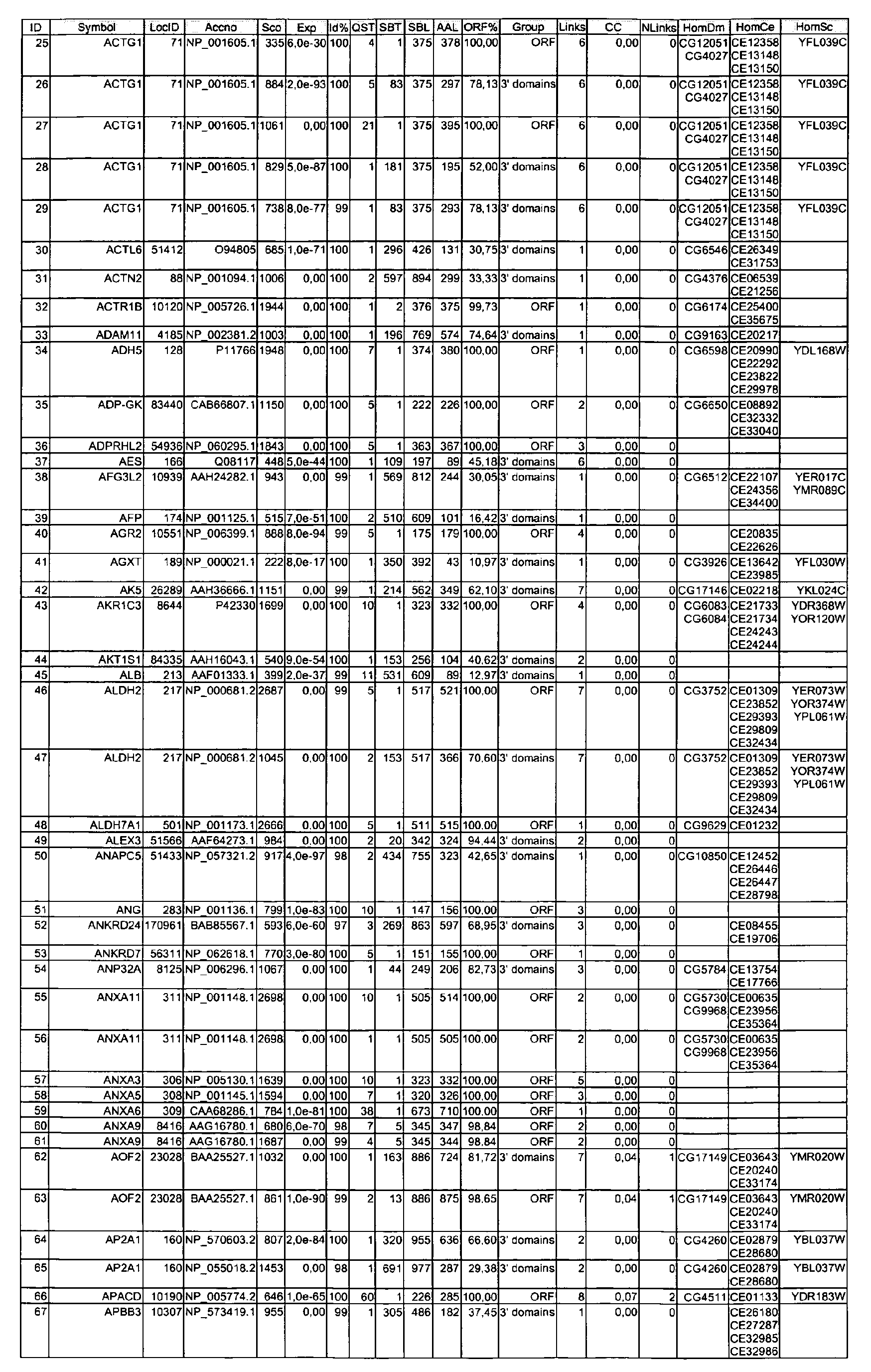

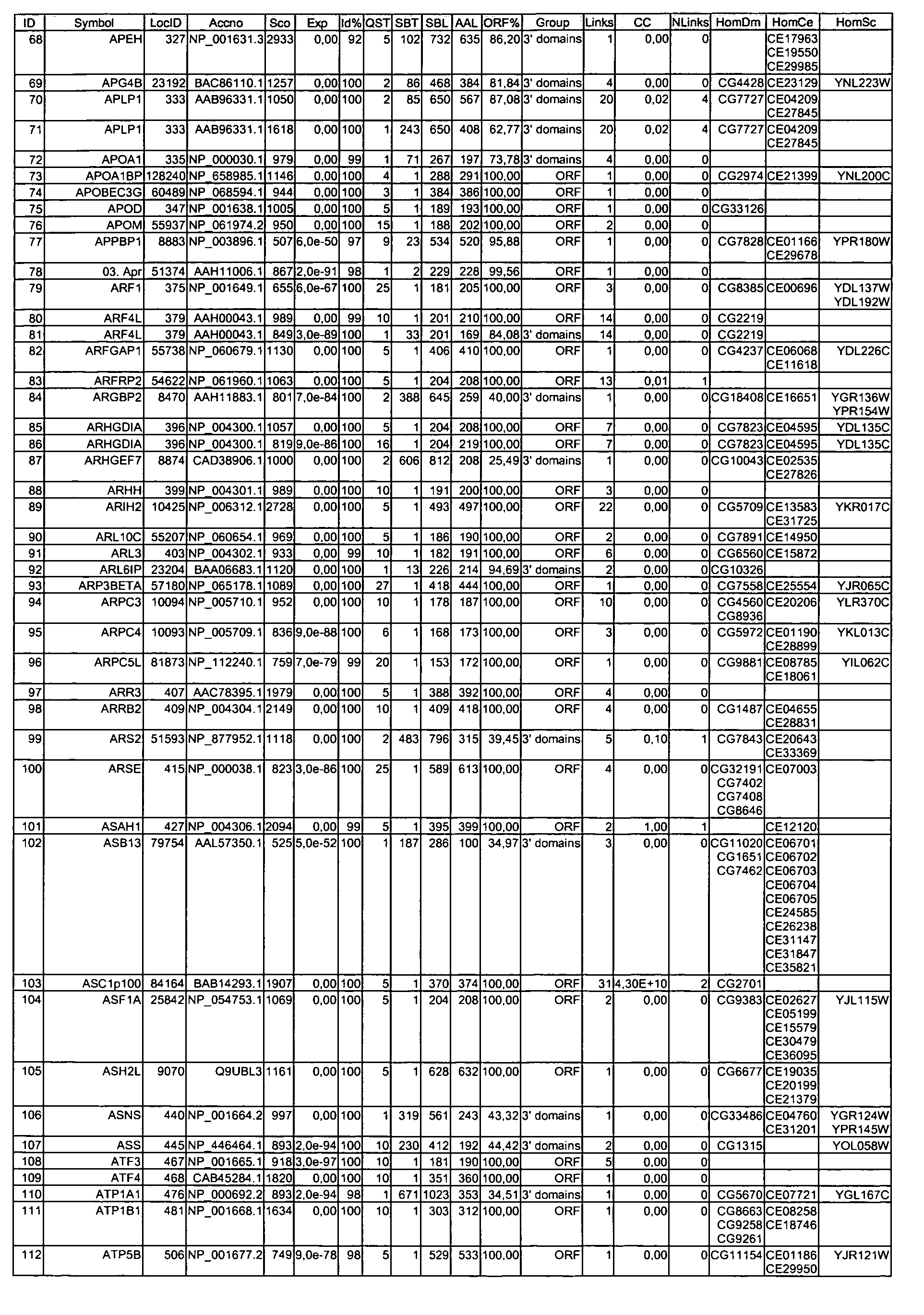

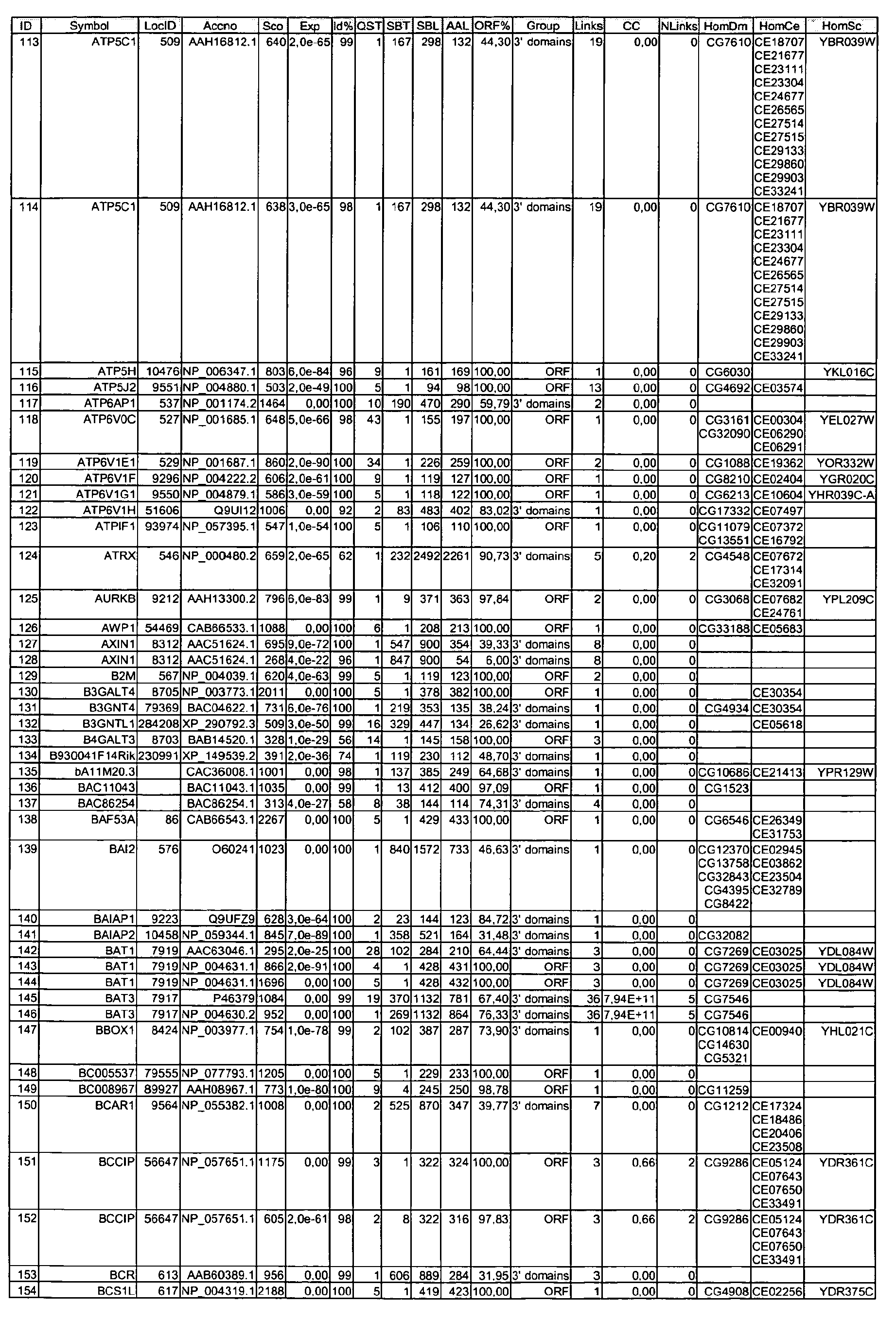

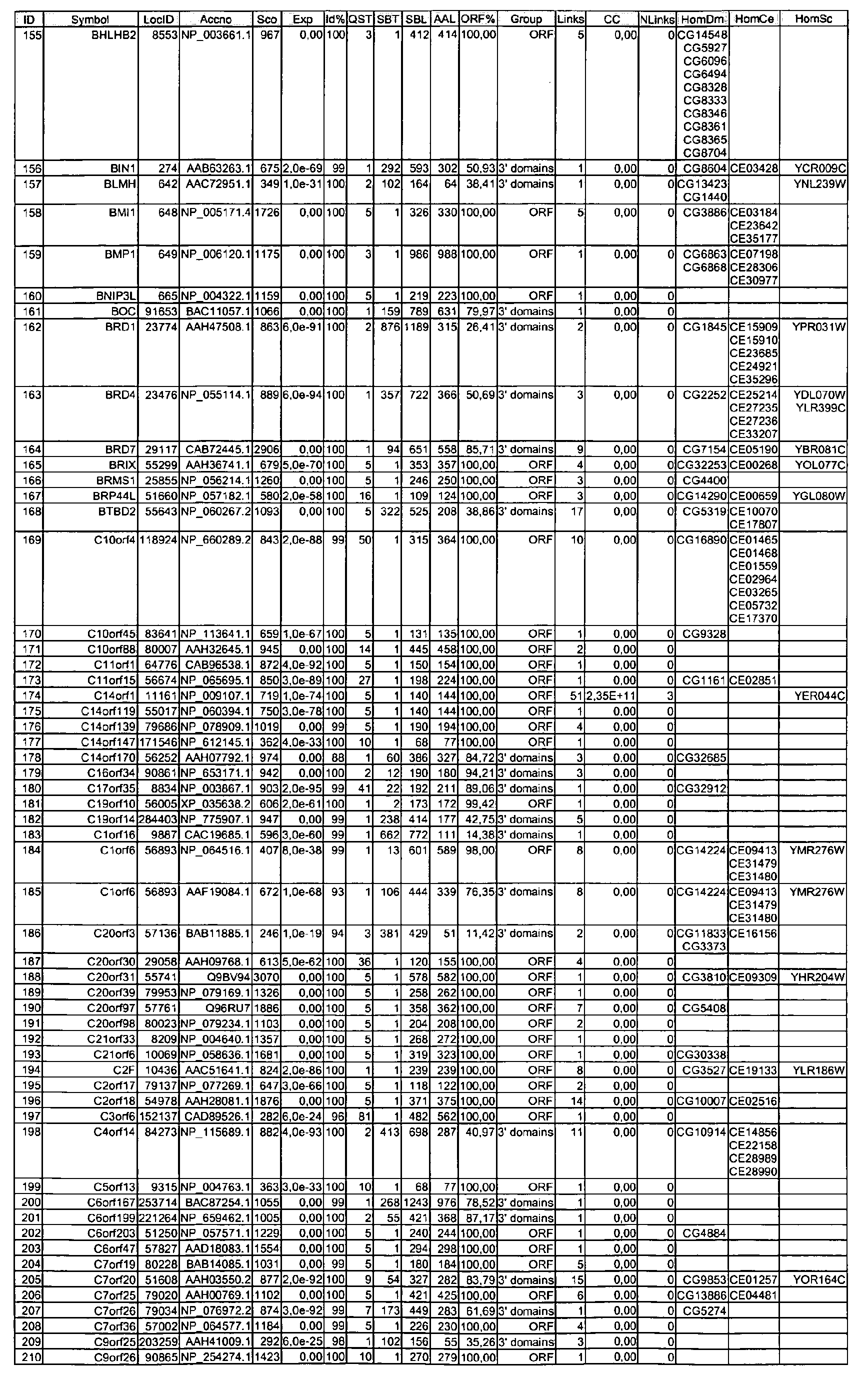

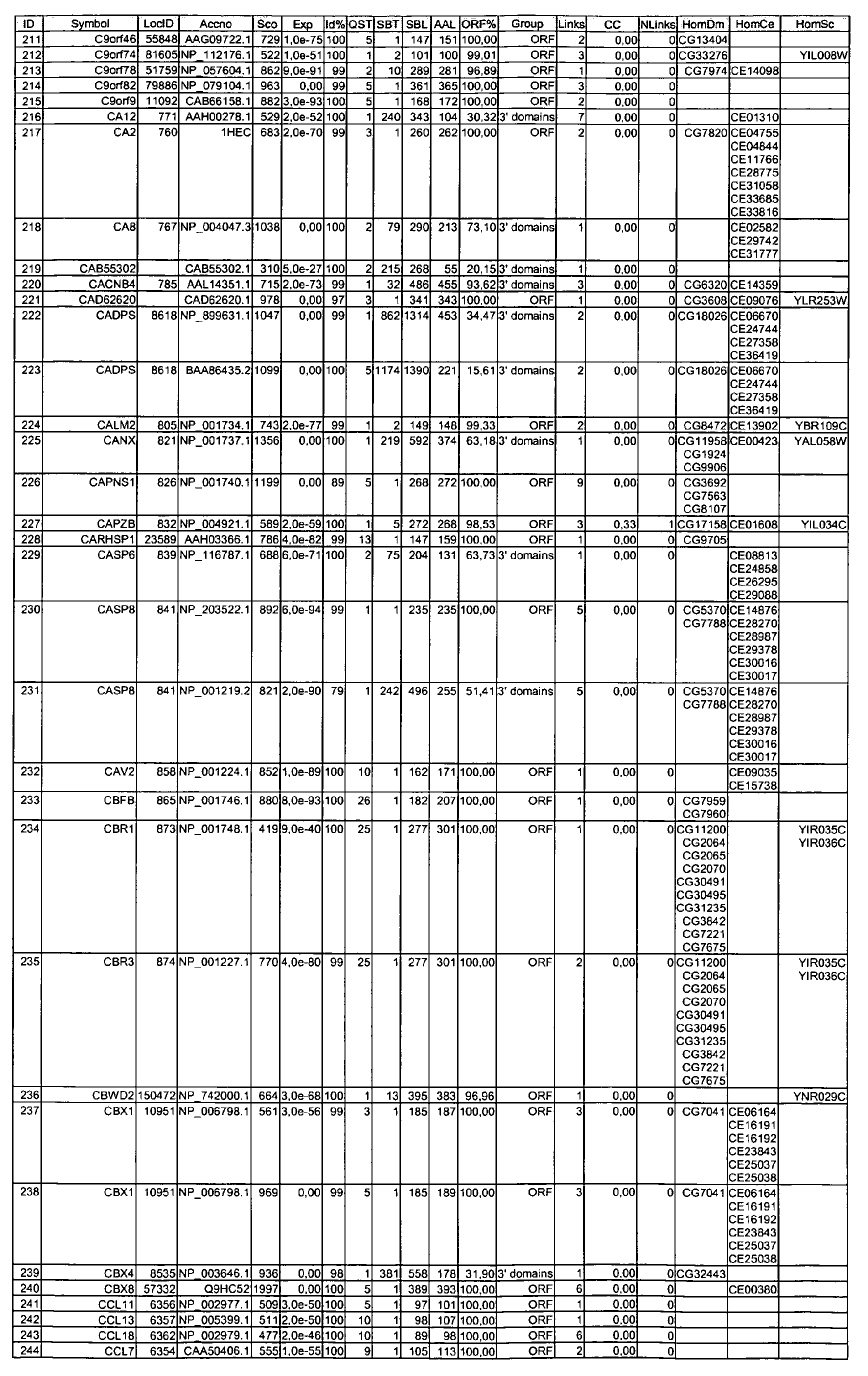

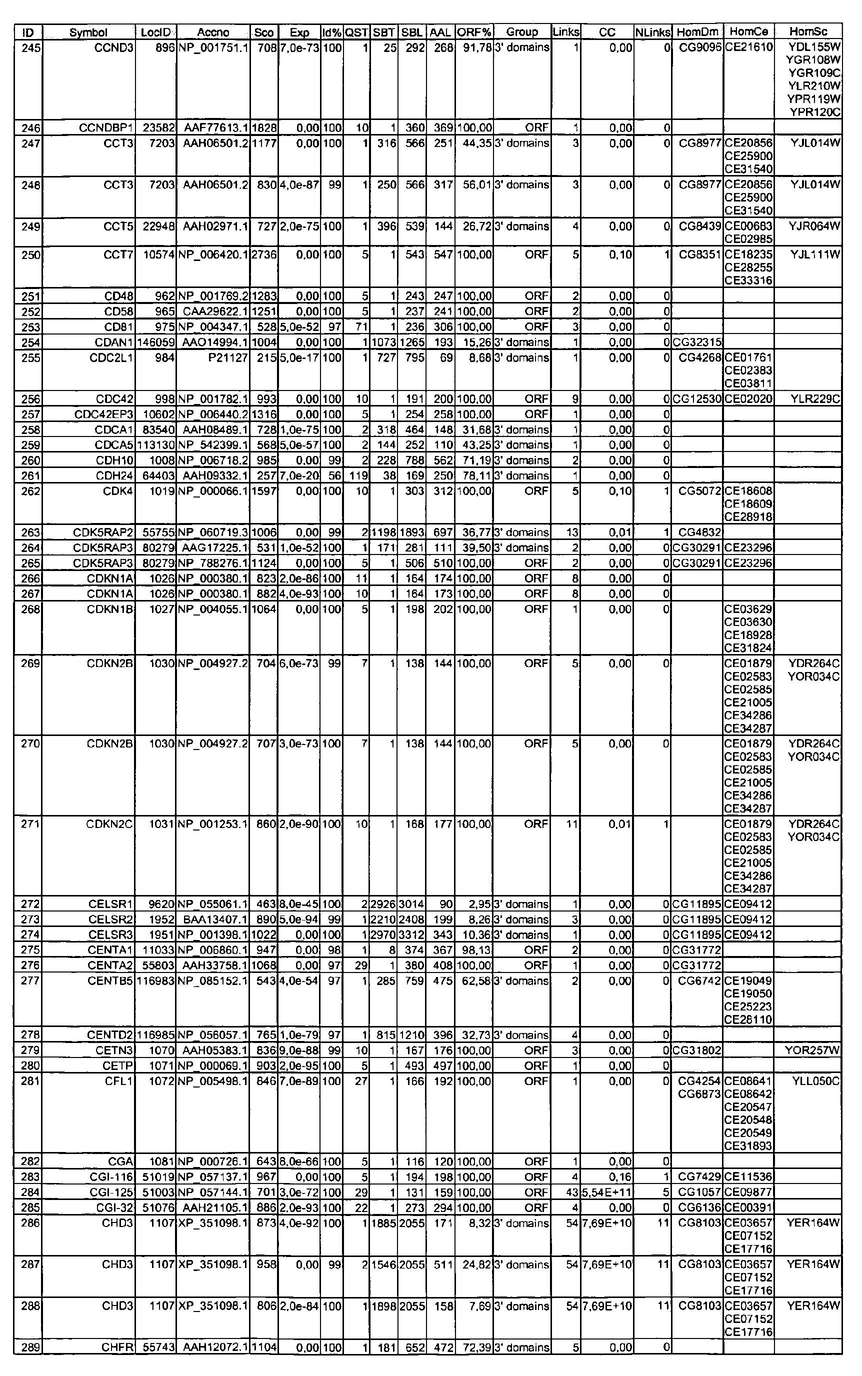

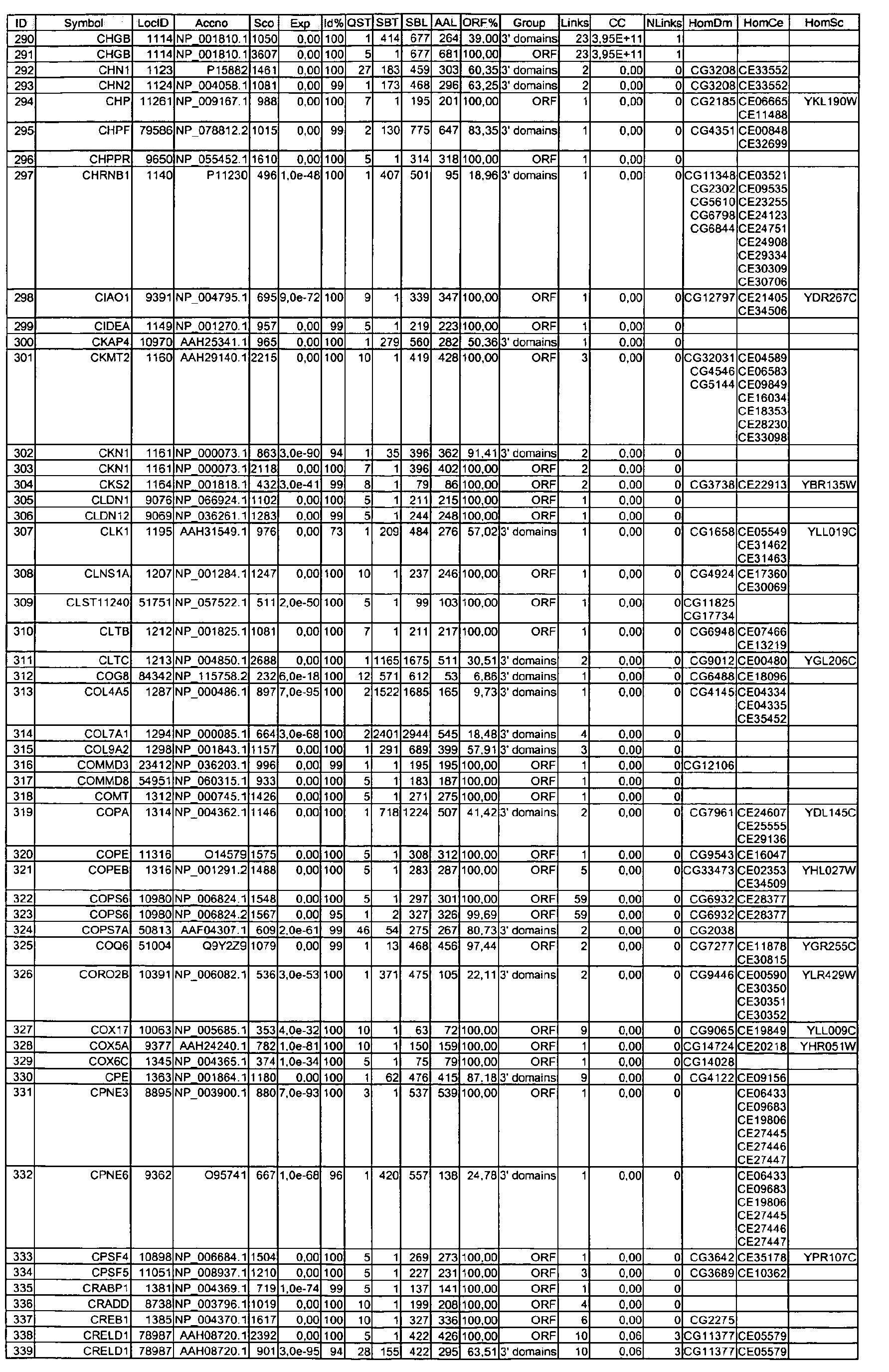

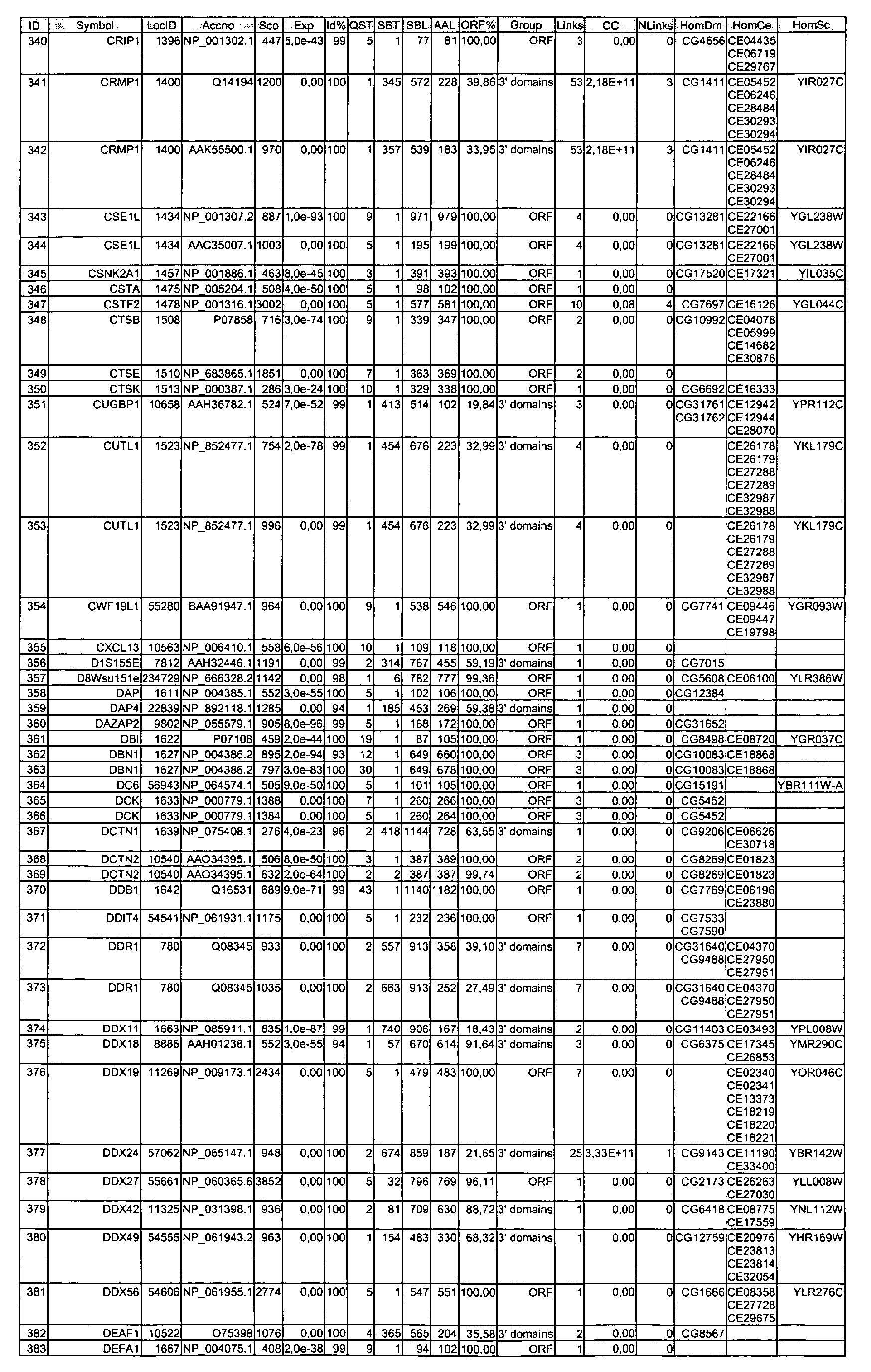

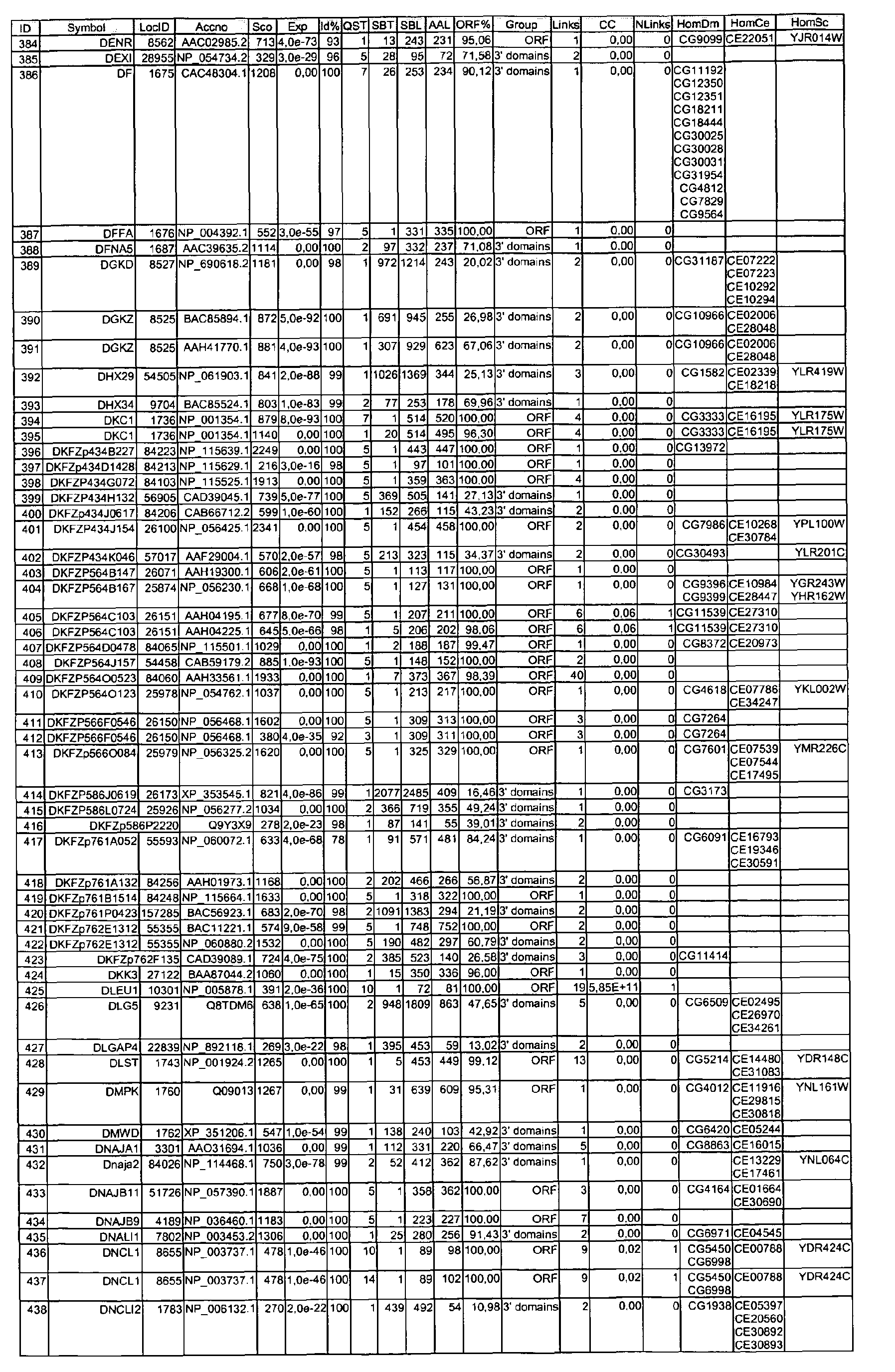

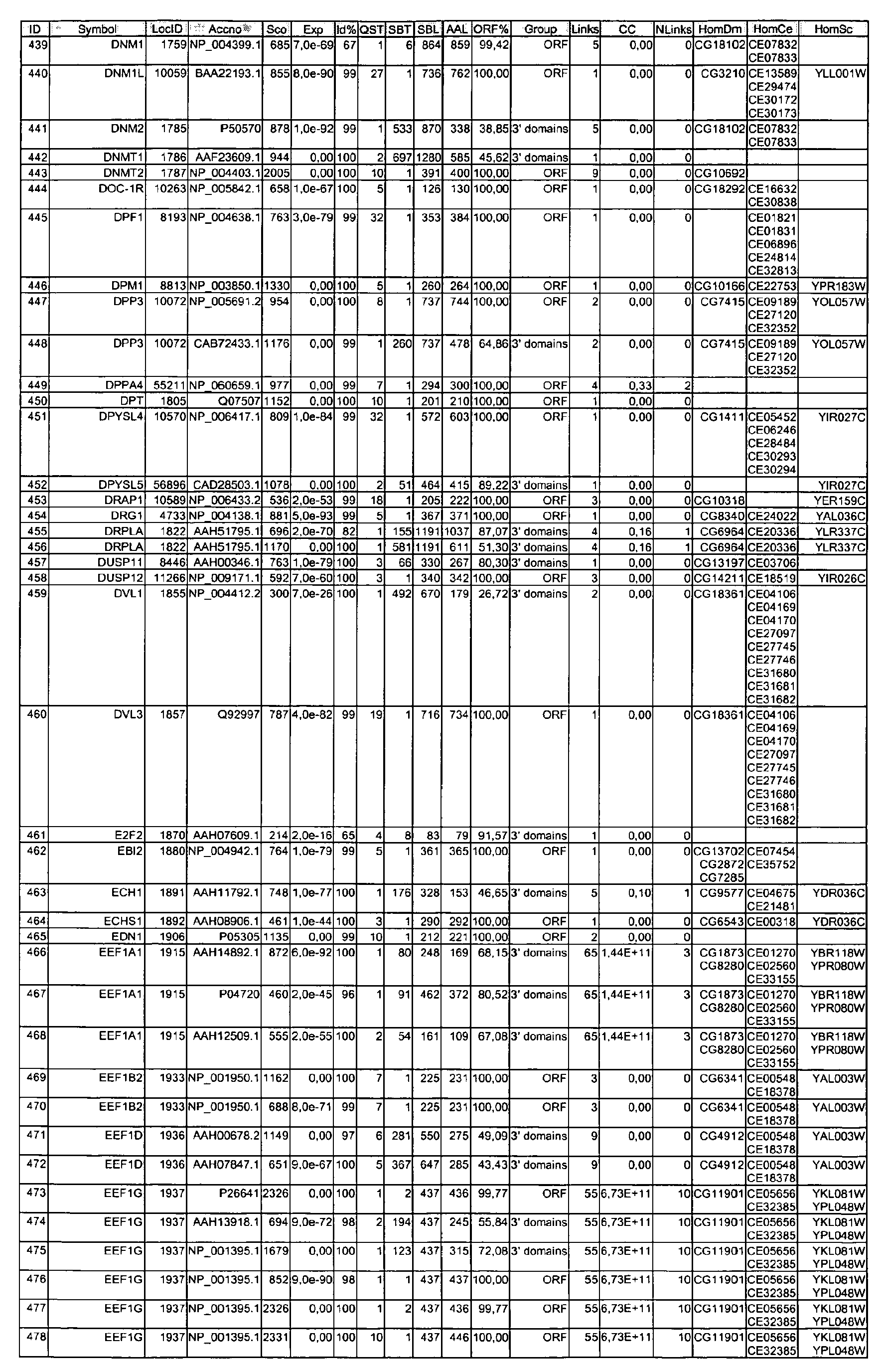

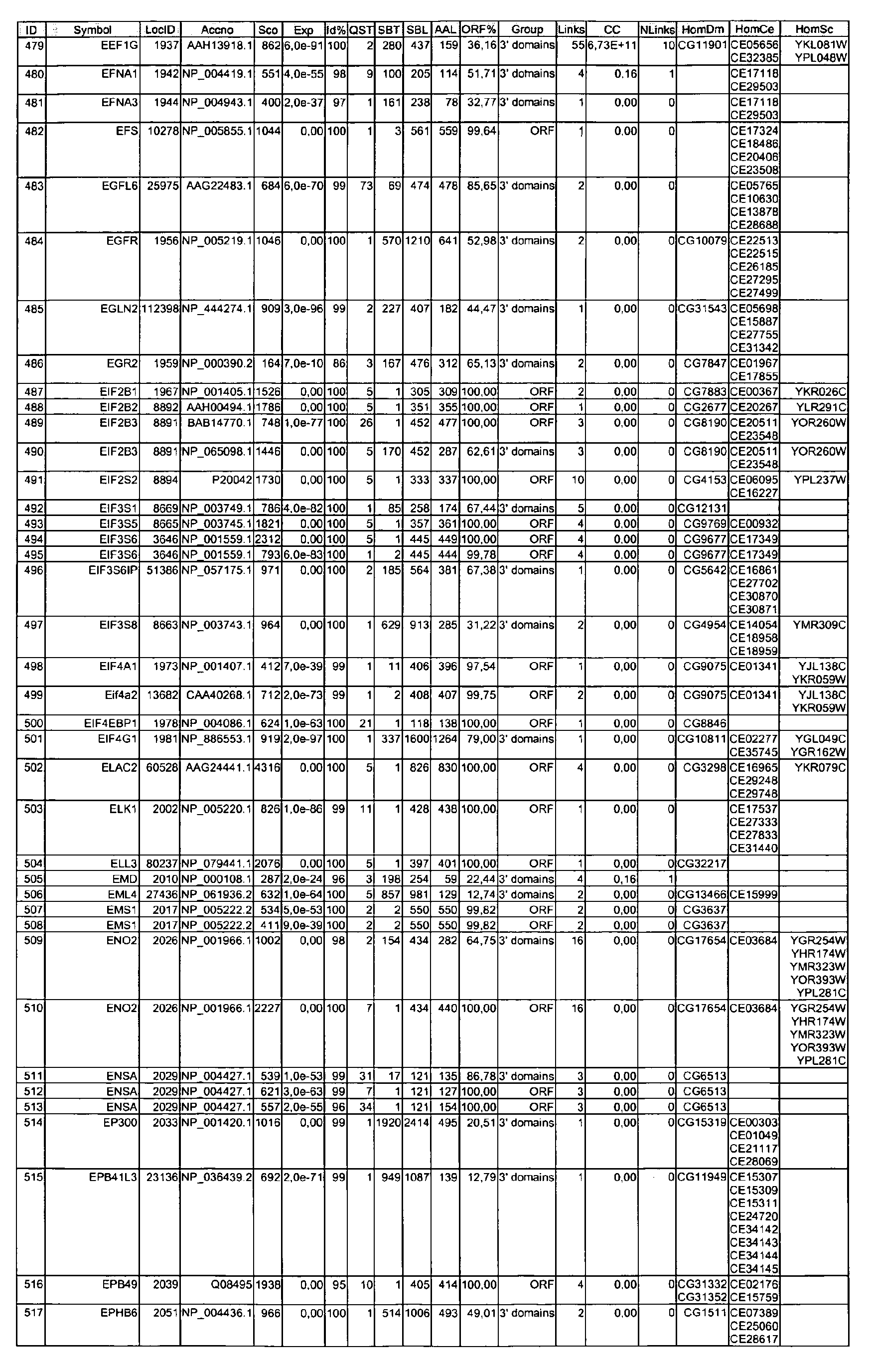

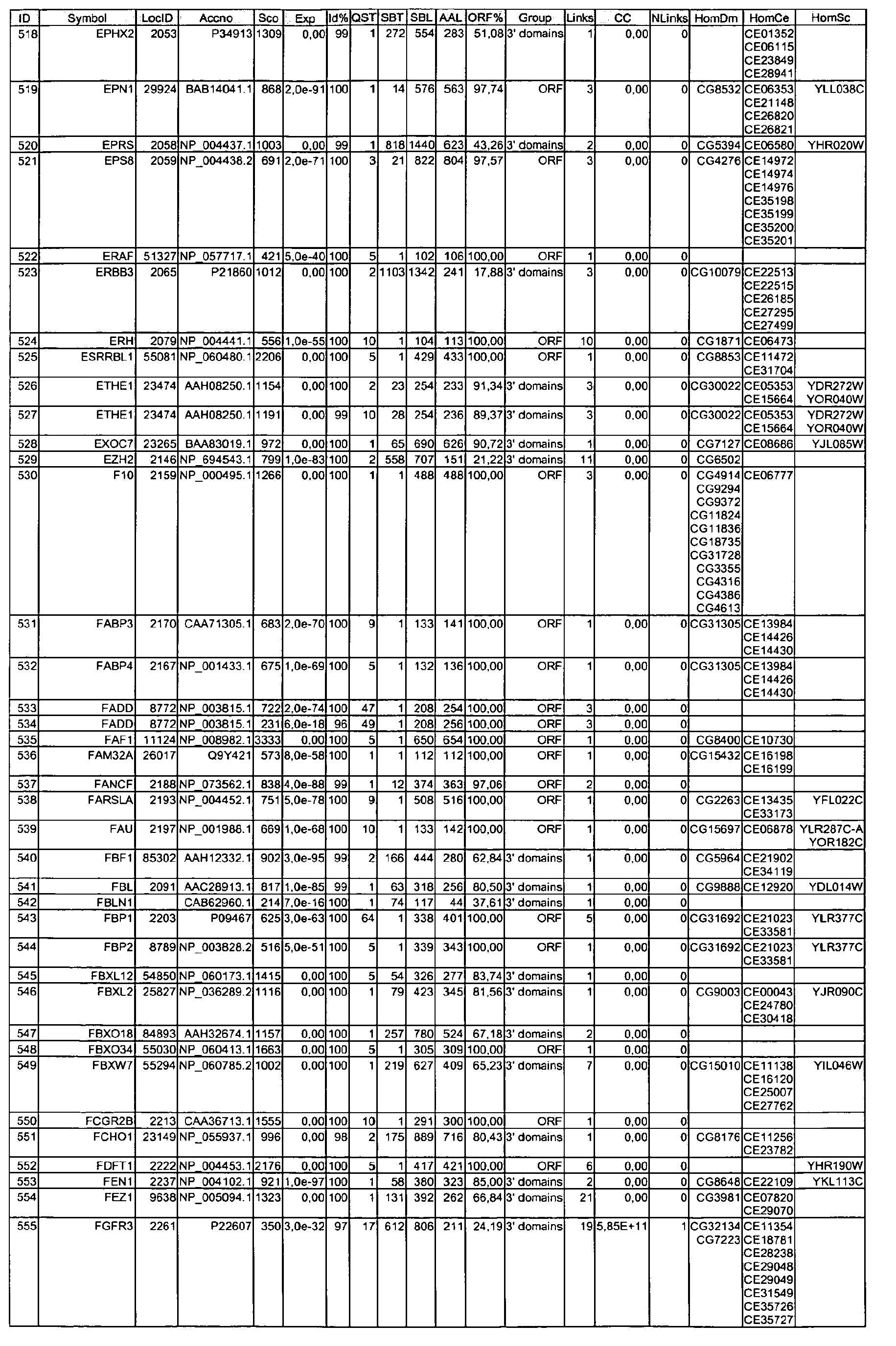

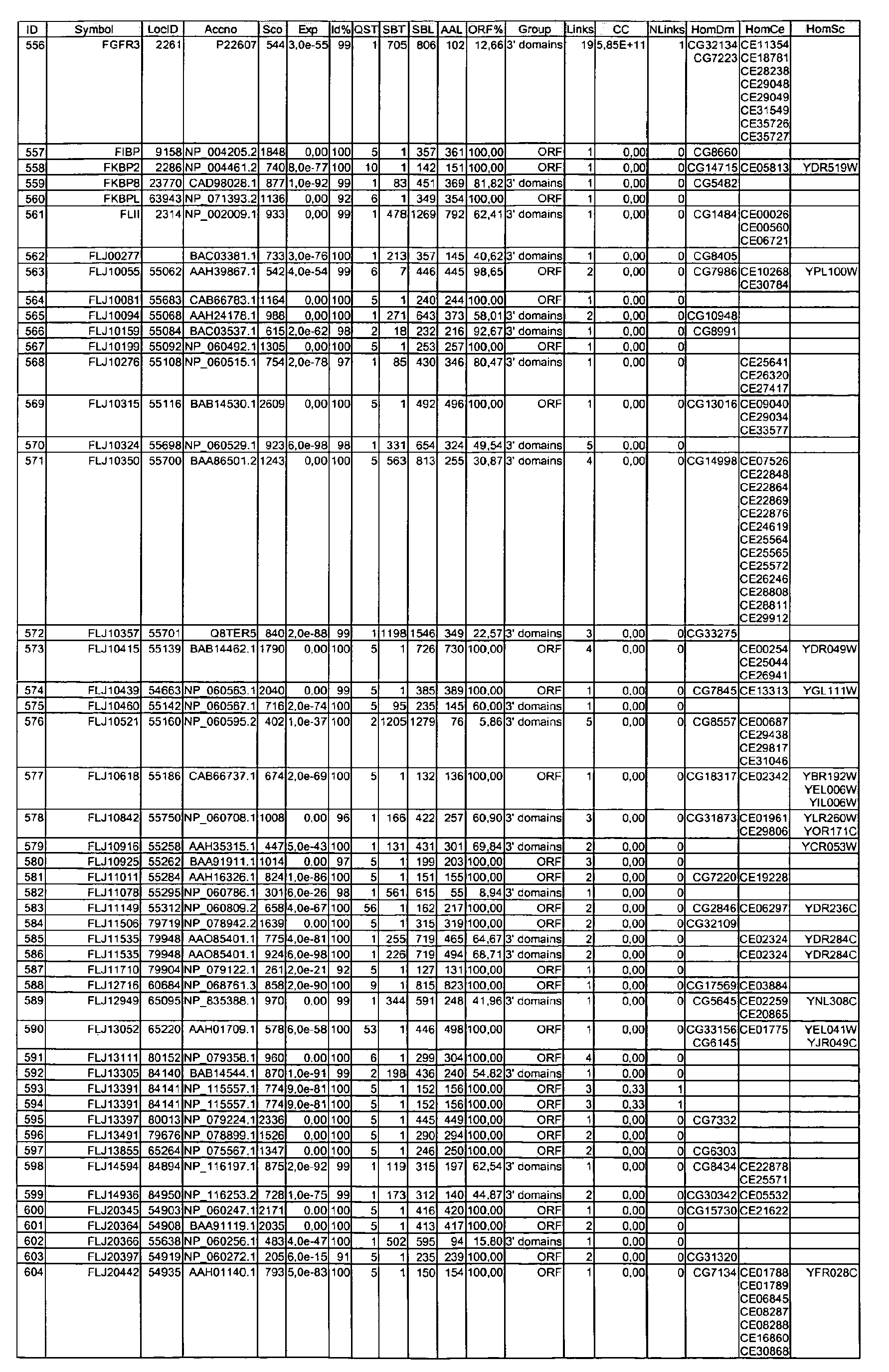

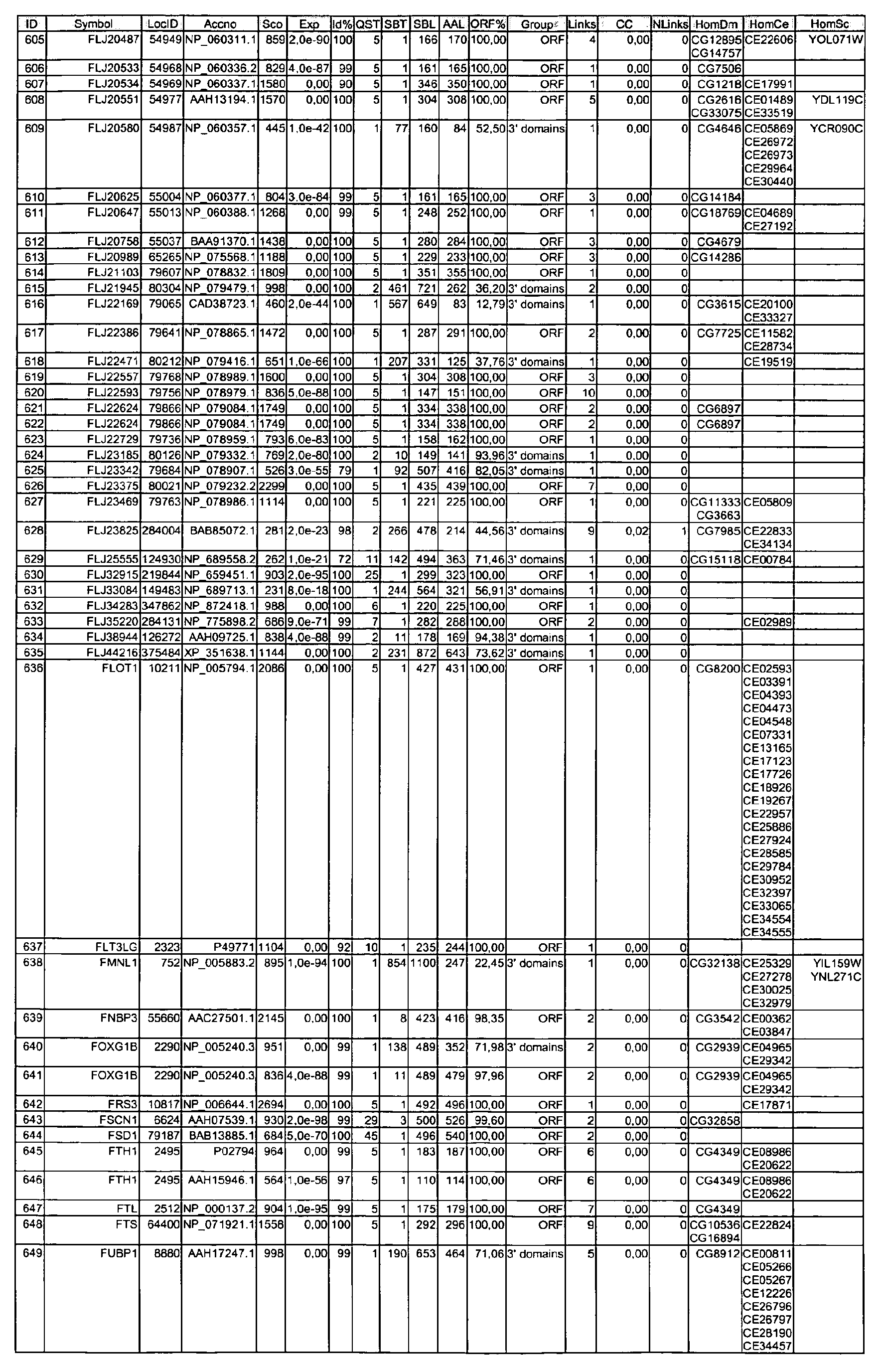

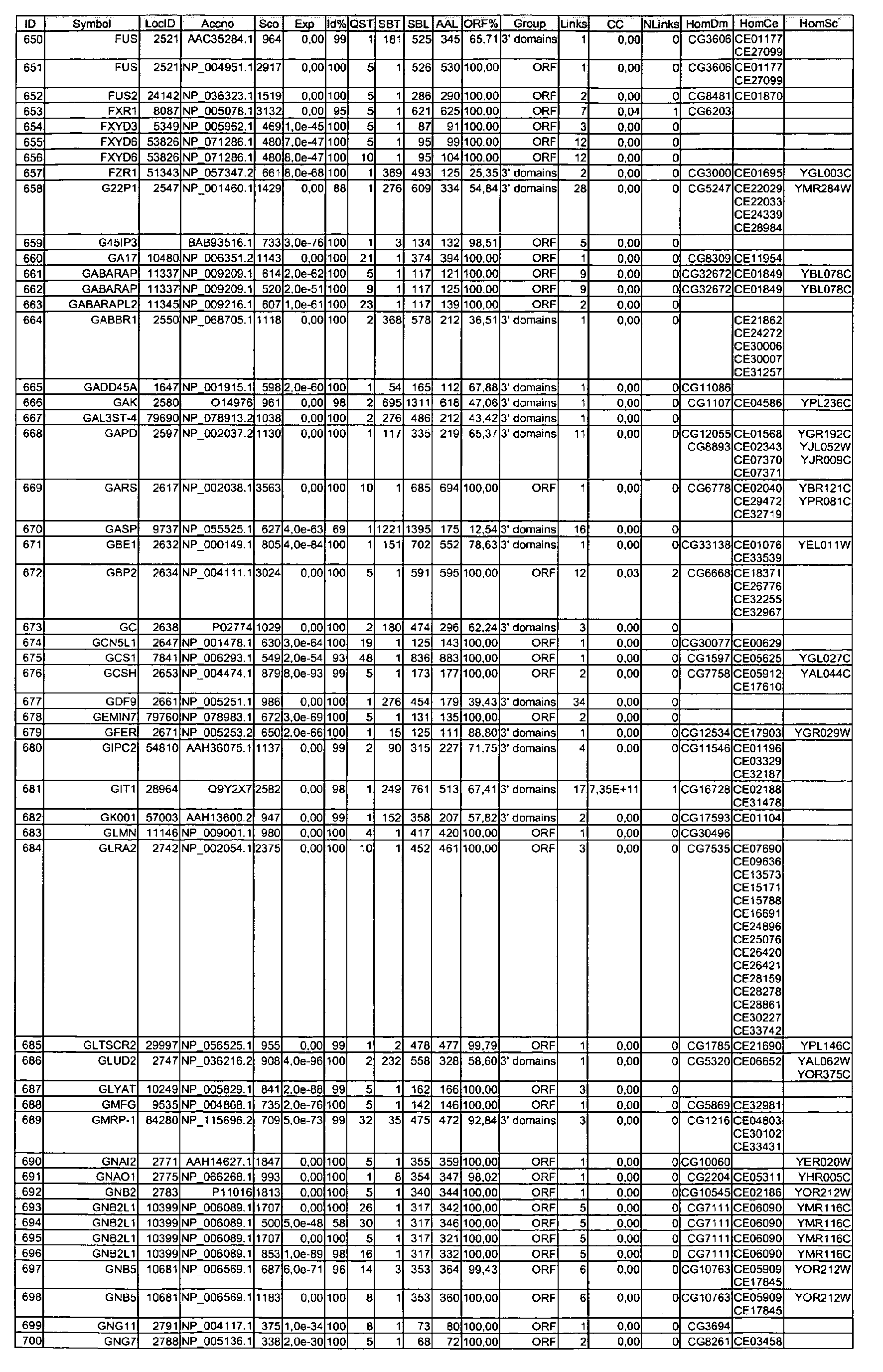

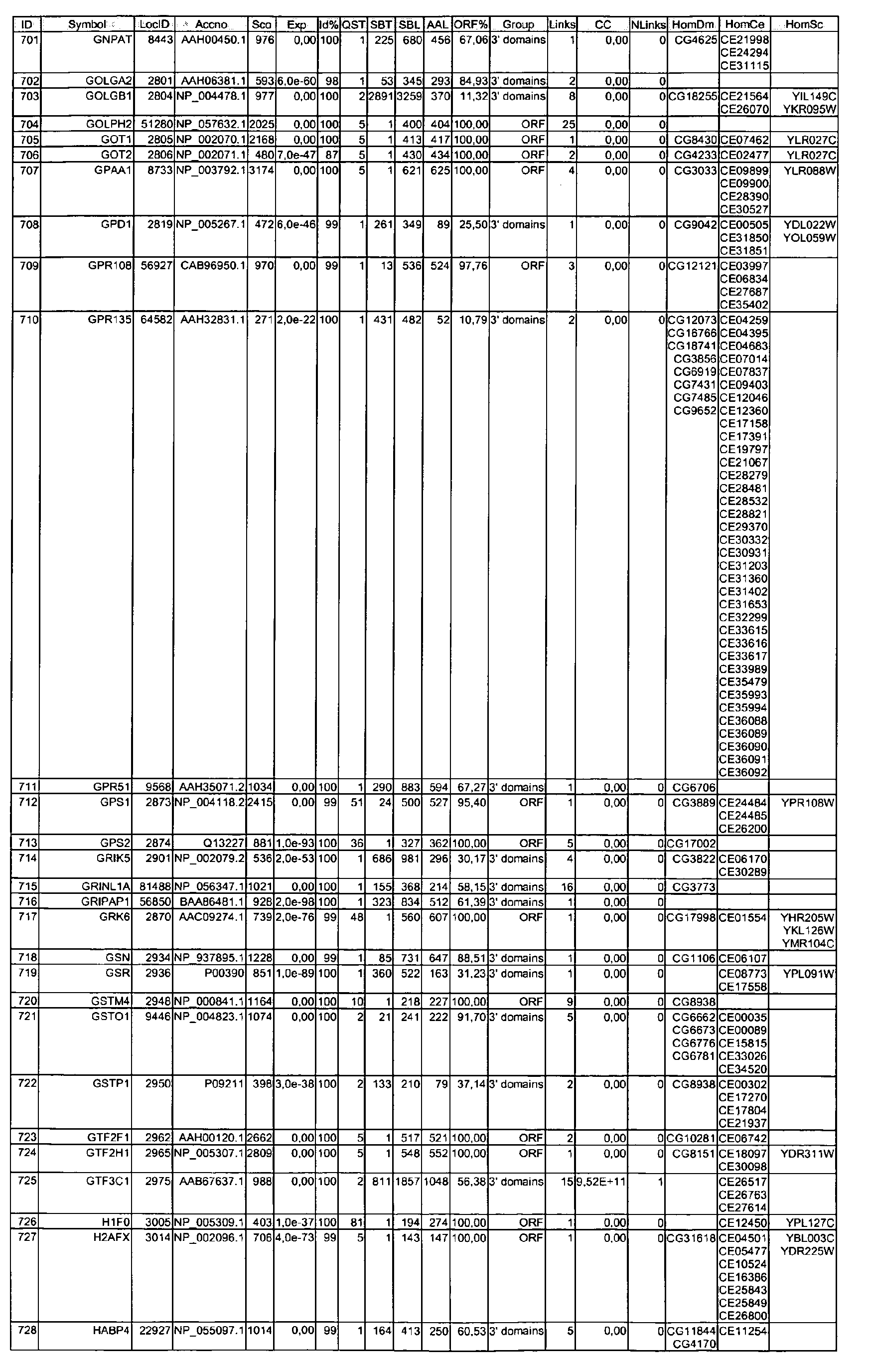

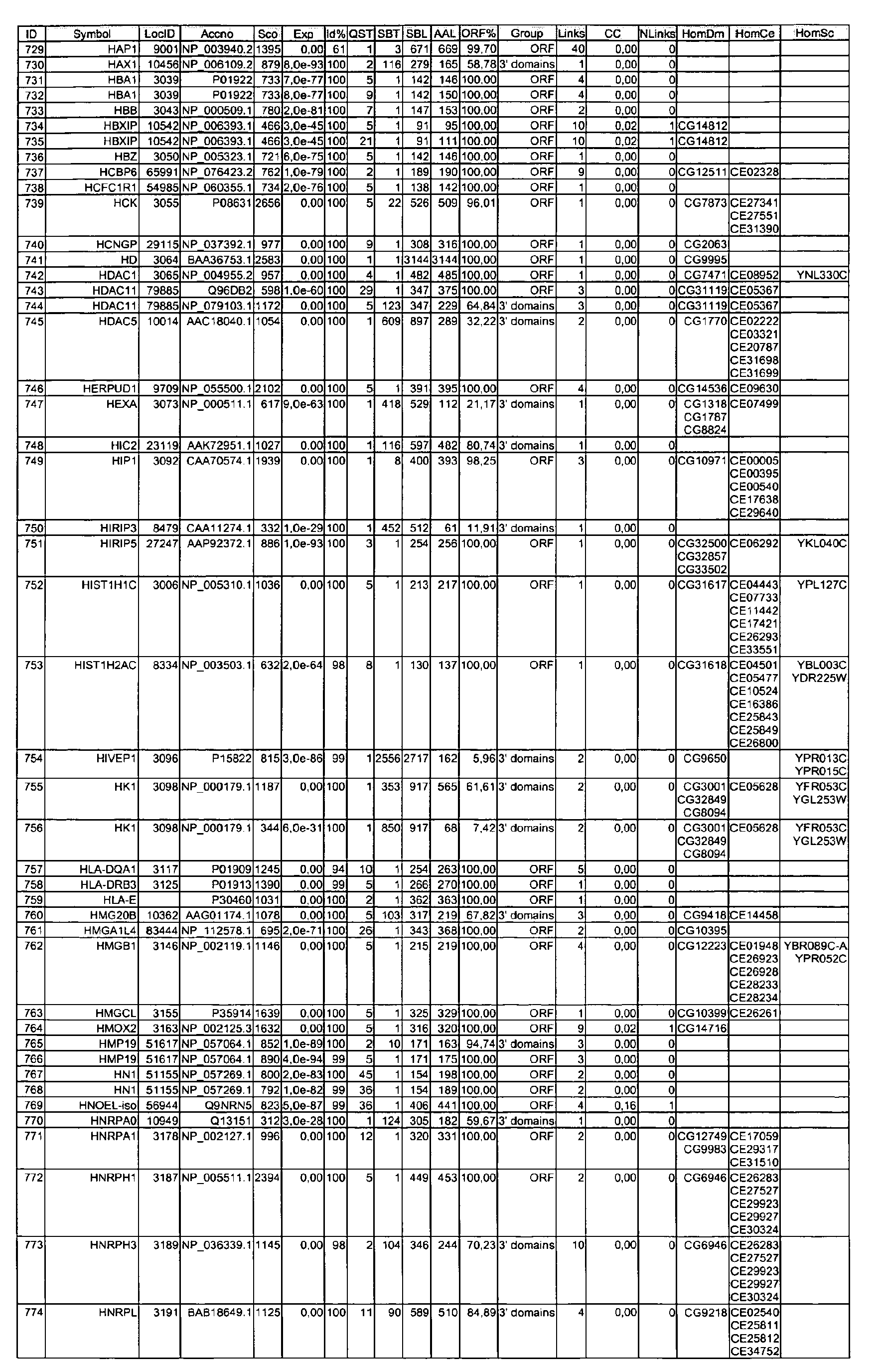

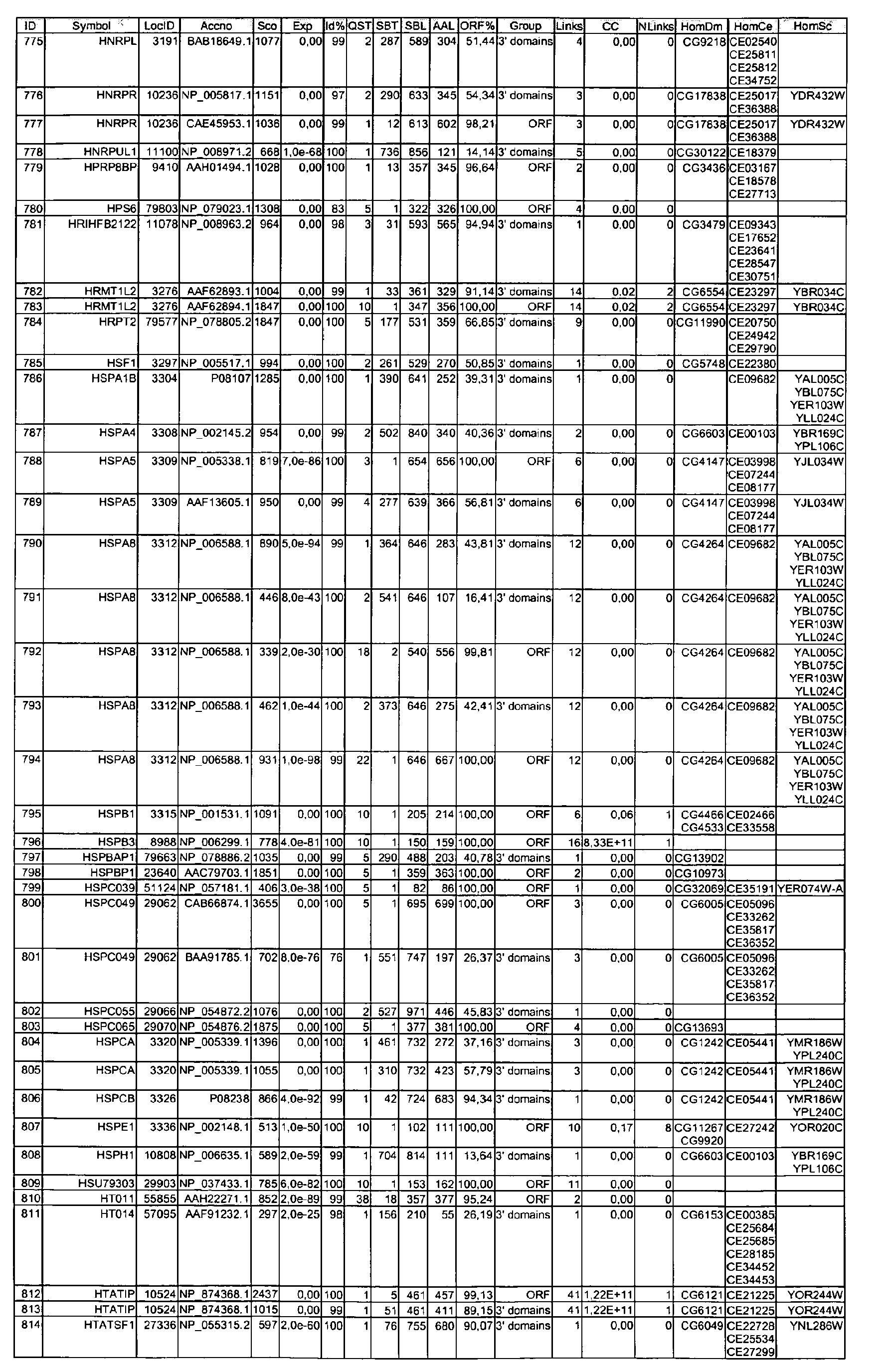

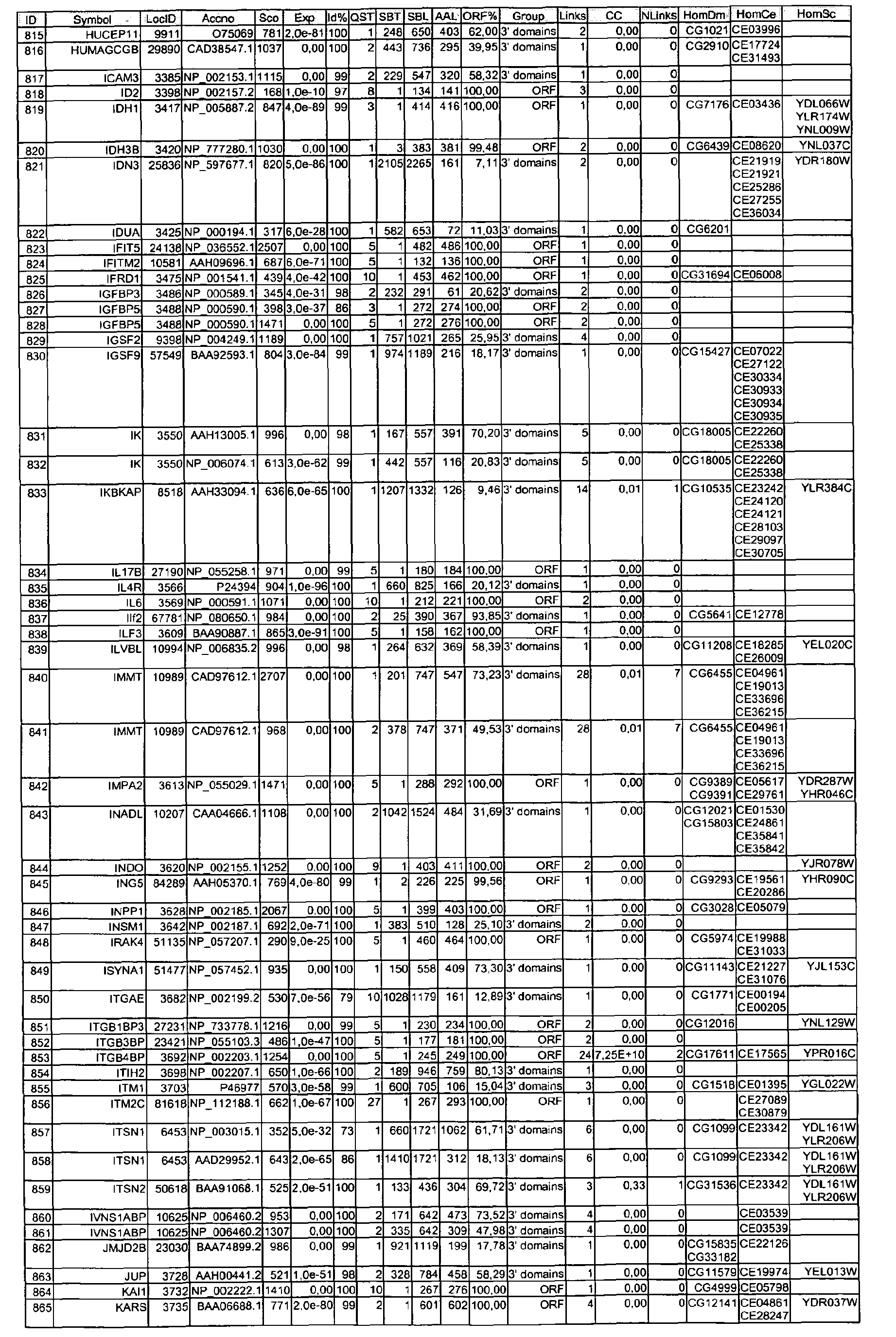

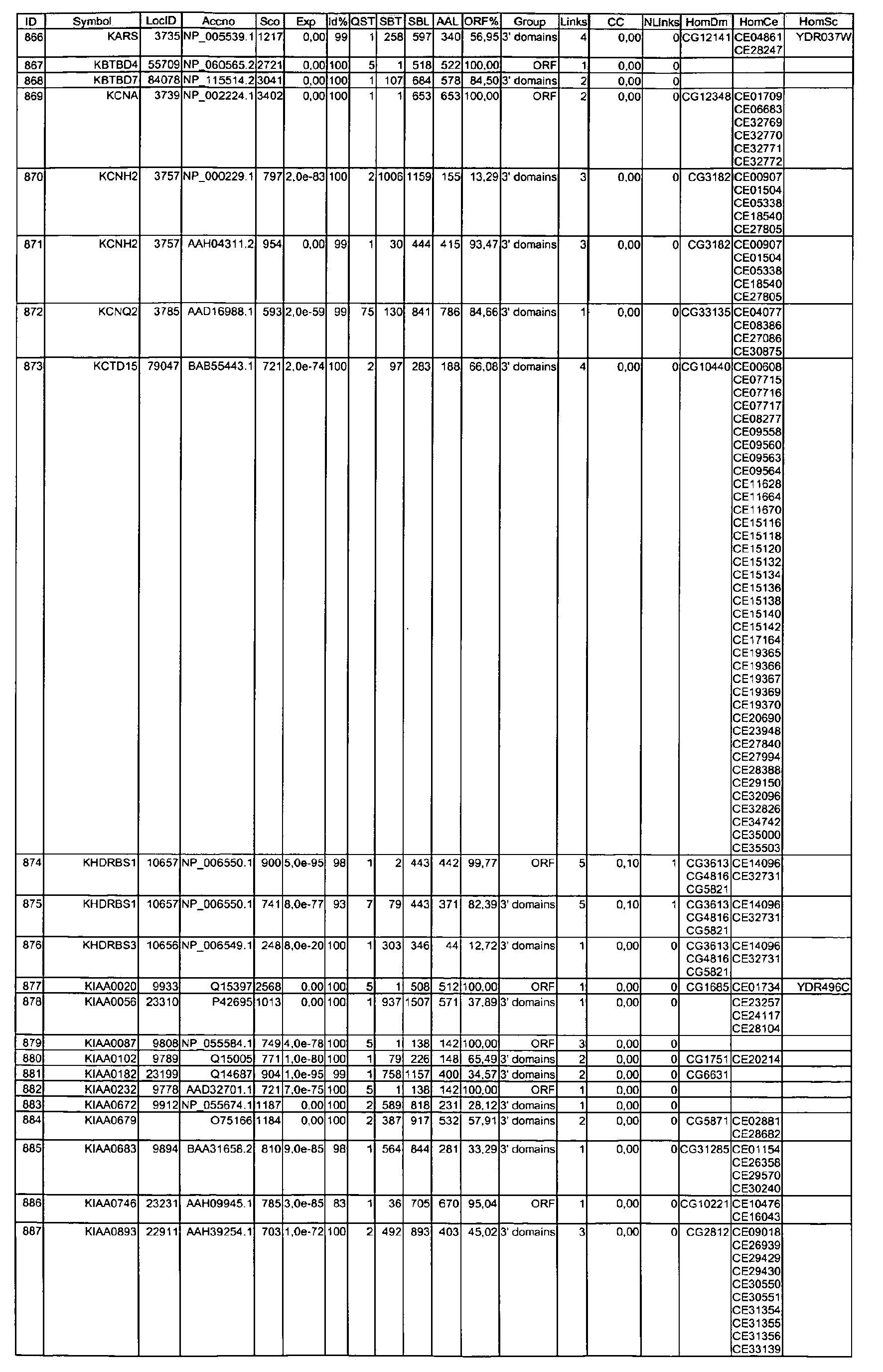

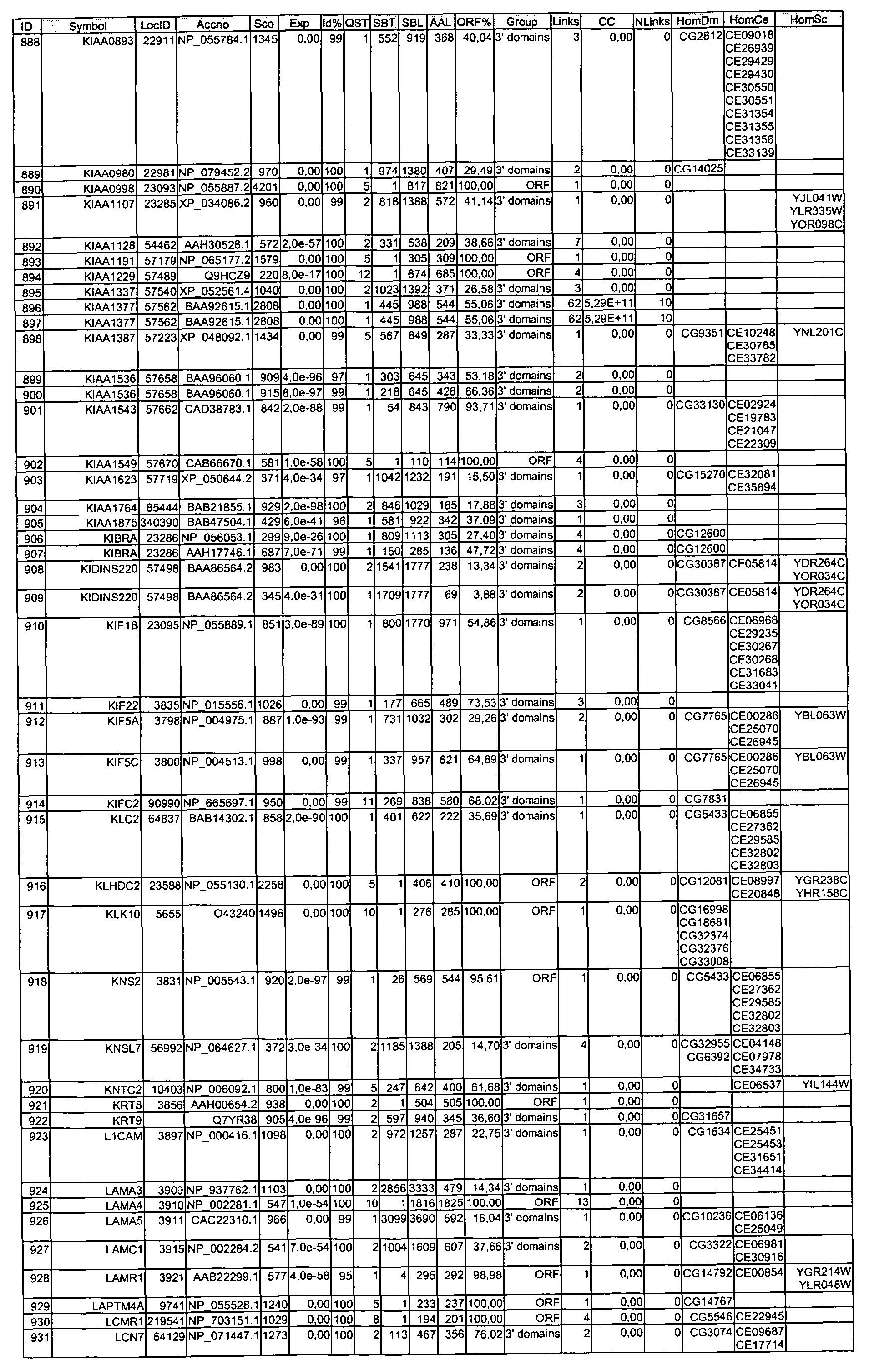

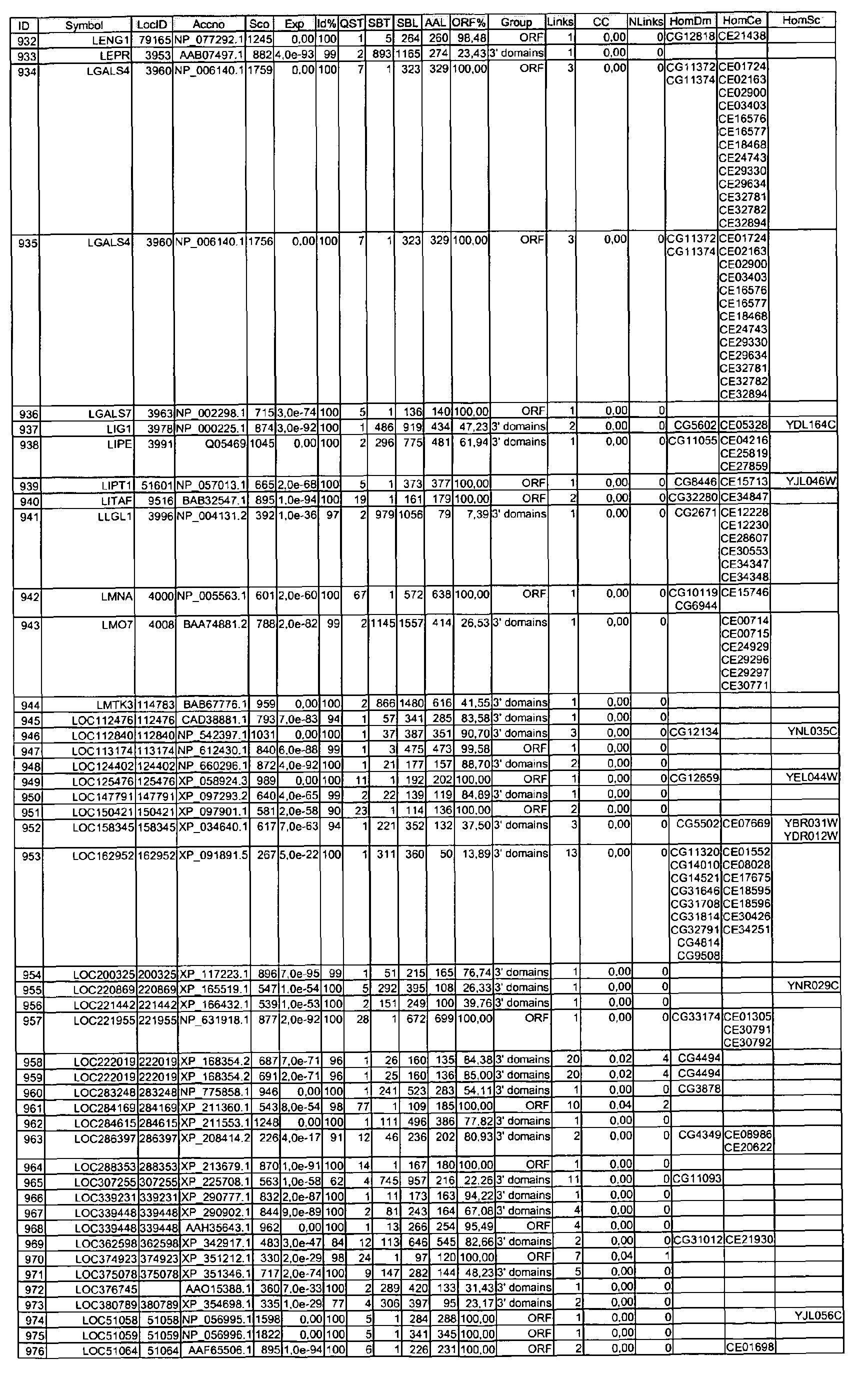

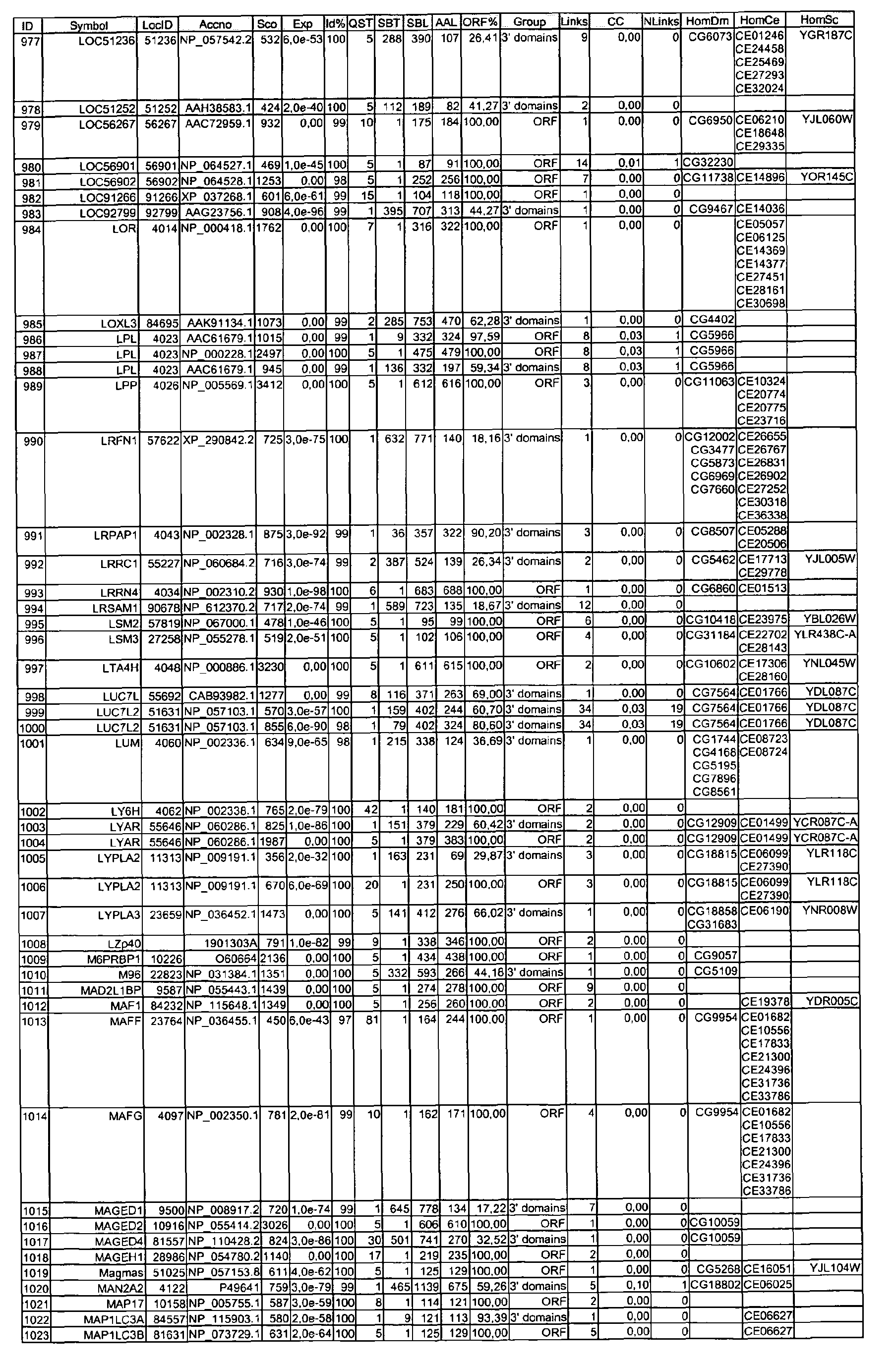

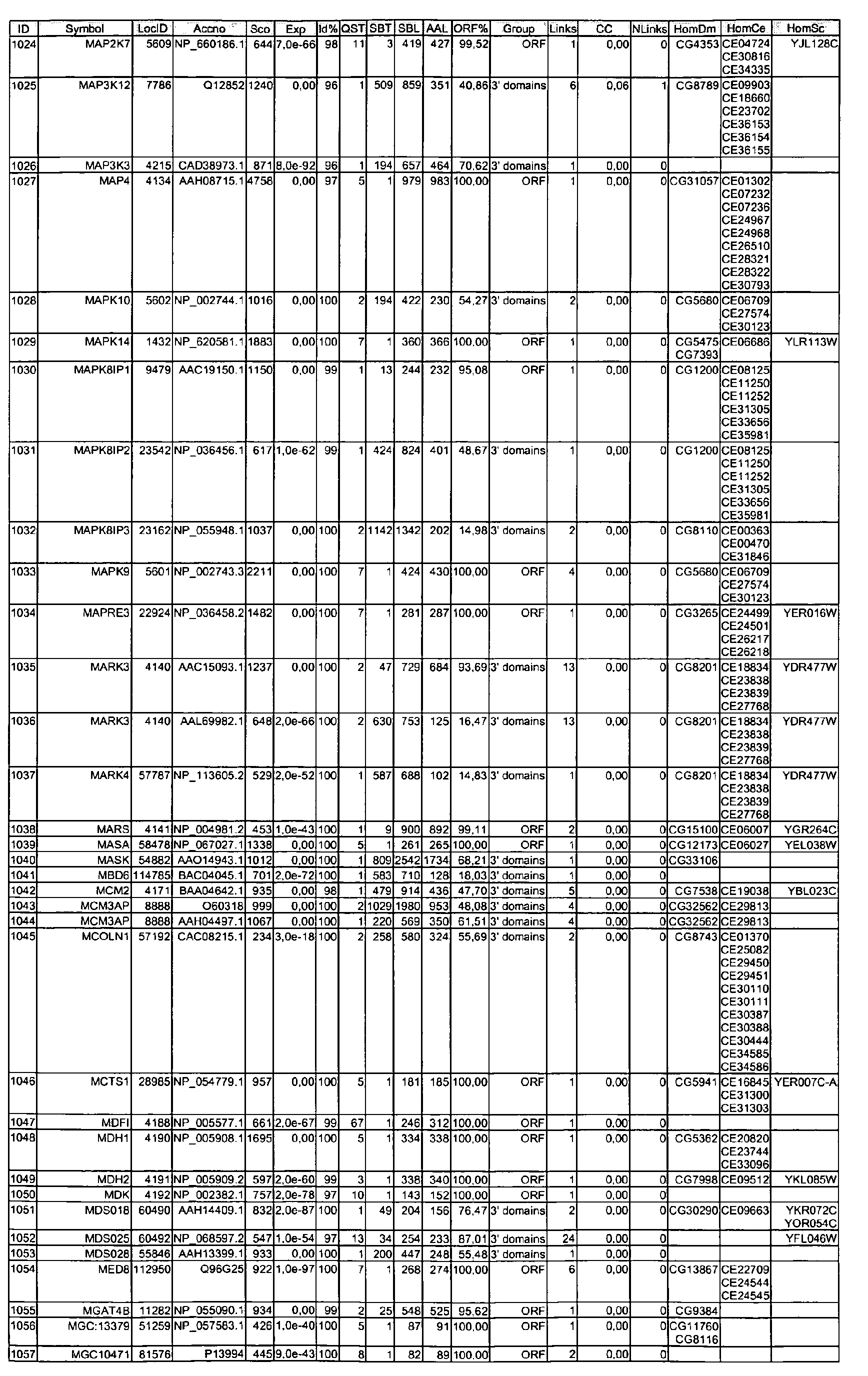

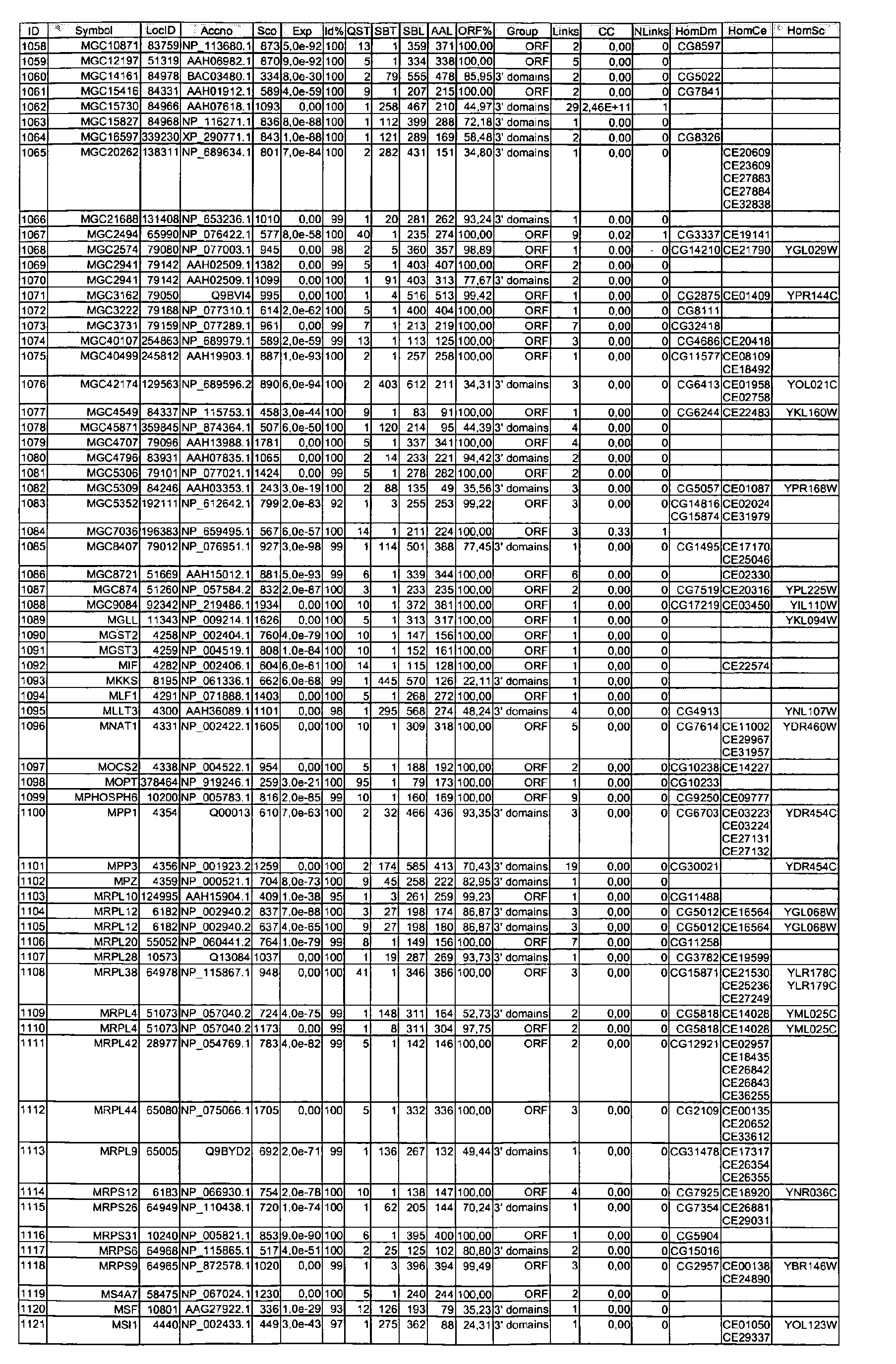

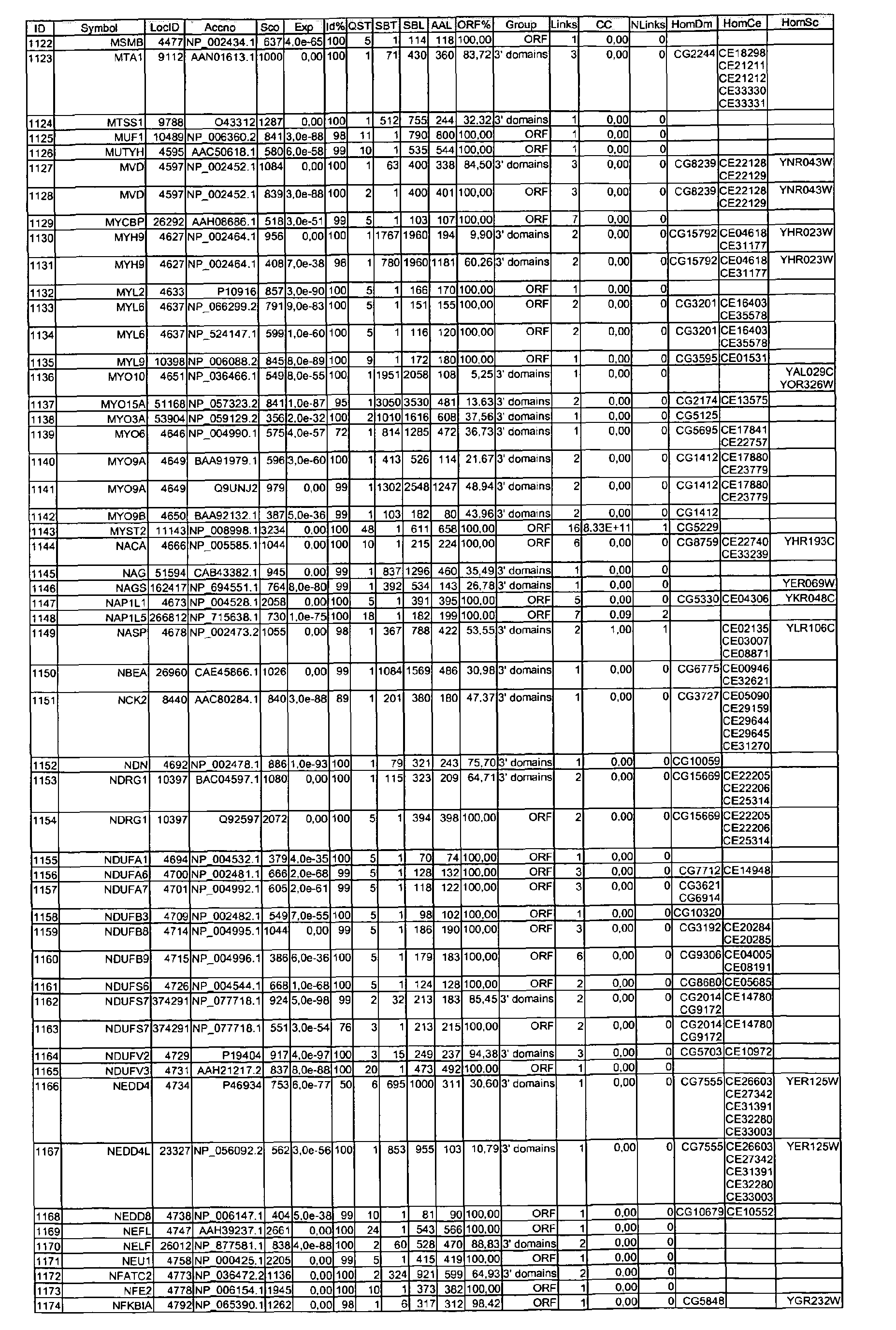

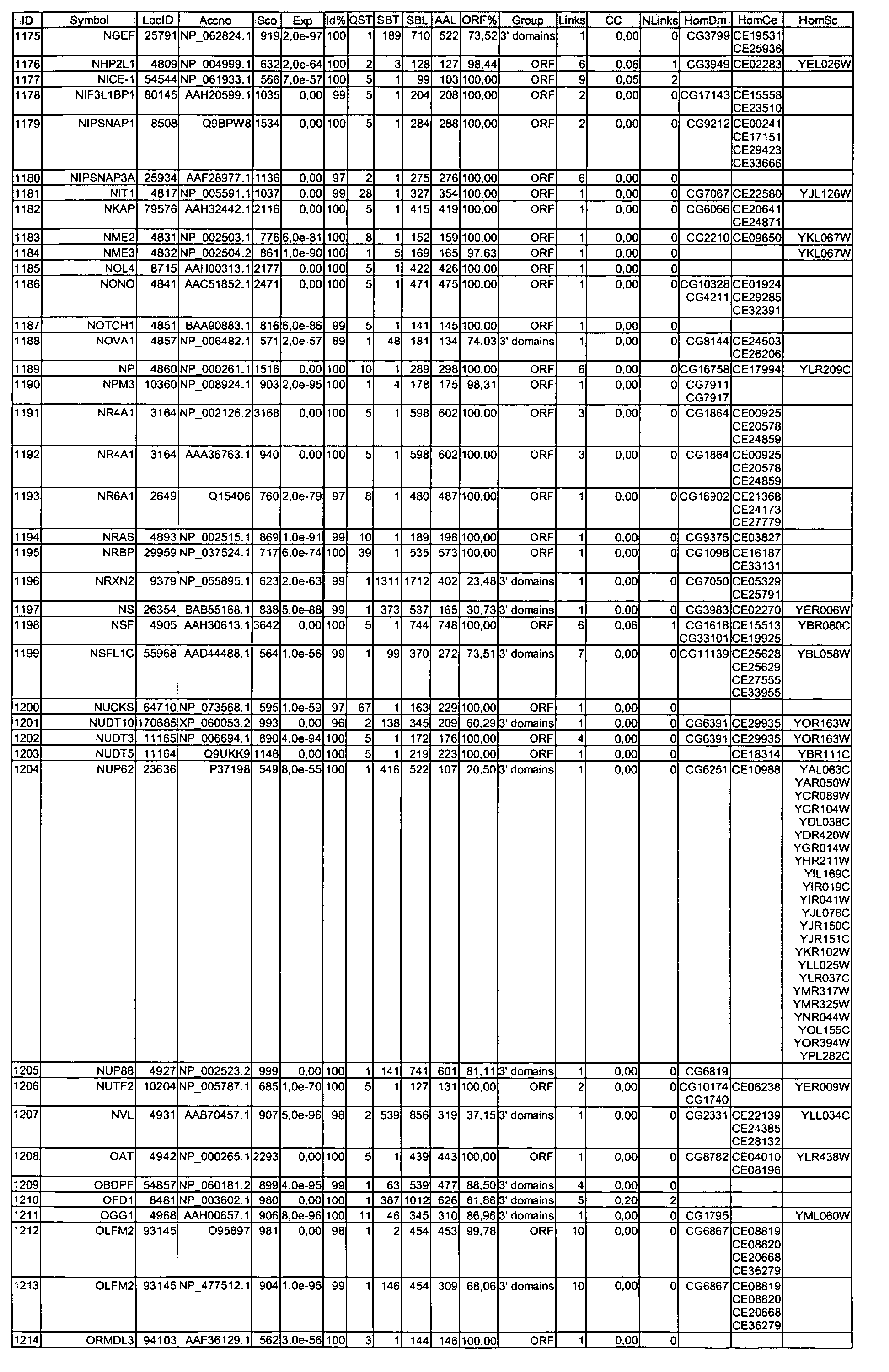

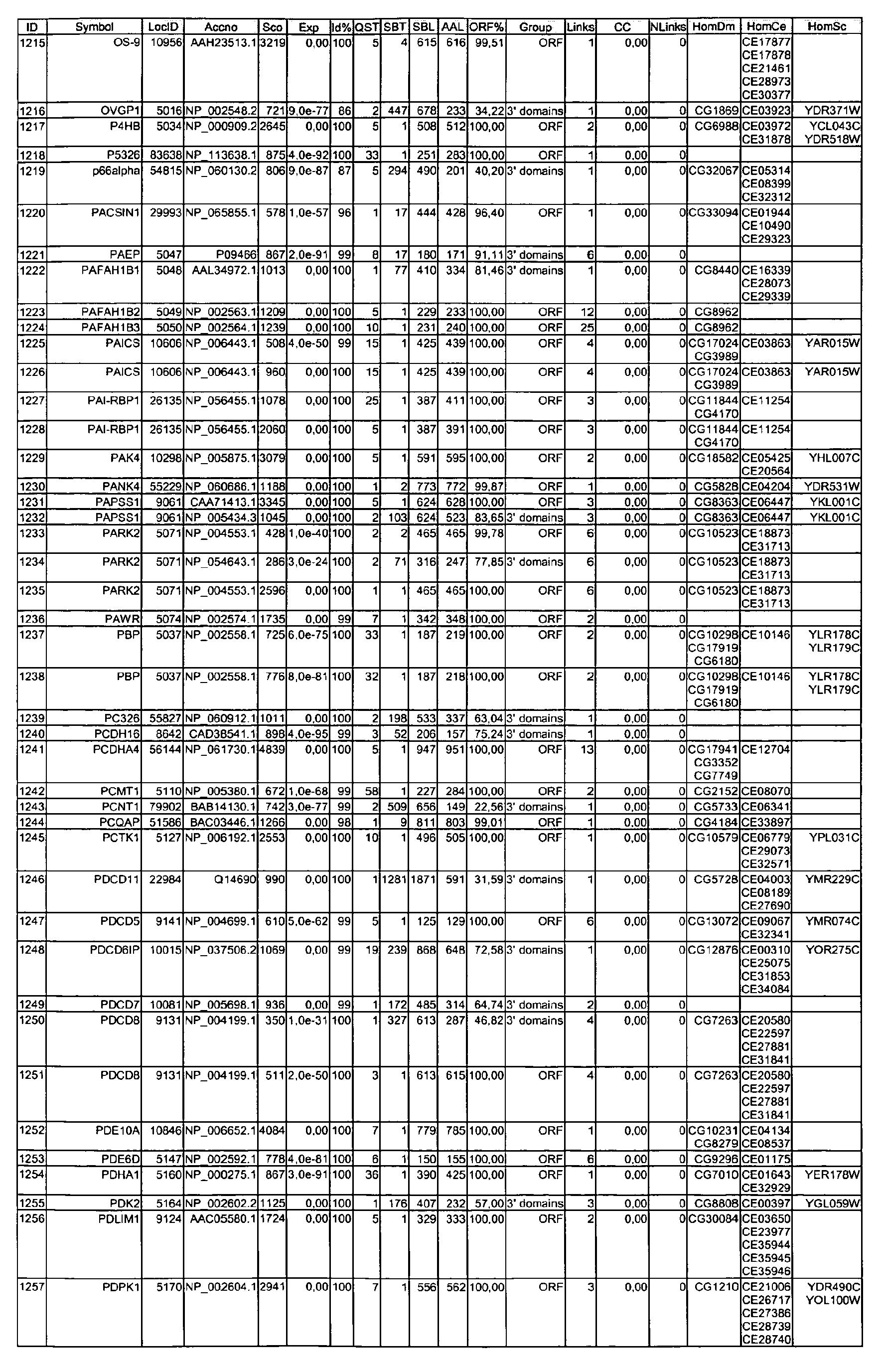

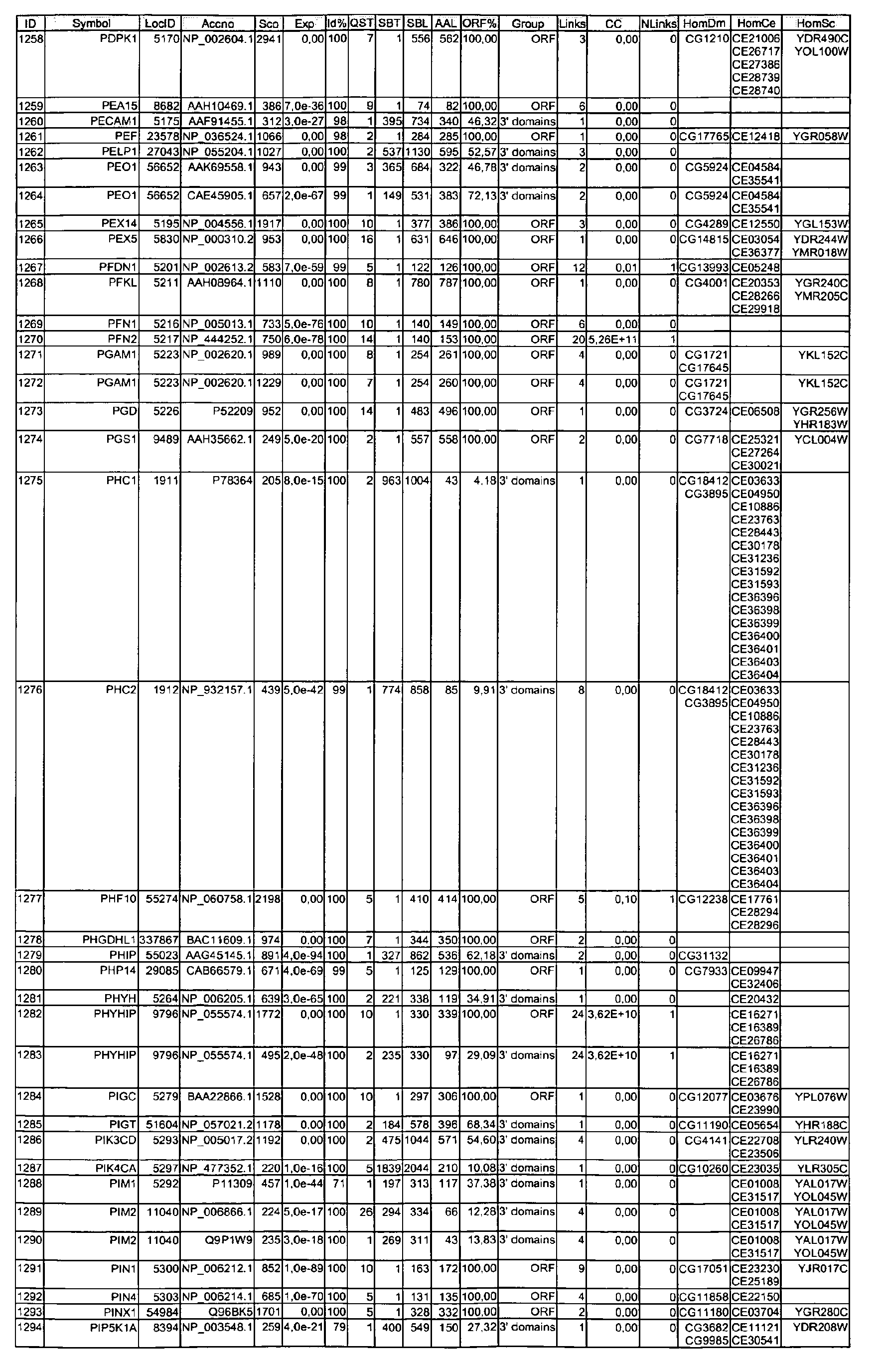

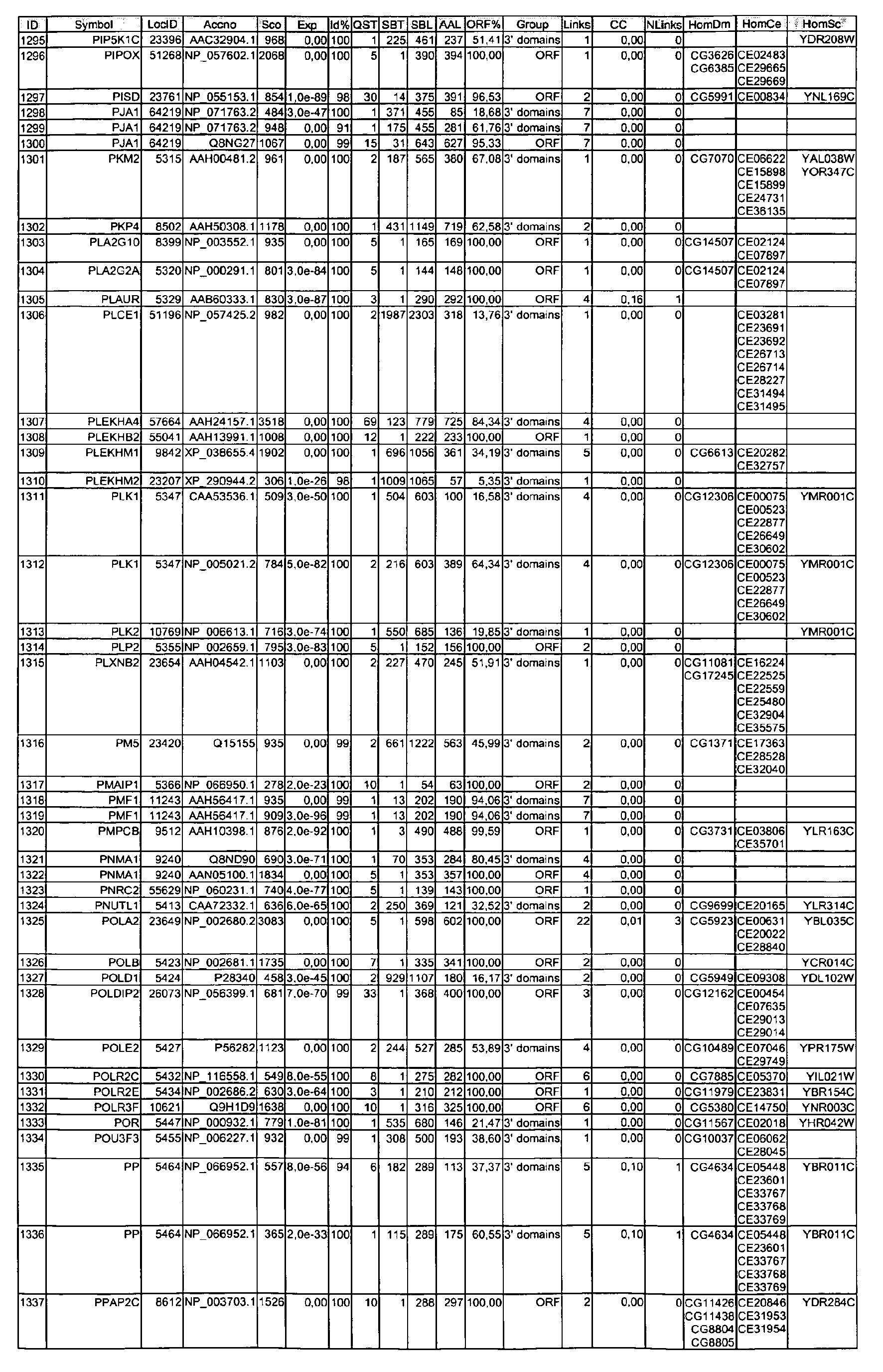

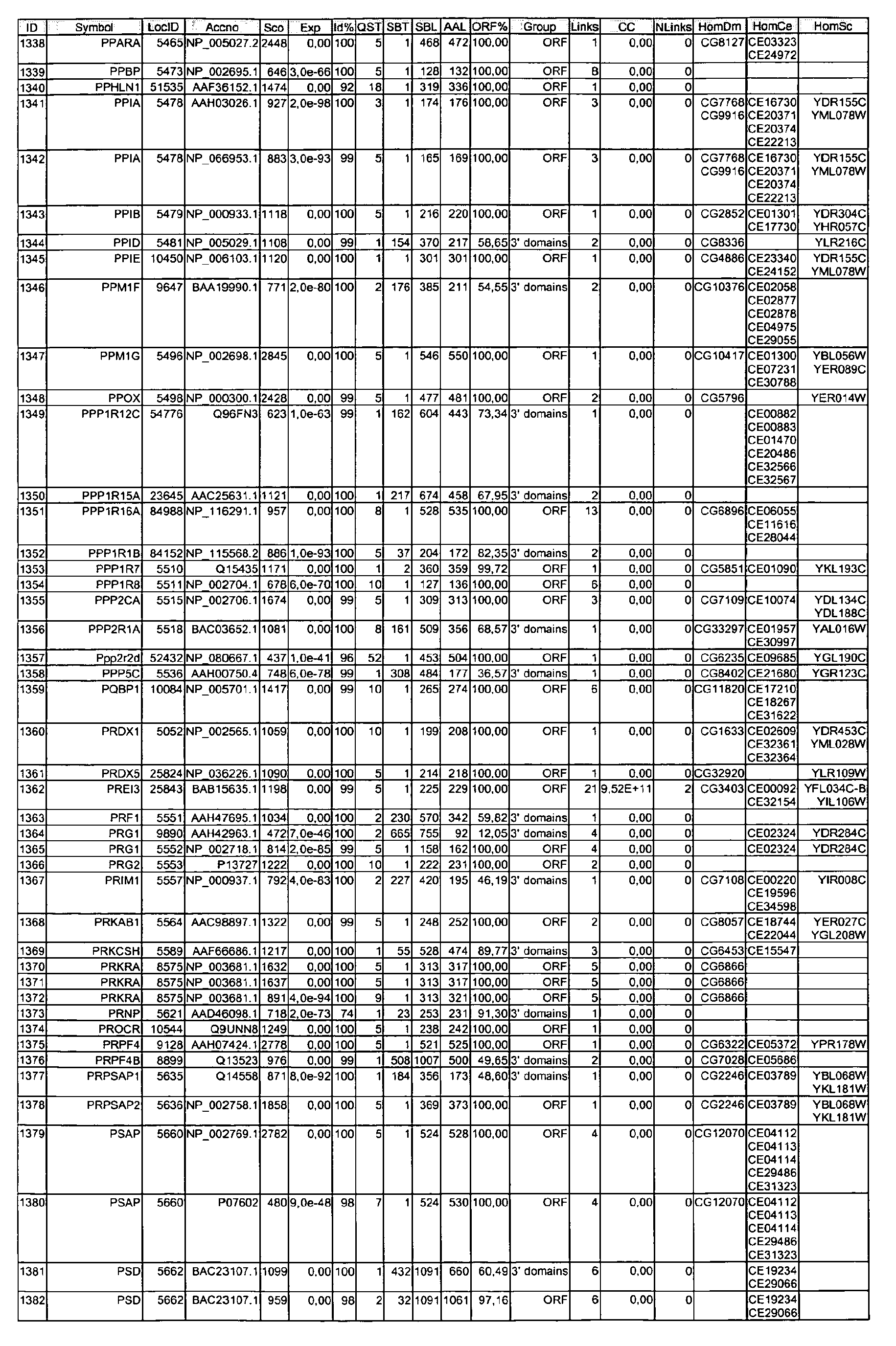

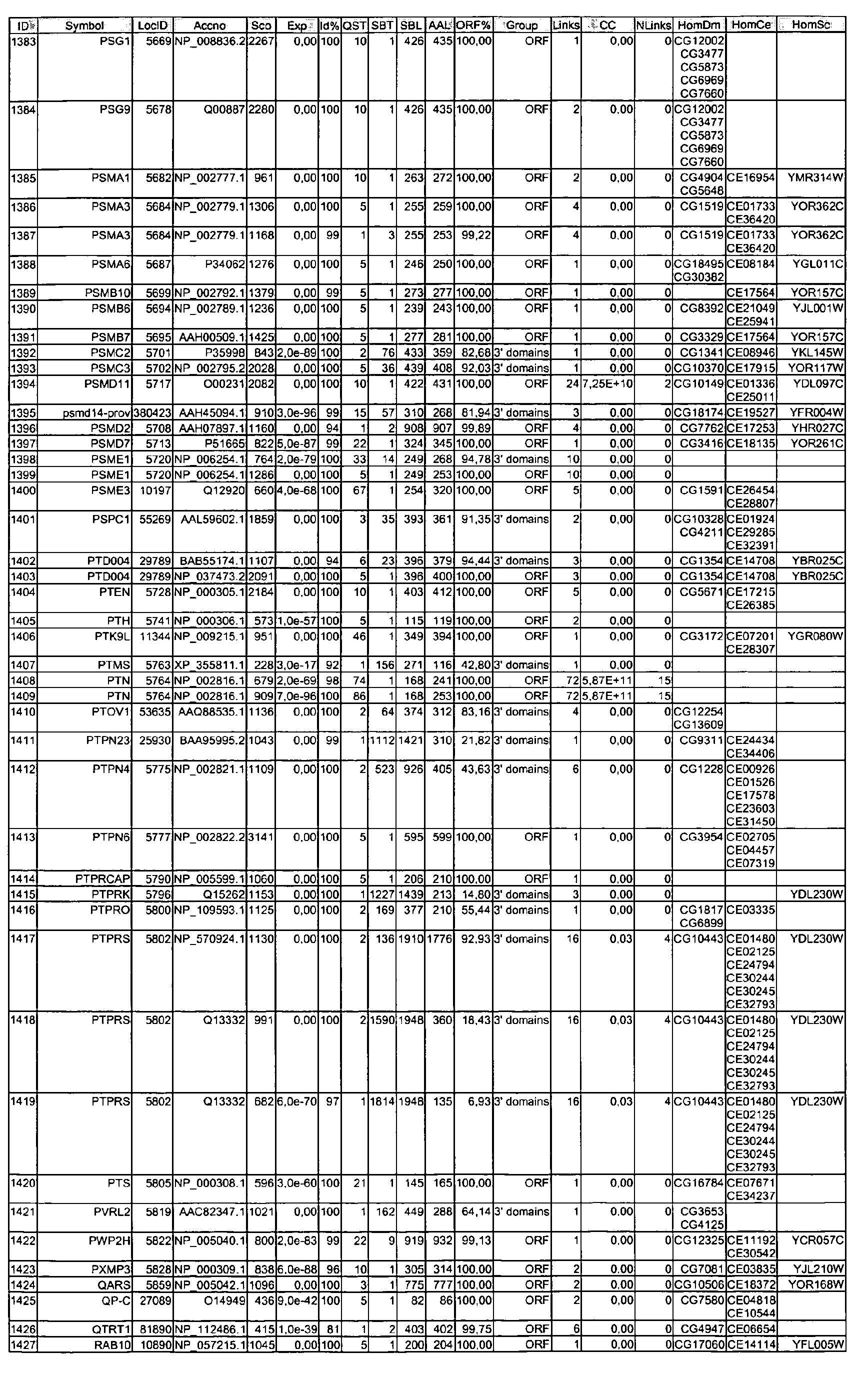

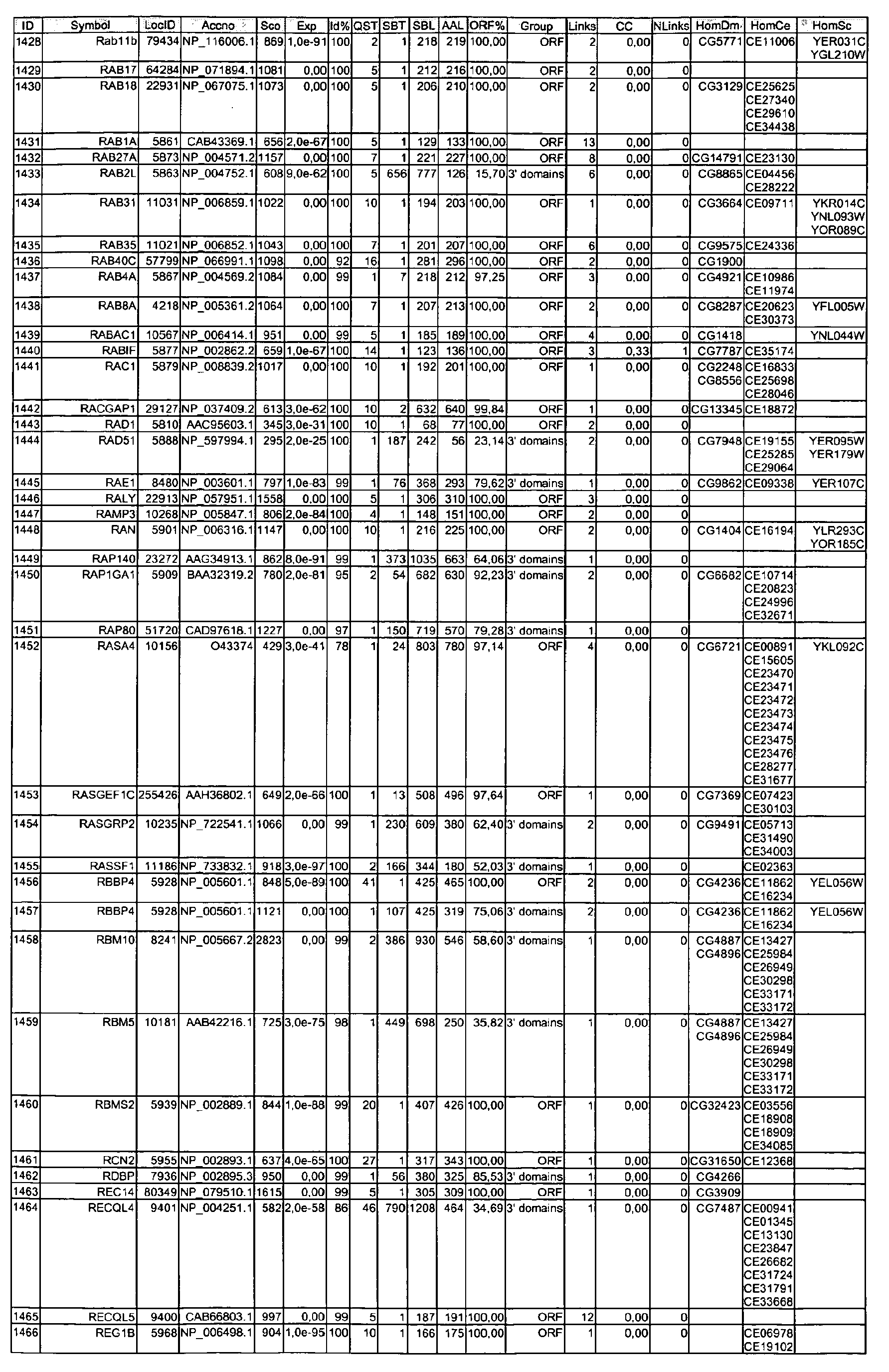

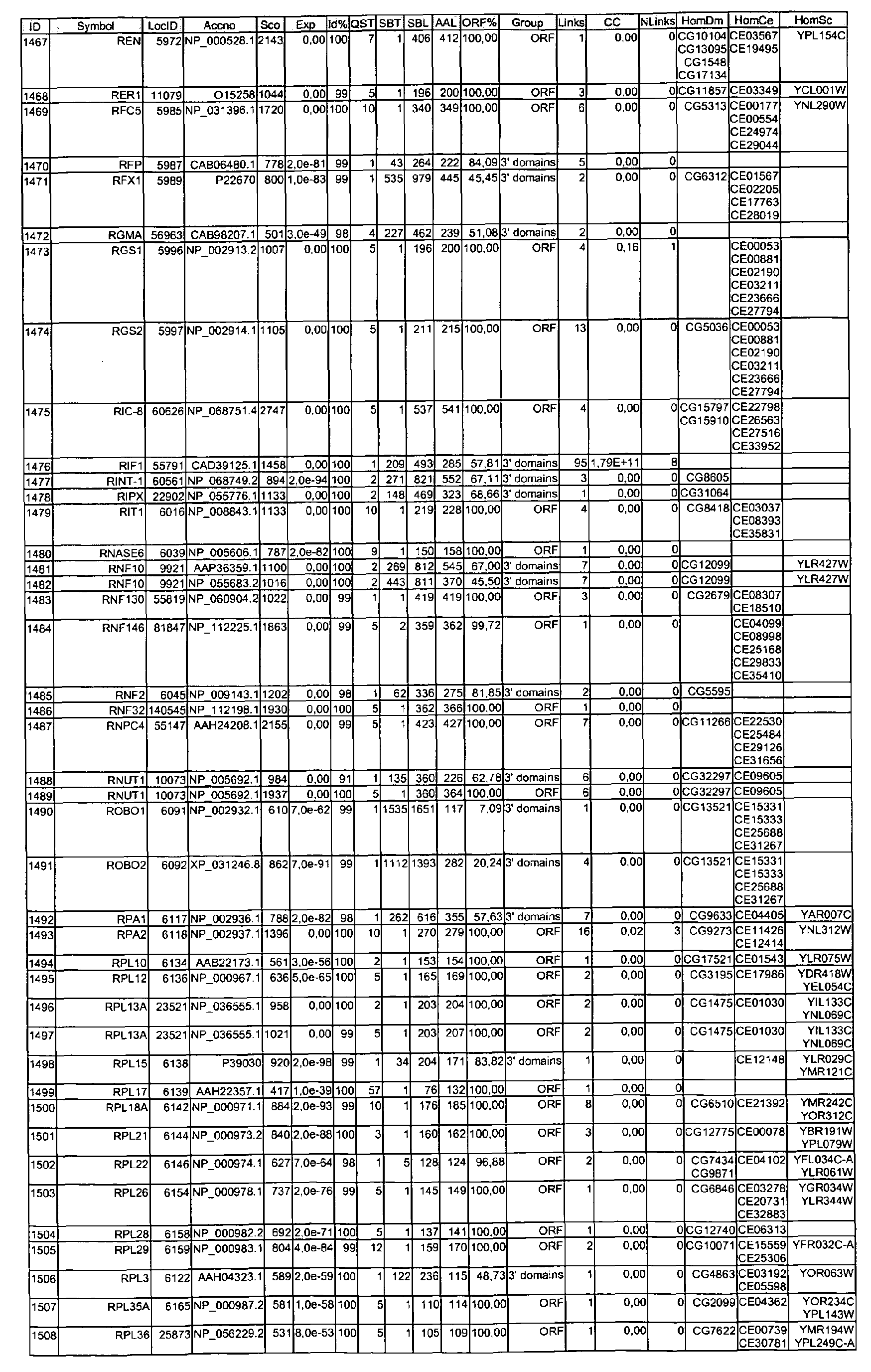

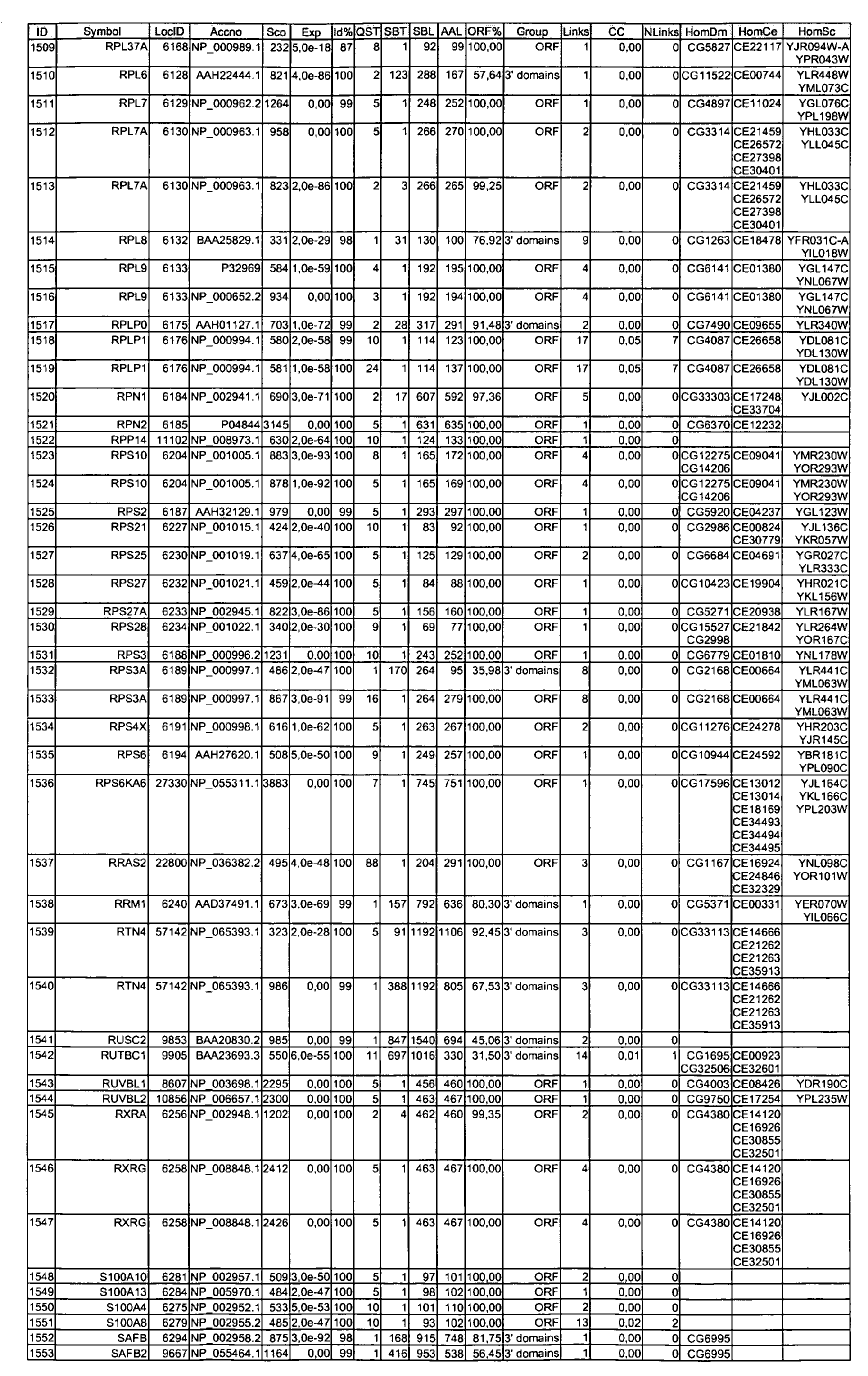

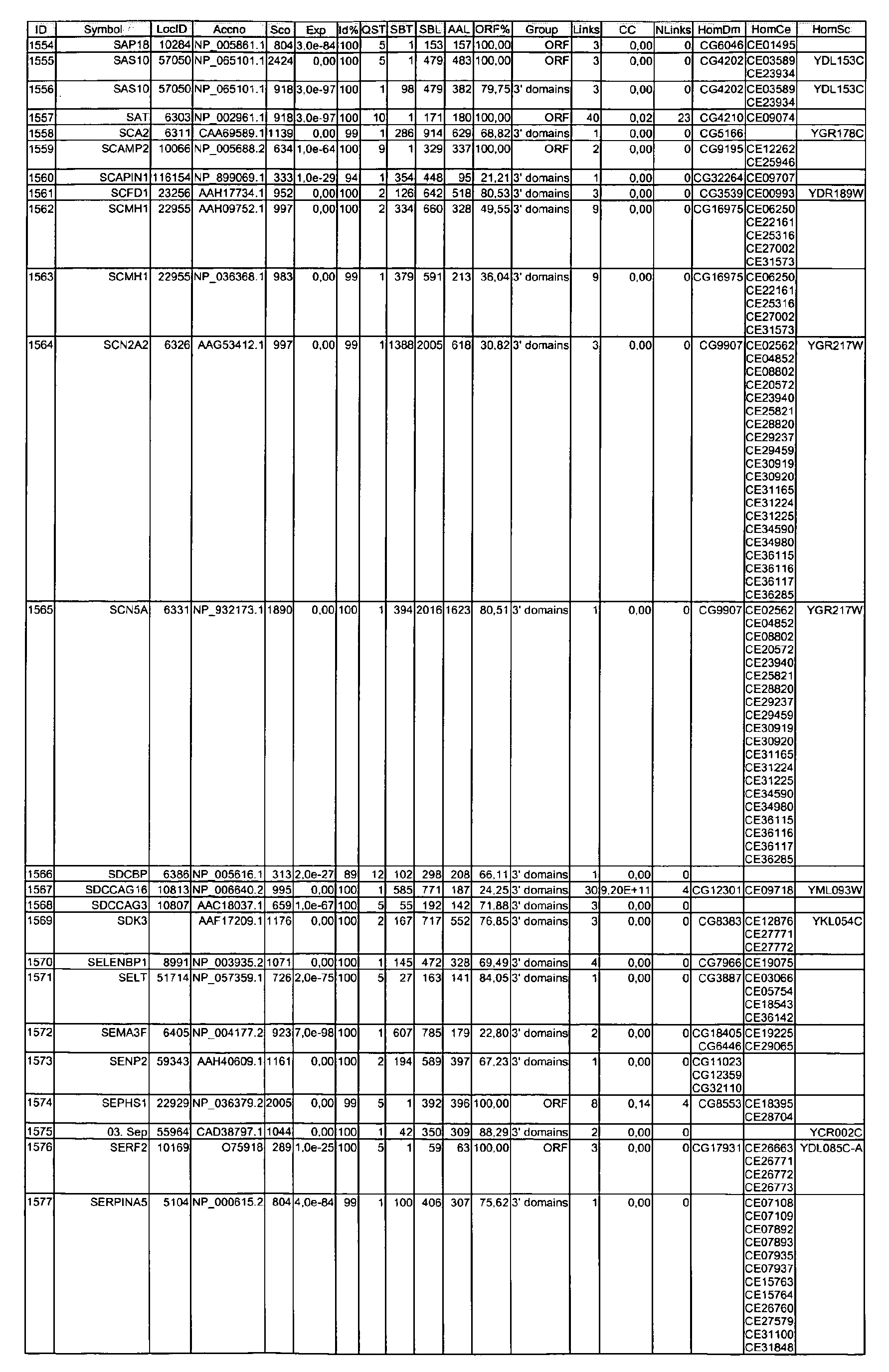

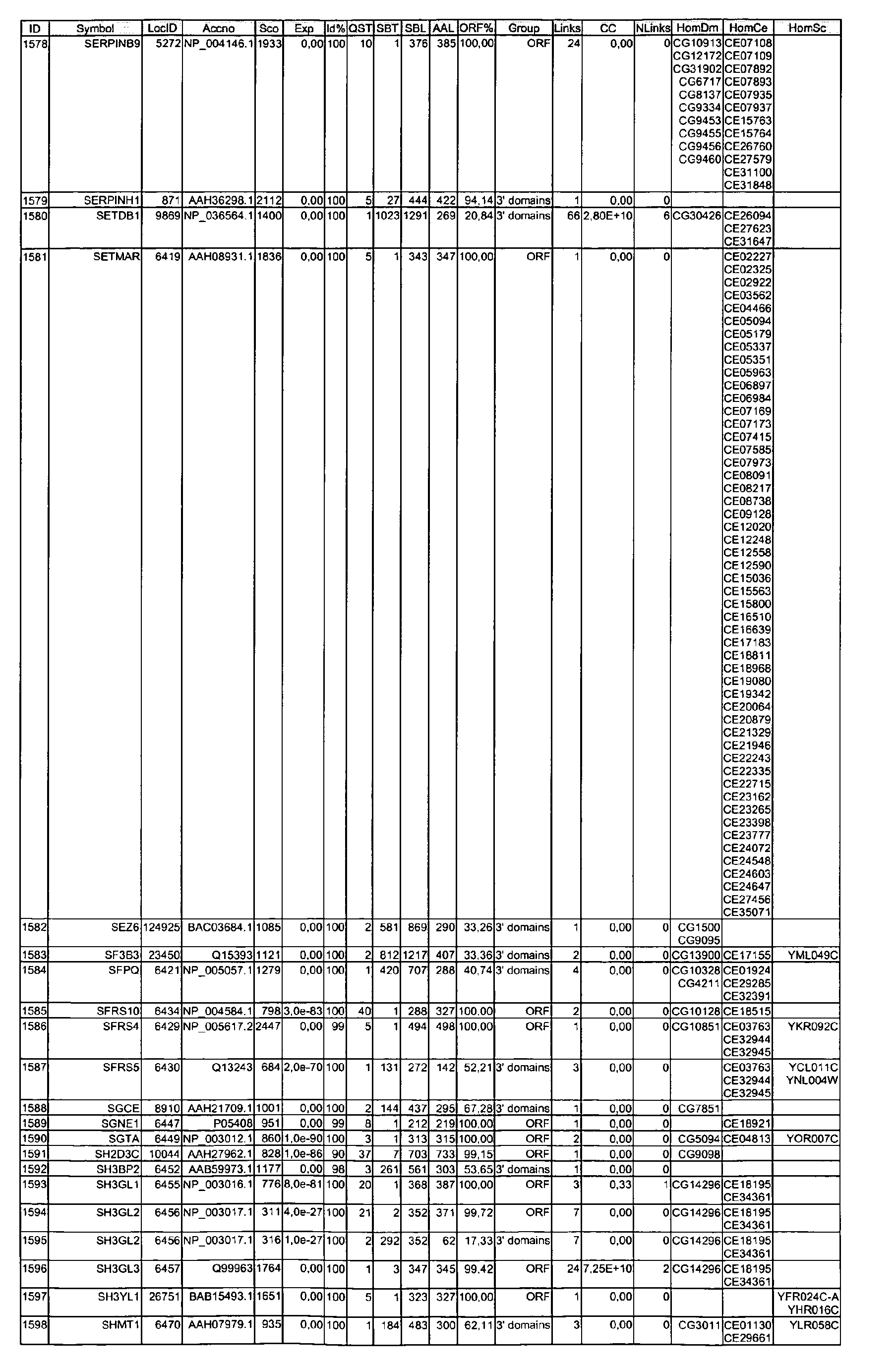

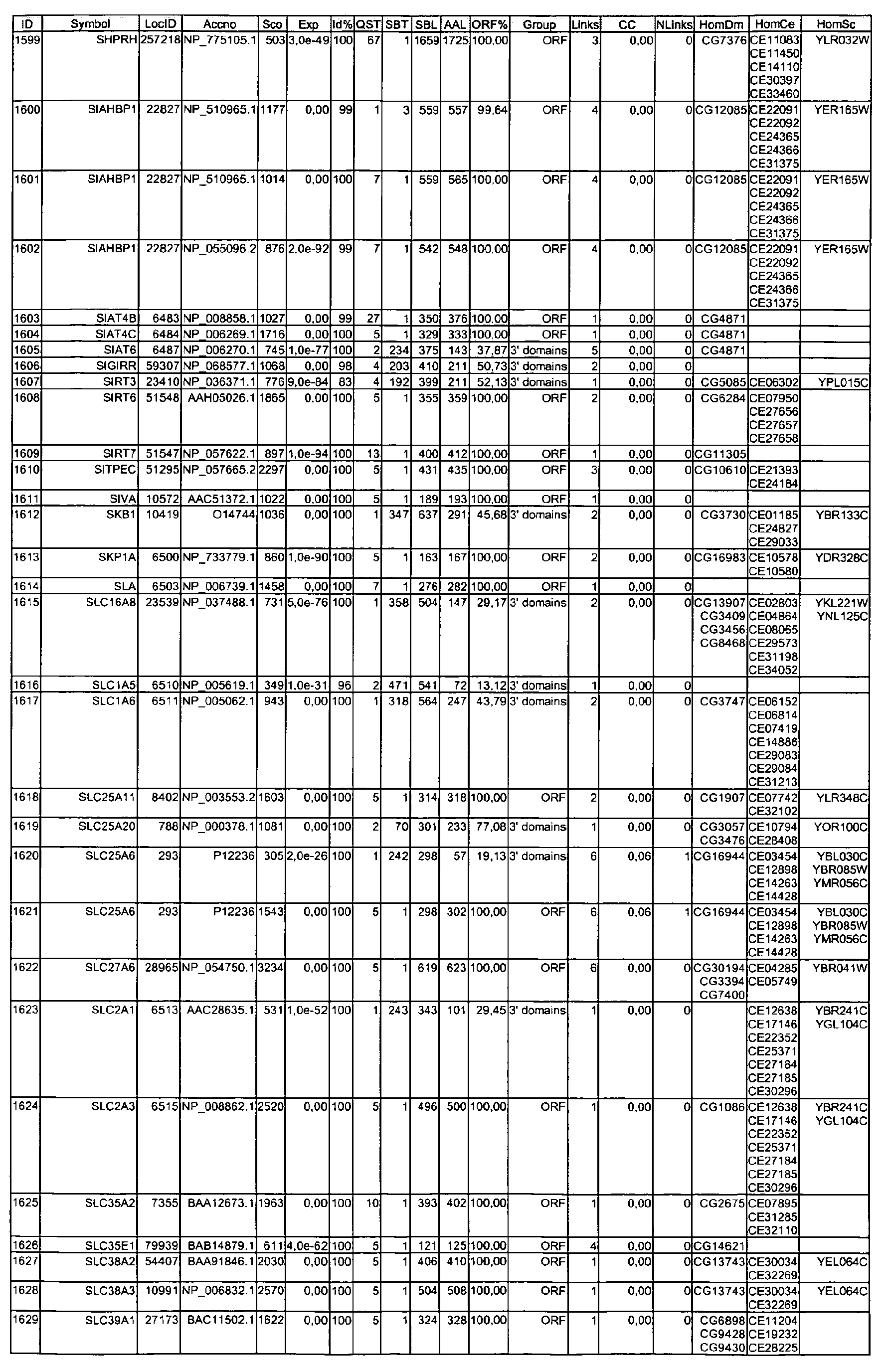

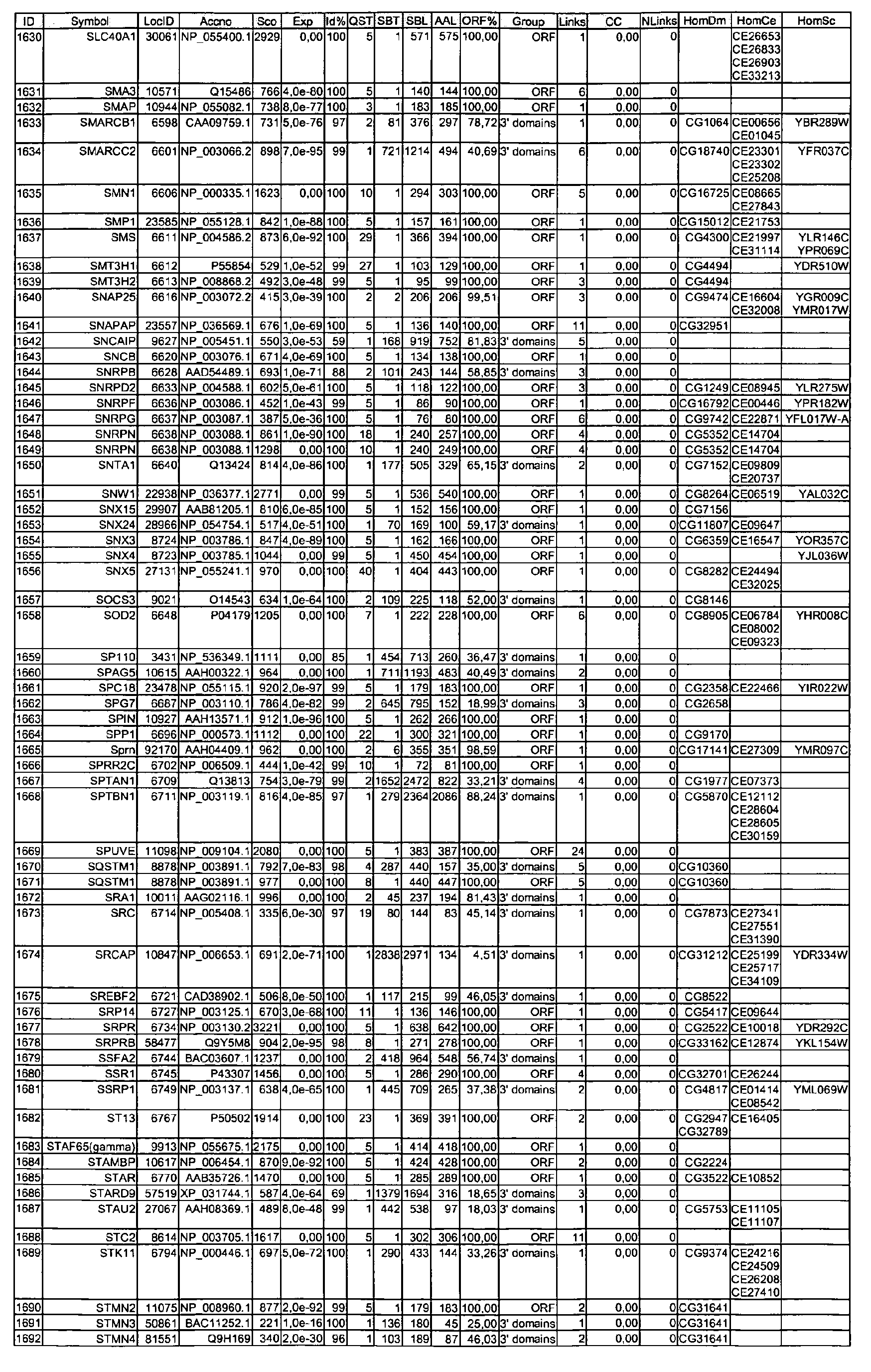

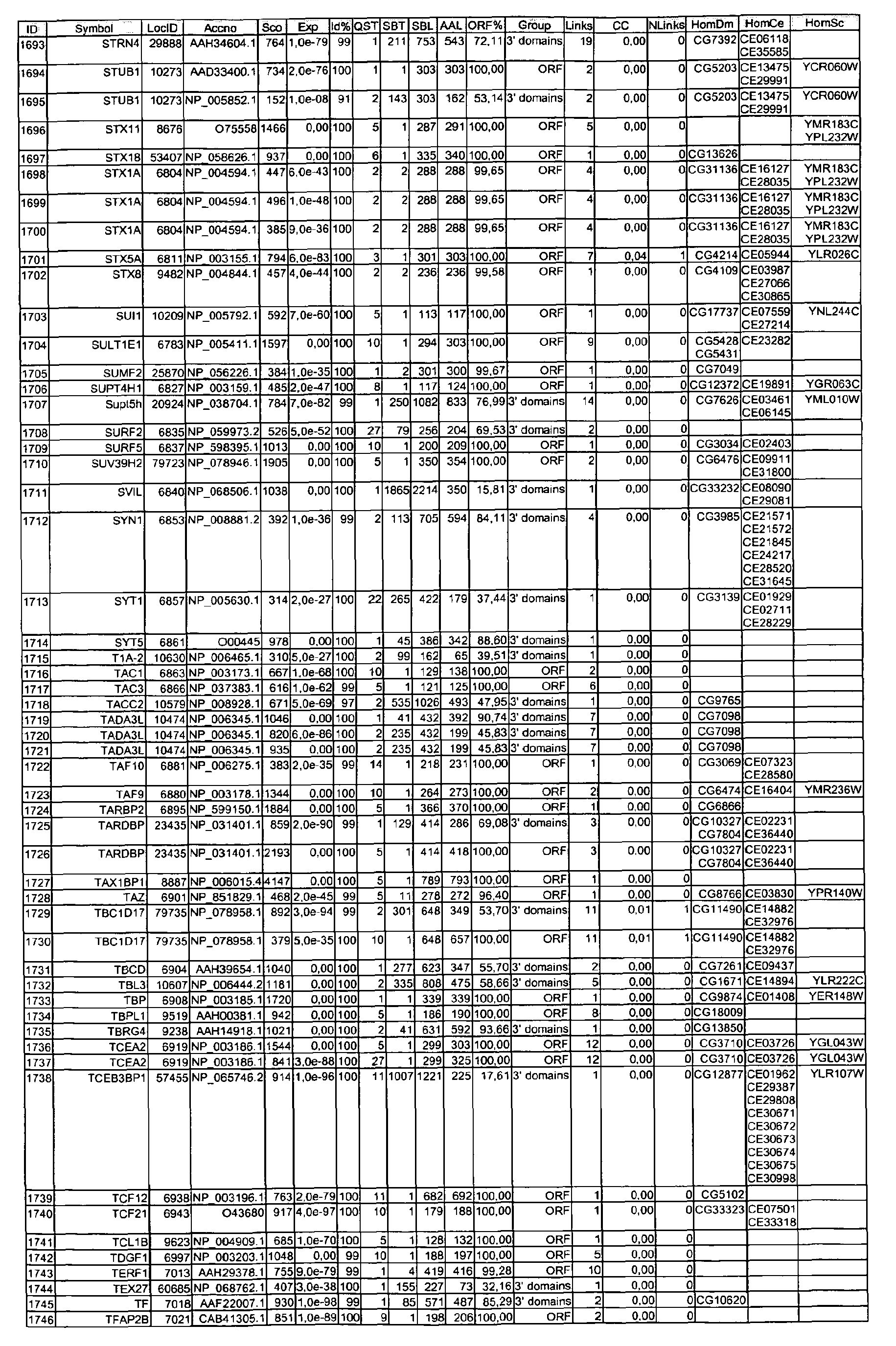

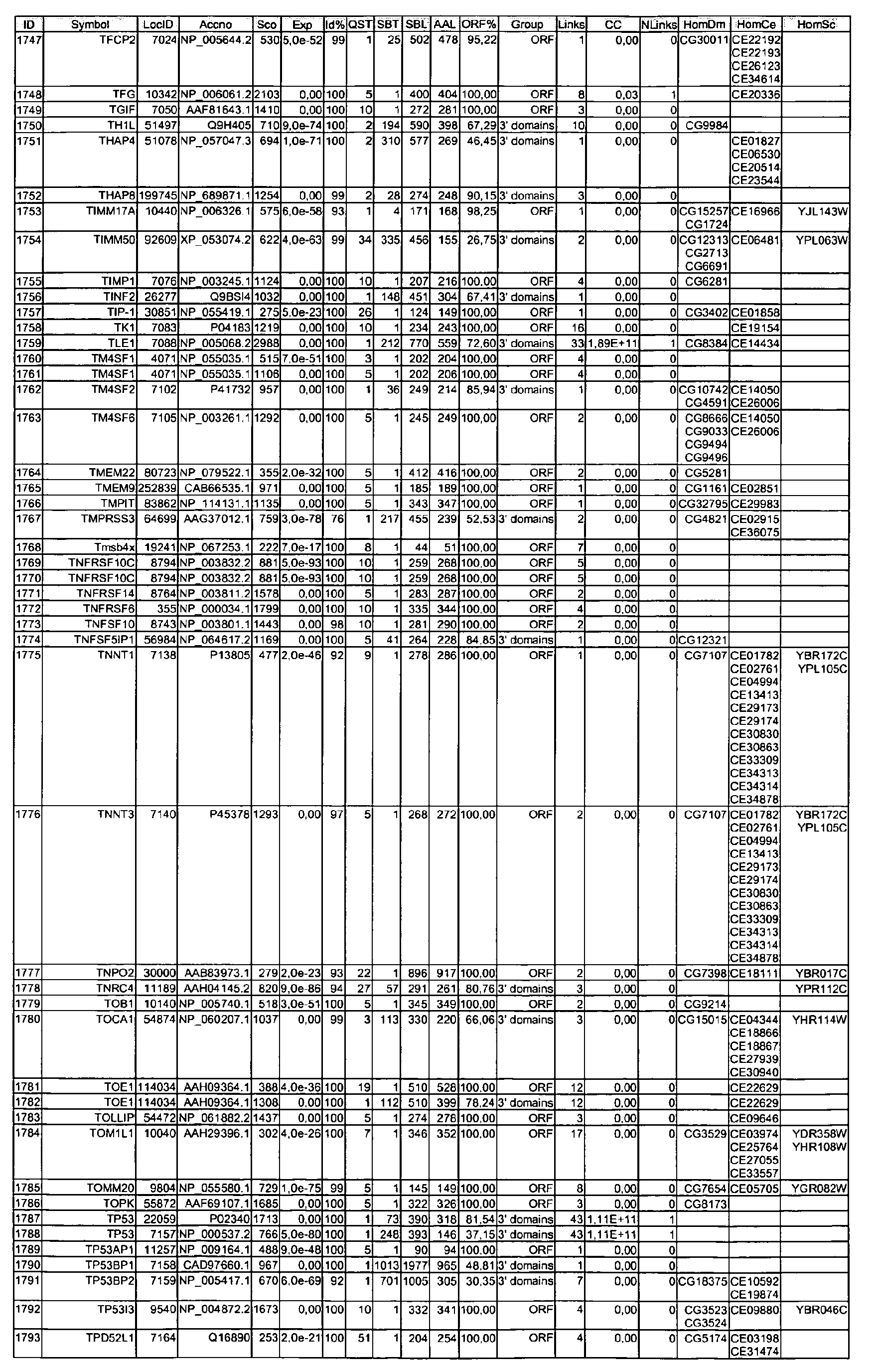

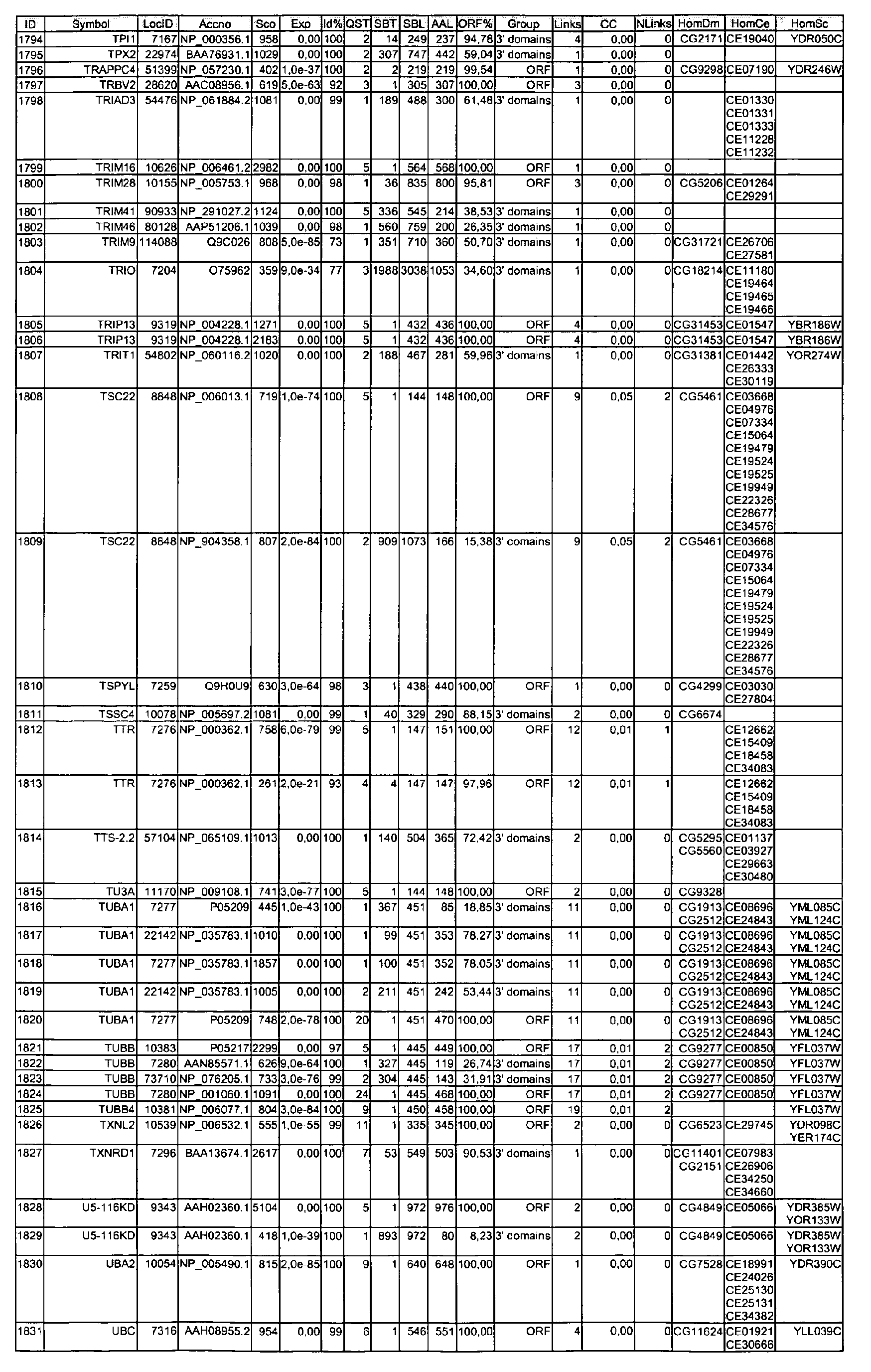

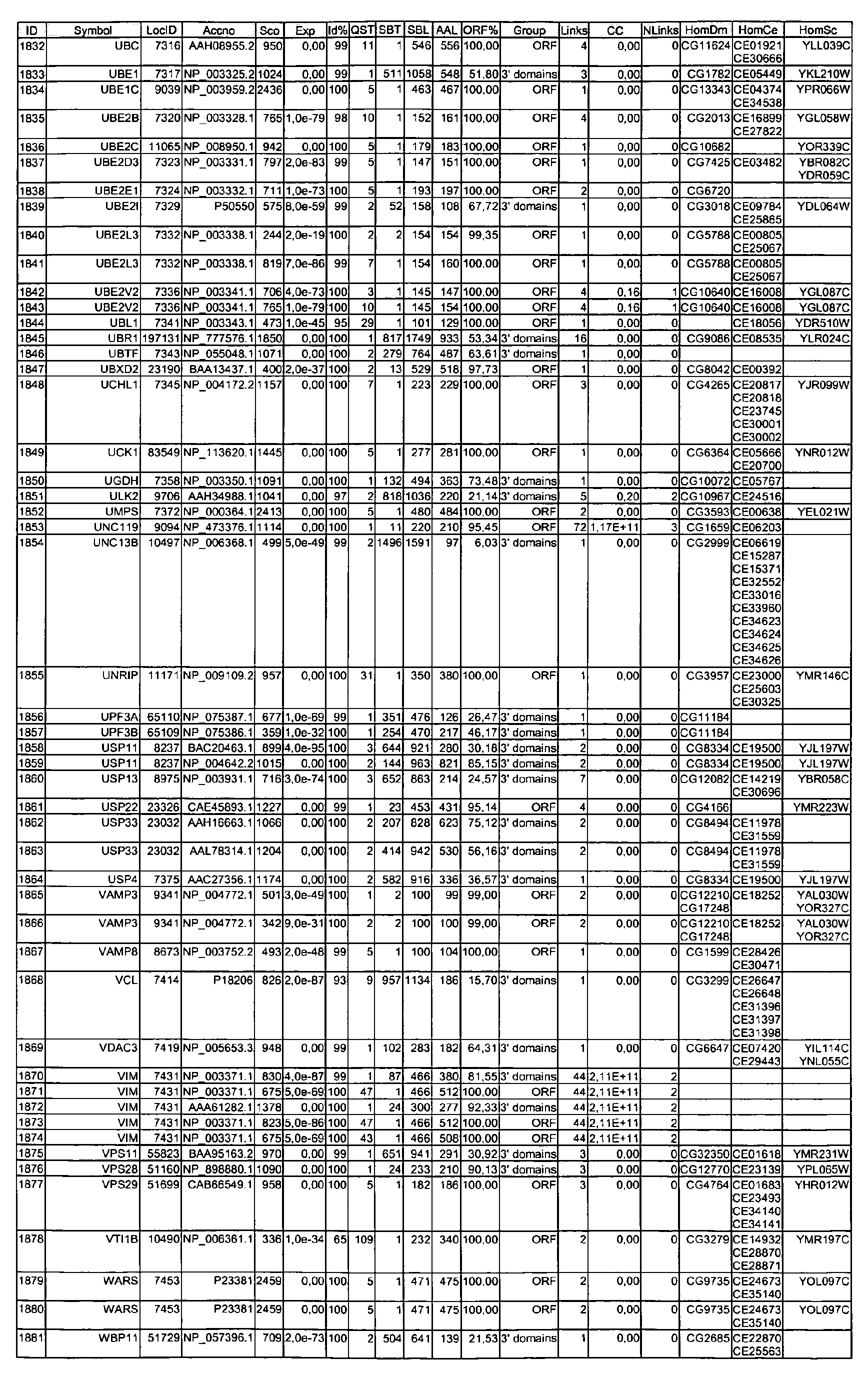

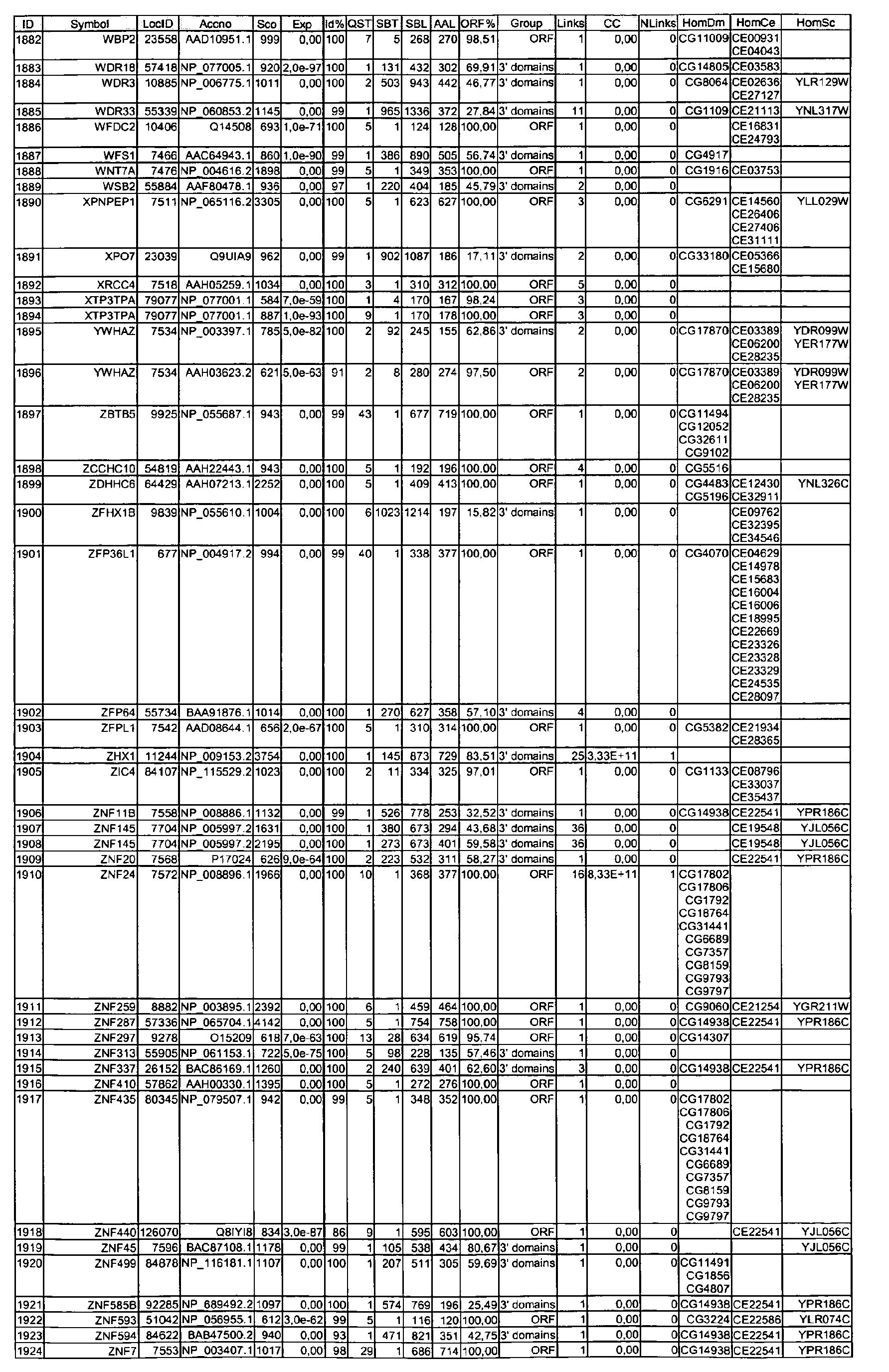

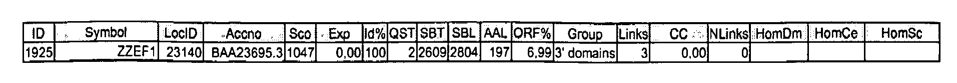

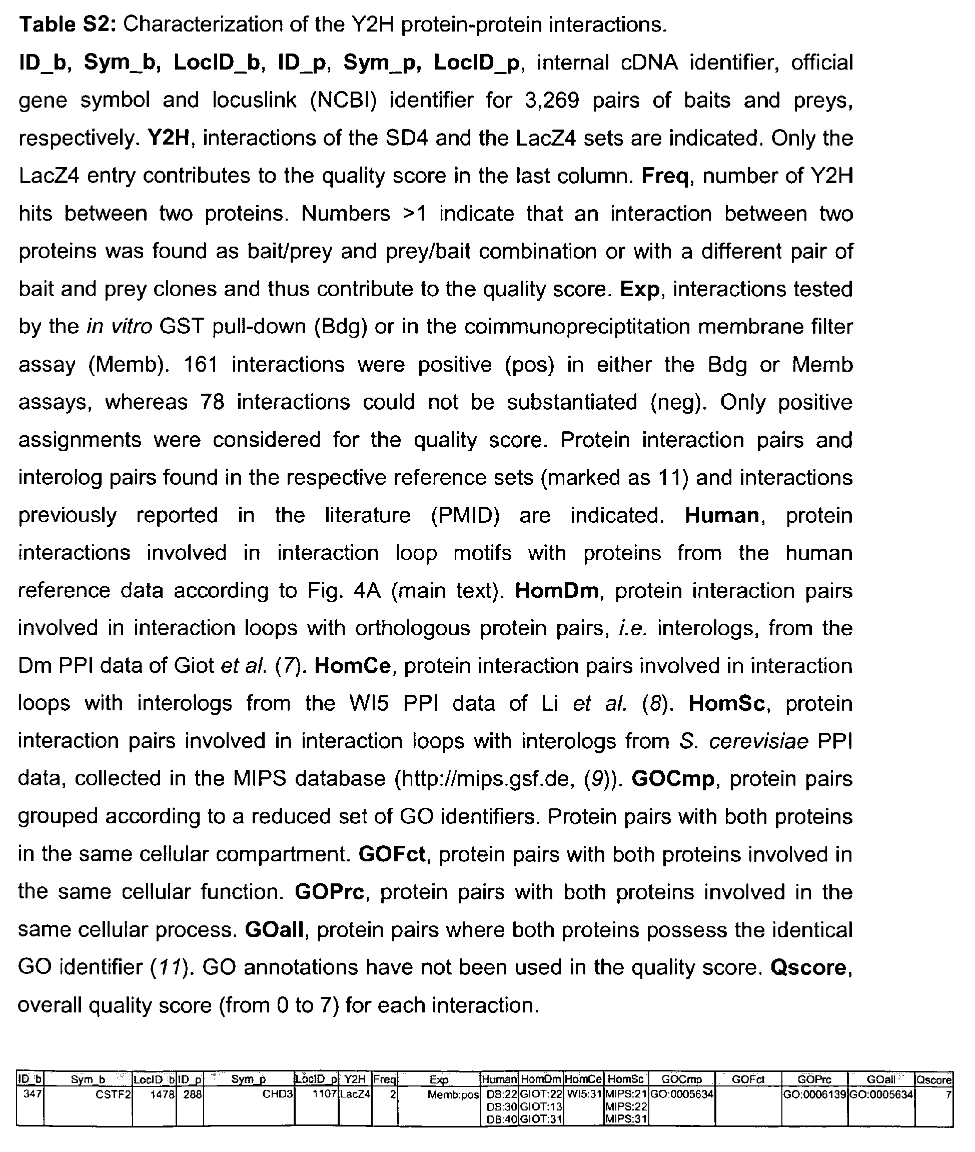

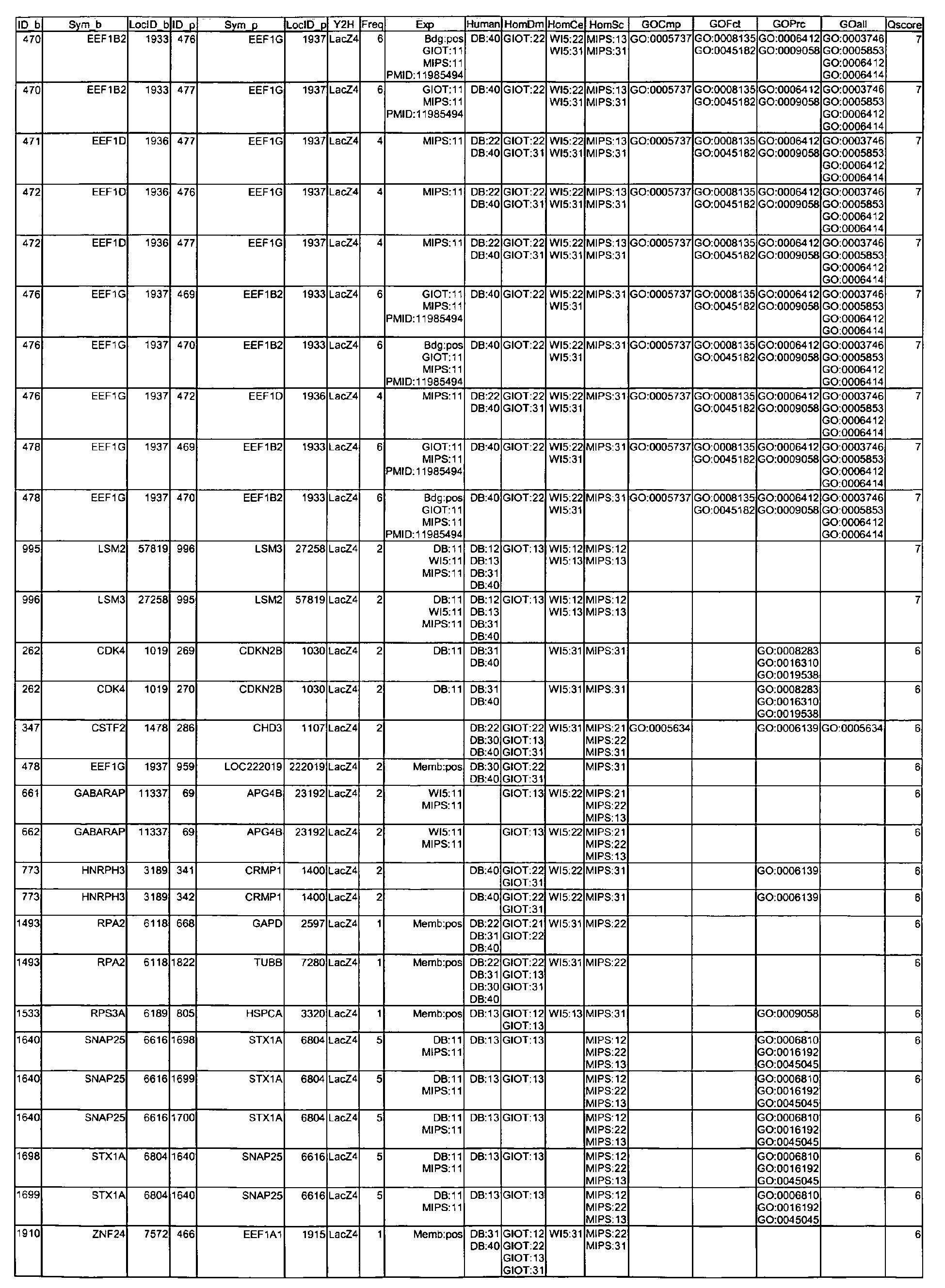

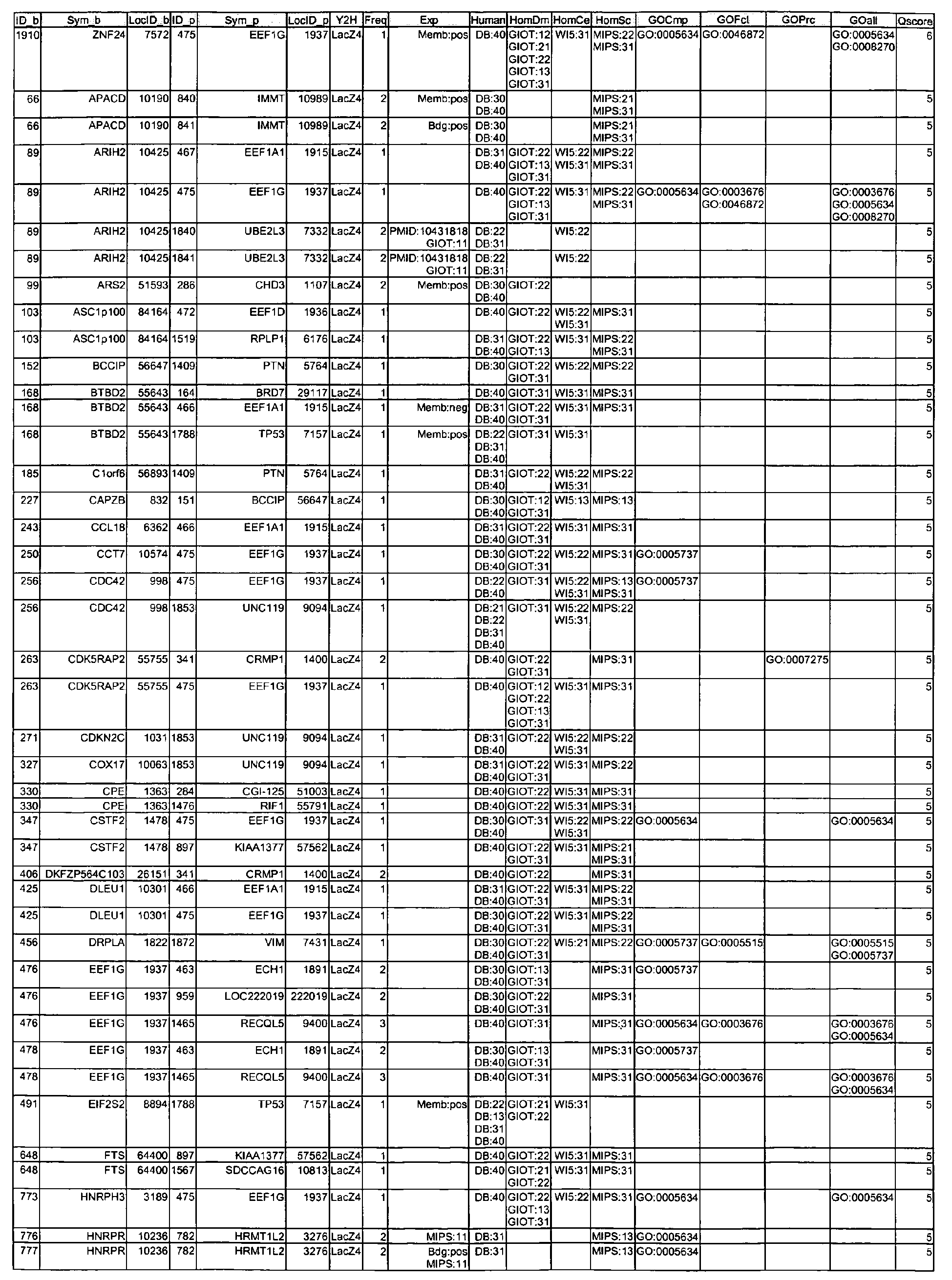

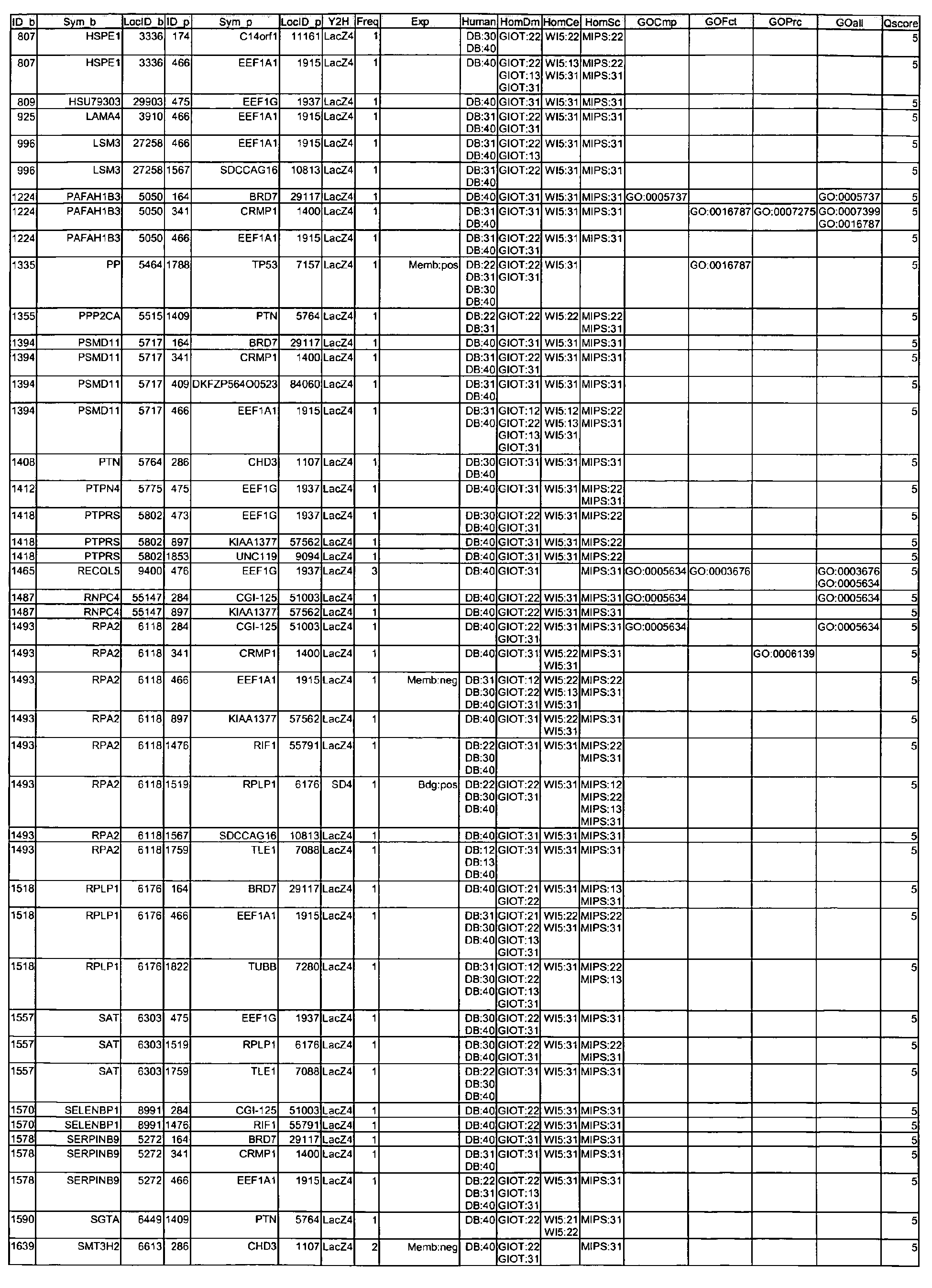

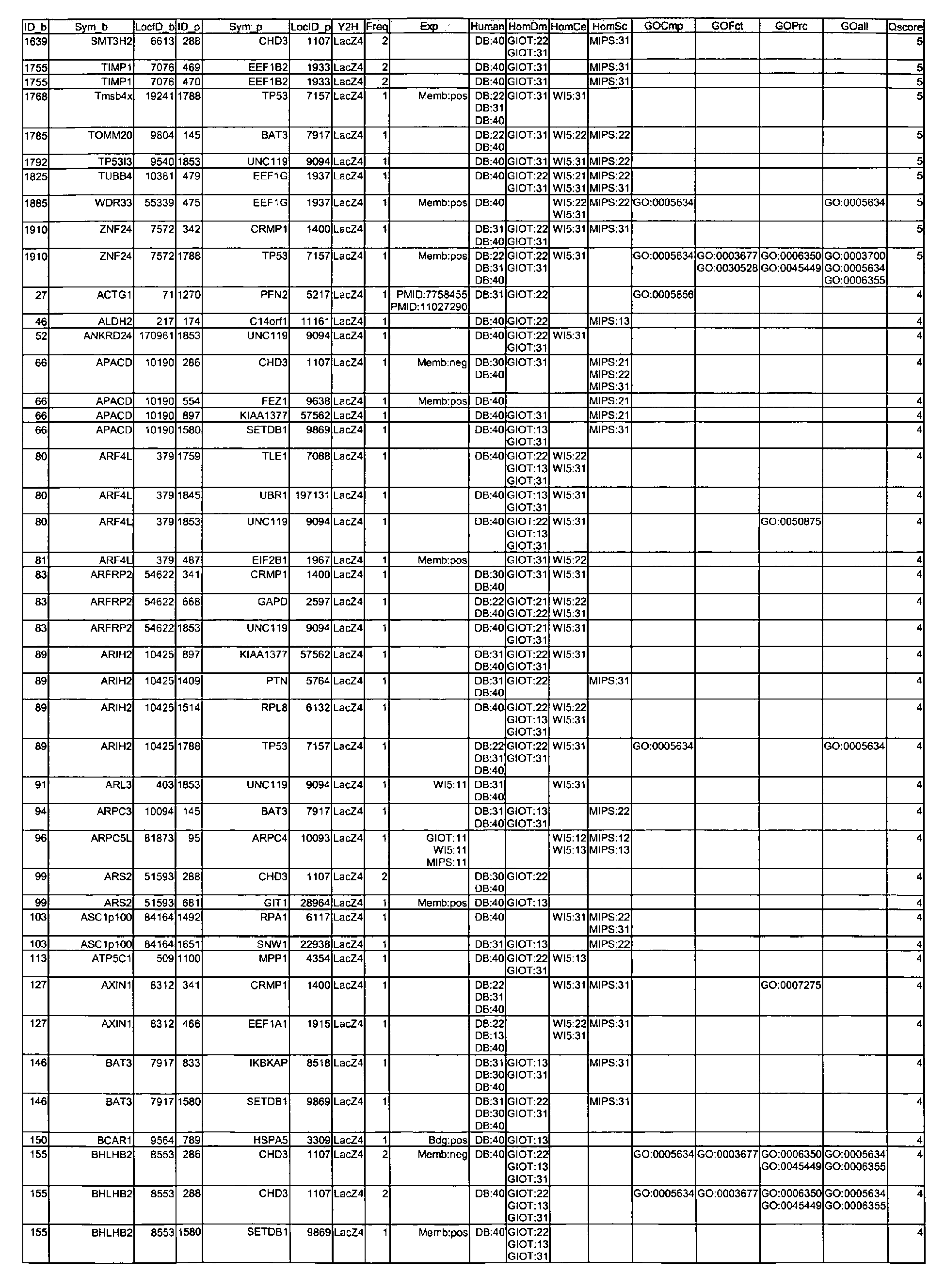

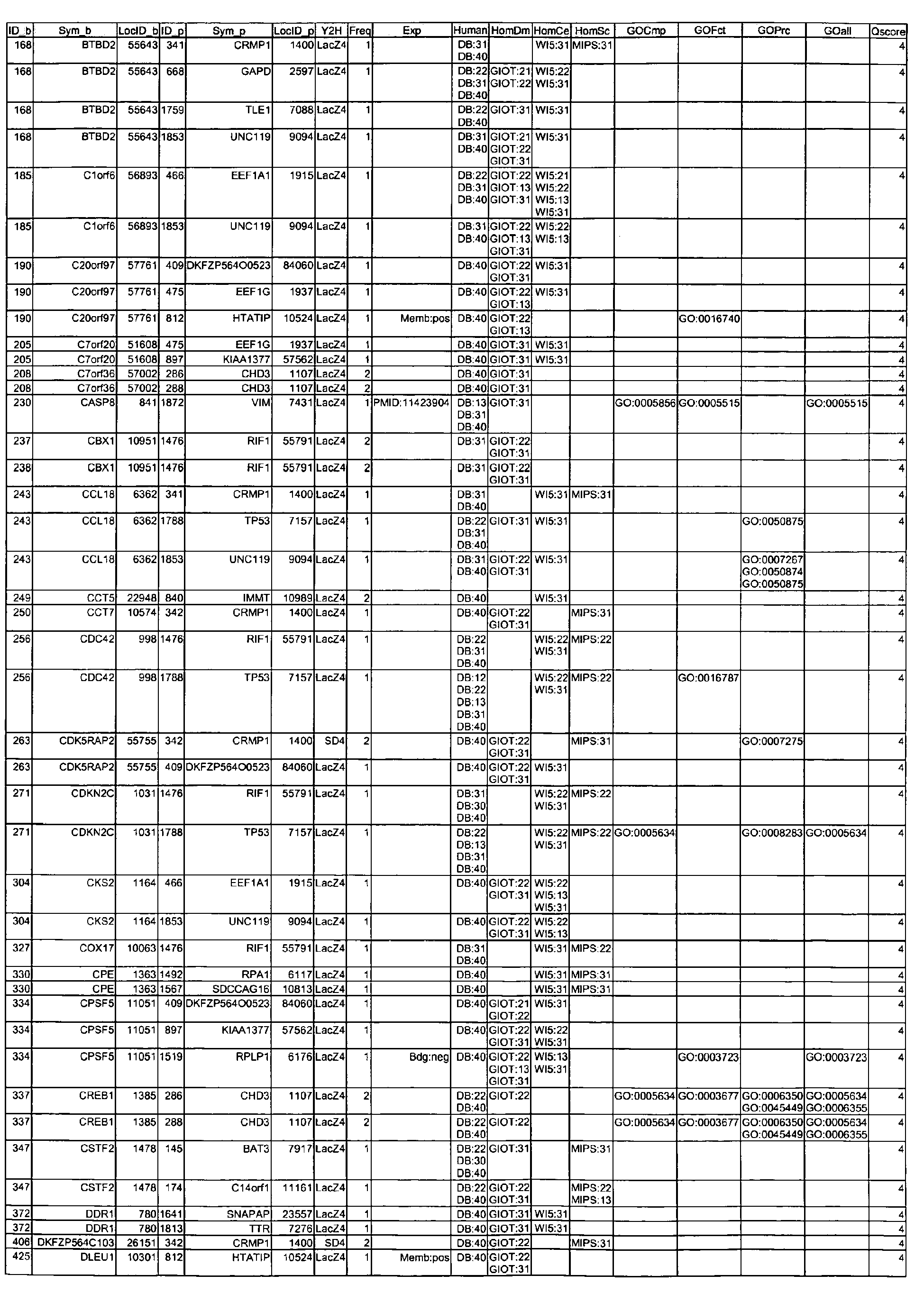

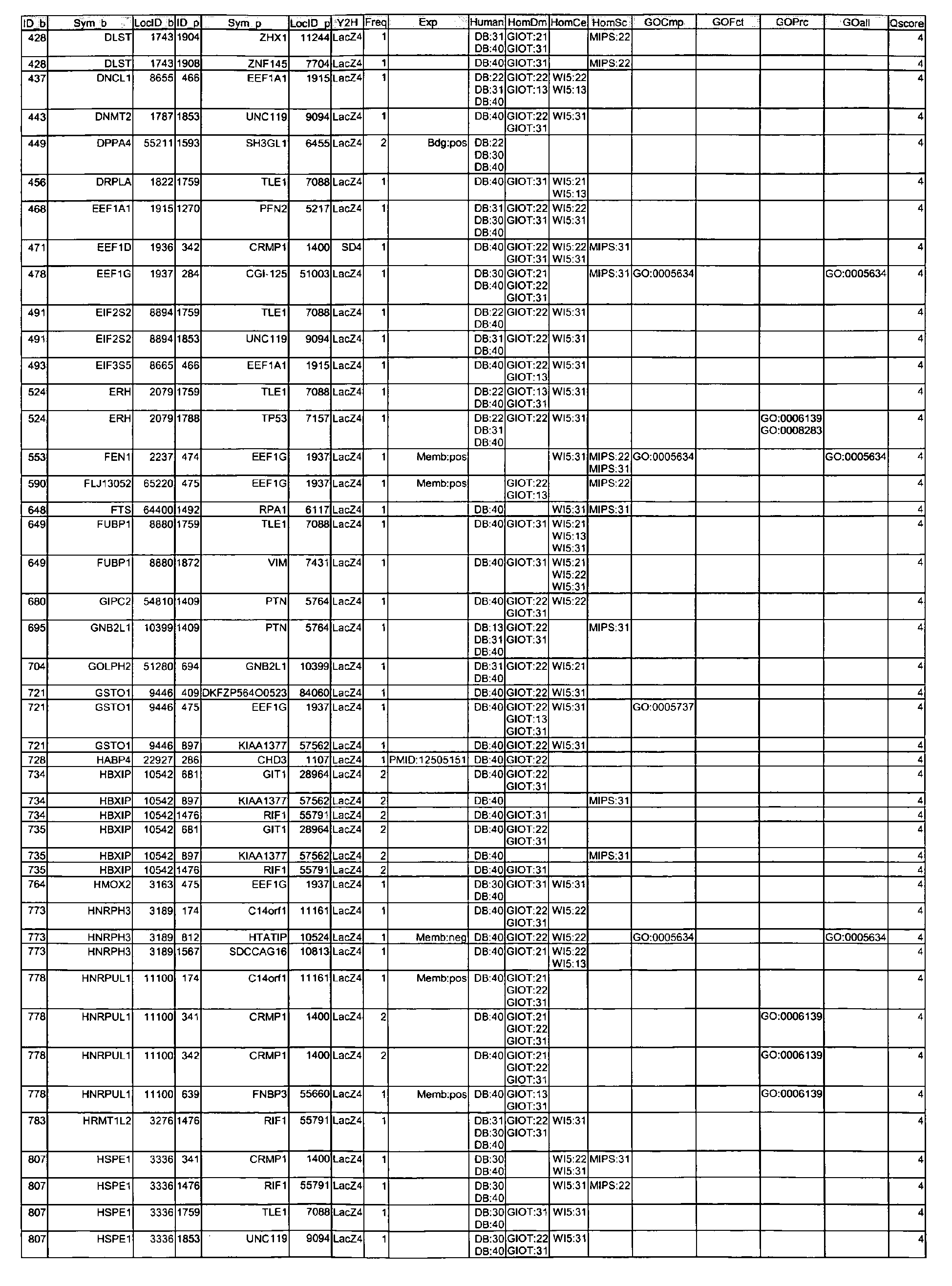

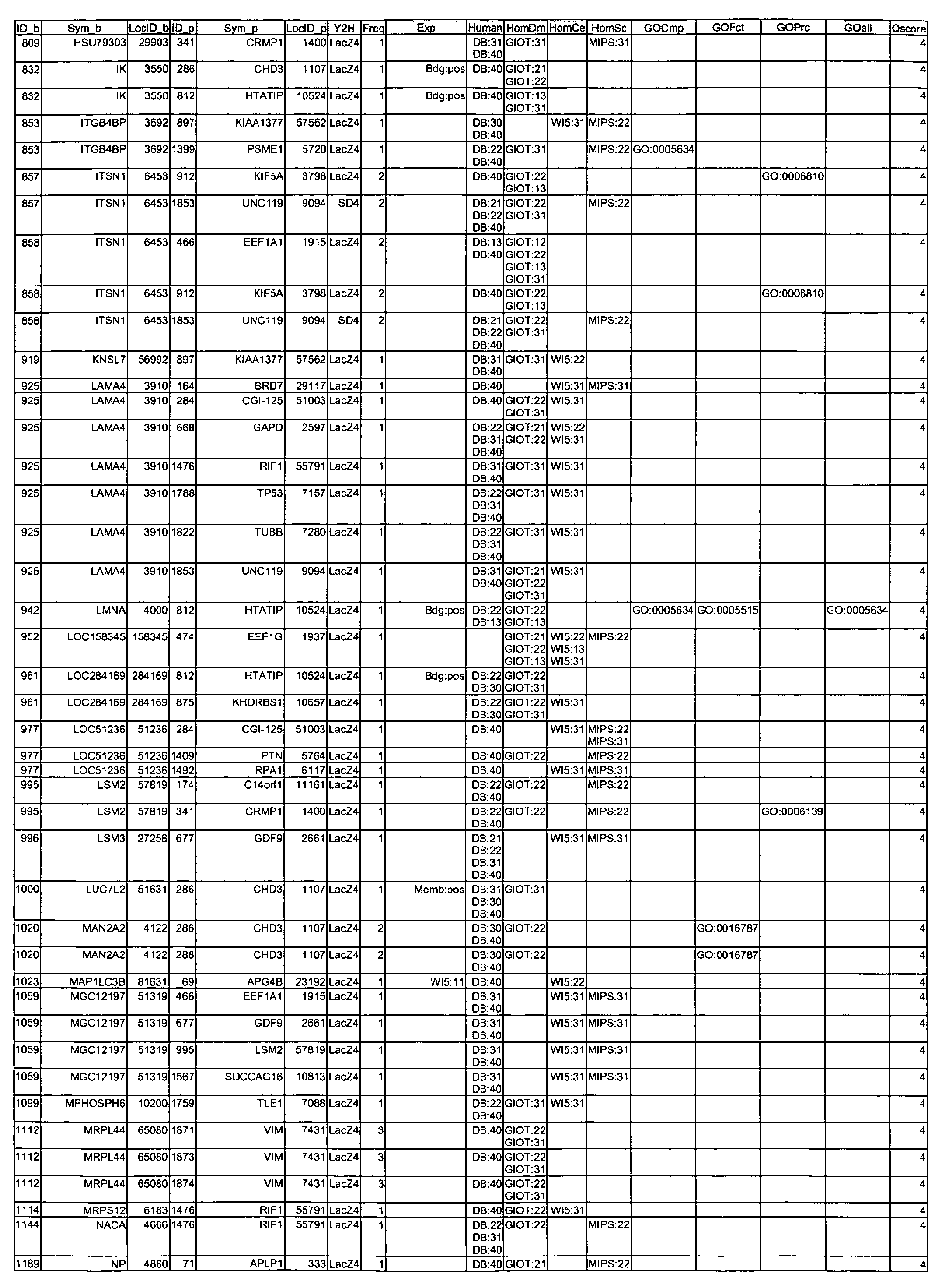

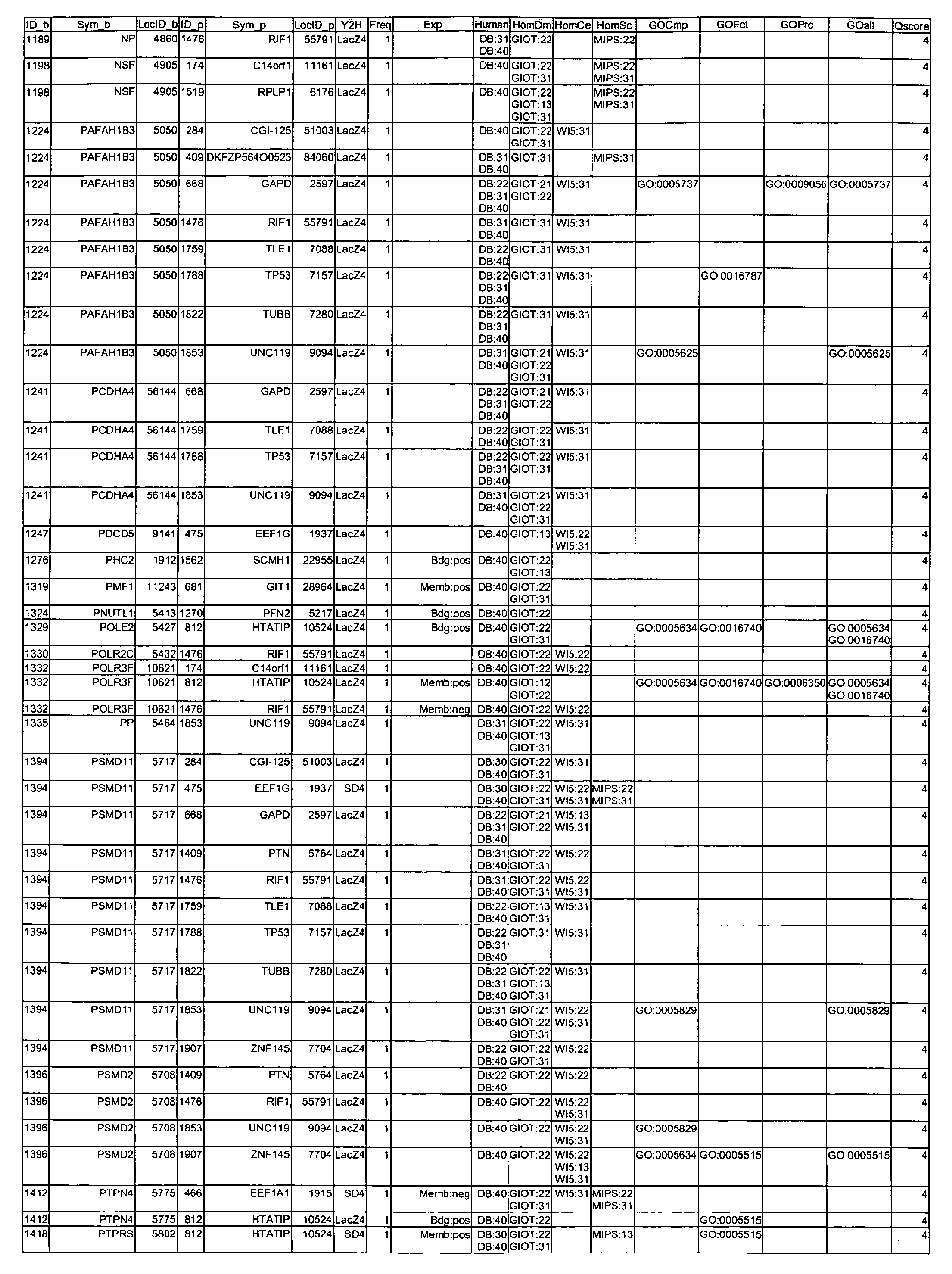

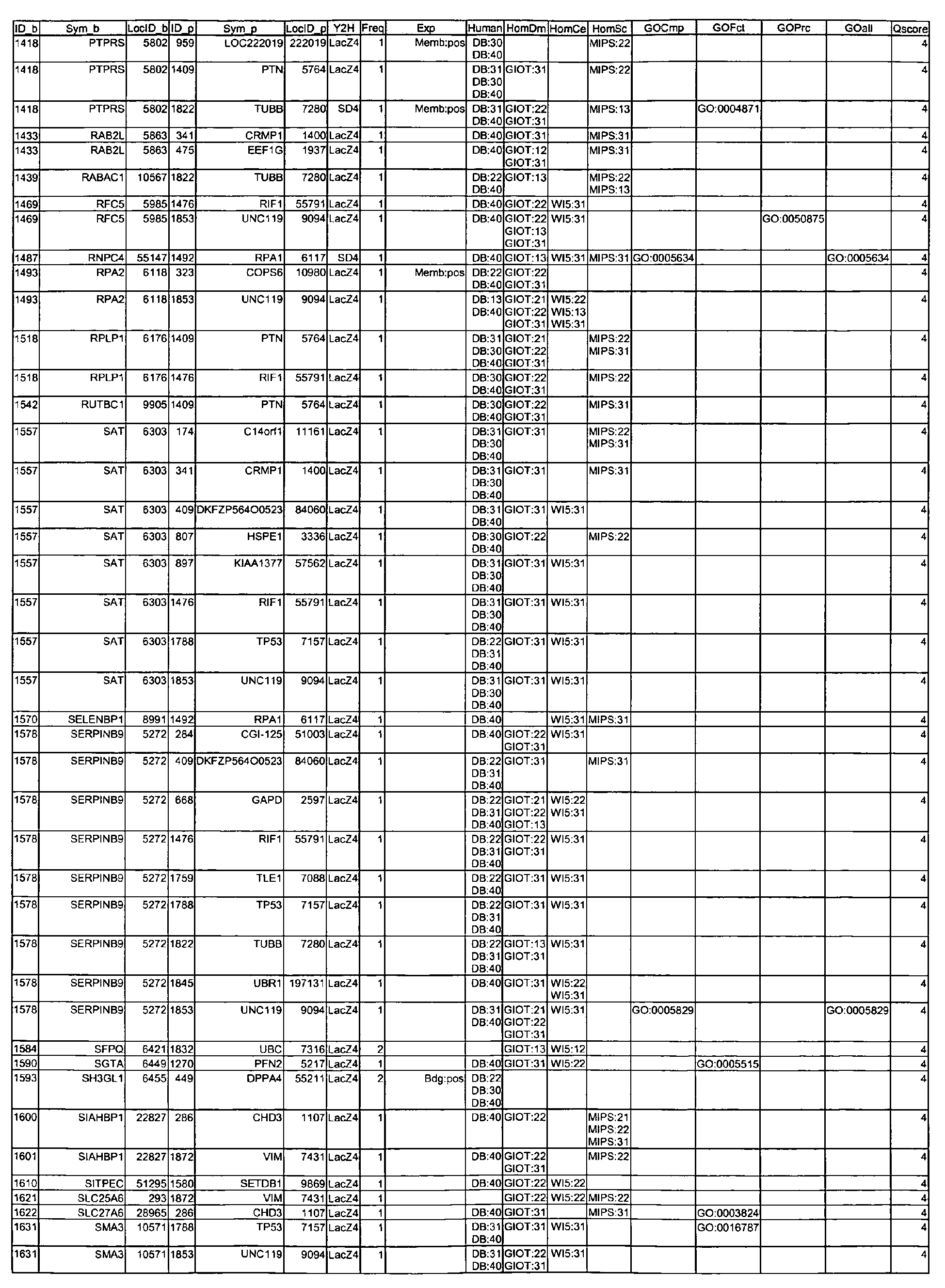

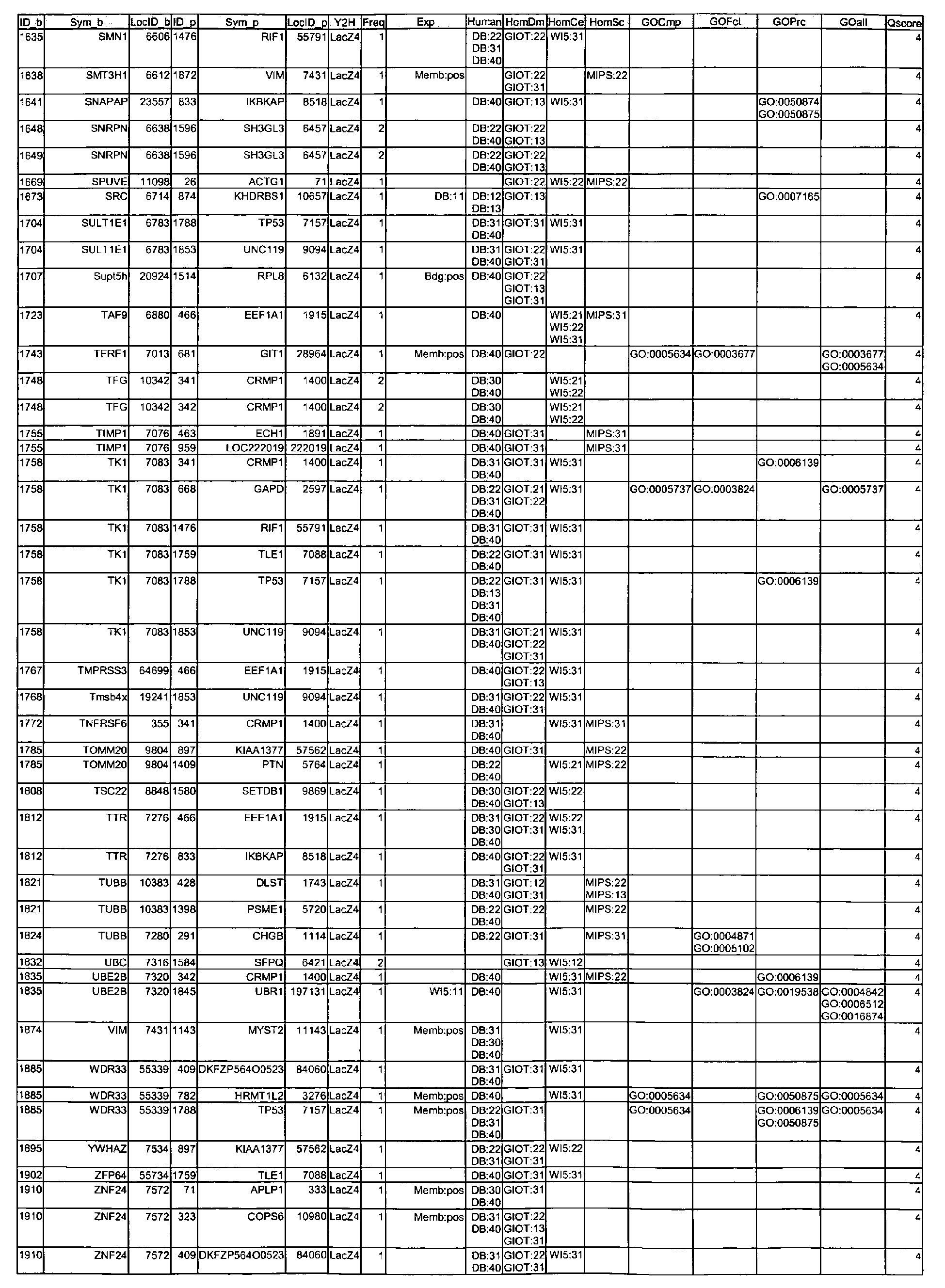

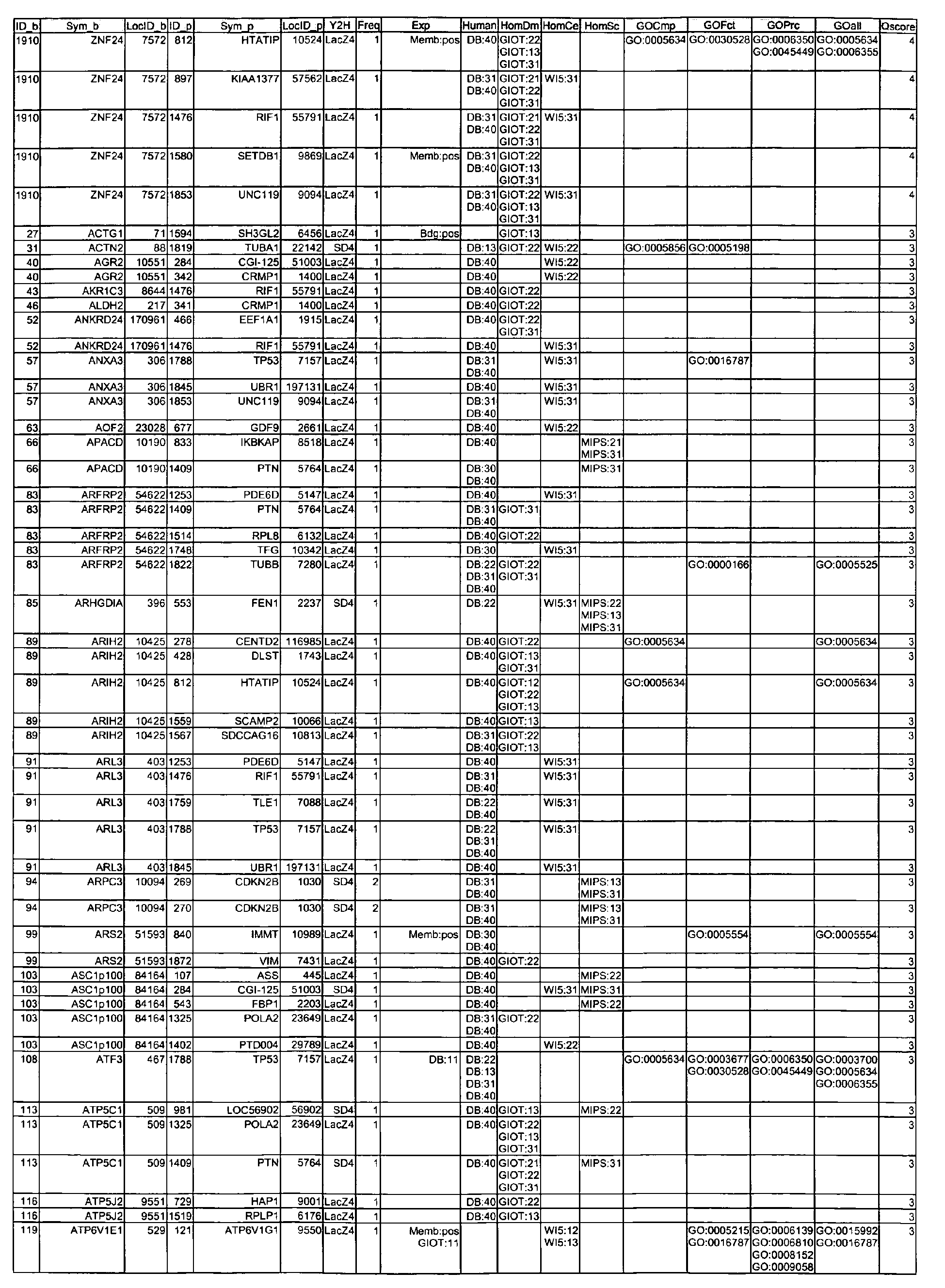

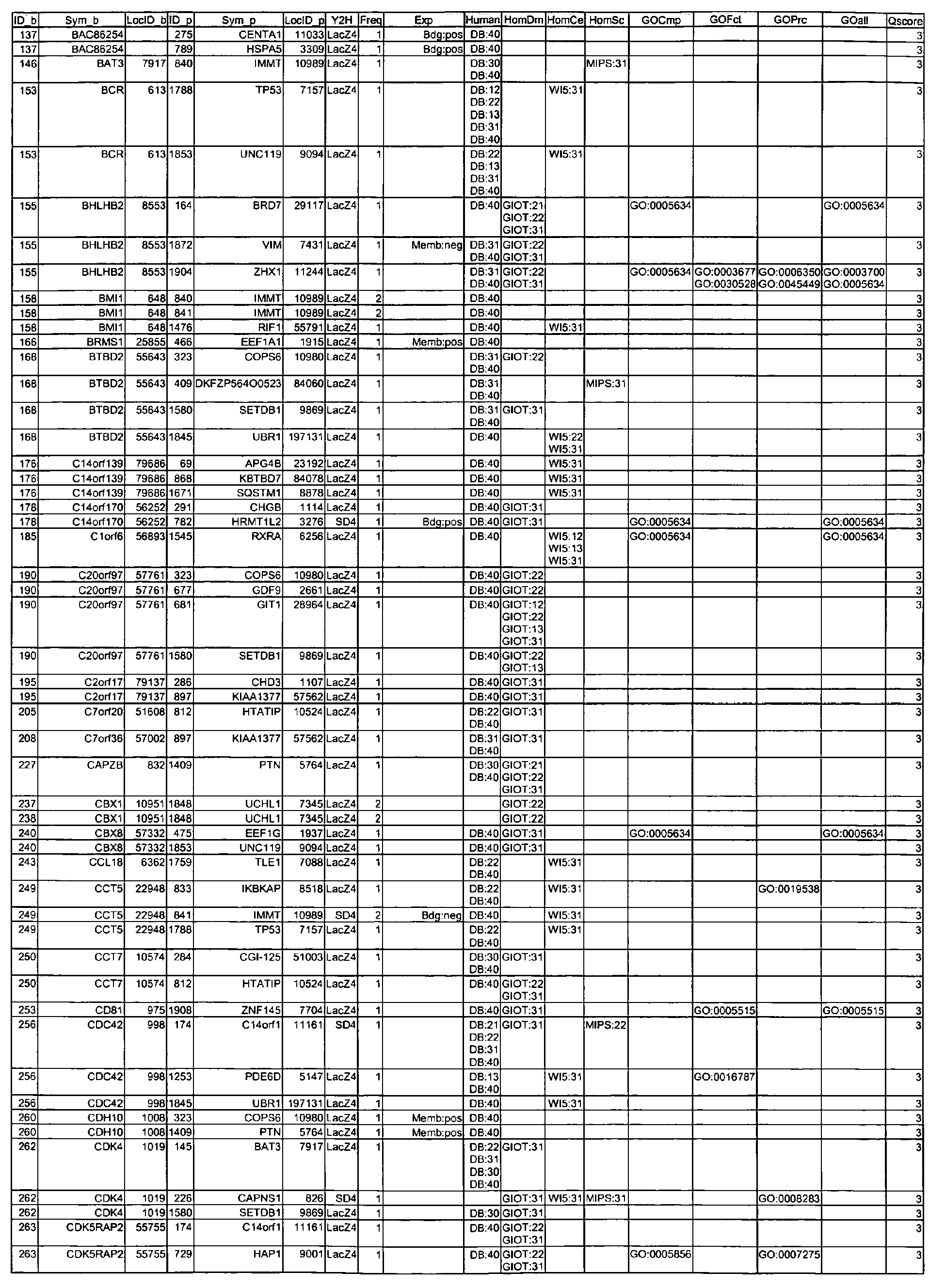

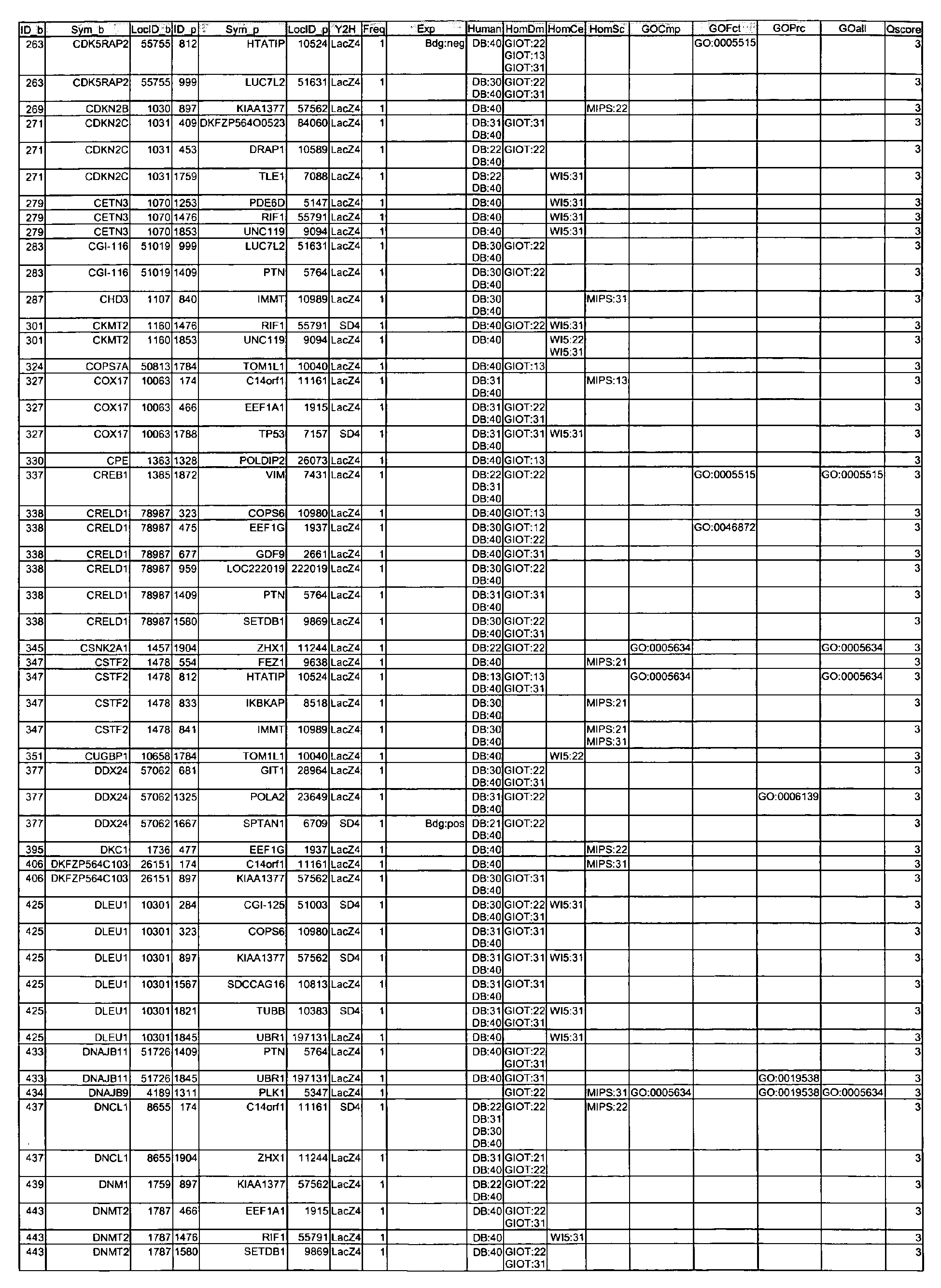

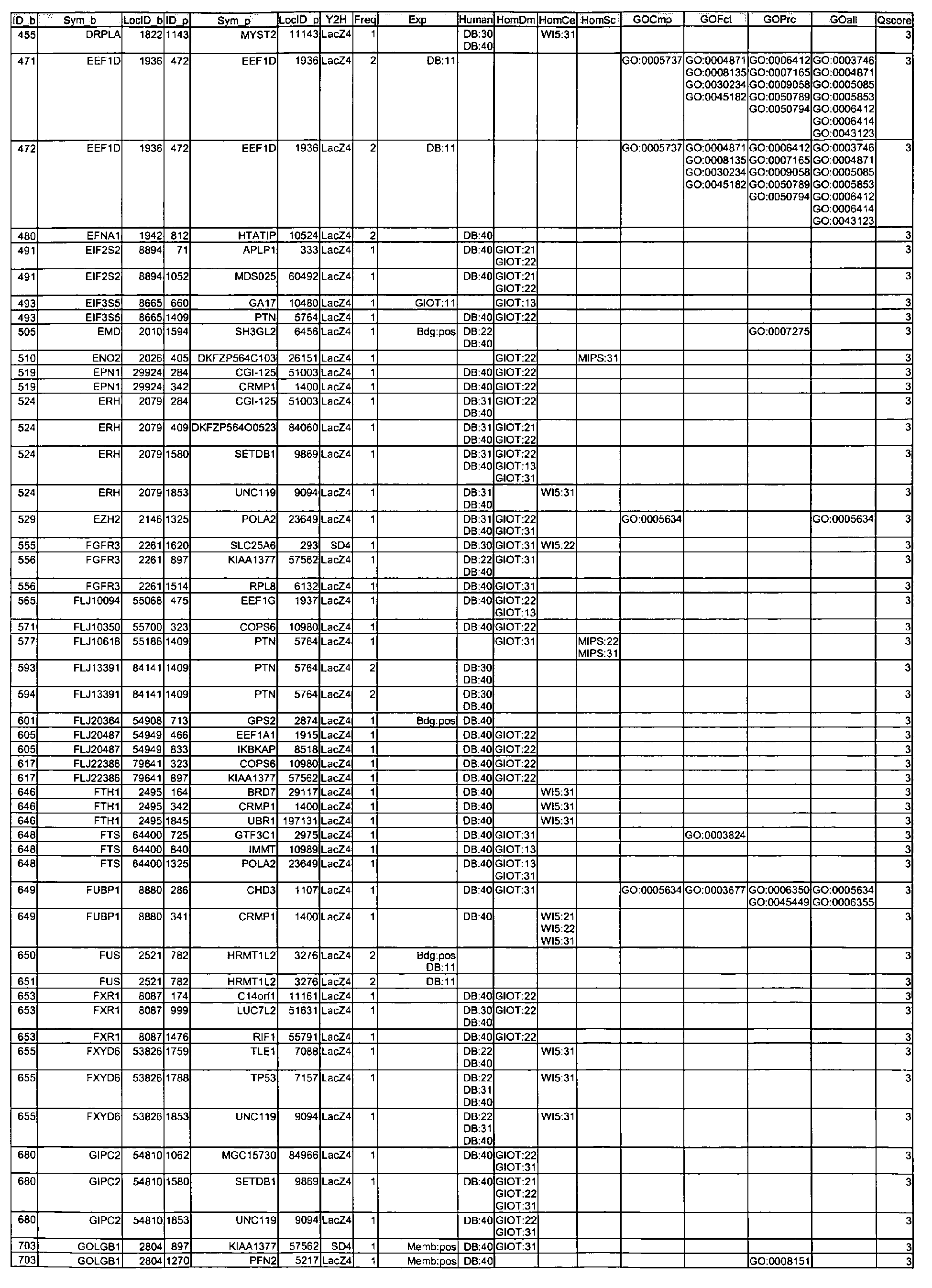

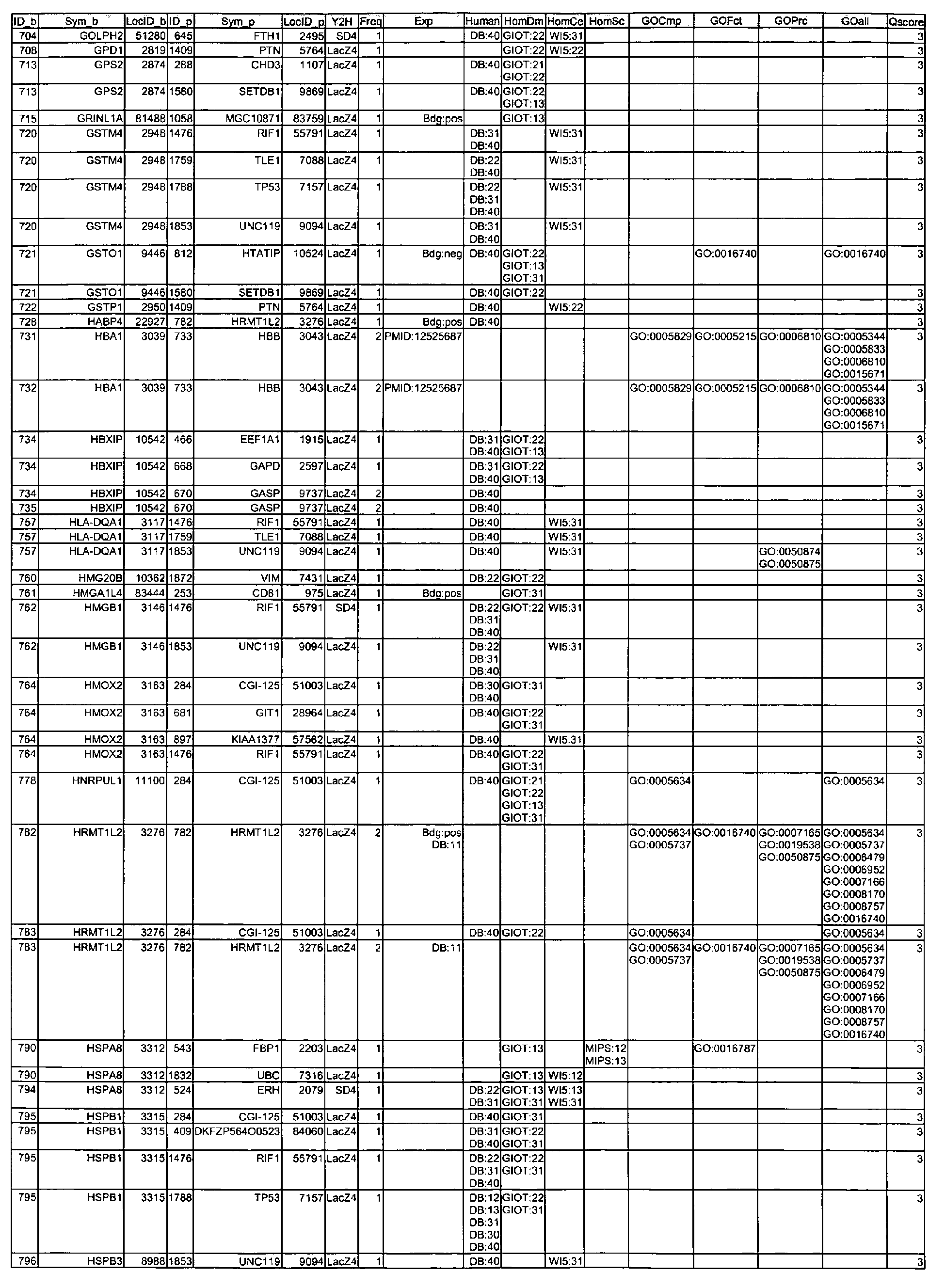

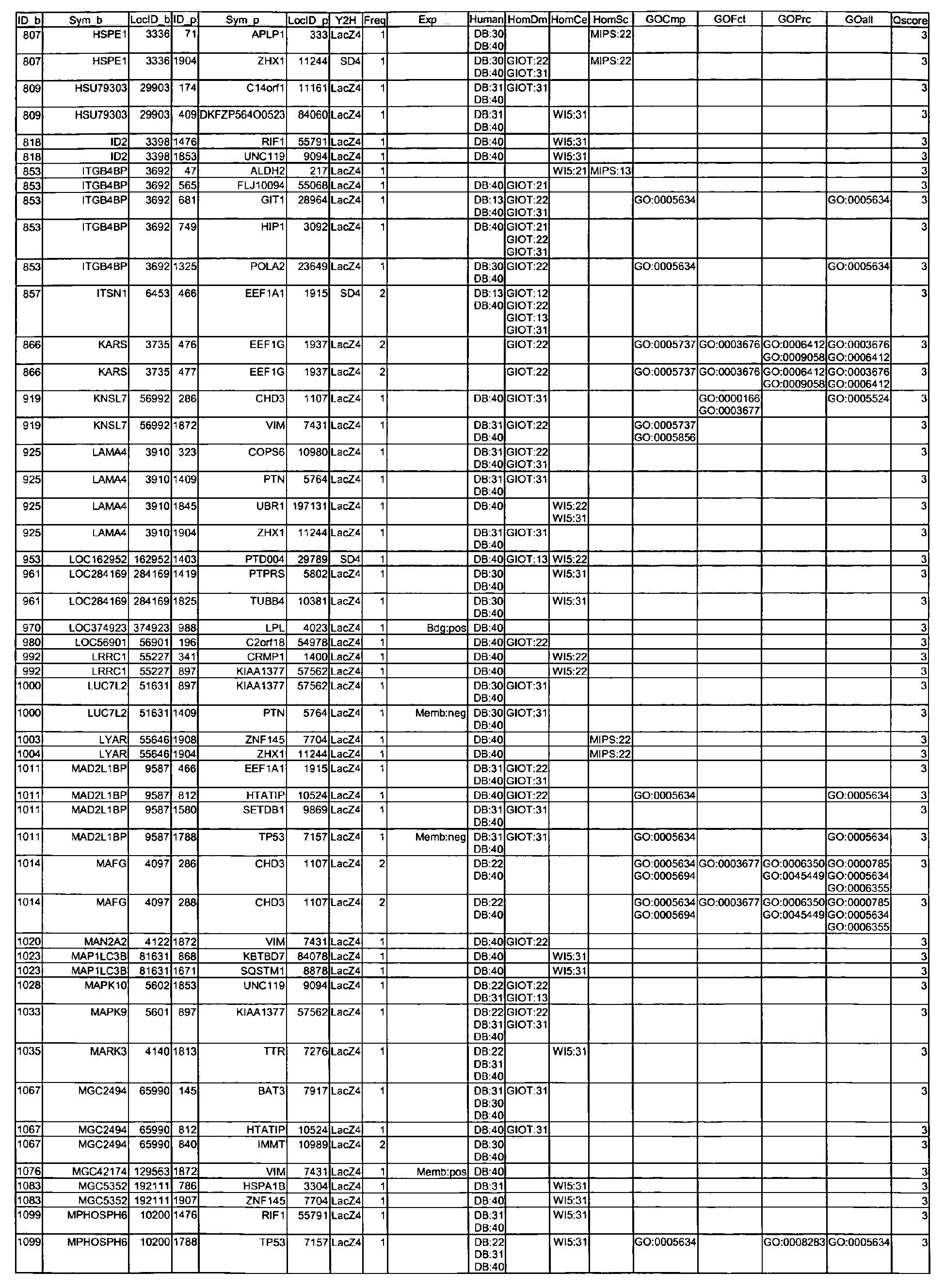

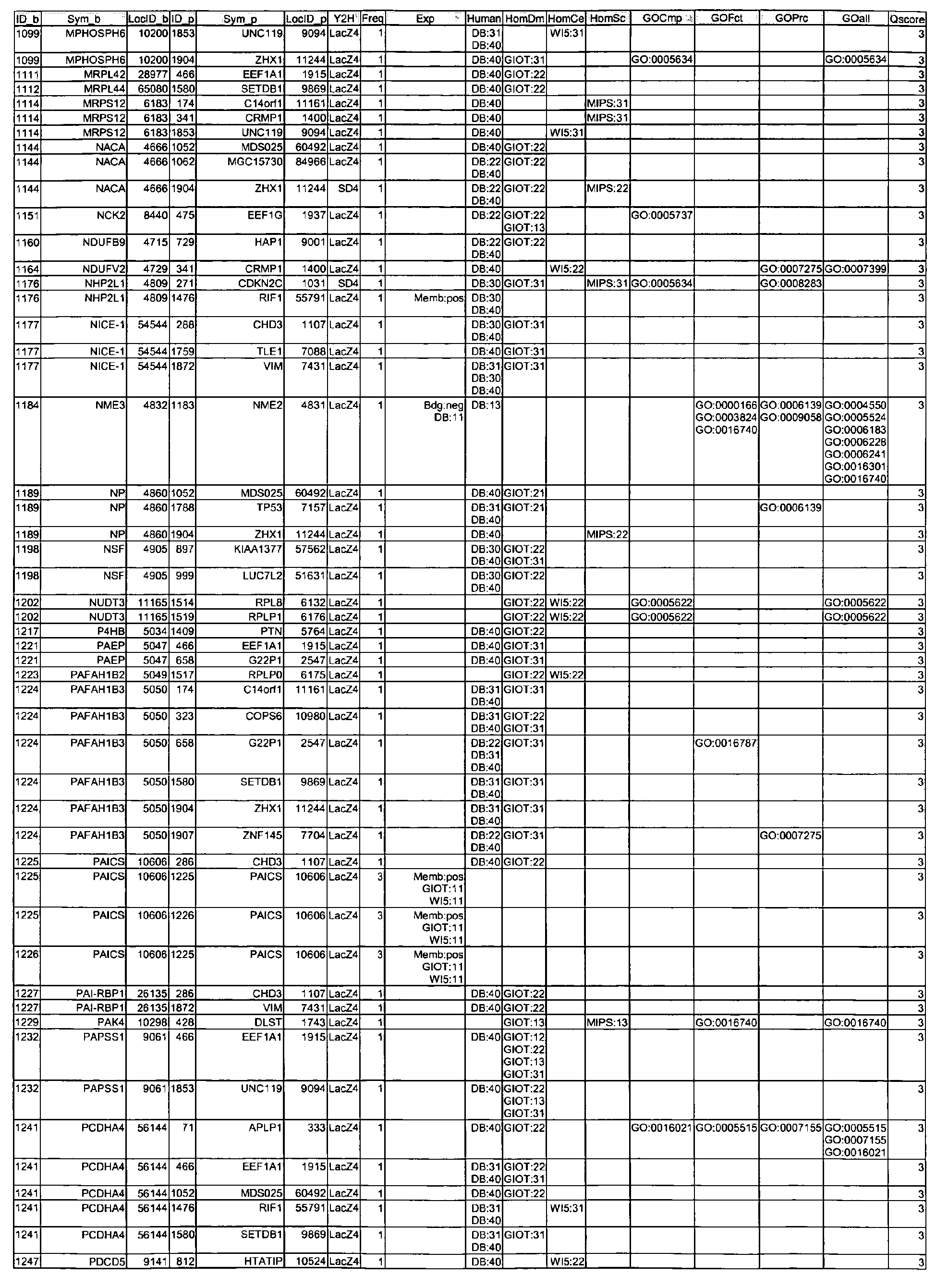

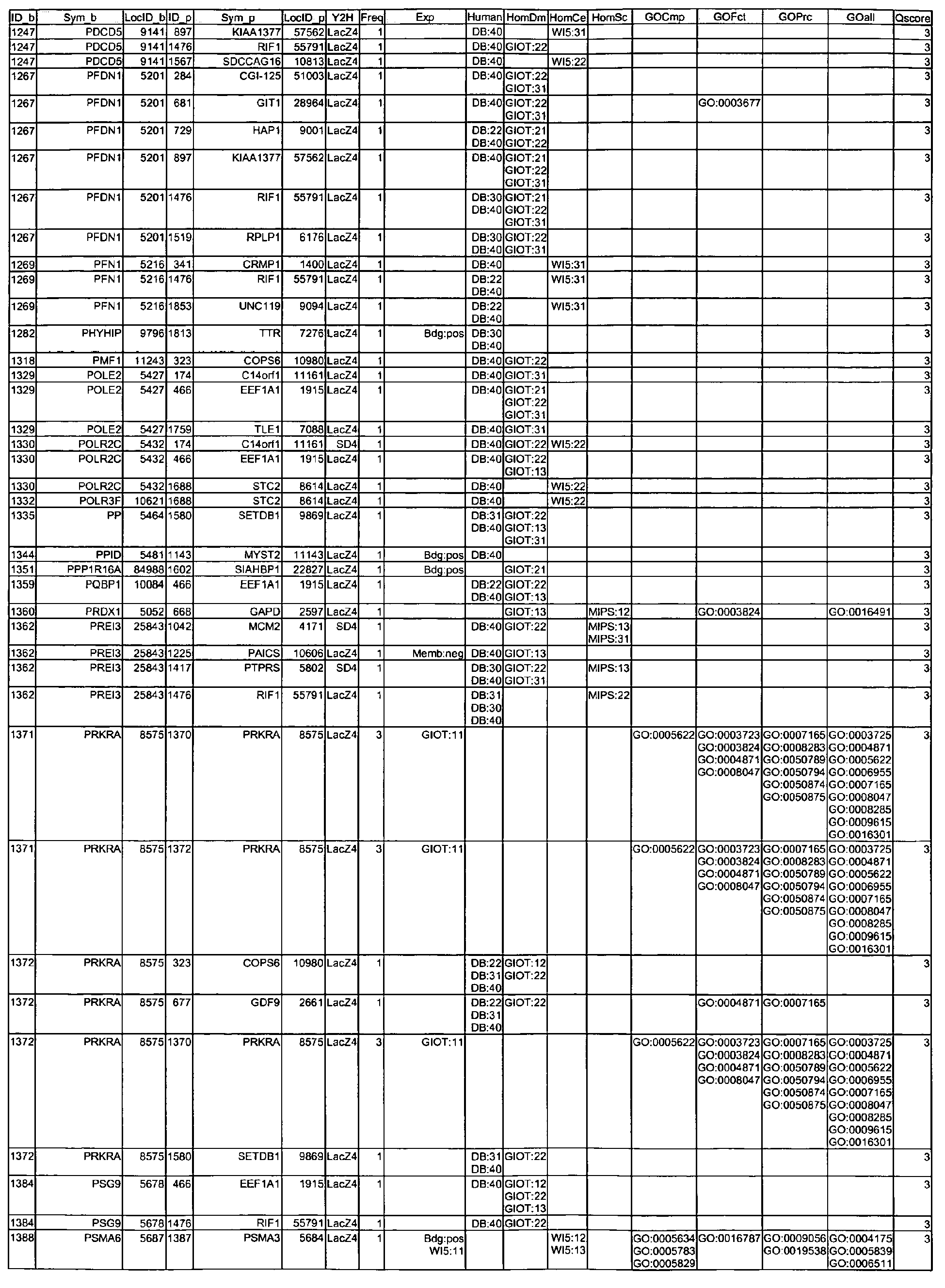

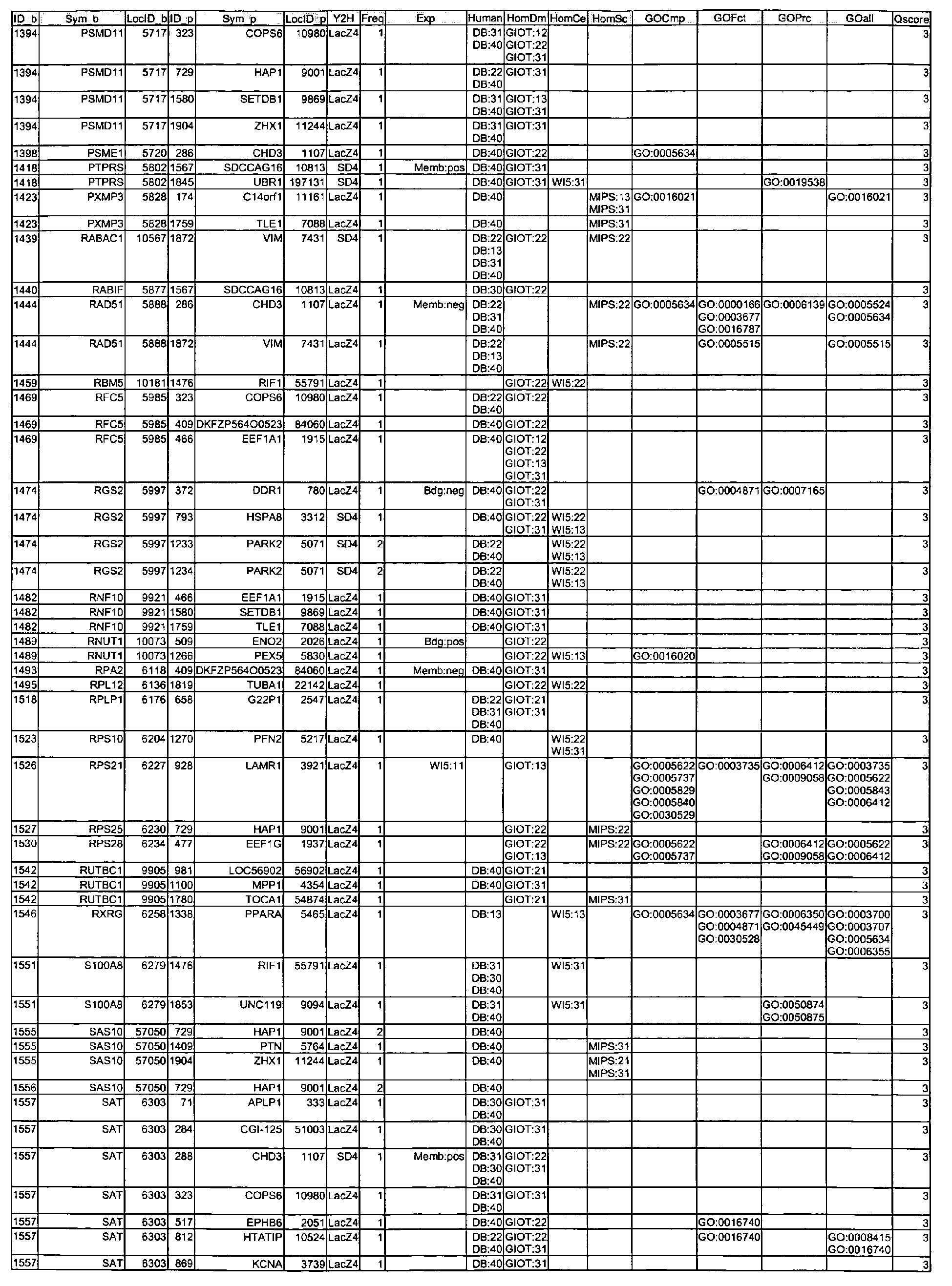

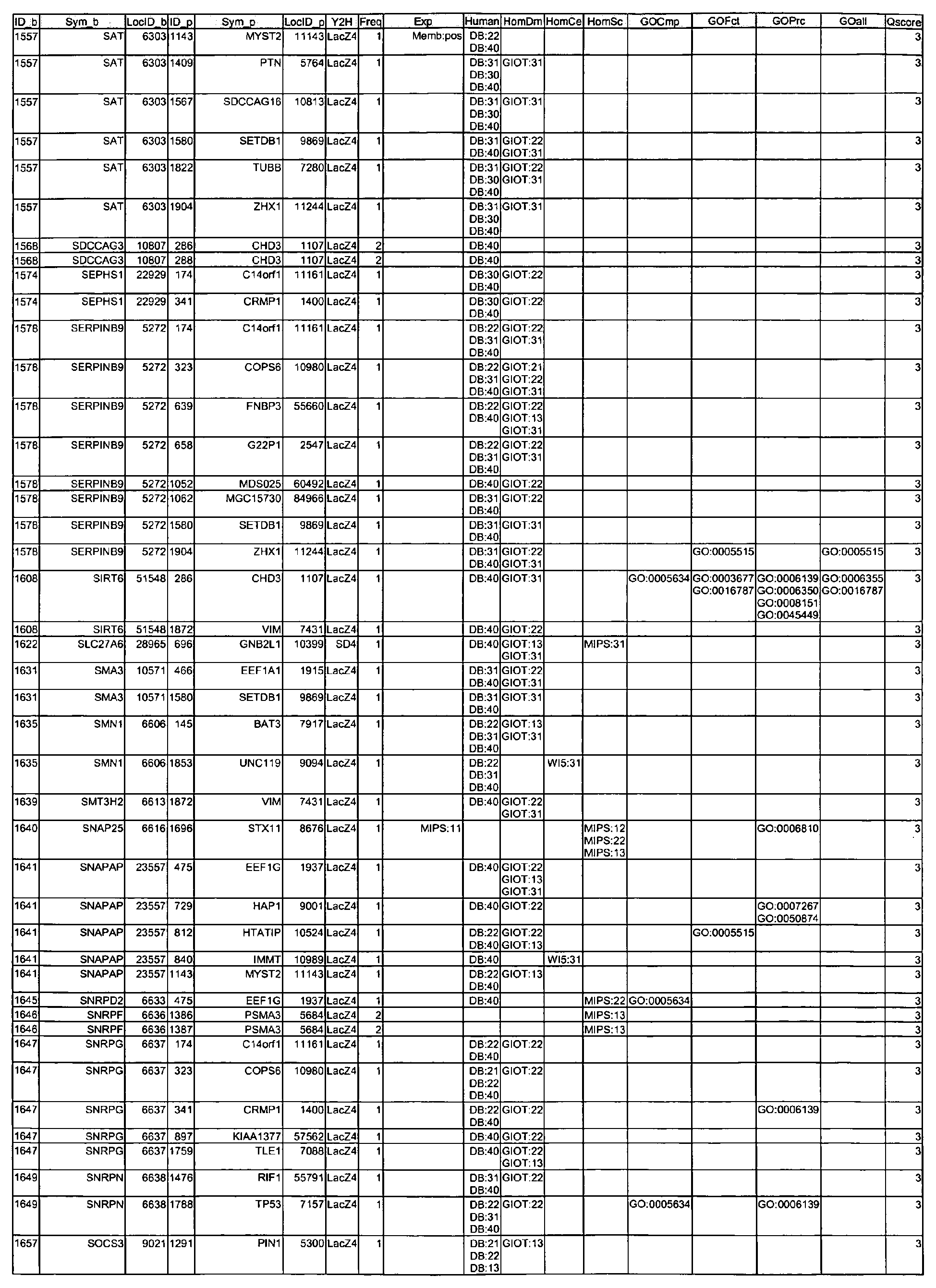

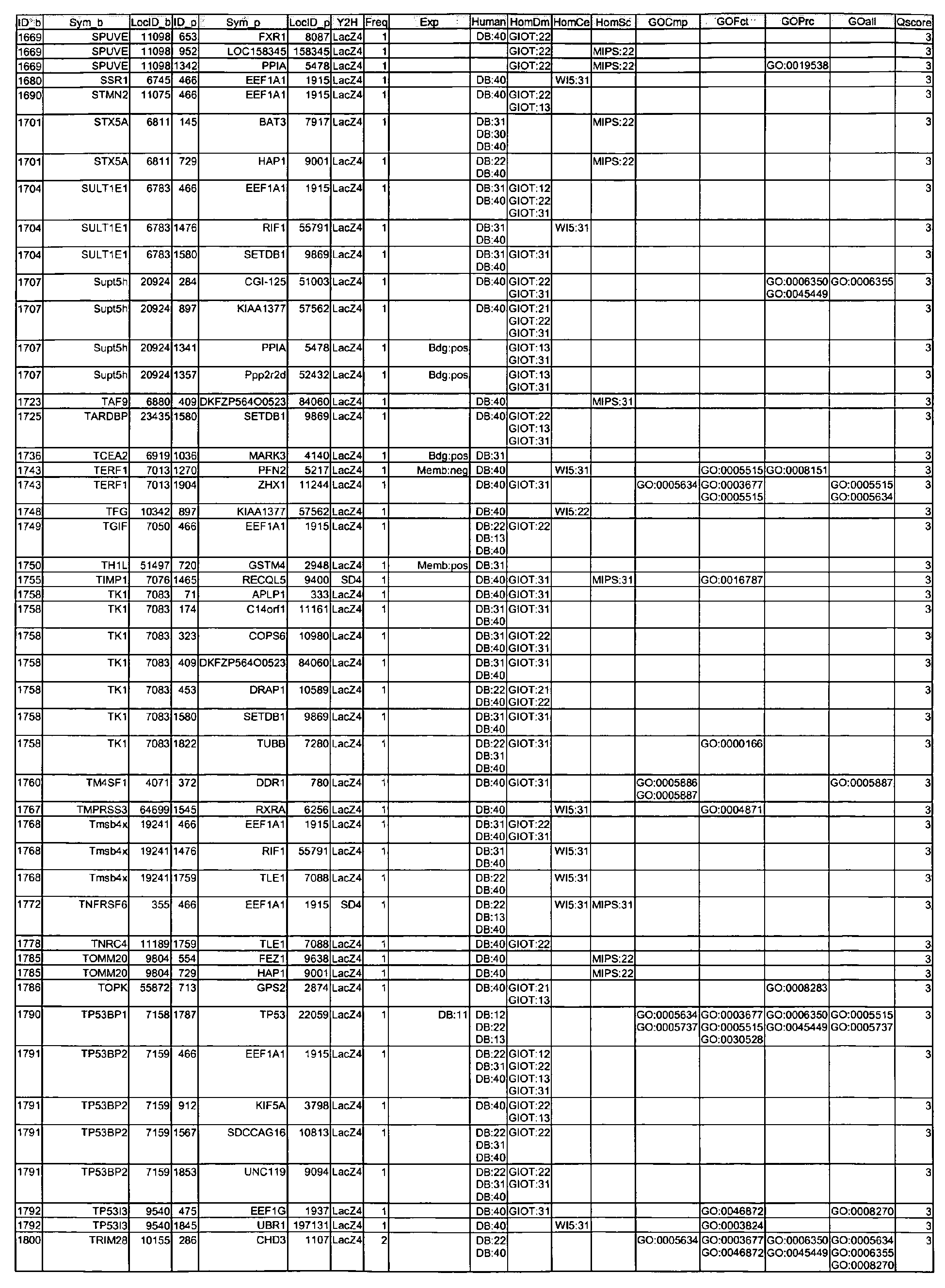

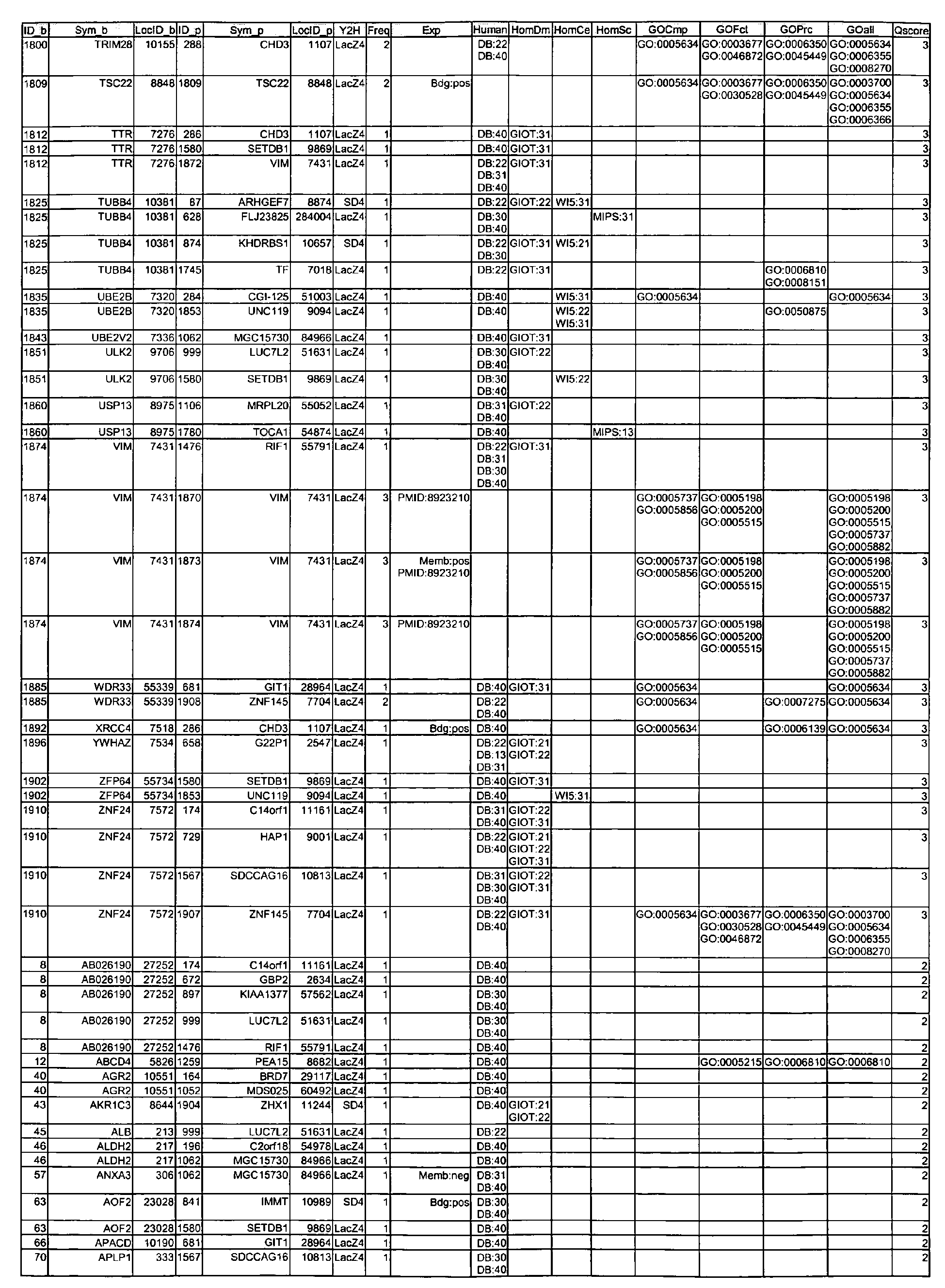

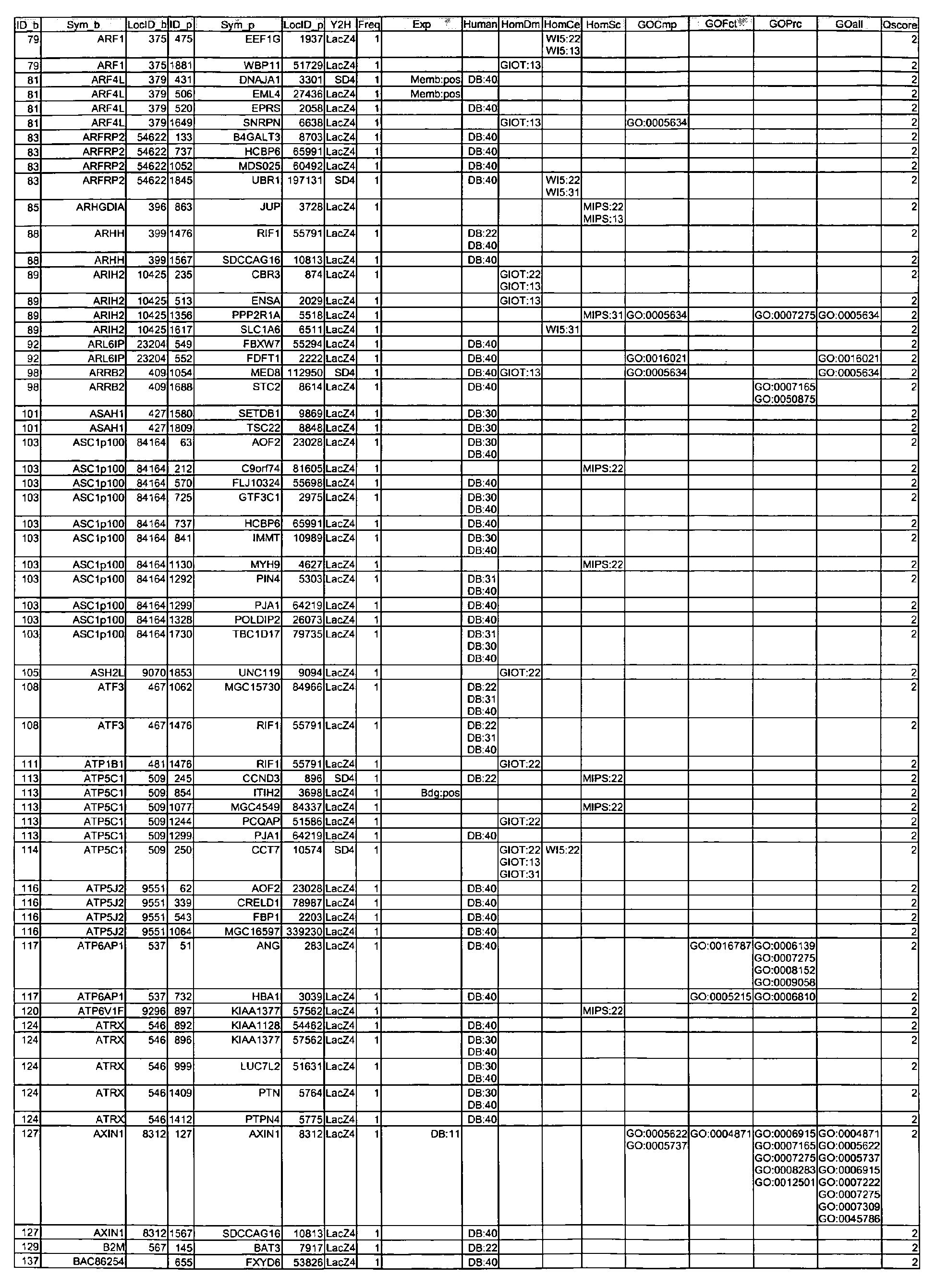

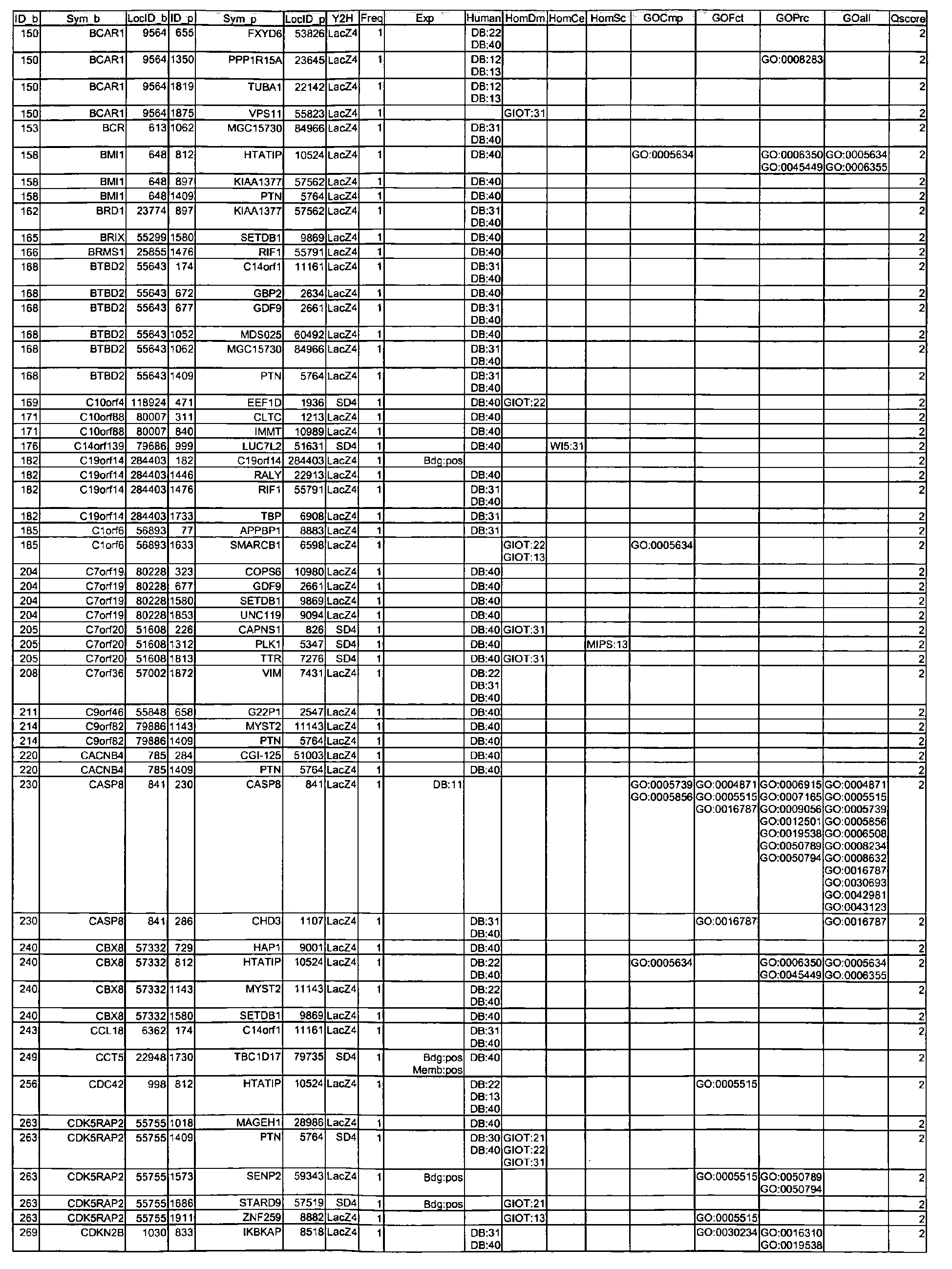

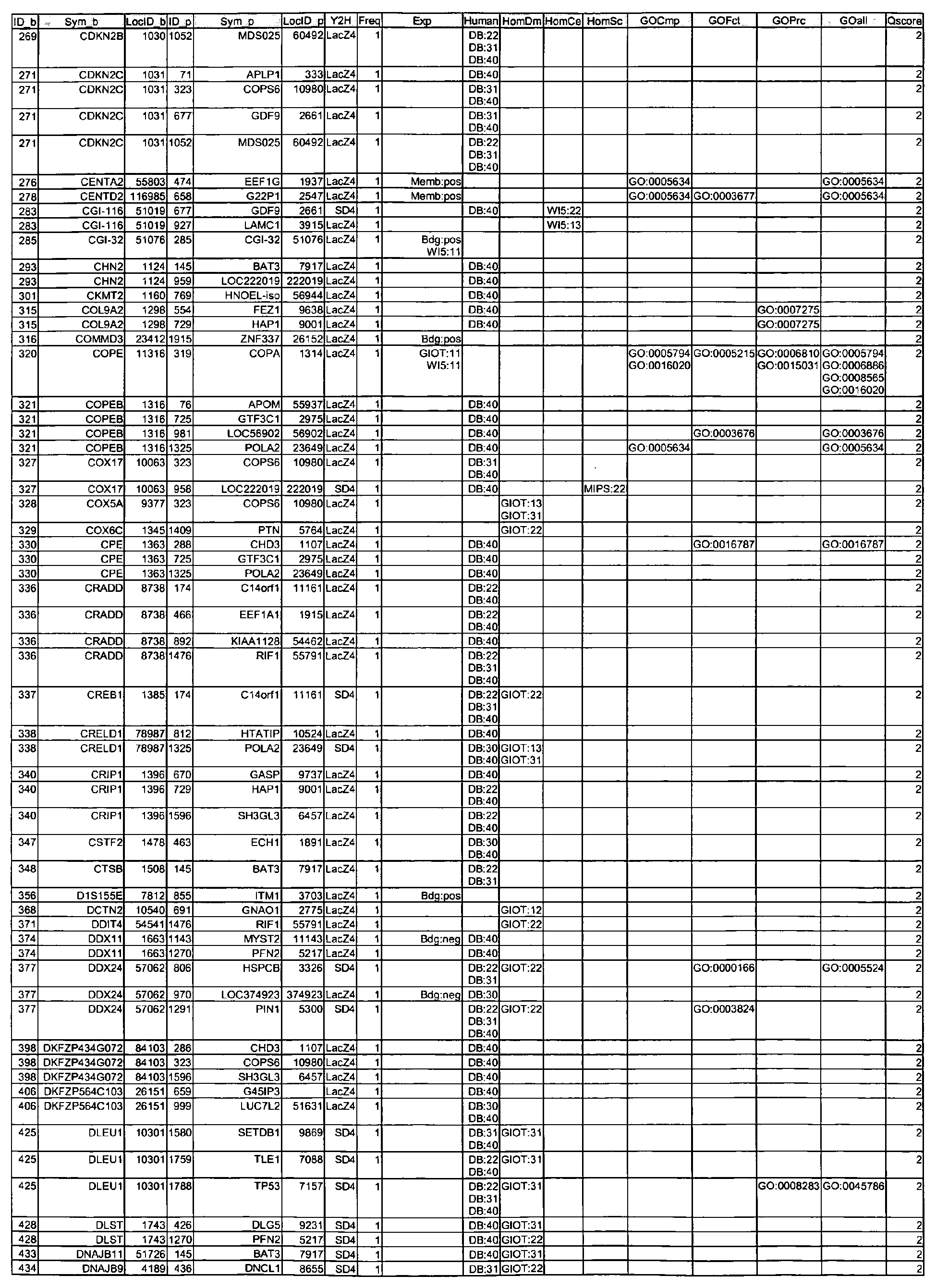

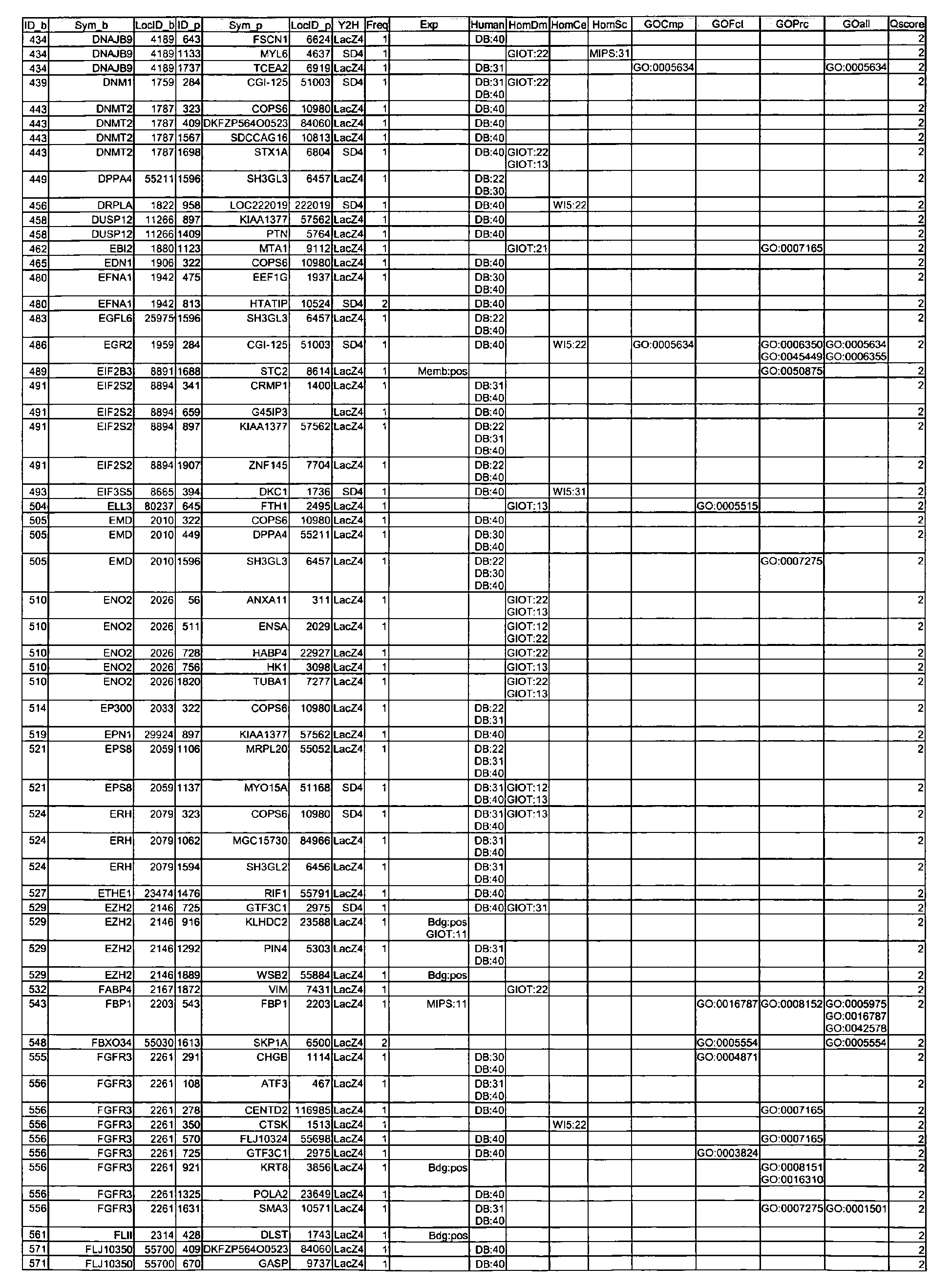

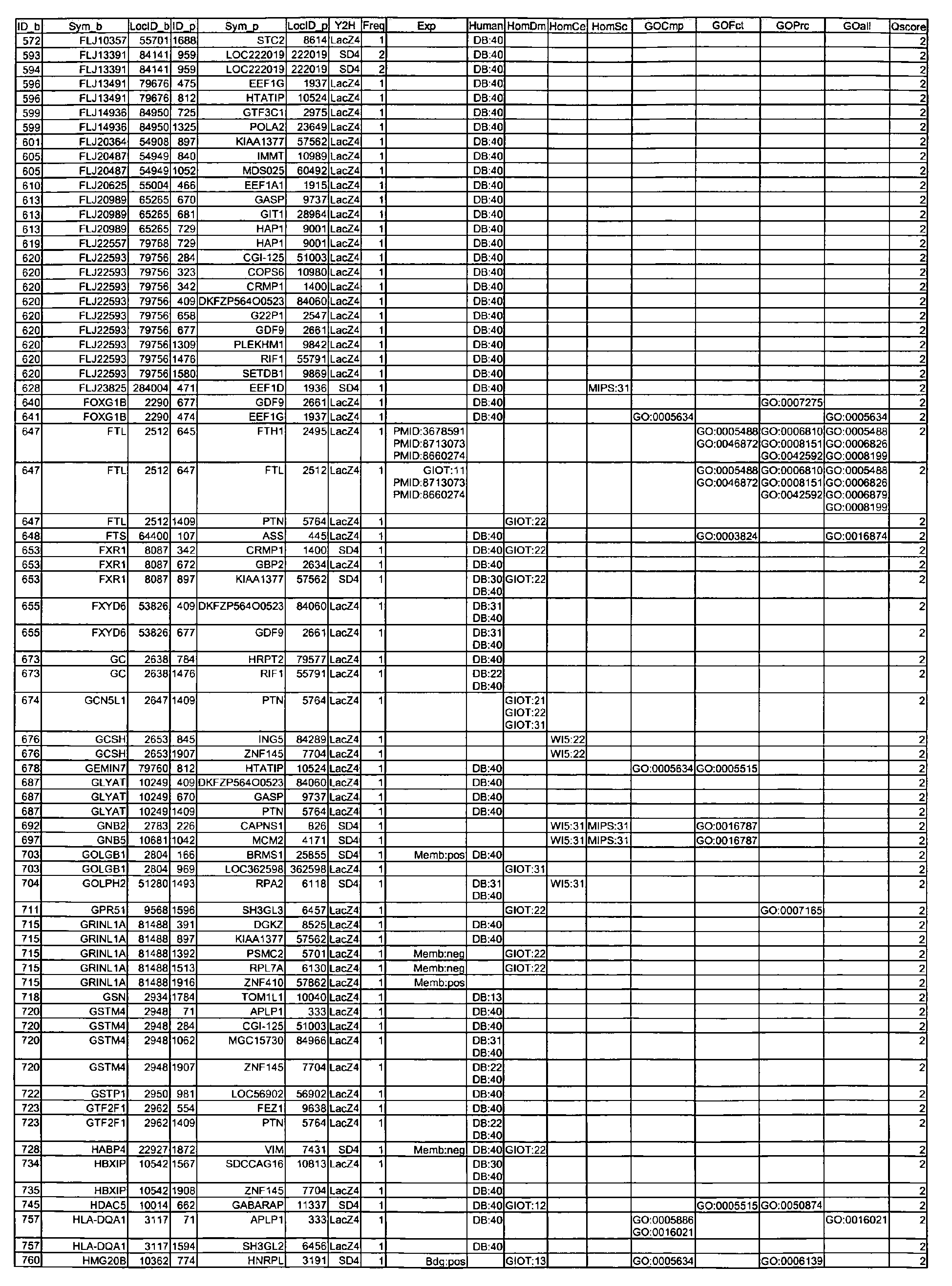

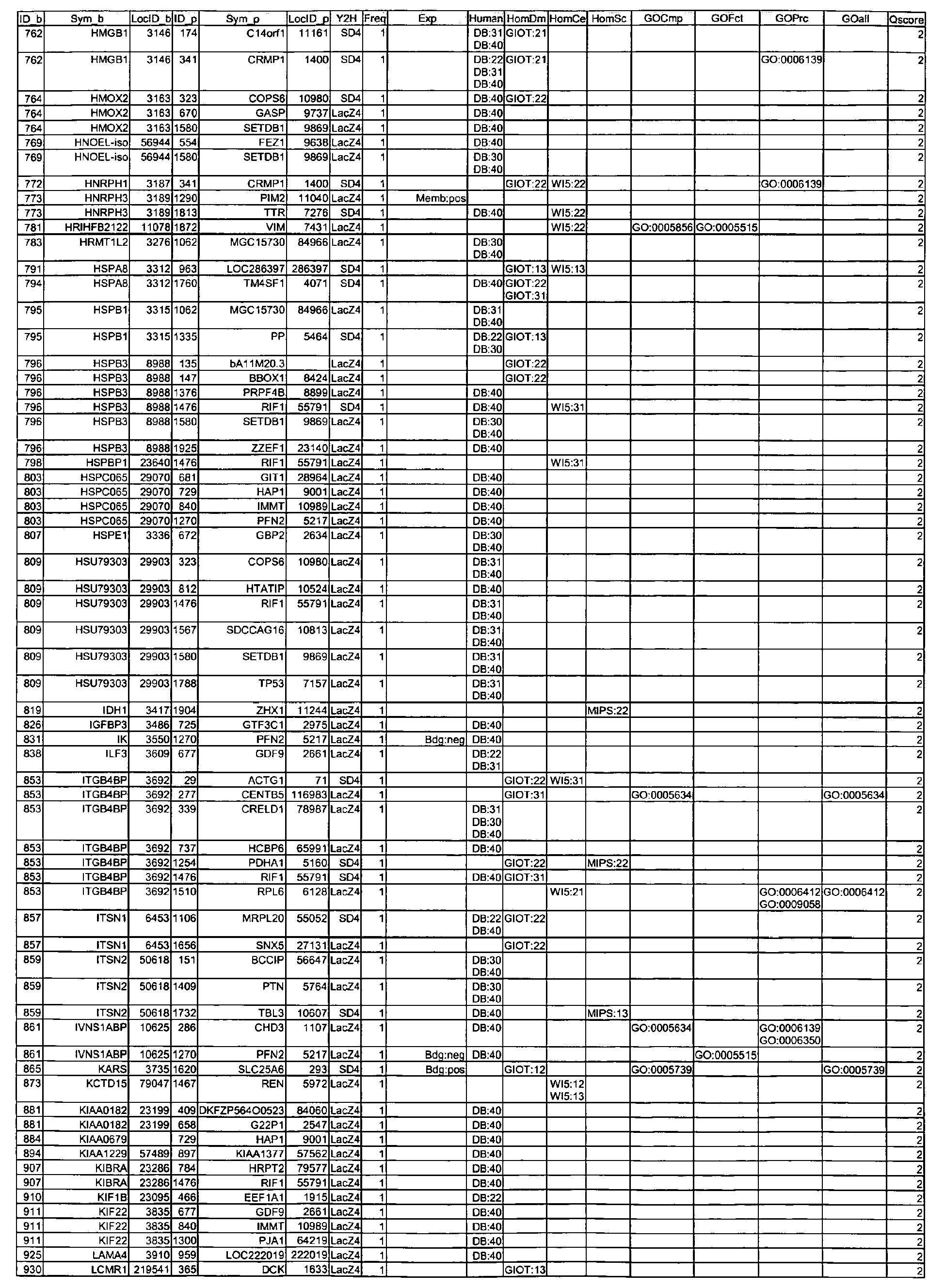

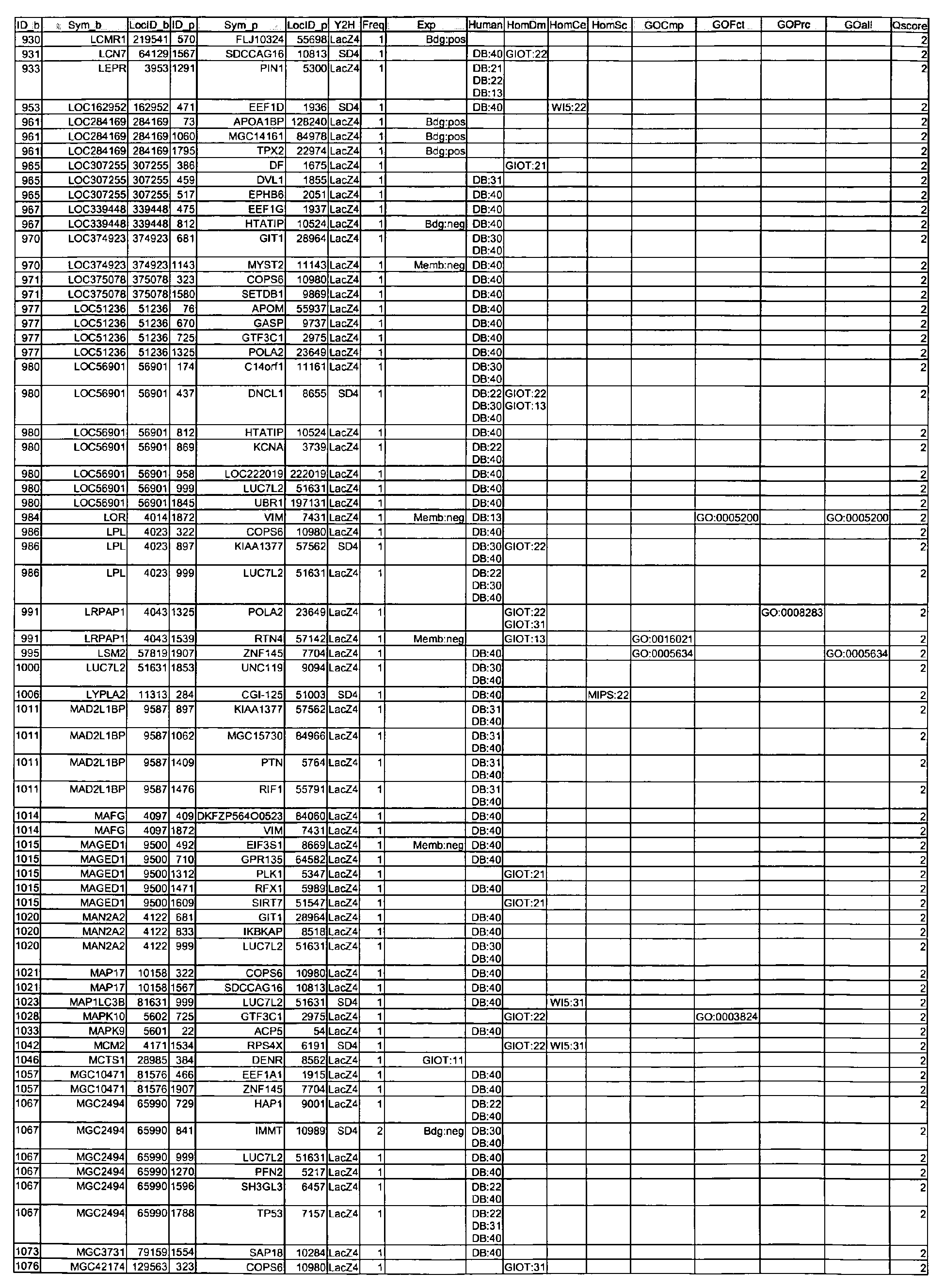

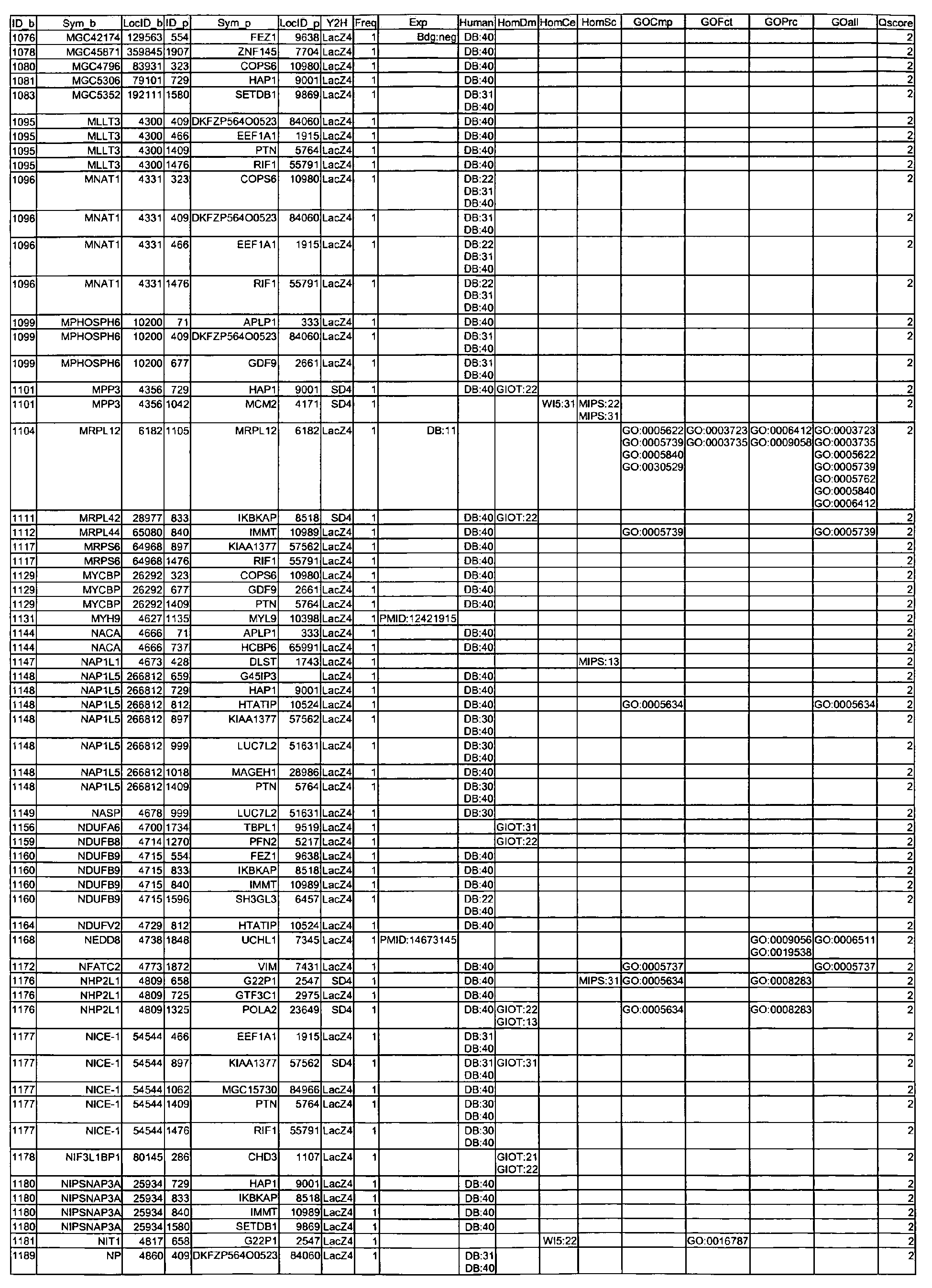

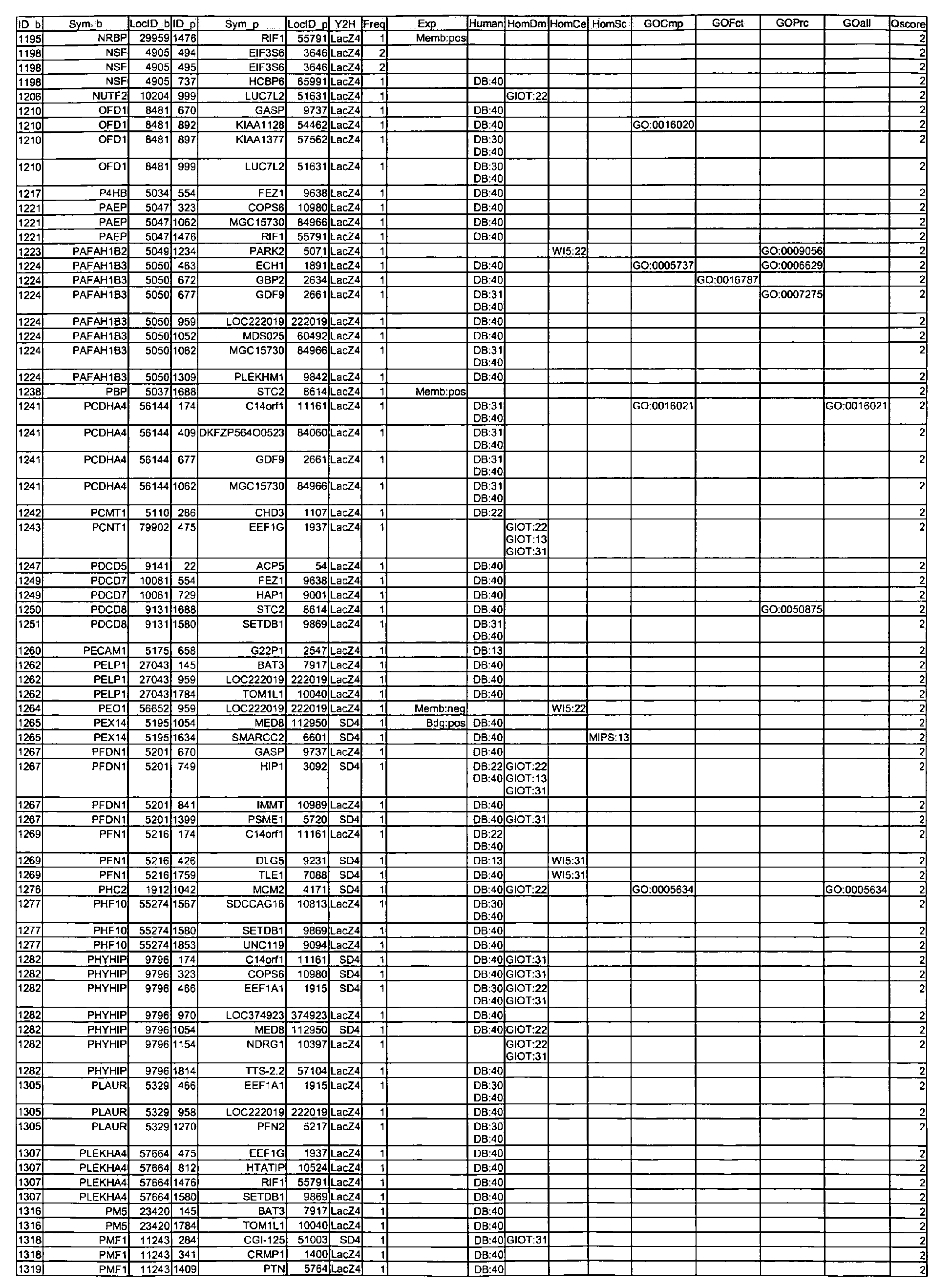

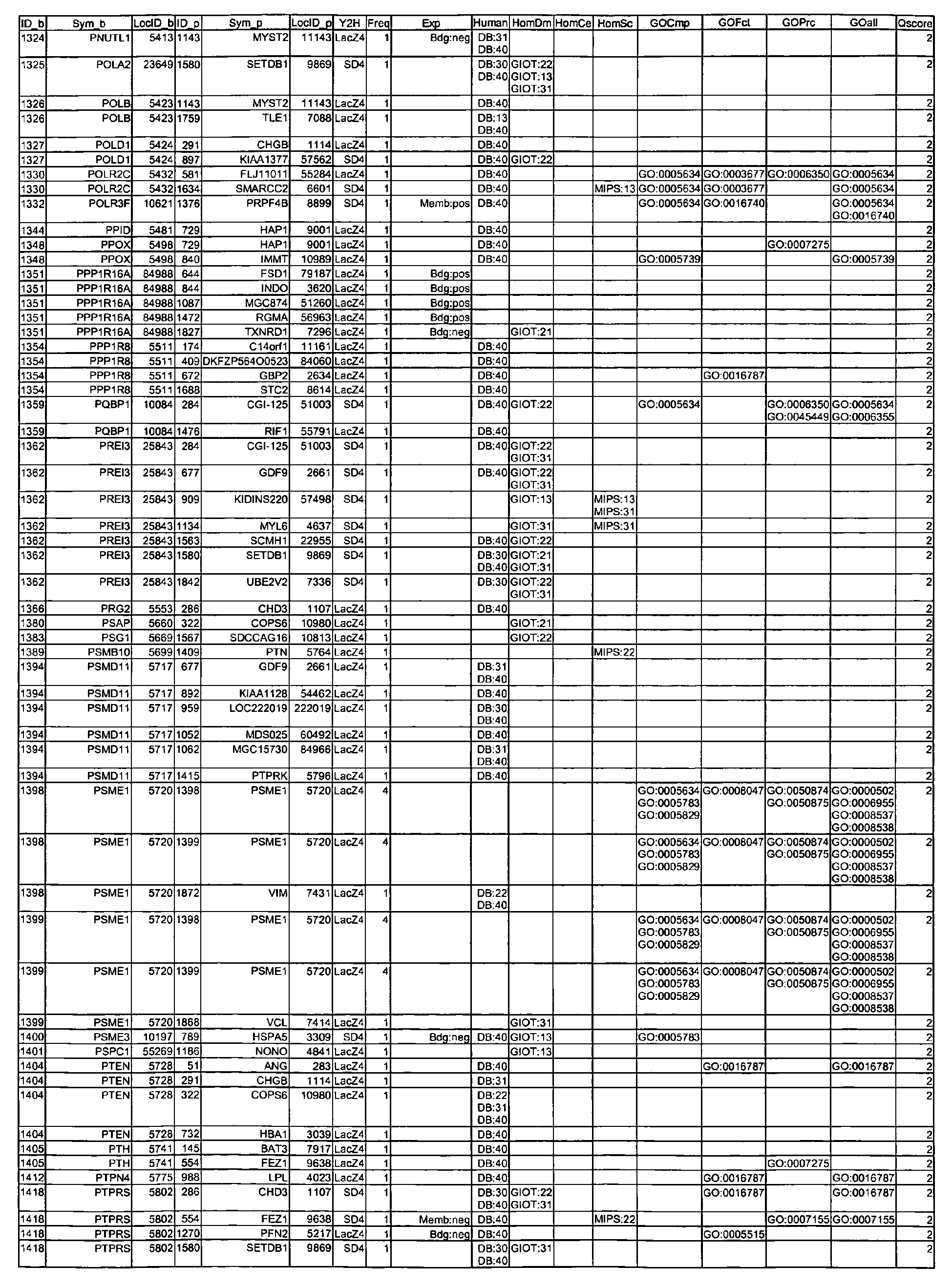

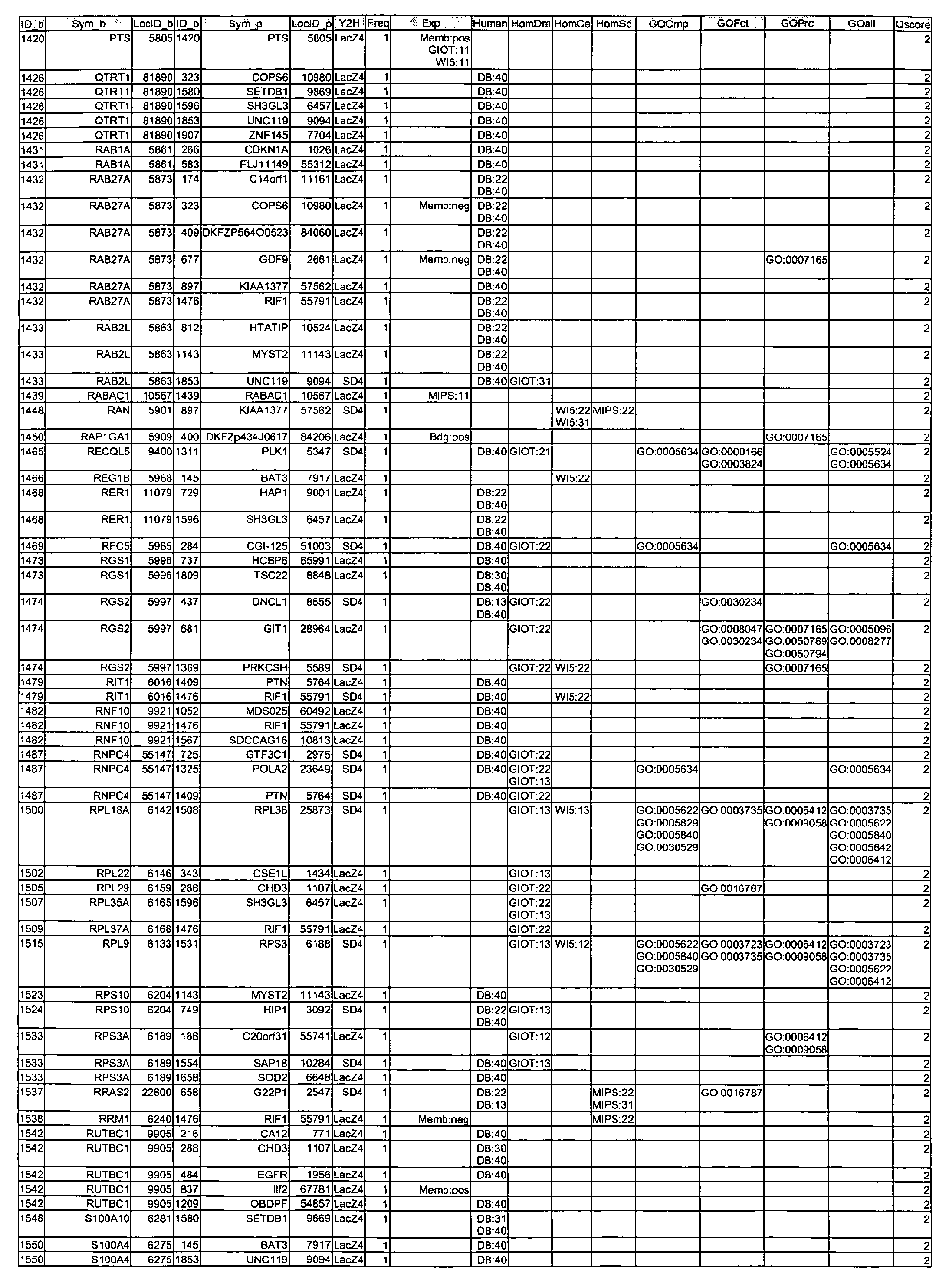

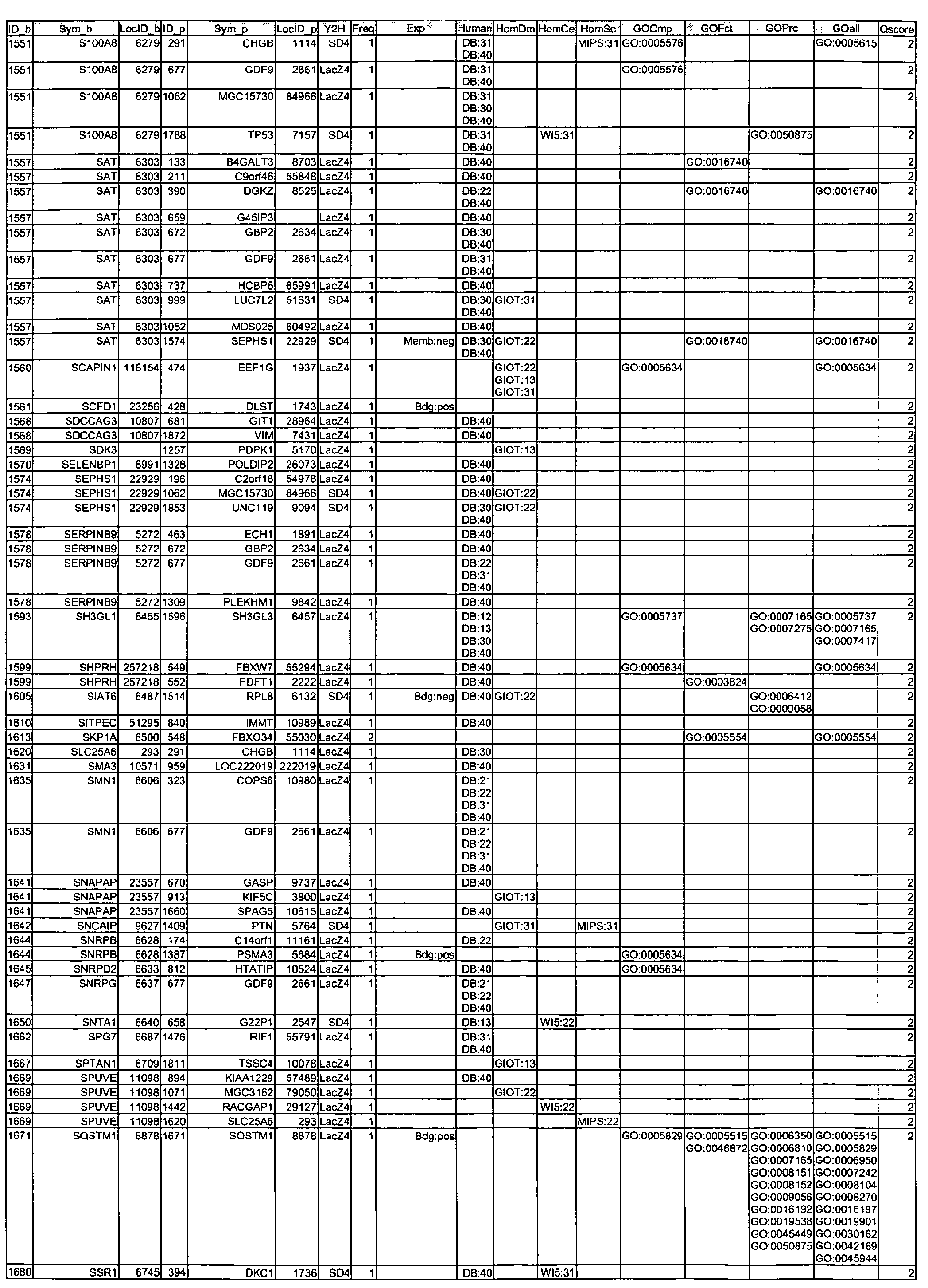

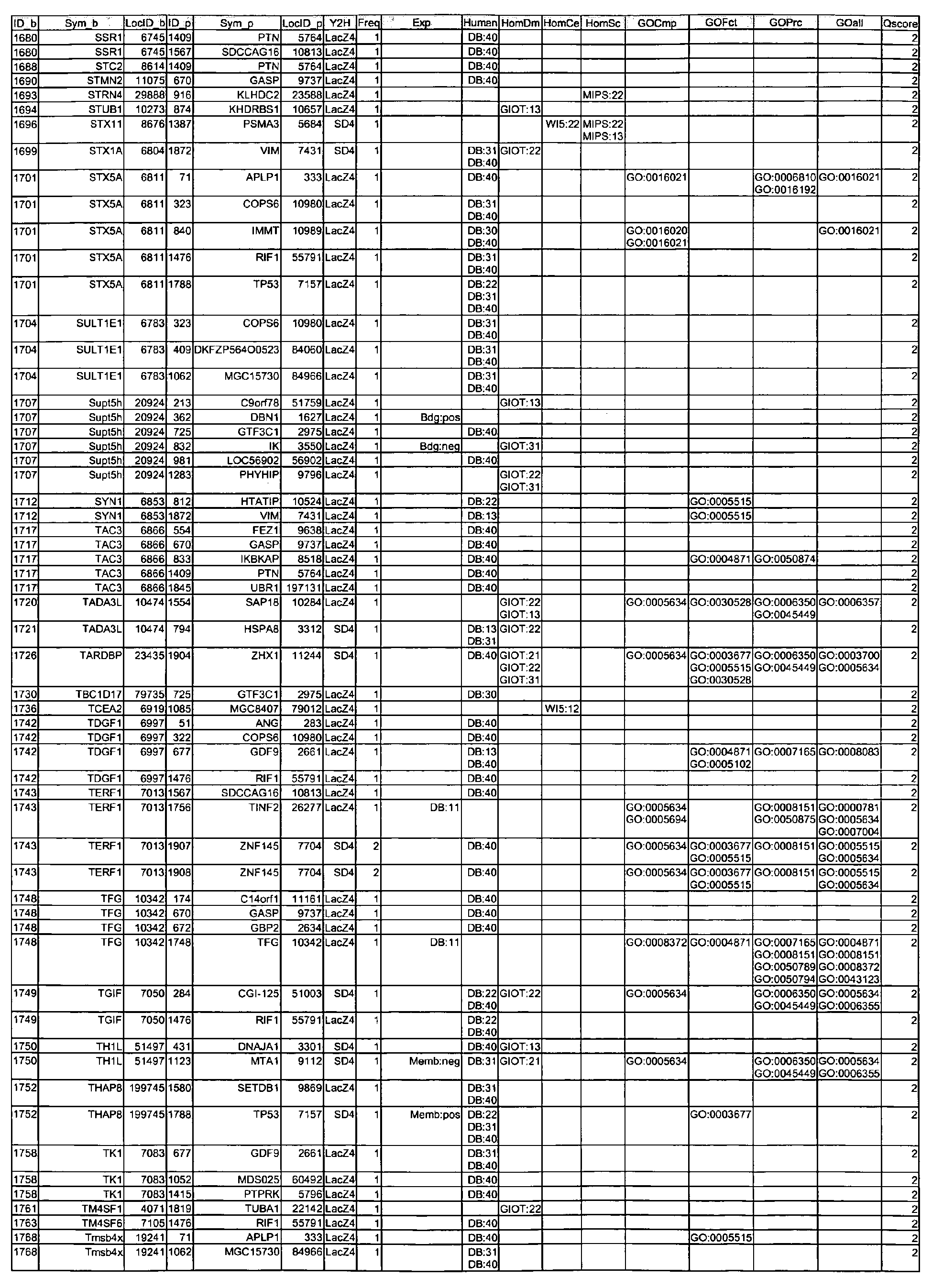

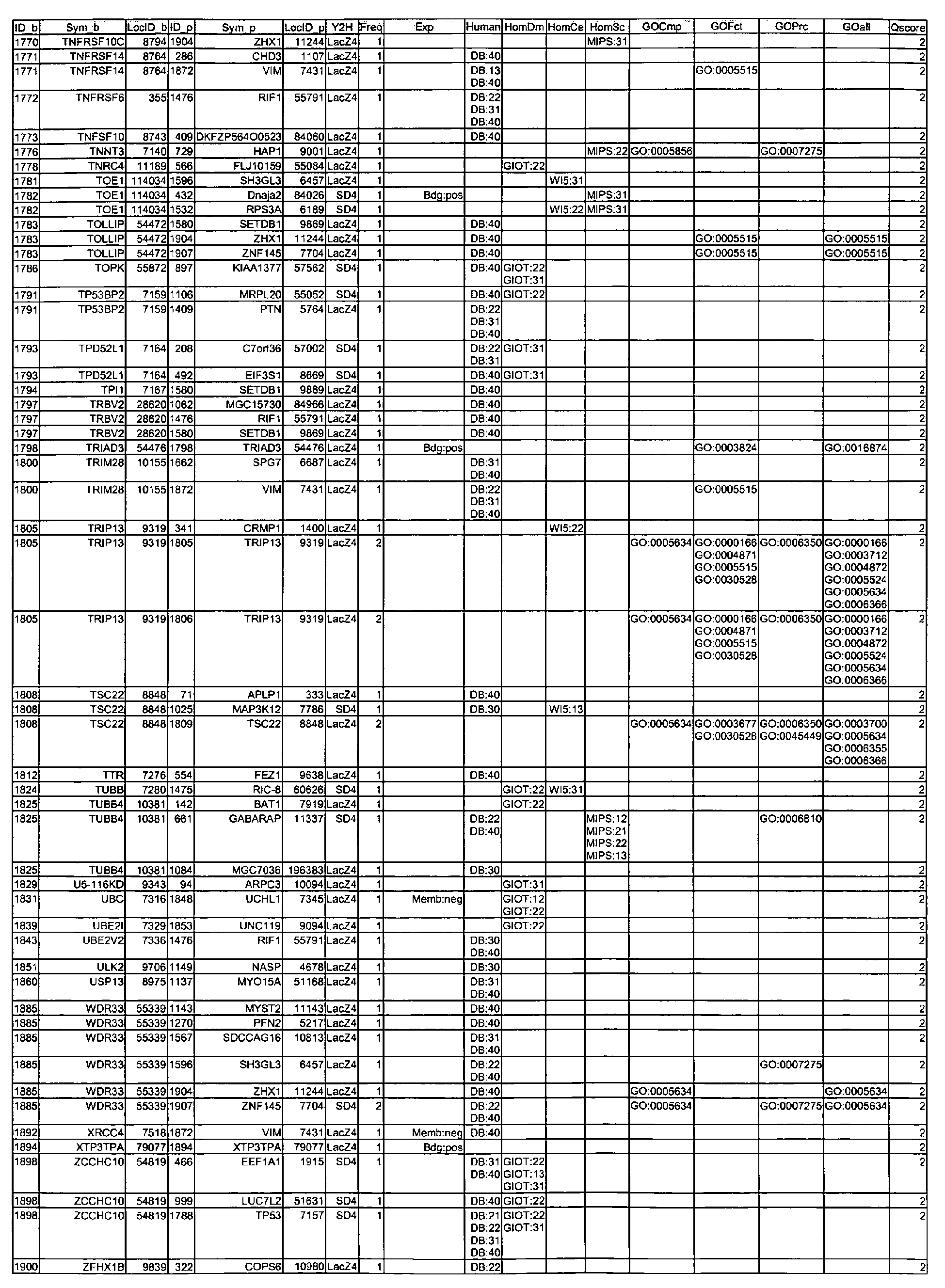

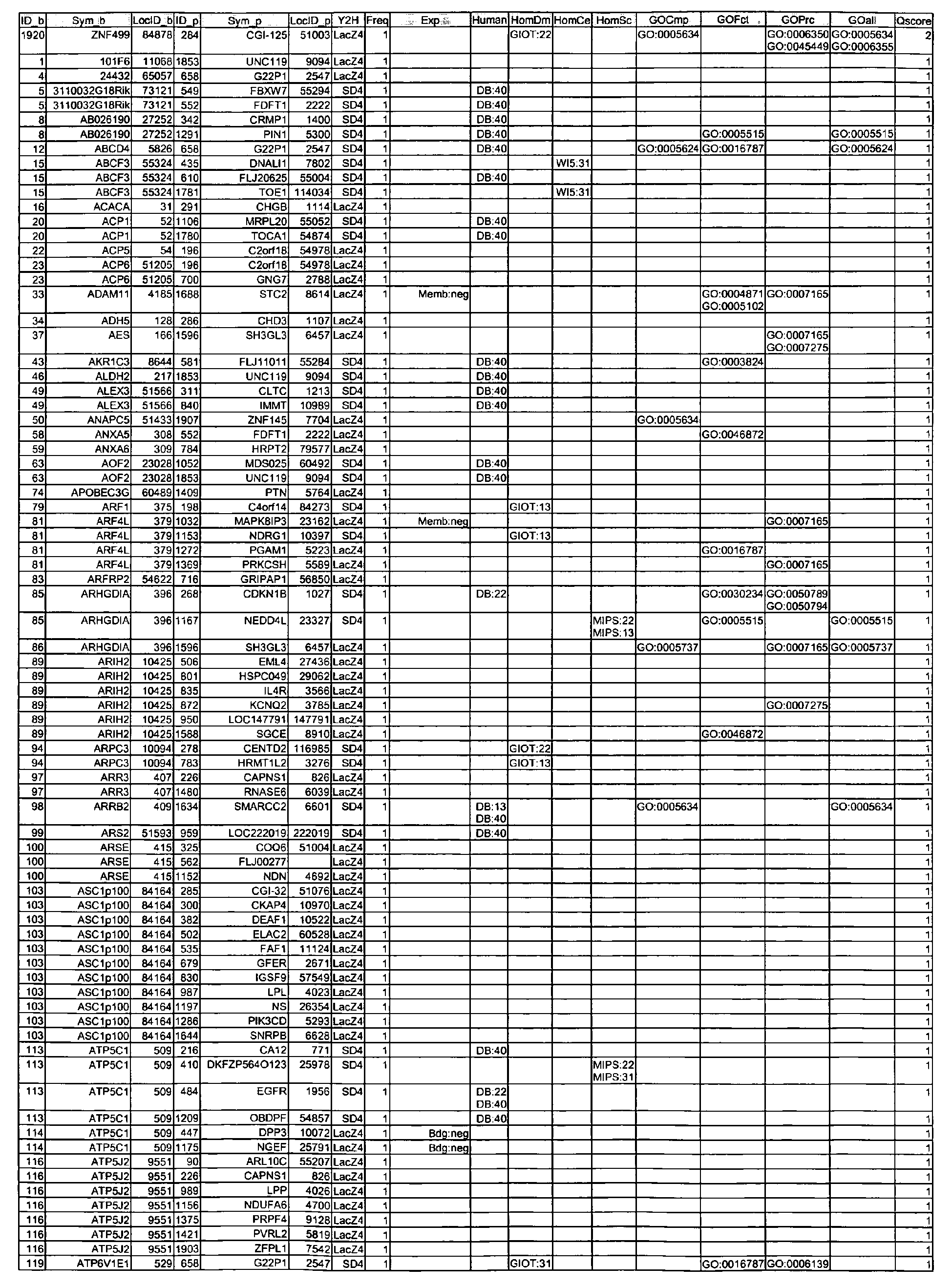

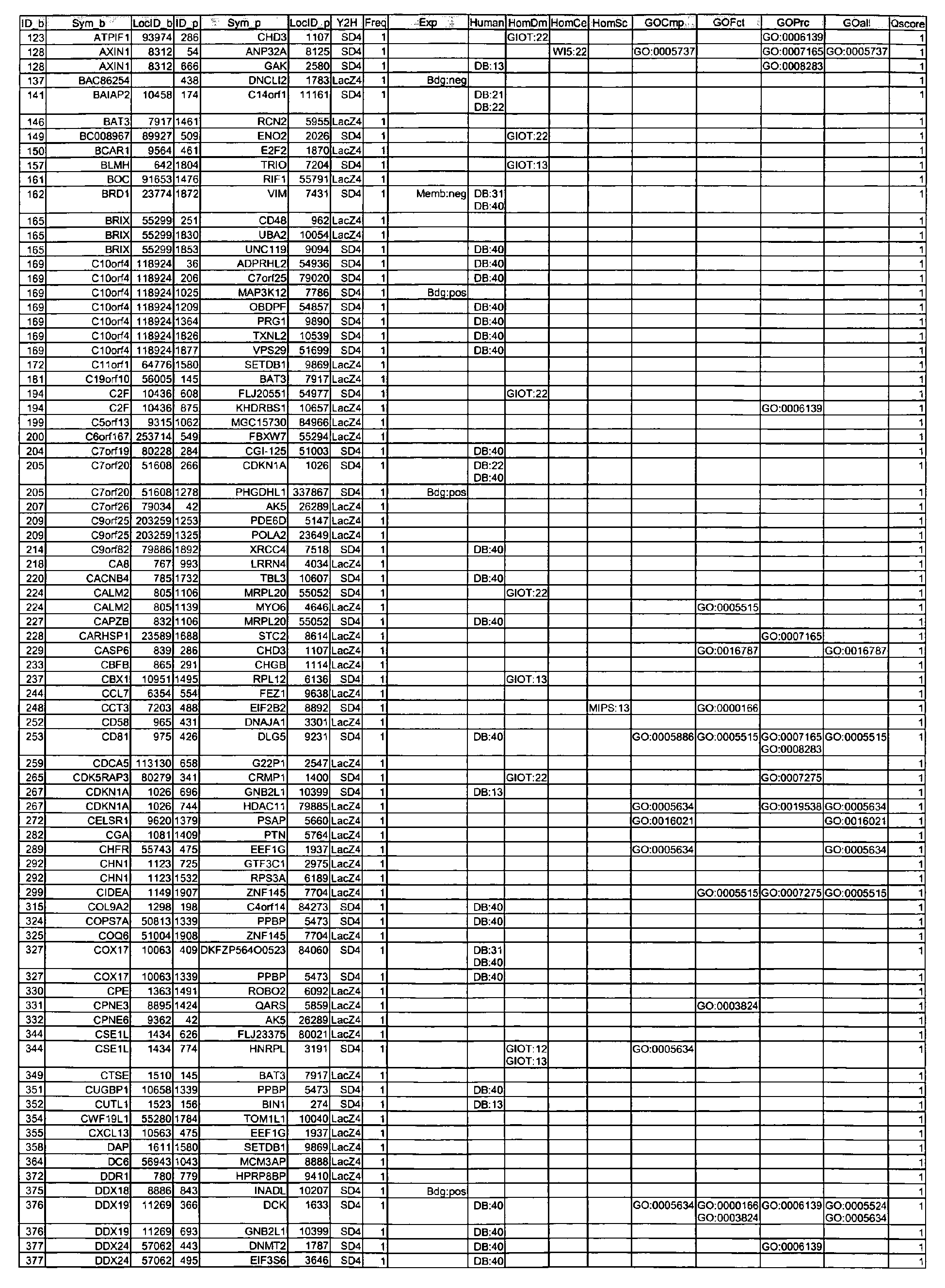

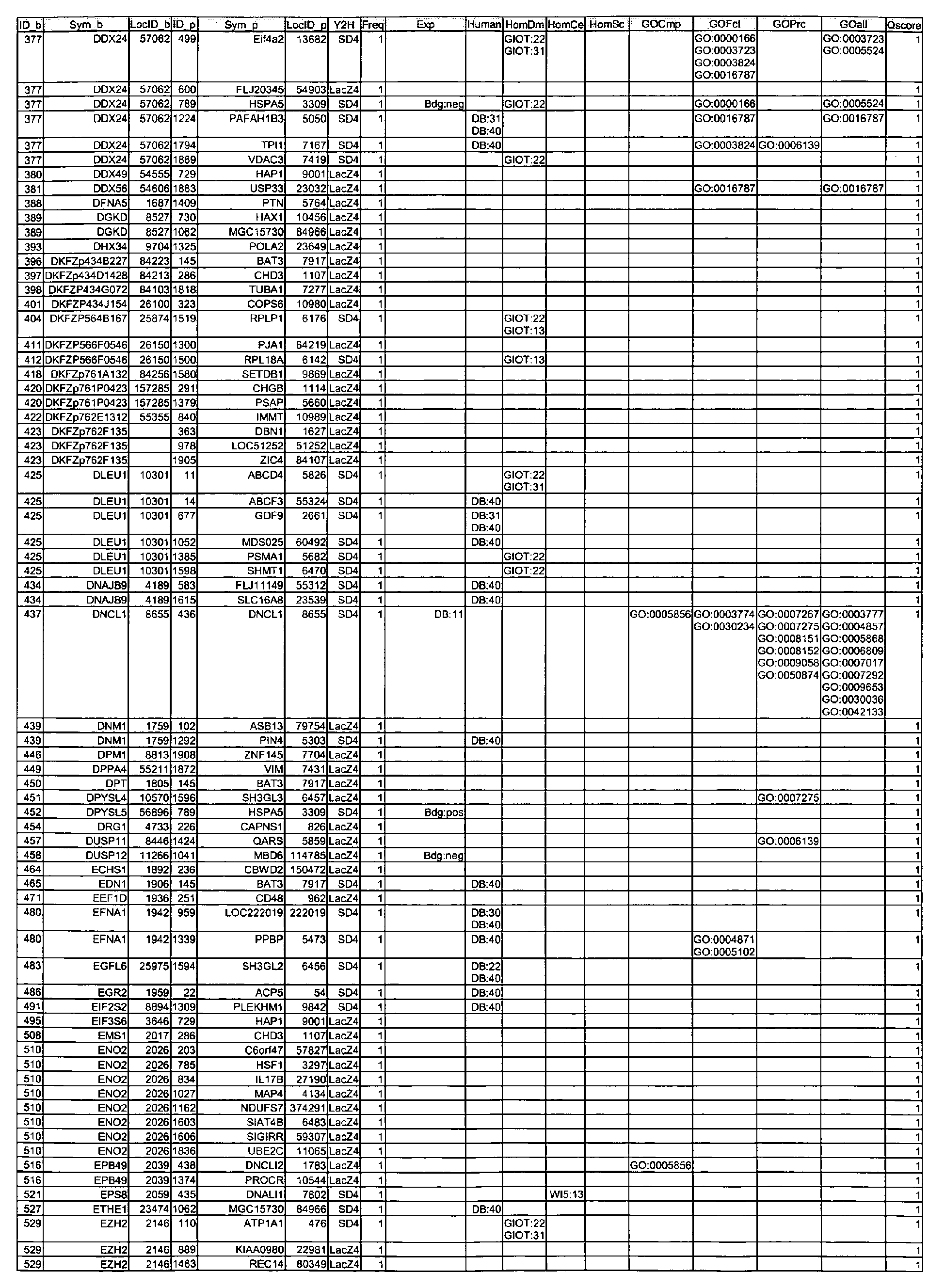

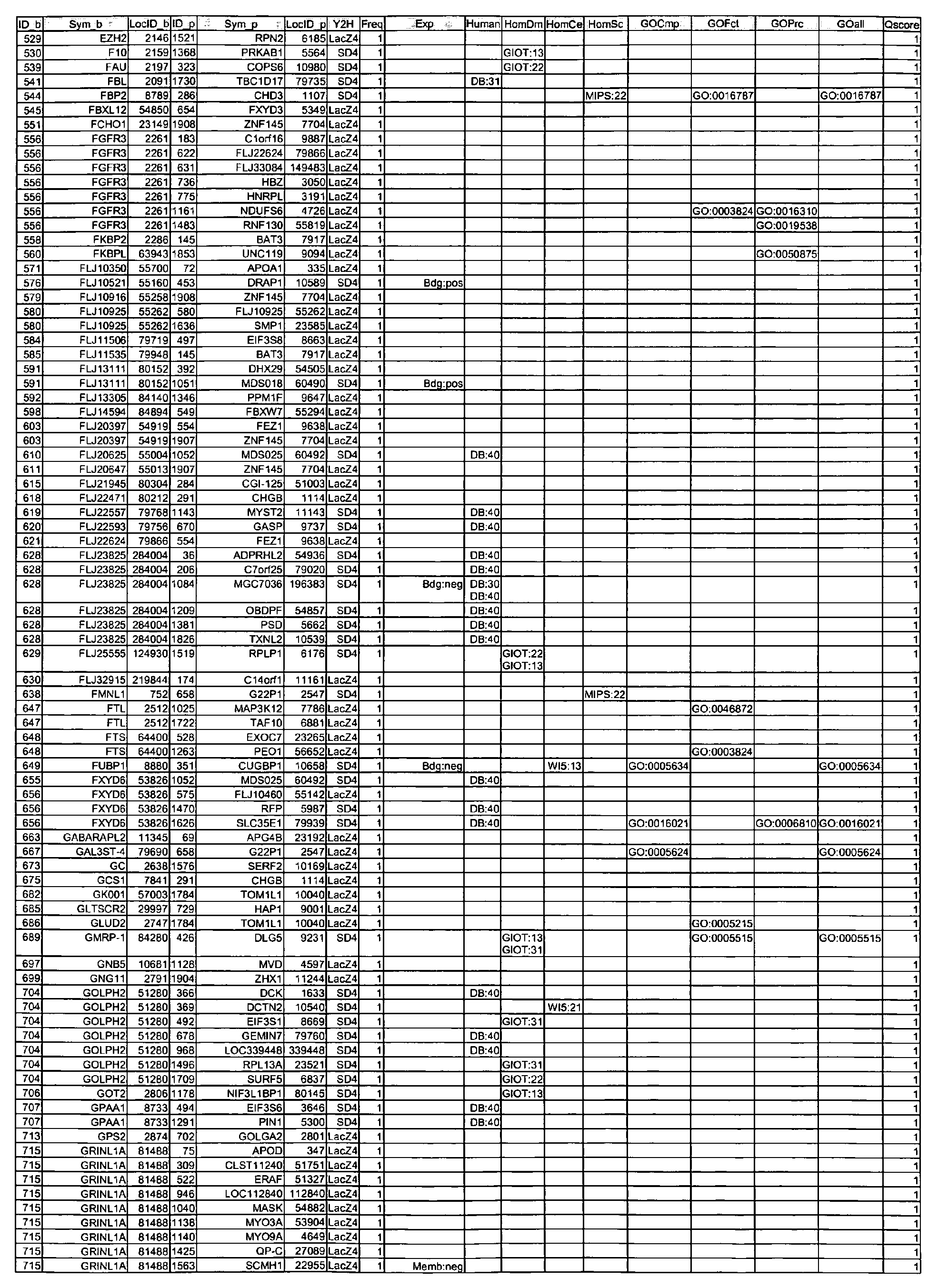

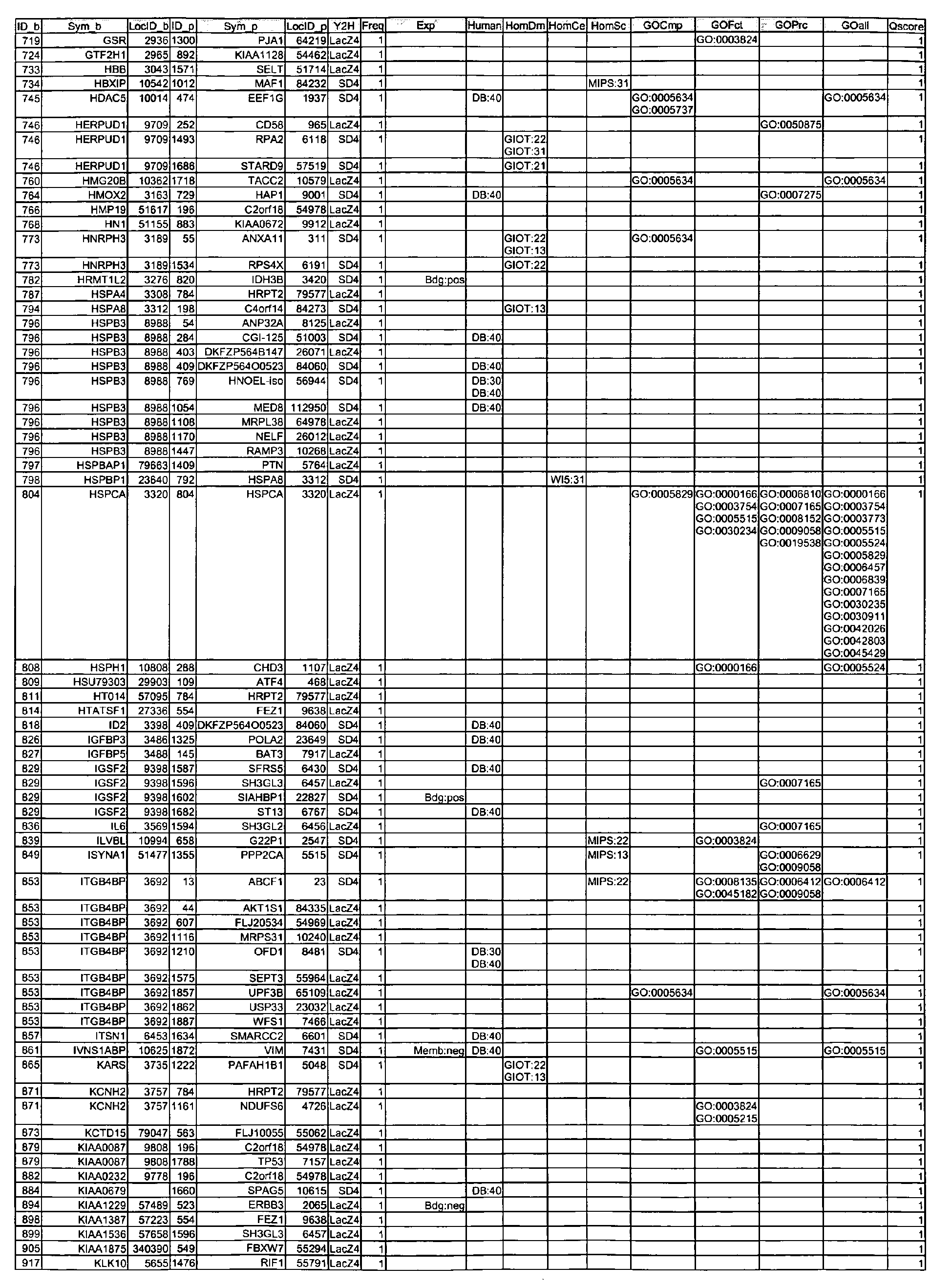

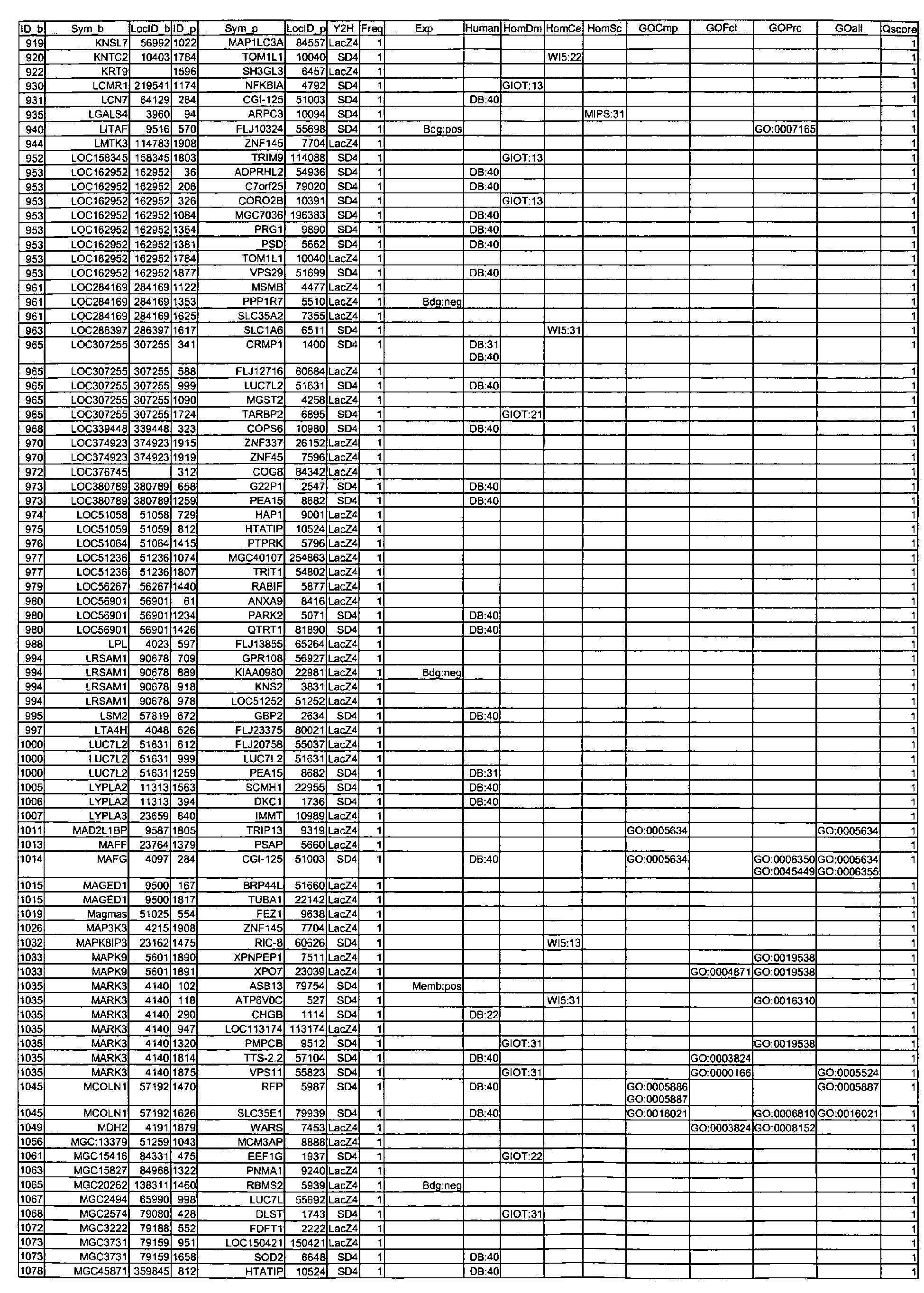

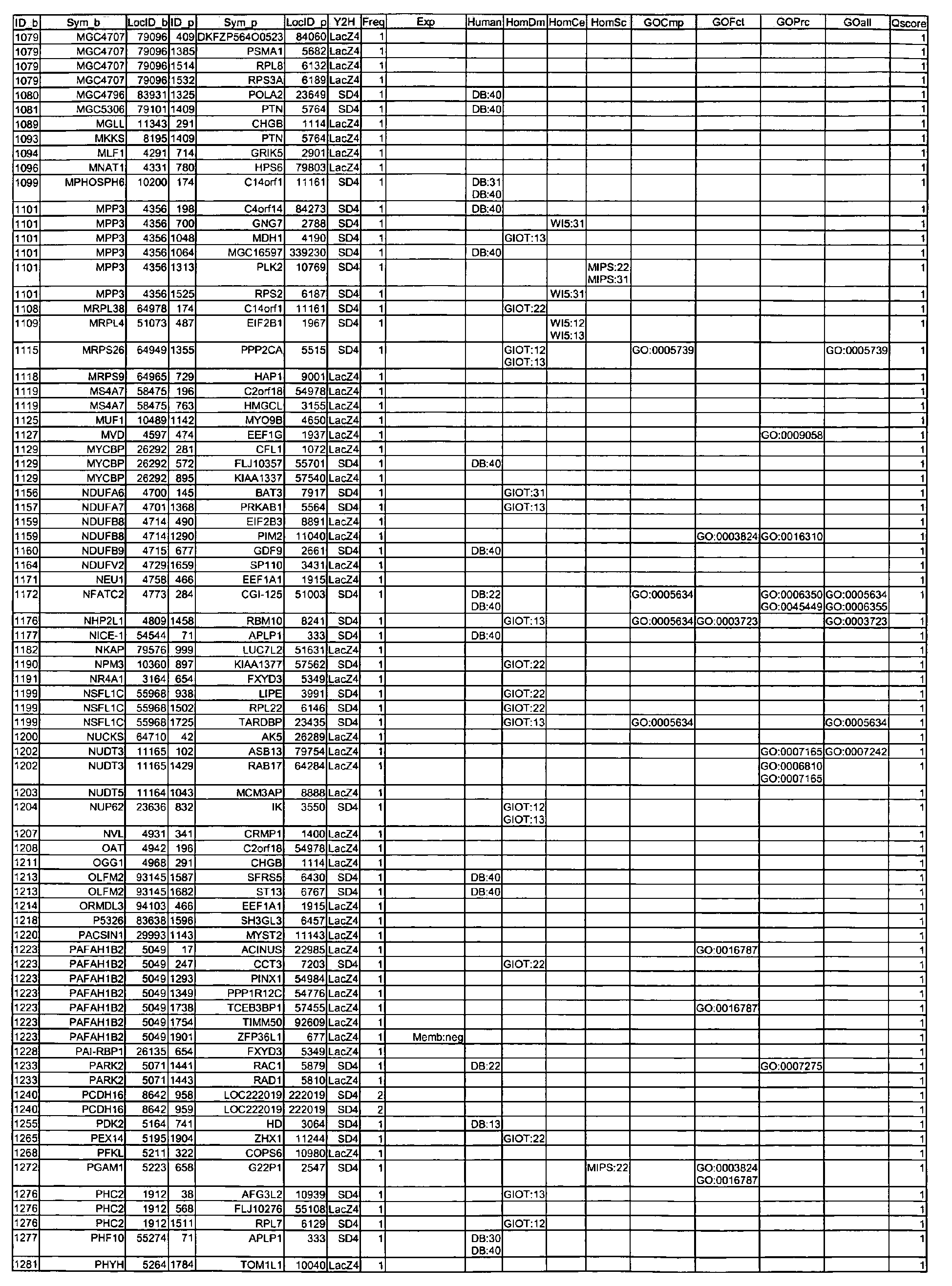

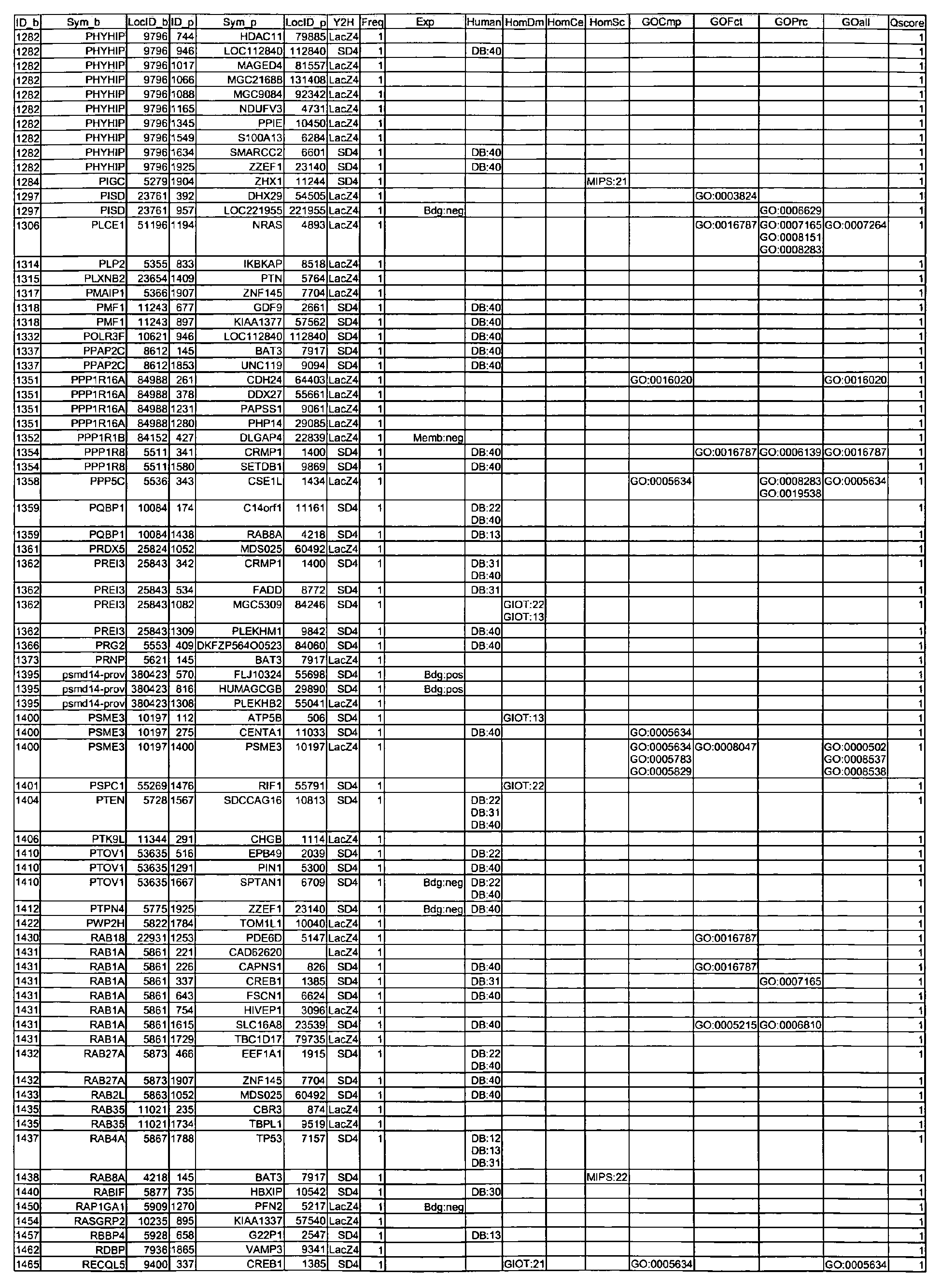

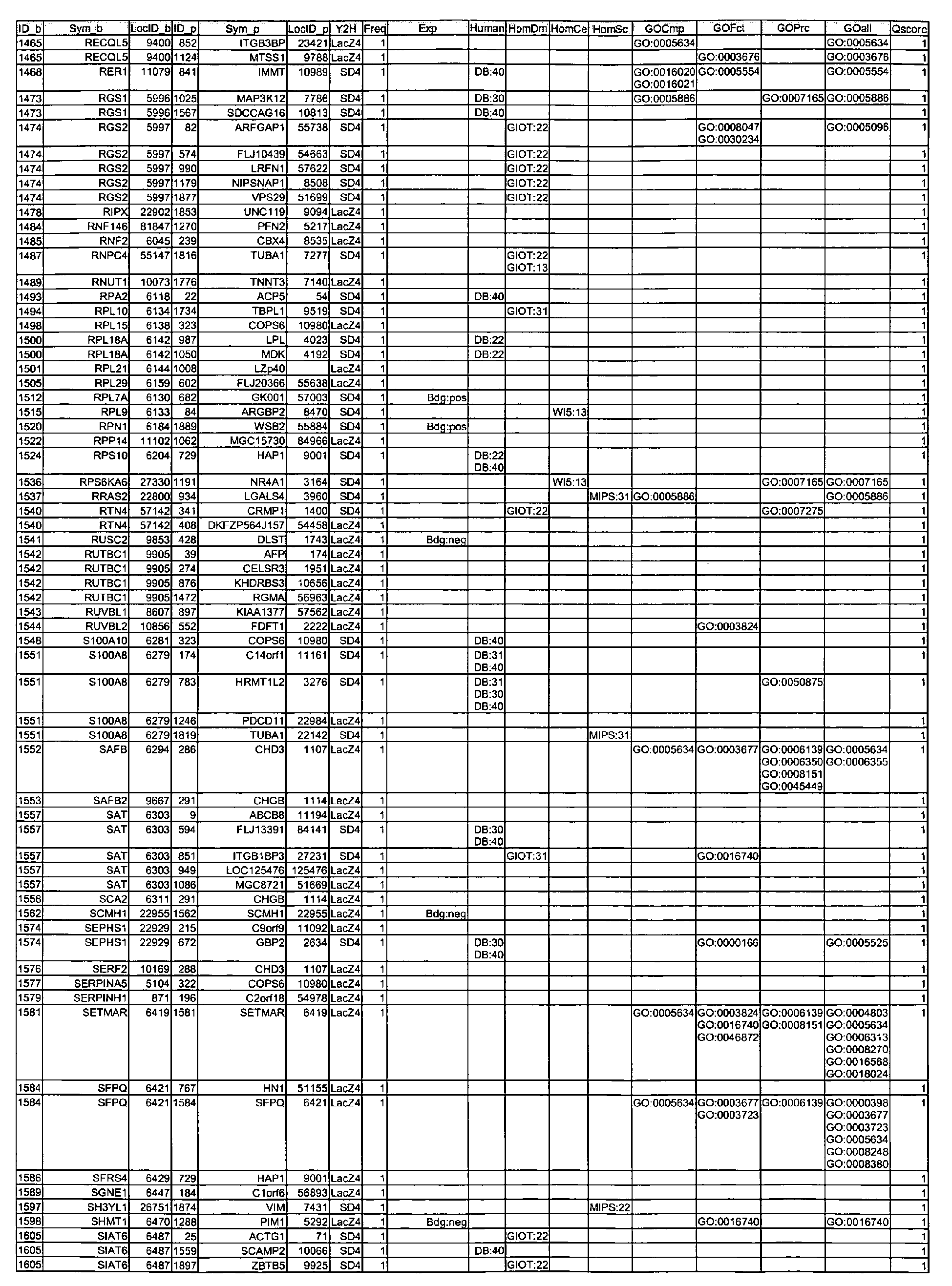

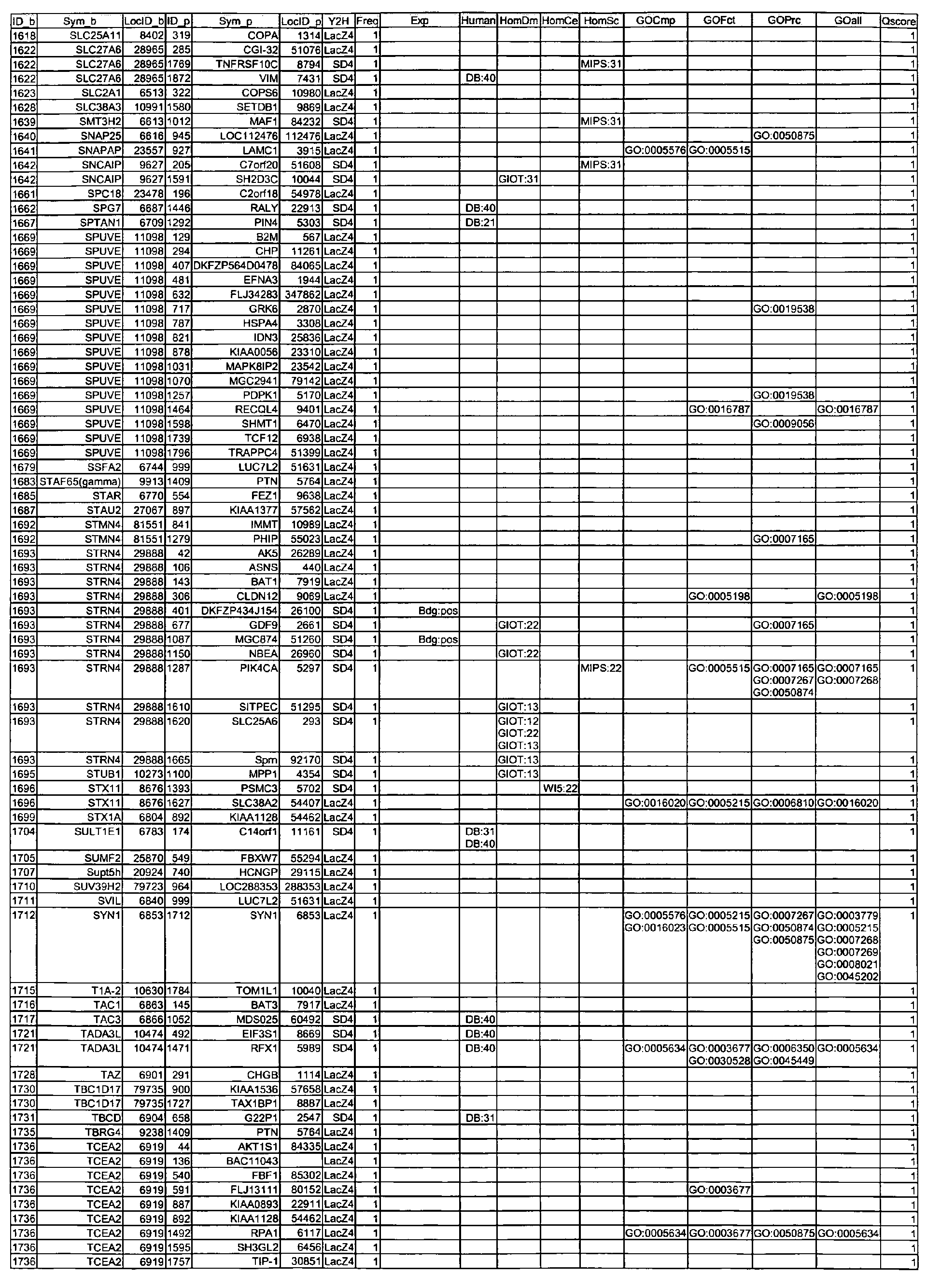

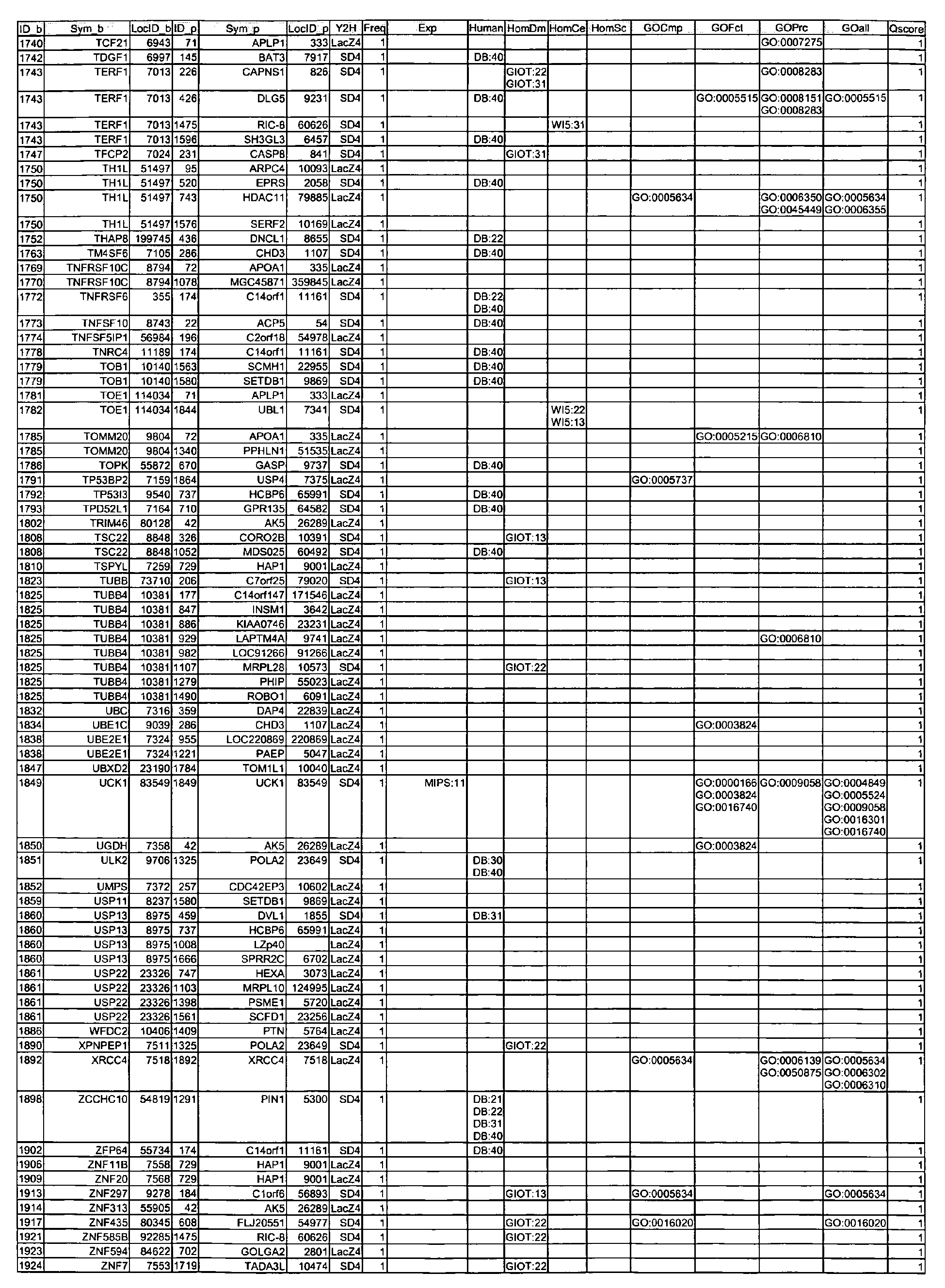

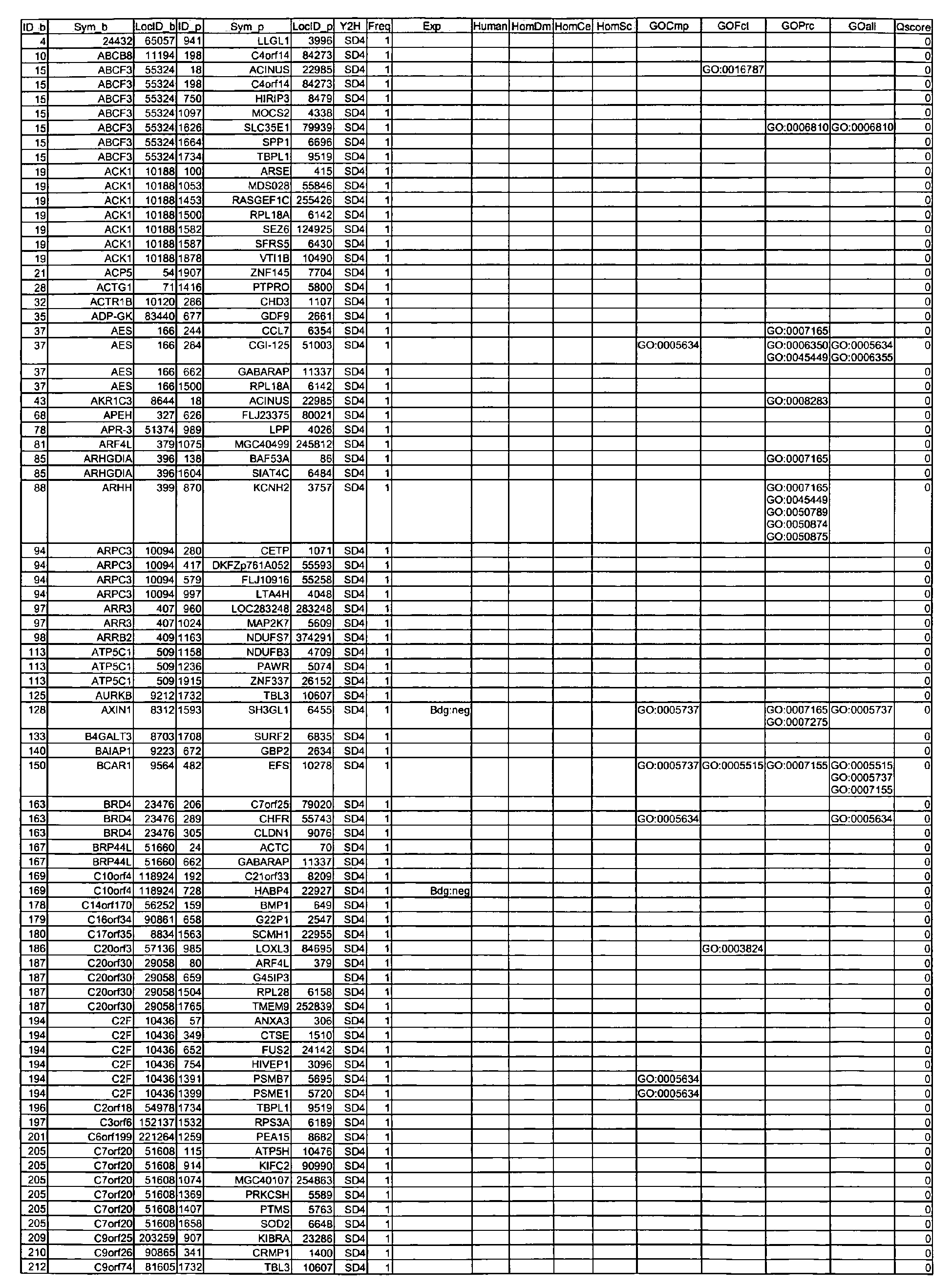

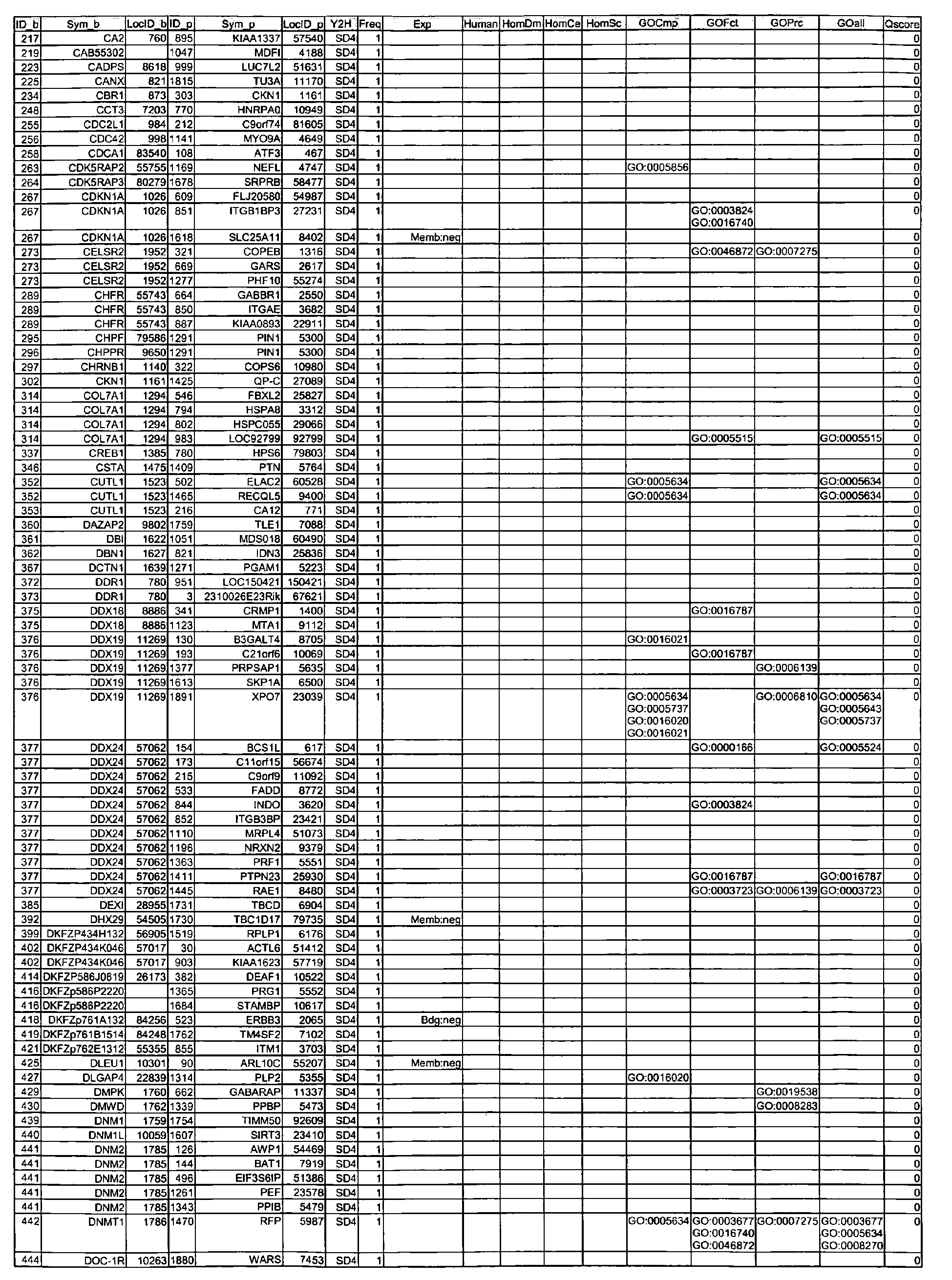

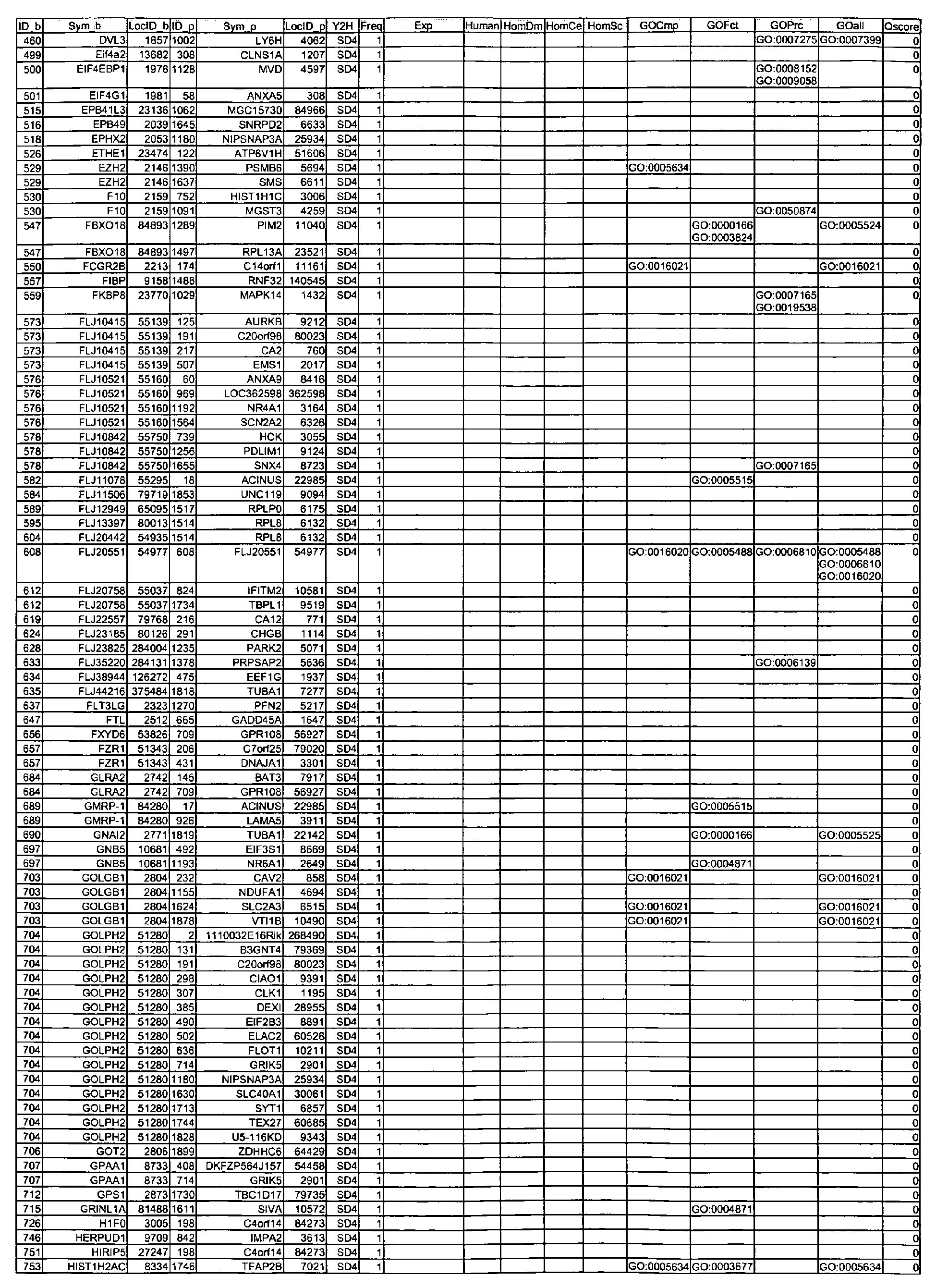

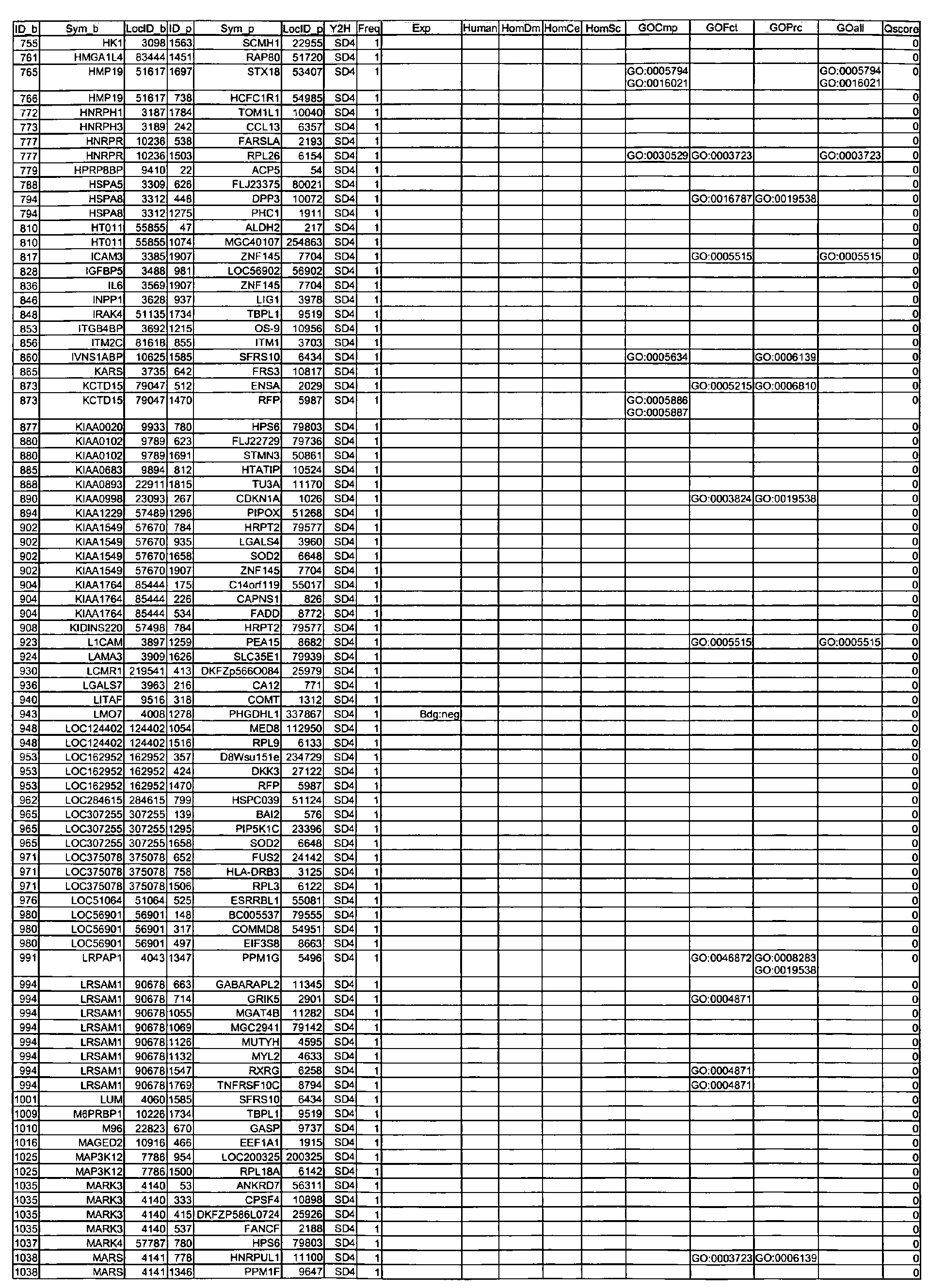

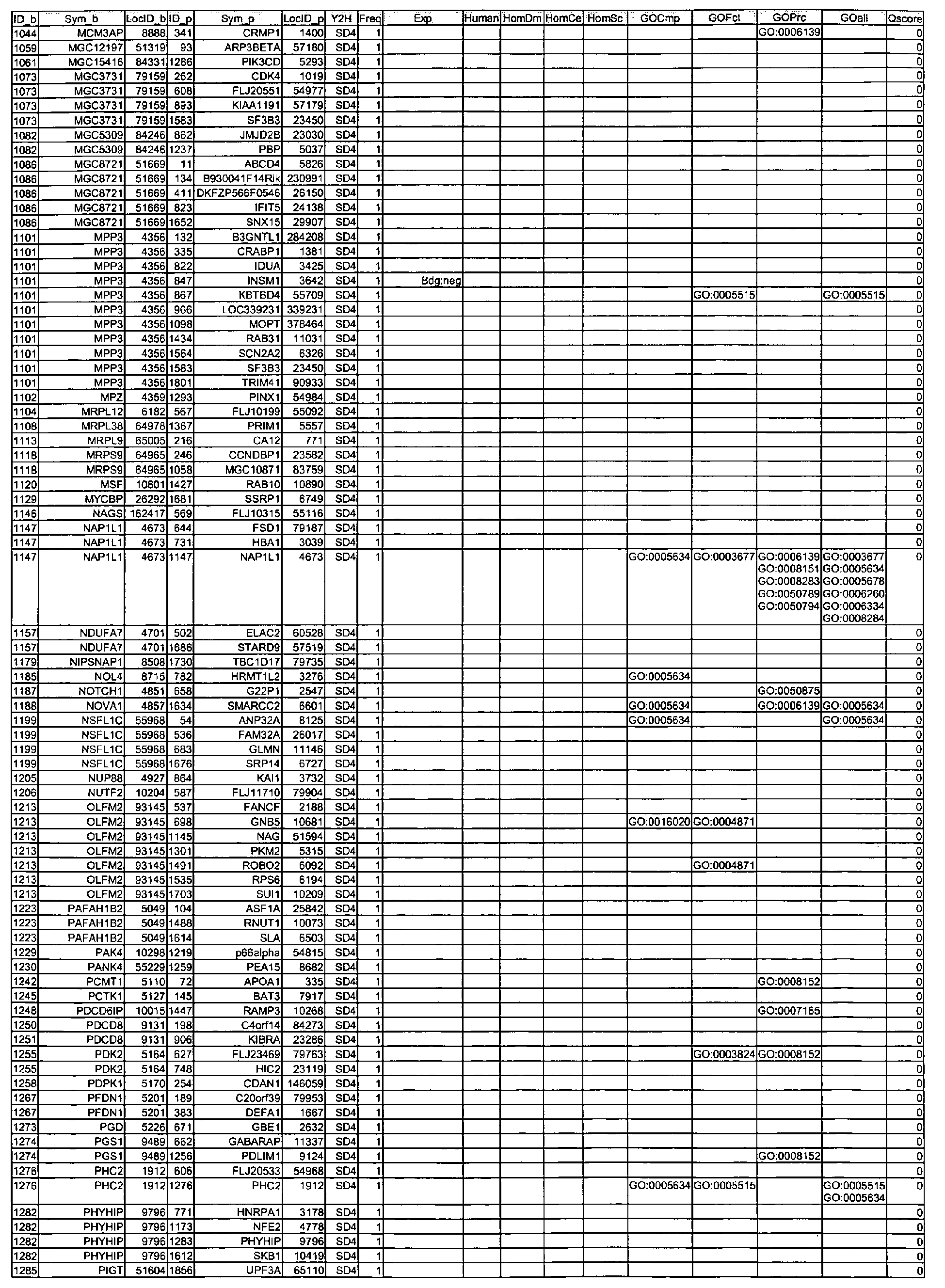

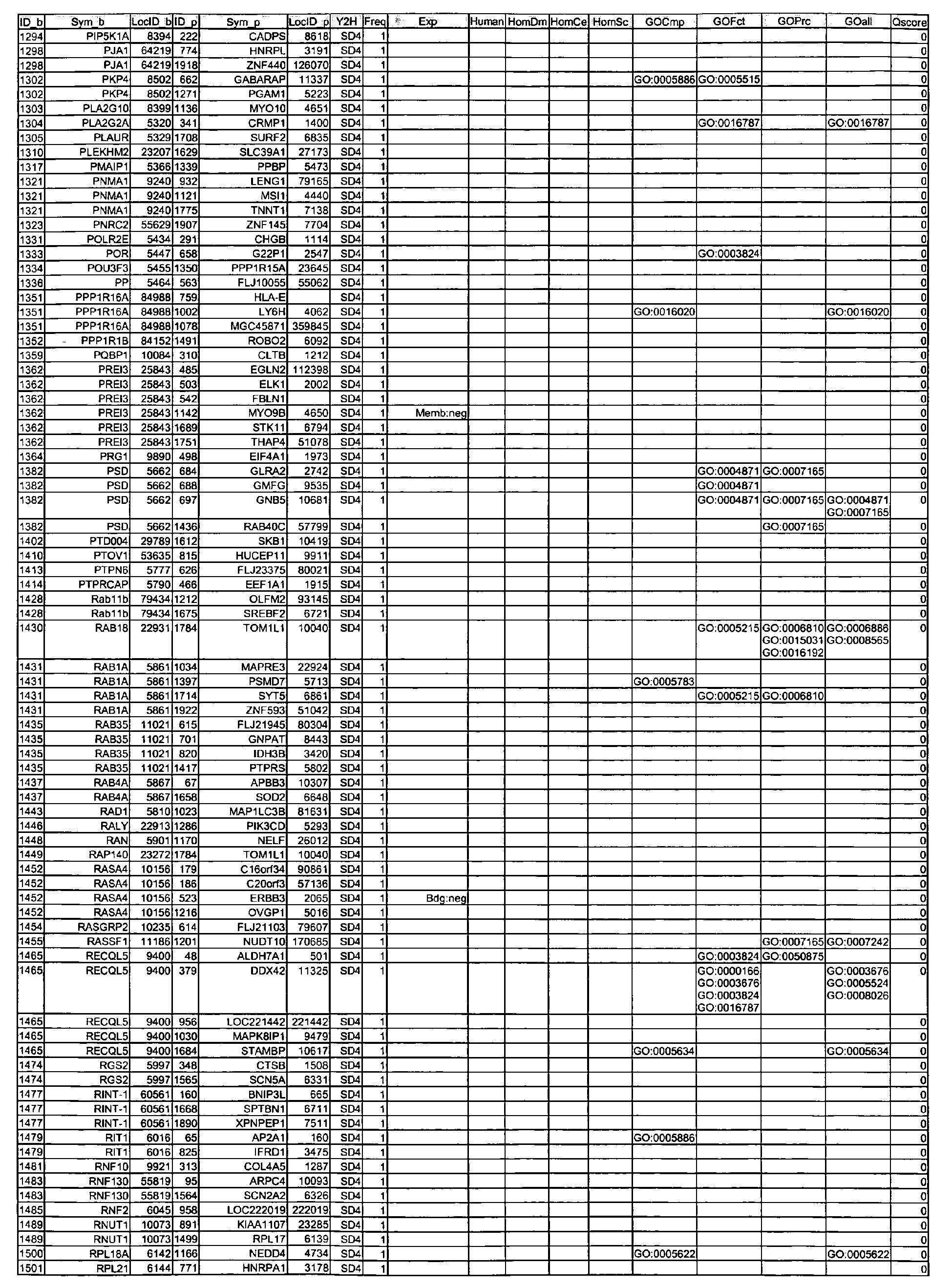

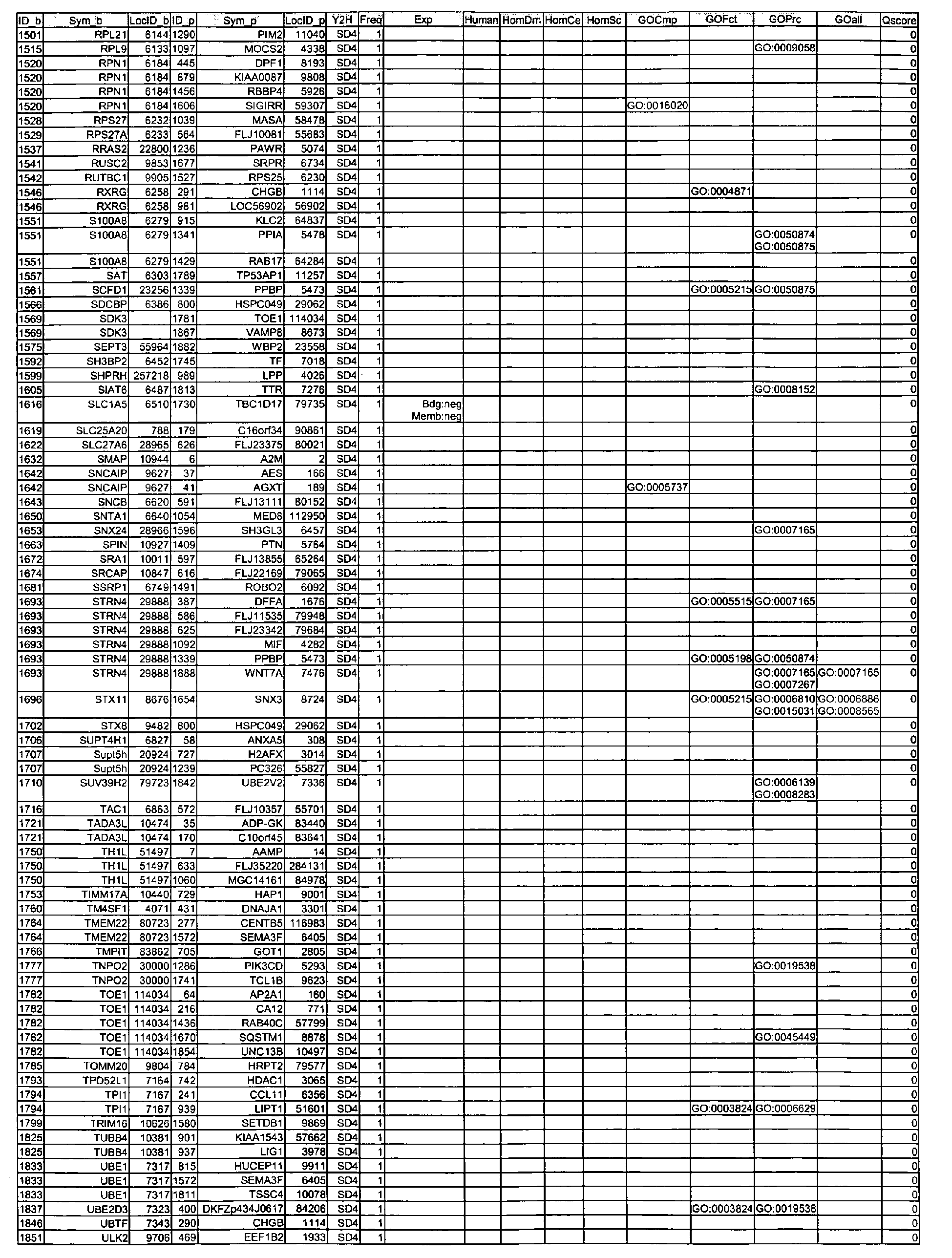

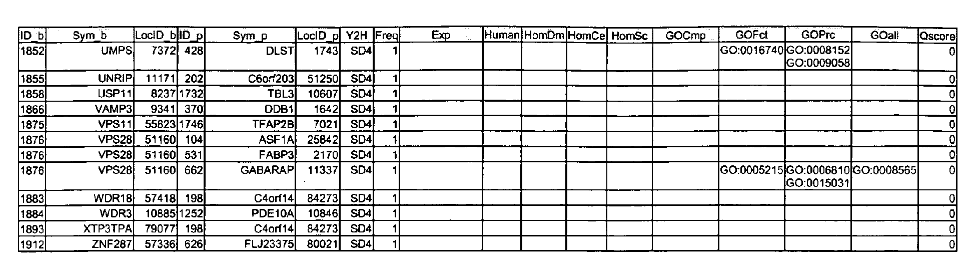

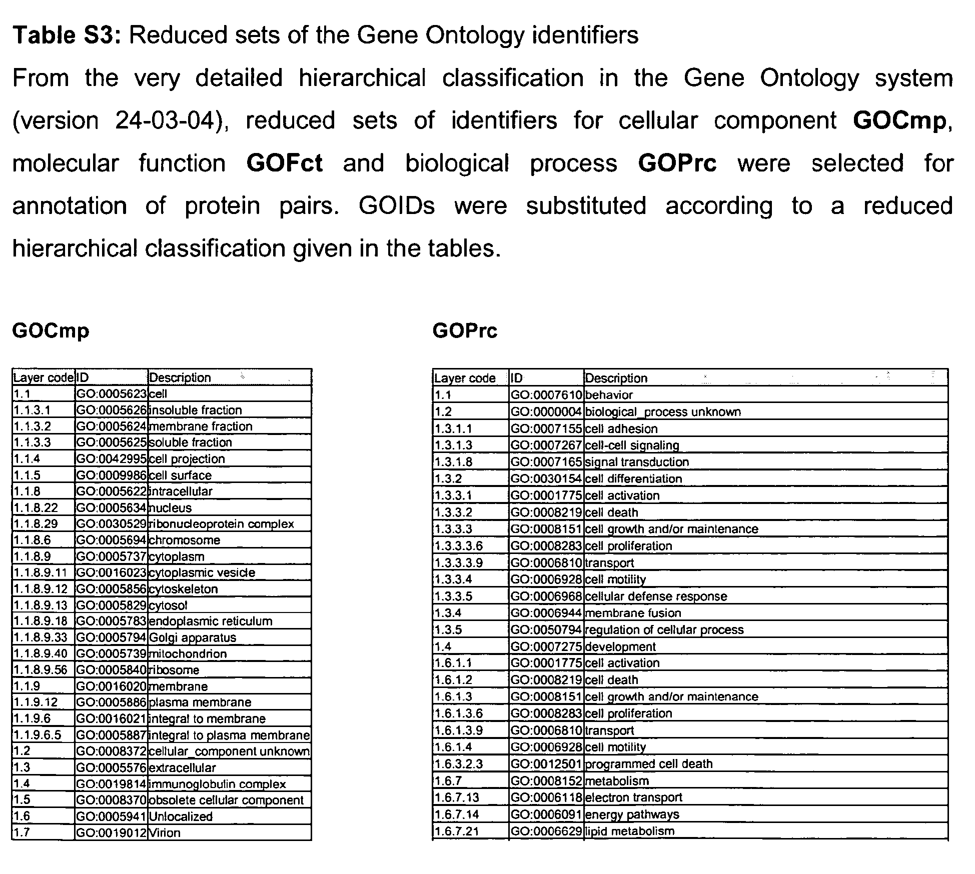

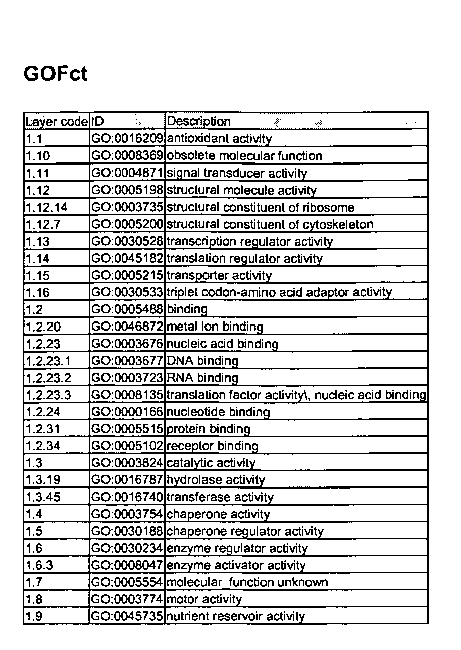

### Table S4: Statistical analysis of protein loop motifs (Fig. 4B).

Relative numbers for Nᵣₑₐₗ were calculated for 3,186, 2,053 and 1,133 interactions of the PPl_all, LacZ4 and SD4 data sets, respectively. Relative standard deviation values for the random model analysis were calculated from nine N_{rand_ma} and N_{rand_Sf} networks, with different randomized sets of 1,705 proteins originating from the Y2H matrix.

| PPI set | Assignment | Y2H | Number of PPIS in Nᵣₑₐₗ | | Relative number of PPIs in N_{rand ma} | Standard deviation N_{rand ma} | Z-score N_{rand ma} | Relative number of PPIs in N_{real sf} | Standard deviation N_{rand sf} | Z-score N_{rand sf} |
|---|---|---|---|---|---|---|---|---|---|---|
| | | | absolute | relative | | | | | | |
| Set 4 | PPIs found in human interaction clusters | all | 1,752 | 0.550 | 0.051 | 0.004 | 132.3 | 0.466 | 0.007 | 11.4 |
| | | LacZ4 | 1,374 | 0.669 | | | 164.0 | | | 27.7 |
| | | SD4 | 378 | 0.334 | | | 74.9 | | | -17.9 |
| Set 5 | PPIs found in orthologous *D. melanogaster clusters* | all | 957 | 0.300 | 0.084 | 0.008 | 28.8 | 0.195 | 0.007 | 14.0 |
| | | LacZ4 | 721 | 0.351 | | | 35.6 | | | 20.8 |
| | | SD4 | 236 | 0.208 | | | 16.6 | | | 1.7 |
| Set 6 | PPIs found in orthologous *C. elegans clusters* | all | 479 | 0.150 | 0.025 | 0.005 | 24.2 | 0.095 | 0.018 | 3.1 |
| | | LacZ4 | 401 | 0.195 | | | 33.0 | | | 5.6 |
| | | SD4 | 78 | 0.069 | | | 8.4 | | | -1.4 |
| Set 7 | PPIs found in orthologous *S. cerevisiae clusters* | all | 316 | 0.099 | 0.029 | 0.004 | 17.2 | 0.090 | 0.006 | 1.5 |
| | | LacZ4 | 244 | 0.119 | | | 22.0 | | | 4.7 |
| | | SD4 | 72 | 0.064 | | | 8.5 | | | -4.2 |

### Table S5: Statistical significance of the quality scoring system (Fig. 4E).

Relative numbers for Nᵣₑₐₗ, N_{rand_ma} and N_{rand_sf} networks were calculated as in **Table S4.**

| Bioinformatic Quality scores | Y2H | Number of PPls in Nᵣₑₐₗ | | Relative number of PPls in N_{rand ma} | Standard deviation N_{rand ma} | Z- score N_{rand ma} | Relative number of PPls in N_{real sf} | Standard deviation N_{rand sf} | Z-score N_{rand sf} |
|---|---|---|---|---|---|---|---|---|---|
| | | absolute | relative | | | | | | |
| 0 | all | 1,116 | 0.350 | 0.834 | 0.010 | -47.9 | 0.411 | 0.005 | -12.2 |
| | LacZ4 | 518 | 0.252 | | | -57.6 | | | -32.0 |
| | SD4 | 598 | 0.528 | | | -30.3 | | | 23.6 |
| 1 | all | 1,072 | 0.336 | 0.146 | 0.008 | 23.5 | 0.391 | 0.008 | -7.2 |
| | LacZ4 | 721 | 0.351 | | | 25.3 | | | -5.2 |
| | SD4 | 351 | 0.310 | | | 20.2 | | | -10.7 |
| 2 | all | 647 | 0.203 | 0.018 | 0.004 | 51.3 | 0.148 | 0.010 | 5.7 |
| | LacZ4 | 503 | 0.245 | | | 63.0 | | | 9.9 |
| | SD4 | 144 | 0.127 | | | 30.2 | | | -2.1 |
| >2 | all | 351 | 0.110 | 0.002 | 0.001 | 148.0 | 0.050 | 0.005 | 11.4 |
| | LacZ4 | 311 | 0.151 | | | 204.4 | | | 19.2 |
| | SD4 | 40 | 0.031 | | | 39.7 | | | -3.7 |

**Table S6**: Disease-related proteins and direct protein interaction partners in the protein-protein interaction network. ID, this identifier belongs to the disease-related protein, the underlying cDNA and the clone and refers to the internal clone identifier of table S1. Symbol disease protein, official symbol obtained from the NCBI database. Locus ID disease protein/gene, locuslink identifier of the disease protein/gene in the NCBI database. Symbol partner, official symbol of the direct interacting partner of a disease-related protein obtained from the NCBI database. Locus ID partner, locuslink identifier of the disease-related protein interaction partner obtained from the NCBI database. Mim number, disease identifier obtained from the OMIM (Online Mendelian Inheritance in Man) database. Disease, short description of the human disease causative related to the disease protein.

| ID | Symbol disease protein | Locus ID disease protein | Symbol partner | Locus ID partner | Mim number | Disease |
|---|---|---|---|---|---|---|
| 6 | A2M | 2 | SMAP | 10944 | 103950 | {Alzheimer disease, susceptibility to} (3) |
| 6 | A2M | 2 | SMAP | 10944 | 103950 | Emphysema due to alpha-2-macroglobulin deficiency (1) |
| 16 | ACACA | 31 | CHGB | 1114 | 200350 | Acetyl-CoA carboxylase deficiency (1) |
| 24 | ACTC | 70 | BRP44L | 51660 | 102540 | Cardiomyopathy, dilated, 115200 (3) |
| 24 | ACTC | 70 | BRP44L | 51660 | 102540 | Cardiomyopathy, familial hypertrophic, 192600 (3) |
| 25 | ACTG1 | 71 | ITGB4BP | 3692 | 102560 | Deafness, autosomal dominant 20/26, 604717 (3) |
| 25 | ACTG1 | 71 | PFN2 | 5217 | 102560 | Deafness, autosomal dominant 20/26, 604717 (3) |
| 25 | ACTG1 | 71 | PTPRO | 5800 | 102560 | Deafness, autosomal dominant 20/26, 604717 (3) |
| 25 | ACTG1 | 71 | SH3GL2 | 6456 | 102560 | Deafness, autosomal dominant 20/26, 604717 (3) |
| 25 | ACTG1 | 71 | SIAT6 | 6487 | 102560 | Deafness, autosomal dominant 20/26, 604717 (3) |
| 25 | ACTG1 | 71 | SPUVE | 11098 | 102560 | Deafness, autosomal dominant 20/26, 604717 (3) |
| 26 | ACTG1 | 71 | ITGB4BP | 3692 | 102560 | Deafness, autosomal dominant 20/26, 604717 (3) |
| 26 | ACTG1 | 71 | PFN2 | 5217 | 102560 | Deafness, autosomal dominant 20126, 604717 (3) |
| 26 | ACTG1 | 71 | PTPRO | 5800 | 102560 | Deafness, autosomal dominant 20/26, 604717 (3) |
| 26 | ACTG1 | 71 | SH3GL2 | 6456 | 102560 | Deafness, autosomal dominant 20/26, 604717 (3) |
| 26 | ACTG1 | 71 | SIAT6 | 6487 | 102560 | Deafness, autosomal dominant 20/26, 604717 (3) |
| 26 | ACTG1 | 71 | SPUVE | 11098 | 102560 | Deafness, autosomal dominant 20/26, 604717 (3) |
| 27 | ACTG1 | 71 | ITGB4BP | 3692 | 102560 | Deafness, autosomal dominant 20/26, 604717 (3) |
| 27 | ACTG1 | 71 | PFN2 | 5217 | 102560 | Deafness, autosomal dominant 20/26, 604717 (3) |
| 27 | ACTG1 | 71 | PTPRO | 5800 | 102560 | Deafness, autosomal dominant 20/26, 604717 (3) |
| 27 | ACTG1 | 71 | SH3GL2 | 6456 | 102560 | Deafness, autosomal dominant 20/26, 604717 (3) |
| 27 | ACTG1 | 71 | SIAT6 | 6487 | 102560 | Deafness, autosomal dominant 20/26, 604717 (3) |
| 27 | ACTG1 | 71 | SPUVE | 11098 | 102560 | Deafness, autosomal dominant 20/26, 604717 (3) |
| 28 | ACTG1 | 71 | ITGB4BP | 3692 | 102560 | Deafness, autosomal dominant 20/26, 604717 (3) |
| 28 | ACTG1 | 71 | PFN2 | 5217 | 102560 | Deafness, autosomal dominant 20/26, 604717 (3) |
| 28 | ACTG1 | 71 | PTPRO | 5800 | 102560 | Deafness, autosomal dominant 20/26, 604717 (3) |
| 28 | ACTG1 | 71 | SH3GL2 | 6456 | 102560 | Deafness, autosomal dominant 20/26, 604717 (3) |
| 28 | ACTG1 | 71 | SIAT6 | 6487 | 102560 | Deafness, autosomal dominant 20/26, 604717 (3) |
| 28 | ACTG1 | 71 | SPUVE | 11098 | 102560 | Deafness, autosomal dominant 20/26, 604717 (3) |
| 29 | ACTG1 | 71 | ITGB4BP | 3692 | 102560 | Deafness, autosomal dominant 20/26, 604717 (3) |
| 29 | ACTG1 | 71 | PFN2 | 5217 | 102560 | Deafness, autosomal dominant 20/26, 604717 (3) |
| 29 | ACTG1 | 71 | PTPRO | 5800 | 102560 | Deafness, autosomal dominant 20/26, 604717 (3) |
| 29 | ACTG1 | 71 | SH3GL2 | 6456 | 102560 | Deafness, autosomal dominant 20/26, 604717 (3) |
| 29 | ACTG1 | 71 | SIAT6 | 6487 | 102560 | Deafness, autosomal dominant 20/26, 604717 (3) |
| 29 | ACTG1 | 71 | SPUVE | 11098 | 102560 | Deafness, autosomal dominant 20/26, 604717 (3) |
| 39 | AFP | 174 | RUTBC1 | 9905 | 104150 | [AFP deficiency, congenital] (1) |
| 39 | AFP | 174 | RUTBC1 | 9905 | 104150 | [Hereditary persistence of alpha-fetoprotein] (3) |
| 41 | AGXT | 189 | SNCAIP | 9627 | 604285 | Hyperoxaluria, primary, type 1, 259900 (3) |
| 45 | ALB | 213 | LUC7L2 | 51631 | 103600 | [Dysalbuminemic hyperthyroxinemia] (3) |
| 45 | ALB | 213 | LUC7L2 | 51631 | 103600 | [Dysalbuminemic hyperzincemia], 194470 (1) |
| 45 | ALB | 213 | LUC7L2 | 51631 | 103600 | Analbuminemia (3) |
| 46 | ALDH2 | 217 | C14orf1 | 11161 | 100650 | {?Fetal alcohol syndrome} (1) |
| 46 | ALDH2 | 217 | C14orf1 | 11161 | 100650 | Alcohol intolerance, acute (3) |
| 46 | ALDH2 | 217 | C2orf18 | 54978 | 100650 | {?Fetal alcohol syndrome} (1) |
| 46 | ALDH2 | 217 | C2orf18 | 54978 | 100650 | Alcohol intolerance, acute (3) |
| 46 | ALDH2 | 217 | CRMP1 | 1400 | 100650 | {?Fetal alcohol syndrome} (1) |
| 46 | ALDH2 | 217 | CRMP1 | 1400 | 100650 | Alcohol intolerance, acute (3) |
| 46 | ALDH2 | 217 | HT011 | 55855 | 100650 | {?Fetal alcohol syndrome} (1) |
| 46 | ALDH2 | 217 | HT011 | 55855 | 100650 | Alcohol intolerance, acute (3) |
| 46 | ALDH2 | 217 | ITGB4BP | 3692 | 100650 | {?Fetal alcohol syndrome} (1) |
| 46 | ALDH2 | 217 | ITGB4BP | 3692 | 100650 | Alcohol intolerance, acute (3) |
| 46 | ALDH2 | 217 | MGC15730 | 84966 | 100650 | {?Fetal alcohol syndrome} (1) |
| 46 | ALDH2 | 217 | MGC15730 | 84966 | 100650 | Alcohol intolerance, acute (3) |
| 46 | ALDH2 | 217 | UNC119 | 9094 | 100650 | {?Fetal alcohol syndrome} (1) |
| 46 | ALDH2 | 217 | UNC119 | 9094 | 100650 | Alcohol intolerance, acute (3) |
| 47 | ALDH2 | 217 | C14orf1 | 11161 | 100650 | {?Fetal alcohol syndrome} (1) |
| 47 | ALDH2 | 217 | C14orf1 | 11161 | 100650 | Alcohol intolerance, acute (3) |
| 47 | ALDH2 | 217 | C2orf18 | 54978 | 100650 | {?Fetal alcohol syndrome} (1) |
| 47 | ALDH2 | 217 | C2orf18 | 54978 | 100650 | Alcohol intolerance, acute (3) |
| 47 | ALDH2 | 217 | CRMP1 | 1400 | 100650 | {?Fetal alcohol syndrome} (1) |
| 47 | ALDH2 | 217 | CRMP1 | 1400 | 100650 | Alcohol intolerance, acute (3) |
| 47 | ALDH2 | 217 | HT011 | 55855 | 100650 | {?Fetal alcohol syndrome} (1) |
| 47 | ALDH2 | 217 | HT011 | 55855 | 100650 | Alcohol intolerance, acute (3) |
| 47 | ALDH2 | 217 | ITGB4BP | 3692 | 100650 | {?Fetal alcohol syndrome} (1) |
| 47 | ALDH2 | 217 | ITGB4BP | 3692 | 100650 | Alcohol intolerance, acute (3) |
| 47 | ALDH2 | 217 | MGC15730 | 84966 | 100650 | {?Fetal alcohol syndrome} (1) |
| 47 | ALDH2 | 217 | MGC15730 | 84966 | 100650 | Alcohol intolerance, acute (3) |
| 47 | ALDH2 | 217 | UNC119 | 9094 | 100650 | {?Fetal alcohol syndrome} (1) |
| 47 | ALDH2 | 217 | UNC119 | 9094 | 100650 | Alcohol intolerance, acute (3) |
| 72 | APOA1 | 335 | FLJ10350 | 55700 | 107680 | Amyloidosis, 3 or more types (3) |
| 72 | APOA1 | 335 | FLJ10350 | 55700 | 107680 | ApoA-I and apoC-III deficiency, combined (3) |
| 72 | APOA1 | 335 | FLJ10350 | 55700 | 107680 | Corneal clouding, autosomal recessive (3) |
| 72 | APOA1 | 335 | FLJ10350 | 55700 | 107680 | Hypertriglyceridemia, one form (3) |
| 72 | APOA1 | 335 | FLJ10350 | 55700 | 107680 | Hypoalphalipoproteinemia (3) |
| 72 | APOA1 | 335 | PCMT1 | 5110 | 107680 | Amyloidosis, 3 or more types (3) |
| 72 | APOA1 | 335 | PCMT1 | 5110 | 107680 | ApoA-I and apoC-III deficiency, combined (3) |
| 72 | APOA1 | 335 | PCMT1 | 5110 | 107680 | Corneal clouding, autosomal recessive (3) |
| 72 | APOA1 | 335 | PCMT1 | 5110 | 107680 | Hypertriglyceridemia, one form (3) |
| 72 | APOA1 | 335 | PCMT1 | 5110 | 107680 | Hypoalphalipoproteinemia (3) |
| 72 | APOA1 | 335 | TNFRSF10C | 8794 | 107680 | Amyloidosis, 3 or more types (3) |
| 72 | APOA1 | 335 | TNFRSF10C | 8794 | 107680 | ApoA-I and apoC-III deficiency, combined (3) |
| 72 | APOA1 | 335 | TNFRSF10C | 8794 | 107680 | Corneal clouding, autosomal recessive (3) |
| 72 | APOA1 | 335 | TNFRSF10C | 8794 | 107680 | Hypertriglyceridemia, one form (3) |
| 72 | APOA1 | 335 | TNFRSF10C | 8794 | 107680 | Hypoalphalipoproteinemia (3) |
| 72 | APOA1 | 335 | TOMM20 | 9804 | 107680 | Amyloidosis, 3 or more types (3) |
| 72 | APOA1 | 335 | TOMM20 | 9804 | 107680 | ApoA-I and apoC-III deficiency, combined (3) |
| 72 | APOA1 | 335 | TOMM20 | 9804 | 107680 | Corneal clouding, autosomal recessive (3) |
| 72 | APOA1 | 335 | TOMM20 | 9804 | 107680 | Hypertriglyceridemia, one form (3) |
| 72 | APOA1 | 335 | TOMM20 | 9804 | 107680 | Hypoalphalipoproteinemia (3) |
| 100 | ARSE | 415 | ACK1 | 10188 | 300180 | Chondrodysplasia punctata, X-linked recessive, 302950 (3) |
| 100 | ARSE | 415 | COQ6 | 51004 | 300180 | Chondrodysplasia punctata, X-linked recessive, 302950 (3) |
| 100 | ARSE | 415 | FLJ00277 | | 300180 | Chondrodysplasia punctata, X-linked recessive, 302950 (3) |
| 100 | ARSE | 415 | NDN | 4692 | 300180 | Chondrodysplasia punctata, X-linked recessive, 302950 (3) |
| 101 | ASAH1 | 427 | SETDB1 | 9869 | 228000 | Farber lipogranulomatosis (3) |
| 101 | ASAH1 | 427 | TSC22 | 8848 | 228000 | Farber lipogranulomatosis (3) |
| 107 | ASS | 445 | ASC1p100 | 84164 | 603470 | Citrullinemia, 215700 (3) |
| 107 | ASS | 445 | FTS | 64400 | 603470 | Citrullinemia, 215700 (3) |
| 124 | ATRX | 546 | KIAA1128 | 54462 | 300032 | Alpha-thalassemia myelodysplasia syndrome, somatic, 300448 (3) |
| 124 | ATRX | 546 | KIAA1128 | 54462 | 300032 | Alpha-thalassemia/mental retardation syndrome, 301040 (3) |
| 124 | ATRX | 546 | KIAA1128 | 54462 | 300032 | Juberg-Marsidi syndrome, 309590 (3) |
| 124 | ATRX | 546 | KIAA1128 | 54462 | 300032 | Smith-Fineman-Myers syndrome, 309580 (3) |
| 124 | ATRX | 546 | KIAA1128 | 54462 | 300032 | Sutherland-Haan syndrome, 309470 (3) |
| 124 | ATRX | 546 | KIAA1128 | 54462 | 300032 | Sutherland-Haan syndrome-like, 300465 (3) |
| 124 | ATRX | 546 | KIAA1377 | 57562 | 300032 | Alpha-thalassemia myelodysplasia syndrome, somatic, 300448 (3) |
| 124 | ATRX | 546 | KIAA1377 | 57562 | 300032 | Alpha-thalassemia/mental retardation syndrome, 301040 (3) |
| 124 | ATRX | 546 | KIAA1377 | 57562 | 300032 | Juberg-Marsidi syndrome, 309590 (3) |
| 124 | ATRX | 546 | KIAA1377 | 57562 | 300032 | Smith-Fineman-Myers syndrome, 309580 (3) |
| 124 | ATRX | 546 | KIAA1377 | 57562 | 300032 | Sutherland-Haan syndrome, 309470 (3) |
| 124 | ATRX | 546 | KIAA1377 | 57562 | 300032 | Sutherland-Haan syndrome-like, 300465 (3) |
| 124 | ATRX | 546 | LUC7L2 | 51631 | 300032 | Alpha-thalassemia myelodysplasia syndrome, somatic, 300448 (3) |
| 124 | ATRX | 546 | LUC7L2 | 51631 | 300032 | Alpha-thalassemia/mental retardation syndrome, 301040 (3) |
| 124 | ATRX | 546 | LUC7L2 | 51631 | 300032 | Juberg-Marsidi syndrome, 309590 (3) |
| 124 | ATRX | 546 | LUC7L2 | 51631 | 300032 | Smith-Fineman-Myers syndrome, 309580 (3) |
| 124 | ATRX | 546 | LUC7L2 | 51631 | 300032 | Sutherland-Haan syndrome, 309470 (3) |
| 124 | ATRX | 546 | LUC7L2 | 51631 | 300032 | Sutherland-Haan syndrome-like, 300465 (3) |
| 124 | ATRX | 546 | PTN | 5764 | 300032 | Alpha-thalassemia myelodysplasia syndrome, somatic, 300448 (3) |
| 124 | ATRX | 546 | PTN | 5764 | 300032 | Alpha-thalassemia/mental retardation syndrome, 301040 (3) |
| 124 | ATRX | 546 | PTN | 5764 | 300032 | Juberg-Marsidi syndrome, 309590 (3) |
| 124 | ATRX | 546 | PTN | 5764 | 300032 | Smith-Fineman-Myers syndrome, 309580 (3) |
| 124 | ATRX | 546 | PTN | 5764 | 300032 | Sutherland-Haan syndrome, 309470 (3) |
| 124 | ATRX | 546 | PTN | 5764 | 300032 | Sutherland-Haan syndrome-like, 300465 (3) |
| 124 | ATRX | 546 | PTPN4 | 5775 | 300032 | Alpha-thalassemia myelodysplasia syndrome, somatic, 300448 (3) |
| 124 | ATRX | 546 | PTPN4 | 5775 | 300032 | Alpha-thalassemia/mental retardation syndrome, 301040 (3) |
| 124 | ATRX | 546 | PTPN4 | 5775 | 300032 | Juberg-Marsidi syndrome, 309590 (3) |
| 124 | ATRX | 546 | PTPN4 | 5775 | 300032 | Smith-Fineman-Myers syndrome, 309580 (3) |
| 124 | ATRX | 546 | PTPN4 | 5775 | 300032 | Sutherland-Haan syndrome, 309470 (3) |
| 124 | ATRX | 546 | PTPN4 | 5775 | 300032 | Sutherland-Haan syndrome-like, 300465 (3) |
| 127 | AXIN1 | 8312 | ANP32A | 8125 | 603816 | Hepatocellular carcinoma, 114550 (3) |
| 127 | AXIN1 | 8312 | AXIN1 | 8312 | 603816 | Hepatocellular carcinoma, 114550 (3) |
| 127 | AXIN1 | 8312 | CRMP1 | 1400 | 603816 | Hepatocellular carcinoma, 114550 (3) |
| 127 | AXIN1 | 8312 | EEF1A1 | 1915 | 603816 | Hepatocellular carcinoma, 114550 (3) |
| 127 | AXIN1 | 8312 | GAK | 2580 | 603816 | Hepatocellular carcinoma, 114550 (3) |
| 127 | AXIN1 | 8312 | SDCCAG16 | 10813 | 603816 | Hepatocellular carcinoma, 114550 (3) |
| 127 | AXIN1 | 8312 | SH3GL1 | 6455 | 603816 | Hepatocellular carcinoma, 114550 (3) |
| 128 | AXIN1 | 8312 | ANP32A | 8125 | 603816 | Hepatocellular carcinoma, 114550 (3) |
| 128 | AXIN1 | 8312 | AXIN1 | 8312 | 603816 | Hepatocellular carcinoma, 114550 (3) |
| 128 | AXIN1 | 8312 | CRMP1 | 1400 | 603816 | Hepatocellular carcinoma, 114550 (3) |
| 128 | AXIN1 | 8312 | EEF1A1 | 1915 | 603816 | Hepatocellular carcinoma, 114550 (3) |
| 128 | AXIN1 | 8312 | GAK | 2580 | 603816 | Hepatocellular carcinoma, 114550 (3) |
| 128 | AXIN1 | 8312 | SDCCAG16 | 10813 | 603816 | Hepatocellular carcinoma, 114550 (3) |
| 128 | AXIN1 | 8312 | SH3GL1 | 6455 | 603816 | Hepatocellular carcinoma, 114550 (3) |
| 129 | B2M | 567 | BAT3 | 7917 | 109700 | Hemodialysis-related amyloidosis (1) |
| 129 | B2M | 567 | SPUVE | 11098 | 109700 | Hemodialysis-related amyloidosis (1) |
| 153 | BCR | 613 | MGC15730 | 84966 | 151410 | Leukemia, acute lymphocytic (3) |
| 153 | BCR | 613 | MGC15730 | 84966 | 151410 | Leukemia, chronic myeloid, 608232 (3) |
| 153 | BCR | 613 | TP53 | 7157 | 151410 | Leukemia, acute lymphocytic (3) |
| 153 | BCR | 613 | TP53 | 7157 | 151410 | Leukemia, chronic myeloid, 608232 (3) |
| 153 | BCR | 613 | UNC119 | 9094 | 151410 | Leukemia, acute lymphocytic (3) |
| 153 | BCR | 613 | UNC119 | 9094 | 151410 | Leukemia, chronic myeloid, 608232 (3) |
| 154 | BCS1L | 617 | DDX24 | 57062 | 603647 | GRACILE syndrome, 603358 (3) |
| 154 | BCS1L | 617 | DDX24 | 57062 | 603647 | Leigh syndrome, 256000 (3) |
| 154 | BCS1L | 617 | DDX24 | 57062 | 603647 | Tubulopathy, encephalopathy, and liver failure due to complex III deficiency, 606104 (3) |
| 157 | BLMH | 642 | TRIO | 7204 | 602403 | {Alzheimer disease, susceptibility to} (3) |
| 217 | CA2 | 760 | FLJ10415 | 55139 | 259730 | Renal tubular acidosis-osteopetrosis syndrome (3) |
| 217 | CA2 | 760 | KIAAL1337 | 57540 | 259730 | Renal tubular acidosis-osteopetrosis syndrome (3) |
| 220 | CACNB4 | 785 | CGI-125 | 51003 | 601949 | Ataxia, episodic (3) |
| 220 | CACNB4 | 785 | CGI-125 | 51003 | 601949 | Epilepsy, generalized idiopathic, 600669 (3) |
| 220 | CACNB4 | 785 | CGI-125 | 51003 | 601949 | Epilepsy, juvenile myoclonic, 606904 (3) |
| 220 | CACNB4 | 785 | PTN | 5764 | 601949 | Ataxia, episodic (3) |
| 220 | CACNB4 | 785 | PTN | 5764 | 601949 | Epilepsy, generalized idiopathic, 600669 (3) |
| 220 | CACNB4 | 785 | PTN | 5764 | 601949 | Epilepsy, juvenile myoclonic, 606904 (3) |
| 220 | CACNB4 | 785 | TBL3 | 10607 | 601949 | Ataxia, episodic (3) |
| 220 | CACNB4 | 785 | TBL3 | 10607 | 601949 | Epilepsy, generalized idiopathic, 600669 (3) |
| 220 | CACNB4 | 785 | TBL3 | 10607 | 601949 | Epilepsy, juvenile myoclonic, 606904 (3) |
| 230 | CASP8 | 841 | CASP8 | 841 | 601763 | Autoimmune lymphoproliferative syndrome, type IIB, 607271 (3) |
| 230 | CASP8 | 841 | CHD3 | 1107 | 601763 | Autoimmune lymphoproliferative syndrome, type IIB, 607271 (3) |
| 230 | CASP8 | 841 | TFCP2 | 7024 | 601763 | Autoimmune lymphoproliferative syndrome, type IIB, 607271 (3) |
| 230 | CASP8 | 841 | VIM | 7431 | 601763 | Autoimmune lymphoproliferative syndrome, type IIB, 607271 (3) |
| 231 | CASP8 | 841 | CASP8 | 841 | 601763 | Autoimmune lymphoproliferative syndrome, type IIB, 607271 (3) |
| 231 | CASP8 | 841 | CHD3 | 1107 | 601763 | Autoimmune lymphoproliferative syndrome, type IIB, 607271 (3) |
| 231 | CASP8 | 841 | TFCP2 | 7024 | 601763 | Autoimmune lymphoproliferative syndrome, type IIB, 607271 (3) |
| 231 | CASP8 | 841 | VIM | 7431 | 601763 | Autoimmune lymphoproliferative syndrome, type IIB, 607271 (3) |
| 233 | CBFB | 865 | CHGB | 1114 | 121360 | Myeloid leukemia, acute, M4Eo subtype (3) |
| 254 | CDAN1 | 146059 | PDPK1 | 5170 | 607465 | Anemia, congenital dyserythropoietic, type I, 224120 (3) |
| 262 | CDK4 | 1019 | BAT3 | 7917 | 123829 | Melanoma (3) |
| 262 | CDK4 | 1019 | CAPNS1 | 826 | 123829 | Melanoma (3) |
| 262 | CDK4 | 1019 | CDKN2B | 1030 | 123829 | Melanoma (3) |
| 262 | CDK4 | 1019 | MGC3731 | 79159 | 123829 | Melanoma (3) |
| 262 | CDK4 | 1019 | SETDB1 | 9869 | 123829 | Melanoma (3) |
| 280 | CETP | 1071 | ARPC3 | 10094 | 118470 | [CETP deficiency], 607322 (3) |
| 280 | CETP | 1071 | ARPC3 | 10094 | 118470 | {Longevity, exceptional}, 152430 (3) |
| 297 | CHRNB1 | 1140 | COPS6 | 10980 | 100710 | Myasthenic syndrome, congenital, associated with acetylcholine receptor deficiency, 608931 (3) |
| 297 | CHRNB1 | 1140 | COPS6 | 10980 | 100710 | Myasthenic syndrome, slow-channel congenital, 601462 (3) |
| 302 | CKN1 | 1161 | CBR1 | 873 | 216400 | Cockayne syndrome-1 (3) |
| 302 | CKN1 | 1161 | QP-C | 27089 | 216400 | Cockayne syndrome-1 (3) |
| 303 | CKN1 | 1161 | CBR1 | 873 | 216400 | Cockayne syndrome-1 (3) |
| 303 | CKN1 | 1161 | QP-C | 27089 | 216400 | Cockayne syndrome-1 (3) |
| 313 | COL4A5 | 1287 | RNF10 | 9921 | 303630 | AIport syndrome, 301050 (3) |
| 314 | COL7A1 | 1294 | FBXL2 | 25827 | 120120 | EBD, Bart type, 132000 (3) |
| 314 | COL7A1 | 1294 | FBXL2 | 25827 | 120120 | EBD, localisata variant (3) |
| 314 | COL7A1 | 1294 | FBXL2 | 25827 | 120120 | Epidermolysis bullosa dystrophica, AD, 131750 (3) |
| 314 | COL7A1 | 1294 | FBXL2 | 25827 | 120120 | Epidermolysis bullosa dystrophica, AR, 226600 (3) |
| 314 | COL7A1 | 1294 | FBXL2 | 25827 | 120120 | Epidermolysis bullosa pruriginosa, 604129 (3) |
| 314 | COL7A1 | 1294 | FBXL2 | 25827 | 120120 | Epidermolysis bullosa, pretibial, 131850 (3) |
| 314 | COL7A1 | 1294 | FBXL2 | 25827 | 120120 | Toenail dystrophy, isolated, 607523 (3) |
| 314 | COL7A1 | 1294 | FBXL2 | 25827 | 120120 | Transient bullous of the newborn, 131705 (3) |
| 314 | COL7A1 | 1294 | HSPA8 | 3312 | 120120 | EBD, Bart type, 132000 (3) |
| 314 | COL7A1 | 1294 | HSPA8 | 3312 | 120120 | EBD, localisata variant (3) |
| 314 | COL7A1 | 1294 | HSPA8 | 3312 | 120120 | Epidermolysis bullosa dystrophica, AD, 131750 (3) |
| 314 | COL7A1 | 1294 | HSPA8 | 3312 | 120120 | Epidermolysis bullosa dystrophica, AR, 226600 (3) |
| 314 | COL7A1 | 1294 | HSPA8 | 3312 | 120120 | Epidermolysis bullosa pruriginosa, 604129 (3) |
| 314 | COL7A1 | 1294 | HSPA8 | 3312 | 120120 | Epidermolysis bullosa, pretibial, 131850 (3) |
| 314 | COL7A1 | 1294 | HSPA8 | 3312 | 120120 | Toenail dystrophy, isolated, 607523 (3) |
| 314 | COL7A1 | 1294 | HSPA8 | 3312 | 120120 | Transient bullous of the newborn, 131705 (3) |
| 314 | COL7A1 | 1294 | HSPC055 | 29066 | 120120 | EBD, Bart type, 132000 (3) |
| 314 | COL7A1 | 1294 | HSPC055 | 29066 | 120120 | EBD, localisata variant (3) |
| 314 | COL7A1 | 1294 | HSPC055 | 29066 | 120120 | Epidermolysis bullosa dystrophica, AD, 131750 (3) |
| 314 | COL7A1 | 1294 | HSPC055 | 29066 | 120120 | Epidermolysis bullosa dystrophica, AR, 226600 (3) |
| 314 | COL7A1 | 1294 | HSPC055 | 29066 | 120120 | Epidermolysis bullosa pruriginosa, 604129 (3) |
| 314 | COL7A1 | 1294 | HSPC055 | 29066 | 120120 | Epidermolysis bullosa, pretibial, 131850 (3) |
| 314 | COL7A1 | 1294 | HSPC055 | 29066 | 120120 | Toenail dystrophy, isolated, 607523 (3) |
| 314 | COL7A1 | 1294 | HSPC055 | 29066 | 120120 | Transient bullous of the newborn, 131705 (3) |
| 314 | COL7A1 | 1294 | LOC92799 | 92799 | 120120 | EBD, Bart type, 132000 (3) |
| 314 | COL7A1 | 1294 | LOC92799 | 92799 | 120120 | EBD, localisata variant (3) |
| 314 | COL7A1 | 1294 | LOC92799 | 92799 | 120120 | Epidermolysis bullosa dystrophica, AD, 131750 (3) |
| 314 | COL7A1 | 1294 | LOC92799 | 92799 | 120120 | Epidermolysis bullosa dystrophica, AR, 226600 (3) |
| 314 | COL7A1 | 1294 | LOC92799 | 92799 | 120120 | Epidermolysis bullosa pruriginosa, 604129 (3) |
| 314 | COL7A1 | 1294 | LOC92799 | 92799 | 120120 | Epidermolysis bullosa, pretibial, 131850 (3) |
| 314 | COL7A1 | 1294 | LOC92799 | 92799 | 120120 | Toenail dystrophy, isolated, 607523 (3) |
| 314 | COL7A1 | 1294 | LOC92799 | 92799 | 120120 | Transient bullous of the newborn, 131705 (3) |
| 315 | COL9A2 | 1298 | C4orf14 | 84273 | 120260 | {Intervertebral disc disease, susceptibility to}, 603932 (3) |
| 315 | COL9A2 | 1298 | C4orf14 | 84273 | 120260 | Epiphyseal dysplasia, multiple, type 2, 600204 (3) |
| 315 | COL9A2 | 1298 | FEZ1 | 9638 | 120260 | {Intervertebral disc disease, susceptibility to}, 603932 (3) |
| 315 | COL9A2 | 1298 | FEZ1 | 9638 | 120260 | Epiphyseal dysplasia, multiple, type 2, 600204 (3) |
| 315 | COL9A2 | 1298 | HAP1 | 9001 | 120260 | {Intervertebral disc disease, susceptibility to}, 603932 (3) |
| 315 | COL9A2 | 1298 | HAP1 | 9001 | 120260 | Epiphyseal dysplasia, multiple, type 2, 600204 (3) |
| 318 | COMT | 1312 | LITAF | 9516 | 116790 | {Schizophrenia, susceptibility to}, 181500 (2) |
| 338 | CRELD1 | 78987 | ATP5J2 | 9551 | 607170 | {Atrioventricular septal defect, susceptibility to, 2}, 606217 (3) |
| 338 | CRELD1 | 78987 | ATP5J2 | 9551 | 607170 | Atrioventricular septal defect, partial, with heterotaxy syndrome, 606217 (3) |
| 338 | CRELD1 | 78987 | COPS6 | 10980 | 607170 | {Atrioventricular septal defect, susceptibility to, 2}, 606217 (3) |
| 338 | CRELD1 | 78987 | COPS6 | 10980 | 607170 | Atrioventricular septal defect, partial, with heterotaxy syndrome, 606217 (3) |
| 338 | CRELD1 | 78987 | EEF1G | 1937 | 607170 | {Atrioventricular septal defect, susceptibility to, 2}, 606217 (3) |
| 338 | CRELD1 | 78987 | EEF1G | 1937 | 607170 | Atrioventricular septal defect, partial, with heterotaxy syndrome, 606217 (3) |
| 338 | CRELD1 | 78987 | GDF9 | 2661 | 607170 | {Atrioventricular septal defect, susceptibility to, 2}, 606217 (3) |
| 338 | CRELD1 | 78987 | GDF9 | 2661 | 607170 | Atrioventricular septal defect, partial, with heterotaxy syndrome, 606217 (3) |
| 338 | CRELD1 | 78987 | HTATIP | 10524 | 607170 | {Atrioventricular septal defect, susceptibility to, 2}, 606217 (3) |
| 338 | CRELD1 | 78987 | HTATIP | 10524 | 607170 | Atrioventricular septal defect, partial, with heterotaxy syndrome, 606217 (3) |
| 338 | CRELD1 | 78987 | ITGB4BP | 3692 | 607170 | {Atrioventricular septal defect, susceptibility to, 2}, 606217 (3) |
| 338 | CRELD1 | 78987 | ITGB4BP | 3692 | 607170 | Atrioventricular septal defect, partial, with heterotaxy syndrome, 606217 (3) |
| 338 | CRELD1 | 78987 | LOC222019 | 222019 | 607170 | {Atrioventricular septal defect, susceptibility to, 2}, 606217 (3) |
| 338 | CRELD1 | 78987 | LOC222019 | 222019 | 607170 | Atrioventricular septal defect, partial, with heterotaxy syndrome, 606217 (3) |
| 338 | CRELD1 | 78987 | POLA2 | 23649 | 607170 | {Atrioventricular septal defect, susceptibility to, 2}, 606217 (3) |
| 338 | CRELD1 | 78987 | POLA2 | 23649 | 607170 | Atrioventricular septal defect, partial, with heterotaxy syndrome, 606217 (3) |
| 338 | CRELD1 | 78987 | PTN | 5764 | 607170 | {Atrioventricular septal defect, susceptibility to, 2}, 606217 (3) |
| 338 | CRELD1 | 78987 | PTN | 5764 | 607170 | Atrioventricular septal defect, partial, with heterotaxy syndrome, 606217 (3) |
| 338 | CRELD1 | 78987 | SETDB1 | 9869 | 607170 | {Atrioventricular septal defect, susceptibility to, 2}, 606217 (3) |
| 338 | CRELD1 | 78987 | SETDB1 | 9869 | 607170 | Atrioventricular septal defect, partial, with heterotaxy syndrome, 606217 (3) |
| 339 | CRELD1 | 78987 | ATP5J2 | 9551 | 607170 | {Atrioventricular septal defect, susceptibility to, 2}, 606217 (3) |
| 339 | CRELD1 | 78987 | ATP5J2 | 9551 | 607170 | Atrioventricular septal defect, partial, with heterotaxy syndrome, 606217 (3) |
| 339 | CRELD1 | 78987 | COPS6 | 10980 | 607170 | {Atrioventricular septal defect, susceptibility to, 2}, 606217 (3) |
| 339 | CRELD1 | 78987 | COPS6 | 10980 | 607170 | Atrioventricular septal defect, partial, with heterotaxy syndrome, 606217 (3) |
| 339 | CRELD1 | 78987 | EEF1G | 1937 | 607170 | {Atrioventricular septal defect, susceptibility to, 2}, 606217 (3) |
| 339 | CRELD1 | 78987 | EEF1G | 1937 | 607170 | Atrioventricular septal defect, partial, with heterotaxy syndrome, 606217 (3) |
| 339 | CRELD1 | 78987 | GDF9 | 2661 | 607170 | {Atrioventricular septal defect, susceptibility to, 2}, 606217 (3) |
| 339 | CRELD1 | 78987 | GDF9 | 2661 | 607170 | Atrioventricuiarseptai defect, partial, with heterotaxy syndrome, 606217 (3) |
| 339 | CRELD1 | 78987 | HTATIP | 10524 | 607170 | {Atrioventricular septal defect, susceptibility to, 2}, 606217 (3) |
| 339 | CRELD1 | 78987 | HTATIP | 10524 | 607170 | Atrioventricular septal defect, partial, with heterotaxy syndrome, 606217 (3) |
| 339 | CRELD1 | 78987 | ITGB4BP | 3692 | 607170 | {Atrioventricular septal defect, susceptibility to, 2}, 606217 (3) |
| 339 | CRELD1 | 78987 | ITGB4BP | 3692 | 607170 | Atrioventricular septal defect, partial, with heterotaxy syndrome, 606217 (3) |
| 339 | CRELD1 | 78987 | LOC222019 | 222019 | 607170 | {Atrioventricular septal defect, susceptibility to, 2}. 606217 (3) |
| 339 | CRELD1 | 78987 | LOC222019 | 222019 | 607170 | Atrioventricular septal defect, partial, with heterotaxy syndrome, 606217 (3) |
| 339 | CRELD1 | 78987 | POLA2 | 23649 | 607170 | {Atrioventricular septal defect, susceptibility to, 2}, 606217 (3) |
| 339 | CRELD1 | 78987 | POLA2 | 23649 | 607170 | Atrioventricular septal defect, partial, with heterotaxy syndrome, 606217 (3) |
| 339 | CRELD1 | 78987 | PTN | 5764 | 607170 | {Atrioventricular septal defect, susceptibility to, 2}, 606217 (3) |
| 339 | CRELD1 | 78987 | PTN | 5764 | 607170 | Atrioventricular septal defect, partial, with heterotaxy syndrome, 606217 (3) |
| 339 | CRELD1 | 78987 | SETDB1 | 9869 | 607170 | {Atrioventricular septal defect, susceptibility to, 2}, 606217 (3) |
| 339 | CRELD1 | 78987 | SETDB1 | 9869 | 607170 | Atrioventricular septal defect, partial, with heterotaxy syndrome, 606217 (3) |
| 350 | CTSK | 1513 | FGFR3 | 2261 | 601105 | Pycnodysostosis, 265800 (3) |
| 367 | DCTN1 | 1639 | PGAM1 | 5223 | 601143 | Lower motor neuron disease, progressive, without sensory symptoms, 607641 (3) |
| 370 | DDB1 | 1642 | VAMP3 | 9341 | 600045 | Xeroderma pigmentosum, group E, subtype 2 (1) |
| 388 | DFNA5 | 1687 | PTN | 5764 | 600994 | Deafness, autosomal dominant 5 (3) |
| 394 | DKC1 | 1736 | EEF1G | 1937 | 300126 | Dyskeratosis congenita-1, 305000 (3) |
| 394 | DKC1 | 1736 | EEF1G | 1937 | 300126 | Hoyeraal-Hreidarsson syndrome, 300240 (3) |
| 394 | DKC1 | 1736 | EIF3S5 | 8665 | 300126 | Dyskeratosis congenita-1, 305000 (3) |
| 394 | DKC1 | 1736 | EIF3S5 | 8665 | 300126 | Hoyeraal-Hreidarsson syndrome, 300240 (3) |
| 394 | DKC1 | 1736 | LYPLA2 | 11313 | 300126 | Dyskeratosis congenita-1, 305000 (3) |
| 394 | DKC1 | 1736 | LYPLA2 | 11313 | 300126 | Hoyeraal-Hreidarsson syndrome, 300240 (3) |
| 394 | DKC1 | 1736 | SSR1 | 6745 | 300126 | Dyskeratosis congenita-1, 305000 (3) |
| 394 | DKC1 | 1736 | SSR1 | 6745 | 300126 | Hoyeraal-Hreidarsson syndrome, 300240 (3) |
| 395 | DKC1 | 1736 | EEF1G | 1937 | 300126 | Dyskeratosis congenita-1, 305000 (3) |
| 395 | DKC1 | 1736 | EEF1G | 1937 | 300126 | Hoyeraal-Hreidarsson syndrome, 300240 (3) |
| 395 | DKC1 | 1736 | EIF3S5 | 8665 | 300126 | Dyskeratosis congenita-1, 305000 (3) |
| 395 | DKC1 | 1736 | EIF3S5 | 8665 | 300126 | Hoyeraal-Hreidarsson syndrome, 300240 (3) |
| 395 | DKC1 | 1736 | LYPLA2 | 11313 | 300126 | Dyskeratosis congenita-1, 305000 (3) |
| 395 | DKC1 | 1736 | LYPLA2 | 11313 | 300126 | Hoyeraal-Hreidarsson syndrome, 300240 (3) |
| 395 | DKC1 | 1736 | SSR1 | 6745 | 300126 | Dyskeratosis congenita-1, 305000 (3) |
| 395 | DKC1 | 1736 | SSR1 | 6745 | 300126 | Hoyeraal-Hreidarsson syndrome, 300240 (3) |
| 429 | DMPK | 1760 | GABARAP | 11337 | 605377 | Myotonic dystrophy, 160900 (3) |
| 455 | DRPLA | 1822 | LOC222019 | 222019 | 607462 | Dentatorubro-pallidoluysian atrophy, 125370 (3) |
| 455 | DRPLA | 1822 | MYST2 | 11143 | 607462 | Dentatorubro-pallidoluysian atrophy, 125370 (3) |
| 455 | DRPLA | 1822 | TLE1 | 7088 | 607462 | Dentatorubro-pallidoluysian atrophy, 125370 (3) |
| 455 | DRPLA | 1822 | VIM | 7431 | 607462 | Dentatorubro-pallidoluysian atrophy, 125370 (3) |
| 456 | DRPLA | 1822 | LOC222019 | 222019 | 607462 | Dentatorubro-pallidoluysian atrophy, 125370 (3) |
| 456 | DRPLA | 1822 | MYST2 | 11143 | 607462 | Dentatorubro-pallidoluysian atrophy, 125370 (3) |
| 456 | DRPLA | 1822 | TLE1 | 7088 | 607462 | Dentatorubro-pallidoluysian atrophy, 125370 (3) |
| 456 | DRPLA | 1822 | VIM | 7431 | 607462 | Dentatorubro-pallidoluysian atrophy, 125370 (3) |
| 486 | EGR2 | 1959 | ACP5 | 54 | 129010 | Charcot-Marie-Tooth disease, type 1D, 607678 (3) |
| 486 | EGR2 | 1959 | ACP5 | 54 | 129010 | Dejerine-Sottas neuropathy, 145900 (3) |
| 486 | EGR2 | 1959 | ACP5 | 54 | 129010 | Neuropathy, congenital hypomyelinating, 1, 605253 (3) |
| 486 | EGR2 | 1959 | CGI-125 | 51003 | 129010 | Charcot-Marie-Tooth disease, type 1D, 607678 (3) |
| 486 | EGR2 | 1959 | CGI-125 | 51003 | 129010 | Dejerine-Sottas neuropathy, 145900 (3) |
| 486 | EGR2 | 1959 | CGI-125 | 51003 | 129010 | Neuropathy, congenital hypomyelinating, 1, 605253 (3) |
| 487 | EIF2B1 | 1967 | ARF4L | 379 | 606686 | Leukoencephalopathy with vanishing white matter, 603896 (3) |
| 487 | EIF2B1 | 1967 | MRPL4 | 51073 | 606686 | Leukoencephalopathy with vanishing white matter, 603896 (3) |
| 488 | EIF2B2 | 8892 | CCT3 | 7203 | 606454 | Leukoencephalopathy with vanishing white matter, 603896 (3) |
| 488 | EIF2B2 | 8892 | CCT3 | 7203 | 606454 | Ovarioleukodystrophy, 603896 (3) |
| 489 | EIF2B3 | 8891 | GOLPH2 | 51280 | 606273 | Leukoencephalopathy with vanishing white matter, 603896 (3) |
| 489 | EIF2B3 | 8891 | NDUFB8 | 4714 | 606273 | Leukoencephalopathy with vanishing white matter, 603896 (3) |
| 489 | EIF2B3 | 8891 | STC2 | 8614 | 606273 | Leukoencephalopathy with vanishing white matter, 603896 (3) |
| 490 | EIF2B3 | 8891 | GOLPH2 | 51280 | 606273 | Leukoencephalopathy with vanishing white matter, 603896 (3) |
| 490 | EIF2B3 | 8891 | NDUFB8 | 4714 | 606273 | Leukoencephalopathy with vanishing white matter, 603896 (3) |
| 490 | EIF2B3 | 8891 | STC2 | 8614 | 606273 | Leukoencephalopathy with vanishing white matter, 603896 (3) |
| 502 | ELAC2 | 60528 | ASC1p100 | 84164 | 605367 | {Prostate cancer, susceptibility to}, 176807 (3) |
| 502 | ELAC2 | 60528 | CUTL1 | 1523 | 605367 | {Prostate cancer, susceptibility to}, 176807 (3) |
| 502 | ELAC2 | 60528 | GOLPH2 | 51280 | 605367 | {Prostate cancer, susceptibility to}, 176807 (3) |
| 502 | ELAC2 | 60528 | NDUFA7 | 4701 | 605367 | {Prostate cancer, susceptibility to}, 176807 (3) |
| 505 | EMD | 2010 | COPS6 | 10980 | 300384 | Emery-Dreifuss muscular dystrophy, 310300 (3) |
| 505 | EMD | 2010 | DPPA4 | 55211 | 300384 | Emery-Dreifuss muscular dystrophy, 310300 (3) |
| 505 | EMD | 2010 | SH3GL2 | 6456 | 300384 | Emery-Dreifuss muscular dystrophy, 310300 (3) |
| 505 | EMD | 2010 | SH3GL3 | 6457 | 300384 | Emery-Dreifuss muscular dystrophy, 310300 (3) |
| 514 | EP300 | 2033 | COPS6 | 10980 | 602700 | Colorectal cancer, 114500 (3) |
| 526 | ETHE1 | 23474 | ATP6V1H | 51606 | 608451 | Ethylmalonic encephalopathy, 602473 (3) |
| 526 | ETHE1 | 23474 | MGC15730 | 84966 | 608451 | Ethylmalonic encephalopathy, 602473 (3) |
| 526 | ETHE1 | 23474 | RIF1 | 55791 | 608451 | Ethylmalonic encephalopathy, 602473 (3) |
| 527 | ETHE1 | 23474 | ATP6V1 H | 51606 | 608451 | Ethylmalonic encephalopathy, 602473 (3) |
| 527 | ETHE1 | 23474 | MGC15730 | 84966 | 608451 | Ethylmalonic encephalopathy, 602473 (3) |
| 527 | ETHE1 | 23474 | RIF1 | 55791 | 608451 | Ethylmalonic encephalopathy, 602473 (3) |
| 530 | F10 | 2159 | HIST1H1C | 3006 | 227600 | Factor X deficiency (3) |
| 530 | F10 | 2159 | MGST3 | 4259 | 227600 | Factor X deficiency (3) |
| 530 | F10 | 2159 | PRKAB1 | 5564 | 227600 | Factor X deficiency (3) |
| 537 | FANCF | 2188 | MARK3 | 4140 | 603467 | Fanconi anemia, complementation group F (3) |
| 537 | FANCF | 2188 | OLFM2 | 93145 | 603467 | Fanconi anemia, complementation group F (3) |
| 543 | FBP1 | 2203 | ASC1p100 | 84164 | 229700 | Fructose-bisphosphatase deficiency (1) |
| 543 | FBP1 | 2203 | ATP5J2 | 9551 | 229700 | Fructose-bisphosphatase deficiency (1) |
| 543 | FBP1 | 2203 | FBP1 | 2203 | 229700 | Fructose-bisphosphatase deficiency (1) |
| 543 | FBP1 | 2203 | HSPA8 | 3312 | 229700 | Fructose-bisphosphatase deficiency (1) |
| 550 | FCGR2B | 2213 | C14orf1 | 11161 | 604590 | Lymphoma, progression of (3) |
| 555 | FGFR3 | 2261 | ATF3 | 467 | 134934 | Achondropiasia, 100800 (3) |
| 555 | FGFR3 | 2261 | ATF3 | 467 | 134934 | Bladder cancer, 109800 (3) |
| 555 | FGFR3 | 2261 | ATF3 | 467 | 134934 | Cervical cancer, somatic, 603956 (3) |
| 555 | FGFR3 | 2261 | ATF3 | 467 | 134934 | Colorectal cancer, somatic, 109800 (3) |
| 555 | FGFR3 | 2261 | ATF3 | 467 | 134934 | Crouzon syndrome with acanthosis nigricans (3) |
| 555 | FGFR3 | 2261 | ATF3 | 467 | 134934 | Hypochondroplasia, 146000 (3) |
| 555 | FGFR3 | 2261 | ATF3 | 467 | 134934 | Muenke syndrome, 602849 (3) |
| 555 | FGFR3 | 2261 | ATF3 | 467 | 134934 | Thanatophoric dysplasia, types I and II, 187600 (3) |
| 555 | FGFR3 | 2261 | C1orf16 | 9887 | 134934 | Achondroplasia, 100800 (3) |
| 555 | FGFR3 | 2261 | C1orf16 | 9887 | 134934 | Bladder cancer, 109800 (3) |
| 555 | FGFR3 | 2261 | C1orf16 | 9887 | 134934 | Cervical cancer, somatic, 603956 (3) |
| 555 | FGFR3 | 2261 | C1orf16 | 9887 | 134934 | Colorectal cancer, somatic, 109800 (3) |
| 555 | FGFR3 | 2261 | C1orf16 | 9887 | 134934 | Crouzon syndrome with acanthosis nigricans (3) |
| 555 | FGFR3 | 2261 | C1orf16 | 9887 | 134934 | Hypochondroplasia, 146000 (3) |
| 555 | FGFR3 | 2261 | C1orf16 | 9887 | 134934 | Muenke syndrome, 602849 (3) |
| 555 | FGFR3 | 2261 | C1orf16 | 9887 | 134934 | Thanatophoric dysplasia, types I and II, 187600 (3) |
| 555 | FGFR3 | 2261 | CENTD2 | 116985 | 134934 | Achondroplasia, 100800 (3) |
| 555 | FGFR3 | 2261 | CENTD2 | 116985 | 134934 | Bladder cancer, 109800 (3) |
| 555 | FGFR3 | 2261 | CENTD2 | 116985 | 134934 | Cervical cancer, somatic, 603956 (3) |
| 555 | FGFR3 | 2261 | CENTD2 | 116985 | 134934 | Colorectal cancer, somatic, 109800 (3) |
| 555 | FGFR3 | 2261 | CENTD2 | 116985 | 134934 | Crouzon syndrome with acanthosis nigricans (3) |
| 555 | FGFR3 | 2261 | CENTD2 | 116985 | 134934 | Hypochondroplasia, 146000 (3) |
| 555 | FGFR3 | 2261 | CENTD2 | 116985 | 134934 | Muenke syndrome, 602849 (3) |
| 555 | FGFR3 | 2261 | CENTD2 | 116985 | 134934 | Thanatophoric dysplasia, types I and II, 187600 (3) |
| 555 | FGFR3 | 2261 | CHGB | 1114 | 134934 | Achondroplasia, 100800 (3) |
| 555 | FGFR3 | 2261 | CHGB | 1114 | 134934 | Bladder cancer, 109800 (3) |
| 555 | FGFR3 | 2261 | CHGB | 1114 | 134934 | Cervical cancer, somatic, 603956 (3) |
| 555 | FGFR3 | 2261 | CHGB | 1114 | 134934 | Colorectal cancer, somatic, 109800 (3) |
| 555 | FGFR3 | 2261 | CHGB | 1114 | 134934 | Crouzon syndrome with acanthosis nigricans (3) |
| 555 | FGFR3 | 2261 | CHGB | 1114 | 134934 | Hypochondroplasia, 146000 (3) |
| 555 | FGFR3 | 2261 | CHGB | 1114 | 134934 | Muenke syndrome, 602849 (3) |
| 555 | FGFR3 | 2261 | CHGB | 1114 | 134934 | Thanatophoric dysplasia, types I and II, 187600 (3) |
| 555 | FGFR3 | 2261 | CTSK | 1513 | 134934 | Achondroplasia, 100800 (3) |
| 555 | FGFR3 | 2261 | CTSK | 1513 | 134934 | Bladder cancer, 109800 (3) |
| 555 | FGFR3 | 2261 | CTSK | 1513 | 134934 | Cervical cancer, somatic, 603956 (3) |
| 555 | FGFR3 | 2261 | CTSK | 1513 | 134934 | Colorectal cancer, somatic, 109800 (3) |
| 555 | FGFR3 | 2261 | CTSK | 1513 | 134934 | Crouzon syndrome with acanthosis nigricans (3) |
| 555 | FGFR3 | 2261 | CTSK | 1513 | 134934 | Hypochondroplasia, 146000 (3) |
| 555 | FGFR3 | 2261 | CTSK | 1513 | 134934 | Muenke syndrome, 602849 (3) |
| 555 | FGFR3 | 2261 | CTSK | 1513 | 134934 | Thanatophoric dysplasia, types I and II, 187600 (3) |
| 555 | FGFR3 | 2261 | FLJ10324 | 55698 | 134934 | Achondroplasia, 100800 (3) |
| 555 | FGFR3 | 2261 | FLJ10324 | 55698 | 134934 | Bladder cancer, 109800 (3) |
| 555 | FGFR3 | 2261 | FLJ10324 | 55698 | 134934 | Cervical cancer, somatic, 603956 (3) |
| 555 | FGFR3 | 2261 | FLJ10324 | 55698 | 134934 | Colorectal cancer, somatic, 109800 (3) |
| 555 | FGFR3 | 2261 | FLJ10324 | 55698 | 134934 | Crouzon syndrome with acanthosis nigricans (3) |
| 555 | FGFR3 | 2261 | FLJ10324 | 55698 | 134934 | Hypochondroplasia, 146000 (3) |
| 555 | FGFR3 | 2261 | FLJ10324 | 55698 | 134934 | Muenke syndrome, 602849 (3) |
| 555 | FGFR3 | 2261 | FLJ10324 | 55698 | 134934 | Thanatophoric dysplasia, types I and II, 187600 (3) |
| 555 | FGFR3 | 2261 | FLJ22624 | 79866 | 134934 | Achondroplasia, 100800 (3) |
| 555 | FGFR3 | 2261 | FLJ22624 | 79866 | 134934 | Bladder cancer, 109800 (3) |
| 555 | FGFR3 | 2261 | FLJ22624 | 79866 | 134934 | Cervical cancer, somatic, 603956 (3) |
| 555 | FGFR3 | 2261 | FLJ22624 | 79866 | 134934 | Colorectal cancer, somatic, 109800 (3) |
| 555 | FGFR3 | 2261 | FLJ22624 | 79866 | 134934 | Crouzon syndrome with acanthosis nigricans (3) |
| 555 | FGFR3 | 2261 | FLJ22624 | 79866 | 134934 | Hypochondroplasia, 146000 (3) |
| 555 | FGFR3 | 2261 | FLJ22624 | 79866 | 134934 | Muenke syndrome, 602849 (3) |
| 555 | FGFR3 | 2261 | FLJ22624 | 79866 | 134934 | Thanatophoric dysplasia, types I and II, 187600 (3) |
| 555 | FGFR3 | 2261 | FLJ33084 | 149483 | 134934 | Achondroplasia, 100800 (3) |
| 555 | FGFR3 | 2261 | FLJ33084 | 149483 | 134934 | Bladder cancer, 109800 (3) |
| 555 | FGFR3 | 2261 | FLJ33084 | 149483 | 134934 | Cervical cancer, somatic, 603956 (3) |
| 555 | FGFR3 | 2261 | FLJ33084 | 149483 | 134934 | Colorectal cancer, somatic, 109800 (3) |
| 555 | FGFR3 | 2261 | FLJ33084 | 149483 | 134934 | Crouzon syndrome with acanthosis nigricans (3) |
| 555 | FGFR3 | 2261 | FLJ33084 | 149483 | 134934 | Hypochondroplasia, 146000 (3) |
| 555 | FGFR3 | 2261 | FLJ33084 | 149483 | 134934 | Muenke syndrome, 602849 (3) |
| 555 | FGFR3 | 2261 | FLJ33084 | 149483 | 134934 | Thanatophoric dysplasia, types I and II, 187600 (3) |
| 555 | FGFR3 | 2261 | GTF3C1 | 2975 | 134934 | Achondroplasia, 100800 (3) |
| 555 | FGFR3 | 2261 | GTF3C1 | 2975 | 134934 | Bladder cancer, 109800 (3) |
| 555 | FGFR3 | 2261 | GTF3C1 | 2975 | 134934 | Cervical cancer, somatic, 603956 (3) |
| 555 | FGFR3 | 2261 | GTF3C1 | 2975 | 134934 | Colorectal cancer, somatic, 109800 (3) |
| 555 | FGFR3 | 2261 | GTF3C1 | 2975 | 134934 | Crouzon syndrome with acanthosis nigricans (3) |
| 555 | FGFR3 | 2261 | GTF3C1 | 2975 | 134934 | Hypochondroplasia, 146000 (3) |
| 555 | FGFR3 | 2261 | GTF3C1 | 2975 | 134934 | Muenke syndrome, 602849 (3) |
| 555 | FGFR3 | 2261 | GTF3C1 | 2975 | 134934 | Thanatophoric dysplasia, types and II, 187600 (3) |
| 555 | FGFR3 | 2261 | HBZ | 3050 | 134934 | Achondroplasia, 100800 (3) |
| 555 | FGFR3 | 2261 | HBZ | 3050 | 134934 | Bladder cancer, 109800 (3) |
| 555 | FGFR3 | 2261 | HBZ | 3050 | 134934 | Cervical cancer, somatic, 603956 (3) |
| 555 | FGFR3 | 2261 | HBZ | 3050 | 134934 | Colorectal cancer, somatic, 109800 (3) |
| 555 | FGFR3 | 2261 | HBZ | 3050 | 134934 | Crouzon syndrome with acanthosis nigricans (3) |
| 555 | FGFR3 | 2261 | HBZ | 3050 | 134934 | Hypochondroplasia, 146000 (3) |
| 555 | FGFR3 | 2261 | HBZ | 3050 | 134934 | Muenke syndrome, 602849 (3) |
| 555 | FGFR3 | 2261 | HBZ | 3050 | 134934 | Thanatophoric dysplasia, types I and II, 187600 (3) |
| 555 | FGFR3 | 2261 | HNRPL | 3191 | 134934 | Achondroplasia, 100800 (3) |
| 555 | FGFR3 | 2261 | HNRPL | 3191 | 134934 | Bladder cancer, 109800 (3) |
| 555 | FGFR3 | 2261 | HNRPL | 3191 | 134934 | Cervical cancer, somatic, 603956 (3) |
| 555 | FGFR3 | 2261 | HNRPL | 3191 | 134934 | Colorectal cancer, somatic, 109800 (3) |
| 555 | FGFR3 | 2261 | HNRPL | 3191 | 134934 | Crouzon syndrome with acanthosis nigricans (3) |
| 555 | FGFR3 | 2261 | HNRPL | 3191 | 134934 | Hypochondroplasia, 146000 (3) |
| 555 | FGFR3 | 2261 | HNRPL | 3191 | 134934 | Muenke syndrome, 602849 (3) |
| 555 | FGFR3 | 2261 | HNRPL | 3191 | 134934 | Thanatophoric dysplasia, types I and II, 187600 (3) |
| 555 | FGFR3 | 2261 | KIAA1377 | 57562 | 134934 | Achondroplasia, 100800 (3) |
| 555 | FGFR3 | 2261 | KIAA1377 | 57562 | 134934 | Bladder cancer, 109800 (3) |
| 555 | FGFR3 | 2261 | KIAA1377 | 57562 | 134934 | Cervical cancer, somatic, 603956 (3) |
| 555 | FGFR3 | 2261 | KIAA1377 | 57562 | 134934 | Colorectal cancer, somatic, 109800 (3) |
| 555 | FGFR3 | 2261 | KIAA1377 | 57562 | 134934 | Crouzon syndrome with acanthosis nigricans (3) |
| 555 | FGFR3 | 2261 | KIAA1377 | 57562 | 134934 | Hypochondroplasia, 146000 (3) |
| 555 | FGFR3 | 2261 | KIAA1377 | 57562 | 134934 | Muenke syndrome, 602849 (3) |
| 555 | FGFR3 | 2261 | KIAA1377 | 57562 | 134934 | Thanatophoric dysplasia, types I and II, 187600 (3) |
| 555 | FGFR3 | 2261 | KRT8 | 3856 | 134934 | Achondroplasia, 100800 (3) |
| 555 | FGFR3 | 2261 | KRT8 | 3856 | 134934 | Bladder cancer, 109800 (3) |
| 555 | FGFR3 | 2261 | KRT8 | 3856 | 134934 | Cervical cancer, somatic, 603956 (3) |
| 555 | FGFR3 | 2261 | KRT8 | 3856 | 134934 | Colorectal cancer, somatic, 109800 (3) |
| 555 | FGFR3 | 2261 | KRT8 | 3856 | 134934 | Crouzon syndrome with acanthosis nigricans (3) |
| 555 | FGFR3 | 2261 | KRT8 | 3856 | 134934 | Hypochondroplasia, 146000 (3) |
| 555 | FGFR3 | 2261 | KRT8 | 3856 | 134934 | Muenke syndrome, 602849 (3) |
| 555 | FGFR3 | 2261 | KRT8 | 3856 | 134934 | Thanatophoric dysplasia, types I and II, 187600 (3) |
| 555 | FGFR3 | 2261 | NDUFS6 | 4726 | 134934 | Achondroplasia, 100800 (3) |
| 555 | FGFR3 | 2261 | NDUFS6 | 4726 | 134934 | Bladder cancer, 109800 (3) |
| 555 | FGFR3 | 2261 | NDUFS6 | 4726 | 134934 | Cervical cancer, somatic, 603956 (3) |
| 555 | FGFR3 | 2261 | NDUFS6 | 4726 | 134934 | Colorectal cancer, somatic, 109800 (3) |
| 555 | FGFR3 | 2261 | NDUFS6 | 4726 | 134934 | Crouzon syndrome with acanthosis nigricans (3) |
| 555 | FGFR3 | 2261 | NDUFS6 | 4726 | 134934 | Hypochondroplasia, 146000 (3) |
| 555 | FGFR3 | 2261 | NDUFS6 | 4726 | 134934 | Muenke syndrome, 602849 (3) |
| 555 | FGFR3 | 2261 | NDUFS6 | 4726 | 134934 | Thanatophoric dysplasia, types I and II, 187600 (3) |
| 555 | FGFR3 | 2261 | POLA2 | 23649 | 134934 | Achondropiasia, 100800 (3) |
| 555 | FGFR3 | 2261 | POLA2 | 23649 | 134934 | Bladder cancer, 109800 (3) |
| 555 | FGFR3 | 2261 | POLA2 | 23649 | 134934 | Cervical cancer, somatic, 603956 (3) |
| 555 | FGFR3 | 2261 | POLA2 | 23649 | 134934 | Colorectal cancer, somatic, 109800 (3) |
| 555 | FGFR3 | 2261 | POLA2 | 23649 | 134934 | Crouzon syndrome with acanthosis nigricans (3) |
| 555 | FGFR3 | 2261 | POLA2 | 23649 | 134934 | Hypochondroplasia, 146000 (3) |
| 555 | FGFR3 | 2261 | POLA2 | 23649 | 134934 | Muenke syndrome, 602849 (3) |
| 555 | FGFR3 | 2261 | POLA2 | 23649 | 134934 | Thanatophoric dysplasia, types I and II, 187600 (3) |
| 555 | FGFR3 | 2261 | RNF130 | 55819 | 134934 | Achondroplasia, 100800 (3) |
| 555 | FGFR3 | 2261 | RNF130 | 55819 | 134934 | Bladder cancer, 109800 (3) |
| 555 | FGFR3 | 2261 | RNF130 | 55819 | 134934 | Cervical cancer, somatic, 603956 (3) |
| 555 | FGFR3 | 2261 | RNF130 | 55819 | 134934 | Colorectal cancer, somatic, 109800 (3) |
| 555 | FGFR3 | 2261 | RNF130 | 55819 | 134934 | Crouzon syndrome with acanthosis nigricans (3) |
| 555 | FGFR3 | 2261 | RNF130 | 55819 | 134934 | Hypochondroplasia, 146000 (3) |
| 555 | FGFR3 | 2261 | RNF130 | 55819 | 134934 | Muenke syndrome, 602849 (3) |
| 555 | FGFR3 | 2261 | RNF130 | 55819 | 134934 | Thanatophoric dysplasia, types I and II, 187600 (3) |
| 555 | FGFR3 | 2261 | RPL8 | 6132 | 134934 | Achondroplasia, 100800 (3) |
| 555 | FGFR3 | 2261 | RPL8 | 6132 | 134934 | Bladder cancer, 109800 (3) |
| 555 | FGFR3 | 2261 | RPL8 | 6132 | 134934 | Cervical cancer, somatic, 603956 (3) |
| 555 | FGFR3 | 2261 | RPL8 | 6132 | 134934 | Colorectal cancer, somatic, 109800 (3) |
| 555 | FGFR3 | 2261 | RPL8 | 6132 | 134934 | Crouzon syndrome with acanthosis nigricans (3) |
| 555 | FGFR3 | 2261 | RPL8 | 6132 | 134934 | Hypochondroplasia, 146000 (3) |
| 555 | FGFR3 | 2261 | RPL8 | 6132 | 134934 | Muenke syndrome, 602849 (3) |
| 555 | FGFR3 | 2261 | RPL8 | 6132 | 134934 | Thanatophoric dysplasia, types I and II, 187600 (3) |
| 555 | FGFR3 | 2261 | SLC25A6 | 293 | 134934 | Achondroplasia, 100800 (3) |
| 555 | FGFR3 | 2261 | SLC25A6 | 293 | 134934 | Bladder cancer, 109800 (3) |
| 555 | FGFR3 | 2261 | SLC25A6 | 293 | 134934 | Cervical cancer, somatic, 603956 (3) |
| 555 | FGFR3 | 2261 | SLC25A6 | 293 | 134934 | Colorectal cancer, somatic, 109800 (3) |
| 555 | FGFR3 | 2261 | SLC25A6 | 293 | 134934 | Crouzon syndrome with acanthosis nigricans (3) |
| 555 | FGFR3 | 2261 | SLC25A6 | 293 | 134934 | Hypochondroplasia, 146000 (3) |
| 555 | FGFR3 | 2261 | SLC25A6 | 293 | 134934 | Muenke syndrome, 602849 (3) |
| 555 | FGFR3 | 2261 | SLC25A6 | 293 | 134934 | Thanatophoric dysplasia, types I and II, 187600 (3) |
| 555 | FGFR3 | 2261 | SMA3 | 10571 | 134934 | Achondroplasia, 100800 (3) |
| 555 | FGFR3 | 2261 | SMA3 | 10571 | 134934 | Bladder cancer, 109800 (3) |
| 555 | FGFR3 | 2261 | SMA3 | 10571 | 134934 | Cervical cancer, somatic, 603956 (3) |
| 555 | FGFR3 | 2261 | SMA3 | 10571 | 134934 | Colorectal cancer, somatic, 109800 (3) |
| 555 | FGFR3 | 2261 | SMA3 | 10571 | 134934 | Crouzon syndrome with acanthosis nigricans (3) |
| 555 | FGFR3 | 2261 | SMA3 | 10571 | 134934 | Hypochondroplasia, 146000 (3) |
| 555 | FGFR3 | 2261 | SMA3 | 10571 | 134934 | Muenke syndrome, 602849 (3) |
| 555 | FGFR3 | 2261 | SMA3 | 10571 | 134934 | Thanatophoric dysplasia, types I and II, 187600 (3) |
| 556 | FGFR3 | 2261 | ATF3 | 467 | 134934 | Achondroplasia, 100800 (3) |
| 556 | FGFR3 | 2261 | ATF3 | 467 | 134934 | Bladder cancer, 109800 (3) |
| 556 | FGFR3 | 2261 | ATF3 | 467 | 134934 | Cervical cancer, somatic, 603956 (3) |
| 556 | FGFR3 | 2261 | ATF3 | 467 | 134934 | Colorectal cancer, somatic, 109800 (3) |
| 556 | FGFR3 | 2261 | ATF3 | 467 | 134934 | Crouzon syndrome with acanthosis nigricans (3) |
| 556 | FGFR3 | 2261 | ATF3 | 467 | 134934 | Hypochondroplasia, 146000 (3) |
| 556 | FGFR3 | 2261 | ATF3 | 467 | 134934 | Muenke syndrome, 602849 (3) |
| 556 | FGFR3 | 2261 | ATF3 | 467 | 134934 | Thanatophoric dysplasia, types I and II, 187600 (3) |
| 556 | FGFR3 | 2261 | Clorf16 | 9887 | 134934 | Achondroplasia, 100800 (3) |
| 556 | FGFR3 | 2261 | Clorfl6 6 | 9887 | 134934 | Bladder cancer, 109800 (3) |
| 556 | FGFR3 | 2261 | C1orf16 | 9887 | 134934 | Cervical cancer, somatic, 603956 (3) |
| 556 | FGFR3 | 2261 | C1orf16 | 9887 | 134934 | Colorectal cancer, somatic, 109800 (3) |
| 556 | FGFR3 | 2261 | C1orf16 | 9887 | 134934 | Crouzon syndrome with acanthosis nigricans (3) |
| 556 | FGFR3 | 2261 | C1orf16 | 9887 | 134934 | Hypochondroplasia, 146000 (3) |
| 556 | FGFR3 | 2261 | C1orf16 | 9887 | 134934 | Muenke syndrome, 602849 (3) |
| 556 | FGFR3 | 2261 | C1orf16 | 9887 | 134934 | Thanatophoric dysplasia, types I and II, 187600 (3) |
| 556 | FGFR3 | 2261 | CENTD2 | 116985 | 134934 | Achondroplasia, 100800 (3) |
| 556 | FGFR3 | 2261 | CENTD2 | 116985 | 134934 | Bladder cancer, 109800 (3) |
| 556 | FGFR3 | 2261 | CENTD2 | 116985 | 134934 | Cervical cancer, somatic, 603956 (3) |
| 556 | FGFR3 | 2261 | CENTD2 | 116985 | 134934 | Colorectal cancer, somatic, 109800 (3) |
| 556 | FGFR3 | 2261 | CENTD2 | 116985 | 134934 | Crouzon syndrome with acanthosis nigricans (3) |
| 556 | FGFR3 | 2261 | CENTD2 | 116985 | 134934 | Hypochondroplasia, 146000 (3) |
| 556 | FGFR3 | 2261 | CENTD2 | 116985 | 134934 | Muenke syndrome, 602849 (3) |
| 556 | FGFR3 | 2261 | CENTD2 | 116985 | 134934 | Thanatophoric dysplasia, types I and II, 187600 (3) |
| 556 | FGFR3 | 2261 | CHGB | 1114 | 134934 | Achondroplasia, 100800 (3) |
| 556 | FGFR3 | 2261 | CHGB | 1114 | 134934 | Bladder cancer, 109800 (3) |
| 556 | FGFR3 | 2261 | CHGB | 1114 | 134934 | Cervical cancer, somatic, 603956 (3) |
| 556 | FGFR3 | 2261 | CHGB | 1114 | 134934 | Colorectal cancer, somatic, 109800 (3) |
| 556 | FGFR3 | 2261 | CHGB | 1114 | 134934 | Crouzon syndrome with acanthosis nigricans (3) |
| 556 | FGFR3 | 2261 | CHGB | 1114 | 134934 | Hypochondroplasia, 146000 (3) |
| 556 | FGFR3 | 2261 | CHGB | 1114 | 134934 | Muenke syndrome, 602849 (3) |
| 556 | FGFR3 | 2261 | CHGB | 1114 | 134934 | Thanatophoric dysplasia, types I and II, 187600 (3) |
| 556 | FGFR3 | 2261 | CTSK | 1513 | 134934 | Achondroplasia, 100800 (3) |
| 556 | FGFR3 | 2261 | CTSK | 1513 | 134934 | Bladder cancer, 109800 (3) |
| 556 | FGFR3 | 2261 | CTSK | 1513 | 134934 | Cervical cancer, somatic, 603956 (3) |
| 556 | FGFR3 | 2261 | CTSK | 1513 | 134934 | Colorectal cancer, somatic, 109800 (3) |
| 556 | FGFR3 | 2261 | CTSK | 1513 | 134934 | Crouzon syndrome with acanthosis nigricans (3) |
| 556 | FGFR3 | 2261 | CTSK | 1513 | 134934 | Hypochondroplasia, 146000 (3) |
| 556 | FGFR3 | 2261 | CTSK | 1513 | 134934 | Muenke syndrome, 602849 (3) |
| 556 | FGFR3 | 2261 | CTSK | 1513 | 134934 | Thanatophoric dysplasia, types I and II, 187600 (3) |
| 556 | FGFR3 | 2261 | FLJ10324 | 55698 | 134934 | Achondroplasia, 100800 (3) |
| 556 | FGFR3 | 2261 | FLJ10324 | 55698 | 134934 | Bladder cancer, 109800 (3) |
| 556 | FGFR3 | 2261 | FLJ10324 | 55698 | 134934 | Cervical cancer, somatic, 603956 (3) |
| 556 | FGFR3 | 2261 | FLJ10324 | 55698 | 134934 | Colorectal cancer, somatic, 109800 (3) |
| 556 | FGFR3 | 2261 | FLJ10324 | 55698 | 134934 | Crouzon syndrome with acanthosis nigricans (3) |
| 556 | FGFR3 | 2261 | FLJ10324 | 55698 | 134934 | Hypochondroplasia, 146000 (3) |
| 556 | FGFR3 | 2261 | FLJ10324 | 55698 | 134934 | Muenke syndrome, 602849 (3) |
| 556 | FGFR3 | 2261 | FLJ10324 | 55698 | 134934 | Thanatophoric dysplasia, types I and II, 187600 (3) |
| 556 | FGFR3 | 2261 | FLJ22624 | 79866 | 134934 | Achondropiasia. 100800 (3) |
| 556 | FGFR3 | 2261 | FLJ22624 | 79866 | 134934 | Bladder cancer, 109800 (3) |
| 556 | FGFR3 | 2261 | FLJ22624 | 79866 | 134934 | Cervical cancer, somatic, 603956 (3) |
| 556 | FGFR3 | 2261 | FLJ22624 | 79866 | 134934 | Colorectal cancer, somatic, 109800 (3) |
| 556 | FGFR3 | 2261 | FLJ22624 | 79866 | 134934 | Crouzon syndrome with acanthosis nigricans (3) |
| 556 | FGFR3 | 2261 | FLJ22624 | 79866 | 134934 | Hypochondroplasia, 146000 (3) |
| 556 | FGFR3 | 2261 | FLJ22624 | 79866 | 134934 | Muenke syndrome, 602849 (3) |
| 556 | FGFR3 | 2261 | FLJ22624 | 79866 | 134934 | Thanatophoric dysplasia, types I and II, 187600 (3) |
| 556 | FGFR3 | 2261 | FLJ33084 | 149483 | 134934 | Achondroplasia, 100800 (3) |
| 556 | FGFR3 | 2261 | FLJ33084 | 149483 | 134934 | Bladder cancer, 109800 (3) |
| 556 | FGFR3 | 2261 | FLJ33084 | 149483 | 134934 | Cervical cancer, somatic, 603956 (3) |
| 556 | FGFR3 | 2261 | FLJ33084 | 149483 | 134934 | Colorectal cancer, somatic, 109800 (3) |
| 556 | FGFR3 | 2261 | FLJ33084 | 149483 | 134934 | Crouzon syndrome with acanthosis nigricans (3) |
| 556 | FGFR3 | 2261 | FLJ33084 | 149483 | 134934 | Hypochondroplasia, 146000 (3) |
| 556 | FGFR3 | 2261 | FLJ33084 | 149483 | 134934 | Muenke syndrome, 602849 (3) |
| 556 | FGFR3 | 2261 | FLJ33084 | 149483 | 134934 | Thanatophoric dysplasia, types I and II, 187600 (3) |
| 556 | FGFR3 | 2261 | GTF3C1 | 2975 | 134934 | Achondroplasia, 100800 (3) |
| 556 | FGFR3 | 2261 | GTF3C1 | 2975 | 134934 | Bladder cancer, 109800 (3) |
| 556 | FGFR3 | 2261 | GTF3C1 | 2975 | 134934 | Cervical cancer, somatic, 603956 (3) |
| 556 | FGFR3 | 2261 | GTF3C1 | 2975 | 134934 | Colorectal cancer, somatic, 109800 (3) |
| 556 | FGFR3 | 2261 | GTF3C1 | 2975 | 134934 | Crouzon syndrome with acanthosis nigricans (3) |
| 556 | FGFR3 | 2261 | GTF3C1 | 2975 | 134934 | Hypochondroplasia, 146000 (3) |
| 556 | FGFR3 | 2261 | GTF3C1 | 2975 | 134934 | Muenke syndrome, 602849 (3) |
| 556 | FGFR3 | 2261 | GTF3C1 | 2975 | 134934 | Thanatophoric dysplasia, types I and II, 187600 (3) |
| 556 | FGFR3 | 2261 | HBZ | 3050 | 134934 | Achondroplasia, 100800 (3) |
| 556 | FGFR3 | 2261 | HBZ | 3050 | 134934 | Bladder cancer, 109800 (3) |
| 556 | FGFR3 | 2261 | HBZ | 3050 | 134934 | Cervical cancer, somatic, 603956 (3) |
| 556 | FGFR3 | 2261 | HBZ | 3050 | 134934 | Colorectal cancer, somatic, 109800 (3) |
| 556 | FGFR3 | 2261 | HBZ | 3050 | 134934 | Crouzon syndrome with acanthosis nigricans (3) |
| 556 | FGFR3 | 2261 | HBZ | 3050 | 134934 | Hypochondroplasia, 146000 (3) |
| 556 | FGFR3 | 2261 | HBZ | 3050 | 134934 | Muenke syndrome, 602849 (3) |
| 556 | FGFR3 | 2261 | HBZ | 3050 | 134934 | Thanatophoric dysplasia, types I and II, 187600 (3) |
| 556 | FGFR3 | 2261 | HNRPL | 3191 | 134934 | Achondroplasia, 100800 (3) |
| 556 | FGFR3 | 2261 | HNRPL | 3191 | 134934 | Bladder cancer, 109800 (3) |
| 556 | FGFR3 | 2261 | HNRPL | 3191 | 134934 | Cervical cancer, somatic, 603956 (3) |
| 556 | FGFR3 | 2261 | HNRPL | 3191 | 134934 | Colorectal cancer, somatic, 109800 (3) |
| 556 | FGFR3 | 2261 | HNRPL | 3191 | 134934 | Crouzon syndrome with acanthosis nigricans (3) |
| 556 | FGFR3 | 2261 | HNRPL | 3191 | 134934 | Hypochondroplasia, 146000 (3) |
| 556 | FGFR3 | 2261 | HNRPL | 3191 | 134934 | Muenke syndrome, 602849 (3) |
| 556 | FGFR3 | 2261 | HNRPL | 3191 | 134934 | Thanatophoric dyspiasia, types I and II, 187600 (3) |
| 556 | FGFR3 | 2261 | KIAA1377 | 57562 | 134934 | Achondroplasia, 100800 (3) |
| 556 | FGFR3 | 2261 | KIAA1377 | 57562 | 134934 | Bladder cancer, 109800 (3) |
| 556 | FGFR3 | 2261 | KIAA1377 | 57562 | 134934 | Cervical cancer, somatic, 603956 (3) |
| 556 | FGFR3 | 2261 | KIAA1377 | 57562 | 134934 | Colorectal cancer, somatic, 109800 (3) |
| 556 | FGFR3 | 2261 | KIAA1377 | 57562 | 134934 | Crouzon syndrome with acanthosis nigricans (3) |
| 556 | FGFR3 | 2261 | KIAA1377 | 57562 | 134934 | Hypochondroplasia, 146000 (3) |
| 556 | FGFR3 | 2261 | KIAA1377 | 57562 | 134934 | Muenke syndrome, 602849 (3) |
| 556 | FGFR3 | 2261 | KIAA1377 | 57562 | 134934 | Thanatophoric dysplasia, types I and II, 187600 (3) |
| 556 | FGFR3 | 2261 | KRT8 | 3856 | 134934 | Achondroplasia, 100800 (3) |
| 556 | FGFR3 | 2261 | KRT8 | 3856 | 134934 | Bladder cancer, 109800 (3) |
| 556 | FGFR3 | 2261 | KRT8 | 3856 | 134934 | Cervical cancer, somatic, 603956 (3) |
| 556 | FGFR3 | 2261 | KRT8 | 3856 | 134934 | Colorectal cancer, somatic, 109800 (3) |
| 556 | FGFR3 | 2261 | KRT8 | 3856 | 134934 | Crouzon syndrome with acanthosis nigricans (3) |
| 556 | FGFR3 | 2261 | KRT8 | 3856 | 134934 | Hypochondroplasia, 146000 (3) |
| 556 | FGFR3 | 2261 | KRT8 | 3856 | 134934 | Muenke syndrome, 602849 (3) |
| 556 | FGFR3 | 2261 | KRT8 | 3856 | 134934 | Thanatophoric dysplasia, types I and II, 187600 (3) |
| 556 | FGFR3 | 2261 | NDUFS6 | 4726 | 134934 | Achondroplasia, 100800 (3) |
| 556 | FGFR3 | 2261 | NDUFS6 | 4726 | 134934 | Bladder cancer, 109800 (3) |
| 556 | FGFR3 | 2261 | NDUFS6 | 4726 | 134934 | Cervical cancer, somatic, 603956 (3) |
| 556 | FGFR3 | 2261 | NDUFS6 | 4726 | 134934 | Colorectal cancer, somatic, 109800 (3) |
| 556 | FGFR3 | 2261 | NDUFS6 | 4726 | 134934 | Crouzon syndrome with acanthosis nigricans (3) |
| 556 | FGFR3 | 2261 | NDUFS6 | 4726 | 134934 | Hypochondroplasia, 146000 (3) |
| 556 | FGFR3 | 2261 | NDUFS6 | 4726 | 134934 | Muenke syndrome, 602849 (3) |
| 556 | FGFR3 | 2261 | NDUFS6 | 4726 | 134934 | Thanatophoric dysplasia, types I and II, 187600 (3) |
| 556 | FGFR3 | 2261 | POLA2 | 23649 | 134934 | Achondroplasia, 100800 (3) |
| 556 | FGFR3 | 2261 | POLA2 | 23649 | 134934 | Bladder cancer, 109800 (3) |
| 556 | FGFR3 | 2261 | POLA2 | 23649 | 134934 | Cervical cancer, somatic, 603956 (3) |
| 556 | FGFR3 | 2261 | POLA2 | 23649 | 134934 | Colorectal cancer, somatic, 109800 (3) |
| 556 | FGFR3 | 2261 | POLA2 | 23649 | 134934 | Crouzon syndrome with acanthosis nigricans (3) |
| 556 | FGFR3 | 2261 | POLA2 | 23649 | 134934 | Hypochondroplasia, 146000 (3) |
| 556 | FGFR3 | 2261 | POLA2 | 23649 | 134934 | Muenke syndrome, 602849 (3) |
| 556 | FGFR3 | 2261 | POLA2 | 23649 | 134934 | Thanatophoric dysplasia, types I and II, 187600 (3) |
| 556 | FGFR3 | 2261 | RNF130 | 55819 | 134934 | Achondroplasia, 100800 (3) |
| 556 | FGFR3 | 2261 | RNF130 | 55819 | 134934 | Bladder cancer, 109800 (3) |
| 556 | FGFR3 | 2261 | RNF130 | 55819 | 134934 | Cervical cancer, somatic, 603956 (3) |
| 556 | FGFR3 | 2261 | RNF130 | 55819 | 134934 | Colorectal cancer, somatic, 109800 (3) |
| 556 | FGFR3 | 2261 | RNF130 | 55819 | 134934 | Crouzon syndrome with acanthosis nigricans (3) |
| 556 | FGFR3 | 2261 | RNF130 | 55819 | 134934 | Hypochondroplasia, 146000 (3) |
| 556 | FGFR3 | 2261 | RNF130 | 55819 | 134934 | Muenke syndrome, 602849 (3) |
| 556 | FGFR3 | 2261 | RNF130 | 55819 | 134934 | Thanatophoric dysplasia, types I and II, 187600 (3) |
| 556 | FGFR3 | 2261 | RPL8 | 6132 | 134934 | Achondroplasia, 100800 (3) |
| 556 | FGFR3 | 2261 | RPL8 | 6132 | 134934 | Bladder cancer, 109800 (3) |
| 556 | FGFR3 | 2261 | RPL8 | 6132 | 134934 | Cervical cancer, somatic, 603956 (3) |
| 556 | FGFR3 | 2261 | RPL8 | 6132 | 134934 | Colorectal cancer, somatic, 109800 (3) |
| 556 | FGFR3 | 2261 | RPL8 | 6132 | 134934 | Crouzon syndrome with acanthosis nigricans (3) |
| 556 | FGFR3 | 2261 | RPL8 | 6132 | 134934 | Hypochondroplasia, 146000 (3) |
| 556 | FGFR3 | 2261 | RPL8 | 6132 | 134934 | Muenke syndrome, 602849 (3) |
| 556 | FGFR3 | 2261 | RPL8 | 6132 | 134934 | Thanatophoric dysplasia, types I and II, 187600 (3) |
| 556 | FGFR3 | 2261 | SLC25A6 | 293 | 134934 | Achondroplasia, 100800 (3) |
| 556 | FGFR3 | 2261 | SLC25A6 | 293 | 134934 | Bladder cancer, 109800 (3) |
| 556 | FGFR3 | 2261 | SLC25A6 | 293 | 134934 | Cervical cancer, somatic, 603956 (3) |
| 556 | FGFR3 | 2261 | SLC25A6 | 293 | 134934 | Colorectal cancer, somatic, 109800 (3) |
| 556 | FGFR3 | 2261 | SLC25A6 | 293 | 134934 | Crouzon syndrome with acanthosis nigricans (3) |
| 556 | FGFR3 | 2261 | SLC25A6 | 293 | 134934 | Hypochondroplasia, 146000 (3) |
| 556 | FGFR3 | 2261 | SLC25A6 | 293 | 134934 | Muenke syndrome, 602849 (3) |
| 556 | FGFR3 | 2261 | SLC25A6 | 293 | 134934 | Thanatophoric dysplasia, types I and II, 187600 (3) |
| 556 | FGFR3 | 2261 | SMA3 | 10571 | 134934 | Achondroplasia, 100800 (3) |
| 556 | FGFR3 | 2261 | SMA3 | 10571 | 134934 | Bladder cancer, 109800 (3) |
| 556 | FGFR3 | 2261 | SMA3 | 10571 | 134934 | Cervical cancer, somatic, 603956 (3) |
| 556 | FGFR3 | 2261 | SMA3 | 10571 | 134934 | Colorectal cancer, somatic, 109800 (3) |
| 556 | FGFR3 | 2261 | SMA3 | 10571 | 134934 | Crouzon syndrome with acanthosis nigricans (3) |
| 556 | FGFR3 | 2261 | SMA3 | 10571 | 134934 | Hypochondroplasia, 146000 (3) |
| 556 | FGFR3 | 2261 | SMA3 | 10571 | 134934 | Muenke syndrome, 602849 (3) |
| 556 | FGFR3 | 2261 | SMA3 | 10571 | 134934 | Thanatophoric dysplasia, types I and II, 187600 (3) |
| 645 | FTH1 | 2495 | BRD7 | 29117 | 134770 | Iron overload, autosomal dominant (3) |
| 645 | FTH1 | 2495 | CRMP1 | 1400 | 134770 | Iron overload, autosomal dominant (3) |
| 645 | FTH1 | 2495 | ELL3 | 80237 | 134770 | Iron overload, autosomal dominant (3) |
| 645 | FTH1 | 2495 | FTL | 2512 | 134770 | Iron overload, autosomal dominant (3) |
| 645 | FTH1 | 2495 | GOLPH2 | 51280 | 134770 | Iron overload, autosomal dominant (3) |
| 645 | FTH1 | 2495 | UBR1 | 197131 | 134770 | Iron overload, autosomal dominant (3) |
| 646 | FTH1 | 2495 | BRD7 | 29117 | 134770 | Iron overload, autosomal dominant (3) |
| 646 | FTH1 | 2495 | CRMP1 | 1400 | 134770 | Iron overload, autosomal dominant (3) |
| 646 | FTH1 | 2495 | ELL3 | 80237 | 134770 | Iron overload, autosomal dominant (3) |
| 646 | FTH1 | 2495 | FTL | 2512 | 134770 | Iron overload, autosomal dominant (3) |
| 646 | FTH1 | 2495 | GOLPH2 | 51280 | 134770 | Iron overload, autosomal dominant (3) |
| 646 | FTH1 | 2495 | UBR1 | 197131 | 134770 | Iron overload, autosomal dominant (3) |
| 647 | FTL | 2512 | FTH1 | 2495 | 134790 | Basal ganglia disease, adult-onset, 606159 (3) |
| 647 | FTL | 2512 | FTH1 | 2495 | 134790 | Hyperferritinemia-cataract syndrome, 600886 (3) |
| 647 | FTL | 2512 | FTL | 2512 | 134790 | Basal ganglia disease, adult-onset, 606159 (3) |
| 647 | FTL | 2512 | FTL | 2512 | 134790 | Hyperferritinemia-cataract syndrome, 600886 (3) |
| 647 | FTL | 2512 | GADD45A | 1647 | 134790 | Basal ganglia disease, adult-onset, 606159 (3) |
| 647 | FTL | 2512 | GADD45A | 1647 | 134790 | Hyperferritinemia-cataract syndrome, 600886 (3) |
| 647 | FTL | 2512 | MAP3K12 | 7786 | 134790 | Basal ganglia disease, adult-onset, 606159 (3) |
| 647 | FTL | 2512 | MAP3K12 | 7786 | 134790 | Hyperferritinemia-cataract syndrome, 600886 (3) |
| 647 | FTL | 2512 | PTN | 5764 | 134790 | Basal ganglia disease, adult-onset, 606159 (3) |
| 647 | FTL | 2512 | PTN | 5764 | 134790 | Hyperferritinemia-cataract syndrome, 600886 (3) |
| 647 | FTL | 2512 | TAF10 | 6881 | 134790 | Basal ganglia disease, adult-onset, 606159 (3) |
| 647 | FTL | 2512 | TAF10 | 6881 | 134790 | Hyperferritinemia-cataract syndrome, 600886 (3) |
| 669 | GARS | 2617 | CELSR2 | 1952 | 600287 | Charcot-Marie-Tooth disease, type 2D, 601472 (3) |
| 669 | GARS | 2617 | CELSR2 | 1952 | 600287 | Spinal muscular atrophy, distal, type V, 600794 (3) |
| 671 | GBE1 | 2632 | PGD | 5226 | 607839 | Glycogen storage disease IV, 232500 (3) |
| 673 | GC | 2638 | HRPT2 | 79577 | 139200 | {Graves disease, susceptibility to, 3}, 275000 (3) |
| 673 | GC | 2638 | RIF1 | 55791 | 139200 | {Graves disease, susceptibility to, 3}, 275000 (3) |
| 673 | GC | 2638 | SERF2 | 10169 | 139200 | {Graves disease, susceptibility to, 3}, 275000 (3) |
| 675 | GCS1 | 7841 | CHGB | 1114 | 601336 | Glucosidase I deficiency, 606056 (3) |
| 676 | GCSH | 2653 | ING5 | 84289 | 238330 | Glycine encephalopathy, 605899 (3) |
| 676 | GCSH | 2653 | ZNF145 | 7704 | 238330 | Glycine encephalopathy, 605899 (3) |
| 683 | GLMN | 11146 | NSFL1C | 55968 | 601749 | Glomuvenous malformations, 138000 (3) |
| 690 | GNAI2 | 2771 | TUBA1 | 22142 | 139360 | Pituitary ACTH-secreting adenoma (3) |
| 690 | GNAI2 | 2771 | TUBA1 | 22142 | 139360 | Ventricular tachycardia, idiopathic, 192605 (3) |
| 701 | GNPAT | 8443 | RAB35 | 11021 | 602744 | Chondrodysplasia punctata, rhizomelic, type 2, 222765 (3) |
| 718 | GSN | 2934 | TOM1L1 | 10040 | 137350 | Amyloidosis, Finnish type, 105120 (3) |
| 719 | GSR | 2936 | PJA1 | 64219 | 138300 | Hemolytic anemia due to glutathione reductase deficiency (1) |
| 731 | HBA1 | 3039 | ATP6AP1 | 537 | 141800 | Erythremias, alpha- (3) |
| 731 | HBA1 | 3039 | ATP6AP1 | 537 | 141800 | Heinz body anemias, alpha- (3) |
| 731 | HBA1 | 3039 | ATP6AP1 | 537 | 141800 | Methemoglobinemias, alpha- (3) |
| 731 | HBA1 | 3039 | ATP6AP1 | 537 | 141800 | Thalassemias, alpha- (3) |
| 731 | HBA1 | 3039 | HBB | 3043 | 141800 | Erythremias, alpha- (3) |
| 731 | HBA1 | 3039 | HBB | 3043 | 141800 | Heinz body anemias, alpha- (3) |
| 731 | HBA1 | 3039 | HBB | 3043 | 141800 | Methemoglobinemias, alpha- (3) |
| 731 | HBA1 | 3039 | HBB | 3043 | 141800 | Thalassemias, alpha- (3) |
| 731 | HBA1 | 3039 | NAP1L1 | 4673 | 141800 | Erythremias, alpha- (3) |
| 731 | HBA1 | 3039 | NAP1L1 | 4673 | 141800 | Heinz body anemias, alpha- (3) |
| 731 | HBA1 | 3039 | NAP1L1 | 4673 | 141800 | Methemoglobinemias, alpha- (3) |
| 731 | HBA1 | 3039 | NAP1L1 | 4673 | 141800 | Thalassemias,alpha-(3) |
| 731 | HBA1 | 3039 | PTEN | 5728 | 141800 | Erythremias, alpha- (3) |
| 731 | HBA1 | 3039 | PTEN | 5728 | 141800 | Heinz body anemias, alpha- (3) |
| 731 | HBA1 | 3039 | PTEN | 5728 | 141800 | Methemoglobinemias, alpha- (3) |
| 731 | HBA1 | 3039 | PTEN | 5728 | 141800 | Thalassemias, alpha-(3) |
| 732 | HBA1 | 3039 | ATP6AP1 | 537 | 141800 | Erythremias, alpha- (3) |
| 732 | HBA1 | 3039 | ATP6AP1 | 537 | 141800 | Heinz body anemias, alpha- (3) |
| 732 | HBA1 | 3039 | ATP6AP1 | 537 | 141800 | Methemoglobinemias, alpha- (3) |
| 732 | HBA1 | 3039 | ATP6AP1 | 537 | 141800 | Thalassemias, alpha- (3) |
| 732 | HBA1 | 3039 | HBB | 3043 | 141800 | Erythremias, alpha- (3) |
| 732 | HBA1 | 3039 | HBB | 3043 | 141800 | Heinz body anemias, alpha- (3) |
| 732 | HBA1 | 3039 | HBB | 3043 | 141800 | Methemoglobinemias, alpha- (3) |
| 732 | HBA1 | 3039 | HBB | 3043 | 141800 | Thalassemias, alpha- (3) |
| 732 | HBA1 | 3039 | NAP1L1 | 4673 | 141800 | Erythremias, alpha- (3) |
| 732 | HBA1 | 3039 | NAP1L1 | 4673 | 141800 | Heinz body anemias, alpha- (3) |
| 732 | HBA1 | 3039 | NAP1L1 | 4673 | 141800 | Methemoglobinemias, alpha- (3) |
| 732 | HBA1 | 3039 | NAP1L1 | 4673 | 141800 | Thalassemias, alpha- (3) |
| 732 | HBA1 | 3039 | PTEN | 5728 | 141800 | Erythremias, alpha- (3) |
| 732 | HBA1 | 3039 | PTEN | 5728 | 141800 | Heinz body anemias, alpha- (3) |
| 732 | HBA1 | 3039 | PTEN | 5728 | 141800 | Methemoglobinemias, alpha- (3) |
| 732 | HBA1 | 3039 | PTEN | 5728 | 141800 | Thalassemias, alpha-(3) |
| 733 | HBB | 3043 | HBA1 | 3039 | 141900 | Erythremias, beta- (3) |
| 733 | HBB | 3043 | HBA1 | 3039 | 141900 | Heinz body anemias, beta- (3) |
| 733 | HBB | 3043 | HBA1 | 3039 | 141900 | HPFH, deletion type (3) |
| 733 | HBB | 3043 | HBA1 | 3039 | 141900 | Methemoglobinemias, beta- (3) |
| 733 | HBB | 3043 | HBA1 | 3039 | 141900 | Sickle cell anemia (3) |
| 733 | HBB | 3043 | HBA1 | 3039 | 141900 | Thalassemia-beta, dominant inclusion-body, 603902 (3) |
| 733 | HBB | 3043 | HBA1 | 3039 | 141900 | Thalassemias, beta-(3) |
| 733 | HBB | 3043 | SELT | 51714 | 141900 | Erythremias, beta- (3) |
| 733 | HBB | 3043 | SELT | 51714 | 141900 | Heinz body anemias, beta- (3) |
| 733 | HBB | 3043 | SELT | 51714 | 141900 | HPFH, deletion type (3) |
| 733 | HBB | 3043 | SELT | 51714 | 141900 | Methemoglobinemias, beta- (3) |
| 733 | HBB | 3043 | SELT | 51714 | 141900 | Sickle cell anemia (3) |
| 733 | HBB | 3043 | SELT | 51714 | 141900 | Thalassemia-beta, dominant inclusion-body, 603902 (3) |
| 733 | HBB | 3043 | SELT | 51714 | 141 900 | Thalassemias, beta-(3) |
| 741 | HD | 3064 | PDK2 | 5164 | 143100 | Huntington disease (3) |
| 747 | HEXA | 3073 | USP22 | 23326 | 606869 | [Hex A pseudodeficiency], 272800 (3) |
| 747 | HEXA | 3073 | USP22 | 23326 | 606869 | GM2-gangliosidosis, several forms, 272800 (3) |
| 747 | HEXA | 3073 | USP22 | 23326 | 606869 | Tay-Sachs disease, 272800 (3) |
| 749 | HIP1 | 3092 | ITGB4BP | 3692 | 601767 | {Prostate cancer, progression of}, 176807 (1) |
| 749 | HIP1 | 3092 | PFDN1 | 5201 | 601767 | {Prostate cancer, progression of}, 176807 (1) |
| 749 | HIP1 | 3092 | RPS10 | 6204 | 601767 | {Prostate cancer, progression of}, 176807 (1) |
| 755 | HK1 | 3098 | EN02 | 2026 | 142600 | Hemolytic anemia due to hexokinase deficiency (3) |
| 755 | HK1 | 3098 | SCMH1 | 22955 | 142600 | Hemolytic anemia due to hexokinase deficiency (3) |
| 756 | HK1 | 3098 | EN02 | 2026 | 142600 | Hemolytic anemia due to hexokinase deficiency (3) |
| 756 | HK1 | 3098 | SCMH1 | 22955 | 142600 | Hemolytic anemia due to hexokinase deficiency (3) |
| 763 | HMGCL | 3155 | MS4A7 | 58475 | 246450 | HMG-CoA lyase deficiency (3) |
| 780 | HPS6 | 79803 | CREB1 | 1385 | 607522 | Hermansky-Pudlak syndrome, 203300 (3) |
| 780 | HPS6 | 79803 | KIAA0020 | 9933 | 607522 | Hermansky-Pudlak syndrome, 203300 (3) |
| 780 | HPS6 | 79803 | MARK4 | 57787 | 607522 | Hermansky-Pudlak syndrome, 203300 (3) |
| 780 | HPS6 | 79803 | MNAT1 | 4331 | 607522 | Hermansky-Pudlak syndrome, 203300 (3) |
| 784 | HRPT2 | 79577 | ANXA6 | 309 | 607393 | Hyperparathyroidism, familial primary, 145000 (3) |
| 784 | HRPT2 | 79577 | ANXA6 | 309 | 607393 | Hyperparathyroidism-jaw tumor syndrome, 145001 (3) |
| 784 | HRPT2 | 79577 | ANXA6 | 309 | 607393 | Parathyroid adenoma with cystic changes, 145001 (3) |
| 784 | HRPT2 | 79577 | ANXA6 | 309 | 607393 | Parathyroid carcinoma, 608266 (3) |
| 784 | HRPT2 | 79577 | GC | 2638 | 607393 | Hyperparathyroidism, familial primary, 145000 (3) |
| 784 | HRPT2 | 79577 | GC | 2638 | 607393 | Hyperparathyroidism-jaw tumor syndrome, 145001 (3) |
| 784 | HRPT2 | 79577 | GC | 2638 | 607393 | Parathyroid adenoma with cystic changes, 145001 (3) |
| 784 | HRPT2 | 79577 | GC | 2638 | 607393 | Parathyroid carcinoma, 608266 (3) |
| 784 | HRPT2 | 79577 | HSPA4 | 3308 | 607393 | Hyperparathyroidism, familial primary, 145000 (3) |
| 784 | HRPT2 | 79577 | HSPA4 | 3308 | 607393 | Hyperparathyroidism-jaw tumor syndrome, 145001 (3) |
| 784 | HRPT2 | 79577 | HSPA4 | 3308 | 607393 | Parathyroid adenoma with cystic changes, 145001 (3) |
| 784 | HRPT2 | 79577 | HSPA4 | 3308 | 607393 | Parathyroid carcinoma, 608266 (3) |
| 784 | HRPT2 | 79577 | HT014 | 57095 | 607393 | Hyperparathyroidism, familial primary, 145000 (3) |
| 784 | HRPT2 | 79577 | HT014 | 57095 | 607393 | Hyperparathyroidism-jaw tumor syndrome, 145001 (3) |
| 784 | HRPT2 | 79577 | HT014 | 57095 | 607393 | Parathyroid adenoma with cystic changes, 145001 (3) |
| 784 | HRPT2 | 79577 | HT014 | 57095 | 607393 | Parathyroid carcinoma, 608266 (3) |
| 784 | HRPT2 | 79577 | KCNH2 | 3757 | 607393 | Hyperparathyroidism, familial primary, 145000 (3) |
| 784 | HRPT2 | 79577 | KCNH2 | 3757 | 607393 | Hyperparathyroidism-jaw tumor syndrome, 145001 (3) |
| 784 | HRPT2 | 79577 | KCNH2 | 3757 | 607393 | Parathyroid adenoma with cystic changes, 145001 (3) |
| 784 | HRPT2 | 79577 | KCNH2 | 3757 | 607393 | Parathyroid carcinoma, 608266 (3) |
| 784 | HRPT2 | 79577 | KIAA1549 | 57670 | 607393 | Hyperparathyroidism, familial primary, 145000 (3) |
| 784 | HRPT2 | 79577 | KIAA1549 | 57670 | 607393 | Hyperparathyroidism-jaw tumor syndrome, 145001 (3) |
| 784 | HRPT2 | 79577 | KIAA1549 | 57670 | 607393 | Parathyroid adenoma with cystic changes, 145001 (3) |
| 784 | HRPT2 | 79577 | KIAA1549 | 57670 | 607393 | Parathyroid carcinoma, 608266 (3) |
| 784 | HRPT2 | 79577 | KIBRA | 23286 | 607393 | Hyperparathyroidism, familial primary, 145000 (3) |
| 784 | HRPT2 | 79577 | KIBRA | 23286 | 607393 | Hyperparathyroidism-jaw tumor syndrome, 145001 (3) |
| 784 | HRPT2 | 79577 | KIBRA | 23286 | 607393 | Parathyroid adenoma with cystic changes, 145001 (3) |
| 784 | HRPT2 | 79577 | KIBRA | 23286 | 607393 | Parathyroid carcinoma, 608266 (3) |
| 784 | HRPT2 | 79577 | KIDINS220 | 57498 | 607393 | Hyperparathyroidism, familial primary, 145000 (3) |
| 784 | HRPT2 | 79577 | KIDINS220 | 57498 | 607393 | Hyperparathyroidism-jaw tumor syndrome, 145001 (3) |
| 784 | HRPT2 | 79577 | KIDINS220 | 57498 | 607393 | Parathyroid adenoma with cystic changes, 145001 (3) |
| 784 | HRPT2 | 79577 | KIDINS220 | 57498 | 607393 | Parathyroid carcinoma, 608266 (3) |
| 784 | HRPT2 | 79577 | TOMM20 | 9804 | 607393 | Hyperparathyroidism, familial primary, 145000 (3) |
| 784 | HRPT2 | 79577 | TOMM20 | 9804 | 607393 | Hyperparathyroidism-jaw tumor syndrome, 145001 (3) |
| 784 | HRPT2 | 79577 | TOMM20 | 9804 | 607393 | Parathyroid adenoma with cystic changes, 145001 (3) |
| 784 | HRPT2 | 79577 | TOMM20 | 9804 | 607393 | Parathyroid carcinoma, 608266 (3) |
| 795 | HSPB1 | 3315 | CGI-125 | 51003 | 602195 | Neuropathy, distal hereditary motor, 608634 (3) |
| 795 | HSPB1 | 3315 | DKFZP564O0523 | 84060 | 602195 | Neuropathy, distal hereditary motor, 608634 (3) |
| 795 | HSPB1 | 3315 | MGC15730 | 84966 | 602195 | Neuropathy, distal hereditary motor, 608634 (3) |
| 795 | HSPB1 | 3315 | PP | 5464 | 602195 | Neuropathy, distal hereditary motor, 608634 (3) |
| 795 | HSPB1 | 3315 | RIF1 | 55791 | 602195 | Neuropathy, distal hereditary motor, 608634 (3) |
| 795 | HSPB1 | 3315 | TP53 | 7157 | 602195 | Neuropathy, distal hereditary motor, 608634 (3) |
| 822 | IDUA | 3425 | MPP3 | 4356 | 252800 | Mucopolysaccharidosis lh, 607014 (3) |
| 822 | IDUA | 3425 | MPP3 | 4356 | 252800 | Mucopolysaccharidosis lh/s, 607015 (3) |
| 822 | IDUA | 3425 | MPP3 | 4356 | 252800 | Mucopolysaccharidosis Is, 607016 (3) |
| 833 | IKBKAP | 8518 | APACD | 10190 | 603722 | Dysautonomia, familial, 223900 (3) |
| 833 | IKBKAP | 8518 | BAT3 | 7917 | 603722 | Dysautonomia, familial, 223900 (3) |
| 833 | IKBKAP | 8518 | CCT5 | 22948 | 603722 | Dysautonomia, familial, 223900 (3) |
| 833 | IKBKAP | 8518 | CDKN2B | 1030 | 603722 | Dysautonomia, familial, 223900 (3) |
| 833 | IKBKAP | 8518 | CSTF2 | 1478 | 603722 | Dysautonomia, familial, 223900 (3) |
| 833 | IKBKAP | 8518 | FLJ20487 | 54949 | 603722 | Dysautonomia, familial, 223900 (3) |
| 833 | IKBKAP | 8518 | MAN2A2 | 4122 | 603722 | Dysautonomia, familial, 223900 (3) |
| 833 | IKBKAP | 8518 | MRPL42 | 28977 | 603722 | Dysautonomia, familial, 223900 (3) |
| 833 | IKBKAP | 8518 | NDUFB9 | 4715 | 603722 | Dysautonomia, familial, 223900 (3) |
| 833 | IKBKAP | 8518 | NIPSNAP3A | 25934 | 603722 | Dysautonomia, familial, 223900 (3) |
| 833 | IKBKAP | 8518 | PLP2 | 5355 | 603722 | Dysautonomia, familial, 223900 (3) |
| 833 | IKBKAP | 8518 | SNAPAP | 23557 | 603722 | Dysautonomia, familial, 223900 (3) |
| 833 | IKBKAP | 8518 | TAC3 | 6866 | 603722 | Dysautonomia, familial, 223900 (3) |
| 833 | IKBKAP | 8518 | TTR | 7276 | 603722 | Dysautonomia, familial, 223900 (3) |
| 835 | IL4R | 3566 | ARIH2 | 10425 | 147781 | {Atopy, susceptibility to} (3) |
| 836 | IL6 | 3569 | SH3GL2 | 6456 | 147620 | {Kaposi sarcoma, susceptibility to}, 148000 (3) |
| 836 | IL6 | 3569 | SH3GL2 | 6456 | 147620 | Osteopenia/osteoporosis, 166710 (2) |
| 836 | IL6 | 3569 | ZNF145 | 7704 | 147620 | {Kaposi sarcoma, susceptibility to}, 148000 (3) |
| 836 | IL6 | 3569 | ZNF145 | 7704 | 147620 | Osteopenia/osteoporosis. 166710 (2) |
| 848 | IRAK4 | 51135 | TBPL1 | 9519 | 606883 | IRAK4 deficiency, 607676 (3) |
| 863 | JUP | 3728 | ARHGDIA | 396 | 173325 | Naxos disease, 601214 (3) |
| 864 | KAI1 | 3732 | NUP88 | 4927 | 600623 | {Prostate cancer, susceptibility to}, 176807 (2) |
| 870 | KCNH2 | 3757 | ARHH | 399 | 152427 | {Acquired long QT syndrome, susceptibility to} (3) |
| 870 | KCNH2 | 3757 | ARHH | 399 | 152427 | Long QT syndrome-2 (3) |
| 870 | KCNH2 | 3757 | HRPT2 | 79577 | 152427 | {Acquired long QT syndrome, susceptibility to} (3) |
| 870 | KCNH2 | 3757 | HRPT2 | 79577 | 152427 | Long QT syndrome-2 (3) |
| 870 | KCNH2 | 3757 | NDUFS6 | 4726 | 152427 | {Acquired long QT syndrome, susceptibility to} (3) |
| 870 | KCNH2 | 3757 | NDUFS6 | 4726 | 152427 | Long QT syndrome-2 (3) |
| 871 | KCNH2 | 3757 | ARHH | 399 | 152427 | {Acquired long QT syndrome, susceptibility to} (3) |
| 871 | KCNH2 | 3757 | ARHH | 399 | 152427 | Long QTsyndrome-2 (3) |
| 871 | KCNH2 | 3757 | HRPT2 | 79577 | 152427 | {Acquired long QT syndrome, susceptibility to} (3) |
| 871 | KCNH2 | 3757 | HRPT2 | 79577 | 152427 | Long QT syndrome-2 (3) |
| 871 | KCNH2 | 3757 | NDUFS6 | 4726 | 152427 | {Acquired long QT syndrome, susceptibility to} (3) |
| 871 | KCNH2 | 3757 | NDUFS6 | 4726 | 152427 | Long QT syndrome-2 (3) |
| 872 | KCNQ2 | 3785 | ARIH2 | 10425 | 602235 | Epilepsy, benign, neonatal, type 1, 121200 (3) |
| 872 | KCNQ2 | 3785 | ARIH2 | 10425 | 602235 | Myokymia with neonatal epilepsy, 606437 (3) |
| 910 | KIF1B | 23095 | EEF1A1 | 1915 | 605995 | Charcot-Marie-Tooth disease, type 2A, 118210 (3) |
| 912 | KIF5A | 3798 | ITSN1 | 6453 | 602821 | Spastic paraplegia 10, 604187 (3) |
| 912 | KIF5A | 3798 | TP53BP2 | 7159 | 602821 | Spastic paraplegia 10, 604187 (3) |
| 921 | KRT8 | 3856 | FGFR3 | 2261 | 148060 | {Cirrhosis, noncryptogenic, susceptibility to}, 215600 (3) |
| 921 | KRT8 | 3856 | FGFR3 | 2261 | 148060 | Cirrhosis, cryptogenic (3) |
| 923 | L1CAM | 3897 | PEA15 | 8682 | 308840 | CRASH syndrome, 303350 (3) |
| 923 | L1CAM | 3897 | PEA15 | 8682 | 308840 | Hydrocephalus due to aqueductal stenosis, 307000 (3) |
| 923 | L1CAM | 3897 | PEA15 | 8682 | 308840 | Hydrocephalus with Hirschsprung disease and cleft palate, 142623 (3) |
| 923 | L1CAM | 3897 | PEA15 | 8682 | 308840 | MASA syndrome, 303350 (3) |
| 924 | LAMA3 | 3909 | SLC35E1 | 79939 | 600805 | Epidermolysis bullosa, generalized atrophic benign, 226650 (3) |
| 924 | LAMA3 | 3909 | SLC35E1 | 79939 | 600805 | Epidermolysis bullosa, junctional, Herlitz type, 226700 (3) |
| 924 | LAMA3 | 3909 | SLC35E1 | 79939 | 600805 | Laryngoonychocutaneous syndrome, 245660 (3) |
| 928 | LAMR1 | 3921 | RPS21 | 6227 | 150370 | Arrhythmogenic right ventricular dysplasia-5, 604400 (1) (?) |
| 933 | LEPR | 3953 | PIN1 | 5300 | 601007 | Obesity, morbid, with hypogonadism (3) |
| 937 | LIG1 | 3978 | INPP1 | 3628 | 126391 | DNA ligase I deficiency (3) |
| 937 | LIG1 | 3978 | TUBB4 | 10381 | 126391 | DNA ligase I deficiency (3) |
| 940 | LITAF | 9516 | COMT | 1312 | 603795 | Charcot-Marie-Tooth disease, type 1C, 601098 (3) |
| 940 | LITAF | 9516 | FLJ10324 | 55698 | 603795 | Charcot-Marie-Tooth disease, type 1C, 601098 (3) |
| 942 | LMNA | 4000 | HTATIP | 10524 | 150330 | Cardiomyopathy, dilated, 1A, 115200 (3) |
| 942 | LMNA | 4000 | HTATIP | 10524 | 150330 | Charcot-Marie-Tooth disease, type 2B1, 605588 (3) |
| 942 | LMNA | 4000 | HTATIP | 10524 | 150330 | Emery-Dreifuss muscular dystrophy, AD, 181350 (3) |
| 942 | LMNA | 4000 | HTATIP | 10524 | 150330 | Emery-Dreifuss muscular dystrophy, AR, 604929 (3) |
| 942 | LMNA | 4000 | HTATIP | 10524 | 150330 | Hutchinson-Gilford progeria, 176670 (3) |
| 942 | LMNA | 4000 | HTATIP | 10524 | 150330 | Lipodystrophy, familial partial, 151660 (3) |
| 942 | LMNA | 4000 | HTATIP | 10524 | 150330 | Mandibuloacral dysplasia, 248370 (3) |
| 942 | LMNA | 4000 | HTATIP | 10524 | 150330 | Muscular dystrophy, limb-girdle, type 1B, 159001 (3) |
| 984 | LOR | 4014 | VIM | 7431 | 152445 | Erythrokeratoderma, progressive symmetric, 602036 (3) |
| 984 | LOR | 4014 | VIM | 7431 | 152445 | Vohwinkel syndrome with ichthyosis, 604117 (3) |
| 986 | LPL | 4023 | ASC1p100 | 84164 | 238600 | Chylomicronemia syndrome, familial (3) |
| 986 | LPL | 4023 | ASC1p100 | 84164 | 238600 | Combined hypertipemia, familial (3) |
| 986 | LPL | 4023 | ASC1p100 | 84164 | 238600 | Hyperlipoproteinemia I(1) |
| 986 | LPL | 4023 | ASC1p100 | 84164 | 238600 | Lipoprotein lipase deficiency (3) |
| 986 | LPL | 4023 | COPS6 | 10980 | 238600 | Chylomicronemia syndrome, familial (3) |
| 986 | LPL | 4023 | COPS6 | 10980 | 238600 | Combined hyperlipemia, familial (3) |
| 986 | LPL | 4023 | COPS6 | 10980 | 238600 | Hyperlipoproteinemia I (1) |
| 986 | LPL | 4023 | COPS6 | 10980 | 238600 | Lipoprotein lipase deficiency (3) |
| 986 | LPL | 4023 | FLJ13855 | 65264 | 238600 | Chylomicronemia syndrome, familial (3) |
| 986 | LPL | 4023 | FLJ13855 | 65264 | 238600 | Combined hyperlipemia, familial (3) |
| 986 | LPL | 4023 | FLJ13855 | 65264 | 238600 | Hyperlipoproteinemia I (1) |
| 986 | LPL | 4023 | FLJ13855 | 65264 | 238600 | Lipoprotein lipase deficiency (3) |
| 986 | LPL | 4023 | KIAA1377 | 57562 | 238600 | Chylomicronemia syndrome, familial (3) |
| 986 | LPL | 4023 | KIAA1377 | 57562 | 238600 | Combined hyperlipemia, familial (3) |
| 986 | LPL | 4023 | KIAA1377 | 57562 | 238600 | Hyperlipoproteinemia I (1) |
| 986 | LPL | 4023 | KIAA1377 | 57562 | 238600 | Lipoprotein lipase deficiency (3) |
| 986 | LPL | 4023 | LOC374923 | 374923 | 238600 | Chylomicronemia syndrome, familial (3) |
| 986 | LPL | 4023 | LOC374923 | 374923 | 238600 | Combined hyperlipemia, familial (3) |
| 986 | LPL | 4023 | LOC374923 | 374923 | 238600 | Hyperlipoproteinemia I (1) |
| 986 | LPL | 4023 | LOC374923 | 374923 | 238600 | Lipoprotein lipase deficiency (3) |
| 986 | LPL | 4023 | LUC7L2 | 51631 | 238600 | Chylomicronemia syndrome, familial (3) |
| 986 | LPL | 4023 | LUC7L2 | 51631 | 238600 | Combined hyperlipemia, familial (3) |
| 986 | LPL | 4023 | LUC7L2 | 51631 | 238600 | Hyperlipoproteinemia I (1) |
| 986 | LPL | 4023 | LUC7L2 | 51631 | 238600 | Lipoprotein lipase deficiency (3) |
| 986 | LPL | 4023 | PTPN4 | 5775 | 238600 | Chylomicronemia syndrome, familial (3) |
| 986 | LPL | 4023 | PTPN4 | 5775 | 238600 | Combined hyperlipemia, familial (3) |
| 986 | LPL | 4023 | PTPN4 | 5775 | 238600 | Hyperlipoproteinemia I (1) |
| 986 | LPL | 4023 | PTPN4 | 5775 | 238600 | Lipoprotein lipase deficiency (3) |
| 986 | LPL | 4023 | RPL18A | 6142 | 238600 | Chylomicronemia syndrome, familial (3) |
| 986 | LPL | 4023 | RPL18A | 6142 | 238600 | Combined hyperlipemia, familial (3) |
| 986 | LPL | 4023 | RPL18A | 6142 | 238600 | Hyperlipoproteinemia I (1) |
| 986 | LPL | 4023 | RPL18A | 6142 | 238600 | Lipoprotein lipase deficiency (3) |
| 987 | LPL | 4023 | ASC1p100 | 84164 | 238600 | Chylomicronemia syndrome, familial (3) |
| 987 | LPL | 4023 | ASC1p100 | 84164 | 238600 | Combined hyperlipemia, familial (3) |
| 987 | LPL | 4023 | ASC1p100 | 84164 | 238600 | Hyperlipoproteinemia I (1) |
| 987 | LPL | 4023 | ASC1p100 | 84164 | 238600 | Lipoprotein lipase deficiency (3) |
| 987 | LPL | 4023 | COPS6 | 10980 | 238600 | Chylomicronemia syndrome, familial (3) |
| 987 | LPL | 4023 | COPS6 | 10980 | 238600 | Combined hyperlipemia, familial (3) |
| 987 | LPL | 4023 | COPS6 | 10980 | 238600 | Hyperlipoproteinemia I (1) |
| 987 | LPL | 4023 | COPS6 | 10980 | 238600 | Lipoprotein lipase deficiency (3) |
| 987 | LPL | 4023 | FLJ13855 | 65264 | 238600 | Chylomicronemia syndrome, familial (3) |
| 987 | LPL | 4023 | FLJ13855 | 65264 | 238600 | Combined hyperlipemia, familial (3) |
| 987 | LPL | 4023 | FLJ13855 | 65264 | 238600 | Hyperlipoproteinemia I (1) |
| 987 | LPL | 4023 | FLJ13855 | 65264 | 238600 | Lipoprotein lipase deficiency (3) |
| 987 | LPL | 4023 | KIAA1377 | 57562 | 238600 | Chylomicronemia syndrome, familial (3) |
| 987 | LPL | 4023 | KIAA1377 | 57562 | 238600 | Combined hyperlipemia, familial (3) |
| 987 | LPL | 4023 | KIAA1377 | 57562 | 238600 | Hyperlipoproteinemia I (1) |
| 987 | LPL | 4023 | KIAA1377 | 57562 | 238600 | Lipoprotein lipase deficiency (3) |
| 987 | LPL | 4023 | LOC374923 | 374923 | 238600 | Chylomicronemia syndrome, familial (3) |
| 987 | LPL | 4023 | LOC374923 | 374923 | 238600 | Combined hyperlipemia, familial (3) |
| 987 | LPL | 4023 | LOC374923 | 374923 | 238600 | Hyperlipoproteinemia I (1) |
| 987 | LPL | 4023 | LOC374923 | 374923 | 238600 | Lipoprotein lipase deficiency (3) |
| 987 | LPL | 4023 | LUC7L2 | 51631 | 238600 | Chylomicronemia syndrome, familial (3) |
| 987 | LPL | 4023 | LUC7L2 | 51631 | 238600 | Combined hyperlipemia, familial (3) |
| 987 | LPL | 4023 | LUC7L2 | 51631 | 238600 | Hyperlipoproteinemia I (1) |
| 987 | LPL | 4023 | LUC7L2 | 51631 | 238600 | Lipoprotein lipase deficiency (3) |
| 987 | LPL | 4023 | PTPN4 | 5775 | 238600 | Chylomicronemia syndrome, familial (3) |
| 987 | LPL | 4023 | PTPN4 | 5775 | 238600 | Combined hyperlipemia, familial (3) |
| 987 | LPL | 4023 | PTPN4 | 5775 | 238600 | Hyperlipoproteinemia I (1) |
| 987 | LPL | 4023 | PTPN4 | 5775 | 238600 | Lipoprotein lipase deficiency (3) |
| 987 | LPL | 4023 | RPL18A | 6142 | 238600 | Chylomicronemia syndrome, familial (3) |
| 987 | LPL | 4023 | RPL18A | 6142 | 238600 | Combined hyperlipemia, familial (3) |
| 987 | LPL | 4023 | RPL18A | 6142 | 238600 | Hyperlipoproteinemia I (1) |
| 987 | LPL | 4023 | RPL18A | 6142 | 238600 | Lipoprotein lipase deficiency (3) |
| 988 | LPL | 4023 | ASC1p100 | 84164 | 238600 | Chylomicronemia syndrome, familial (3) |
| 988 | LPL | 4023 | ASC1p100 | 84164 | 238600 | Combined hyperlipemia, familial (3) |
| 988 | LPL | 4023 | ASC1p100 | 84164 | 238600 | Hypedipoproteinemia I (1) |
| 988 | LPL | 4023 | ASC1p100 | 84164 | 238600 | Lipoprotein lipase deficiency (3) |
| 988 | LPL | 4023 | COPS6 | 10980 | 238600 | Chylomicronemia syndrome, familial (3) |
| 988 | LPL | 4023 | COPS6 | 10980 | 238600 | Combined hyperlipemia, familial (3) |
| 988 | LPL | 4023 | COPS6 | 10980 | 238600 | Hyperlipoproteinemia I (1) |
| 988 | LPL | 4023 | COPS6 | 10980 | 238600 | Lipoprotein lipase deficiency (3) |
| 988 | LPL | 4023 | FLJ13855 | 65264 | 238600 | Chylomicronemia syndrome, familial (3) |
| 988 | LPL | 4023 | FLJ13855 | 65264 | 238600 | Combined hyperlipemia, familial (3) |
| 988 | LPL | 4023 | FLJ13855 | 65264 | 238600 | Hyperlipoproteinemia I (1) |
| 988 | LPL | 4023 | FLJ13855 | 65264 | 238600 | Lipoprotein lipase deficiency (3) |
| 988 | LPL | 4023 | KIAA1377 | 57562 | 238600 | Chylomicronemia syndrome, familial (3) |
| 988 | LPL | 4023 | KIAA1377 | 57562 | 238600 | Combined hyperlipemia, familial (3) |
| 988 | LPL | 4023 | KIAA1377 | 57562 | 238600 | Hyperlipoproteinemia I(1) |
| 988 | LPL | 4023 | KIAA1377 | 57562 | 238600 | Lipoprotein lipase deficiency (3) |
| 988 | LPL | 4023 | LOC374923 | 374923 | 238600 | Chylomicronemia syndrome, familial (3) |
| 988 | LPL | 4023 | LOC374923 | 374923 | 238600 | Combined hyperlipemia, familial (3) |
| 988 | LPL | 4023 | LOC374923 | 374923 | 238600 | Hyperlipoproteinemia I (1) |
| 988 | LPL | 4023 | LOC374923 | 374923 | 238600 | Lipoprotein lipase deficiency (3) |
| 988 | LPL | 4023 | LUC7L2 | 51631 | 238600 | Chylomicronemia syndrome, familial (3) |
| 988 | LPL | 4023 | LUC7L2 | 51631 | 238600 | Combined hyperlipemia, familial (3) |
| 988 | LPL | 4023 | LUC7L2 | 51631 | 238600 | Hyperlipoproteinemia I (1) |
| 988 | LPL | 4023 | LUC7L2 | 51631 | 238600 | Lipoprotein lipase deficiency (3) |
| 988 | LPL | 4023 | PTPN4 | 5775 | 238600 | Chylomicronemia syndrome, familial (3) |
| 988 | LPL | 4023 | PTPN4 | 5775 | 238600 | Combined hyperlipemia, familial (3) |
| 988 | LPL | 4023 | PTPN4 | 5775 | 238600 | Hyperlipoproteinemia I(1) |
| 988 | LPL | 4023 | PTPN4 | 5775 | 238600 | Lipoprotein lipase deficiency (3) |
| 988 | LPL | 4023 | RPL18A | 6142 | 238600 | Chylomicronemia syndrome, familial (3) |
| 988 | LPL | 4023 | RPL18A | 6142 | 238600 | Combined hyperlipemia, familial (3) |
| 988 | LPL | 4023 | RPL18A | 6142 | 238600 | Hyperlipoproteinemia I (1) |
| 988 | LPL | 4023 | RPL18A | 6142 | 238600 | Lipoprotein lipase deficiency (3) |
| 989 | LPP | 4026 | APR-3 | 51374 | 600700 | Leukemia, myeloid (3) |
| 989 | LPP | 4026 | APR-3 | 51374 | 600700 | Lipoma (3) |
| 989 | LPP | 4026 | ATP5J2 | 9551 | 600700 | Leukemia, myeloid (3) |
| 989 | LPP | 4026 | ATP5J2 | 9551 | 600700 | Lipoma (3) |
| 989 | LPP | 4026 | SHPRH | 257218 | 600700 | Leukemia, myeloid (3) |
| 989 | LPP | 4026 | SHPRH | 257218 | 600700 | Lipoma (3) |
| 1030 | MAPK81P1 | 9479 | RECQL5 | 9400 | 604641 | {Diabetes mellitus, noninsulin-dependent}, 125853 (3) |
| 1045 | MCOLN1 | 57192 | RFP | 5987 | 605248 | Mucolipidosis IV, 252650 (3) |
| 1045 | MCOLN1 | 57192 | SLC35E1 | 79939 | 605248 | Mucolipidosis IV, 252650 (3) |
| 1092 | MIF | 4282 | STRN4 | 29888 | 153620 | {Rheumatoid arthritis, systemic juvenile, susceptibility to}, 604302 (3) |
| 1093 | MKKS | 8195 | PTN | 5764 | 604896 | Bardet-Biedl syndrome 6, 209900 (3) |
| 1093 | MKKS | 8195 | PTN | 5764 | 604896 | McKusick-Kaufman syndrome, 236700 (3) |
| 1094 | MLF1 | 4291 | GRIK5 | 2901 | 601402 | Leukemia, myeloid, acute (1) |
| 1097 | MOCS2 | 4338 | ABCF3 | 55324 | 603708 | Molybdenum cofactor deficiency, type B, 252150 (3) |
| 1097 | MOCS2 | 4338 | RPL9 | 6133 | 603708 | Molybdenum cofactor deficiency, type B, 252150 (3) |
| 1102 | MPZ | 4359 | PINX1 | 54984 | 159440 | Charcot-Marie-Tooth disease, dominant intermediate 3, 607791 (3) |
| 1102 | MPZ | 4359 | PINX1 | 54984 | 159440 | Charcot-Marie-Tooth disease, type 1 B, 118200 (3) |
| 1102 | MPZ | 4359 | PINX1 | 54984 | 159440 | Charcot-Marie-Tooth disease, type 21, 607677 (3) |
| 1102 | MPZ | 4359 | PINX1 | 54984 | 159440 | Charcot-Marie-Tooth disease, type 2J, 607736 (3) |
| 1102 | MPZ | 4359 | PINX1 | 54984 | 159440 | Dejerine-Sottas syndrome, 145900 (3) |
| 1102 | MPZ | 4359 | PINX1 | 54984 | 159440 | Neuropathy, congenital hypomyelinating, 605253 (3) |
| 1102 | MPZ | 4359 | PINX1 | 54984 | 159440 | Roussy-Levy syndrome, 180800 (3) |
| 1120 | MSF | 10801 | RAB10 | 10890 | 604061 | Leukemia, acute myeloid, therapy-related (1) |
| 1120 | MSF | 10801 | RAB10 | 10890 | 604061 | Ovarian carcinoma (1) |
| 1126 | MUTYH | 4595 | LRSAM1 | 90678 | 604933 | Adenomas, multiple colorectal, 608456 (3) |
| 1130 | MYH9 | 4627 | ASC1p100 | 84164 | 160775 | Deafness, autosomal dominant 17, 603622 (3) |
| 1130 | MYH9 | 4627 | ASC1p100 | 84164 | 160775 | Epstein syndrome, 153650 (3) |
| 1130 | MYH9 | 4627 | ASC1p100 | 84164 | 160775 | Fechtner syndrome, 153640 (3) |
| 1130 | MYH9 | 4627 | ASC1p100 | 84164 | 160775 | May-Hegglin anomaly, 155100 (3) |
| 1130 | MYH9 | 4627 | ASC1p100 | 84164 | 160775 | Sebastian syndrome, 605249 (3) |
| 1130 | MYH9 | 4627 | MYL9 | 10398 | 160775 | Deafness, autosomal dominant 17, 603622 (3) |
| 1130 | MYH9 | 4627 | MYL9 | 10398 | 160775 | Epstein syndrome, 153650 (3) |
| 1130 | MYH9 | 4627 | MYL9 | 10398 | 160775 | Fechtner syndrome, 153640 (3) |
| 1130 | MYH9 | 4627 | MYL9 | 10398 | 160775 | May-Hegglin anomaly, 155100 (3) |
| 1130 | MYH9 | 4627 | MYL9 | 10398 | 160775 | Sebastian syndrome, 605249 (3) |
| 1131 | MYH9 | 4627 | ASC1p100 | 84164 | 160775 | Deafness, autosomal dominant 17, 603622 (3) |
| 1131 | MYH9 | 4627 | ASC1p100 | 84164 | 160775 | Epstein syndrome, 153650 (3) |
| 1131 | MYH9 | 4627 | ASC1p100 | 84164 | 160775 | Fechtner syndrome, 153640 (3) |
| 1131 | MYH9 | 4627 | ASC1p100 | 84164 | 160775 | May-Hegglin anomaly, 155100 (3) |
| 1131 | MYH9 | 4627 | ASC1p100 | 84164 | 160775 | Sebastian syndrome, 605249 (3) |
| 1131 | MYH9 | 4627 | MYL9 | 10398 | 160775 | Deafness, autosomal dominant 17, 603622 (3) |
| 1131 | MYH9 | 4627 | MYL9 | 10398 | 160775 | Epstein syndrome, 153650 (3) |
| 1131 | MYH9 | 4627 | MYL9 | 10398 | 160775 | Fechtner syndrome, 153640 (3) |
| 1131 | MYH9 | 4627 | MYL9 | 10398 | 160775 | May-Hegglin anomaly, 155100 (3) |
| 1131 | MYH9 | 4627 | MYL9 | 10398 | 160775 | Sebastian syndrome, 605249 (3) |
| 1132 | MYL2 | 4633 | LRSAM1 | 90678 | 160781 | Cardiomyopathy, familial hypertrophic, 10, 608758 (3) |
| 1132 | MYL2 | 4633 | LRSAM1 | 90678 | 160781 | Cardiomyopathy, hypertrophic, mid-left ventricular chamber type, 608758 (3) |
| 1137 | MYO15A | 51168 | EPS8 | 2059 | 602666 | Deafness, autosomal recessive 3, 600316 (3) |
| 1137 | MYO15A | 51168 | USP13 | 8975 | 602666 | Deafness, autosomal recessive 3, 600316 (3) |
| 1138 | MYO3A | 53904 | GRINL1A | 81488 | 606808 | Deafness, autosomal recessive 30, 607101 (3) |
| 1139 | MYO6 | 4646 | CALM2 | 805 | 600970 | Deafness, autosomal dominant 22, 606346 (3) |
| 1139 | MY06 | 4646 | CALM2 | 805 | 600970 | Deafness, autosomal recessive 37, 607821 (3) |
| 1139 | MYO6 | 4646 | CALM2 | 805 | 600970 | Deafness, sensorineural, with hypertrophic cardiomyopathy, 606346 (3) |
| 1146 | NAGS | 162417 | FLJ10315 | 55116 | 608300 | N-acetylglutamate synthase deficiency, 237310 (3) |
| 1152 | NDN | 4692 | ARSE | 415 | 602117 | Prader-Willi syndrome, 176270 (3) |
| 1153 | NDRG1 | 10397 | ARF4L | 379 | 605262 | Charcot-Marie-Tooth disease, type 4D, 601455 (3) |
| 1153 | NDRG1 | 10397 | PHYHIP | 9796 | 605262 | Charcot-Marie-Tooth disease, type 4D, 601455 (3) |
| 1154 | NDRG1 | 10397 | ARF4L | 379 | 605262 | Charcot-Marie-Tooth disease, type 4D, 601455 (3) |
| 1154 | NDRG1 | 10397 | PHYHIP | 9796 | 605262 | Charcot-Marie-Tooth disease, type 4D, 601455 (3) |
| 1162 | NDUFS7 | 374291 | ARRB2 | 409 | 601825 | Leigh syndrome, 256000 (3) |
| 1162 | NDUFS7 | 374291 | EN02 | 2026 | 601825 | Leigh syndrome, 256000 (3) |
| 1163 | NDUFS7 | 374291 | ARRB2 | 409 | 601825 | Leigh syndrome, 256000 (3) |
| 1163 | NDUFS7 | 374291 | EN02 | 2026 | 601825 | Leigh syndrome, 256000 (3) |
| 1164 | NDUFV2 | 4729 | CRMP1 | 1400 | 600532 | {Parkinson disease, susceptibility to}, 168600 (3) |
| 1164 | NDUFV2 | 4729 | HTATIP | 10524 | 600532 | {Parkinson disease, susceptibility to}, 168600 (3) |
| 1164 | NDUFV2 | 4729 | SP110 | 3431 | 600532 | {Parkinson disease, susceptibility to}, 168600 (3) |
| 1169 | NEFL | 4747 | CDK5RAP2 | 55755 | 162280 | Charcot-Marie-Tooth disease, type 1F, 607734 (3) |
| 1169 | NEFL | 4747 | CDK5RAP2 | 55755 | 162280 | Charcot-Marie-Tooth disease, type 2E, 607684 (3) |
| 1171 | NEU1 | 4758 | EEF1A1 | 1915 | 608272 | Sialidosis, type I, 256550 (3) |
| 1171 | NEU1 | 4758 | EEF1A1 | 1915 | 608272 | Sialidosis, type II, 256550 (3) |
| 1174 | NFKBIA | 4792 | LCMR1 | 219541 | 164008 | Ectodermal dysplasia, anhidrotic, with T-cell immunodeficiency (3) |
| 1187 | NOTCH1 | 4851 | G22P1 | 2547 | 190198 | Leukemia, T-cell acute lymphoblastic (2) |
| 1189 | NP | 4860 | APLP1 | 333 | 164050 | Nucleoside phosphorylase deficiency, immunodeficiency due to (3) |
| 1189 | NP | 4860 | DKFZP564O0523 | 84060 | 164050 | Nucleoside phosphorylase deficiency, immunodeficiency due to (3) |
| 1189 | NP | 4860 | MDS025 | 60492 | 164050 | Nucleoside phosphorylase deficiency, immunodeficiency due to (3) |
| 1189 | NP | 4860 | RIF1 | 55791 | 164050 | Nucleoside phosphorylase deficiency, immunodeficiency due to (3) |
| 1189 | NP | 4860 | TP53 | 7157 | 164050 | Nucleoside phosphorylase deficiency, immunodeficiency due to (3) |
| 1189 | NP | 4860 | ZHX1 | 11244 | 164050 | Nucleoside phosphorylase deficiency, immunodeficiency due to (3) |
| 1194 | NRAS | 4893 | PLCE1 | 51196 | 164790 | Colorectal cancer (3) |
| 1208 | OAT | 4942 | C2orf18 | 54978 | 258870 | Gyrate atrophy of choroid and retina with omithinemia, B6 responsive or unresponsive (3) |
| 1210 | OFD1 | 8481 | GASP | 9737 | 300170 | Oral-facial-digital syndrome 1, 311200 (3) |
| 1210 | OFD1 | 8481 | ITGB4BP | 3692 | 300170 | Oral-facial-digital syndrome 1, 311200 (3) |
| 1210 | OFD1 | 8481 | KIAA1128 | 54462 | 300170 | Oral-facial-digital syndrome 1, 311200 (3) |
| 1210 | OFD1 | 8481 | KIAA1377 | 57562 | 300170 | Oral-facial-digital syndrome 1, 311200 (3) |
| 1210 | OFD1 | 8481 | LUC7L2 | 51631 | 300170 | Oral-facial-digital syndrome 1, 311200 (3) |
| 1211 | OGG1 | 4968 | CHGB | 1114 | 601982 | Renal cell carcinoma, clear cell, 144700 (3) |
| 1222 | PAFAH1B1 | 5048 | KARS | 3735 | 601545 | Lissencephaly-1.607432 (3) |
| 1222 | PAFAH1B1 | 5048 | KARS | 3735 | 601545 | Subcortical laminar heterotopia (3) |
| 1233 | PARK2 | 5071 | FLJ23825 | 284004 | 602544 | {Leprosy, susceptibility to}, 607572 (3) |
| 1233 | PARK2 | 5071 | FLJ23825 | 284004 | 602544 | Adenocarcinoma of lung, somatic, 211980 (3) |
| 1233 | PARK2 | 5071 | FLJ23825 | 284004 | 602544 | Adenocarcinoma, ovarian, somatic (3) |
| 1233 | PARK2 | 5071 | FLJ23825 | 284004 | 602544 | Parkinson disease, juvenile, type 2, 600116 (3) |
| 1233 | PARK2 | 5071 | LOC56901 | 56901 | 602544 | {Leprosy, susceptibility to}, 607572 (3) |
| 1233 | PARK2 | 5071 | LOC56901 | 56901 | 602544 | Adenocarcinoma of lung, somatic, 211980 (3) |
| 1233 | PARK2 | 5071 | LOC56901 | 56901 | 602544 | Adenocarcinoma, ovarian, somatic (3) |
| 1233 | PARK2 | 5071 | LOC56901 | 56901 | 602544 | Parkinson disease, juvenile, type 2, 600116 (3) |
| 1233 | PARK2 | 5071 | PAFAH1B2 | 5049 | 602544 | {Leprosy, susceptibility to}, 607572 (3) |
| 1233 | PARK2 | 5071 | PAFAH1B2 | 5049 | 602544 | Adenocarcinoma of lung, somatic, 211980 (3) |
| 1233 | PARK2 | 5071 | PAFAH1B2 | 5049 | 602544 | Adenocarcinoma, ovarian, somatic (3) |
| 1233 | PARK2 | 5071 | PAFAH1B2 | 5049 | 602544 | Parkinson disease, juvenile, type 2, 600116 (3) |
| 1233 | PARK2 | 5071 | RAC1 | 5879 | 602544 | {Leprosy, susceptibility to}, 607572 (3) |
| 1233 | PARK2 | 5071 | RAC1 | 5879 | 602544 | Adenocarcinoma of lung, somatic, 211980 (3) |
| 1233 | PARK2 | 5071 | RAC1 | 5879 | 602544 | Adenocarcinoma, ovarian, somatic (3) |
| 1233 | PARK2 | 5071 | RAC1 | 5879 | 602544 | Parkinson disease, juvenile, type 2, 600116 (3) |
| 1233 | PARK2 | 5071 | RAD1 | 5810 | 602544 | {Leprosy, susceptibility to}, 607572 (3) |
| 1233 | PARK2 | 5071 | RAD1 | 5810 | 602544 | Adenocarcinoma of lung, somatic, 211980 (3) |
| 1233 | PARK2 | 5071 | RAD1 | 5810 | 602544 | Adenocarcinoma, ovarian, somatic (3) |
| 1233 | PARK2 | 5071 | RAD1 | 5810 | 602544 | Parkinson disease, juvenile, type 2, 600116 (3) |
| 1233 | PARK2 | 5071 | RGS2 | 5997 | 602544 | {Leprosy, susceptibility to}, 607572 (3) |
| 1233 | PARK2 | 5071 | RGS2 | 5997 | 602544 | Adenocarcinoma of lung, somatic, 211980 (3) |
| 1233 | PARK2 | 5071 | RGS2 | 5997 | 602544 | Adenocarcinoma, ovarian, somatic (3) |
| 1233 | PARK2 | 5071 | RGS2 | 5997 | 602544 | Parkinson disease, juvenile, type 2, 600116 (3) |
| 1234 | PARK2 | 5071 | FLJ23825 | 284004 | 602544 | {Leprosy, susceptibility to}, 607572 (3) |
| 1234 | PARK2 | 5071 | FLJ23825 | 284004 | 602544 | Adenocarcinoma of lung, somatic, 211980 (3) |
| 1234 | PARK2 | 5071 | FLJ23825 | 284004 | 602544 | Adenocarcinoma, ovarian, somatic (3) |
| 1234 | PARK2 | 5071 | FLJ23825 | 284004 | 602544 | Parkinson disease, juvenile, type 2, 600116 (3) |
| 1234 | PARK2 | 5071 | LOC56901 | 56901 | 602544 | {Leprosy, susceptibility to}, 607572 (3) |
| 1234 | PARK2 | 5071 | LOC56901 | 56901 | 602544 | Adenocarcinoma of lung, somatic, 211980 (3) |
| 1234 | PARK2 | 5071 | LOC56901 | 56901 | 602544 | Adenocarcinoma, ovarian, somatic (3) |
| 1234 | PARK2 | 5071 | LOC56901 | 56901 | 602544 | Parkinson disease, juvenile, type 2, 600116 (3) |
| 1234 | PARK2 | 5071 | PAFAH1B2 | 5049 | 602544 | {Leprosy, susceptibility to}, 607572 (3) |
| 1234 | PARK2 | 5071 | PAFAH1B2 | 5049 | 602544 | Adenocarcinoma of lung, somatic, 211980 (3) |
| 1234 | PARK2 | 5071 | PAFAH1B2 | 5049 | 602544 | Adenocarcinoma, ovarian, somatic (3) |
| 1234 | PARK2 | 5071 | PAFAH1B2 | 5049 | 602544 | Parkinson disease, juvenile, type 2, 600116 (3) |
| 1234 | PARK2 | 5071 | RAC1 | 5879 | 602544 | {Leprosy, susceptibility to}, 607572 (3) |
| 1234 | PARK2 | 5071 | RAC1 | 5879 | 602544 | Adenocarcinoma of lung, somatic, 211980 (3) |
| 1234 | PARK2 | 5071 | RAC1 | 5879 | 602544 | Adenocarcinoma, ovarian, somatic (3) |
| 1234 | PARK2 | 5071 | RAC1 | 5879 | 602544 | Parkinson disease, juvenile, type 2, 600116 (3) |
| 1234 | PARK2 | 5071 | RAD1 | 5810 | 602544 | {Leprosy, susceptibility to}, 607572 (3) |
| 1234 | PARK2 | 5071 | RAD1 | 5810 | 602544 | Adenocarcinoma of lung, somatic, 211980 (3) |
| 1234 | PARK2 | 5071 | RAD1 | 5810 | 602544 | Adenocarcinoma, ovarian, somatic (3) |
| 1234 | PARK2 | 5071 | RAD1 | 5810 | 602544 | Parkinson disease, juvenile, type 2, 600116 (3) |
| 1234 | PARK2 | 5071 | RGS2 | 5997 | 602544 | {Leprosy, susceptibility to}, 607572 (3) |
| 1234 | PARK2 | 5071 | RGS2 | 5997 | 602544 | Adenocarcinoma of lung, somatic, 211980 (3) |
| 1234 | PARK2 | 5071 | RGS2 | 5997 | 602544 | Adenocarcinoma, ovarian, somatic (3) |
| 1234 | PARK2 | 5071 | RGS2 | 5997 | 602544 | Parkinson disease, juvenile, type 2, 600116 (3) |
| 1235 | PARK2 | 5071 | FLJ23825 | 284004 | 602544 | {Leprosy, susceptibility to}, 607572 (3) |
| 1235 | PARK2 | 5071 | FLJ23825 | 284004 | 602544 | Adenocarcinoma of lung, somatic, 211980 (3) |
| 1235 | PARK2 | 5071 | FLJ23825 | 284004 | 602544 | Adenocarcinoma, ovarian, somatic (3) |
| 1235 | PARK2 | 5071 | FLJ23825 | 284004 | 602544 | Parkinson disease, juvenile, type 2, 600116 (3) |
| 1235 | PARK2 | 5071 | LOC56901 | 56901 | 602544 | {Leprosy, susceptibility to}, 607572 (3) |
| 1235 | PARK2 | 5071 | LOC56901 | 56901 | 602544 | Adenocarcinoma of lung, somatic, 211980 (3) |
| 1235 | PARK2 | 5071 | LOC56901 | 56901 | 602544 | Adenocarcinoma, ovarian, somatic (3) |
| 1235 | PARK2 | 5071 | LOC56901 | 56901 | 602544 | Parkinson disease, juvenile, type 2, 600116 (3) |
| 1235 | PARK2 | 5071 | PAFAH1B2 | 5049 | 602544 | {Leprosy, susceptibility to}, 607572 (3) |
| 1235 | PARK2 | 5071 | PAFAH1B2 | 5049 | 602544 | Adenocarcinoma of lung, somatic, 211980 (3) |
| 1235 | PARK2 | 5071 | PAFAH1B2 | 5049 | 602544 | Adenocarcinoma, ovarian, somatic (3) |
| 1235 | PARK2 | 5071 | PAFAH1B2 | 5049 | 602544 | Parkinson disease, juvenile, type 2, 600116 (3) |
| 1235 | PARK2 | 5071 | RAC1 | 5879 | 602544 | {Leprosy, susceptibility to}, 607572 (3) |
| 1235 | PARK2 | 5071 | RAC1 | 5879 | 602544 | Adenocarcinoma of lung, somatic, 211980 (3) |
| 1235 | PARK2 | 5071 | RAC1 | 5879 | 602544 | Adenocarcinoma, ovarian, somatic (3) |
| 1235 | PARK2 | 5071 | RAC1 | 5879 | 602544 | Parkinson disease, juvenile, type 2, 600116 (3) |
| 1235 | PARK2 | 5071 | RAD1 | 5810 | 602544 | {Leprosy, susceptibility to}, 607572 (3) |
| 1235 | PARK2 | 5071 | RAD1 | 5810 | 602544 | Adenocarcinoma of lung, somatic, 211980 (3) |
| 1235 | PARK2 | 5071 | RAD1 | 5810 | 602544 | Adenocarcinoma, ovarian, somatic (3) |
| 1235 | PARK2 | 5071 | RAD1 | 5810 | 602544 | Parkinson disease, juvenile, type 2, 600116 (3) |
| 1235 | PARK2 | 5071 | RGS2 | 5997 | 602544 | {Leprosy, susceptibility to}, 607572 (3) |
| 1235 | PARK2 | 5071 | RGS2 | 5997 | 602544 | Adenocarcinoma of lung, somatic, 211980 (3) |
| 1235 | PARK2 | 5071 | RGS2 | 5997 | 602544 | Adenocarcinoma, ovarian, somatic (3) |
| 1235 | PARK2 | 5071 | RGS2 | 5997 | 602544 | Parkinson disease, juvenile, type 2, 600116 (3) |
| 1254 | PDHA1 | 5160 | ITGB4BP | 3692 | 300502 | |
| 1263 | PEO1 | 56652 | FTS | 64400 | 606075 | Progressive external ophthalmoplegia with mitochondrial DNA deletions, 157640 (3) |
| 1263 | PEO1 | 56652 | LOC222019 | 222019 | 606075 | Progressive external ophthalmoplegia with mitochondrial DNA deletions, 157640 (3) |
| 1263 | PEO1 | 56652 | FTS | 64400 | 606075 | Progressive external ophthalmoplegia with mitochondrial DNA deletions, 157640 (3) |
| 1263 | PEO1 | 56652 | LOC222019 | 222019 | 606075 | Progressive external ophthalmoplegia with mitochondrial DNA deletions, 157640 (3) |
| 1266 | PEX5 | 5830 | RNUT1 | 10073 | 600414 | Adrenoleukodystrophy, neonatal, 202370 (3) |
| 1266 | PEX5 | 5830 | RNUT1 | 10073 | 600414 | Zellweger syndrome, 214100 (3) |
| 1268 | PFKL | 5211 | COPS6 | 10980 | 171860 | Hemolytic anemia due to phosphofructokinase deficiency (1) |
| 1281 | PHYH | 5264 | TOM1L1 | 10040 | 602026 | Refsum disease, 266500 (3) |
| 1304 | PLA2G2A | 5320 | CRMP1 | 1400 | 172411 | {?Colorectal cancer, resistance to} (1) |
| 1333 | POR | 5447 | G22P1 | 2547 | 124015 | Adrenal hyperplasia, congenital, due to combined P450C17 and P450C21 deficiency, 201750 (3) |
| 1333 | POR | 5447 | G22P1 | 2547 | 124015 | Antley-Bixler syndrome, 207410 (3) |
| 1333 | POR | 5447 | G22P1 | 2547 | 124015 | Disordered steroidogenesis, isolated (3) |
| 1348 | PPOX | 5498 | HAP1 | 9001 | 600923 | Porphyria variegata, 176200 (3) |
| 1348 | PPOX | 5498 | IMMT | 10989 | 600923 | Porphyria variegata, 176200 (3) |
| 1359 | PQBP1 | 10084 | C14orf1 | 11161 | 300463 | Mental retardation, X-linked 55, 309470 (3) |
| 1359 | PQBP1 | 10084 | C14orf1 | 11161 | 300463 | Renpenning syndrome, 309500 (3) |
| 1359 | PQBP1 | 10084 | C14orf1 | 11161 | 300463 | Sutherland-Haan syndrome, 309470 (3) |
| 1359 | PQBP1 | 10084 | CGI-125 | 51003 | 300463 | Mental retardation, X-linked 55, 309470 (3) |
| 1359 | PQBP1 | 10084 | CGI-125 | 51003 | 300463 | Renpenning syndrome, 309500 (3) |
| 1359 | PQBP1 | 10084 | CGI-125 | 51003 | 300463 | Sutherland-Haan syndrome, 309470 (3) |
| 1359 | PQBP1 | 10084 | CLTB | 1212 | 300463 | Mental retardation, X-linked 55, 309470 (3) |
| 1359 | PQBP1 | 10084 | CLTB | 1212 | 300463 | Renpenning syndrome, 309500 (3) |
| 1359 | PQBP1 | 10084 | CLTB | 1212 | 300463 | Sutherland-Haan syndrome, 309470 (3) |
| 1359 | PQBP1 | 10084 | EEF1A1 | 1915 | 300463 | Mental retardation, X-linked 55, 309470 (3) |
| 1359 | PQBP1 | 10084 | EEF1A1 | 1915 | 300463 | Renpenning syndrome, 309500 (3) |
| 1359 | PQBP1 | 10084 | EEF1A1 | 1915 | 300463 | Sutherland-Haan syndrome, 309470 (3) |
| 1359 | PQBP1 | 10084 | RAB8A | 4218 | 300463 | Mental retardation, X-linked 55, 309470 (3) |
| 1359 | PQBP1 | 10084 | RAB8A | 4218 | 300463 | Renpenning syndrome, 309500 (3) |
| 1359 | PQBP1 | 10084 | RAB8A | 4218 | 300463 | Sutherland-Haan syndrome, 309470 (3) |
| 1359 | PQBP1 | 10084 | RIF1 | 55791 | 300463 | Mental retardation, X-linked 55, 309470 (3) |
| 1359 | PQBP1 | 10084 | RIF1 | 55791 | 300463 | Renpenning syndrome, 309500 (3) |
| 1359 | PQBP1 | 10084 | RIF1 | 55791 | 300463 | Sutherland-Haan syndrome, 309470 (3) |
| 1363 | PRF1 | 5551 | DDX24 | 57062 | 170280 | Hemophagocytic lymphohistiocytosis, familial, 2, 603553 (3) |
| 1369 | PRKCSH | 5589 | ARF4L | 379 | 177060 | Polycystic liver disease, 174050 (3) |
| 1369 | PRKCSH | 5589 | C7orf20 | 51608 | 177060 | Polycystic liver disease, 174050 (3) |
| 1369 | PRKCSH | 5589 | RGS2 | 5997 | 177060 | Polycystic liver disease, 174050 (3) |
| 1373 | PRNP | 5621 | BAT3 | 7917 | 176640 | Creutzfeldt-Jakob disease, 123400 (3) |
| 1373 | PRNP | 5621 | BAT3 | 7917 | 176640 | Gerstmann-Straussler disease, 137440 (3) |
| 1373 | PRNP | 5621 | BAT3 | 7917 | 176640 | Huntington disease-like 1, 603218 (3) |
| 1373 | PRNP | 5621 | BAT3 | 7917 | 176640 | Insomnia, fatal familial, 600072 (3) |
| 1373 | PRNP | 5621 | BAT3 | 7917 | 176640 | Prion disease with protracted course, 606688 (3) |
| 1379 | PSAP | 5660 | CELSR1 | 9620 | 176801 | Combined SAP deficiency (3) |
| 1379 | PSAP | 5660 | CELSR1 | 9620 | 176801 | Gaucher disease, variant form (3) |
| 1379 | PSAP | 5660 | CELSR1 | 9620 | 176801 | Metachromatic leukodystrophy due to deficiency of SAP-1 (3) |
| 1379 | PSAP | 5660 | COPS6 | 10980 | 176801 | Combined SAP deficiency (3) |
| 1379 | PSAP | 5660 | COPS6 | 10980 | 176801 | Gaucher disease, variant form (3) |
| 1379 | PSAP | 5660 | COPS6 | 10980 | 176801 | Metachromatic leukodystrophy due to deficiency of SAP-1 (3) |
| 1379 | PSAP | 5660 | DKFZp761 P0423 | 157285 | 176801 | Combined SAP deficiency (3) |
| 1379 | PSAP | 5660 | DKFZp761 P0423 | 157285 | 176801 | Gaucher disease, variant form (3) |
| 1379 | PSAP | 5660 | DKFZp761 P0423 | 157285 | 176801 | Metachromatic leukodystrophy due to deficiency of SAP-1 (3) |
| 1379 | PSAP | 5660 | MAFF | 23764 | 176801 | Combined SAP deficiency (3) |
| 1379 | PSAP | 5660 | MAFF | 23764 | 176801 | Gaucher disease, variant form (3) |
| 1379 | PSAP | 5660 | MAFF | 23764 | 176801 | Metachromatic leukodystrophy due to deficiency of SAP-1 (3) |
| 1380 | PSAP | 5660 | CELSR1 | 9620 | 176801 | Combined SAP deficiency (3) |
| 1380 | PSAP | 5660 | CELSR1 | 9620 | 176801 | Gaucher disease, variant form (3) |
| 1380 | PSAP | 5660 | CELSR1 | 9620 | 176801 | Metachromatic leukodystrophy due to deficiency of SAP-1 (3) |
| 1380 | PSAP | 5660 | COPS6 | 10980 | 176801 | Combined SAP deficiency (3) |
| 1380 | PSAP | 5660 | COPS6 | 10980 | 176801 | Gaucher disease, variant form (3) |
| 1380 | PSAP | 5660 | COPS6 | 10980 | 176801 | Metachromatic leukodystrophy due to deficiency of SAP-1 (3) |
| 1380 | PSAP | 5660 | DKFZp761 P0423 | 157285 | 176801 | Combined SAP deficiency (3) |
| 1380 | PSAP | 5660 | DKFZp761 P0423 | 157285 | 176801 | Gaucher disease, variant form (3) |
| 1380 | PSAP | 5660 | DKFZp761 P0423 | 157285 | 176801 | Metachromatic leukodystrophy due to deficiency of SAP-1 (3) |
| 1380 | PSAP | 5660 | MAFF | 23764 | 176801 | Combined SAP deficiency (3) |
| 1380 | PSAP | 5660 | MAFF | 23764 | 176801 | Gaucher disease, variant form (3) |
| 1380 | PSAP | 5660 | MAFF | 23764 | 176801 | Metachromatic leukodystrophy due to deficiency of SAP-1 (3) |
| 1404 | PTEN | 5728 | ANG | 283 | 601728 | {Prostate cancer}, 176807 (3) |
| 1404 | PTEN | 5728 | ANG | 283 | 601728 | Bannayan-Riley-Ruvalcaba syndrome, 153480 (3) |
| 1404 | PTEN | 5728 | ANG | 283 | 601728 | Bannayan-Zonana syndrome, 153480 (3) |
| 1404 | PTEN | 5728 | ANG | 283 | 601728 | Cowden disease, 158350 (3) |
| 1404 | PTEN | 5728 | ANG | 283 | 601728 | Endometrial carcinoma (3) |
| 1404 | PTEN | 5728 | ANG | 283 | 601728 | Lhermitte-Duclos syndrome (3) |
| 1404 | PTEN | 5728 | ANG | 283 | 601728 | Meningioma, 156100 (3) |
| 1404 | PTEN | 5728 | ANG | 283 | 601728 | Oligodendroglioma, 137800 (3) |
| 1404 | PTEN | 5728 | ANG | 283 | 601728 | Proteus syndr |
| 1404 | PTEN | 5728 | ANG | 283 | 601728 | Thyroid carcinoma, follicular, 188470 (3) |
| 1404 | PTEN | 5728 | ANG | 283 | 601728 | VATER association with hydrocephalus, 276950 (3) |
| 1404 | PTEN | 5728 | CHGB | 1114 | 601728 | {Prostate cancer}, 176807 (3) |
| 1404 | PTEN | 5728 | CHGB | 1114 | 601728 | Bannayan-Riley-Ruvalcaba syndrome, 153480 (3) |
| 1404 | PTEN | 5728 | CHGB | 1114 | 601728 | Bannayan-Zonana syndrome, 153480 (3) |
| 1404 | PTEN | 5728 | CHGB | 1114 | 601728 | Cowden disease, 158350 (3) |
| 1404 | PTEN | 5728 | CHGB | 1114 | 601728 | Endometrial carcinoma (3) |
| 1404 | PTEN | 5728 | CHGB | 1114 | 601728 | Lhermitte-Duclos syndrome (3) |
| 1404 | PTEN | 5728 | CHGB | 1114 | 601728 | Meningioma, 156100 (3) |
| 1404 | PTEN | 5728 | CHGB | 1114 | 601728 | Oligodendroglioma, 137800 (3) |
| 1404 | PTEN | 5728 | CHGB | 1114 | 601728 | Proteus syndr |
| 1404 | PTEN | 5728 | CHGB | 1114 | 601728 | Thyroid carcinoma, follicular, 188470 (3) |
| 1404 | PTEN | 5728 | CHGB | 1114 | 601728 | VATER association with hydrocephalus, 276950 (3) |
| 1404 | PTEN | 5728 | COPS6 | 10980 | 601728 | {Prostate cancer}, 176807 (3) |
| 1404 | PTEN | 5728 | COPS6 | 10980 | 601728 | Bannayan-Riley-Ruvalcaba syndrome, 153480 (3) |
| 1404 | PTEN | 5728 | COPS6 | 10980 | 601728 | Bannayan-Zonana syndrome, 153480 (3) |
| 1404 | PTEN | 5728 | COPS6 | 10980 | 601728 | Cowden disease, 158350 (3) |
| 1404 | PTEN | 5728 | COPS6 | 10980 | 601728 | Endometrial carcinoma (3) |
| 1404 | PTEN | 5728 | COPS6 | 10980 | 601728 | Lhermitte-Duclos syndrome (3) |
| 1404 | PTEN | 5728 | COPS6 | 10980 | 601728 | Meningioma, 156100 (3) |
| 1404 | PTEN | 5728 | COPS6 | 10980 | 601728 | Oligodendroglioma, 137800 (3) |
| 1404 | PTEN | 5728 | COPS6 | 10980 | 601728 | Proteus syndr |
| 1404 | PTEN | 5728 | COPS6 | 10980 | 601728 | Thyroid carcinoma, follicular, 188470 (3) |
| 1404 | PTEN | 5728 | COPS6 | 10980 | 601728 | VATER association with hydrocephalus, 276950 (3) |
| 1404 | PTEN | 5728 | HBA1 | 3039 | 601728 | {Prostate cancer}, 176807 (3) |
| 1404 | PTEN | 5728 | HBA1 | 3039 | 601728 | Bannayan-Riley-Ruvalcaba syndrome, 153480 (3) |
| 1404 | PTEN | 5728 | HBA1 | 3039 | 601728 | Bannayan-Zonana syndrome, 153480 (3) |
| 1404 | PTEN | 5728 | HBA1 | 3039 | 601728 | Cowden disease, 158350 (3) |
| 1404 | PTEN | 5728 | HBA1 | 3039 | 601728 | Endometrial carcinoma (3) |
| 1404 | PTEN | 5728 | HBA1 | 3039 | 601728 | Lhermitte-Duclos syndrome (3) |
| 1404 | PTEN | 5728 | HBA1 | 3039 | 601728 | Meningioma, 156100 (3) |
| 1404 | PTEN | 5728 | HBA1 | 3039 | 601728 | Oligodendroglioma, 137800 (3) |
| 1404 | PTEN | 5728 | HBA1 | 3039 | 601728 | Proteus syndr |
| 1404 | PTEN | 5728 | HBA1 | 3039 | 601728 | Thyroid carcinoma, follicular, 188470 (3) |
| 1404 | PTEN | 5728 | HBA1 | 3039 | 601728 | VATER association with hydrocephalus, 276950 (3) |
| 1404 | PTEN | 5728 | SDCCAG16 | 10813 | 601728 | {Prostate cancer}, 176807 (3) |
| 1404 | PTEN | 5728 | SDCCAG16 | 10813 | 601728 | Bannayan-Riley-Ruvalcaba syndrome, 153480 (3) |
| 1404 | PTEN | 5728 | SDCCAG16 | 10813 | 601728 | Bannayan-Zonana syndrome, 153480 (3) |
| 1404 | PTEN | 5728 | SDCCAG16 | 10813 | 601728 | Cowden disease, 158350 (3) |
| 1404 | PTEN | 5728 | SDCCAG16 | 10813 | 601728 | Endometrial carcinoma (3) |
| 1404 | PTEN | 5728 | SDCCAG16 | 10813 | 601728 | Lhermitte-Duclos syndrome (3) |
| 1404 | PTEN | 5728 | SDCCAG16 | 10813 | 601728 | Meningioma, 156100 (3) |
| 1404 | PTEN | 5728 | SDCCAG16 | 10813 | 601728 | Oligodendroglioma, 137800 (3) |
| 1404 | PTEN | 5728 | SDCCAG16 | 10813 | 601728 | Proteus syndr |
| 1404 | PTEN | 5728 | SDCCAG16 | 10813 | 601728 | Thyroid carcinoma, follicular, 188470 (3) |
| 1404 | PTEN | 5728 | SDCCAG16 | 10813 | 601728 | VATER association with hydrocephalus, 276950 (3) |
| 1405 | PTH | 5741 | BAT3 | 7917 | 168450 | Hypoparathyroidism, autosomal dominant(3) |
| 1405 | PTH | 5741 | BAT3 | 7917 | 168450 | Hypoparathyroidism, autosomal recessive (3) |
| 1405 | PTH | 5741 | FEZ1 | 9638 | 168450 | Hypoparathyroidism, autosomal dominant (3) |
| 1405 | PTH | 5741 | FEZ1 | 9638 | 168450 | Hypoparathyroidism, autosomal recessive (3) |
| 1420 | PTS | 5805 | PTS | 5805 | 261640 | Phenylketonuria due to PTS deficiency (3) |
| 1423 | PXMP3 | 5828 | C14orf1 | 11161 | 170993 | Refsum disease, infantile form, 266510 (3) |
| 1423 | PXMP3 | 5828 | C14orf1 | 11161 | 170993 | Zellweger syndrome-3 (3) |
| 1423 | PXMP3 | 5828 | TLE1 | 7088 | 170993 | Refsum disease, infantile form, 266510 (3) |
| 1423 | PXMP3 | 5828 | TLE1 | 7088 | 170993 | Zellweger syndrome-3 (3) |
| 1432 | RAB27A | 5873 | C14orf1 | 11161 | 603868 | Griscelli syndrome, type 2, 607624 (3) |
| 1432 | RAB27A | 5873 | COPS6 | 10980 | 603868 | Griscelli syndrome, type 2, 607624 (3) |
| 1432 | RAB27A | 5873 | DKFZP564O0523 | 84060 | 603868 | Griscelli syndrome, type 2, 607624 (3) |
| 1432 | RAB27A | 5873 | EEF1A1 | 1915 | 603868 | Griscelli syndrome, type 2, 607624 (3) |
| 1432 | RAB27A | 5873 | GDF9 | 2661 | 603868 | Griscelli syndrome, type 2, 607624 (3) |
| 1432 | RAB27A | 5873 | KIAA1377 | 57562 | 603868 | Griscelli syndrome, type 2, 607624 (3) |
| 1432 | RAB27A | 5873 | RIF1 | 55791 | 603868 | Griscelli syndrome, type 2, 607624 (3) |
| 1432 | RAB27A | 5873 | ZNF145 | 7704 | 603868 | Griscelli syndrome, type 2, 607624 (3) |
| 1444 | RAD51 | 5888 | CHD3 | 1107 | 179617 | {Breast cancer, susceptibility to}, 114480 (3) |
| 1444 | RAD51 | 5888 | VIM | 7431 | 179617 | {Breast cancer, susceptibility to}, 114480 (3) |
| 1464 | RECQL4 | 9401 | SPUVE | 11098 | 603780 | RAPADILINO syndrome, 266280 (3) |
| 1464 | RECQL4 | 9401 | SPUVE | 11098 | 603780 | Rothmund-Thomson syndrome, 268400 (3) |
| 1467 | REN | 5972 | KCTD15 | 79047 | 179820 | [Hyperproreninemia] (3) |
| 1537 | RRAS2 | 22800 | G22P1 | 2547 | 600098 | Ovarian carcinoma (3) |
| 1537 | RRAS2 | 22800 | LGALS4 | 3960 | 600098 | Ovarian carcinoma (3) |
| 1537 | RRAS2 | 22800 | PAWR | 5074 | 600098 | Ovarian carcinoma (3) |
| 1557 | SAT | 6303 | ABCB8 | 11194 | 313020 | Keratosis follicularis spinulosa decalvans, 308800 (3) |
| 1557 | SAT | 6303 | APLP1 | 333 | 313020 | Keratosis follicularis spinulosa decalvans, 308800 (3) |
| 1557 | SAT | 6303 | B4GALT3 | 8703 | 313020 | Keratosis follicularis spinulosa decalvans, 308800 (3) |
| 1557 | SAT | 6303 | C14orf1 | 11161 | 313020 | Keratosis follicularis spinulosa decalvans, 308800 (3) |
| 1557 | SAT | 6303 | C9orf46 | 55848 | 313020 | Keratosis follicularis spinulosa decalvans, 308800 (3) |
| 1557 | SAT | 6303 | CGI-125 | 51003 | 313020 | Keratosis follicularis spinulosa decalvans, 308800 (3) |
| 1557 | SAT | 6303 | CHD3 | 1107 | 313020 | Keratosis follicularis spinulosa decalvans, 308800 (3) |
| 1557 | SAT | 6303 | COPS6 | 10980 | 313020 | Keratosis follicularis spinulosa decalvans, 308800 (3) |
| 1557 | SAT | 6303 | CRMP1 | 1400 | 313020 | Keratosis follicularis spinulosa decalvans, 308800 (3) |
| 1557 | SAT | 6303 | DGKZ | 8525 | 313020 | Keratosis follicularis spinulosa decalvans, 308800 (3) |
| 1557 | SAT | 6303 | DKFZP564O0523 | 84060 | 313020 | Keratosis follicularis spinulosa decalvans, 308800 (3) |
| 1557 | SAT | 6303 | EEF1G | 1937 | 313020 | Keratosis follicularis spinulosa decalvans, 308800 (3) |
| 1557 | SAT | 6303 | EPHB6 | 2051 | 313020 | Keratosis follicularis spinulosa decalvans, 308800 (3) |
| 1557 | SAT | 6303 | FLJ13391 | 84141 | 313020 | Keratosis follicularis spinulosa decalvans, 308800 (3) |
| 1557 | SAT | 6303 | G451P3 | | 313020 | Keratosis follicularis spinulosa decalvans, 308800 (3) |
| 1557 | SAT | 6303 | GBP2 | 2634 | 313020 | Keratosis follicularis spinulosa decalvans, 308800 (3) |
| 1557 | SAT | 6303 | GDF9 | 2661 | 313020 | Keratosis follicularis spinulosa decalvans, 308800 (3) |
| 1557 | SAT | 6303 | HCBP6 | 65991 | 313020 | Keratosis follicularis spinulosa decalvans, 308800 (3) |
| 1557 | SAT | 6303 | HSPE1 | 3336 | 313020 | Keratosis follicularis spinulosa decalvans, 308800 (3) |
| 1557 | SAT | 6303 | HTATIP | 10524 | 313020 | Keratosis follicularis spinulosa decalvans, 308800 (3) |
| 1557 | SAT | 6303 | ITGB1BP3 | 27231 | 313020 | Keratosis follicularis spinulosa decalvans, 308800 (3) |
| 1557 | SAT | 6303 | KCNA | 3739 | 313020 | Keratosis follicularis spinulosa decalvans, 308800 (3) |
| 1557 | SAT | 6303 | KIAA1377 | 57562 | 313020 | Keratosis follicularis spinulosa decalvans, 308800 (3) |
| 1557 | SAT | 6303 | LOC125476 | 125476 | 313020 | Keratosis follicularis spinulosa decalvans, 308800 (3) |
| 1557 | SAT | 6303 | LUC7L2 | 51631 | 313020 | Keratosis follicularis spinulosa decalvans, 308800 (3) |
| 1557 | SAT | 6303 | MDS025 | 60492 | 313020 | Keratosis follicularis spinulosa decalvans, 308800 (3) |
| 1557 | SAT | 6303 | MGC8721 | 51669 | 313020 | Keratosis follicularis spinulosa decalvans, 308800 (3) |
| 1557 | SAT | 6303 | MYST2 | 11143 | 313020 | Keratosis follicularis spinulosa decalvans, 308800 (3) |
| 1557 | SAT | 6303 | PTN | 5764 | 313020 | Keratosis follicularis spinulosa decalvans, 308800 (3) |
| 1557 | SAT | 6303 | RIF1 | 55791 | 313020 | Keratosis follicularis spinulosa decalvans, 308800 (3) |
| 1557 | SAT | 6303 | RPLP1 | 6176 | 313020 | Keratosis follicularis spinulosa decalvans, 308800 (3) |
| 1557 | SAT | 6303 | SDCCAG16 | 10813 | 313020 | Keratosis follicularis spinulosa decalvans, 308800 (3) |
| 1557 | SAT | 6303 | SEPHS1 | 22929 | 313020 | Keratosis follicularis spinulosa decalvans, 308800 (3) |
| 1557 | SAT | 6303 | SETDB1 | 9869 | 313020 | Keratosis follicularis spinulosa decalvans, 308800 (3) |
| 1557 | SAT | 6303 | TLE1 | 7088 | 313020 | Keratosis follicularis spinulosa decalvans, 308800 (3) |
| 1557 | SAT | 6303 | TP53 | 7157 | 313020 | Keratosis follicularis spinulosa decalvans, 308800 (3) |
| 1557 | SAT | 6303 | TP53AP1 | 11257 | 313020 | Keratosis follicularis spinulosa decalvans, 308800 (3) |
| 1557 | SAT | 6303 | TUBB | 7280 | 313020 | Keratosis follicularis spinulosa decalvans, 308800 (3) |
| 1557 | SAT | 6303 | UNC119 | 9094 | 313020 | Keratosis follicularis spinulosa decalvans, 308800 (3) |
| 1557 | SAT | 6303 | ZHX1 | 11244 | 313020 | Keratosis follicularis spinulosa decalvans, 308800 (3) |
| 1558 | SCA2 | 6311 | CHGB | 1114 | 601517 | Spinocerebellar ataxia-2, 183090 (3) |
| 1565 | SCN5A | 6331 | RGS2 | 5997 | 600163 | Brugada syndrome, 601144 (3) |
| 1565 | SCN5A | 6331 | RGS2 | 5997 | 600163 | Heart block, nonprogressive, 113900 (3) |
| 1565 | SCN5A | 6331 | RGS2 | 5997 | 600163 | Heart block, progressive, type I, 113900 (3) |
| 1565 | SCN5A | 6331 | RGS2 | 5997 | 600163 | Long QT syndrome-3, 603830 (3) |
| 1565 | SCN5A | 6331 | RGS2 | 5997 | 600163 | Sick sinus syndrome, 608567 (3) |
| 1565 | SCN5A | 6331 | RGS2 | 5997 | 600163 | Ventricular fibrillation, idiopathic, 603829 (3) |
| 1577 | SERPINA5 | 5104 | COPS6 | 10980 | 601841 | Protein C inhibitor deficiency (2) |
| 1588 | SGCE | 8910 | ARIH2 | 10425 | 604149 | Dystonia, myoclonic, 159900 (3) |
| 1592 | SH3BP2 | 6452 | TF | 7018 | 602104 | Cherubism, 118400 (3) |
| 1593 | SH3GL1 | 6455 | AXIN1 | 8312 | 601768 | Leukemia, acute myeloid (1) |
| 1593 | SH3GL1 | 6455 | DPPA4 | 55211 | 601768 | Leukemia, acute myeloid (1) |
| 1593 | SH3GL1 | 6455 | SH3GL3 | 6457 | 601768 | Leukemia, acute myeloid (1) |
| 1619 | SLC25A20 | 788 | C16orf34 | 90861 | 212138 | Carnitine-acylcamitine translocase deficiency (3) |
| 1623 | SLC2A1 | 6513 | COPS6 | 10980 | 138140 | Glucose transport defect, blood-brain barrier, 606777 (3) |
| 1630 | SLC40A1 | 30061 | GOLPH2 | 51280 | 604653 | Hemochromatosis, type 4, 606069 (3) |
| 1633 | SMARCB1 | 6598 | C1orf6 | 56893 | 601607 | Rhabdoid predisposition syndrome, familial (3) |
| 1633 | SMARCB1 | 6598 | C1orf6 | 56893 | 601607 | Rhabdoid tumors (3) |
| 1635 | SMN1 | 6606 | BAT3 | 7917 | 600354 | Spinal muscular atrophy-1, 253300 (3) |
| 1635 | SMN1 | 6606 | BAT3 | 7917 | 600354 | Spinal muscular atrophy-2, 253550 (3) |
| 1635 | SMN1 | 6606 | BAT3 | 7917 | 600354 | Spinal muscular atrophy-3, 253400 (3) |
| 1635 | SMN1 | 6606 | BAT3 | 7917 | 600354 | Spinal muscular atrophy-4, 271150 (3) |
| 1635 | SMN1 | 6606 | COPS6 | 10980 | 600354 | Spinal muscular atrophy-1, 253300 (3) |
| 1635 | SMN1 | 6606 | COPS6 | 10980 | 600354 | Spinal muscular atrophy-2, 253550 (3) |
| 1635 | SMN1 | 6606 | COPS6 | 10980 | 600354 | Spinal muscular atrophy-3, 253400 (3) |
| 1635 | SMN1 | 6606 | COPS6 | 10980 | 600354 | Spinal muscular atrophy-4, 271150 (3) |
| 1635 | SMN1 | 6606 | GDF9 | 2661 | 600354 | Spinal muscular atrophy-1, 253300 (3) |
| 1635 | SMN1 | 6606 | GDF9 | 2661 | 600354 | Spinal muscular atrophy-2, 253550 (3) |
| 1635 | SMN1 | 6606 | GDF9 | 2661 | 600354 | Spinal muscular atrophy-3, 253400 (3) |
| 1635 | SMN1 | 6606 | GDF9 | 2661 | 600354 | Spinal muscular atrophy-4, 271150 (3) |
| 1635 | SMN1 | 6606 | RIF1 | 55791 | 600354 | Spinal muscular atrophy-1, 253300 (3) |
| 1635 | SMN1 | 6606 | RIF1 | 55791 | 600354 | Spinal muscular atrophy-2, 253550 (3) |
| 1635 | SMN1 | 6606 | RIF1 | 55791 | 600354 | Spinal muscular atrophy-3, 253400 (3) |
| 1635 | SMN1 | 6606 | RIF1 | 55791 | 600354 | Spinal muscular atrophy-4, 271150 (3) |
| 1635 | SMN1 | 6606 | UNC119 | 9094 | 600354 | Spinal muscular atrophy-1, 253300 (3) |
| 1635 | SMN1 | 6606 | UNC119 | 9094 | 600354 | Spinal muscular atrophy-2, 253550 (3) |
| 1635 | SMN1 | 6606 | UNC119 | 9094 | 600354 | Spinal muscular atrophy-3, 253400 (3) |
| 1635 | SMN1 | 6606 | UNC119 | 9094 | 600354 | Spinal muscular atrophy-4, 271150 (3) |
| 1637 | SMS | 6611 | EZH2 | 2146 | 300105 | Mental retardation, X-linked, Snyder-Robinson type, 309583 (3) |
| 1648 | SNRPN | 6638 | ARF4L | 379 | 182279 | Prader-Willi syndrome, 176270 (3) |
| 1648 | SNRPN | 6638 | RIF1 | 55791 | 182279 | Prader-Willi syndrome, 176270 (3) |
| 1648 | SNRPN | 6638 | SH3GL3 | 6457 | 182279 | Prader-Willi syndrome, 176270 (3) |
| 1648 | SNRPN | 6638 | TP53 | 7157 | 182279 | Prader-Willi syndrome, 176270 (3) |
| 1649 | SNRPN | 6638 | ARF4L | 379 | 182279 | Prader-Willi syndrome, 176270 (3) |
| 1649 | SNRPN | 6638 | RIF1 | 55791 | 182279 | Prader-Willi syndrome, 176270 (3) |
| 1649 | SNRPN | 6638 | SH3GL3 | 6457 | 182279 | Prader-Willi syndrome, 176270 (3) |
| 1649 | SNRPN | 6638 | TP53 | 7157 | 182279 | Prader-Willi syndrome, 176270 (3) |
| 1662 | SPG7 | 6687 | RALY | 22913 | 602783 | Spastic paraplegia-7 (3) |
| 1662 | SPG7 | 6687 | RIF1 | 55791 | 602783 | Spastic paraplegia-7 (3) |
| 1662 | SPG7 | 6687 | TRIM28 | 10155 | 602783 | Spastic paraplegia-7 (3) |
| 1670 | SQSTM1 | 8878 | C14orf139 | 79686 | 601530 | Paget disease of bone, 602080 (3) |
| 1670 | SQSTM1 | 8878 | MAP1LC3B | 81631 | 601530 | Paget disease of bone, 602080 (3) |
| 1670 | SQSTM1 | 8878 | SQSTM1 | 8878 | 601530 | Paget disease of bone, 602080 (3) |
| 1670 | SQSTM1 | 8878 | TOE1 | 114034 | 601530 | Paget disease of bone, 602080 (3) |
| 1671 | SQSTM1 | 8878 | C14orf139 | 79686 | 601530 | Paget disease of bone, 602080 (3) |
| 1671 | SQSTM1 | 8878 | MAP1LC3B | 81631 | 601530 | Paget disease of bone, 602080 (3) |
| 1671 | SQSTM1 | 8878 | SQSTM1 | 8878 | 601530 | Paget disease of bone, 602080 (3) |
| 1671 | SQSTM1 | 8878 | TOE1 | 114034 | 601530 | Paget disease of bone, 602080 (3) |
| 1673 | SRC | 6714 | KHDRBS1 | 10657 | 190090 | Colon cancer, advanced (3) |
| 1685 | STAR | 6770 | FEZ1 | 9638 | 600617 | Lipoid adrenal hyperplasia, 201710 (3) |
| 1689 | STK11 | 6794 | PREI3 | 25843 | 602216 | Melanoma, malignant sporadic (3) |
| 1689 | STK11 | 6794 | PRE13 | 25843 | 602216 | Pancreatic cancer, sporadic (3) |
| 1689 | STK11 | 6794 | PREI3 | 25843 | 602216 | Peutz-Jeghers syndrome, 175200 (3) |
| 1728 | TAZ | 6901 | CHGB | 1114 | 300394 | Barth syndrome, 302060 (3) |
| 1728 | TAZ | 6901 | CHGB | 1114 | 300394 | Cardiomyopathy, X-linked dilated, 300069 (3) |
| 1728 | TAZ | 6901 | CHGB | 1114 | 300394 | Endocardial fibroelastosis-2 (2) |
| 1728 | TAZ | 6901 | CHGB | 1114 | 300394 | Noncompaction of left ventricular myocardium, isolated, 300183 (3) |
| 1733 | TBP | 6908 | C19orf14 | 284403 | 600075 | Huntington disease-like-4, 607136 (3) |
| 1733 | TBP | 6908 | C19orf14 | 284403 | 600075 | Parkinson disease, 168600 (3) |
| 1733 | TBP | 6908 | C19orf14 | 284403 | 600075 | Spinocerebellar ataxia 17, 607136 (3) |
| 1741 | TCL1B | 9623 | TNP02 | 30000 | 603769 | Leukemia/lymphoma, T-cell (2) |
| 1742 | TDGF1 | 6997 | ANG | 283 | 187395 | Forebrain defects (3) |
| 1742 | TDGF1 | 6997 | BAT3 | 7917 | 187395 | Forebrain defects (3) |
| 1742 | TDGF1 | 6997 | COPS6 | 10980 | 187395 | Forebrain defects (3) |
| 1742 | TDGF1 | 6997 | GDF9 | 2661 | 187395 | Forebrain defects (3) |
| 1742 | TDGF1 | 6997 | RIF1 | 55791 | 187395 | Forebrain defects (3) |
| 1745 | TF | 7018 | SH3BP2 | 6452 | 190000 | {Iron deficiency anemia, susceptibility to} (3) |
| 1745 | TF | 7018 | SH3BP2 | 6452 | 190000 | Atransferrinemia, 209300 (3) |
| 1745 | TF | 7018 | TUBB4 | 10381 | 190000 | {Iron deficiency anemia, susceptibility to} (3) |
| 1745 | TF | 7018 | TUBB4 | 10381 | 190000 | Atransferrinemia, 209300 (3) |
| 1746 | TFAP2B | 7021 | HIST1H2AC | 8334 | 601601 | Char syndrome, 169100 (3) |
| 1746 | TFAP2B | 7021 | VPS11 | 55823 | 601601 | Char syndrome, 169100 (3) |
| 1749 | TGIF | 7050 | CGI-125 | 51003 | 602630 | Holoprosencephaly-4, 142946 (3) |
| 1749 | TGIF | 7050 | EEF1A1 | 1915 | 602630 | Holoprosencephaly-4, 142946 (3) |
| 1749 | TGIF | 7050 | RIF1 | 55791 | 602630 | Holoprosencephaly-4, 142946 (3) |
| 1762 | TM4SF2 | 7102 | DKFZp761B1514 | 84248 | 300096 | Mental retardation, X-linked 58, 300218 (3) |
| 1767 | TMPRSS3 | 64699 | EEF1A1 | 1915 | 605511 | Deafness, autosomal recessive 10, congenital, 605316 (3) |
| 1767 | TMPRSS3 | 64699 | EEF1A1 | 1915 | 605511 | Deafness, autosomal recessive 8, childhood onset, 601072 (3) |
| 1767 | TMPRSS3 | 64699 | RXRA | 6256 | 605511 | Deafness, autosomal recessive 10, congenital, 605316 (3) |
| 1767 | TMPRSS3 | 64699 | RXRA | 6256 | 605511 | Deafness, autosomal recessive 8, childhood onset, 601072 (3) |
| 1772 | TNFRSF6 | 355 | C14orf1 | 11161 | 134637 | {Autoimmune lymphoproliferative syndrome}, 601859 (3) |
| 1772 | TNFRSF6 | 355 | C14orf1 | 11161 | 134637 | Autoimmune lymphoproliferative syndrome, type IA, 601859 (3) |
| 1772 | TNFRSF6 | 355 | C14orf1 | 11161 | 134637 | Squamous cell carcinoma, bum scar-related, somatic (3) |
| 1772 | TNFRSF6 | 355 | CRMP1 | 1400 | 134637 | {Autoimmune lymphoproliferative syndrome}, 601859 (3) |
| 1772 | TNFRSF6 | 355 | CRMP1 | 1400 | 134637 | Autoimmune lymphoproliferative syndrome, type IA, 601859 (3) |
| 1772 | TNFRSF6 | 355 | CRMP1 | 1400 | 134637 | Squamous cell carcinoma, bum scar-related, somatic (3) |
| 1772 | TNFRSF6 | 355 | EEF1A1 | 1915 | 134637 | {Autoimmune lymphoproliferative syndrome}, 601859 (3) |
| 1772 | TNFRSF6 | 355 | EEF1A1 | 1915 | 134637 | Autoimmune lymphoproliferative syndrome, type IA, 601859 (3) |
| 1772 | TNFRSF6 | 355 | EEF1A1 | 1915 | 134637 | Squamous cell carcinoma, bum scar-related, somatic (3) |
| 1772 | TNFRSF6 | 355 | RIF1 | 55791 | 134637 | {Autoimmune lymphoproliferative syndrome}, 601859 (3) |
| 1772 | TNFRSF6 | 355 | RIF1 | 55791 | 134637 | Autoimmune lymphoproliferative syndrome, type IA, 601859 (3) |
| 1772 | TNFRSF6 | 355 | RIF1 | 55791 | 134637 | Squamous cell carcinoma, bum scar-related, somatic (3) |
| 1775 | TNNT1 | 7138 | PNMA1 | 9240 | 191041 | Nemaline myopathy, Amish type, 605355 (3) |
| 1776 | TNNT3 | 7140 | HAP1 | 9001 | 600692 | Arthyrgryposis multiplex congenita, distal, type 2B, 601680 (3) |
| 1776 | TNNT3 | 7140 | RNUT1 | 10073 | 600692 | Arthyrgryposis multiplex congenita, distal, type 2B, 601680 (3) |
| 1794 | TPI1 | 7167 | CCL11 | 6356 | 190450 | Hemolytic anemia due to triosephosphate isomerase deficiency (3) |
| 1794 | TPI1 | 7167 | DDX24 | 57062 | 190450 | Hemolytic anemia due to triosephosphate isomerase deficiency (3) |
| 1794 | TPI1 | 7167 | LIPT1 | 51601 | 190450 | Hemolytic anemia due to triosephosphate isomerase deficiency (3) |
| 1794 | TPI1 | 7167 | SETDB1 | 9869 | 190450 | Hemolytic anemia due to triosephosphate isomerase deficiency (3) |
| 1812 | TTR | 7276 | C7orf20 | 51608 | 176300 | [Dystransthyretinemic hyperthyroxinemia](3) |
| 1812 | TTR | 7276 | C7orf20 | 51608 | 176300 | Amyloid neuropathy, familial, several allelic types (3) |
| 1812 | TTR | 7276 | C7orf20 | 51608 | 176300 | Amyloidosis, senile systemic (3) |
| 1812 | TTR | 7276 | C7orf20 | 51608 | 176300 | Carpal tunnel syndrome, familial (3) |
| 1812 | TTR | 7276 | CHD3 | 1107 | 176300 | [Dystransthyretinemic hyperthyroxinemia](3) |
| 1812 | TTR | 7276 | CHD3 | 1107 | 176300 | Amyloid neuropathy, familial, several allelic types (3) |
| 1812 | TTR | 7276 | CHD3 | 1107 | 176300 | Amyloidosis, senile systemic (3) |
| 1812 | TTR | 7276 | CHD3 | 1107 | 176300 | Carpal tunnel syndrome, familial (3) |
| 1812 | TTR | 7276 | DDR1 | 780 | 176300 | [Dystransthyretinemic hyperthyroxinemia](3) |
| 1812 | TTR | 7276 | DDR1 | 780 | 176300 | Amyloid neuropathy, familial, several allelic types (3) |
| 1812 | TTR | 7276 | DDR1 | 780 | 176300 | Amyloidosis, senile systemic (3) |
| 1812 | TTR | 7276 | DDR1 | 780 | 176300 | Carpal tunnel syndrome, familial (3) |
| 1812 | TTR | 7276 | EEF1A1 | 1915 | 176300 | [Dystransthyretinemic hyperthyroxinemia](3) |
| 1812 | TTR | 7276 | EEF1A1 | 1915 | 176300 | Amyloid neuropathy, familial, several allelic types (3) |
| 1812 | TTR | 7276 | EEF1A1 | 1915 | 176300 | Amyloidosis, senile systemic (3) |
| 1812 | TTR | 7276 | EEF1A1 | 1915 | 176300 | Carpal tunnel syndrome, familial (3) |
| 1812 | TTR | 7276 | FEZ1 | 9638 | 176300 | [Dystransthyretinemic hyperthyroxinemia](3) |
| 1812 | TTR | 7276 | FEZ1 | 9638 | 176300 | Amyloid neuropathy, familial, several allelic types (3) |
| 1812 | TTR | 7276 | FEZ1 | 9638 | 176300 | Amyloidosis, senile systemic (3) |
| 1812 | TTR | 7276 | FEZ1 | 9638 | 176300 | Carpal tunnel syndrome, familial (3) |
| 1812 | TTR | 7276 | HNRPH3 | 3189 | 176300 | [Dystransthyretinemic hyperthyroxinemia](3) |
| 1812 | TTR | 7276 | HNRPH3 | 3189 | 176300 | Amyloid neuropathy, familial, several allelic types (3) |
| 1812 | TTR | 7276 | HNRPH3 | 3189 | 176300 | Amyloidosis, senile systemic (3) |
| 1812 | TTR | 7276 | HNRPH3 | 3189 | 176300 | Carpal tunnel syndrome, familial (3) |
| 1812 | TTR | 7276 | IKBKAP | 8518 | 176300 | [Dystransthyretinemic hyperthyroxinemia](3) |
| 1812 | TTR | 7276 | IKBKAP | 8518 | 176300 | Amyloid neuropathy, familial, several allelic types (3) |
| 1812 | TTR | 7276 | IKBKAP | 8518 | 176300 | Amyloidosis, senile systemic (3) |
| 1812 | TTR | 7276 | IKBKAP | 8518 | 176300 | Carpal tunnel syndrome, familial (3) |
| 1812 | TTR | 7276 | MARK3 | 4140 | 176300 | [Dystransthyretinemic hyperthyroxinemia](3) |
| 1812 | TTR | 7276 | MARK3 | 4140 | 176300 | Amyloid neuropathy, familial, several allelic types (3) |
| 1812 | TTR | 7276 | MARK3 | 4140 | 176300 | Amyloidosis, senile systemic (3) |
| 1812 | TTR | 7276 | MARK3 | 4140 | 176300 | Carpal tunnel syndrome, familial (3) |
| 1812 | TTR | 7276 | PHYHIP | 9796 | 176300 | [Dystransthyretinemic hyperthyroxinemia](3) |
| 1812 | TTR | 7276 | PHYHIP | 9796 | 176300 | Amyloid neuropathy, familial, several allelic types (3) |
| 1812 | TTR | 7276 | PHYHIP | 9796 | 176300 | Amyloidosis, senile systemic (3) |
| 1812 | TTR | 7276 | PHYHIP | 9796 | 176300 | Carpal tunnel syndrome, familial (3) |
| 1812 | TTR | 7276 | SETDB1 | 9869 | 176300 | [Dystransthyretinemic hyperthyroxinemia](3) |
| 1812 | TTR | 7276 | SETDB1 | 9869 | 176300 | Amyloid neuropathy, familial, several allelic types (3) |
| 1812 | TTR | 7276 | SETDB1 | 9869 | 176300 | Amyloidosis, senile systemic (3) |
| 1812 | TTR | 7276 | SETDB1 | 9869 | 176300 | Carpal tunnel syndrome, familial (3) |
| 1812 | TTR | 7276 | SIAT6 | 6487 | 176300 | [Dystransthyretinemic hyperthyroxinemia](3) |
| 1812 | TTR | 7276 | SIAT6 | 6487 | 176300 | Amyloid neuropathy, familial, several allelic types (3) |
| 1812 | TTR | 7276 | SIAT6 | 6487 | 176300 | Amyloidosis, senile systemic (3) |
| 1812 | TTR | 7276 | SIAT6 | 6487 | 176300 | Carpal tunnel syndrome, familial (3) |
| 1812 | TTR | 7276 | VIM | 7431 | 176300 | [Dystransthyretinemic hyperthyroxinemia](3) |
| 1812 | TTR | 7276 | VIM | 7431 | 176300 | Amyloid neuropathy, familial, several allelic types (3) |
| 1812 | TTR | 7276 | VIM | 7431 | 176300 | Amyloidosis, senile systemic (3) |
| 1812 | TTR | 7276 | VIM | 7431 | 176300 | Carpal tunnel syndrome, familial (3) |
| 1813 | TTR | 7276 | C7 orf 20 | 51608 | 176300 | [Dystransthyretinemic hyperthyroxinemia](3) |
| 1813 | TTR | 7276 | C7 orf 20 | 51608 | 176300 | Amyloid neuropathy, familial, several allelic types (3) |
| 1813 | TTR | 7276 | C7 orf 20 | 51608 | 176300 | Amyloidosis, senile systemic (3) |
| 1813 | TTR | 7276 | C7 orf 20 | 51608 | 176300 | Carpal tunnel syndrome, familial (3) |
| 1813 | TTR | 7276 | CHD3 | 1107 | 176300 | [Dystransthyretinemic hyperthyroxinemia](3) |
| 1813 | TTR | 7276 | CHD3 | 1107 | 176300 | Amyloid neuropathy, familial, several allelic types (3) |
| 1813 | TTR | 7276 | CHD3 | 1107 | 176300 | Amyloidosis, senile systemic (3) |
| 1813 | TTR | 7276 | CHD3 | 1107 | 176300 | Carpal tunnel syndrome, familial (3) |
| 1813 | TTR | 7276 | DDR1 | 780 | 176300 | [Dystransthyretinemic hyperthyroxinemia](3) |
| 1813 | TTR | 7276 | DDR1 | 780 | 176300 | Amyloid neuropathy, familial, several allelic types (3) |
| 1813 | TTR | 7276 | DDR1 | 780 | 176300 | Amyloidosis, senile systemic (3) |
| 1813 | TTR | 7276 | DDR1 | 780 | 176300 | Carpal tunnel syndrome, familial (3) |
| 1813 | TTR | 7276 | EEF1A1 | 1915 | 176300 | [Dystransthyretinemic hyperthyroxinemia](3) |
| 1813 | TTR | 7276 | EEF1A1 | 1915 | 176300 | Amyloid neuropathy, familial, several allelic types (3) |
| 1813 | TTR | 7276 | EEF1A1 | 1915 | 176300 | Amyloidosis, senile systemic (3) |
| 1813 | TTR | 7276 | EEF1A1 | 1915 | 176300 | Carpal tunnel syndrome, familial (3) |
| 1813 | TTR | 7276 | FEZ1 | 9638 | 176300 | [Dystransthyretinemic hyperthyroxinemia](3) |
| 1813 | TTR | 7276 | FEZ1 | 9638 | 176300 | Amyloid neuropathy, familial, several allelic types (3) |
| 1813 | TTR | 7276 | FEZ1 | 9638 | 176300 | Amyloidosis, senile systemic (3) |
| 1813 | TTR | 7276 | FEZ1 | 9638 | 176300 | Carpal tunnel syndrome, familial (3) |
| 1813 | TTR | 7276 | HNRPH3 | 3189 | 176300 | [Dystransthyretinemic hyperthyroxinemia](3) |
| 1813 | TTR | 7276 | HNRPH3 | 3189 | 176300 | Amyloid neuropathy, familial, several allelic types (3) |
| 1813 | TTR | 7276 | HNRPH3 | 3189 | 176300 | Amyloidosis, senile systemic (3) |
| 1813 | TTR | 7276 | HNRPH3 | 3189 | 176300 | Carpal tunnel syndrome, familial (3) |
| 1813 | TTR | 7276 | IKBKAP | 8518 | 176300 | [Dystransthyretinemic hyperthyroxinemia](3) |
| 1813 | TTR | 7276 | IKBKAP | 8518 | 176300 | Amyloid neuropathy, familial, several allelic types (3) |
| 1813 | TTR | 7276 | IKBKAP | 8518 | 176300 | Amyloidosis, senile systemic (3) |
| 1813 | TTR | 7276 | IKBKAP | 8518 | 176300 | Carpal tunnel syndrome, familial (3) |
| 1813 | TTR | 7276 | MARK3 | 4140 | 176300 | [Dystransthyretinemic hyperthyroxinemia](3) |
| 1813 | TTR | 7276 | MARK3 | 4140 | 176300 | Amyloid neuropathy, familial, several allelic types (3) |
| 1813 | TTR | 7276 | MARK3 | 4140 | 176300 | Amyloidosis, senile systemic (3) |
| 1813 | TTR | 7276 | MARK3 | 4140 | 176300 | Carpal tunnel syndrome, familial (3) |
| 1813 | TTR | 7276 | PHYHIP | 9796 | 176300 | [Dystransthyretinemic hyperthyroxinemia](3) |
| 1813 | TTR | 7276 | PHYHIP | 9796 | 176300 | Amyloid neuropathy, familial, several allelic types (3) |
| 1813 | TTR | 7276 | PHYHIP | 9796 | 176300 | Amyloidosis, senile systemic (3) |
| 1813 | TTR | 7276 | PHYHIP | 9796 | 176300 | Carpal tunnel syndrome, familial (3) |
| 1813 | TTR | 7276 | SETDB1 | 9869 | 176300 | [Dystransthyretinemic hyperthyroxinemia](3) |
| 1813 | TTR | 7276 | SETDB1 | 9869 | 176300 | Amyloid neuropathy, familial, several allelic types (3) |
| 1813 | TTR | 7276 | SETDB1 | 9869 | 176300 | Amyloidosis, senile systemic (3) |
| 1813 | TTR | 7276 | SETDB1 | 9869 | 176300 | Carpal tunnel syndrome, familial (3) |
| 1813 | TTR | 7276 | SIAT6 | 6487 | 176300 | [Dystransthyretinemic hyperthyroxinemia](3) |
| 1813 | TTR | 7276 | SIAT6 | 6487 | 176300 | Amyloid neuropathy, familial, several allelic types (3) |
| 1813 | TTR | 7276 | SIAT6 | 6487 | 176300 | Amyloidosis, senile systemic (3) |
| 1813 | TTR | 7276 | SIAT6 | 6487 | 176300 | Carpal tunnel syndrome, familial (3) |
| 1813 | TTR | 7276 | VIM | 7431 | 176300 | [Dystransthyretinemic hyperthyroxinemia](3) |
| 1813 | TTR | 7276 | VIM | 7431 | 176300 | Amyloid neuropathy, familial, several allelic types (3) |
| 1813 | TTR | 7276 | VIM | 7431 | 176300 | Amyloidosis, senile systemic (3) |
| 1813 | TTR | 7276 | VIM | 7431 | 176300 | Carpal tunnel syndrome, familial (3) |
| 1835 | UBE2B | 7320 | CGI-125 | 51003 | 179095 | Male infertility (1) (?) |
| 1835 | UBE2B | 7320 | CRMP1 | 1400 | 179095 | Male infertility (1) (?) |
| 1835 | UBE2B | 7320 | UBR1 | 197131 | 179095 | Male infertility (1) (?) |
| 1835 | UBE2B | 7320 | UNC119 | 9094 | 179095 | Male infertility (1) (?) |
| 1848 | UCHL1 | 7345 | CBX1 | 10951 | 191342 | Parkinson disease, familial, 168600 (3) |
| 1848 | UCHL1 | 7345 | NEDD8 | 4738 | 191342 | Parkinson disease, familial, 168600 (3) |
| 1848 | UCHL1 | 7345 | UBC | 7316 | 191342 | Parkinson disease, familial, 168600 (3) |
| 1852 | UMPS | 7372 | CDC42EP3 | 10602 | 258900 | Oroticaciduria (3) |
| 1852 | UMPS | 7372 | DLST | 1743 | 258900 | Oroticaciduria (3) |
| 1887 | WFS1 | 7466 | ITGB4BP | 3692 | 606201 | Hearing loss, low-frequency sensorineural, 600965 (3) |
| 1887 | WFS1 | 7466 | ITGB4BP | 3692 | 606201 | Wolfram syndrome, 222300 (3) |
| 1900 | ZFHX1B | 9839 | COPS6 | 10980 | 605802 | Mowat-Wilson syndrome, 235730 (3) |
| 1907 | ZNF145 | 7704 | ACP5 | 54 | 176797 | Leukemia, acute promyelocytic, PL2F/RARA type (3) |
| 1907 | ZNF145 | 7704 | ANAPC5 | 51433 | 176797 | Leukemia, acute promyelocytic, PL2F/RARA type (3) |
| 1907 | ZNF145 | 7704 | CD81 | 975 | 176797 | Leukemia, acute promyelocytic, PL2F/RARA type (3) |
| 1907 | ZNF145 | 7704 | CIDEA | 1149 | 176797 | Leukemia, acute promyelocytic, PL2F/RARA type (3) |
| 1907 | ZNF145 | 7704 | COQ6 | 51004 | 176797 | Leukemia, acute promyelocytic, PL2F/RARA type (3) |
| 1907 | ZNF145 | 7704 | DLST | 1743 | 176797 | Leukemia, acute promyelocytic, PL2F/RARA type (3) |
| 1907 | ZNF145 | 7704 | DPM1 | 8813 | 176797 | Leukemia, acute promyelocytic, PL2F/RARA type (3) |
| 1907 | ZNF145 | 7704 | EIF2S2 | 8894 | 176797 | Leukemia, acute promyelocytic, PL2F/RARA type (3) |
| 1907 | ZNF145 | 7704 | FCHO1 | 23149 | 176797 | Leukemia, acute promyelocytic, PL2F/RARA type (3) |
| 1907 | ZNF145 | 7704 | FLJ10916 | 55258 | 176797 | Leukemia, acute promyelocytic, PL2F/RARA type (3) |
| 1907 | ZNF145 | 7704 | FLJ20397 | 54919 | 176797 | Leukemia, acute promyelocytic, PL2F/RARA type (3) |
| 1907 | ZNF145 | 7704 | FLJ20647 | 55013 | 176797 | Leukemia, acute promyelocytic, PL2F/RARA type (3) |
| 1907 | ZNF145 | 7704 | GCSH | 2653 | 176797 | Leukemia, acute promyelocytic, PL2F/RARA type (3) |
| 1907 | ZNF145 | 7704 | GSTM4 | 2948 | 176797 | Leukemia, acute promyelocytic, PL2F/RARA type (3) |
| 1907 | ZNF145 | 7704 | HBXIP | 10542 | 176797 | Leukemia, acute promyelocytic, PL2F/RARA type (3) |
| 1907 | ZNF145 | 7704 | ICAM3 | 3385 | 176797 | Leukemia, acute promyelocytic, PL2F/RARA type (3) |
| 1907 | ZNF145 | 7704 | IL6 | 3569 | 176797 | Leukemia, acute promyelocytic, PL2F/RARA type (3) |
| 1907 | ZNF145 | 7704 | KIAA1549 | 57670 | 176797 | Leukemia, acute promyelocytic, PL2F/RARA type (3) |
| 1907 | ZNF145 | 7704 | LMTK3 | 114783 | 176797 | Leukemia, acute promyelocytic, PL2F/RARA type (3) |
| 1907 | ZNF145 | 7704 | LSM2 | 57819 | 176797 | Leukemia, acute promyelocytic, PL2F/RARA type (3) |
| 1907 | ZNF145 | 7704 | LYAR | 55646 | 176797 | Leukemia, acute promyelocytic, PL2F/RARA type (3) |
| 1907 | ZNF145 | 7704 | MAP3K3 | 4215 | 176797 | Leukemia, acute promyelocytic, PL2F/RARA type (3) |
| 1907 | ZNF145 | 7704 | MGC10471 | 81576 | 176797 | Leukemia, acute promyelocytic, PL2F/RARA type (3) |
| 1907 | ZNF145 | 7704 | MGC45871 | 359845 | 176797 | Leukemia, acute promyelocytic, PL2F/RARA type (3) |
| 1907 | ZNF145 | 7704 | MGC5352 | 192111 | 176797 | Leukemia, acute promyelocytic, PL2F/RARA type (3) |
| 1907 | ZNF145 | 7704 | PAFAH1B3 | 5050 | 176797 | Leukemia, acute promyelocytic, PL2F/RARA type (3) |
| 1907 | ZNF145 | 7704 | PMAIP1 | 5366 | 176797 | Leukemia, acute promyelocytic, PL2F/RARA type (3) |
| 1907 | ZNF145 | 7704 | PNRC2 | 55629 | 176797 | Leukemia, acute promyelocytic, PL2F/RARA type (3) |
| 1907 | ZNF145 | 7704 | PSMD11 | 5717 | 176797 | Leukemia, acute promyelocytic, PL2F/RARA type (3) |
| 1907 | ZNF145 | 7704 | PSMD2 | 5708 | 176797 | Leukemia, acute promyelocytic, PL2F/RARA type (3) |
| 1907 | ZNF145 | 7704 | QTRT1 | 81890 | 176797 | Leukemia, acute promyelocytic, PL2F/RARA type (3) |
| 1907 | ZNF145 | 7704 | RAB27A | 5873 | 176797 | Leukemia, acute promyelocytic, PL2F/RARA type (3) |
| 1907 | ZNF145 | 7704 | TERF1 | 7013 | 176797 | Leukemia, acute promyelocytic, PL2F/RARA type (3) |
| 1907 | ZNF145 | 7704 | TOLLIP | 54472 | 176797 | Leukemia, acute promyelocytic, PL2F/RARA type (3) |
| 1907 | ZNF145 | 7704 | WDR33 | 55339 | 176797 | Leukemia, acute promyelocytic, PL2F/RARA type (3) |
| 1907 | ZNF145 | 7704 | ZNF24 | 7572 | 176797 | Leukemia, acute promyelocytic, PL2F/RARA type (3) |
| 1908 | ZNF145 | 7704 | ACP5 | 54 | 176797 | Leukemia, acute promyelocytic, PL2F/RARA type (3) |
| 1908 | ZNF145 | 7704 | ANAPC5 | 51433 | 176797 | Leukemia, acute promyelocytic, PL2F/RARA type (3) |
| 1908 | ZNF145 | 7704 | CD81 | 975 | 176797 | Leukemia, acute promyelocytic, PL2F/RARA type (3) |
| 1908 | ZNF145 | 7704 | CIDEA | 1149 | 176797 | Leukemia, acute promyelocytic, PL2F/RARA type (3) |
| 1908 | ZNF145 | 7704 | COQ6 | 51004 | 176797 | Leukemia, acute promyelocytic, PL2F/RARA type (3) |
| 1908 | ZNF145 | 7704 | DLST | 1743 | 176797 | Leukemia, acute promyelocytic, PL2F/RARA type (3) |
| 1908 | ZNF145 | 7704 | DPM1 | 8813 | 176797 | Leukemia, acute promyelocytic, PL2F/RARA type (3) |
| 1908 | ZNF145 | 7704 | EIF2S2 | 8894 | 176797 | Leukemia, acute promyelocytic, PL2F/RARA type (3) |
| 1908 | ZNF145 | 7704 | FCHO1 | 23149 | 176797 | Leukemia, acute promyelocytic, PL2F/RARA type (3) |
| 1908 | ZNF145 | 7704 | FLJ10916 | 55258 | 176797 | Leukemia, acute promyelocytic, PL2F/RARA type (3) |
| 1908 | ZNF145 | 7704 | FLJ20397 | 54919 | 176797 | Leukemia, acute promyelocytic, PL2F/RARA type (3) |
| 1908 | ZNF145 | 7704 | FLJ20647 | 55013 | 176797 | Leukemia, acute promyelocytic, PL2F/RARA type (3) |
| 1908 | ZNF145 | 7704 | GCSH | 2653 | 176797 | Leukemia, acute promyelocytic, PL2F/RARA type (3) |
| 1908 | ZNF145 | 7704 | GSTM4 | 2948 | 176797 | Leukemia, acute promyelocytic, PL2F/RARA type (3) |
| 1908 | ZNF145 | 7704 | HBXIP | 10542 | 176797 | Leukemia, acute promyelocytic, PL2F/RARA type (3) |
| 1908 | ZNF145 | 7704 | ICAM3 | 3385 | 176797 | Leukemia, acute promyelocytic, PL2F/RARA type (3) |
| 1908 | ZNF145 | 7704 | IL6 | 3569 | 176797 | Leukemia, acute promyelocytic, PL2F/RARA type (3) |
| 1908 | ZNF145 | 7704 | KIAA1549 | 57670 | 176797 | Leukemia, acute promyelocytic, PL2F/RARA type (3) |
| 1908 | ZNF145 | 7704 | LMTK3 | 114783 | 176797 | Leukemia, acute promyelocytic, PL2F/RARA type (3) |
| 1908 | ZNF145 | 7704 | LSM2 | 57819 | 176797 | Leukemia, acute promyelocytic, PL2F/RARA type (3) |
| 1908 | ZNF145 | 7704 | LYAR | 55646 | 176797 | Leukemia, acute promyelocytic, PL2F/RARA type (3) |
| 1908 | ZNF145 | 7704 | MAP3K3 | 4215 | 176797 | Leukemia, acute promyelocytic, PL2F/RARA type (3) |
| 1908 | ZNF145 | 7704 | MGC10471 | 81576 | 176797 | Leukemia, acute promyelocytic, PL2F/RARA type (3) |
| 1908 | ZNF145 | 7704 | MGC45871 | 359845 | 176797 | Leukemia, acute promyelocytic, PL2F/RARA type (3) |
| 1908 | ZNF145 | 7704 | MGC5352 | 192111 | 176797 | Leukemia, acute promyelocytic, PL2F/RARA type (3) |
| 1908 | ZNF145 | 7704 | PAFAH1B3 | 5050 | 176797 | Leukemia, acute promyelocytic, PL2F/RARA type (3) |
| 1908 | ZNF145 | 7704 | PMAIP1 | 5366 | 176797 | Leukemia, acute promyelocytic, PL2F/RARA type (3) |
| 1908 | ZNF145 | 7704 | PNRC2 | 55629 | 176797 | Leukemia, acute promyelocytic, PL2F/RARA type (3) |
| 1908 | ZNF145 | 7704 | PSMD11 | 5717 | 176797 | Leukemia, acute promyelocytic, PL2F/RARA type (3) |
| 1908 | ZNF145 | 7704 | PSMD2 | 5708 | 176797 | Leukemia, acute promyelocytic, PL2F/RARA type (3) |
| 1908 | ZNF145 | 7704 | QTRT1 | 81890 | 176797 | Leukemia, acute promyelocytic, PL2F/RARA type (3) |
| 1908 | ZNF145 | 7704 | RAB27A | 5873 | 176797 | Leukemia, acute promyelocytic, PL2F/RARA type (3) |
| 1908 | ZNF145 | 7704 | TERF1 | 7013 | 176797 | Leukemia, acute promyelocytic, PL2F/RARA type (3) |
| 1908 | ZNF145 | 7704 | TOLLIP | 54472 | 176797 | Leukemia, acute promyelocytic, PL2F/RARA type (3) |
| 1908 | ZNF145 | 7704 | WDR33 | 55339 | 176797 | Leukemia, acute promyelocytic, PL2F/RARA type (3) |
| 1908 | ZNF145 | 7704 | ZNF24 | 7572 | 176797 | Leukemia, acute promyelocytic, PL2F/RARA type (3) |

### Supporting references

1. K. Bussow *et al., Nucleic Acids* Res. **26,** 5007 (1998).
2. H. Goehler *et al., Mol. Cell,* in press (2004).
3. M. Remm, C. E. Storm, E. L. Sonnhammer, J. *Mol. Biol.* **314,** 1041 (2001).
4. S. Peri *et al., Genome* Res. **13,** 2363 (2003).
5. A. Zanzoni *et al., FEBS Lett.* **513,** 135 (2002).
6. G. D. Bader, D. Betel, C. W. Hogue, *Nucleic Acids* Res **31,** 248 (2003).
7. L. Giot *et al., Science* **302,** 1727 (2003).
8. S. Li *et al*., *Science* **303**, 540 (2004).
9. H. W. Mewes *et al*., *Nucleic Acids Res*. **32**, D41 (2004).
10. H. Yu, X. Zhu, D. Greenbaum, J. Karro, M. Gerstein, *Nucleic Acids Res* **32**, 328 (2004).
11. M. Ashburner *et al*., *Nat*. *Genet*. **25**, 25 (2000).

## Claims

1. A method for identification of a protein-protein interaction of protein interaction partners in a disease-related network of proteins comprising the steps of:
(a) identifying nucleic acid molecules by at least partial 5' sequencing and, optionally, additionally adding recombinantly cloned and sequenced nucleic acid molecules,
wherein said nucleic acid molecules encode a selection of proteins suspected to contain one or several of said interaction partners and
wherein said nucleic acid molecules are annotated with a positional information;
(b) in frame cloning of the nucleic acid molecules of step (a) into prey and bait vectors, wherein one copy of each nucleic acid molecule is cloned into said prey vector and a second copy of each nucleic acid molecule is cloned into said bait vector;
(c) transforming a first suitable host cell with the prey vector obtained in step (b) and a second suitable host cell with the bait vector obtained in step (b), wherein said first and said second host cell have a different genetic constitution and can be mated;
(d) mating the first suitable host cell of step (c) with the second suitable host cell of step (c), and expressing the proteins encoded by the nucleic acid molecules obtained in step (b);
(e) selecting the mated host cell obtained in step (d) on the basis of a selection advantage which is caused by the protein-protein interaction between the protein interaction partner encoded by the nucleic acid molecule of the prey vector contained in said cell and the protein interaction partner encoded by the nucleic acid molecule of the bait vector contained in said cell; and
(f) identifying with the positional information obtained in step (a) the protein interaction partners of step (e) and thereby identifying the protein-protein interaction.

2. A method for identification of a protein-protein interaction of protein interaction partners in a disease-related network of proteins comprising the steps of:
(a) identifying nucleic acid molecules by partial 5' sequencing and, optionally, additionally adding recombinantly cloned and sequenced nucleic acid molecules,
wherein said nucleic acid molecules encode a selection of proteins suspected to contain one or several of said interaction partners and
wherein said nucleic acid molecules are annotated with a positional information;
(b) in frame cloning of the nucleic acid molecules of step (a) into prey and bait vectors, wherein one copy of each nucleic acid molecule is cloned into said prey vector and a second copy of each nucleic acid molecule is cloned into said bait vector;
(c) transforming a first suitable host cell with the prey vector obtained in step (b) and a second suitable host cell with the bait vector obtained in step (b), wherein said first and said second host cell have a different genetic constitution and can be mated;
(d) pooling at least two of the second suitable host cells obtained in step (c);
(e) mating the first suitable host cell of step (c) with the at least two second suitable host cells of step (d), and expressing the proteins encoded by the nucleic acid molecules obtained in step (b);
(f) selecting the mated host cell obtained in step (e) on the basis of a selection advantage which is caused by the protein-protein interaction between the protein interaction partner encoded by the nucleic acid molecule of the prey vector contained in said cell and the protein interaction partner encoded by the nucleic acid molecule of the bait vector contained in said cell; and
(g) identifying with the positional information obtained in step (a) the protein interaction partners of step (f) and thereby identifying the protein-protein interaction.

3. The method of claim 1 or 2, wherein at least one of said protein interaction partners in the disease related network of proteins, when mutated or unphysiologically expressed, is involved in the etiology of a disease.

4. The method of any one of claims 1 to 3, wherein said annotation of positional information is achieved by arraying the nucleic acid molecules.

5. The method of any one of claims 1 to 4, wherein said nucleic acid molecules identified by said at least partial 5' sequencing are completely sequenced and further **characterized by** bioinformatic means.

6. The method of any one of claims 1 to 5, wherein said first suitable host cell is a MAT alpha yeast cell and said second suitable host cell is a MAT a yeast cell.

7. The method of any one of claims 1 to 6, wherein said proteins expressed in the mated host cell are chimaeric proteins.

8. The method of any one of claims 1 to 7, wherein said selection of the mated host cell comprises the step of carrying out a beta-Galactosidase assay.

9. The method of any one of claims 1 to 8, wherein the identified protein-protein interactions are biologically evaluated.

10. The nucleic acid molecule encoding a protein interaction partner identified by the method of any one of claims 1 to 9, wherein said protein interaction partner is a protein selected from any one of tables S1, S2 or S6.

11. The nucleic acid molecule of claim 10, wherein said nucleic acid molecule is DNA, preferably cDNA, genomic DNA, or synthetic DNA or RNA, preferably mRNA.

12. The nucleic acid molecule of claim 10 or 11 fused to a heterologous nucleic acid molecule.

13. A vector comprising the nucleic acid molecule of any one of claims 10 to 12.

14. A host cell comprising the nucleic acid molecule of any one of claims 10 to 12 or the vector of claim 13.

15. A method of producing a protein, comprising culturing the host cell of claim 14 under conditions such that the protein encoded by said nucleic acid is expressed and recovering said protein.

16. A protein comprising an amino acid sequence encoded by a nucleic acid molecule of any one of claims 10 to 12, or which is chemically synthesized, or is obtainable from the host cell of claim 14, or which is obtainable by the method of claim 15 or which is obtainable from an in vitro translation system by expressing the nucleic acid molecule of any one of claims 10 to 12 or the vector of claim 13.

17. The protein of claim 16 fused to a heterologous protein.

18. A protein complex comprising at least two protein interaction partners selected from the group of interaction proteins listed in table S2 and/or S6.

19. An antibody specifically recognizing the protein of claim 16 or 17 or specifically reacting with the protein complex of claim 18.

20. The antibody of claim 19 which is polyclonal, monoclonal, chimeric, single chain, single chain Fv, human antibody, humanized antibody, or Fab fragment.

21. A method for identifying compounds affecting protein interaction partners comprising
(a) contacting protein interaction partners detected from the group of interacting proteins listed in table S2 and/or S6 in the presence or absence of a potential modulator of interaction; and
(b) identifying compounds capable of modulating said interaction

22. The method of claim 21, further comprising
(a) modeling said compound by peptidomimetics; and
(b) chemically synthesizing the modeled compound

23. The method of claims 21 or 22, wherein said compound is further modified to achieve
(i) modified site of action, spectrum of activity, organ specificity, and/or
(ii) improved potency, and/or
(iii) decreased toxicity (improved therapeutic index), and/or
(iv) decreased side effects, and/or
(v) modified onset of therapeutic action, duration of effect, and/or
(vi) modified pharmakinetic parameters (resorption, distribution, metabolism and excretion), and/or
(vii) modified physico-chemical parameters (solubility, hygroscopicity, color, taste, odor, stability, state), and/or
(viii) improved general specificity, organ/tissue specificity, and/or
(ix) optimized application form and route
by
(i) esterification of carboxyl groups, or
(ii) esterification of hydroxyl groups with carbon acids, or
(iii) esterification of hydroxyl groups to, e.g. phosphates, pyrophosphates or sulfates or hemi succinates, or
(iv) formation of pharmaceutically acceptable salts, or
(v) formation of pharmaceutically acceptable complexes, or
(vi) synthesis of pharmacologically active polymers, or
(vii) introduction of hydrophilic moieties, or
(viii) introduction/exchange of substituents on aromates or side chains, change of substituent pattern, or
(ix) modification by introduction of isosteric or bioisosteric moieties, or
(x) synthesis of homologous compounds, or
(xi) introduction of branched side chains, or
(xii) conversion of alkyl substituents to cyclic analogues, or
(xiii) derivatisation of hydroxyl group to ketales, acetales, or
(xiv) N-acetylation to amides, phenylcarbamates, or
(xv) synthesis of Mannich bases, imines, or
(xvi) transformation of ketones or aldehydes to Schiffs bases, oximes, acetales, ketales, enolesters, oxazolidines, thiozolidines or combinations thereof.

24. A method of diagnosing a disease in a biological sample comprising the steps of
(a) contacting the sample with an antibody specific for a protein selected from any one of tables S1, S2 or S6 or an antibody specific for the protein complex of claim 18; and
(b) detecting binding of the antibody to a protein complex,
wherein the detection of binding is indicative said disease or of a predisposition to develop said disease.

25. A diagnostic agent/composition or pharmaceutical composition comprising the nucleic acid molecule of any one of claims 10 to 12 or the protein of claim 16 or 17 or the protein mentioned in any one of tables S1, S2 or S6 , the antibody of claim 19 or 20, and /or an antibody specifically reacting with a protein selected from any one of tables S1, S2 or S6.

26. Use of the nucleic acid molecule of any one of claims 10 to 12, the protein of claim 16 or 17 or the protein mentioned in anyone of tables S1, S2 or S6, the antibody of claim 19 or 20, an antibody specifically reacting with a protein selected from tables S1, S2 S6, for the preparation of a pharmaceutical composition for the treatment of a disease.
